# EUROPEAN PATENT APPLICATION

(11) **EP 4 520 348 A1**
(43) Date of publication of application: **12.03.2025**
(21) Application number: 23799296.1
(22) Date of filing: 05.05.2023
(51) Int. Cl.: A61K 45/00, A61K 47/54, A61K 47/61, A61K 47/64, A61K 31/20, A01P 1/00, A01P 3/00, A61P 31/04, A61P 31/10, A61P 31/12, A61P 31/16, A61P 31/20, A61P 31/18, A61P 31/22

(54) **CARBON CHAIN SUBSTANCE FOR REGULATING TRANSMEMBRANE TRANSPORT AND FLUIDITY OF CELL MEMBRANES, AND PREPARATION AND USE THEREOF**

(30) Priority: 06.05.2022 CN 202210484471; 06.05.2022 CN 202210483104; 06.05.2022 CN 202211331633
(71) Applicant: Sunforest (Beijing) Biopharma Ltd, Beijing 100085 (CN)
(72) Inventor: ZHAO, Yilin, Xiamen, Fujian 361104 (CN); CHEN, Weibin, Xiamen, Fujian 361104 (CN); ZHOU, Yuanyuan, Xiamen, Fujian 361104 (CN); LIU, Fengwu, Xiamen, Fujian 361104 (CN); LIU, Hongyi, Xiamen, Fujian 361104 (CN); ZHOU, Xu, Xiamen, Fujian 361104 (CN); WAN, Zheng, Xiamen, Fujian 361104 (CN); MA, Lin, Xiamen, Fujian 361104 (CN)
(74) Representative: karo IP
(86) International application number: PCT/CN2023/092349
(87) International publication number: WO 2023/213310

(57) **Abstract**

A water-soluble carbon chain substance for regulating transmembrane transport and fluidity of cell membranes, and the preparation and use thereof. The water-soluble carbon chain substance comprises a carbon chain which originally has a certain water solubility, and the structure thereof comprises at least one or more hydrophobic carbon chain parts and one or more hydrophilic groups or residues; and also comprises a high-hydrophilicity complex formed by coupling a hydrophobic carbon chain part to a water-soluble molecule; the water-soluble carbon chain can be used for preventing and treating infection including viruses, bacteria and fungi, anti-ageing, and preventing and reducing the occurrence of neurodegenerative diseases such as Alzheimer's disease; by reducing the non-specific phagocytosis of the nano-drug by cells, the effect of the nano-drug on target lesions is improved.

## Description

### Technical Field

The present invention relates to the field of pharmaceuticals, and relates to a water-soluble carbon chain affecting the structural function of cell membranes and the substance transmembrane transport; in particular, it relates to a carbon chain substance, including a complex that can be used for preventing and treating viral, bacterial, and fungal infections, anti-ageing and anti-inflammatory, and to a process for the preparation of the complex, the use of the prepared complex in preventing and treating of viral, bacterial and fungal infectious diseases and in the preparations of anti-ageing and anti-inflammatory agents.

### Background Art

### I. Structure and function of cell membrane

Cell membranes are one of the most important structures in organisms, which are mainly composed of lipid bilayer and its embedded proteins, saccharides, cholesterol, and the like. The lipid bilayer is composed of two mutually parallel layers of phospholipid molecules, each layer of phospholipid molecules having a polar head being hydrophilic at one end and a non-polar tail being hydrophobic at the other end. Some phospholipid molecules also contain a variety of groups such as choline and glycerol, which make the arrangement and spatial structure of phospholipid molecules complex. The lipid bilayer has many proteins embedded therein, either across the entire membrane layer, cross the layer, or partially embedded therein. These proteins vary in function and are, for example, messenger receptors, ion channels, enzymes, or membrane structural proteins. In addition to lipids and proteins, cell membranes contain molecules such as saccharides and cholesterols, which combine with protein or phospholipid molecules through chemical reactions such as glycosylation or esterification to form complex glycoproteins and lipoproteins.

Overall, the structure of the cell membrane is a semi-permeable barrier that can control and regulate the ingress and egress of substances, and transmit information between inside and outside the cell. The special structure and function of the cell membrane enable it to play an important role in life activities.

The cell membrane is the outer layer of all cells and has multiple functions, including cell boundary: the cell membrane is the boundary between the cell and the external environment, which isolates the inside and outside of the cell and maintains the stability of the internal environment of the cell. Substance exchange: the cell membrane can control the ingress and egress of substances, allowing cells to obtain necessary substances and eliminate waste and toxins. This is accomplished through channels and carrier proteins in the cell membrane. Signaling: receptor proteins on the cell membrane can interact with external molecules and transmit information to the interior of the cell. This can help the cells respond appropriately to external stimuli. Cell adhesion: the cell membrane can adhere cells together to form tissues and organs. Membrane fluidity: the cell membrane is a dynamic structure whose composition and morphology can be tailored to different environmental and physiological conditions by internal transport and membrane flow. The cell membrane plays a very important role in maintaining cell life and adapting to the environment.

### II. Cell membrane fluidity

Cell membrane fluidity refers to the flowing property of a cell membrane, i.e. lipid molecules in the cell membrane can freely diffuse and rotate on a plane. The cell membrane fluidity is closely related to its function because it can affect the conformation and distribution of membrane proteins on the cell membrane, thereby regulating cell signal transduction and ingress and egress of substances. In addition, cell membrane fluidity can also affect cell morphology and movement, and participate in cell adhesion and movement. For example, cholesterols on cell membranes can affect the cell membrane fluidity, thereby regulating signal transduction and adhesion of cells. Thus, cell membrane fluidity is an important aspect of the cell membrane functions. This fluidity is due to the phospholipid bilayer structure in the cell membrane and the dynamic action of proteins. The cell membrane fluidity is crucial for many biological processes of cells, such as cell signaling, cell movement, and cell phagocytosis. Since fluidity affects the orderliness of the lipid bilayer inside the cell membrane, it may affect the distribution and localization of enzymes on the membrane, thereby affecting its activity. In addition, changes in cell membrane structure and enzyme activity may also affect biological cell growth and development, cell signaling, and other processes. This effect is usually dependent on the specific function of the enzyme. For example, enzymes may require specific enzyme active sites or cofactors dependent on certain enzymes. Thus, changes in the cell membrane fluidity may directly or indirectly affect the activity of cell membrane enzymes, thereby affecting the physiological and biochemical processes of cells.

The cell membrane is an important barrier between the internal and external environment of a cell, consisting of a phospholipid bilayer and associated proteins. If the fluidity of the cell membrane decreases, it may lead to the following consequences: the transport of substances within the cell is obstructed: the decreased fluidity of the cell membrane affects the activity and fluidity of transport proteins on the membrane, thereby hindering the transmembrane transport of substances and the decrease in transport speed. Reduction of cell signaling: the activity of many receptors and signaling molecules on the membrane is associated with membrane fluidity, which, if decreased, may decrease the efficiency of cell signaling. Changes in cytoskeleton: the decreased fluidity of the cell membrane may affect the interaction of proteins on the cell membrane with the cytoskeleton, possibly leading to altered cytoskeleton. Reduced ability of cells to adhere and migrate: the decreased fluidity of the cell membrane may affect cell adhesion and hinder the transport of substances: proteins and lipids within the cell membrane can transfer substances from the outside of the cell to the inside of the cell by free diffusion, etc. When the fluidity of the cell membrane is decreased, these processes are hindered, thereby affecting the normal metabolism and function of the cell. Increased membrane fragility: the decreased fluidity of the cell membrane leads to a more compact arrangement of phospholipid molecules in the cell membrane, which makes the cell membrane more fragile and breakable and easily destroyed by substances in the external environment. Affecting cell signaling: the cell membrane is an important part of cell signal transduction, and signal molecules need to bind to receptors on the cell membrane to transmit signals. As the cell membrane fluidity decreases, the distribution and structure of these receptors may change, thereby affecting the normal process of cell signal transduction. Increasing membrane permeability: the decreased fluidity of the cell membrane may increase the permeability of the cell membrane, thus leading to the disorder of the substance exchange process in the internal and external environment of the cell and affecting the normal function of a cell. The decreased the fluidity of the cell membrane affects the activity of enzymes on the membrane. Membrane enzymes are usually embedded in the cell membrane, and the fluidity of the cell membrane affects the conformation and spatial orientation of the enzyme, thereby affecting its activity. As cell membrane fluidity decreases, the activity of these enzymes may decrease or even completely stop. Therefore, the fluidity of the cell membrane is crucial for many biological processes in cells.

Increased membrane fluidity may have some effects on cells, including increased plasticity of the cell membrane: the increased fluidity of the cell membrane means that the cell membrane becomes more plastic. This may make the cells more adaptable to environmental changes, such as cell migration and morphological changes. Increased membrane permeability: increased membrane fluidity may result in increased membrane permeability, making the cells more susceptible to toxins, viruses, and other external factors. Enhanced membrane protein transport: increased membrane fluidity can enhance membrane protein transport. This may increase the rate of absorption and excretion of certain substances, and may also increase the rate of entry of harmful substances into cells. Signal transduction disorders: increased membrane fluidity may interfere with intercellular signaling. This may lead to problems with cell growth, differentiation, and apoptosis. Increasing membrane fluidity may affect the enzyme activity of the cell membrane, as changes in the internal and external environment of the cell membrane can alter the structure and function of membrane proteins. There are many enzymes in the cell membrane that require the support of the cell membrane lipid environment to exert their enzymatic activity. Changes in cell membrane fluidity may affect the composition and structure of the lipid environment, thereby affecting the activity and specificity of cell membrane enzymes.

### III. Transmembrane transport of cell membrane

Transmembrane transport of a cell membrane refers to the process by which a substance enters or leaves a cell through different mechanisms at the cell membrane. Transmembrane transport modes include active transport, passive transport, and endocytosis. 1. Active transport: it is desirable to consume cellular energy to transport substances from low-concentration areas to high-concentration areas. The active transport can be divided into in-situ active transport and secondary active transport. In-situ active transport: the ions are transferred from the low-concentration region to the high-concentration region by an ion pump. For example, a sodium potassium pump transports three sodium ions from inside the cell to the outside, while transporting two potassium ions from the outside to the inside. Secondary active transport: use the concentration difference created by in situ active transport to transport other substances. For example, glucose transporters use intracellular ATP enzymes to transport glucose from regions of low concentration to regions of high concentration. Passive transport: there is no need to consume cellular energy to transport substances from high-concentration areas to low-concentration areas. Passive transport can be divided into two types: simple diffusion and carrier-mediated diffusion. Simple diffusion: non-polar or slightly polar small molecules pass freely through the lipid bilayer of the cell membrane. For example, oxygen, carbon dioxide, and the like. Carrier-mediated diffusion: specific substances are transported from high-concentration areas to low-concentration areas by transmembrane proteins without consuming cellular energy. For example, water molecules are transported through water channel proteins, and ion channels are transported through ion channel proteins. Endocytosis and exocytosis refer to the uptake or removal of substances by cells through the formation of vesicles. Endocytosis and exocytosis proceed by different mechanisms. Endocytosis refers to the process by which cells fuse or invaginate substances within vesicles and introduce them into the cell. For example, the process of phagocytosing bacteria by cells.

Exocytosis refers to the process in which cells encapsulate substances inside vesicles and then expel them from the outside of the cell through membrane separation or protrusion. For example, the process of releasing insulin by secreting cells. Transmembrane transport refers to the transport of biological macromolecules (such as proteins, nucleic acids, etc.) into and out of cells or different organelles or regions within cells in a certain way on the cell membrane.

Transmembrane transport of substances refers to the process by which substances pass across the cell membrane through the mediation of membrane proteins. The following are factors that affect the transmembrane transport of substances. 1. Concentration gradient: the concentration gradient refers to the difference in concentration between two sides of a substance, where the high concentration side can diffuse towards the low concentration side. Such a concentration gradient may facilitate transmembrane transport of the substance, particularly passive transport that does not require energy. 2. Molecular size and shape: molecular size and shape can affect the ability of a substance to pass through a membrane channel or carrier protein. Small molecules can pass through the pores of the membrane, while large molecules need to be transported across the membrane by carrier proteins. 3. Hydrophobicity: the membrane is composed of lipid bilayers, while hydrophobic substances more readily pass through this barrier. Water-soluble substances need to be transported across the membrane by carrier proteins. 4. Electrochemical gradient: electrochemical gradient refers to the difference in charge between two sides of a substance, and the charged substances are affected by electric field forces. This electrochemical gradient can affect transmembrane ion channels and electrokinetic-driven passive transport. 5. Number and activity of carrier proteins and channel proteins: carrier proteins and channel proteins are important factors that promote transmembrane transport, and their quantity and activity can affect the transmembrane transport rate of substances. In summary, transmembrane transport of substances is a complex process involving the interaction of multiple factors. Different substances and cell types may respond differently to these factors.

### IV. Cell membrane fluidity and transmembrane transport

The fluidity of the cell membrane is one of the important characteristics of the structure and function of the cell membrane. The fluidity of the cell membrane refers to the free movement of the phospholipid bilayer in the cell membrane on the cell surface due to thermal movement between phospholipid molecules. Increased or decreased fluidity of cell membranes can affect the transmembrane transport of substances. The main component of the cell membrane is the phospholipid bilayer, in which phospholipid molecules have fluidity. The free movement of phospholipid molecules allows cell membranes to change shape and size as cells are required to accommodate different cellular physiological and biochemical activities. If the fluidity of the cell membrane is increased, the movement of phospholipid molecules is accelerated and the fluidity of the cell membrane is increased. In this case, the conformation of membrane proteins (e.g. channel proteins, carrier proteins, etc.) on the cell membrane can be altered to affect their function. The transmembrane transport of substances requires crossing the cell membrane, and the increased fluidity of the cell membrane can increase the movement of lipid molecules on the membrane surface, thereby increasing the diffusion speed of substances on the membrane surface and reducing the friction between substances and the membrane surface, thereby promoting the transmembrane transport of substances. In addition, many cell membrane proteins can promote the transmembrane transport of substances by changing their conformation, and increased membrane fluidity can also affect the conformational changes of these proteins. Therefore, the increased fluidity of the cell membrane can promote the transmembrane transport of substances in various ways. The decreased fluidity of the cell membrane may affect the transmembrane transport of substances, as the function of many transmembrane transport proteins depends on the fluidity of the cell membrane. For example, some ion channel proteins open and close their channels through the fluidity of the cell membrane, while some transport proteins also require the fluidity of the cell membrane to transport substances from one side to another. Thus, if cell membrane fluidity is reduced, the function of these proteins can be affected, resulting in restricted transmembrane transport of substances.

### V. Cell membrane fluidity and cell division proliferation

Cell division is one of the most important events in the cell life cycle and refers to the division of a cell into two or more daughter cells. Cell division includes two types: mitosis and meiosis. In mitosis, the chromosomes of a cell replicate and arrange in the cytoplasm into a structure called the spindle, which are then separated into two daughter cells. In meiosis, the number of chromosomes is halved, yielding four cells for the reproductive process.

Cell membrane fluidity also plays an important role in cell division. Before cell division, the cell membrane needs to grow and expand in order to envelop two new cells during division. The fluidity of the cell membrane allows it to expand and change shape, thereby helping the cell to complete the process. In addition, the fluidity of the cell membrane can enable cells to better perceive their surrounding environment during division and adjust their behavior to adapt to new environments. Thus, the fluidity of the cell membrane plays a crucial role in cell division.

In cell division, increased membrane fluidity may play a certain role. Cell division is a complex biological process, including various stages of the cell cycle, cytoskeletal changes, chromosome segregation, and cytoplasmic division. In this process, the increased fluidity of the cell membrane may contribute to the change of cell morphology and the progress of cytoplasmic division. In addition, increased cell membrane fluidity can be associated with biological processes such as cell signal transduction, recombination of cell membrane proteins, etc.

However, cell division and proliferation are not solely dependent on increased membrane fluidity. Cell division and proliferation also involve many other biological factors, such as regulation of the cell cycle, DNA replication and repair, apoptosis, etc. Thus, flow from the cell membrane alone
Decreased membrane fluidity may affect cell division and proliferation. Cell membranes are composed of lipid bilayers containing many different lipid molecules such as phospholipids, cholesterol, etc. The fluidity of these lipid molecules can affect the structure and function of the cell membrane, thereby affecting various physiological processes of a cell.

Decreased fluidity of the cell membrane may affect cell division and proliferation because cell membranes play an important role in these processes. For example, during cell division, the cell membrane needs to undergo major changes to form two new cells. The decreased fluidity of the cell membrane may hinder this process, thereby affecting cell division. In addition, the fluidity of the cell membrane is also associated with cellular signaling, and cell division and proliferation are also dependent on complex signaling pathways.

However, the exact relationship between reduced cell membrane fluidity and cell division and proliferation is not fully understood because this process is very complex and is influenced by many different factors. More research is needed to determine the specific relationship between cell membrane fluidity and cell division and proliferation.

### VI. Cell membrane fluidity and cell senescence

The cell membrane is a critical barrier between the internal and external environment of a cell and is also the main site for molecular transmission and signal transduction within the cell. The fluidity of the cell membrane refers to the free movement and exchange of its internal molecules, which is an important factor in maintaining the structure and function of the cell membrane. Cell membrane changes associated with aging include the fluidity of the cell membrane being weakened, and the content and type of lipid molecules in the membrane being changed. These changes may lead to diminished cellular function and senescence. Studies have shown that changes in the content and type of lipid molecules in cell membranes are closely related to cell senescence. For example, as age increases, the content of saturated fatty acids in the cell membrane increases, and the content of unsaturated fatty acids decreases, which decreases the fluidity of the cell membrane, thereby affecting the function and life cycle of the cell. In addition, oxidative stress may also lead to lipid peroxidation in the cell membrane, which can damage the integrity and fluidity of the cell membrane, affecting cell function and lifespan. As cell senescence progresses, the density and activity of receptors and channels on the membrane also change, leading to a decrease in the capacity of intracellular molecular transmission and signal transduction. Therefore, maintaining the fluidity of the cell membrane can be a way to delay cell senescence.

### VII. Cell membrane fluidity and cell migration

Increased membrane fluidity may promote cell migration and movement. Cell membranes are composed of lipid bilayers in which the lipid has the fluidity. The movement of these lipid molecules is regulated by the cytoskeleton and other proteins. When a cell needs to move, it changes the structure of the cell membrane, thereby enhancing its fluidity. Enhancing the fluidity of cell membranes allows cells to move and migrate more easily. When leukocytes need to look for a source of infection in the blood, they enlarge the contact area by enhancing membrane fluidity and more easily penetrate the vessel wall. In general, enhancing cell membrane fluidity can increase the diffusion rate of proteins and other molecules on the cell membrane, thereby promoting signal transmission and cell adhesion on the cell membrane, so that cells move more easily in tissues. In addition, enhanced membrane fluidity may affect cytoskeleton and cell membrane remodeling. The cytoskeleton is an intracellular scaffolding structure that helps cells maintain shape and stability. Remodeling of cell membranes refers to the rearrangement of protein and lipid molecules on cell membranes to change cell shape. These processes are critical for both cell migration and movement. The increased fluidity of the cell membrane may facilitate both cell migration and movement because the deformation and migration of the cell membrane require the fluidity of the membrane to support. When cells need to pass through fine pores or through complex tissue structures, enhanced fluidity of cell membranes can help cells adapt to the environment and successfully complete migration. As a cell expands outward, its membrane becomes more fluid, which can make it easier for the cell to pass through narrow spaces and move within the extracellular matrix. In addition, enhanced fluidity of cell membranes also facilitates cell interaction with the surrounding environment. Receptors and channels on cell membranes can more easily interact with extracellular molecules, which can lead to changes in signaling and promote cell migration and movement. Thus, enhancement of cell membrane fluidity is critical for cell migration and movement. The movement and migration of cells require the deformation and extension of the cell membrane, which requires the fluidity and flexibility of the cell membrane. Decreased membrane fluidity may inhibit the movement and migration of the cells. For example, phosphatidylinositol 3, 4, 5-triphosphate (PIP3) is a signaling molecule that plays a critical role in cell polarity and movement. It was found that the accumulation of PIP3 at the cell front and the movement and migration of cells were inhibited when the fluidity of the cell membrane was decreased.

### VIII. Method and pathway for virus entry into cell

Transmembrane transport of the virus refers to the process in which a virus enters a cell and, through a series of chemical and physical processes, crosses its genetic materials or proteins from outside the cell membrane and enters the interior of the cell. Viruses enter cells in a variety of ways, including 1. membrane fusion: membrane proteins on the surface of the virus can bind to the corresponding proteins on the cell membrane, thereby fusing the virus with the cell membrane and entering the cell interior. 2. Endocytosis: viruses recognize and enter cells by utilizing receptor proteins on the cell surface. Once the virus binds to the cell receptor, the cell membrane forms vesicles that encapsulate the virus, thereby endocytosing the virus into the interior of the cell. 3. Direct penetration: certain viruses, such as poxviruses, can enter the interior of a cell by directly penetrating the cell membrane without the need for membrane fusion or endocytosis. Either way, after the virus enters the cell, the genetic materials of the virus will be released, thereby beginning to infect the cell and replicate itself.

### IX. Method and pathway for bacteria and fungi entry into cell

Bacteria enter cells through several ways: 1. Endocytosis: some bacteria can enter cells through endocytosis. Endocytosis is the process by which a cell encapsulates substances from the outside of the cell through a cell membrane and then transports them to the inside of the cell. Bacteria can enter cells by this process, and the most common way is "triggered endocytosis". 2. Across cell membranes: some bacteria can enter cells directly through cell membranes, such as *rickettsia*, mycoplasma, etc. 3. Active invasion: some bacteria can invade cells by means of active activity using structures such as flagella, cilia, or pseudopodia. 4. Toxins: some bacteria secrete toxins that enter cells by disrupting cell membranes or otherwise. These toxins can enter cells by endocytosis or other means. It should be noted that different types of bacteria will choose different modes of invasion and that the mode of invasion will also be influenced by the interaction between the bacteria and the host cell.

Fungi enter cells primarily thought two ways: 1. Direct penetration: fungi penetrate the cell membrane and cell wall of host cells directly into the cell interior by secreting enzymes and toxins. 2. Endocytosis: fungi, through some special structures, such as hyphae and pseudopodia, encapsulate the host cells to form a "food vacuole", and then use endocytosis to phagocytose the substances inside the host cells into the interior of fungal cells. Either way, fungi enter host cells and utilize their metabolites and organelle structures for nutrient uptake and growth, resulting in cell damage or death, causing diseases.

X. Chronic and recurrent viral infections can lead to a variety of chronic diseases, depending on the type of virus-infected and the immune status of the individual. The following are some common chronic diseases associated with viral infections: hepatitis B and C viruses (HBV, HCV) are common chronic viral infections that can lead to hepatitis and cirrhosis. Respiratory viral infections, such as influenza virus, coronavirus, etc. can cause chronic bronchitis and emphysema. Certain viral infections, such as the Coxsackie virus and German measles virus, are thought to cause damage to pancreatic cells and inadequate insulin secretion, thereby increasing the risk of diabetes. Human immunodeficiency virus (HIV) infection may lead to acquired immunodeficiency syndrome (AIDS) and other immune system-related diseases. Varicella-Zoster Virus (VZV) infection can lead to herpes zoster and sequelae such as neuralgia. Parainfluenza virus infection can lead to asthma and chronic obstructive pulmonary disease (COPD). Human HCMV infection may lead to diseases in immunosuppressed patients, such as infection and reactivation after organ transplantation.

### XI. Compounds affecting cell membrane

Compounds that affect cell membrane fluidity are numerous. Common compounds that can affect cell membrane fluidity include fatty acids: phospholipid molecules in cell membranes consist of two hydrophobic fatty acids and one hydrophobic phosphorylcholine or phosphoethanolamine molecule. Fatty acids of different lengths, degrees of unsaturation, and patterns of distribution can affect the fluidity of membranes, whereas unsaturated fatty acids can make membranes more fluid. Cholesterol: cholesterol is one of the most common lipids in cell membranes, which can increase the stability and rigidity of the membrane. Cholesterol reduces membrane fluidity at low temperatures and increases membrane fluidity at high temperatures. Phosphatidylinositol: phosphatidylinositol is a minor component in cell membranes that regulates cell signaling and cell membrane fluidity. Phospholipids: cell membranes are mainly composed of phospholipids, and different kinds of phospholipids can affect the fluidity of cell membranes. Alcohol: alcohol can disrupt the structure of cell membranes, affecting their fluidity and stability. Squalene: squalene is a cholesterol-like substance that increases cell membrane fluidity and improves cell membrane softness. Choline: choline is a member of the vitamin B group that promotes cell membrane fluidity and has antioxidant activity.

Tartaric acid: it is a natural organic acid that reduces the fluidity of cell membranes, thereby affecting cell function. Citric acid: it is another natural organic acid that can also reduce cell membrane fluidity. Surfactants: these compounds can interact with cell membranes and reduce their fluidity. Non-ionic surfactants: these compounds have a similar action as surfactants, but interact more gently with cell membranes.

Among the numerous microorganisms, the pathogenic microorganisms capable of directly causing human diseases are generally viruses, bacteria, and fungi. Except for a few non-enveloped viruses, most microorganisms have lipid membranes, and the lipid membranes of microorganisms have the same function as the cell membranes of other organisms. In the structure, the phospholipid bilayer constitutes the basic scaffold of the membrane, and proteins penetrate, intercalate, and attach to the surface of the phospholipid bilayer. There are glycoproteins composed of proteins and a small number of polysaccharides on the outer surface of the membrane. Some saccharides also combine with lipids to form glycolipids. The lipid membrane of microorganisms is usually 7-8 nm in size and has a certain fluidity. It not only serves as a barrier to creating a stable internal environment for microbial life activities, but also has semi-permeability or selective permeability, which selectively allows substances to enter cells through diffusion, permeation, and active transport, thereby ensuring the normal metabolism of cells. The present invention is directed to a group of complexes and preparations thereof that target disruption and influence the structure and function of microbial lipid membranes or non-enveloped viral nucleocapsids.

### 1. Virus

### 1.1 Enveloped virus and non-enveloped virus

Viruses are a type of infectious particle that is the smallest, consisting of one or more nucleic acid (DNA or RNA) molecules wrapped in a protein coat. They are non-cellular microorganisms that must replicate within susceptible living cells. Outside the cell, the virus exists in a granular form and the structurally intact infectious virus particles are called virions. The viral genome is enveloped by a protein coat called the nucleocapsid, while the protein coat is called the capsid. The basic structure of virions is the nucleocapsid, but some viruses also have a bilayer lipid envelope outside the nucleocapsid, such viruses are called enveloped viruses, while viruses without an envelope are correspondingly called naked viruses.

### 1.2 Virus envelope structure and function

The envelope is the cytoplasmic membrane obtained when the virus is released from the host cell, and may also be an intracellular organelle membrane or a nuclear membrane, so that the virus envelope has certain properties of the host cell membrane, allowing the virus to exhibit a specific "tropism" for the host cell membrane. The envelope contains a bilayer of lipids, as well as a number of proteins encoded by viral genes, called envelope proteins, which are virus-specific and often form glycoprotein subunits with polysaccharides, which are embedded in the lipid layer and have a spined surface called "spike or vesicular particle". They are located on the surface of virosomes, are highly antigenic, and bind selectively to host cell receptors, promoting fusion of the viral envelope with the host cell membrane and entry of the infectious nucleocapsid into the cell, leading to infection. Therefore, the envelope protein of enveloped viruses determines their infectivity, and the nucleocapsid of the enveloped virus is the virus core, which is non-infectious when it exists alone or loses its envelope.

### 1.3 Structure and function of non-enveloped virus

Due to the lack of an envelope, naked viruses have a mature nucleocapsid, and their infectivity is determined by the capsid protein. The capsid protein is a viral gene product that can give the virus its inherent shape and protect the internal nucleic acid from damage by nucleases in the external environment such as blood. At the same time, the capsid protein has an auxiliary infection effect. The virus surface-specific receptor edge binding protein has a special affinity with the corresponding receptors on the cell surface, which is the primary step for the virus to selectively adsorb host cells and establish infection foci. The capsid protein also exhibits virus-specific antigenicity, which can stimulate the body to produce antigen virus immune responses.

### 1.4 Enveloped virus species

Envelope-containing viruses include influenza virus, coronavirus, HIV, hepatitis B virus, hepatitis C virus, rabies virus, herpes virus, Ebola virus, Hantavirus, Dengue virus, Japanese encephalitis virus, Zika virus, etc.

The immune system relies on proteins on the cell membrane to recognize friend or foe, and enveloped viruses are recognized by the host immune system as friendly precisely because they have an additional layer of lipid membrane. The glycosylation modification of envelope proteins, on the one hand, exerts an antigen-shielding effect, making vaccine development more difficult; on the other hand, modified polysaccharides also have a spatial reconstruction effect on the antigenic epitope structure.

### 1.5 Coronavirus

Coronavirus: there are currently 7 coronaviruses that can infect humans, which are HCoV-229E, HCoV-OC43, SARS-CoV, HCoV-NL63, HCoV-HKU1, MERS-CoV, and SARS-CoV-2, respectively. Coronaviruses are approximately 60-220 nm in diameter. The virus has an envelope structure with three proteins: spike glycoprotein (S protein), small envelope glycoprotein, and membrane glycoprotein (M protein). A few species also have hemagglutinin glycoprotein (HE protein). S protein plays a crucial role in recognizing and binding to host cell surface receptors and mediating the fusion of viral envelope and cell membrane. The M protein is involved in the formation and budding process of the viral envelope. HE protein is a short protrusion that constitutes the envelope, which can be related to the early adsorption of coronaviruses. The HE protein of some coronaviruses can cause agglutination of red blood cells and adsorption of red blood cells.

### 1.6 Treatment of coronavirus

Currently, COVID-19 vaccines and therapeutic drugs under clinical research are mainly divided into the following four types:
The first type is small molecule antiviral drugs: including, e.g. Molnupiravir from Merck Sharp& Dohme (MSD), Paxlovid from Pfizer, Ensitrelvir from SHIONOGI, and marketed drugs such as Remdesivir, lopinavir/Ritonavir, Favipiravir, etc. Small molecule drugs such as lopinavir/Ritonavir, although are widely used in antiviral treatment, they are not specific drugs for novel coronavirus.

The second type is anti-inflammatory drugs: various biological agents are used to inhibit inflammatory factor storms, such as Tocilizumab, Siltuximab, etc. There are also some clinical trials of small-molecule anti-inflammatory drugs, such as Baricitinib, Ruxolitinib, etc.

The third type, neutralizing antibodies: refers to the antibody that can eliminate the ability of virus infection after binding to a virus. The mechanism is to change the surface configuration of the virus, prevent the virus from adsorbing to susceptible cells, and prevent the virus from penetrating into cells for proliferation. The immune complex formed by virus and neutralizing antibodies is easily phagocytosed by macrophages.

The fourth type is vaccines: including, e.g. recombinant protein vaccines, nucleic acid vaccines, viral vector vaccines, inactivated vaccines, and live attenuated vaccines.

### 1.7 Non-enveloped virus

Non-enveloped naked viruses include hepatitis A virus, human papilloma virus, adenovirus, polio virus, Coxsackie virus, etc.

Human papilloma virus (HPV) belongs to the genus Papillomavirus in the family Papillomaviridae. It is a spherical, non-enveloped double-stranded DNA virus with a diameter of 52-55 nm. The viral genome is a double-stranded circular DNA, about 7.8-8.0 kb, which is divided into an early region, a late region, and a regulatory region. The early region encodes proteins involved in viral replication, transcriptional regulation, and cellular transformation (e.g. E5, E6, E7), and the late region encodes the major capsid protein L1 and the minor capsid protein L2. At present, more than 130 types have been isolated, each causing different clinical manifestations. According to the different sites of invaded tissue, they can be divided into: skin low-risk type, skin high-risk type, mucosal low-risk type, and mucosal high-risk type. Skin-type infections in the HPV population are very common, such as common verruca vulgaris, verrucaplantaris, verruca plana, etc. but specific infection rates cannot be obtained.

### 2 Bacteria

The basic structure of bacteria includes the cell wall, cell membrane, cytoplasm, and nucleoplasm.

### 2.1 Bacterial cell membrane

Bacterial cell membranes are elastic, semi-permeable membranes composed of phospholipid bilayers and embedded proteins. The membrane thickness is 8-10 nm and the outer side is closely linked to the cell wall. The absence of cholesterol in bacterial cell membranes is a distinct point from eukaryotic cell membranes. Bacterial cell membranes are rich in enzymes and perform many important metabolic functions. The versatility of bacterial cell membranes is quite distinct from other cell membranes, e.g. the inner side of the cell membrane contains electron transport and oxidative phosphorylation enzymes that perform some of the functions of eukaryotic mitochondria.

Structural characteristics of bacterial cell membrane: ① the main body of the membrane is a lipid bilayer; ② the lipid bilayer has fluidity; ③ integrin can be "dissolved" in the hydrophobic inner layer of the lipid bilayer due to its hydrophobic surface; ④ the peripheral protein surface contains hydrophilic groups and can be linked to the polar head of the lipid bilayer surface by electrostatic attraction; ⑤ there is no covalent binding between lipid molecules or between lipid and protein molecules; and ⑥ the lipid bilayer is like a "sea" on which the peripheral protein can "float", while the integrin seems to be immersed in it for lateral movement.

The physiological function of bacterial cell membrane: ① selectively controlling the transport of nutrients and metabolites inside and outside the cell; ② it is a structural barrier to maintain normal osmotic pressure inside the cell; ③ it is an important site for synthesis of the cell wall and components related to saccharide coat (such as peptidoglycan, teichoic acid, LPS, and capsular polysaccharide, etc.); ④ the membrane contains enzyme system related to energy metabolisms such as oxidative phosphorylation or photophosphorylation, so it is the energy production site of the cell; and ⑤ the membrane is the birth site of a flagellar organism and can provide the energy required for flagellar rotational movement.

### 2.2 Bacterial cell wall

The major component of the cell wall is peptidoglycan, also known as mucopeptide. The peptidoglycan is a polysaccharide scaffold formed by the interlocking arrangement of two aminosaccharides, N-acetylglucosamine and N-acetylcytosine, through β-1,4 glycosidic bond. N-acetylmuramic acid molecules are linked to tetrapeptide side chains, which are linked by peptide bridges or chains to form a highly mechanical network structure.

### 2.2.1 Gram-positive bacteria

The cell wall of Gram-positive bacteria is thick, about 20-80 mm. The peptidoglycan content is abundant, with 15-50 layers and a thickness of 1 nm per layer, accounting for approximately 50-80% of the dry weight of the cell wall. In addition, there are also a large number of special components such as teichoic acid. Teichoic acid has strong acidity and is an important surface antigen for Gram-positive bacteria. It plays a role in regulating the passage of ions through the mucopeptide layer. It may also be related to the activity of certain enzymes. The teichoic acid of certain bacteria can adhere to the surface of human cells, acting similarly to pili, and can be related to pathogenicity.

### 2.2.2 Gram-negative bacteria

Gram-negative bacterial cell walls have multiple structures. The cell wall is thin, about 10-15 nm, with 1-2 layers of peptidoglycan, accounting for about 5-20% of the dry weight of the cell wall. There is also a bacterial outer membrane formed by proteins, phospholipids, and other substances outside the cell wall. The outer membrane has a lower content of phospholipids than the cytoplasmic membrane, but a higher content of lipopolysaccharides. The protein of the outer membrane differs from the cytoplasmic membrane in that the protein on the outer membrane is covalently linked at one end to the tetrapeptide side chain of the peptidoglycan by a protein moiety and at the other end to the phosphate of the outer membrane by a lipid moiety. Its function is to stabilize the outer membrane and immobilize it in the peptidoglycan layer. Lipopolysaccharide, known as bacterial endotoxin, is present in the outermost layer of the outer membrane. The outer membrane is the main structure of the cell wall of Gram-negative bacteria. In addition to transporting nutrients, it also has a barrier effect that can prevent various substances from passing through and resist the effects of many chemical drugs.

### 2.3 Antibiotics

Antibiotics are mainly secondary metabolites or artificially synthesized analogs produced by bacteria, molds, or other microorganisms, and are mainly used for the treatment of various bacterial infections or diseases caused by pathogenic microorganisms, and generally have no serious side effects on their host. The mechanism of action of antibiotics is generally to hinder the synthesis of bacterial cell walls, leading to bacterial expansion, rupture, and death in low osmotic pressure environments; interact with bacterial cell membrane, enhance the permeability of bacterial cell membrane, open ion channels on the membrane, allowing useful substances inside the bacteria to leak out of the bacterial body or cause electrolyte imbalance and death; interact with bacterial ribosomes or their reactive substrates (such as tRNA, mRNA) and inhibit protein synthesis, resulting in the inability to synthesize structural proteins and enzymes necessary for cell survival; obstruct the replication and transcription of bacterial DNA and hinder the process of bacterial cell division, reproduction, and transcription into proteins.

### 2.4 Antibiotic resistance

It is well known that over-range, high-dose, long-term use of antibiotic drugs can lead to the development of resistance. Humans constantly develop new antibiotics to combat pathogenic microorganisms, while bacteria and other microorganisms gradually adapt to this drug environment and constantly mutate to form new and more powerful bacteria in order to survive, thus repeating the cycle. Even multidrug-resistant bacteria have emerged, where a single bacterium is resistant to three or more types of antibiotics simultaneously. Further research has found that bacteria exhibit drug resistance due to the presence of resistance genes in their bodies. NDM-1 is a new super-drug resistant gene discovered by scientists, encoding a new drug-resistant enzyme NDM-1, known as "New Delthi Metallo-bata-Lactamase 1", which is a highly efficient enzyme that can break down most antibiotics and lose its efficacy. Drug-resistant genes can not only cause bacterial resistance but also spread in the environment, transfer to other bacteria, and become resistant bacteria resistant to antibiotics. Due to the lack of new antibiotics and the inability of existing antibiotics to effectively kill drug-resistant bacteria, once infected, drug-resistant bacteria greatly increase the risk of patient death. The mortality rate of patients infected with drug-resistant bacteria is approximately twice that of those infected with non-drug-resistant bacteria. Therefore, the infection and transmission of drug-resistant bacteria have become a major challenge in the contemporary medical field.

### 3. Fungi, chlamydia, and mycoplasma

The basic structures of fungal cells include a cell wall, cell membrane, nucleus, endoplasmic reticulum, mitochondria, etc. The main component of the fungal cell wall is chitin, and the fungal cell membrane is also composed of phospholipid bilayers. However, the plasma membrane contains sterols and ergosterol that play an important role in maintaining membrane permeability and fluidity. There are three classes of antifungal drugs: polyenes (amphotericin B preparations), triazoles (voriconazole, itraconazole, posaconazole), and pneumocandins (caspofungin, micafungin, anidulafungin).
Chlamydia is a Gram-negative pathogen with a cell wall and membrane but without peptidoglycan. It is supported by peptides linked by disulfide bonds. Mycoplasma, which has no cell wall and only a cell membrane, is composed of a phospholipid bilayer and has some effect on maintaining the integrity of the cell membrane.

### 4. Taking the lipid membrane component of the microorganism as the target

The main structure of microbial lipid membrane is a phospholipid bilayer, and the main components are phospholipids, proteins, and polysaccharides, which can play an anti-microbial role by destroying the continuity and stability of microbial lipid membrane, causing changes in membrane permeability and enhancing permeability.

### 4.1 Structure and composition of microbial cell membrane

The microbial lipid membrane generally has a thickness of 7-8 nm. Lipid membranes are mainly composed of lipids and proteins. Lipid accounts for 50%, protein accounts for 40%, and polysaccharide accounts for about 1-10%. Membrane lipids mainly include phospholipids and glycolipids, in which phospholipids account for more than 50% of membrane lipids. Phospholipids are mainly glycerophospholipids, which use glycerol as the skeleton and bind two fatty acid chains and a phosphate group on the skeleton. Molecules such as choline, ethanolamine, serine, or inositol are connected to lipid molecules through phosphate groups. The hydrophilic end of phospholipid molecules is the phosphate group, known as the head. The hydrophobic end of phospholipid molecules consists of two hydrocarbon chains of varying lengths, known as tails, typically containing 14-24 even carbon atoms. One of the hydrocarbon chains often contains one or several double bonds, which causes the unsaturated chain to twist at a certain angle. Glycolipids accounted for less than 5% of membrane lipids. The simplest glycolipid is galactocerebroside. It only has one galactose as the polar head. The function of glycolipids is to integrate membrane proteins.

### 4.2 Microbial membrane lipids

They are basic skeletons constituting a membrane, and when membrane lipids are removed, the membrane is disintegrated. Membrane lipids are the solvents of membrane proteins. Some proteins interact with membrane lipids through hydrophobic ends, so that the proteins are embedded in the membrane to perform special functions. Membrane lipids provide an environment for certain membrane proteases to maintain conformation and exhibit activity. The activity of many enzymes on membranes depends on the presence of membrane lipids.

### 4.3 Microbial membrane proteins

They account for 40%-50% of the membrane. The more complex the function of the membrane, the higher the protein content on it. According to the different binding modes with membrane lipids and their positions in the membrane, membrane proteins are divided into integrin, peripheral protein, and lipid anchoring protein. Some or all the integrin proteins embedded in or on both sides of the cell membrane bind tightly to the membrane and can only be washed from the membrane with detergents. SDS and Triton-X100 are commonly used. Peripheral proteins, also known as extrinsic proteins, are water-soluble and distributed on the surface of cell membranes. By binding to the hydrophilic parts of protein molecules or lipid molecules on the membrane surface through ionic bonds or other weaker bonds, separation from the membrane can be achieved by changing the ionic strength of the solution or even increasing the temperature. Lipoanchored proteins: also known as adiponectin, have two ways of binding to lipids: one is through indirect binding to lipids in the lipid bilayer through a saccharide molecule, and the other is that proteins directly bind to the lipids in the lipid bilayer. Lipidanchored proteins are anchored and covalently bound, by phospholipids or fatty acids. Membrane proteins have the functions of transporting, catalyzing the related metabolic reactions, and linking proteins and receptors.

### 4.4 Microbial membrane saccharides

They account for 2%-10% of membrane components and are mainly located on the outer surface of the membrane lipid. There are 7 main types of saccharides present on animal cell membranes: D-glucose, D-galactose, D-mannose, L-fucose, N-acetylglucosamine, and N-acetylglucosamine. There are two main forms of linkage between saccharides and amino acids: N-connection: that is, the saccharide chain is connected to an asparagine residue in the peptide chain; and O-connection: the saccharide chain is linked to a serine or threonine residue in the peptide chain.

### 4.5 Asymmetry of microbial lipid membrane

There are significant differences in the composition and function of the inner and outer layers of the lipid membrane. This is called the asymmetry of the membrane. Membrane lipids, membrane proteins, and membrane saccharides are asymmetrically distributed on the membrane, resulting in asymmetry and directionality of membrane function, i.e. the fluidity of the two layers inside and outside the membrane is different, so that there is a certain direction for substance transmission, and there is also a certain direction for signal reception and transmission. The asymmetry and directionality of the membrane function ensure a high degree of order in life activities. Intercellular recognition, movement, substance transport, signal transmission, etc. all have directionality. The maintenance of these directionalities relies on the asymmetric distribution of membrane proteins, lipids, and saccharides.

### 4.6 Fluidity of microbial lipid membrane

Microbial lipid membranes have fluidity, and the membrane lipid molecules on the lipid membrane can diffuse laterally, rotate, swing, expand contract, flip, and undergo rotational isomerization. Several forms of membrane protein movement include lateral diffusion and rotational diffusion. The fluidity of the lipid membrane is a necessary condition to ensure its normal function. When the fluidity of the lipid membrane is below a certain threshold, the activity of many enzymes and the transmembrane transport will stop, otherwise, if the fluidity is too high, the lipid membrane will be dissolved.

### 5. Method and preparation for disrupting the microbial cell membrane

### 5.1 Physical disruption

The simplest method *in vitro* is to place microorganisms in distilled water, utilizing the principle of permeation to allow cells to absorb water and burst. Both low and high temperatures can damage microbial lipid membranes. Direct differential centrifugation can also disrupt the structure of lipid membranes.

### 5.2 Protease and phospholipase disruption

Proteases can catalyze the hydrolysis of proteins in the lipid membrane, thereby disrupting the lipid membrane. Phospholipids also disrupt lipid membranes by hydrolyzing phospholipids in the lipid membrane.

### 5.3 Ionic, nonionic, and zwitterionic detergents disrupting cell membranes

Detergents are amphiphilic molecules that contain both hydrophilic and hydrophobic regions, capable of disrupting the binding of proteins, protein lipids, and lipids, and denaturing proteins and other macromolecules. Ionic detergents commonly used in experiments include such as sodium lauryl sulfate (SDS), deoxycholate, cholate, and sarcosine. Common nonionic detergents include Triton X-100, DDM, digitonin, tween 20, and tween 80. Detergents are amphiphilic organic compounds consisting of a hydrophobic nonpolar hydrocarbon moiety and a hydrophilic polar group. This molecular structure is very similar to the amphipathic phospholipids that make up lipid membranes. Phospholipids have two fatty acid hydrophobic tails each linked to a hydrophilic group. When at high concentrations, amphiphilic molecules self assembles into structures, keeping their hydrophilic head groups on the outside and hydrophobic tails on the inside away from water. Due to their molecular differences, detergent molecules form spherical micelles. The similarity in molecular structure allows the detergent to penetrate the phospholipid bilayer membrane, thereby disrupting the lipid membrane.

### 5.4. In vitro bactericidal action of fatty acids

Fatty acids are a type of compound composed of carbon, hydrogen, and oxygen, and are the main components of neutral fats, phospholipids, and glycolipids. Fatty Acid can be further classified according to their carbon chain length as follows: short-chain fatty acids having less than 6 carbon atoms in the carbon chain, also known as volatile fatty acids; medium-chain fatty acids, fatty acids having 6-12 carbon atoms in the carbon chain; long chain fatty acids having more than 12 carbon atoms in the carbon chain. Fatty acids can be divided into three groups depending on the saturation and unsaturation of the hydrocarbon chain, namely: saturated fatty acids having no unsaturated bonds on the hydrocarbon; monounsaturated fatty acids having one unsaturated bond in the hydrocarbon chain; a polyunsaturated fatty acid having a hydrocarbon chain with two or more unsaturated bonds.

Fatty acids in food are re-esterified in the intestinal cells and mixed with bile salts and monoglycerides to form 4-6 nm fat particles which are directly absorbed by the intestinal epithelial cells by pinocytosis and coat the outside with a lecithin and protein membrane to become chylomicrons which enter the lymphatic system, pass through the lymphatic vessels and the thoracic duct and return to the blood circulation in the form of an oil-in-water emulsion. Most medium chain fatty acids, except for a small amount that exists in the surrounding blood for a short period of time, non-covalently bind to serum proteins and quickly reach the liver through the portal vein system. In the liver, medium-chain fatty acids can rapidly pass through the mitochondrial bilayer membrane and are rapidly acylated under the action of capryloyl CoA, while hardly being synthesized into fat. The excess acetyl CoA produced by acylation undergoes various metabolic actions in the mitochondrial cytoplasm, most of which tend to synthesize ketone bodies.

Studies have shown that the antibacterial effect of fatty acids is generally broad-spectrum. Although little is known about its antibacterial mechanism, many studies have speculated that the main targets of fatty acids are cell membranes, where fatty acids disrupt electron transport chains and oxidative phosphorylation. In addition to interfering with cellular energy production, the action of fatty acids may also be due to inhibition of enzyme activity, damage to nutrient uptake, production of peroxidation and autoxidative degradation products, or direct breakdown of bacterial cells.

It has potential for development because its mechanism of action is different from most traditional antibiotics. However, there are problems hitherto hindering progress. First, some free fatty acids have a bad taste. Second, free fatty acids are unstable, and they also have a tendency to bind non-specifically to proteins, with more fatty acids being delivered *in vivo* in the form of stable lipids (e.g. triglycerides). Third, fatty acids are fat-soluble and rapidly metabolized, making them unavailable for direct use in the body. Free fatty acids are insoluble in water or have low water solubility and cannot be directly injected into the blood circulation. Direct injection into the vein can lead to pulmonary embolism, while injection into the artery can cause tissue necrosis due to arterial embolism.

The concentrations of bacteriostatic and bactericidal fatty acids *in vitro* used in studies tend to be high, and such high concentrations must destroy the cell membranes of human cells. Human cells are also composed of phospholipid bilayers, which are targets of high-concentration fatty acid attacks. Therefore, the present invention relates to a group of stable water-soluble carbon chain complexes that can be injected intravenously, intraarterially, or orally, and change the fat-soluble hydrophobic carbon chain into water-soluble complexes capable of targeting and killing microorganisms *in vivo* through hydrophobic carbon chain covalently combined with large, medium and small water-soluble molecules, and binding molecules. At therapeutic concentrations, this group of complexes has targeted binding and killing of pathogenic microorganisms, without affecting or damaging human cellular tissues, and is not easily cleared and metabolized by the liver in the short term. The highly water-soluble and high-affinity complex of the present invention has an anti-microbial effect and can be used in intravenous injection and oral dosage forms in addition to a nasal spray and dry powder inhalant.

### Summary of the Invention

The present invention provides a complex capable of preventing, blocking, or treating microbial infections, in response to the lack of agents that have no toxic side effects and cannot widely kill, prevent, block, or treat microbial infections.

In the study on the substances for preventing, blocking, or treating microbial infections in the present invention, after careful study, it has been found that the water-soluble carbon chain substances of the present invention can affect the structural function of a cell membrane and the transmembrane transport of the substances. Furthermore, by studying and controlling the specific hydrophilicity and hydrophobicity of the carbon chain substances, and/or controlling the concentration of the carbon chain substances as required, the cell membrane of a microorganism can be disrupted so as to achieve the effect of preventing, blocking, or treating microbial infections. It is also possible to control the transport of substances across membranes by controlling the concentration within a desired range to affect the structure and function of cell membranes. It is also possible to suppress the fluidity of the cell membrane and improve its stability by controlling the concentration within the desired range, thus making it possible to develop an anti-aging or anti-inflammatory drug preparation according to the usage site.

The present invention provides a group of water-soluble carbon chain substances that affect the structure and functions of cell membranes and transmembrane transport of substances. That is, the present inventors have found that after the water-soluble carbon chain is in contact with the cell, the carbon chain part can be inserted into the phospholipid bilayer of the cell membrane, changing or influencing the length, saturation, and arrangement of the fatty acid chain of cholesterol and phospholipid molecules, change or influence the ratio of glycerophospholipid/sphingomyelin, thereby changing the structure of the cell membrane; or the water-soluble carbon chain binds to the membrane protein, changes or affects the structure of the membrane protein, and further affects the structure of the cell membrane. The change of cell membrane structure changes the asymmetry and direction of membrane function, thus affecting the direction of substance transport and signal transmission on the cell membrane, and affecting the transport function of the cell membrane to drug molecules and micro-nanoparticles.

In particular, the present invention provides the following first set of technical solutions in order to solve the lack of agents in the prior art that have no toxic side effects and, in particular, cannot widely kill, block, prevent, or treat microbial infections:
(1) The present invention provides a complex capable of preventing, blocking, and/or treating a microbial infection, comprising an acting moiety, a binding moiety, and a water-soluble moiety,
   the acting moiety is a fat-soluble saturated and/or unsaturated carbon chain with a branched, cyclic, and/or linear structure; the carbon chain is a molecule or a residue of the molecule, and the carbon chain is a carbon chain with 3-100 carbon atoms;
   the water-soluble moiety is a water-soluble molecule or a residue of the molecule; the molecule contains one or two or more functional groups selected from the group consisting of amide group, phosphoryloxy group, carboxyl group, phosphate group, sulfonyl group, sulfonyloxy group, hydroxyl group, quaternary ammonium group, thioether group, disulfide bond, ether group, thiol group, aldehyde group, ester group, amine group, amino group, urea group, and guanidine group; and the water-soluble moiety can be one or two or more of the above functional groups linked to the carbon chain as the acting moiety and/or binding moiety;
   the binding moiety is a molecule or a residue of the molecule capable of binding to a microbial lipid membrane, a microorganism surface protein, a microorganism surface polysaccharide, or a cell wall component or capable of binding to a polysaccharide or a protein or polypeptide in the microorganism; the binding moiety can be the same as the water-soluble moiety, which is a protein, polypeptide, amino acid, oligopeptide, oligosaccharide, monosaccharide and/or polysaccharide molecule or a residue thereof capable of binding to a microbial lipid membrane and a surface domain; and
   wherein the number of any one moiety of the acting moiety, the water-soluble moiety, and the binding moiety can be 1 or 2 or more.
(2) The complex according to technical solution 1, wherein the acting moiety is a carbon chain or carbon chain residue with 3-100, preferably 3-48, more preferably 3-26 carbon atoms,selected from the group consisting of carbon chain or carbon chain residue formed by saturated and/or unsaturated aliphatic hydrocarbons, saturated and/or unsaturated aliphatic or oxo-fatty alcohols, saturated and/or unsaturated fatty acids, hydrophobic amino acids, fat-soluble vitamins, steroid lipids, phospholipids, sphingomyelin, glycolipids, and surfactants; wherein the number of carbon atoms is preferably 3-26;
   the water-soluble moiety is a water-soluble molecule or a residue of the molecule of one or two or more groups selected from the group consisting of a thiol group, amino group, phosphate group, carboxyl group, sulfonyl group, hydroxyl group, amine group, urea group, guanidine group, and disulfide group;
   the binding moiety has a group for binding, i.e. capable of binding to the microbial lipid membrane, the microorganism surface protein, the microorganism surface polysaccharide or the cell wall component or capable of binding to a polysaccharide, protein, or polypeptide in the microorganism; the group is one or two or more groups from the water-soluble moiety or from groups independently as the binding moiety selected from the group consisting of a thiol group, amino group, phosphate group, carboxyl group, sulfonyl group, hydroxyl group, amine group, urea group, guanidine group, and disulfide group, or from one or two or more groups providing a carbon chain to carbon chain connection which is selected from the group consisting of a thiol group, amino group, phosphate group, carboxyl group, sulfonyl group, hydroxyl group, amine group, urea group, guanidine group, and disulfide group, such that the complex has one or two or more groups selected from the group consisting of thiol group, amino group, phosphate group, carboxyl group, sulfonyl group, hydroxyl group, amine group, urea group, guanidine group, and disulfide group;
      That is, for the complex of the invention, the binding moiety may in some cases be the same as the water-soluble moiety, or may simultaneously function as a carbon chain providing the acting moiety.
   Preferably, the binding moiety is one or two or more selected from the group consisting of dibasic or polybasic fatty acid, amino acid, targeting protein, targeting peptide, and targeting polysaccharide.
   More preferably, the complex is a complex formed by linking a fatty acid with 3-100 carbon atoms, preferably 3-50 carbon atoms, and a water-soluble amino acid; or, the complex is a complex formed by linking a fatty acid with 3-50 carbon atoms and a targeting polypeptide;
   or the complex is a complex formed by the reaction of a fatty acid with 3-100 carbon atoms, preferably 3-50 carbon atoms, with a targeting polypeptide and PEG;
   or the complex is a complex formed by the reaction of a surfactant and one or two or more selected from the group consisting of dibasic or polybasic fatty acid, amino acid, targeting protein, targeting polypeptide, and targeting polysaccharide, wherein the surfactant is preferably one or two or more selected from the group consisting of fatty alcohol polyoxyethylene ether, fatty acid polyoxyethylene ester, alkyl glycoside, fatty acid sucrose ester, sorbitan fatty acid ester, sorbitan polyoxyethylene fatty acid ester, N-fatty acyl-N-methylglucamine, and mannose erythritol lipid.
(3). The complex according to technical solution 2, wherein the saturated and/or unsaturated fatty acid is selected from the group consisting of saturated fatty acids or unsaturated fatty acids with 3-100 carbon atoms; the fatty acid is a fatty acid or amino acid containing a double bond, triple bond, hydroxyl group, amino group and/or oxo group, and can be a monobasic, dibasic, or polybasic acid.
(4). The complex according to technical solution 3, wherein the saturated and/or unsaturated fatty acid is one or two or more selected from the group consisting of saturated fatty acids with 3-46 carbon atoms, monoenoic acids with 3-34 carbon atoms, dienoic acids with 5-30 carbon atoms, trienoic acids with 7-30 carbon atoms, tetraenoic acids with 12-38 carbon atoms, pentaenoic acids with 12-38 carbon atoms, hexaenoic acids with 22-38 carbon atoms, alkynoic acids with 6-22 carbon atoms, dialkynoic acids with 10-22 carbon atoms, trialkynoic acids with 12-22 carbon atoms, enynic acids with 8- 20 carbon atoms, preferably acids contain one or two C=C double bonds and one or two or three triple bonds, fatty acids with 3-30 carbon atoms in the main chain and 1-10 alkyl and/or 1-3 hydroxyl groups in the branches, preferably saturated fatty acids with 1-3 methyl carbon atoms or fatty acids with C=C double bonds, saturated linear and branched dicarboxylic and tricarboxylic acids with 3-38 carbon atoms and unsaturated linear or branched dicarboxylic and tricarboxylic acids with 4-18 carbon atoms that is substitutable with hydroxyl groups, carboxylic acids with 3-18 carbon atoms substituted with amino, hydroxyl, oxo and/or methyl, N-fatty acyl amino acids with 6-30 carbon atoms, amino acids containing 2 or more fatty acyl groups, and polybasic carboxylic acids linked by thioether and amide bonds; and
   the saturated and/or unsaturated fatty alcohol is a saturated aliphatic straight- or branched-chain alcohol with 3-33 carbon atoms; and/or an unsaturated aliphatic linear or branched alcohol with 1-3 hydroxyl groups having 3-33 carbon atoms and 1-5 double bonds and 1-5 triple bonds; the oxo fatty alcohol is an alcohol ketone having 8-31 carbon atoms and 1-3 double or triple bonds and 1-3 hydroxyl groups; the ketone is a mono-or di-ketone.
(5). The complex according to technical solution 4, wherein the saturated or unsaturated fatty acid is one or two or more selected from the group consisting of fumaric acid, octanoic acid, glutaconic acid, hexanoic acid, nonanoic acid, dodecanoic acid, tetradecanoic acid, hexadecanoic acid, octadecanoic acid, eicosanoic acid, oleic acid, linoleic acid, linolenic acid, eicosapentaenoic acid, docosapentaenoic acid, docosahexaenoic acid, pentacosanoic acid, heptanoic acid, decanoic acid, dodecenoic acid, tetradecenoic acid, dotriacontahexaenoic acid, octacosanoic acid, or a carbon chain residue formed thereof.
(6). The complex according to any one of technical solutions 1-5, wherein the water-soluble moiety is a molecule or a residue of the molecule containing one or two or more groups selected from the group consisting of a thiol group, amine group, carboxyl group, hydroxyl group, and disulfide group; the molecule is selected from the group consisting of one or two or more water-soluble macromolecules selected from the group consisting of proteins, polysaccharides, nucleic acids, and artificially synthesized water-soluble polymers, or residues thereof;
   and/or, one or two or more medium molecules selected from the group consisting of polypeptides, oligopeptides, oligosaccharides, oligonucleotides, and synthetic water-soluble medium molecular weight polymers, or residues thereof;
   and/or, one or two or more water-soluble small molecules selected from the group consisting of amino acids, monosaccharides, disaccharides, nucleotides, water-soluble vitamins, and deoxynucleotides, or residues thereof;
   and/or, a molecule linked to the carbon chain as the acting moiety or a residue thereof, and the molecule or residue thereof contains one or two or more groups selected from the group consisting of a thiol group, amine group, carboxyl group, hydroxyl group, and disulfide group.
(7). The complex according to technical solution 6, wherein the protein as the water-soluble macromolecule is one or two or more water-soluble macromolecules selected from the group consisting of serum albumins, immunoglobulins, water-soluble collagens, chaperones, water-soluble glycoproteins, and CD14; the polysaccharide as the macromolecule is one or two or more water-soluble macromolecules selected from the group consisting of dextran, hyaluronic acid, sialic acid, heparin sulfate, heparan sulfate, chondroitin sulfate, dermatan sulfate, keratan sulfate, acetyl water-soluble cellulose derivatives, β-cyclodextrin and derivatives thereof, and water-soluble chitosan derivatives; the water-soluble polymer as the macromolecule is one or two or more water-soluble macromolecules selected from the group consisting of polyethylene glycol and carboxylated or aminated polyethylene glycol, polyvinyl alcohol and carboxylated or quaternized polyvinyl alcohol, polyacrylic acid and ammonium polyacrylate;
   the water-soluble medium molecular weight polymer is selected from the group consisting of targeting polypeptides, oligopeptides, oligosaccharides, oligonucleotides, and/or water-soluble polyamino acids; preferably, the targeting polypeptide comprises a protein or neutralizing antibody fragment that specifically targets a microbial lipid membrane, bacterial and fungal cell wall, virus surface protein domain, including, e.g. taurine transporter peptide, SBP1; preferably, the water-soluble polyamino acid is selected from the group consisting of polyglutamic acid, polylysine, and/or polyaspartic acid; and oligopeptides, oligosaccharides, oligonucleotides; and
   the monosaccharide and/or disaccharide as the water-soluble small molecular is one or two or more selected from the group consisting of glucose, fructose, rhamnose, sorbose, sucrose, maltose, lactose, and trehalose; the nucleotide and/or deoxynucleotide as the water-soluble small molecule is one or two or more selected from the group consisting of adenosine, guanylate, uranylate, cytidylate, thymidylate, inosine, deoxyadenosine, deoxyguanosine, deoxycytidylate, and deoxythymidylate; the amine acid as the water-soluble small molecule is one or two or more of amino acids such as serine, threonine, cysteine, asparagine, glutamine, tyrosine, lysine, arginine, histidine, aspartate, glutamate, citrulline, ornithine, taurine, and aminobutyric acid; the vitamin as the water-soluble small molecule is one or two or more selected from the group consisting of vitamin B1, pantothenic acid, vitamin B6, and vitamin C.
(8). The complex according to any one of technical solutions 1-7, wherein the binding moiety and the water-soluble moiety are the same, which is a protein, polypeptide, oligopeptide, oligosaccharide, monosaccharide, disaccharide, amino acid, nucleotide, vitamin, water-soluble polymer, water-soluble polyamino acid and/or polysaccharide molecule capable of binding to a lipid membrane, surface domain of a microorganism, or a residue thereof; the molecule or residue thereof comprises one or two or more groups selected from the group consisting of a thiol group, amine group, carboxyl group, hydroxyl group, and disulfide group.
(9). The complex according to any one of technical solutions 1-8, wherein the complex is a compound obtained by the reaction of a substance containing a carbon chain with 3-100 carbon atoms as the acting moiety with one or two or more selected from the group consisting of protein, polypeptide, oligopeptide, oligosaccharide, monosaccharide, disaccharide, amino acid, nucleotide, vitamin, water-soluble polymer, water-soluble polyamino acid and/or polysaccharide; or a mixture of compound obtained by the reaction of a substance containing a carbon chain with 3-100 carbon atoms as the acting moiety with one or two or more selected from the group consisting of protein, polypeptide, oligopeptide, oligosaccharide, monosaccharide, disaccharide, amino acid, nucleotide, vitamin, water-soluble polymer, water-soluble polyamino acid and/or polysaccharide, and unreacted substance as the acting moiety and/or unreacted protein, polypeptide, oligopeptide, oligosaccharide, monosaccharide, disaccharide, amino acid, nucleotide, vitamin, water-soluble polymer, water-soluble polyamino acid and/or polysaccharide; wherein preferably, the substance as the acting moiety is one or two or more substances selected from the group consisting of saturated and/or unsaturated aliphatic hydrocarbons, saturated and/or unsaturated aliphatic or oxo-fatty alcohols, saturated and/or unsaturated fatty acids, hydrophobic amino acids, fat-soluble vitamins, steroid lipidoids, phospholipids, sphingomyelins, glycolipids, and surfactants, that provide or have carbon chains or carbon chain-forming residues with 3-100, preferably 3-48, more preferably 3-26 carbon atoms.
   That is, the complex for preventing, blocking or treating microbial infection includes the compound obtained by the reaction, and also includes a reaction mixture (also referred to as "reaction product", "reaction mixture", "reaction product solution", or "reaction mixture solution ") containing the compound obtained by the reaction, and a purified product after purifying the reaction mixture to separate unreacted reaction starting material and catalyst, etc.)
(10). The complex according to any one of technical solutions 1-8, wherein the complex is a complex obtained by compounding a substance containing a carbon chain with 3-100 carbon atoms as the acting moiety with one or two or more molecules selected from the group consisting of protein, polypeptide, oligopeptide, oligosaccharide, monosaccharide, disaccharide, amino acid, nucleotide, vitamin, water-soluble polymer, water-soluble polyamino acid, and/or polysaccharide by a physicochemical action comprising hydrogen bond, van der Waals force, or a combination thereof, or a mixture obtained by directly physically mixing the same; wherein preferably, the substance used as the acting moiety is one or two or more substance that provide or have a carbon chain or carbon chain-forming residue with 3-100, preferably 3-48, more preferably 3-26 carbon atoms, selected from the group consisting of saturated and/or unsaturated aliphatic hydrocarbons, saturated and/or unsaturated aliphatic or oxo-fatty alcohols, saturated and/or unsaturated fatty acids, hydrophobic amino acids, fat-soluble vitamins, steroid lipids, phospholipids, sphingomyelin, glycolipids, and surfactants.
(11). The complex according to technical solution 9, wherein the complex is a compound obtained by the reaction of a substance having a carbon chain with 3-100 carbon atoms as the acting moiety with at least one selected from the group consisting of protein, polypeptide, oligopeptide, and amino acid; or a mixture of the compound obtained by the reaction of the substance having a carbon chain with 3-100 carbon atoms as the acting moiety with at least one selected from the group consisting of protein, polypeptide, oligopeptide, and amino acid, unreacted substance as the acting moiety, and/or unreacted at least one selected from the group consisting of protein, polypeptide, oligopeptide, and amino acid.
(12). The complex according to technical solution 9, wherein the complex is a compound obtained by the reaction of a substance having a carbon chain with 3-100 carbon atoms as the acting moiety, and PEG, with at least one selected from the group consisting of protein, polypeptide, oligopeptide, and amino acid; or a mixture of the compound obtained by the reaction of the carbon chain with 3-100 carbon atoms as the acting moiety, and PEG, with at least one selected from the group consisting of protein, polypeptide, oligopeptide, and amino acid, unreacted PEG, and/or an unreacted at least one selected from the group consisting of protein, polypeptide, oligopeptide, and amino acid.
(13). The complex according to technical solution 9, wherein the complex is a compound obtained by the reaction of a substance having a carbon chain with 3-100 carbon atoms as the acting moiety with one or two or more selected from the group consisting of polysaccharide, monosaccharide, disaccharide, and/or oligosaccharide; or a mixture of the compound obtained by the reaction of the substance having the carbon chain with 3-100 carbon atoms as the acting moiety with one or two or more selected from the group consisting of polysaccharide, monosaccharide, disaccharide, and/or oligosaccharide, unreacted substance as the acting moiety and/or unreacted polysaccharide, monosaccharide, disaccharide, and/or oligosaccharide.
(14). The complex according to technical solution 9, wherein the complex is a compound obtained by the reaction of a substance having a carbon chain with 3-100 carbon atoms as the acting moiety, and PEG, with one or two or more selected from the group consisting of polysaccharide, monosaccharide, disaccharide, and/or oligosaccharide; or a mixture of the compound obtained by the reaction of the saturated and/or unsaturated fatty acid with 3-100 carbon atoms, and PEG, with one or two or more selected from the group consisting of polysaccharide, monosaccharide, disaccharide, and/or oligosaccharide, unreacted substance as the acting moiety, unreacted PEG and/or unreacted polysaccharide, monosaccharide, disaccharide, and/or oligosaccharide.
(15). The complex according to any one of technical solutions 6-12, wherein the protein is one or two or more selected from the group consisting of serum albumins, immunoglobulins, water-soluble collagens, chaperones, water-soluble glycoproteins and CD14.
(16). The complex according to technical solution 13, wherein the polysaccharide is one or two or more selected from the group consisting of dextran and/or hyaluronic acid, sialic acid, heparin sulfate, heparan sulfate, chondroitin sulfate, dermatan sulfate, keratan sulfate, acetyl water-soluble cellulose derivatives, β-cyclodextrin and derivative thereof, and water-soluble chitosan derivatives.
(17). The complex according to technical solution 11 or 12, wherein the complex is a compound obtained by the reaction of a substance having a carbon chain with 3-100 carbon atoms as the acting moiety, a linker, and a thiol-containing protein; or a mixture of the compound obtained from the above reaction, unreacted substance as the acting moiety, unreacted linker, and/or unreacted thiol-containing protein; wherein the linker is one or two or more selected from the group consisting of an amino acid, succinic acid, butadienoic acid, glutaconic acid, hexaminodioic acid, carbamate, short peptide, N-hydroxybutenimide, polyethylene glycol and derivatives of the above compounds;
   preferably, the complex is a compound obtained by the reaction of a substance containing a carbon chain with 3-100 carbon atoms as the acting moiety and N-hydroxybuteneimide with a thiol-containing protein; or a mixture of the compound obtained from the above reaction, unreacted substances as the acting moiety, unreacted N-hydroxybuteneimide, and/or unreacted thiol-containing protein.
(18). The complex according to technical solution 13, wherein the complex is a compound obtained by the reaction of a substance having a carbon chain with 3-100 carbon atoms as the acting moiety, cystamine, and one or two or more selected from the group consisting of polysaccharide, monosaccharide, disaccharide and/or oligosaccharide; or a mixture of the compound obtained by the reaction of the substance having the carbon chain with 3-100 carbon atoms as the acting moiety, cystamine, and one or two or more selected from the group consisting of polysaccharide, monosaccharide, disaccharide and/or oligosaccharide, unreacted substance as the acting moiety, unreacted one or two or more selected from the group consisting of polysaccharide, monosaccharide, disaccharide and oligosaccharide, and/or unreacted cystamine.
(19). The complex according to any one of technical solutions 8-17, wherein the compound obtained by the reaction contains one or two or more selected from the group consisting of an amide group, ester group, thioether group, or ether group as a linking moiety between the water-soluble moiety and the acting moiety.
(20) The complex according to any one of technical solutions 3-18, wherein the substance providing the carbon chain or the residue of the carbon chain as the acting moiety is one or two or more selected from the group consisting of saturated and/or unsaturated aliphatic hydrocarbons, saturated and/or unsaturated aliphatic or oxo-fatty alcohols, saturated and/or unsaturated fatty acids, hydrophobic amino acids, fat-soluble vitamins, steroid lipids, phospholipids, sphingomyelin, glycolipids and surfactants; the carbon number of the carbon chain is 3-100, preferably 3-50, further preferably 3-48, more preferably 3-26.
(21). The complex according to any one of technical solutions 3-18, wherein the substance providing the carbon chain or the residue of the carbon chain as the acting moiety is one or two or more selected from the group consisting of saturated and/or unsaturated aliphatic hydrocarbons, saturated and/or unsaturated aliphatic or oxo-fatty alcohols, saturated and/or unsaturated fatty acids, hydrophobic amino acids, fat-soluble vitamins, steroid lipids, phospholipids, sphingomyelin, glycolipids and surfactants, preferably saturated and/or unsaturated fatty acids; wherein the saturated and/or unsaturated fatty acid is a fatty acid with 3-100, preferably 3-50, still preferably 3-48, more preferably 3-40 carbon atoms, which is a fatty acid with 1-8 C=C double bonds, can be a fatty acid with 1-7 C=C double bonds, a fatty acid with 1-6 double bonds, a fatty acid with 1-5 double bonds, a fatty acid with 1-4 double bonds, a fatty acid with 1-3 double bonds, a fatty acid with 1-2 double bonds.
(22). The complex according to any one of technical solutions 3-18, wherein the substance providing carbon chains or carbon chain residues as the acting moiety is a saturated and/or unsaturated fatty acid, that can be a fatty acid with 1-6 double bonds and 2-30 carbon atoms, preferably 2-26, and more preferably 2-22.
(23). The complex according to any one of technical solutions 3-18, wherein the number of carbon atoms of the saturated and/or unsaturated fatty acid is 3-30, preferably 3-26, preferably 8-22, preferably 8-20, preferably 8-18.
(24). The complex according to any one of technical solutions 3-18, wherein the saturated and/or unsaturated fatty acid is one or two or more fatty acids selected from the group consisting of fumaric acid, octanoic acid, glutaconic acid, hexanoic acid, nonanoic acid, dodecanoic acid, tetradecanoic acid, hexadecanoic acid, octadecanoic acid, eicosanoic acid, oleic acid, linoleic acid, linolenic acid, eicosapentaenoic acid, docosapentaenoic acid, docosahexaenoic acid, pentacosanoic acid, heptanoic acid, decanoic acid, dodecenoic acid, tetradecenoic acid, dotriacontahexaenoic acid, and octacosanoic acid.
(25). The complex according to any one of technical solutions 8-23, wherein the protein is human or bovine serum albumin, or CD14; alternatively, the polysaccharide is dextran and/or hyaluronic acid.
(26). The complex according to technical solution 11, wherein the compound obtained by the reaction is any one or two or more compounds that are obtained by the reaction of a fatty acid with albumin or SBP1 and have the following structural formulas: 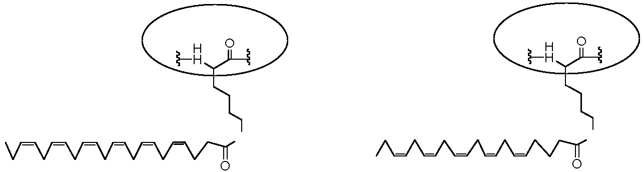 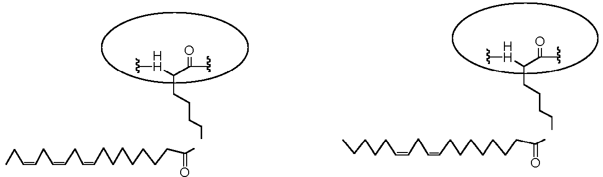 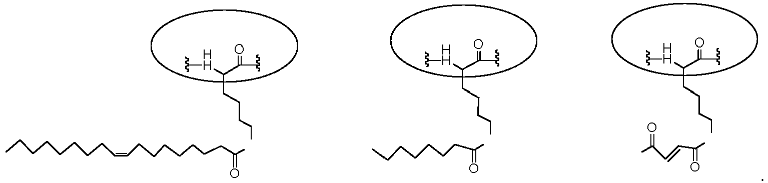
(27). The complex according to technical solution 12, wherein the compound obtained by the reaction is a compound obtained by the reaction of a monobasic fatty acid with 3-10 carbon atoms and PEG, with an amino acid, or a compound obtained by the reaction of a monobasic fatty acid with 3-10 carbon atoms, a saturated dibasic fatty acid with 5-8 carbon atoms, and PEG with taurine, preferably a compound with at least one of the following structural formulas: 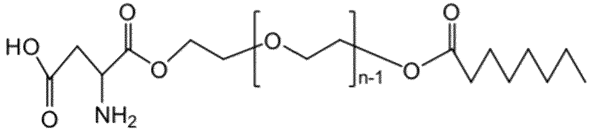 or 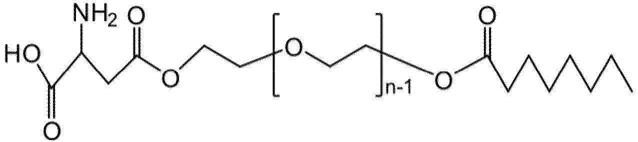 Or 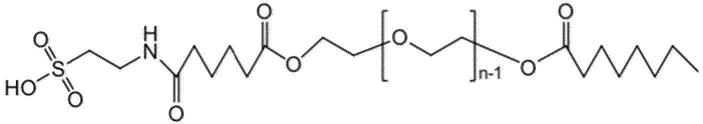 where n is an integer of 1-200.
(28). The complex according to technical solution 13, wherein the compound obtained by the reaction is any one or two or more compounds that are obtained by the reaction of a fatty acid with dextran and have the following structural formulas: 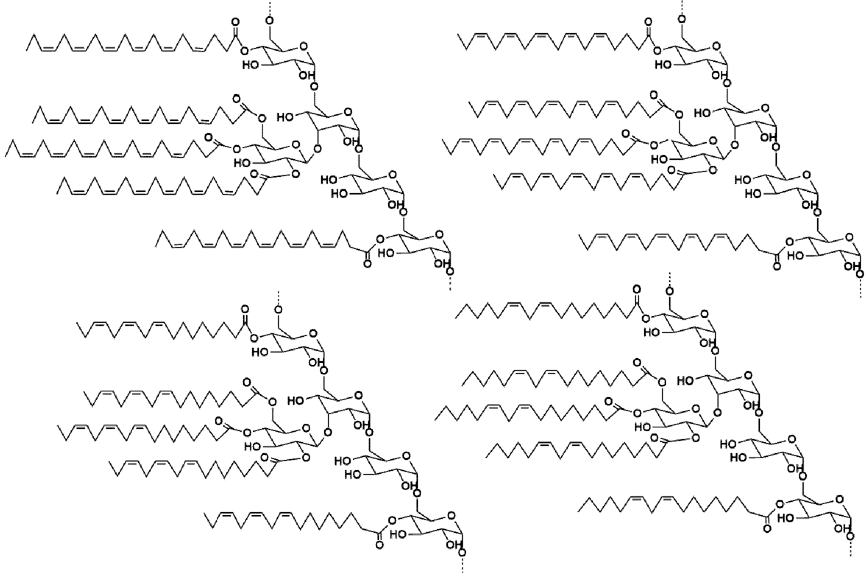 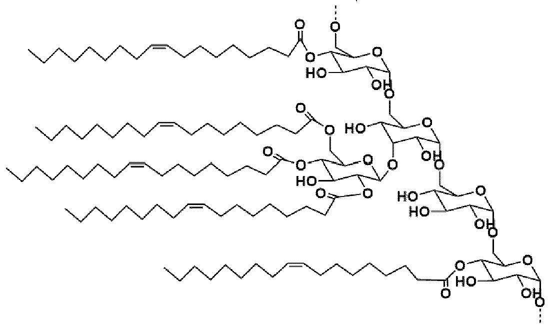 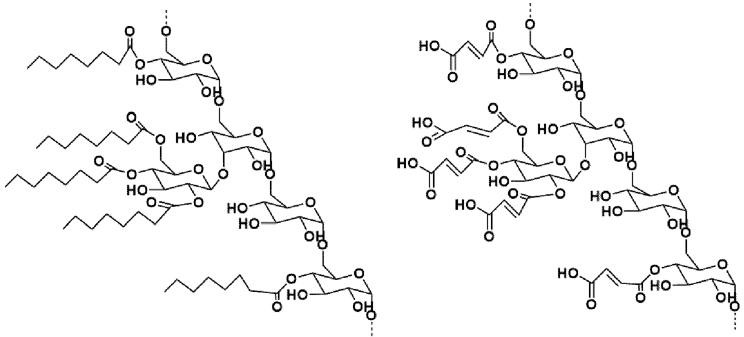
(29) . The complex according to technical solution 13, wherein the compound obtained by the reaction is any one or two or more compounds that are obtained by the reaction of a fatty acid with hyaluronic acid and have the following structural formulas: 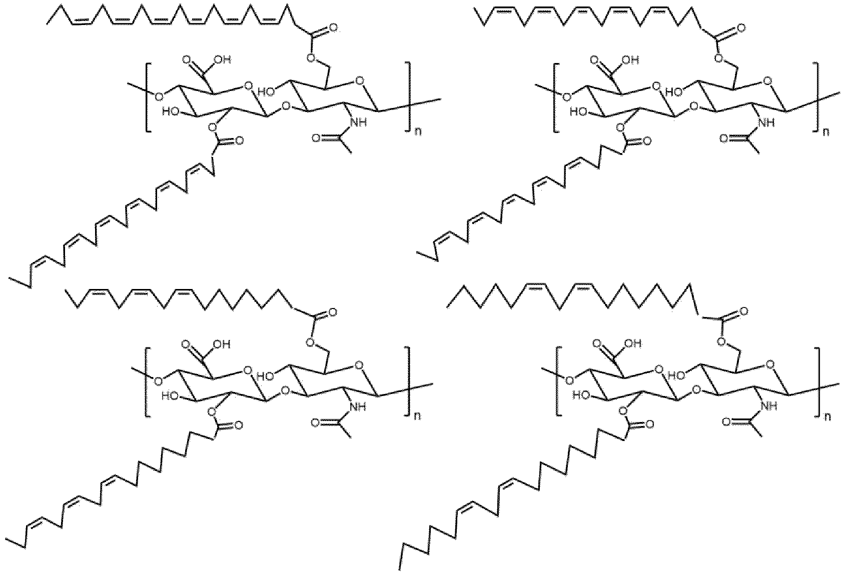 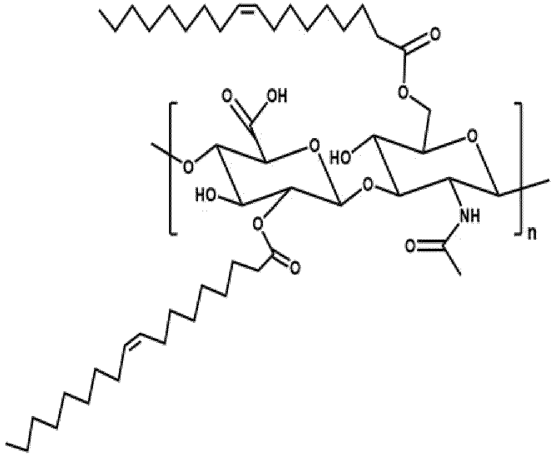 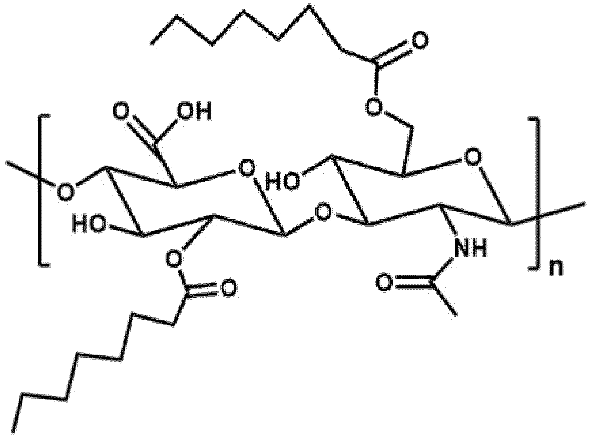 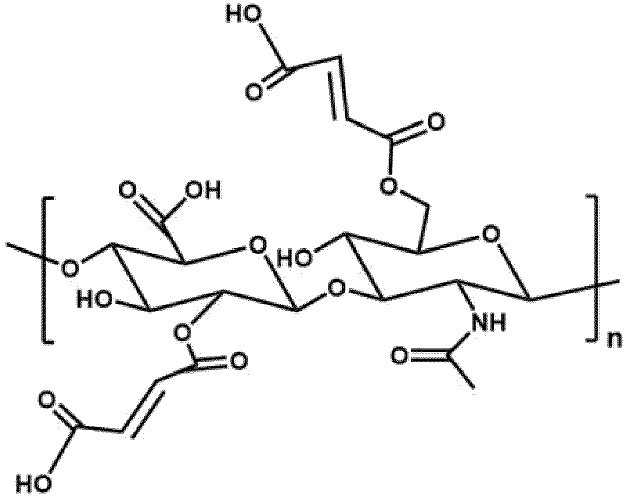 where n is an integer of 1-2000.
(30). The complex according to technical solution 14, wherein the compound obtained by the reaction is a compound obtained by the reaction of a fatty acid with 3-10 carbon atoms with PEG and glucose, preferably a compound with the following structural formula: 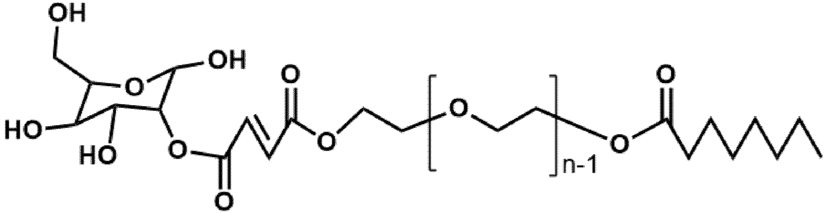 where n is an integer of 1-200.
(31). The complex according to technical solution 17, wherein the compound obtained by the reaction is any one or two or more compounds having a thioether bond, that are obtained by the reaction of a fatty acid, N-hydroxybutenimide, and albumin and have the following structural formulas: 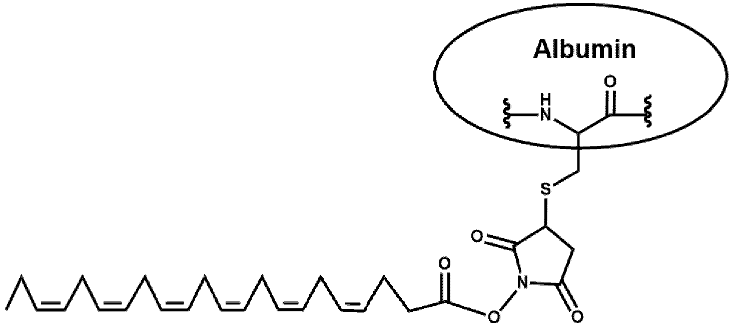 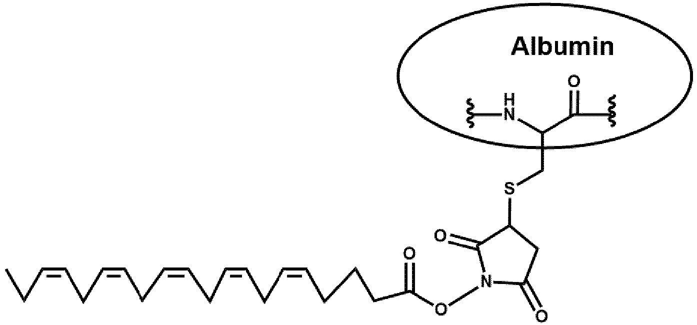 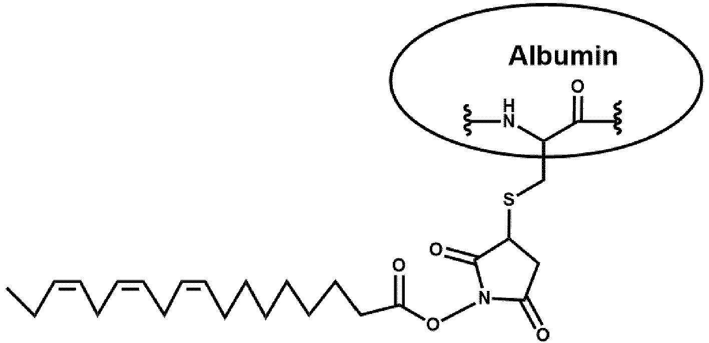 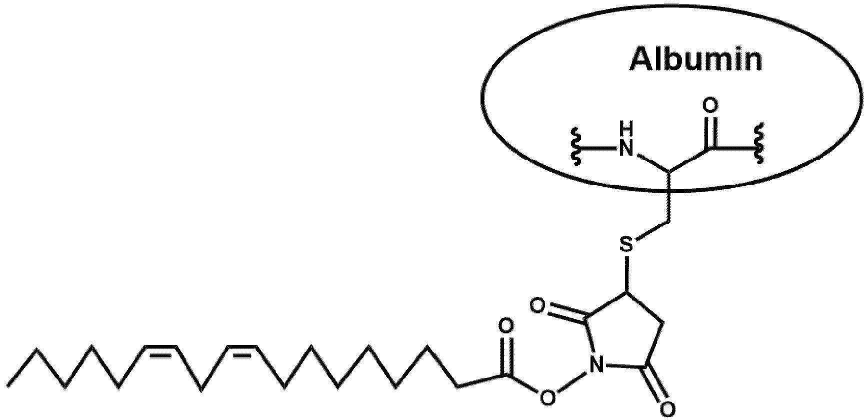 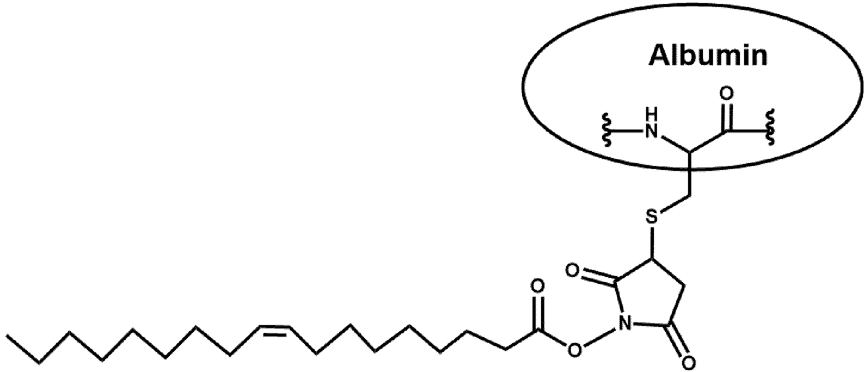 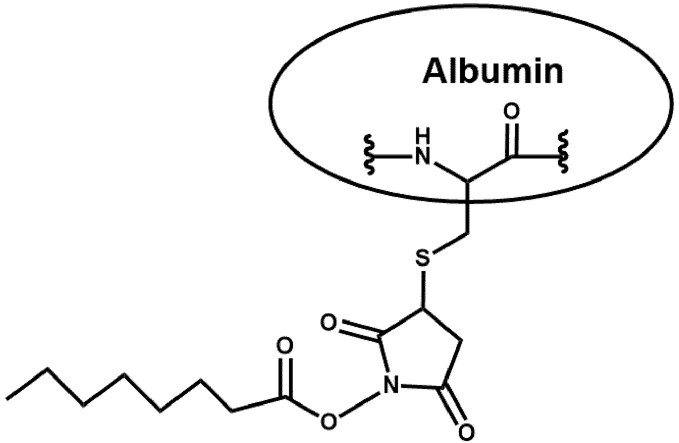 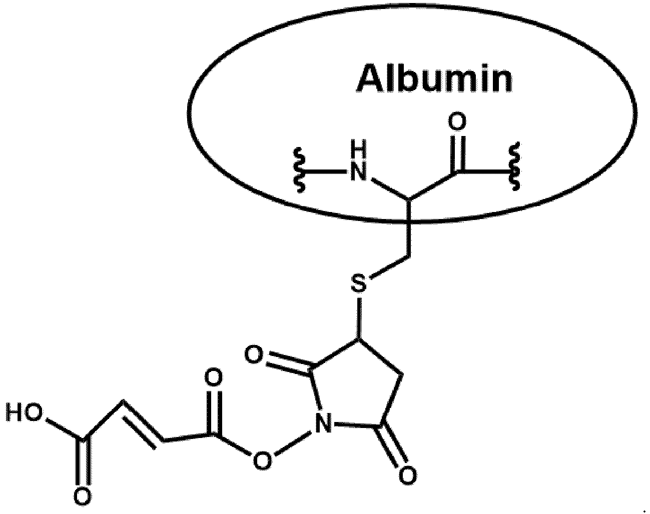
(32). The complex according to technical solution 18, wherein the compound obtained by the reaction is any one or two or more compounds that are obtained by the reaction of a fatty acid, cystamine and dextran and have the following structural formulas: 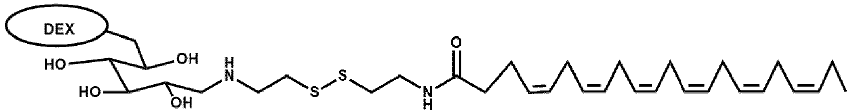 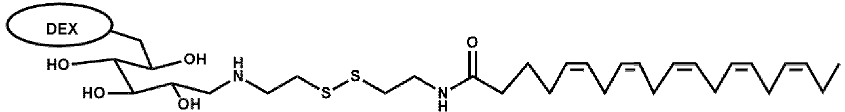 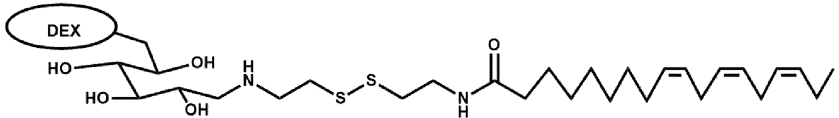 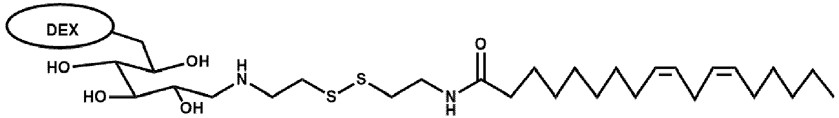 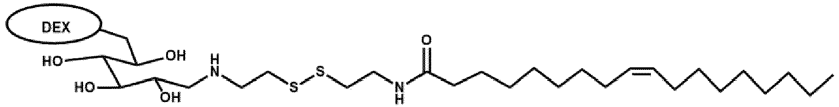 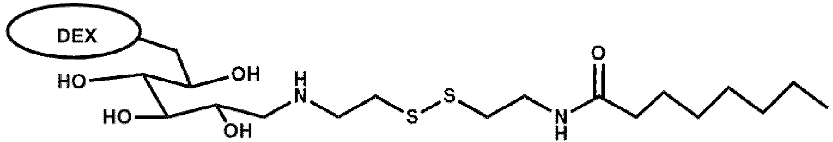 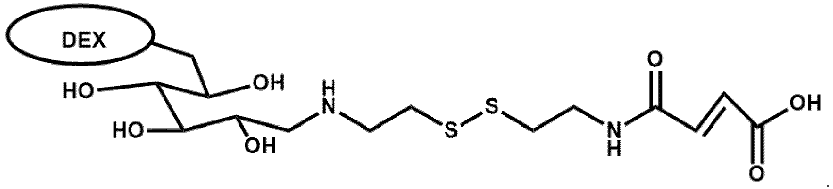
(33). The complex according to technical solution 18, wherein the compound obtained by the reaction is any one or two or more compounds that obtained by the reaction of a fatty acid, cystamine, and hyaluronic acid and have the following structural formulas: 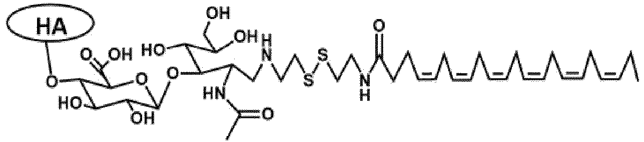 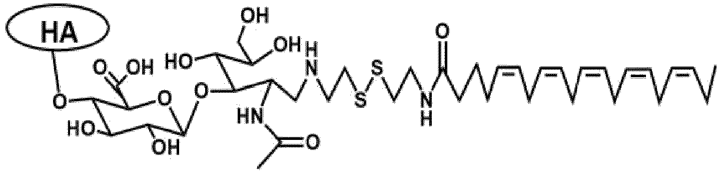 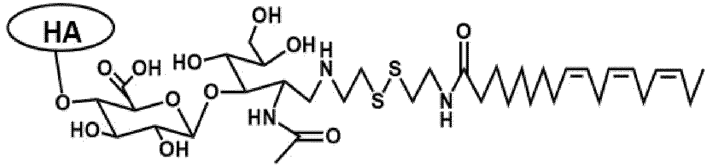 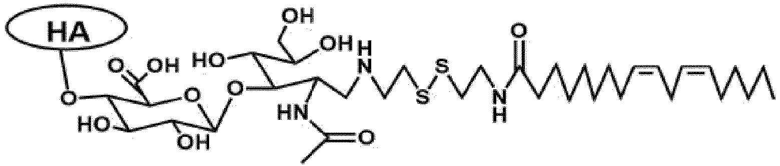 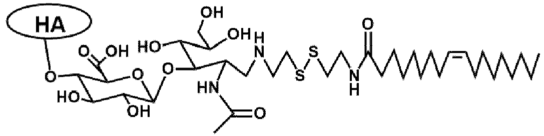 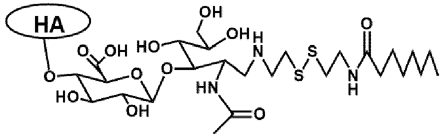 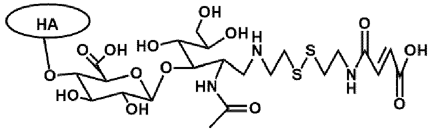
(34). The complex according to any one of technical solutions 1-8, wherein the complex is a compound obtained by the reaction of a surfactant containing a carbon chain having 3-30 carbon atoms with a dibasic or polybasic fatty acid, an amino acid, a targeting protein, a targeting polypeptide, a targeting polysaccharide, and/or a targeting polysaccharide; or a mixture of the compound obtained by the above reaction, unreacted surfactant and/or unreacted dibasic or polybasic fatty acid, amino acid, targeting protein, targeting polypeptide, targeting polysaccharide, and/or targeting polysaccharide.
(35). The complex according to technical solution 34, wherein the surfactant is one or two or more selected from the group consisting of fatty alcohol polyoxyethylene ethers, fatty acid polyoxyethylene esters, alkyl glycosides, fatty acid sucrose esters, sorbitan fatty acid esters, sorbitan polyoxyethylene fatty acid esters, mannose erythritol esters and N-fatty acyl-N-methylglucamine.
(36). The complex according to any one of technical solutions 1-35, wherein the microbial infection comprises an infection caused by a virus.
(37). A preparation for preventing, blocking or treating microbial infections made using the complex according to any one of technical solutions 1-35.
(38). The preparation according to technical solution 37 being a pharmaceutical preparation or an environmental disinfection and sterilization preparation.
(39). The preparation according to technical solution 38, wherein the pharmaceutical preparation is one selected from the group consisting of an inhalant, a nasal spray, an injection, an oral preparation, and a transdermal topical formulation.
(40). Use of the complex according to any one of technical solutions 1-35 in the preparation of a pharmaceutical preparation for preventing, blocking or treating microbial infections.
(41). The use according to technical solution 40, wherein the microorganism is any one or more selected from the group consisting of viruses, bacteria, fungi, chlamydia, or mycoplasma.
(42). The use according to technical solution 41, wherein the virus is an enveloped virus; and/or a non-enveloped virus.
(43). The use according to technical solution 42, wherein the enveloped virus is one or two or more selected from the group consisting of coronavirus, influenza virus, AIDS virus, hepatitis B virus, hepatitis C virus, herpes virus, Zika virus, Dengue virus, Japanese encephalitis virus, Ebola virus, rabies virus, and/or Hantavirus; the non-enveloped virus is two or more selected from the group consisting of hepatitis A virus, human papilloma virus, polio virus and/or Coxsackie virus.
(44). The use according to technical solution 43, wherein the virus is any one or two or more selected from the group consisting of coronavirus, AIDS virus, hepatitis B virus, hepatitis C virus, herpes virus, Japanese encephalitis virus, rabies virus, human papilloma virus and Ebola virus.
(45). The use according to technical solution 41, wherein the bacteria are Gram-positive and/or Gram-negative bacteria and the fungi are pathogenic fungi and/or conditionally pathogenic fungi; the Chlamydia is *Chlamydia trachomatis, Chlamydia pneumoniae,* and/or *Chlamydia psittaci*; the mycoplasma include *Mycoplasma pneumoniae, Ureaplasma urealyticum, Mycoplasma hominis, and*/*or Mycoplasma genitalium.*
(46). The use according to technical solution 41, wherein the bacteria are one or two or more selected from the group consisting of *Escherichia coli, Staphylococcus aureus,* Methicillin-resistant *Staphylococcus aureus, Streptococcus pneumoniae, Klebsiella pneumoniae* and *Pseudomonas aeruginosa*; the fungus is one or two or more selected from the group consisting of *Candida albicans, Aspergillus niger, Actinomyces viscosus, Chaetomium globosum, Aspergillus protuberus* and *Microsporum canis.*
(47). The use according to technical solution 41, wherein the virus is one or two more selected from the group consisting of H7N9 influenza virus, H5N1 influenza virus, HIV virus, novel coronavirus, HPV virus, and rabies virus.
(48). A preparation method of the complex according to any one of technical solutions 1-35, the complex being obtained by the reaction of a compound having a fat-soluble saturated and/or unsaturated carbon chain with a branched, cyclic, and/or linear structure with a water-soluble molecule, and optionally added protein, polypeptide, amino acid, oligopeptide, oligosaccharide, monosaccharide, and/or polysaccharide molecule when needed capable of binding to a microbial lipid membrane, virus surface domain, or cell wall, and optionally added a linker molecule when needed, in the presence of a catalyst.
(49). The preparation method of the complex according to technical solution 48, wherein the complex is a purified product of the compound obtained by the reaction.
(50). A preparation method of the complex according to any one of technical solutions 1-35, the complex being obtained by physical mixing of a compound having a fat-soluble saturated and/or unsaturated carbon chain with a branched, cyclic, and/or linear structure with a water-soluble molecule, and optionally added protein, polypeptide, amino acid, oligopeptide, oligosaccharide, monosaccharide, and/or polysaccharide molecule when needed capable of binding to a microbial lipid membrane, microorganism surface domain, or cell wall.
(51). A preparation method of the complex according to any one of technical solutions 1-35, the complex being obtained by the reaction of a saturated and/or unsaturated fatty acid with 3-100 carbon atoms with any one selected from the group consisting of a protein, peptide, oligopeptide, oligosaccharide, monosaccharide, disaccharide, nucleotide, vitamin, water-soluble polymer, water-soluble polyamino acid, and/or polysaccharide in the presence of a catalyst.
(52). The preparation method of the complex according to technical solution 51, wherein the complex is a purified product of the compound obtained by the reaction.
(53). The preparation method of the complex according to any one of technical solutions 1-35, the complex being obtained by directly physical compounding a saturated and/or unsaturated fatty acid with 3-100 carbon atoms and at least one selected from the group consisting of protein, polypeptide, oligopeptide, oligosaccharide, monosaccharide, disaccharide, nucleotide, vitamin, water-soluble polymer, water-soluble polyamino acid, and/or polysaccharide molecule, by a physicochemical action, or by directly physical mixing the same.

In particular, the present invention provides the following second set of technical solutions in order to solve the lack of agents in the prior art which have no toxic side effects and, in particular, cannot widely kill and block, prevent or treat microbial infections:
○,1 A complex capable of preventing, blocking, and/or treating a microbial infection, comprising an acting moiety, a binding moiety and a water-soluble moiety,
   the acting moiety is a fat-soluble saturated and/or unsaturated carbon chain with a branched, cyclic and/or linear structure; the acting moiety is a carbon chain or a residue of carbon chain with 3-100 carbon atoms formed by one or more substances selected from the group consisting of hydrophobic amino acids, fat-soluble vitamins, steroid lipids, phospholipids, sphingomyelins, glycolipids, surfactants, saturated and/or unsaturated fatty hydrocarbons and saturated and/or unsaturated fatty alcohols or oxo fatty alcohols;
   the water-soluble moiety is a water-soluble molecule or a residue of the molecule; the molecule contains one or two or more functional groups selected from the group consisting of amide group, phosphoryloxy group, carboxyl group, phosphate group, sulfonyl group, sulfonyloxy group, hydroxyl group, quaternary ammonium group, thioether group, disulfide bond, ether group, thiol group, aldehyde group, ester group, amine group, amino group, urea group and guanidine group; and the water-soluble moiety can be one or two or more of the above functional groups linked to the carbon chain as the acting moiety;
   the binding moiety is a molecule or a residue of the molecule capable of binding to a microbial lipid membrane, a microorganism surface protein, a microorganism surface polysaccharide, or a cell wall component or capable of binding to a polysaccharide or a protein or polypeptide in the microorganism; the binding moiety can be the same as the water-soluble moiety, i.e. a protein, polypeptide, amino acid, oligopeptide, oligosaccharide, monosaccharide and/or polysaccharide molecule or a residue thereof capable of binding to a microbial lipid membrane and a surface domain; and
   wherein the number of any one moiety of the acting moiety, the water-soluble moiety, and the binding moiety can be 1 or 2 or more.
○,2 The complex according to technical solution 1, wherein the number of carbon atoms is 3-48.
○,3 The complex according to technical solution 1, wherein the number of carbon atoms is 3-26.
○,4 The complex according to any one of technical solution 1-3, wherein the water-soluble moiety is a water-soluble molecule or a residue of the molecule containing one or two or more groups selected from the group consisting of a thiol group, amino group, phosphate group, carboxyl group, sulfonyl group, hydroxyl group, amine group, urea group, guanidine group, and disulfide group;
   the binding moiety has a group for binding, i.e. capable of binding to the microbial lipid membrane, the microorganism surface protein, the microorganism surface polysaccharide or the cell wall component or capable of binding to a polysaccharide, protein, or polypeptide in the microorganism; the group is two or more groups from the water-soluble moiety or from groups independently as the binding moiety selected from the group consisting of a thiol group, amino group, phosphate group, carboxyl group, sulfonyl group, hydroxyl group, amine group, urea group, guanidine group, and disulfide group, or from one or two or more groups providing a carbon chain to carbon chain connection which is selected from the group consisting of a thiol group, amino group, phosphate group, carboxyl group, sulfonyl group, hydroxyl group, amine group, urea group, guanidine group and disulfide group, such that the complex has one or two or more groups selected from the group consisting of thiol group, amino group, phosphate group, carboxyl group, sulfonyl group, hydroxyl group, amine group, urea group, guanidine group and disulfide group.
○,5 The complex according to technical solution 4, wherein the binding moiety is one or two or more selected from the group consisting of dibasic or polybasic fatty acid, amino acid, targeting protein, targeting peptide and targeting polysaccharide.
○,6 The complex according to any one of technical solutions 1-4, wherein the complex is formed by the reaction of a surfactant with one or two or more selected from the group consisting of dibasic or polybasic fatty acid, amino acids, targeting proteins, targeting peptides and targeting polysaccharides.
○,7 The complex according to any one of technical solutions 1-6, wherein the saturated and/or unsaturated fatty alcohol is a saturated fat alcohol with 3-33 carbon atoms with a cyclic structure, straight or branched chain; and/or an unsaturated aliphatic linear or branched alcohol with 1-3 hydroxyl groups containing 1-5 double bonds and 1-5 triple bonds with a carbon number of 3-33; the oxo fatty alcohol is an alcohol ketone having from 8 to 31 carbon atoms and from 1 to 3 double or triple bonds and from 1 to 3 hydroxyl groups, the ketone being a mono-or di-ketone.
○,8The complex according to any one of technical solution 1-5 and technical solution 7, wherein the water-soluble moiety is a molecule or a residue of the molecule containing one or two or more groups selected from the group consisting of a thiol group, amine group, carboxyl group, hydroxyl group and disulfide group; the molecule is one or two or more water-soluble macromolecules selected from the group consisting of proteins, polysaccharides, nucleic acids, and artificially synthesized water-soluble polymers, or residues thereof;
   and/or, one or two or more medium molecules selected from the group consisting of polypeptides, oligopeptides, oligosaccharides, oligonucleotides, and synthetic water-soluble medium molecular weight polymers, or residues thereof;
   and/or, one or two or more water-soluble small molecules selected from the group consisting of amino acids, monosaccharides, disaccharides, nucleotides, water-soluble vitamins, and deoxynucleotides, or residues thereof;
   and/or, a molecule linked to the carbon chain as the acting moiety or a residue thereof, and the molecule or residue thereof contains one or two or more groups selected from the group consisting of a thiol group, amine group, carboxyl group, hydroxyl group and disulfide group.
○,9The complex according to claim 8, wherein the protein as the water-soluble macromolecule is one or two or more water-soluble macromolecules selected from the group consisting of serum albumins, immunoglobulins, water-soluble collagens, chaperones, water-soluble glycoproteins, and CD14; the polysaccharide as the macromolecule is one or two or more water-soluble macromolecules selected from the group consisting of dextran, hyaluronic acid, sialic acid, heparin sulfate, heparan sulfate, chondroitin sulfate, dermatan sulfate, keratan sulfate, acetyl water-soluble cellulose derivatives, β-cyclodextrin and derivatives thereof, and water-soluble chitosan derivatives; the water-soluble polymer as the macromolecule is one or two or more water-soluble macromolecules selected from the group consisting of polyethylene glycol and carboxylated or aminated polyethylene glycol, polyvinyl alcohol and carboxylated or quaternized polyvinyl alcohol, polyacrylic acid and ammonium polyacrylate;
   the water-soluble medium molecular weight polymer is one or two or more substances selected from the group consisting of targeting polypeptides, oligopeptides, oligosaccharides, oligonucleotides and/or water-soluble polyamino acids;
   the water-soluble small molecular weight monosaccharide and/or disaccharide is one or two or more selected from the group consisting of glucose, fructose, rhamnose, sorbose, sucrose, maltose, lactose, and trehalose; the nucleotide and/or deoxynucleotide as the water-soluble small molecule is one or two or more selected from the group consisting of adenosine, guanylate, uranylate, cytidylate, thymidylate, inosine, deoxyadenosine, deoxyguanosine, deoxycytidylate, and deoxythymidylate; the amine acid as the water-soluble small molecule is one or two or more selected from the group consisting of amino acids such as serine, threonine, cysteine, asparagine, glutamine, tyrosine, lysine, arginine, histidine, aspartate, glutamate, citrulline, ornithine, taurine, and aminobutyric acid; the vitamin as the water-soluble small molecule is one or two or more selected from the group consisting of vitamin B1, pantothenic acid, vitamin B6, and vitamin C.
○,10 The complex according to any one of technical solutions 5-9, wherein the targeting polypeptide comprises any one of a protein or neutralizing antibody fragment that specifically targets a microbial lipid membrane, a bacterial and fungal cell wall, a virus surface protein domain.
○,11 The complex according to technical solution 9, wherein the water-soluble polyamino acid is selected from the group consisting of polyglutamate, polylysine, and/or polyaspartate.
○,12 The complex according to any one of technical solutions 1-11, wherein the binding moiety and the water-soluble moiety are the same, i.e. a protein, polypeptide, oligopeptide, oligosaccharide, monosaccharide, disaccharide, amino acid, nucleotide, vitamin, water-soluble polymer, water-soluble polyamino acid and/or polysaccharide molecule capable of binding to a lipid membrane, surface domain of a microorganism, or a residue thereof; the molecule or residue thereof comprises one or two or more groups selected from the group consisting of a thiol group, amine group, carboxyl group, hydroxyl group and disulfide group.
○,13 The complex according to technical solutions 1-12, wherein the complex is a compound obtained by the reaction of a substance containing a carbon chain with 3-100 carbon atoms as the acting moiety with any one or two or more selected from the group consisting of protein, polypeptide, oligopeptide, oligosaccharide, amino acid, monosaccharide, disaccharide, nucleotide, vitamin, water-soluble polymer, water-soluble polyamino acid, and/or polysaccharide; or a mixture of the compound obtained by the reaction of the substance containing the carbon chain with 3-100 carbon atoms as the acting moiety with any one or two or more selected from the group consisting of protein, polypeptide, oligopeptide, oligosaccharide, amino acid, monosaccharide, disaccharide, nucleotide, vitamin, water-soluble polymer, water-soluble polyamino acid, and/or polysaccharide molecular, and unreacted substance as the acting moiety and/or unreacted protein, polypeptide, oligopeptide, oligosaccharide, amino acid, monosaccharide, disaccharide, oligonucleotide, vitamin, water-soluble polymer, water-soluble polyamino acid, and/or polysaccharide; and
   wherein the substance having the carbon chain with 3-100 carbon atoms as the acting moiety is a substance selected from the group consisting of saturated and/or unsaturated aliphatic hydrocarbons, saturated and/or unsaturated aliphatic or oxo-fatty alcohols, hydrophobic amino acids, fat-soluble vitamins, steroidal lipidoids, phospholipids, sphingomyelin, glycolipids, and/or surfactants.
○,14 The complex according to technical solutions 1-12, wherein the complex is a complex obtained by directly physical compounding a substance having the carbon chain with 3-100 carbon atoms as the acting moiety and any one or two or more selected from the group consisting of protein, polypeptide, oligopeptide, oligosaccharide, amino acid, monosaccharide, disaccharide, nucleotide, vitamin, water-soluble polymer, water-soluble polyamino acid, and/or polysaccharide molecule, in a physicochemical action including hydrogen bond or van der Waals force or a combination thereof, or a mixture obtained by directly physical mixing the same;
   wherein the substance containing the carbon chain with 3-100 carbon atoms used as the acting moiety is a substance selected from the group consisting of saturated and/or unsaturated aliphatic hydrocarbons, saturated and/or unsaturated aliphatic or oxo-fatty alcohols, hydrophobic amino acids, fat-soluble vitamins, steroidal lipidoids, phospholipids, sphingomyelins, glycolipids and/or surfactants.
○,15 The complex according to technical solution 13, wherein the complex is a compound obtained by the reaction of a substance having a carbon chain with 3-100 carbon atoms as the acting moiety with at least one selected from the group consisting of protein, polypeptide, oligopeptide, and amino acid; or a mixture of the compound obtained by the reaction of the substance having a carbon chain with 3-100 carbon atoms as the acting moiety with at least one selected from the group consisting of protein, polypeptide, oligopeptide, and amino acid, unreacted substance as the acting moiety, and/or unreacted at least one selected from the group consisting of protein, polypeptide, oligopeptide, and amino acid.
○,16 The complex according to technical solution 13, wherein the complex is a compound obtained by the reaction of a substance having a carbon chain with 3-100 carbon atoms as the acting moiety, and PEG, with at least one selected from the group consisting of protein, polypeptide, oligopeptide, and amino acid; or a mixture of the compound obtained by the reaction of the carbon chain with 3-100 carbon atoms as the acting moiety, and PEG, with at least one selected from the group consisting of protein, polypeptide, oligopeptide, and amino acid, unreacted PEG, and/or an unreacted at least one selected from the group consisting of protein, polypeptide, oligopeptide, and amino acid.
○,17 The complex according to technical solution 13, wherein the complex is a compound obtained by the reaction of a substance having a carbon chain with 3-100 carbon atoms as the acting moiety with at least one selected from the group consisting of polysaccharide, monosaccharide, disaccharide, and oligosaccharide; or a mixture of the compound obtained by the reaction of the substance having the carbon chain with 3-100 carbon atoms as the acting moiety with at least one selected from the group consisting of polysaccharide, monosaccharide, disaccharide, and oligosaccharide, unreacted substance as the acting moiety and/or unreacted polysaccharide, monosaccharide, disaccharide, and/or oligosaccharide.
○,18 The complex according to technical solution 13, wherein the complex is a compound obtained by the reaction of a substance having a carbon chain with 3-100 carbon atoms as the acting moiety, PEG, with at least one selected from the group consisting of polysaccharide, monosaccharide, disaccharide, and oligosaccharide; or a mixture of the compound obtained by the reaction of the substance having the carbon chain with 3-100 carbon atoms as the acting moiety, PEG, with at least one selected from the group consisting of polysaccharide, monosaccharide, disaccharide, and/or oligosaccharide, unreacted substance as the acting moiety, unreacted PEG, and/or unreacted polysaccharide, monosaccharide, disaccharide, and/or oligosaccharide.
○,19 The complex according to technical solution 13, wherein the protein is one or two or more selected from the group consisting of serum albumins, immunoglobulins, water-soluble collagens, chaperones, water-soluble glycoproteins, and CD14.
0,20 The complex according to any one of technical solutions 13-19, wherein the polysaccharide is one or two or more selected from the group consisting of dextran and/or hyaluronic acid, sialic acid, heparin sulfate, heparan sulfate, chondroitin sulfate, dermatan sulfate, keratan sulfate, acetyl water-soluble cellulose derivatives, β-cyclodextrin and derivative thereof, and water-soluble chitosan derivatives.
0,21 The complex according to technical solution 13, wherein the complex is a compound obtained by the reaction of a substance having a substance having a carbon chain with 3-100 carbon atoms as the acting moiety, a linker, and a thiol-containing protein; or a mixture of the compound obtained from the above reaction, unreacted substance as the acting moiety, unreacted linker, and/or unreacted thiol-containing protein; wherein the linker is one or two or more of an amino acid, succinic acid, butadienoic acid, glutaconic acid, hexaminodioic acid, carbamate, short peptide, N-hydroxybutenimide, polyethylene glycol, and derivatives of the above compounds.
0,22 The complex according to technical solution 21, wherein, the complex is a compound obtained by the reaction of a substance containing a carbon chain with 3-100 carbon atoms as the acting moiety and N-hydroxybuteneimide with a thiol-containing protein; or a mixture of the compound obtained from the above reaction, unreacted substances as the acting moiety, unreacted N-hydroxybuteneimide, and/or unreacted thiol-containing protein.
0,23 The complex according to technical solution 13, wherein the complex is a compound obtained by the reaction of a substance having a carbon chain with 3-100 carbon atoms as the acting moiety, cystamine, with at least one selected from the group consisting of polysaccharide, monosaccharide, disaccharide, and oligosaccharide; or a mixture of the compound obtained by the reaction of the substance having the carbon chain with 3-50 carbon atoms as the acting moiety, cystamine, with at least one selected from the group consisting of polysaccharide, monosaccharide, disaccharide, and oligosaccharide, unreacted substance as the acting moiety and/or unreacted polysaccharide, monosaccharide, disaccharide, and/or oligosaccharide and/or unreacted cystamine.
○,24 The complex according to any one of technical solutions 13-23, wherein the compound obtained by the reaction contains one or two or more selected from the group consising of an amide group, ester group, thioether group, or ether group as a linking moiety between the water-soluble moiety and the acting moiety.
○,25 The complex according to any one of technical solutions 4-23, wherein the number of carbon atoms in the saturated and/or unsaturated fatty hydrocarbons, saturated and/or unsaturated fatty alcohols, or oxo fatty alcohols is 3-50.
○,26 The complex according to technical solution 25, wherein the number of carbon atoms is 3-48.
○,27 The complex according to technical solution 26, wherein the number of carbon atoms is 3-26.
○,28 The complex according to any one of technical solutions 4-23, wherein the protein is human or bovine serum albumin, or CD14; alternatively, the polysaccharide is dextran, heparin, and/or hyaluronic acid.
○,29 The complex according to any one of technical solutions 4-12, wherein the complex is a compound obtained by the reaction of a surfactant containing a carbon chain with 3-30 carbon atoms with at least one selected from the group consisting of a dibasic or polybasic fatty acid, amino acid, targeting protein, targeting polypeptide, and a targeting polysaccharide; or a mixture of the compounds resulting from the above reaction, unreacted surfactant and/or unreacted dibasic or polybasic fatty acid, amino acid, targeting protein, targeting polypeptide and/or targeting polysaccharide.
○,30 The complex according to any one of technical solutions 1-29, wherein the surfactant is one or two or more selected from the group consisting of fatty alcohol polyoxyethylene ethers, fatty acid polyoxyethylene esters, alkyl glycosides, fatty acid sucrose esters, sorbitan fatty acid esters, sorbitan polyoxyethylene fatty acid esters, mannose erythritol esters and N-fatty acyl-N-methylglucamine.
○,31 The complex according to any one of technical solutions 1-30, wherein the microbial infection comprises an infection caused by any one or more of virus, bacteria, fungi, chlamydia, or mycoplasma.
○,32 A preparation for preventing, blocking or treating microbial infections made using the complex according to any one of technical solutions 1-31.
0,33 The preparation according to technical solution 32 being a pharmaceutical preparation or an environmental disinfection and sterilization preparation.
0,34 The preparation according to technical solution 33, wherein the pharmaceutical preparation is one selected from the group consisting of an inhalant, a nasal spray, an injection, an oral preparation, and a transdermal topical formulation.
0,35 Use of the complex according to any one of technical solutions 1-31 in the preparation of a pharmaceutical preparation or environmental microbial disinfection and sterilization reagent for preventing, blocking and/or treating microbial infections.
0,36 The use according to technical solution 35, wherein the microorganism is any one or more selected from the group consisting of viruses, bacteria, fungi, chlamydia, or mycoplasma.
○,37 The use according to technical solution 36, wherein the virus is an enveloped virus; and/or a non-enveloped virus.
○,38 The use according to technical solution 37, wherein the enveloped virus is one or two or more selected from the group consisting of coronavirus, influenza virus, AIDS virus, hepatitis B virus, hepatitis C virus, herpes virus, Zika virus, Dengue virus, Japanese encephalitis virus, Ebola virus, rabies virus, and Hantavirus; the non-enveloped virus is or two or more of hepatitis A virus, human papilloma virus, polio virus and Coxsackie virus.
○,39 The use according to technical solution 36, wherein the virus is any one or two or more selected from the group consisting of coronavirus, AIDS virus, hepatitis B virus, hepatitis C virus, herpes virus, Japanese encephalitis virus, rabies virus, human papilloma virus and Ebola virus.
0,40 The use according to technical solution 36, wherein the bacteria are Gram-positive and/or Gram-negative bacteria and the fungi are pathogenic fungi and/or conditionally pathogenic fungi; the Chlamydia is Chlamydia trachomatis, Chlamydia pneumoniae, and/or Chlamydia psittaci; the mycoplasma include Mycoplasma pneumoniae, Ureaplasma urealyticum, Mycoplasma hominis, and/or Mycoplasma genitalium.
0,41 The use according to technical solution 36, wherein the bacteria are one or two or more selected from the group consisting of Escherichia coli, Staphylococcus aureus, Methicillin-resistant Staphylococcus aureus, Streptococcus pneumoniae, Klebsiella pneumoniae and Pseudomonas aeruginosa; the fungus is one or two or more selected from the group consisting of Candida albicans, Aspergillus niger, Actinomyces viscosus, Chaetomium globosum, Aspergillus protuberus and Microsporum canis.
0,42 The use according to technical solution 36, wherein the virus is one or more selected from the group consisting of H7N9 influenza virus, H5N1 influenza virus, HIV virus, novel coronavirus, HPV virus, and rabies virus.
0,43 A preparation method of the complex according to any one of technical solutions 1-32, the complex being obtained by the reaction of a compound having a fat-soluble saturated and/or unsaturated carbon chain with a branched, cyclic, and/or linear structure with a water-soluble molecule, and optionally added protein, polypeptide, amino acid, oligopeptide, oligosaccharide, monosaccharide, and/or polysaccharide molecule when needed capable of binding to a microbial lipid membrane, microorganism surface domain, or cell wall, and optionally added a linker molecule when needed, in the presence of a catalyst.
0,44 The preparation method of the complex according to technical solution 43, wherein the complex is a purified product of the compound obtained by the reaction.
0,45 A preparation method of the complex according to any one of technical solutions 1-31, the complex being obtained by physical mixing of a compound having a fat-soluble saturated and/or unsaturated carbon chain with a branched, cyclic, and/or linear structure with a water-soluble molecule, and at least optionally added one selected from the group consisting of protein, polypeptide, amino acid, oligopeptide, oligosaccharide, monosaccharide, and polysaccharide molecule when needed capable of binding to a microbial lipid membrane, virus surface domain, or cell wall.
0,46 A preparation method of the complex according to any one of technical solutions 1-31, the complex being obtained by the reaction of a substance having the carbon chain with 3-100 carbon atoms as the acting moiety with at least one selected from the group consisting of a protein, peptide, oligopeptide, oligosaccharide, amino acid, monosaccharide, disaccharide, nucleotide, vitamin, water-soluble polymer, water-soluble polyamino acid, and polysaccharide in the presence of a catalyst.
   Wherein the catalyst can be one or more selected from the group consisting of EDC, DCC, NHS, DMAP, HoBt and derivatives and analogues thereof, and the catalyst is preferably a carbodiimide and a succinimide in a molar ratio of 0.1: 1 to 10: 1, preferably 1: 1 to 1: 10.
0,47 The preparation method of the complex according to technical solution 46, wherein the complex is a purified product of the compound obtained by the reaction.
0,48 The preparation method of the complex according to any one of technical solutions 1-31, wherein the complex is a complex obtained by directly physical compounding a substance having the carbon chain with 3-100 carbon atoms as the acting moiety and at least one selected from the group consisting of protein, polypeptide, oligopeptide, oligosaccharide, amino acid, monosaccharide, disaccharide, nucleotide, vitamin, water-soluble polymer, water-soluble polyamino acid, and polysaccharide molecule, in a physicochemical action, or by directly physical mixing the same;

In particular, the present invention provides the following third set of technical solutions in order to solve the lack of agents in the prior art which have no toxic side effects and, in particular, cannot widely kill and block, prevent or treat microbial infections:
○,1 A complex capable of preventing, blocking and/or treating a viral or bacterial infection comprising an acting moiety, a binding moiety, and a water-soluble moiety;
   wherein the virus is one or two or more viruses selected from the group consisting of novel coronavirus, influenza virus, AIDS virus, hepatitis B virus, human herpes virus, Ebola virus, rabies virus, and a human papilloma virus, and the bacteria is one or two or more bacteria selected from the group consisting of *Escherichia coli,* Staphylococcus *aureus,* methicillin-resistant *Staphylococcus aureus, Streptococcus pneumoniae, Klebsiella pneumoniae* and *Pseudomonas aeruginosa;*
      the acting moiety is a fat-soluble saturated and/or unsaturated carbon chain with a branched, cyclic and/or linear structure; the carbon chain is a molecule or a residue of the molecule, and the carbon chain is a carbon chain with 3-100 carbon atoms; wherein the acting moiety is a carbon chain or residue of the carbon chain with 3-100 carbon atoms formed by saturated and/or unsaturated fatty acids;
   the water-soluble moiety is a water-soluble molecule or a residue of the molecule; the molecule contains one or two or more functional groups selected from the group consisting of amide group, phosphoryloxy group, carboxyl group, phosphate group, sulfonyl group, sulfonyloxy group, hydroxyl group, quaternary ammonium group, thioether group, disulfide bond, ether group, thiol group, aldehyde group, ester group, amine group, amino group, urea group, and guanidine group; and the water-soluble moiety can be one or two or more of the above functional groups linked to the carbon chain as the acting moiety;
   the binding moiety is a molecule or a residue of the molecule capable of binding to a microbial lipid membrane, a microorganism surface protein, a microorganism surface polysaccharide, or a cell wall component or capable of binding to a polysaccharide or a protein or polypeptide in the microorganism; the binding moiety can be the same as the water-soluble moiety, i.e. a protein, polypeptide, amino acid, oligopeptide, oligosaccharide, monosaccharide and/or polysaccharide molecule or a residue thereof capable of binding to a microbial lipid membrane and a surface domain; and
   wherein the number of any one moiety of the acting moiety, the water-soluble moiety, and the binding moiety can be 1 or 1 or more.
○,2 The complex according to technical solution 1, wherein the number of carbon atoms is 3-48.
○,3 The complex according to technical solution 1, wherein the number of carbon atoms is 3-26.
○,4 The complex according to technical solution 1, wherein the water-soluble moiety is a water-soluble molecule or a residue of the molecule of one or two or more groups selected from the group consisting of a thiol group, amino group, phosphate group, carboxyl group, sulfonyl group, hydroxyl group, amine group, urea group, guanidine group, and disulfide group;
   the binding moiety has a group for binding, i.e. capable of binding to the microbial lipid membrane, the microorganism surface protein, the microorganism surface polysaccharide or the cell wall component or capable of binding to a polysaccharide, protein, or polypeptide in the microorganism; the group is two or more groups from the water-soluble moiety or from groups independently as the binding moiety selected from the group consisting of a thiol group, amino group, phosphate group, carboxyl group, sulfonyl group, hydroxyl group, amine group, urea group, guanidine group, and disulfide group, or from one or two or more groups providing a carbon chain to carbon chain connection which is selected from the group consisting of a thiol group, amino group, phosphate group, carboxyl group, sulfonyl group, hydroxyl group, amine group, urea group, guanidine group, and disulfide group, such that the complex has one or two or more groups of thiol group, amino group, phosphate group, carboxyl group, sulfonyl group, hydroxyl group, amine group, urea group, guanidine group, and disulfide group.
○,5 The complex according to technical solution 4, wherein the binding moiety is one or two or more selected from the group consisting of dibasic or polybasic fatty acid, amino acid, targeting protein, targeting peptide, and targeting polysaccharide.
0,6 The complex according to technical solution 4, wherein the complex is a complex formed by linking a fatty acid with 3-50 carbon atoms and a water-soluble amino acid; or the complex is a complex formed by linking a fatty acid with 3-50 carbon atoms to the targeting polypeptide; or the complex is a complex formed by the reaction of a fatty acid with 3-50 carbon atoms and the targeting polypeptide with PEG; or the complex is a complex formed by the reaction of a surfactant and one or two or more selected from the group consisting of a dibasic or polybasic fatty acid, an amino acid, a targeting protein, a targeting polypeptide and a targeting polysaccharide.
0,7 The complex according to technical solution 4, wherein the saturated and/or unsaturated fatty acid is selected from the group consisting of saturated fatty acids or unsaturated fatty acids with 3-50 carbon atoms; the fatty acid is a fatty acid or amino acid containing a double bond, triple bond, hydroxyl group, amino group and/or oxo group, and is a monobasic, dibasic, or polybasic acid.
0,8 The complex according to technical solution 4, wherein the saturated and/or unsaturated fatty acid is one or two or more selected from the group consisting of saturated fatty acids with 3-46 carbon atoms, monoenoic acids with 3-34 carbon atoms, dienoic acids with 5-30 carbon atoms, trienoic acids with 7-30 carbon atoms, tetraenoic acids with 12-38 carbon atoms, pentaenoic acids with 12-38 carbon atoms, hexaenoic acids with 22-38 carbon atoms, alkynoic acids with 6-22 carbon atoms, dialkynoic acids with 10-22 carbon atoms, trialkynoic acids with 12-22 carbon atoms, enynic acids with 8- 20 carbon atoms, fatty acids with 3-30 carbon atoms in the main chain and 1-10 alkyl and/or 1-3 hydroxyl groups in the branches, saturated linear and branched dicarboxylic and tricarboxylic acids with 3-38 carbon atoms and unsaturated linear or branched dicarboxylic and tricarboxylic acids with 4-18 carbon atoms that is substitutable with hydroxyl groups, carboxylic acids with 3-18 carbon atoms substituted with amino, hydroxyl, oxo and/or methyl, N-fatty acyl amino acids with 6-30 carbon atoms, amino acids containing 2 or more fatty acyl groups, polybasic carboxylic acids linked by thioether and amide bonds.
0,9 The complex according to technical solution 4, wherein the saturated or unsaturated fatty acid is one or two or more selected from the group consisting of fumaric acid, octanoic acid, glutaconic acid, hexanoic acid, nonanoic acid, dodecanoic acid, tetradecanoic acid, hexadecanoic acid, octadecanoic acid, eicosanoic acid, oleic acid, linoleic acid, linolenic acid, eicosapentaenoic acid, docosapentaenoic acid, docosahexaenoic acid, pentacosanoic acid, heptanoic acid, decanoic acid, dodecenoic acid, tetradecenoic acid, dotriacontahexaenoic acid, octacosanoic acid, or a carbon chain residue formed thereof.
○,10 The complex according to technical solution 4, wherein the water-soluble moiety is a molecule or a residue of the molecule containing one or two or more groups selected from the group consisting of a thiol group, amine group, carboxyl group, hydroxyl group, and disulfide group; the molecule is one or two or more water-soluble macromolecules selected from the group consisting of proteins, polysaccharides, nucleic acids, and artificially synthesized water-soluble polymers, or residues thereof;
   and/or, one or two or more medium molecules selected from the group consisting of polypeptides, oligopeptides, oligosaccharides, oligonucleotides, and synthetic water-soluble medium molecular weight polymers, or residues thereof;
   and/or, one or two or more water-soluble small molecules selected from the group consisting of amino acids, monosaccharides, disaccharides, nucleotides, water-soluble vitamins, and deoxynucleotides, or residues thereof;
   and/or, a molecule linked to the carbon chain as the acting moiety or a residue thereof, and the molecule or residue thereof contains one or two or more groups selected from the group consisting of a thiol group, amine group, carboxyl group, hydroxyl group, and disulfide group.
○,11 The complex according to technical solution 10, wherein the protein as the water-soluble macromolecule is one or two or more water-soluble macromolecules selected from the group consisting of serum albumins, immunoglobulins, water-soluble collagens, chaperones, water-soluble glycoproteins, and CD14; the polysaccharide as the macromolecule is one or two or more water-soluble macromolecules selected from the group consisting of dextran, hyaluronic acid, sialic acid, heparin sulfate, heparan sulfate, chondroitin sulfate, dermatan sulfate, keratan sulfate, acetyl water-soluble cellulose derivatives, β-cyclodextrin and derivatives thereof, and water-soluble chitosan derivatives; the water-soluble polymer as the macromolecule is one or two or more water-soluble macromolecules selected from the group consisting of polyethylene glycol and carboxylated or aminated polyethylene glycol, polyvinyl alcohol and carboxylated or quaternized polyvinyl alcohol, polyacrylic acid and ammonium polyacrylate;
   the water-soluble medium molecular weight polymer is one or two or more substances selected from the group consisting of targeting polypeptides, oligopeptides, oligosaccharides, oligonucleotides and/or water-soluble polyamino acids;
   the water-soluble small molecular weight monosaccharide and/or disaccharide is one or two or more selected from the group consisting of glucose, fructose, rhamnose, sorbose, sucrose, maltose, lactose, and trehalose; the nucleotide and/or deoxynucleotide as the water-soluble small molecule is one or two or more selected from the group consisting of adenosine, guanylate, uranylate, cytidylate, thymidylate, inosine, deoxyadenosine, deoxyguanosine, deoxycytidylate, and deoxythymidylate; the amine acid as the water-soluble small molecule is one or two or more amino acids such as serine, threonine, cysteine, asparagine, glutamine, tyrosine, lysine, arginine, histidine, aspartate, glutamate, citrulline, ornithine, taurine, and aminobutyric acid; the vitamin as the water-soluble small molecule is one or two or more selected from the group consisting of vitamin B1, pantothenic acid, vitamin B6, and vitamin C.
0,12 The complex according to technical solution 11, wherein the targeting polypeptide comprises any one of a protein or neutralizing antibody fragment that specifically targets a microbial lipid membrane, a bacterial and fungal cell wall, a virus surface protein domain.
0,13 The complex according to technical solution 11, wherein the water-soluble polyamino acid is selected from the group consisting of polyglutamate, polylysine, and/or polyaspartate.
○,14 The complex according to technical solution 1, wherein the binding moiety and the water-soluble moiety are the same, i.e. a protein, polypeptide, oligopeptide, oligosaccharide, monosaccharide, disaccharide, amino acid, nucleotide, vitamin, water-soluble polymer, water-soluble polyamino acid and/or polysaccharide molecule capable of binding to a lipid membrane, surface domain of a microorganism, or a residue thereof; the molecule or residue thereof comprises one or two or more groups selected from the group consisting of a thiol group, amine group, carboxyl group, hydroxyl group, and disulfide group.
○,15 The complex according to technical solution 1, wherein the complex is a compound obtained by the reaction of a saturated and/or unsaturated fatty acid with 3-100 carbon atoms with a protein, polypeptide, oligopeptide, oligosaccharide, monosaccharide, disaccharide, nucleotide, vitamin, amino acid, water-soluble polymer, water-soluble polyamino acid, and/or polysaccharide; or is a mixture of the compound obtained by the reaction of the saturated and/or unsaturated fatty acid with 3-50 carbon atoms with a protein, polypeptide, oligopeptide, oligosaccharide, monosaccharide, disaccharide, nucleotide, vitamin, amino acid, water-soluble polymer, water-soluble polyamino acid, and/or polysaccharide, and unreacted fatty acid and/or unreacted protein, polypeptide, oligopeptide, oligosaccharide, monosaccharide, disaccharide, nucleotide, vitamin, amino acid, water-soluble polymer, water-soluble polyamino acid, and/or polysaccharide molecule.
0,16 The complex according to technical solution 1, wherein the complex is a complex obtained by directly physical compounding a saturated and/or unsaturated fatty acid with 3-100 carbon atoms and a protein, polypeptide, oligopeptide, oligosaccharide, monosaccharide, disaccharide, nucleotide, vitamin, amino acid, water-soluble polymer, water-soluble polyamino acid, and/or polysaccharide molecule, in a physicochemical action including hydrogen bond or van der Waals force or a combination thereof, or a mixture obtained by directly physical mixing the same.
0,17 The complex according to technical solution 15, wherein the complex is a compound obtained by the reaction of a saturated and/or unsaturated fatty acid with 3-100 carbon atoms with at least one selected from the group consisting of protein, polypeptide, oligopeptide, and amino acid; or a mixture of the compound obtained by the reaction of the saturated and/or unsaturated fatty acid with 3-100 carbon atoms with at least one selected from the group consisting of protein, polypeptide, oligopeptide, and amino acid, unreacted fatty acid, and/or unreacted at least one selected from the group consisting of protein, polypeptide, oligopeptide, and amino acid.
○,18 The complex according to technical solution 15, wherein the complex is a compound obtained by the reaction of a saturated and/or unsaturated fatty acid with 3-100 carbon atoms, and PEG, with at least one selected from the group consisting of protein, polypeptide, oligopeptide, and amino acid; or a mixture of the compound obtained by the reaction of the saturated and/or unsaturated fatty acid with 3-100 carbon atoms, and PEG, with at least one selected from the group consisting of protein, polypeptide, oligopeptide, and amino acid, unreacted PEG, and/or an unreacted at least one selected from the group consisting of protein, polypeptide, oligopeptide, and amino acid.
○,19 The complex according to technical solution 15, wherein the complex is a compound obtained by the reaction of a saturated and/or unsaturated fatty acid with 3-100 carbon atoms with a polysaccharide, monosaccharide, disaccharide, and/or oligosaccharide; or a mixture of the compound obtained by the reaction of the saturated and/or unsaturated fatty acid with 3-100 carbon atoms polysaccharide, monosaccharide, disaccharide, and/or oligosaccharide, unreacted fatty acid and/or unreacted polysaccharide, monosaccharide, disaccharide, and/or oligosaccharide.
0,20 The complex according to technical solution 15, wherein the complex is a compound obtained by the reaction of a saturated and/or unsaturated fatty acid with 3-100 carbon atoms, and PEG, with at least one selected from the group consisting of polysaccharide, monosaccharide, disaccharide, and oligosaccharide; or a mixture of the compound obtained by the reaction of the saturated and/or unsaturated fatty acid with 3-100 carbon atoms, and PEG, with at least one selected from the group consisting of polysaccharide, monosaccharide, disaccharide, and oligosaccharide, unreacted fatty acids, unreacted PEG and/or unreacted polysaccharide, monosaccharide, disaccharide, and/or oligosaccharide.
0,21 The complex according to technical solution 15, wherein the protein is one or two or more selected from the group consisting of serum albumins, immunoglobulins, water-soluble collagens, chaperones, water-soluble glycoproteins, and CD14.
0,22 The complex according to technical solution 15, wherein the polysaccharide is one or two or more selected from the group consisting of dextran and/or hyaluronic acid, sialic acid, heparin sulfate, heparan sulfate, chondroitin sulfate, dermatan sulfate, keratan sulfate, acetyl water-soluble cellulose derivatives, β-cyclodextrin and derivative thereof, and water-soluble chitosan derivatives.
0,23 The complex according to technical solution 15, wherein the complex is a compound obtained by the reaction of a saturated and/or unsaturated fatty acid with 3-100 carbon atoms, a linker, and a thiol-containing protein; or a mixture of the compound obtained from the above reaction, unreacted fatty acid, unreacted linker, and/or unreacted thiol-containing protein; wherein the linker is one or two or more of an amino acid, succinic acid, butadienoic acid, glutaconic acid, hexaminodioic acid, carbamate, short peptide, N-hydroxybutenimide, polyethylene glycol, and derivatives of the above compounds.
0,24 The complex according to technical solution 23, wherein the complex is a compound obtained by the reaction of a saturated and/or unsaturated fatty acid with 3-100 carbon atoms, N-hydroxybutenimide and a thiol-containing protein; or a mixture of the compound obtained by the above reaction, unreacted fatty acid, unreacted N-hydroxybutenimide, and/or unreacted thiol-containing protein.
0,25 The complex according to technical solution 15, wherein the complex is a compound obtained by the reaction of a saturated and/or unsaturated fatty acid with 3-100 carbon atoms and cystamine with at least one selected from the group consisting of polysaccharide, monosaccharide, disaccharide, and oligosaccharide; or a mixture of the compound obtained by the reaction of the saturated and/or unsaturated fatty acid with 3-50 carbon atoms and cystamine with at least one selected from the group consisting of polysaccharide, monosaccharide, disaccharide, and oligosaccharide, unreacted fatty acid, unreacted at least one selected from the group consisting of polysaccharide, monosaccharide, disaccharide, and oligosaccharide, and/or unreacted cystamine.
0,26 The complex according to any one of technical solutions 15-25, wherein the compound obtained by the reaction contains one or two or more selected from the group consisting of an amide group, ester group, thioether group, or ether group as a linking moiety between the water-soluble moiety and the acting moiety.
0,27 The complex according to any one of technical solutions 4-25, wherein the number of carbon atoms of the saturated and/or unsaturated fatty acid is 3-50.
0,28 The complex according to technical solution 27, wherein the number of carbon atoms is 3-48.
0,29 The complex according to technical solution 27, wherein the number of carbon atoms is 3-26.
0,30 The complex according to any one of technical solutions 4-25, wherein the saturated and/or unsaturated fatty acid is a fatty acid with 3-40 carbon atoms, which is a fatty acid with 1-8 C=C double bonds, a fatty acid with 1-7 C=C double bonds, a fatty acid with 1-6 double bonds, a fatty acid with 1-5 double bonds, a fatty acid with 1-4 double bonds, a fatty acid with 1-3 double bonds, or a fatty acid with 1-2 double bonds.
0,31 The complex according to any one of technical solutions 4-25, wherein the saturated and/or unsaturated fatty acid is a fatty acid with 1-6 double bonds and 3-30 carbon atoms.
0,32 The complex according to any one of technical solutions 4-25, wherein the number of carbon atoms of the saturated and/or unsaturated fatty acid is 3-30.
0,33 The complex according to any one of claims 4-25, wherein the saturated and/or unsaturated fatty acid is one or two or more fatty acids selected from the group consisting of fumaric acid, octanoic acid, glutaconic acid, hexanoic acid, nonanoic acid, dodecanoic acid, tetradecanoic acid, hexadecanoic acid, octadecanoic acid, eicosanoic acid, oleic acid, linoleic acid, linolenic acid, eicosapentaenoic acid, docosapentaenoic acid, docosahexaenoic acid, pentacosanoic acid, heptanoic acid, decanoic acid, dodecenoic acid, tetradecenoic acid, dotriacontahexaenoic acid, and octacosanoic acid.
0,34 The complex according to any one of technical solutions 4-25, wherein the protein is human or bovine serum albumin, or CD14; alternatively, the polysaccharide is dextran and/or hyaluronic acid.
0,35 The present invention also provides a preparation for preventing, blocking or treating microbial infections made using the complex described above.
0,36 The preparation according to the present invention being a pharmaceutical preparation or an environmental disinfection and sterilization preparation.
○,37 The preparation according to the present invention, wherein the pharmaceutical preparation is one selected from the group consisting of an inhalant, a nasal spray, an injection, an oral preparation, and a transdermal topical formulation.
○,38 Use of the complex according to the present invention in the preparation of a pharmaceutical preparation or environmental microbial disinfection and sterilization reagent for preventing, blocking and/or treating microbial infections.

The use according to the present invention, wherein the microorganism is either or both selected from the group consisting of viruses and bacteria.

The use according to the present invention, wherein the virus is an enveloped virus; and/or a non-enveloped virus.

The use according to the present invention, wherein the virus is one or two or more viruses selected from the group consisting of novel coronavirus, influenza virus, AIDS virus (HIV), hepatitis B virus, human herpes virus, Ebola virus, rabies virus, and a human papilloma virus (HPV), and the bacteria is one or two or more bacteria selected from the group consisting of Escherichia coli, Staphylococcus aureus, methicillin-resistant Staphylococcus aureus, Streptococcus pneumoniae, Klebsiella pneumoniae and Pseudomonas aeruginosa.

The use according to the present invention, wherein the virus is one or more selected from the group consisting of H7N9 influenza virus, H5N1 influenza virus, HIV virus, novel coronavirus, HPV virus, and rabies virus.

0,39 The present invention further provide a preparation method of the complex, the complex being obtained by the reaction of a fatty acid having a fat-soluble saturated and/or unsaturated carbon chain with a branched, cyclic, and/or linear structure with a water-soluble molecule, and optionally added protein, polypeptide, amino acid, oligopeptide, oligosaccharide, monosaccharide, and/or polysaccharide molecule as needed capable of binding to a microbial lipid membrane, microorganism surface domain, or cell wall, and optionally added a linker molecule as needed, in the presence of a catalyst.

Further preferably, the preparation method of the complex according to the present invention, wherein the complex is a purified product of the compound obtained by the reaction.

0,40 The present invention further provide a preparation method of the complex, the complex being obtained by physical mixing of a fatty acid having a fat-soluble saturated and/or unsaturated carbon chain with a branched, cyclic, and/or linear structure with a water-soluble molecule, and optionally added protein, polypeptide, amino acid, oligopeptide, oligosaccharide, monosaccharide, and/or polysaccharide molecule as needed capable of binding to a microbial lipid membrane, virus surface domain, or cell wall.

○,41 The present invention further provide a preparation method of the complex, the complex being obtained by the reaction of a saturated and/or unsaturated fatty acid with 3-100 carbon atoms with any one of a protein, peptide, oligopeptide, oligosaccharide, monosaccharide, disaccharide, nucleotide, vitamin, amino acid, water-soluble polymer, water-soluble polyamino acid, and/or polysaccharide in the presence of a catalyst.

Further preferably, the complex is a purified product of the compound obtained by the reaction.

0,42 The present invention further provide a preparation method of the complex, the complex being obtained by directly physical compounding a saturated and/or unsaturated fatty acid with 3-100 carbon atoms and at least one selected from the group consisting of protein, polypeptide, oligopeptide, polysaccharide, oligosaccharide, monosaccharide, disaccharide, nucleotide, vitamin, amino acid, water-soluble polymer, water-soluble polyamino acid, and/or polysaccharide molecule, in a physicochemical action, or by directly physical mixing the same.

The use of the complex of the present invention capable of preventing and treating viral, bacterial, and fungal infections and the preparation thereof in the prevention or treatment of various viral, bacterial, and fungal infectious diseases; specific application modes include:
it can be used before infection to prevent viral, bacterial, and fungal infections;
it can be used after infection to kill viruses, bacteria and fungi in vivo; and
It can disinfect and sterilize the environment of the article to prevent the spread of viruses, bacteria and fungi.

Compared with the prior art, the present invention has the beneficial effects as follows:
(1) The effect of the complex provided by the invention on the virus is not affected by viral variation.
   The target of the complex provided by the present invention is the basic structure of the virus-the envelope and the nucleocapsid. In the case of enveloped viruses, the complex destroys the viral envelope, so that the virus loses its ability to infect cells. In the case of non-enveloped viruses, the complex directly encapsulates the virus nucleocapsid for hydrophobic isolation, so that the virus cannot infect cells. The complexes do not fail due to viral variation.
(2) The complex provided by the present invention can kill resistant microorganisms without causing resistance.
   Bacteria exhibit drug resistance due to the presence of resistance genes in their bodies, which can express enzymes that break down antibiotics, rendering them ineffective. The microbial killing mechanism of the complex of the present invention is different from that of antibiotics, which directly acts on the basic structure of the microorganism, i.e. lipid membrane, by partially integrating into the lipid membrane, affecting lipid membrane homeostasis, and destroying cell wall and cell membrane to achieve the killing effect. So, it is not affected by the enzymes that break down antibiotics in resistant bacteria.
(3) The complex provided by the present invention is safe for human somatic cells.
   Viral particles, as well as bacterial and fungal cells, are much smaller than human cells, and therapeutic doses of the complex preferentially bind to viruses, bacteria and fungi. Through cell experiments, it has been verified that the complex has no significant effect on the cell membrane at the therapeutic dose. The complex is safe for human cells.
(4) The complex provided herein may function in different regions depending on molecular size; the macromolecular of macromolecules can be retained on the mucosal surface of the respiratory tract or in the blood circulation for the first time to inactivate viruses, bacteria or fungi, preventing the spread of viruses, bacteria or fungi in the body. Macromolecular complexes cannot enter normal tissues and can only enter inflammatory sites after viral, bacterial or fungal infection; while small molecule complexes can cross the vessel wall into interstitial space and interstitial fluid, targeting viral, bacterial or fungal for killing.
(5) The fatty acids, fatty alcohols, fat-soluble vitamins and steroids are insoluble in water or have very low water solubility, and cannot be directly injected into human body. The direct injection into vein may cause pulmonary embolism. Fatty acids in food are absorbed by the human body in the form of emulsions, which pass through the lymphatic system, lymphatic vessels, and thoracic ducts, and reflux into the bloodstream in the form of chylomicrons. Fatty acids are also encapsulated in emulsions in a non covalent manner with proteins. This form of hydrophobic group is encapsulated inside and cannot come into contact with infected viruses and bacteria, thus failing to exert bactericidal and antiviral effects. The above problems can be avoided by converting the liposoluble hydrophobic compound into an aqueous compound having a high affinity for pathogenic microorganisms.

Furthermore, based on in-depth research on the mechanism of action, the present invention has discovered and provided the following fourth set of technical solutions.
1. A water-soluble carbon chain substance for regulating transmembrane transport and/or fluidity of a cell membrane, comprising an acting moiety, a binding moiety, and a water-soluble moiety; the acting moiety is a fat-soluble saturated and/or unsaturated carbon chain with a branched, cyclic and/or linear structure; the carbon chain is a molecule or a residue of the molecule, and the carbon chain is a carbon chain with 3-100 carbon atoms; wherein the acting moiety is a carbon chain or residue of the carbon chain with 3-100 carbon atoms formed by any one or more of unsaturated fatty acids, fatty hydrocarbons, or cyclic hydrocarbons; or aromatic or heterocyclic compounds; or their salts, alcohols, ethers, esters, or other derivatives;
   the water-soluble moiety is a water-soluble molecule or a residue of the molecule; the molecule contains one or two or more functional groups selected from the group consisting of amide group, phosphoryloxy group, carboxyl group, phosphate group, sulfonyl group, sulfonyloxy group, hydroxyl group, quaternary ammonium group, thioether group, disulfide bond, ether group, thiol group, aldehyde group, ester group, amine group, amino group, urea group, and guanidine group; and the water-soluble moiety can be one or two or more of the above functional groups linked to the carbon chain as the acting moiety;
   the binding moiety is a molecule or a residue of the molecule capable of binding to a microbial lipid membrane, microorganism surface protein, microorganism surface polysaccharide, or a cell wall component, or binding to a polysaccharide, protein, or peptide in a microorganism, plant, animal, or human body, or a molecule or a residue of the molecule capable of binding to a cell membrane or a cell membrane surface polysaccharide or cell wall component in a plant, animal, or human body tissue; the binding moiety can be the same as the water-soluble moiety, that is, the protein, peptide, amino acid, oligopeptide, oligosaccharide, monosaccharide, disaccharide, amino acid, nucleotide, vitamin, water-soluble polymer, water-soluble polymeric amino acid and/or polysaccharide molecule or their residues capable of binding to a microbial lipid membrane, or the surface domain of a cell membrane in a plant, animal, or human body tissue; and
   wherein the number of any one moiety of the acting moiety, the water-soluble moiety, and the binding moiety can be 1 or 2 or more.
2. The water-soluble carbon chain substance according to technical solution 1, wherein the number of carbon atoms is 3-50, preferably 3-48, and more preferably 3-26.
3. The water-soluble carbon chain substance according to technical solution 1, wherein the water-soluble moiety is a water-soluble molecule or a residue of the molecule of one or two or more groups selected from the group consisting of a thiol group, amino group, phosphate group, carboxyl group, sulfonyl group, hydroxyl group, amine group, urea group, guanidine group, and disulfide group; and
   the binding moiety has a group for binding, i.e. capable of binding to a microbial lipid membrane, microorganism surface protein, microorganism surface polysaccharide, or a cell wall component, or binding to a polysaccharide, protein, or peptide in a microorganism, plant, animal, or human body, or a molecule or a residue of the molecule capable of binding to a cell membrane or a cell membrane surface polysaccharide or cell wall component in a plant, animal, or human body tissue; the group is one or two or more groups from the water-soluble moiety or from groups independently as the binding moiety which is selected from the group consisting of a thiol group, amino group, phosphate group, carboxyl group, sulfonyl group, hydroxyl group, amine group, urea group, guanidine group, and disulfide group, or from one or two or more groups providing a carbon chain to carbon chain connection which is selected from the group consisting of a thiol group, amino group, phosphate group, carboxyl group, sulfonyl group, hydroxyl group, amine group, urea group, guanidine group, and disulfide group, such that the complex has one or two or more groups of thiol group, amino group, phosphate group, carboxyl group, sulfonyl group, hydroxyl group, amine group, urea group, guanidine group, and disulfide group.
4. The water-soluble carbon chain substance according to any one of technical solutions 1-3, wherein the binding moiety is one or two or more selected from the group consisting of dibasic or polybasic fatty acid, amino acid, targeting protein, targeting peptide, and targeting polysaccharide,
5. The water-soluble carbon chain substance according to any one of technical solutions 1-3, wherein the water-soluble carbon chain substance is a compound, complex, or mixture.
6. The water-soluble carbon chain substance according to technical solution 5, wherein the water-soluble carbon chain substance is a complex formed by linking a fatty acid with 3-50 carbon atoms and a water-soluble amino acid; or a complex formed by linking a fatty acid with 3-50 carbon atoms to the targeting polypeptide; or a complex formed by the reaction of a fatty acid with 3-50 carbon atoms and the targeting polypeptide with PEG; or a complex formed by the reaction of a surfactant and one or two or more selected from the group consisting of a dibasic or polybasic fatty acid, an amino acid, a targeting protein, a targeting polypeptide and a targeting polysaccharide.
7. The water-soluble carbon chain substance according to any one of technical solutions 1-4, wherein the saturated and/or unsaturated fatty acid is selected from the group consisting of saturated fatty acids or unsaturated fatty acids with 3-50 carbon atoms; the fatty acid is a fatty acid or amino acid containing a double bond, triple bond, hydroxyl group, amino group and/or oxo group, and is a monobasic, dibasic, or polybasic acid.
8. The water-soluble carbon chain substance according to technical solution 7, wherein the saturated and/or unsaturated fatty acid is one or two or more selected from the group consisting of saturated fatty acids with 3-46 carbon atoms, monoenoic acids with 3-34 carbon atoms, dienoic acids with 5-30 carbon atoms, trienoic acids with 7-30 carbon atoms, tetraenoic acids with 12-38 carbon atoms, pentaenoic acids with 12-38 carbon atoms, hexaenoic acids with 22-38 carbon atoms, alkynoic acids with 6-22 carbon atoms, dialkynoic acids with 10-22 carbon atoms, trialkynoic acids with 12-22 carbon atoms, enynic acids with 8-20 carbon atoms, fatty acids with 3-30 carbon atoms in the main chain and 1-10 alkyl and/or 1-3 hydroxyl groups in the branches, saturated linear and branched dicarboxylic and tricarboxylic acids with 3-38 carbon atoms and unsaturated linear or branched dicarboxylic and tricarboxylic acids with 4-18 carbon atoms that is substitutable with hydroxyl groups, carboxylic acids with 3-18 carbon atoms substituted with amino, hydroxyl, oxo and/or methyl, N-fatty acyl amino acids with 6-30 carbon atoms, amino acids containing 2 or more fatty acyl groups, polybasic carboxylic acids linked by thioether and amide bonds.
9. The water-soluble carbon chain substance according to technical solution 7, wherein the saturated or unsaturated fatty acid is one or two or more selected from the group consisting of fumaric acid, octanoic acid, octenoic acid, glutaconic acid, hexanoic acid, octanedioic acid, nonanoic acid, dodecanoic acid, dodecanedioic acid, tridecaneoic acid, tridecanedioic acid, tetradecanoic acid, hexadecanoic acid, hexadecanedioic acid, octadecanoic acid, eicosanoic acid, eicosanedioic acid, oleic acid, linoleic acid, linolenic acid, eicosapentaenoic acid, docosenoic acid, docosapentaenoic acid, docosahexaenoic acid, pentacosanoic acid, heptanoic acid, decanoic acid, undecenoic acid, dodecenoic acid, tetradecenoic acid, hexadecenoic acid, triacontenoic acid, dotriacontahexaenoic acid, octacosanoic acid, or a carbon chain residue formed thereof.
10. The water-soluble carbon chain substance according to any one of technical solutions 1-4, wherein the water-soluble moiety is a molecule or a residue of the molecule containing one or two or more groups selected from the group consisting of a thiol group, amine group, carboxyl group, hydroxyl group, and disulfide group; the molecule is one or two or more water-soluble macromolecules selected from the group consisting of proteins, polysaccharides, nucleic acids, and artificially synthesized water-soluble polymers, or residues thereof;
   and/or, one or two or more medium molecules selected from the group consisting of polypeptides, oligopeptides, oligosaccharides, oligonucleotides, and synthetic water-soluble medium molecular weight polymers, or residues thereof;
   and/or, one or two or more water-soluble small molecules selected from the group consisting of amino acids, monosaccharides, disaccharides, nucleotides, water-soluble vitamins, and deoxynucleotides, or residues thereof;
   and/or, a molecule linked to the carbon chain as the acting moiety or a residue thereof, and the molecule or residue thereof contains one or two or more groups selected from the group consisting of a thiol group, amine group, carboxyl group, hydroxyl group, and disulfide group.
11. The water-soluble carbon chain substance according to technical solution 10, wherein the protein as the water-soluble macromolecule is one or two or more water-soluble macromolecules selected from the group consisting of serum albumins, immunoglobulins, water-soluble collagens, chaperones, water-soluble glycoproteins, and CD14; the polysaccharide as the macromolecule is one or two or more water-soluble macromolecules selected from the group consisting of dextran, hyaluronic acid, sialic acid, heparin sulfate, heparan sulfate, chondroitin sulfate, dermatan sulfate, keratan sulfate, acetyl water-soluble cellulose derivatives, β-cyclodextrin and derivatives thereof, and water-soluble chitosan derivatives; the water-soluble polymer as the macromolecule is one or two or more water-soluble macromolecules selected from the group consisting of polyethylene glycol and carboxylated or aminated polyethylene glycol, polyvinyl alcohol and carboxylated or quaternized polyvinyl alcohol, polyacrylic acid and ammonium polyacrylate;
   the water-soluble medium molecular weight polymer is one or two or more substances selected from the group consisting of targeting polypeptides, oligopeptides, oligosaccharides, oligonucleotides and/or water-soluble polyamino acids; and
   the water-soluble small molecular weight monosaccharide and/or disaccharide is one or two or more selected from the group consisting of glucose, fructose, rhamnose, sorbose, sucrose, maltose, lactose, and trehalose; the nucleotide and/or deoxynucleotide as the water-soluble small molecule is one or two or more selected from the group consisting of adenosine, guanylate, uranylate, cytidylate, thymidylate, inosine, deoxyadenosine, deoxyguanosine, deoxycytidylate, and deoxythymidylate; the amine acid as the water-soluble small molecule is one or two or more of amino acids such as serine, threonine, cysteine, asparagine, glutamine, tyrosine, lysine, arginine, histidine, aspartate, glutamate, citrulline, ornithine, taurine, and aminobutyric acid; the vitamin as the water-soluble small molecule is one or two or more selected from the group consisting of vitamin B1, pantothenic acid, vitamin B6, and vitamin C.
12. The water-soluble carbon chain substance according to technical solution 11, wherein the targeting polypeptide comprises any one of a protein or neutralizing antibody fragment that specifically targets a microbial lipid membrane, a bacterial and fungal cell wall, a virus surface protein domain.
13. The water-soluble carbon chain substance according to technical solution 11, wherein the water-soluble polyamino acid is selected from the group consisting of polyglutamate, polylysine, and/or polyaspartate.
14. The water-soluble carbon chain substance according to technical solution 1, wherein the binding moiety and the water-soluble moiety are the same, i.e. a protein, polypeptide, oligopeptide, oligosaccharide, monosaccharide, disaccharide, amino acid, nucleotide, vitamin, water-soluble polymer, water-soluble polyamino acid and/or polysaccharide molecule capable of binding to a microbial lipid membrane, a cell membrane or surface domain in a cell membrane of an animal or human body tissue, or a residue thereof; the molecule or residue thereof comprises one or two or more groups selected from the group consisting of a thiol group, amine group, carboxyl group, hydroxyl group, and disulfide group.
15. The water-soluble carbon chain substance according to technical solution 1, wherein the water-soluble carbon chain substance is a compound obtained by the reaction of a saturated and/or unsaturated fatty acid with 3-100 carbon atoms with at least one selected from the group consisting of protein, polypeptide, oligopeptide, oligosaccharide, monosaccharide, disaccharide, nucleotide, vitamin, amino acid, water-soluble polymer, water-soluble polyamino acid, and polysaccharide molecule; or is a mixture of the compound obtained by the reaction of the saturated and/or unsaturated fatty acid with 3-100 carbon atoms with at least one selected from the group consisting of protein, polypeptide, oligopeptide, oligosaccharide, monosaccharide, disaccharide, nucleotide, vitamin, amino acid, water-soluble polymer, water-soluble polyamino acid, and polysaccharide molecule, and unreacted fatty acid and/or unreacted protein, polypeptide, oligopeptide, oligosaccharide, monosaccharide, disaccharide, nucleotide, vitamin, amino acid, water-soluble polymer, water-soluble polyamino acid, and/or polysaccharide molecule.
16. The water-soluble carbon chain substance according to technical solution 1, wherein the water-soluble carbon chain substance is a complex obtained by directly physical compounding a saturated and/or unsaturated fatty acid with 3-100 carbon atoms and at least one selected from the group consisting of protein, polypeptide, oligopeptide, oligosaccharide, monosaccharide, disaccharide, nucleotide, vitamin, amino acid, water-soluble polymer, water-soluble polyamino acid, and/or polysaccharide molecule, in a physicochemical action including hydrogen bond or van der Waals force or a combination thereof, or a mixture obtained by directly physical mixing the same.
17. The water-soluble carbon chain substance according to technical solution 15, wherein the complex is a compound obtained by the reaction of a saturated and/or unsaturated fatty acid with 3-100 carbon atoms with at least one selected from the group consisting of protein, polypeptide, oligopeptide, and amino acid; or a mixture of the compound obtained by the reaction of the saturated and/or unsaturated fatty acid with 3-100 carbon atoms with at least one selected from the group consisting of protein, polypeptide, oligopeptide, and amino acid, unreacted fatty acid, and/or unreacted at least one selected from the group consisting of protein, polypeptide, oligopeptide, and amino acid.
18. The water-soluble carbon chain substance according to technical solution 15, wherein the complex is a compound obtained by the reaction of a saturated and/or unsaturated fatty acid with 3-100 carbon atoms, and PEG, with at least one selected from the group consisting of protein, polypeptide, oligopeptide, and amino acid; or a mixture of the compound obtained by the reaction of the saturated and/or unsaturated fatty acid with 3-100 carbon atoms, and PEG, with at least one selected from the group consisting of protein, polypeptide, oligopeptide, and amino acid, unreacted PEG, and/or an unreacted at least one selected from the group consisting of protein, polypeptide, oligopeptide, and amino acid.
19. The complex according to technical solution 15, wherein the complex is a compound obtained by the reaction of a saturated and/or unsaturated fatty acid with 3-100 carbon atoms with at least one selected from the group consisting of polysaccharide, monosaccharide, disaccharide, and oligosaccharide; or a mixture of the compound obtained by the reaction of the saturated and/or unsaturated fatty acid with 3-100 carbon atoms with at least one selected from the group consisting of polysaccharide, monosaccharide, disaccharide, and oligosaccharide, unreacted fatty acid and/or unreacted polysaccharide, monosaccharide, disaccharide, and/or oligosaccharide.
20. The water-soluble carbon chain substance according to technical solution 15, wherein the water-soluble carbon chain substance is a compound obtained by the reaction of a saturated and/or unsaturated fatty acid with 3-100 carbon atoms, and PEG, with at least one soelected from polysaccharide, monosaccharide, disaccharide, and oligosaccharide; or a mixture of the compound obtained by the reaction of the saturated and/or unsaturated fatty acid with 3-100 carbon atoms, and PEG, with at least one soelected from polysaccharide, monosaccharide, disaccharide, and oligosaccharide, unreacted fatty acids, unreacted PEG and/or unreacted polysaccharide, monosaccharide, disaccharide, and/or oligosaccharide.
21. The water-soluble carbon chain substance according to technical solution 15 or 16, wherein the protein is one or two or more selected from the group consisting of serum albumins, immunoglobulins, water-soluble collagens, chaperones, water-soluble glycoproteins, and CD14.
22. The water-soluble carbon chain substance according to technical solution 15 or 16, wherein the polysaccharide is one or two or more selected from the group consisting of dextran and/or hyaluronic acid, sialic acid, heparin sulfate, heparan sulfate, chondroitin sulfate, dermatan sulfate, keratan sulfate, acetyl water-soluble cellulose derivatives, β-cyclodextrin and derivative thereof, and water-soluble chitosan derivatives.
23. The water-soluble carbon chain substance according to technical solution 15, wherein the complex is a compound obtained by the reaction of a saturated and/or unsaturated fatty acid with 3-100 carbon atoms, a linker, and a thiol-containing protein; or a mixture of the compound obtained from the above reaction, unreacted fatty acid, unreacted linker, and/or unreacted thiol-containing protein; wherein the linker is one or two or more of an amino acid, succinic acid, butadienoic acid, glutaconic acid, hexaminodioic acid, carbamate, short peptide, N-hydroxybutenimide, polyethylene glycol, and derivatives of the above compounds.
24. The water-soluble carbon chain substance according to technical solution 23, wherein the water-soluble carbon chain substance is a compound obtained by the reaction of a saturated and/or unsaturated fatty acid with 3-100 carbon atoms, N-hydroxybutenimide and a thiol-containing protein; or a mixture of the compound obtained by the above reaction, unreacted fatty acid, unreacted N-hydroxybutenimide, and/or unreacted thiol-containing protein.
25. The water-soluble carbon chain substance according to technical solution 15, wherein the water-soluble carbon chain substance is a compound obtained by the reaction of a saturated and/or unsaturated fatty acid with 3-100 carbon atoms and cystamine with at least one selected from the group consisting of polysaccharide, monosaccharide, disaccharide, and oligosaccharide; or a mixture of the compound obtained by the reaction of a saturated and/or unsaturated fatty acid with 3-50 carbon atoms and cystamine with at least one selected from the group consisting of polysaccharide, monosaccharide, disaccharide, and oligosaccharide, unreacted fatty acid, unreacted at least one soelected from polysaccharide, monosaccharide, disaccharide, and oligosaccharide, and/or unreacted cystamine.
26. The water-soluble carbon chain substance according to any one of technical solutions 15-25, wherein the compound obtained by the reaction contains one or two or more selected from the group consisting of an amide group, ester group, thioether group, or ether group as a linking moiety between the water-soluble moiety and the acting moiety.
27. The water-soluble carbon chain substance according to any one of technical solutions 15-25, wherein the number of carbon atoms in the saturated and/or unsaturated fatty acids is 3-50, preferably 3-48, more preferably 3-30, and even more preferably 3-26.
28. The water-soluble carbon chain substance of any one according to technical solutions 15-25, wherein the saturated and/or unsaturated fatty acid is a fatty acid with 3-40 carbon atoms , which is a fatty acid with 1-8 C=C double bonds, a fatty acid with 1-7 C=C double bonds, a fatty acid with 1-6 double bonds, a fatty acid with 1-5 double bonds, a fatty acid with 1-4 double bonds, a fatty acid with 1-3 double bonds, or a fatty acid with 1-2 double bonds; preferably, the saturated and/or unsaturated fatty acid is a fatty acid with 1-6 double bonds and 3-30 carbon atoms.
29. The water-soluble carbon chain substance according to any one of technical solutions 15-25, wherein the number of carbon atoms of the saturated and/or unsaturated fatty acid is 3-30.
30. The water-soluble carbon chain substance according to any one of technical solutions 15-25, wherein the saturated and/or unsaturated fatty acid is one or two or more fatty acid selected from the group consisting of fumaric acid, octanoic acid, octenoic acid, glutaconic acid, hexanoic acid, octanedioic acid, nonanoic acid, dodecanoic acid, dodecanedioic acid, tridecaneoic acid, tridecanedioic acid, tetradecanoic acid, hexadecanoic acid, hexadecanedioic acid, octadecanoic acid, eicosanoic acid, eicosanedioic acid, oleic acid, linoleic acid, linolenic acid, eicosapentaenoic acid, docosenoic acid, docosapentaenoic acid, docosahexaenoic acid, pentacosanoic acid, heptanoic acid, decanoic acid, undecenoic acid, dodecenoic acid, tetradecenoic acid, hexadecenoic acid, triacontenoic acid, dotriacontahexaenoic acid, and octacosanoic acid.
31. The water-soluble carbon chain substance according to any one of technical solutions 15-25, wherein the protein is human or bovine serum albumin, or CD14; alternatively, the polysaccharide is dextran and/or hyaluronic acid.
32. The water-soluble carbon chain substance according to technical solution 75, wherein the number of carbon atoms of the saturated and/or unsaturated fatty acid
   is 3-30.

Preferably, the saturated and/or unsaturated fatty acid is one or two or more fatty acid selected from the group consisting of fumaric acid, octanoic acid, octenoic acid, glutaconic acid, hexanoic acid, octanedioic acid, nonanoic acid, dodecanoic acid, dodecanedioic acid, tridecaneoic acid, tridecanedioic acid, tetradecanoic acid, hexadecanoic acid, hexadecanedioic acid, octadecanoic acid, eicosanoic acid, eicosanedioic acid, oleic acid, linoleic acid, linolenic acid, eicosapentaenoic acid, docosenoic acid, docosapentaenoic acid, docosahexaenoic acid, pentacosanoic acid, heptanoic acid, decanoic acid, undecenoic acid, dodecenoic acid, tetradecenoic acid, hexadecenoic acid, triacontenoic acid, dotriacontahexaenoic acid, and octacosanoic acid.

Further preferably, the protein is human or bovine serum albumin, or CD14; alternatively, the polysaccharide is dextran and/or hyaluronic acid.
1. The water-soluble carbon chain substance according to the technical solution described above, wherein the compound obtained by the reaction is a compound obtained by the reaction of a fatty acid and albumin or SBP1 and has any one or two of the following structural formulas:

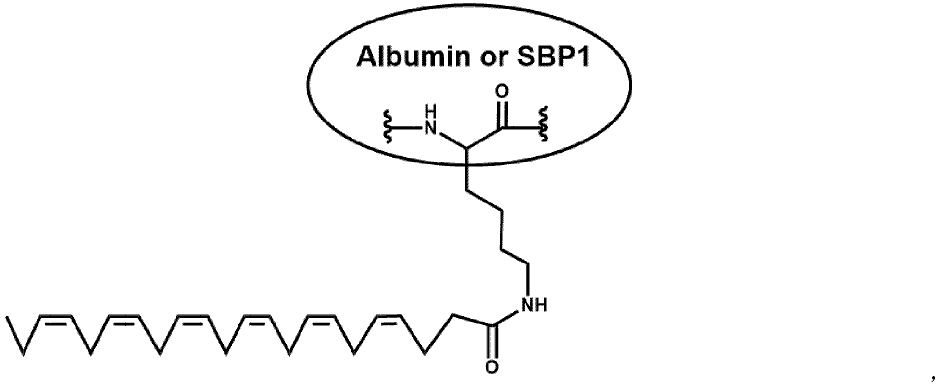

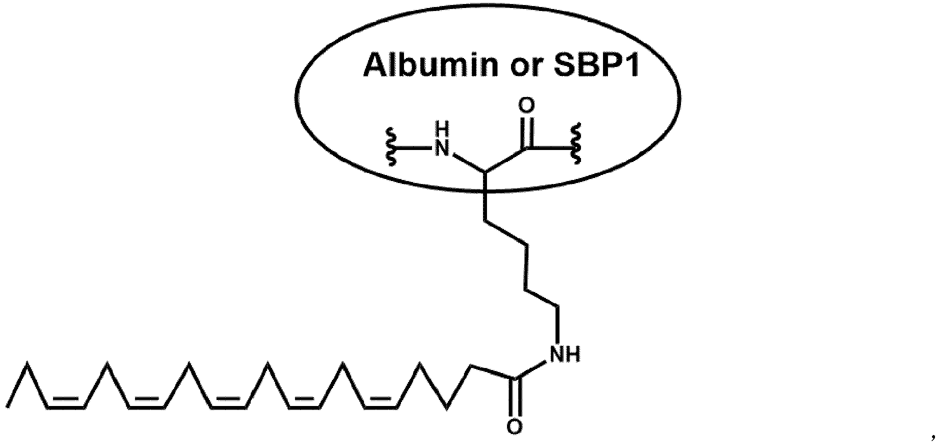

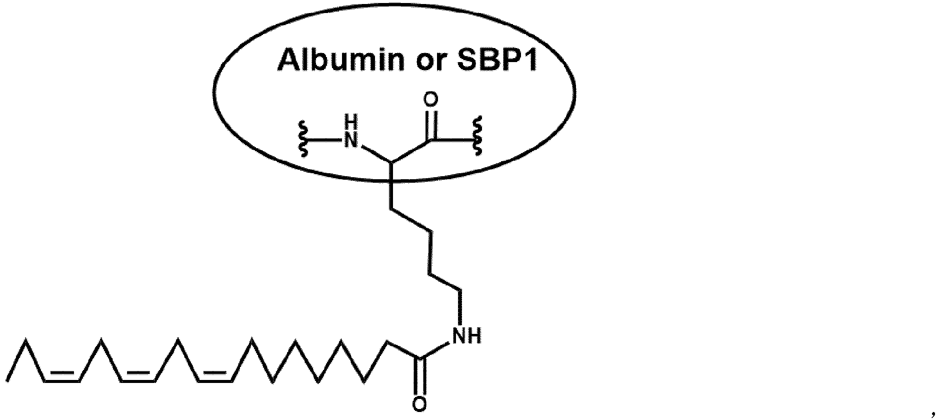

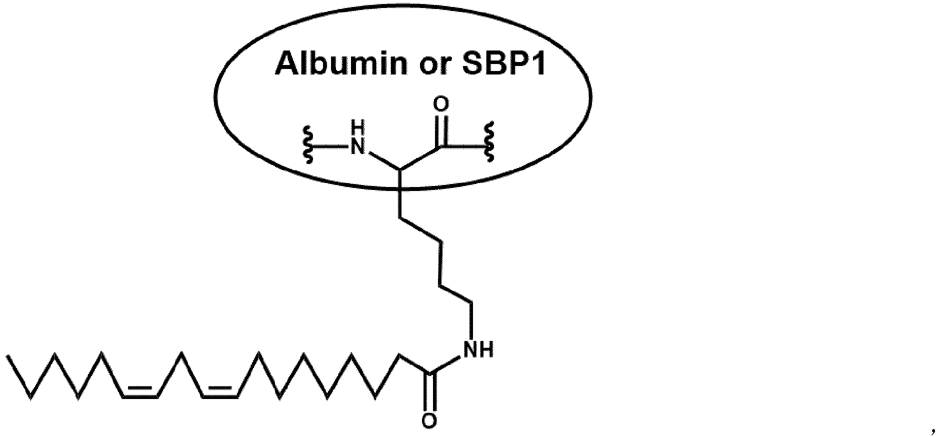

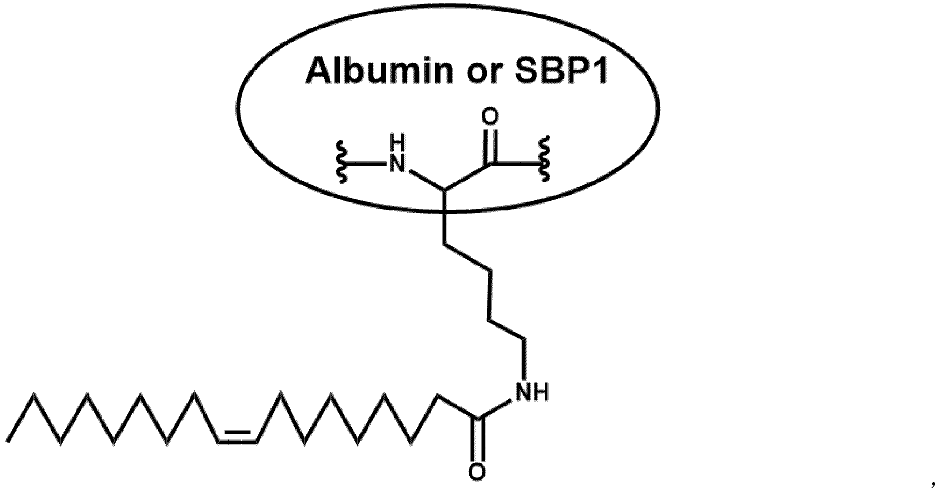

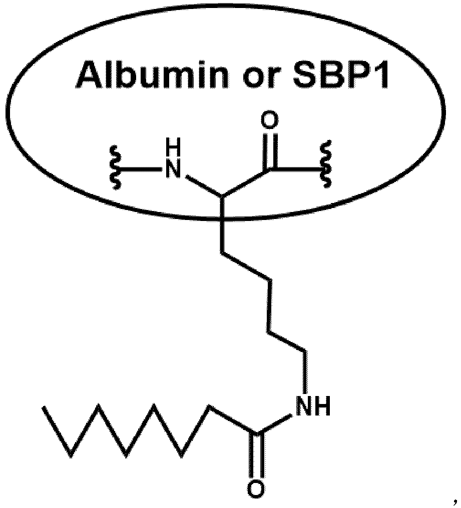

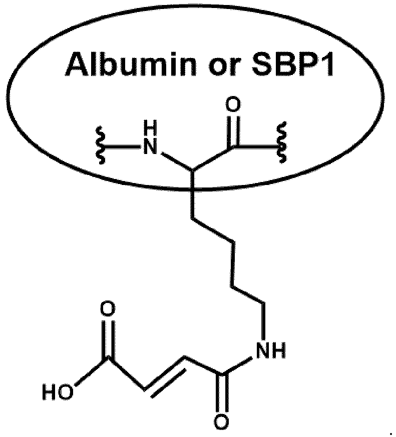

33. The water-soluble carbon chain substance according to the technical solution described above, wherein the compound obtained by the reaction is a compound obtained by the reaction of a monobasic fatty acid with 3-10 carbon atoms and PEG, with an amino acid, or a compound obtained by the reaction of a monobasic fatty acid with 3-10 carbon atoms, a saturated dibasic fatty acid with 5-8 carbon atoms, and PEG with taurine.

34. The water-soluble carbon chain substance according to the technical solution described above, wherein the compound obtained by the reaction is a compound having at least one of the following structural formulas: 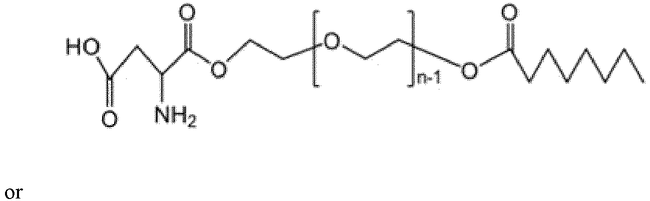 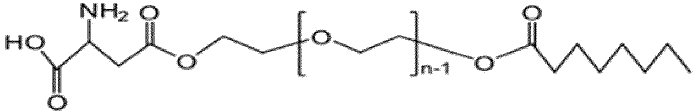 or 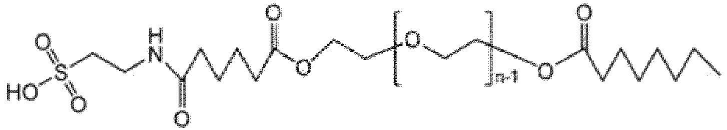 where n is an integer of 1-200.

35. The water-soluble carbon chain substance according to the technical solution described above, wherein the compound obtained by the reaction is any one or two or more compounds that are obtained by the reaction of a fatty acid with dextran and have the following structural formulas:

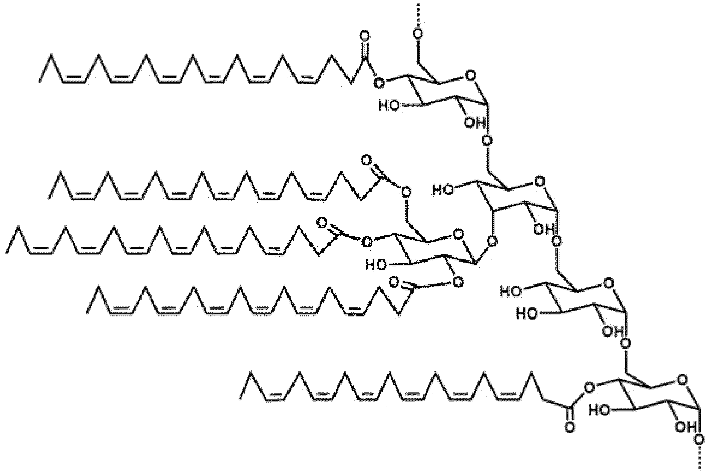

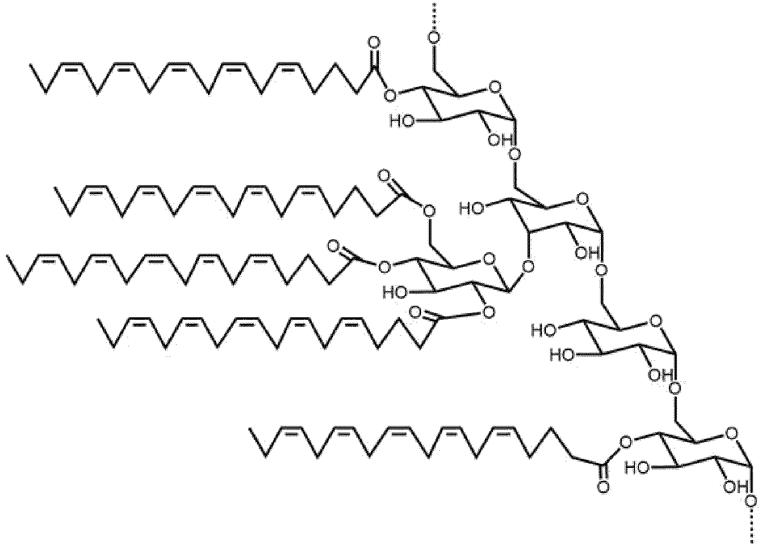

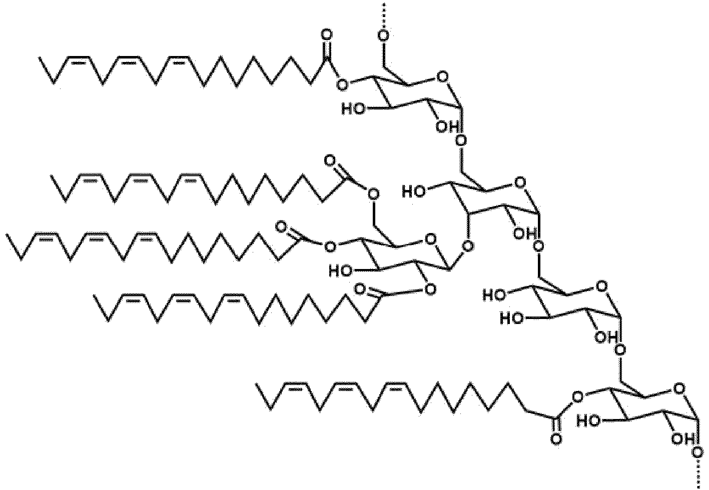

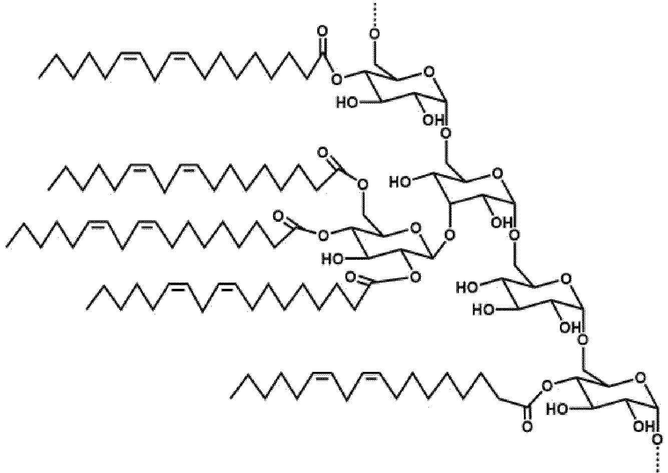

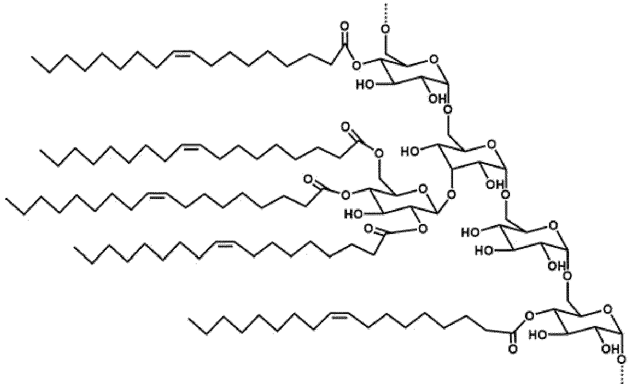

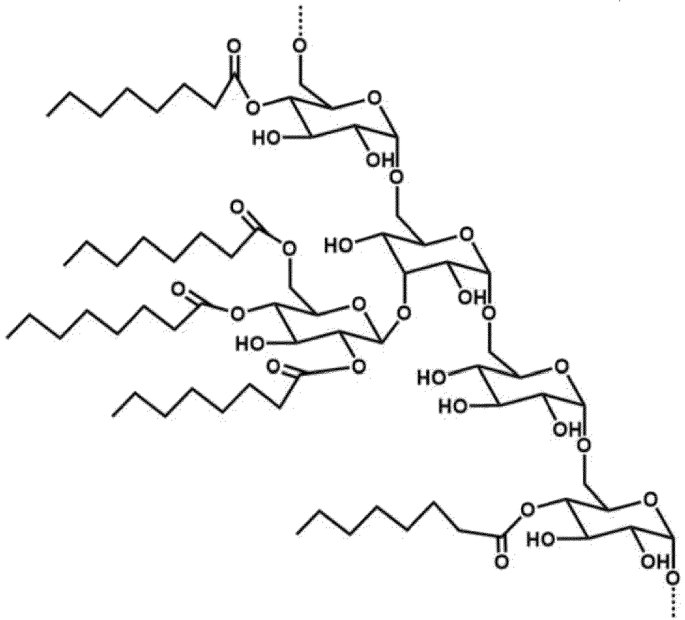

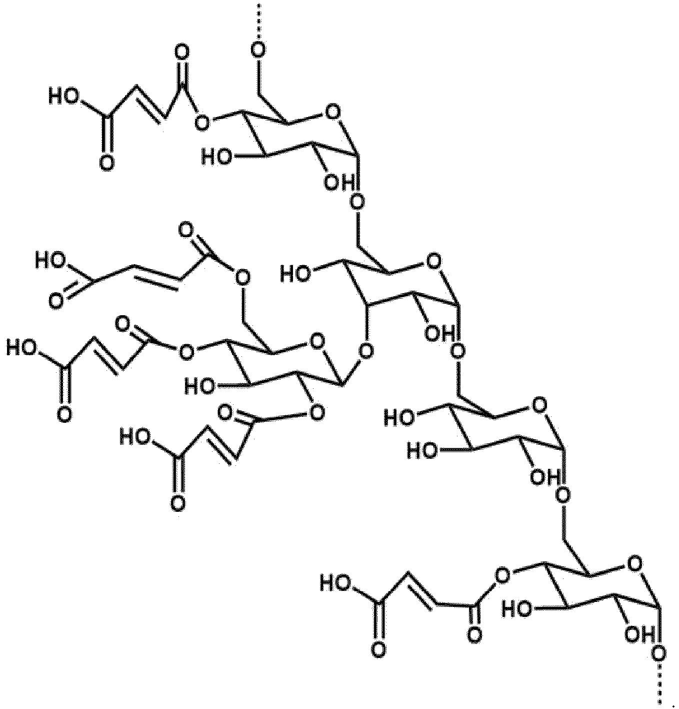

36. The water-soluble carbon chain substance according to the technical solution described above, wherein the compound obtained by the reaction is any one or two or more compounds that are obtained by the reaction of a fatty acid with hyaluronic acid and have the following structural formulas: 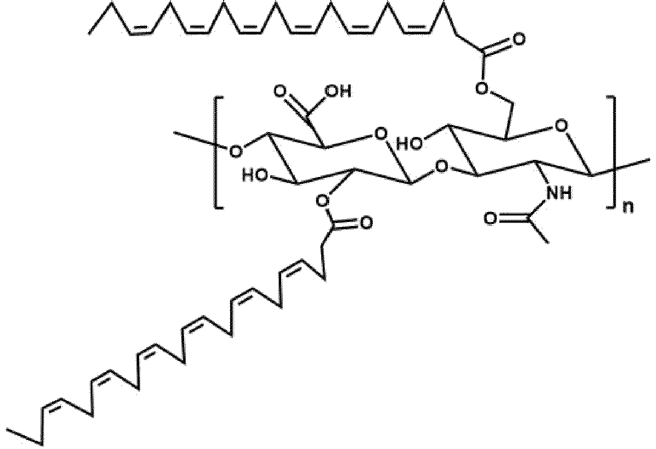 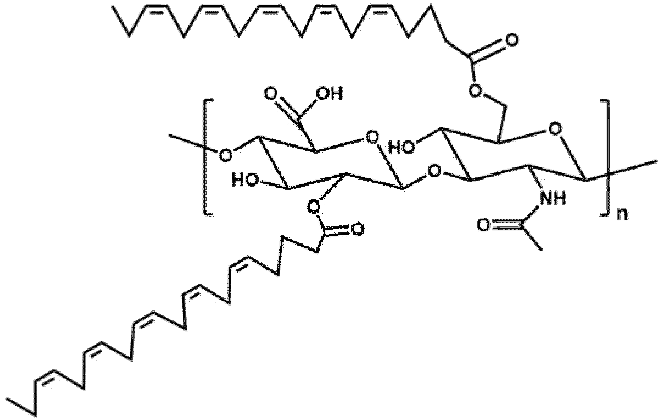 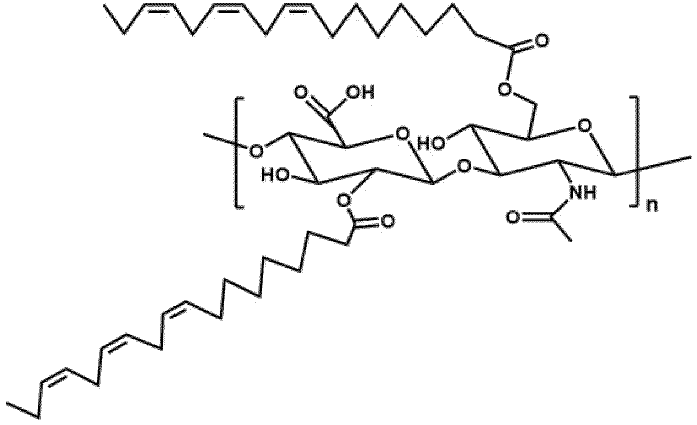 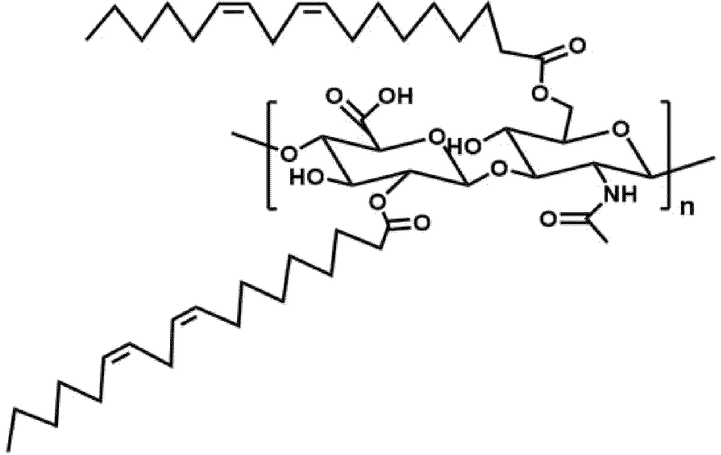 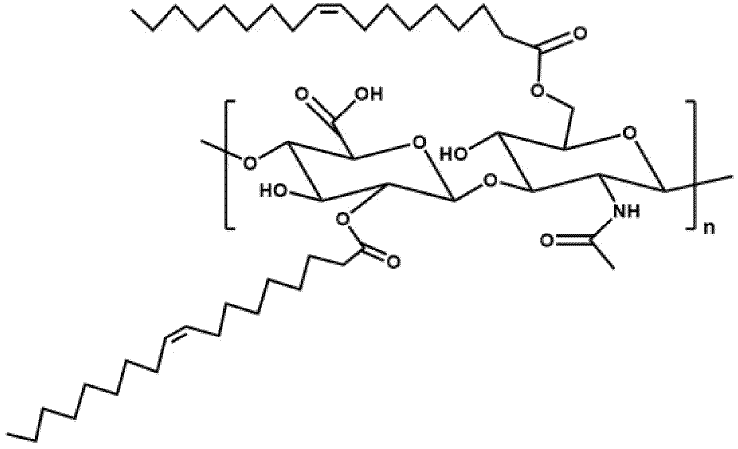 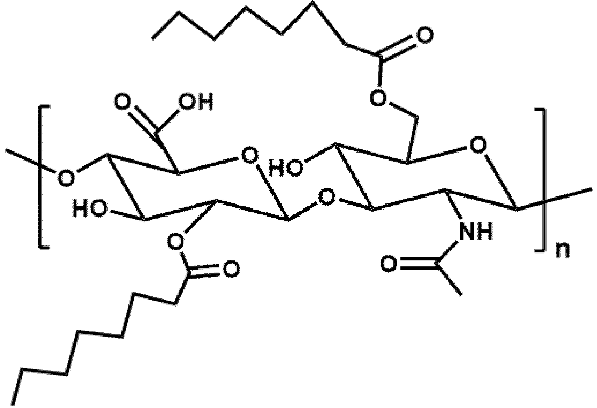 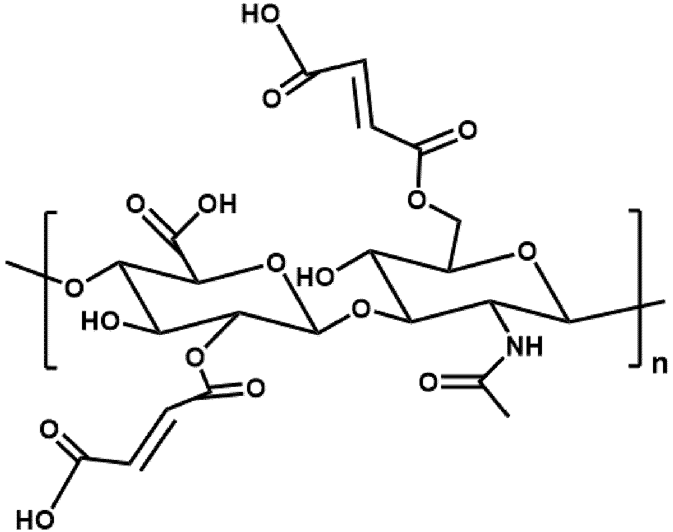 where n is an integer of 1-2000.

37. The water-soluble carbon chain substance according to the technical solution described above, wherein the compound obtained by the reaction is a compound obtained by the reaction of a fatty acid with 3-10 carbon atoms with PEG and glucose.

38. The water-soluble carbon chain substance according to the technical solution described above, wherein the compound obtained by the reaction is a compound having the following structural formula: 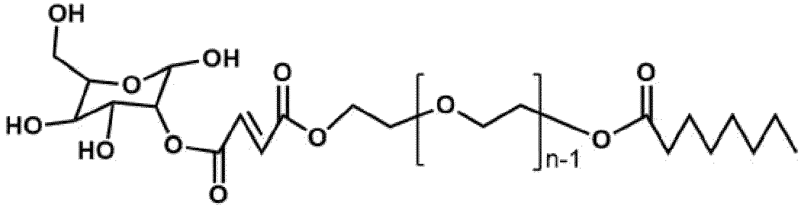 where n is an integer of 1-200.

39. The water-soluble carbon chain substance according to the technical solution described above, wherein the compound obtained by the reaction is any one or two or more compounds having a thioether bond, that are obtained by the reaction of a fatty acid, N-hydroxybutenimide, and a protein and have the following structural formulas:

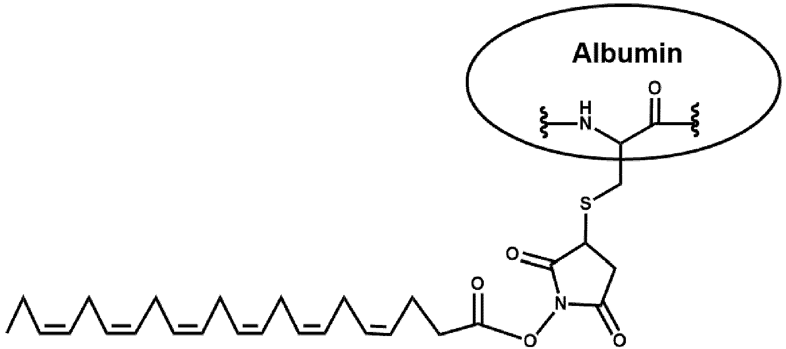

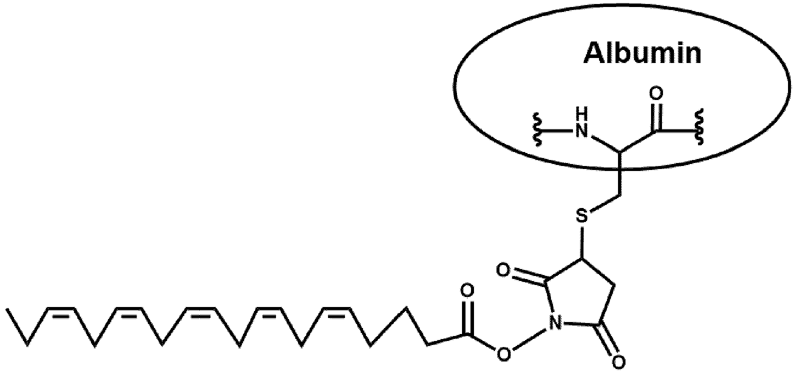

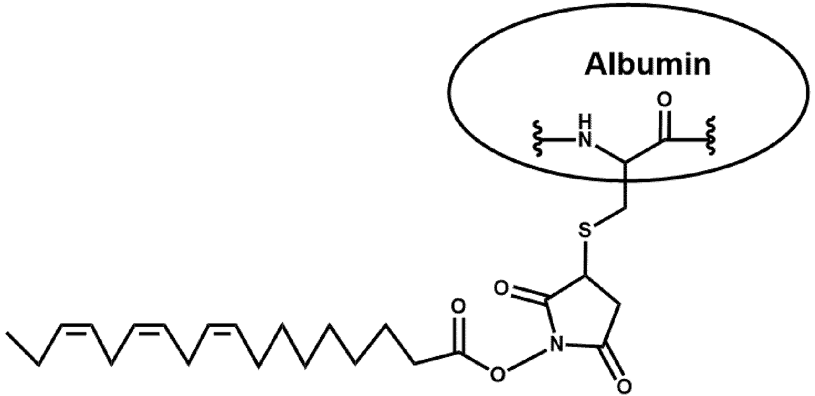

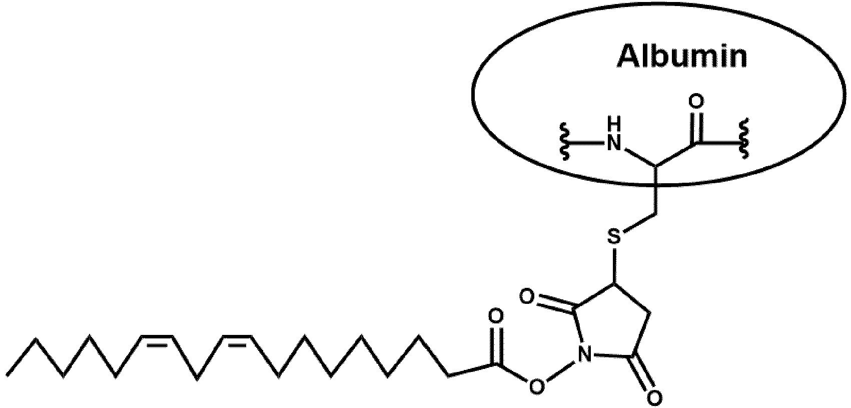

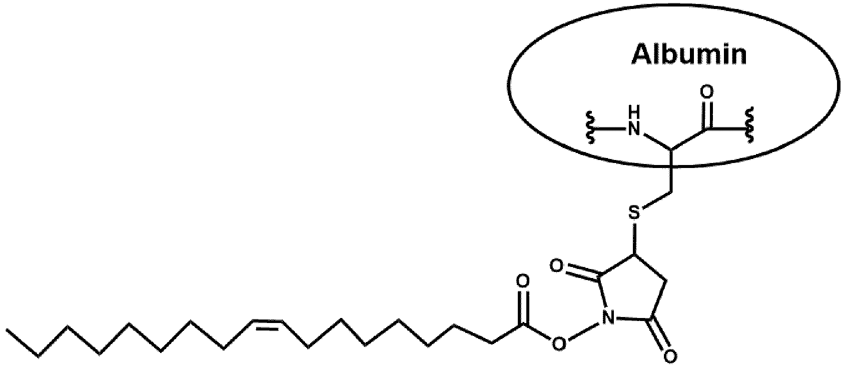

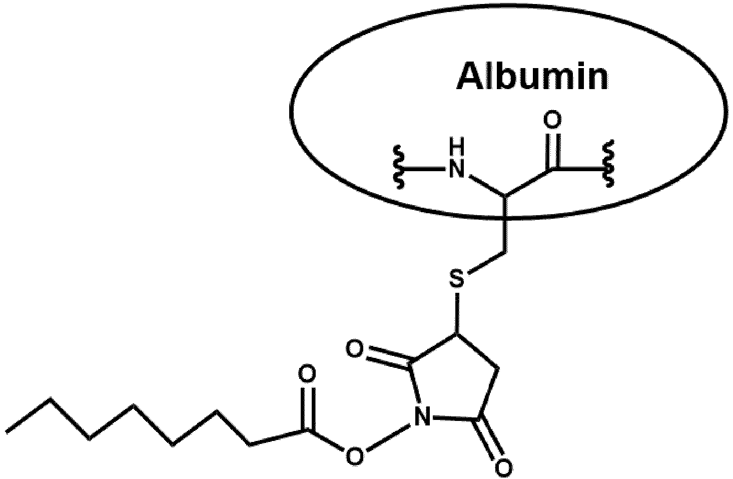

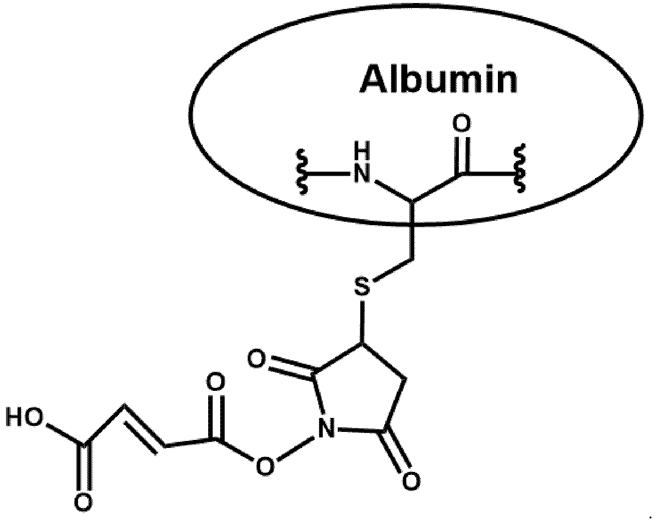

40. The water-soluble carbon chain substance according to the technical solution described above, wherein the compound obtained by the reaction is any one or two or more compounds that are obtained by the reaction of a fatty acid, cystamine and dextran and have the following structural formulas:

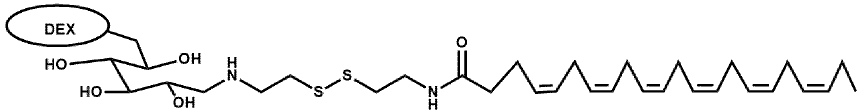

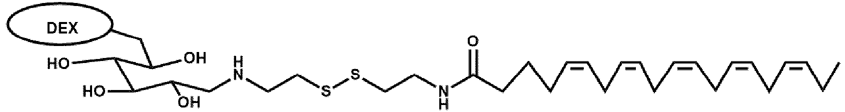

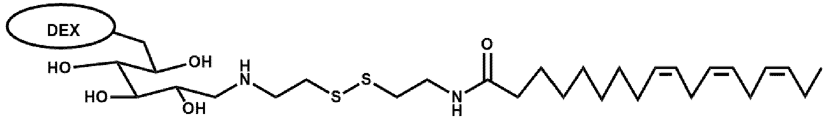

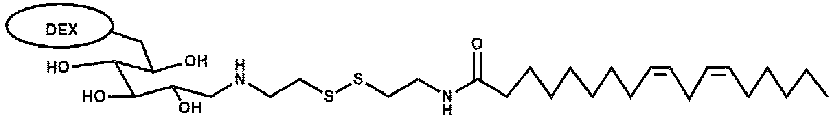

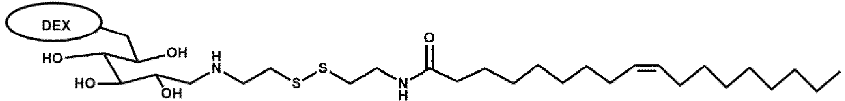

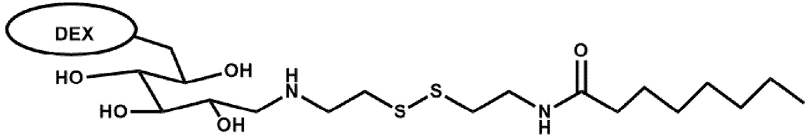

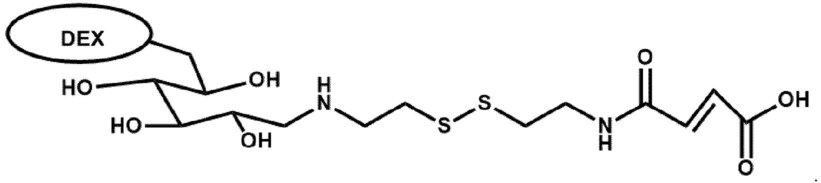

41. The water-soluble carbon chain substance according to the technical solution described above, wherein the compound obtained by the reaction is any one or two or more compounds that are obtained by the reaction of a fatty acid and cystamine with hyaluronic acid and have the following structural formulas:

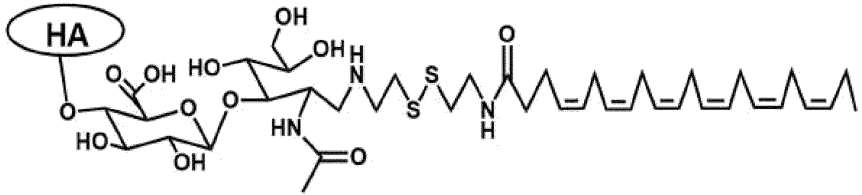

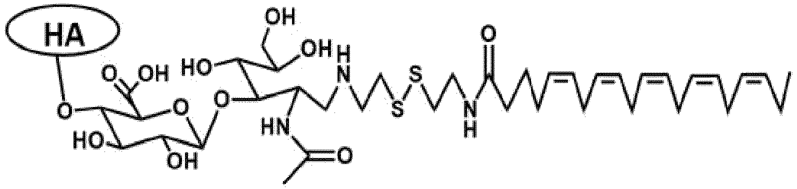

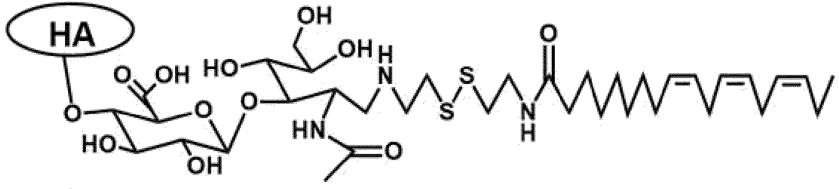

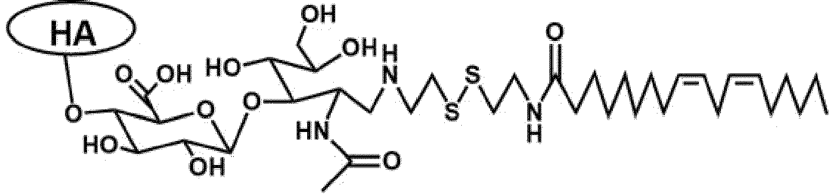

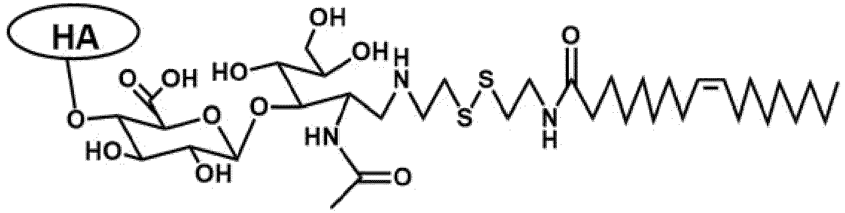

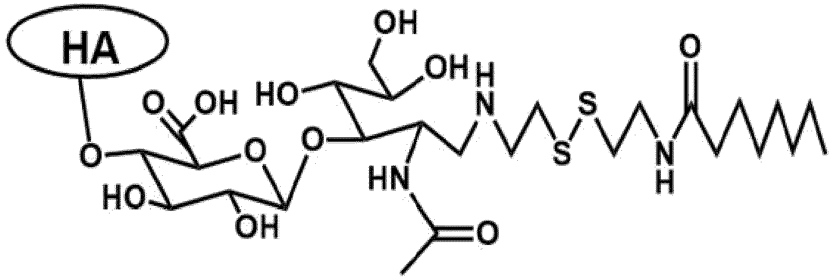

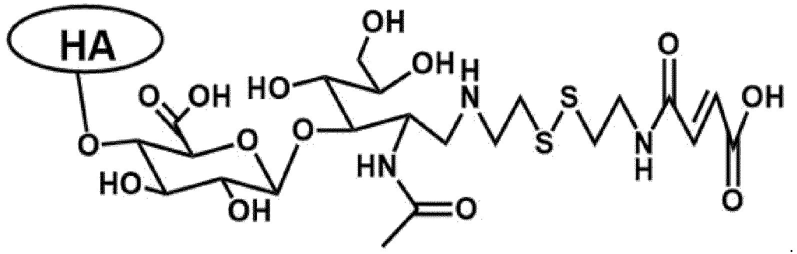

42. The water-soluble carbon chain substance according to the technical solution described above,
comprising a small molecule compound comprising a hydroxyl group and a benzene ring; preferably, one or two or more substances selected from a group consisting of:
flavone, isoflavone, anthocyanin, *Glycine max* (soybean) isoflavone, *Aloe vera* (aloe) emodin, *Vitis vinifera* (grape) seed extract, green tea flavone, naringenin, *Citrus limon* (lemon) flavone, baicalein, riboflavin, quercetin, graphite flavone, *Cinnamomum cassia* (cinnamon) flavone, *Buxus sinica* (buxolin) flavone, *Crataegus pinnatifida* (hawthorn) flavone, morin, luteolin, *Melilotus officinalis*(sweet clover) flavone, gypsum flavone, *Lonicera japonica* (honeysuckle) flavone, *Gentiana scabra Bunge* (gentian) flavone, *Platycodon grandiflorus* (platycodon) flavone, *Viola phillipina* (Chinese violet) flavone, *Perilla frutescens* (perilla) flavone, *Dendranthema morifolium* (chrysanthemum) flavone, artemisinin, *Cynanchum officinale* (red peony) flavone, *Salvia miltiorrhiza Bunge* (salvia) flavone, *Saposhnikovia divaricata* flavone, *Chelonopsis pseudobracteata* (safflower) flavone, *Rhodiola rosea* L.(rhodiola) flavone, *Ziziphus jujuba var. inermis* (jujube) flavone, *Lycium chinense Mill.* (wolfberry) flavone, prepared *Rehmannia glutinosa* (rehmannia) flavone, *Ganoderma lucidum* flavone, *Schisandra chinensis* (schizandrin) flavone, *Glycyrrhiza uralensis* (licorice) flavone, *Panax pseudoginseng* (notoginseng) flavone, curcumin, apigenin, carotene, anthocyanin, lutein, zeaxanthin, Sinapis alba L. flavone, Ruta graveolens L. flavone, genkwanin, pollen flavone, *Hippophae rhamnoides L.* (sea-buckthorn) flavone, *Calendula officinalis L.* (marigold) flavone, cinnamon flavone, isoflavonoids and anthocyanins; rutin, emodin, fucoxanthin, gallic acid, persimmon peel extract, morin, echinacoside, grapeseed procyanidin, phenolic acid, tea polyphenol, naringin, citric acid, flavonol, glycyrrhizic acid, cinnamic acid, flavone glycoside, silymarin, matrine, tanshinone, Mangrove bark extract, hippophaetic acid, perillyl alcohol, anisic acid, amurensin, hesperidin, punicalin, morchellin, naringin, onionoside(Cleistocalyx operculatus extract), gentiopicrin, jasmine, naringol, carotene, apigenin, *Vatica mangachapoi Blanco* (green plum) extract, *Folium mori* (mulberry leaf) extract, loniceroside, *Dendranthema morifolium* (chrysanthemumin) extract, *Canarium album* (olive) extract, Oolong tea extract, theanine, vin rouge essence, platycodin, perillyl alcohol glycoside, mulberry bark extract, carthaminol, matsuba enzyme, pachymic acid, caffeic acid, chlorogenic acid, resveratrol, white tea polyphenol, resveratrol disaccharide, *Musa basjoo* (banana) lutein, anthocyanidin, arachidonic acid, *Arachis hypogaea* (peanut) flavone, xanthotol, anethol, flavonoid, baicalein, flavonoid glycoside, flavanol, vin rouge polyphenol, rhodiol, carthaminol, black tea flavone, black sesamin, *Secale cereale* (rye) phenol, trehalose alcohol, seaweed polysaccharide, alginic acid, *Albizzia julibrissin* extract (albizarin), cannabidiol, polydatin, cucurbitacin, *Trigonella foenum-graecum* (Huluba) extract, cucurbic acid, pollen phenol, pollen flavone, pollen glycoside, pollen ester, arachidic acid, peanut flavone glycoside, peanut isoflavone, peanut isoflavone glycoside, peanut isoflavone disaccharide, peanut isoflavone trisaccharide, peanut resveratrol, peanut resveratrol disaccharide, peanut resveratrol trisaccharide, peanut resveratrol tetrasaccharide, peanut resveratrol pentasaccharide, peanut resveratrol hexasaccharide, peanut resveratrol heptasaccharide, peanut resveratrol octasaccharide, peanut resveratrol nonasaccharide, peanut resveratrol decasaccharide, peanut resveratrol undecasaccharide, catechin, epicatechin, tea polyphenol, catechol, chlorophyll, protocatechin, hesperidin, anthocyanin, cyanine glucoside, cyanine aglycone, cyanine alcohol, glucoside, glucose aglycone, alfalfa extract, soybean isoflavone, flavanol, quercitannin, *Fagopyrum tataricum* (buckwheat) extract, persimmon peel extract, persimmon tannin, punicic acid, punicalin, blueberry extract, resveratrol, lycopene, naringenin, morin, chlorogenic acid, chlorogenic acid triglycoside, chlorogenic acid diglucoside, methyl chlorogenate, ethyl chlorogenate, propyl chlorogenate, butyl chlorogenate, isoamyl chlorogenate, hexyl chlorogenate, octyl chlorogenate, benzyl chlorogenate, phenethyl chlorogenate, phenylpropyl chlorogenate, phenylbutyl chlorogenate, phenyl isoamyl chlorogenate, phenylhexyl chlorogenate, phenyloctyl chlorogenate, styrene chlorogenate, chlorogenic acid benzyl alcohol ester, chlorogenic acid phenethyl alcohol ester, anthocyanin, proanthocyanidin glycoside, and catechin.

43. The water-soluble carbon chain substance according to the technical solution described above, wherein the water-soluble carbon chain substance comprises a water-soluble medium-short chain fatty acid or a fatty acid salt or a fatty acid derivative, and preferably comprises one or two or more kinds of water-soluble medium-short chain fatty acid or a fatty acid salt or a fatty acid derivative selected from the group consisting of:
water-soluble medium-short chain fatty acids such as butyric acid, succinic acid, fumaric acid, pentanoic acid, glutaric acid, hexanoic acid, hexane diacid, heptanoic acid, heptanedioic acid, octoic acid, octenoic acid, octanedioic acid, decanoic acid, decanedioic acid, and the like; and any one or more **of** the following fatty acid derivatives including surfactants and the like: fatty acid salts, alkyl sulfonic acid salts, alkyl sulfuric acid ester salts, alkyl phosphoric acid ester salts, alkyl amine salts, alkyl quaternary ammonium salts, fatty acyl amino acids, betaines, fatty alcohol polyoxyethylene ethers, alkylphenol polyoxyethylene ethers, fatty amine polyoxyethylene ethers, polyol fatty acid esters, polyol polyoxyethylene ether fatty acid esters, fatty acid polyoxyethylene esters, alkyl glycosides, and alcohol ether glycosides.

44. A preparation for regulating transmembrane transport of cell membranes, for regulating structure or function of cell membrane, for regulating cell division proliferation or cell migration, for regulating cell senescence, and/or for regulating cell membrane fluidity manufactured by the water-soluble carbon chain substance according to the technical solution described above.

45. The preparation according to the technical solution described above, wherein the regulating cell membrane fluidity refers to increasing the fluidity of the cell membrane.

46. The preparation according to the technical solution described above, wherein the regulating cell membrane fluidity refers to decreasing the fluidity of the cell membrane.

47. The preparation according to the technical solution described above, wherein the cell membrane comprises a cytoplasmic membrane and an organelle membrane; the cell membrane or cell comprises a cell membrane or cell of a microorganism, or a cell membrane or cell of a plant, animal or human body tissue; the regulating comprises increasing or promoting, and inhibiting or decreasing, wherein the cell membranes of microorganisms include cell membranes of bacteria and fungi and envelopes of viruses;
wherein the cell membrane structure comprises a phospholipid bilayer, proteins and polysaccharides adsorbed on the surface of the phospholipid bilayer and embedded throughout the phospholipid bilayer, specifically, including receptor proteins, transmembrane proteins, cytoskeletal proteins, and enzymes; and
wherein, the transmembrane transport includes active transport, passive transport, and endocytosis.

48. The preparation according to the technical solution described above, wherein the regulating transmembrane transport of cell membranes comprises increasing and promoting the release of extracellular vesicles from inside cells to outside cells; also comprises inhibiting or reducing the release of extracellular vesicles from inside cells to outside cells; and can prevent the virus from entering the cell; the inhibiting or reducing the transmembrane transport of the virus refers to inhibiting or reducing the entry of the virus into the cell by endocytosis or inhibiting or reducing viral entry into the cell through fusion of the viral envelope with the cell membrane; it is applied to prevention of all viral infections, prevention of mycoplasma and chlamydial infections, prevention of bacterial infections; substances of transmembrane transport include: at least one or two or more selected from the group consisting of a small molecule compound, a medium molecule compound, a macromolecule compound, a pathogenic microorganism such as a virus, a bacterium, a mycoplasma, a chlamydia and a fungus, and a nanoparticle and a nano-drug.

49. The preparation according to the technical solution described above, wherein the preparation for increasing cell membrane fluidity comprises a preparation for preventing, blocking, ameliorating and/or treating drug delivery, gene transfection, cell therapy, diabetes, amelioration of the nervous system, Alzheimer's disease, enhancement of the immune system, leukemia, hypercholesterolemia, and/or inflammatory bowel disease.

50. The preparation according to the technical solution described above, wherein the preparation for reducing cell membrane fluidity comprises a preparation for preventing, blocking, ameliorating and/or treating cardiovascular diseases including coronary heart disease, hypertension, myocardial infarction, heart failure, obesity, autism, osteoporosis, inflammatory diseases, autoimmune diseases, chronic fatigue syndrome, cell autolysis, viral infection, and/or neurodegenerative diseases.

51. A preparation for preventing, blocking or treating microbial infections made using the water-soluble carbon chain substance according to the technical solution described above.

52. The preparation according to the technical solution described above, wherein the preparation pharmaceutical preparation or an environmental disinfection and sterilization preparation, and the pharmaceutical preparation is preferably one selected from the group consisting of an inhalant, a nasal spray, an injection, an oral preparation, and a transdermal topical formulation.

53. The use of the water-soluble carbon chain substance according to the technical solution described above in the preparation of a pharmaceutical preparation for preventing, blocking and/or treating microbial infections or environmental microbial disinfection and sterilization reagents, wherein preferably, the microorganism is any one or two selected from the group consisting of viruses, bacteria, and fungi; the virus is an enveloped virus and/or non enveloped viruses; more preferably, the virus is one or two or more viruses selected from the group consisting of novel coronavirus, influenza virus, AIDS virus (HIV), hepatitis B virus, human herpes virus, Ebola virus, rabies virus, and a human papilloma virus (HPV), and the bacteria is one or two or more bacteria selected from the group consisting of Escherichia coli, Staphylococcus aureus, methicillin-resistant Staphylococcus aureus, Streptococcus pneumoniae, Klebsiella pneumoniae and Pseudomonas aeruginosa; more further preferably, the virus is one or two of more selected from the group consisting of H7N9 influenza virus, H5N1 influenza virus, HIV virus, novel coronavirus, HPV virus, and rabies virus.

54. The use according to the technical solution described above, wherein the water-soluble carbon chain substance according to any one of technical solutions 59-99, when applied to a microorganism, is formulated to a solution having a concentration of 0.2 mM-10 mM.

55. The use according to the technical solution described above, wherein the water-soluble carbon chain substance is formulated to a solution having a concentration of 0.5 mM-10 mM.

56. A preparation for preventing, blocking or treating inflammatory response and/or body aging made using the water-soluble carbon chain substance according to the technical solution described above.

57. The preparation according to the technical solution described above, wherein the body aging comprises resisting skin aging, and the preventing, blocking, or treating the inflammatory response comprises altering the conformation of an inflammatory factor (protein).

58. The preparation according to the technical solution described above, wherein the carbon chain substance binds to an oxygen free radical.

59. The preparation according the technical solution described above, wherein the carbon chain substance binds to, or is, or mixed to obtain a preparation with, one or two or more selected from the group consisting of:
flavone, isoflavone, anthocyanin, *Glycine max* (soybean) isoflavone, *Aloe vera* (aloe) emodin, *Vitis vinifera* (grape) seed extract, green tea flavone, naringenin, *Citrus limon* (lemon) flavone, baicalein, riboflavin, quercetin, graphite flavone, *Cinnamomum cassia* (cinnamon) flavone, *Buxus sinica* (buxolin) flavone, *Crataegus pinnatifida* (hawthorn) flavone, morin, luteolin, *Melilotus officinalis*(sweet clover) flavone, gypsum flavone, *Lonicera japonica* (honeysuckle) flavone, *Gentiana scabra Bunge* (gentian) flavone, *Platycodon grandiflorus* (platycodon) flavone, *Viola phillipina* (Chinese violet) flavone, *Perilla frutescens* (perilla) flavone, *Dendranthema morifolium* (chrysanthemum) flavone, artemisinin, *Cynanchum officinale* (red peony) flavone, *Salvia miltiorrhiza Bunge* (salvia) flavone, *Saposhnikovia divaricata* flavone, *Chelonopsis pseudobracteata* (safflower) flavone, *Rhodiola rosea* L.(rhodiola) flavone, *Ziziphus jujuba var. inermis* (jujube) flavone, *Lycium chinense Mill.* (wolfberry) flavone, prepared *Rehmannia glutinosa* (rehmannia) flavone, *Ganoderma lucidum* flavone, *Schisandra chinensis* (schizandrin) flavone, *Glycyrrhiza uralensis* (licorice) flavone, *Panax pseudoginseng* (notoginseng) flavone, curcumin, apigenin, carotene, anthocyanin, lutein, zeaxanthin, Sinapis alba L. flavone, Ruta graveolens L. flavone, genkwanin, pollen flavone, *Hippophae rhamnoides L.* (sea-buckthorn) flavone, *Calendula officinalis L.* (marigold) flavone, cinnamon flavone, isoflavonoids and anthocyanins; rutin, emodin, fucoxanthin, gallic acid, persimmon peel extract, morin, echinacoside, grapeseed procyanidin, phenolic acid, tea polyphenol, naringin, citric acid, flavonol, glycyrrhizic acid, cinnamic acid, flavone glycoside, silymarin, matrine, tanshinone, Mangrove bark extract, hippophaetic acid, perillyl alcohol, anisic acid, amurensin, hesperidin, punicalin, morchellin, naringin, onionoside(Cleistocalyx operculatus extract), gentiopicrin, jasmine, naringol, carotene, apigenin, *Vatica mangachapoi Blanco* (green plum) extract, *Folium mori* (mulberry leaf) extract, loniceroside, *Dendranthema morifolium* (chrysanthemumin) extract, *Canarium album* (olive) extract, Oolong tea extract, theanine, vin rouge essence, platycodin, perillyl alcohol glycoside, mulberry bark extract, carthaminol, matsuba enzyme, pachymic acid, caffeic acid, chlorogenic acid, resveratrol, white tea polyphenol, resveratrol disaccharide, *Musa basjoo* (banana) lutein, anthocyanidin, arachidonic acid, *Arachis hypogaea* (peanut) flavone, xanthotol, anethol, flavonoid, baicalein, flavonoid glycoside, flavanol, vin rouge polyphenol, rhodiol, carthaminol, black tea flavone, black sesamin, *Secale cereale* (rye) phenol, trehalose alcohol, seaweed polysaccharide, alginic acid, *Albizzia julibrissin* extract (albizarin), cannabidiol, polydatin, cucurbitacin, *Trigonella foenum-graecum* (Huluba) extract, cucurbic acid, pollen phenol, pollen flavone, pollen glycoside, pollen ester, arachidic acid, peanut flavone glycoside, peanut isoflavone, peanut isoflavone glycoside, peanut isoflavone disaccharide, peanut isoflavone trisaccharide, peanut resveratrol, peanut resveratrol disaccharide, peanut resveratrol trisaccharide, peanut resveratrol tetrasaccharide, peanut resveratrol pentasaccharide, peanut resveratrol hexasaccharide, peanut resveratrol heptasaccharide, peanut resveratrol octasaccharide, peanut resveratrol nonasaccharide, peanut resveratrol decasaccharide, peanut resveratrol undecasaccharide, catechin, epicatechin, tea polyphenol, catechol, chlorophyll, protocatechin, hesperidin, anthocyanin, cyanine glucoside, cyanine aglycone, cyanine alcohol, glucoside, glucose aglycone, alfalfa extract, soybean isoflavone, flavanol, quercitannin, *Fagopyrum tataricum* (buckwheat) extract, persimmon peel extract, persimmon tannin, punicic acid, punicalin, blueberry extract, resveratrol, lycopene, naringenin, morin, chlorogenic acid, chlorogenic acid triglycoside, chlorogenic acid diglucoside, methyl chlorogenate, ethyl chlorogenate, propyl chlorogenate, butyl chlorogenate, isoamyl chlorogenate, hexyl chlorogenate, octyl chlorogenate, benzyl chlorogenate, phenethyl chlorogenate, phenylpropyl chlorogenate, phenylbutyl chlorogenate, phenyl isoamyl chlorogenate, phenylhexyl chlorogenate, phenyloctyl chlorogenate, styrene chlorogenate, chlorogenic acid benzyl alcohol ester, chlorogenic acid phenethyl alcohol ester, anthocyanin, proanthocyanidin glycoside, and catechin.

60. The preparation according to the technical solution described above for use in preventing, blocking or treating an inflammatory response, and/or body ageing, wherein the water-soluble carbon chain substance is formulated as a solution having a concentration of 0.1 nM-5 mM; preferably the carbon chain substance is a complex of a fatty acid and an amino acid, more preferably the concentration of the complex solution is 0.1 nM-300 uM, more preferably 5 nM-100 uM.

61. The preparation according to the technical solution described above for use in preventing, blocking or treating an inflammatory response, and/or body ageing, wherein the water-soluble carbon chain substance is formulated to a solution having a concentration of 0.1 nM-300 uM, preferably 5 nM-100 uM.

62. The pharmaceutical preparation for preventing, blocking, slowing down, or treating neurodegenerative diseases made from the water-soluble carbon chain substance according to the technical solution described above.

63. The pharmaceutical preparation according to the technical solution described above, wherein the neurodegenerative disease comprises Alzheimer's disease (AD), Parkinson's disease (PD), Huntington's disease (HD), senile dementia, amyotrophic lateral sclerosis (ALS), different types of spinocerebellar ataxia (SCA), or Pick's disease.

64. The pharmaceutical preparation of the technical solution described above, wherein the water-soluble carbon chain substance is formulated to function at a concentration of 0.1 nM-5 mM.

65. The pharmaceutical preparation according to the technical solution described above, wherein the water-soluble carbon chain substance is formulated to act as a solution having a concentration of 0.1 nM-300 uM, preferably 5 nM-100 uM.

The preparation of the carbon chain substance of the present invention for reducing the fluidity of cell membranes can be applied to the following aspects and diseases:
1) Cardiovascular diseases (including coronary heart disease, hypertension, myocardial infarction, heart failure): reducing membrane fluidity can reduce the risk of heart disease and stroke; moderate reduction of cell membrane fluidity can reduce cardiac load and relieve symptoms of cardiovascular diseases; moderate reduction in cell membrane fluidity can reduce the activity of sodium and calcium channels on cell membranes, thereby reducing the influx of intracellular calcium ions, reducing the excitability of myocardial cells and cardiac load, and alleviating the symptoms of cardiovascular diseases.
2) Obesity: reduction in cell membrane fluidity can improve fat metabolism, reduce fat storage, and help alleviate obesity symptoms.
3) Autism: reduction in cell membrane fluidity can improve the function of brain cells, thereby helping to alleviate the symptoms of autism.
4) Osteoporosis: reduction in cell membrane fluidity can promote the growth of bone cells and increase bone density, thereby preventing and treating osteoporosis.
5) Inflammatory diseases: reduction in cell membrane fluidity can reduce the inflammatory response and help control the symptoms of inflammatory diseases.
6) Autoimmune diseases: moderate reduction of membrane fluidity can reduce the autoimmune response and alleviate the symptoms of autoimmune diseases.
7) Chronic fatigue syndrome: reduction in cell membrane fluidity can alleviate fatigue symptoms.
8) Cell Autolysis: reduction in cell membrane fluidity can prevent the loss of intracellular substance, thereby preventing cell autolysis.
9) Neurodegenerative diseases: reduction in cell membrane fluidity can prevent the loss of useful substances from neurons, thereby preventing neurodegenerative diseases.
10) Prevention of viral infection, etc. reduction in cell membrane fluidity can reduce the likelihood of viral entry into cells and spread of tumor cells.

The preparation made of carbon chain substance of the present invention can increase cell membrane permeability by moderately improving cell membrane fluidity, thereby promoting substance crossing the cell membrane, and can be applied in the following aspects and diseases:
Drug delivery: drug molecules need to pass through the cell membrane to enter the interior of the cell, but due to the biological characteristics of the cell membrane, many drugs cannot cross the cell membrane. Thus, by increasing the cell membrane fluidity, the rate and efficiency of passage of drug molecules through the cell membrane can be increased, improving drug delivery.

Gene transfection: similarly, by increasing the cell membrane fluidity, the rate and efficiency of exogenous genes entering the cell interior through the membrane can be increased, thereby achieving gene transfection.

Cell therapy: some cell therapies require introducing exogenous cells into the host cell, and exogenous cells must pass through the host cell membrane to enter the host cell. Therefore, increasing the fluidity of the cell membrane can improve the efficiency of exogenous cells crossing the host cell membrane.

Tumor treatment: by increasing the cell membrane fluidity, the rate and efficiency of chemotherapeutic agents entering tumor cells through cell membranes can be increased, thereby increasing the efficacy of chemotherapeutic agents.

Diabetes: cell membrane fluidity is generally lower in diabetic patients than in healthy humans. By decreasing the membrane fluidity, insulin sensitivity can be increased, thereby helping to control blood glucose levels.

Improvement of neurological disorders: water-soluble fatty acid complexes can promote neuronal cell regeneration and improve the symptoms of neurological diseases by increasing cell membrane fluidity.

Alzheimer's disease: reduction in membrane fluidity may lead to neuronal cell death, and this can be reduced by increasing membrane fluidity.

Enhancement of the immune system: reduction in cell membrane fluidity may lead to decreased immune system function. By increasing the cell membrane fluidity, the function of the immune system can be enhanced, thereby helping to prevent infections and diseases.

Leukemia: leukemia is a cancer caused by abnormal proliferation of white blood cells. Water-soluble fatty acid complexes that enhance cell membrane fluidity may help chemotherapy drugs better enter the interior of white blood cells, thereby improving treatment efficacy.

Hypercholesterolemia: hypercholesterolemia can lead to vascular sclerosis and cardiovascular disease, and water-soluble fatty acid complexes increase cell membrane fluidity, thereby helping to lower cholesterol levels in the blood.

Inflammatory bowel disease: inflammatory bowel disease is a chronic inflammatory bowel disease, including Crohn's disease and ulcerative colitis. Some studies have shown that water-soluble fatty acid complexes can improve the fluidity of intestinal epithelial cell membrane, thereby reducing intestinal inflammation.

Cell membrane permeability can be increased by moderately improving cell membrane fluidity, thereby promoting substance crossing the cell membrane, and can be applied in the following aspects and diseases:
Drug delivery: drug molecules need to pass through the cell membrane to enter the interior of the cell, but due to the biological characteristics of the cell membrane, many drugs cannot cross the cell membrane. Thus, by increasing the cell membrane fluidity, the rate and efficiency of passage of drug molecules through the cell membrane can be increased, improving drug delivery.

Gene transfection: similarly, by increasing the cell membrane fluidity, the rate and efficiency of exogenous genes entering the cell interior through the membrane can be increased, thereby achieving gene transfection.

Cell therapy: some cell therapies require introducing exogenous cells into the host cell, and exogenous cells must pass through the host cell membrane to enter the host cell. Therefore, increasing the fluidity of the cell membrane can improve the efficiency of exogenous cells crossing the host cell membrane.

Tumor treatment: by increasing the cell membrane fluidity, the rate and efficiency of chemotherapeutic agents entering tumor cells through cell membranes can be increased, thereby increasing the efficacy of chemotherapeutic agents.

Diabetes: cell membrane fluidity is generally lower in diabetic patients than in healthy humans. By decreasing the membrane fluidity, insulin sensitivity can be increased, thereby helping to control blood glucose levels.

Improvement of neurological disorders: water-soluble fatty acid complexes can promote neuronal cell regeneration and improve the symptoms of neurological diseases by increasing cell membrane fluidity.

Alzheimer's disease: reduction in membrane fluidity may lead to neuronal cell death, and this can be reduced by increasing membrane fluidity.

Enhancement of the immune system: reduction in cell membrane fluidity may lead to decreased immune system function. By increasing the cell membrane fluidity, the function of the immune system can be enhanced, thereby helping to prevent infections and diseases.

Leukemia: leukemia is a cancer caused by abnormal proliferation of white blood cells. Water-soluble fatty acid complexes that enhance cell membrane fluidity may help chemotherapy drugs better enter the interior of white blood cells, thereby improving treatment efficacy.

Hypercholesterolemia: hypercholesterolemia can lead to vascular sclerosis and cardiovascular disease, and water-soluble fatty acid complexes increase cell membrane fluidity, thereby helping to lower cholesterol levels in the blood.

Inflammatory bowel disease: inflammatory bowel disease is a chronic inflammatory bowel disease, including Crohn's disease and ulcerative colitis.

The water-soluble carbon chain substance comprises a carbon chain which originally has a certain water solubility, and the structure thereof comprises at least one or more hydrophobic carbon chain parts and one or more hydrophilic groups or residues; and also comprises a high-hydrophilicity complex formed by coupling a hydrophobic carbon chain part to a water-soluble molecule; the water-soluble carbon chain can be used for preventing and treating infection including viruses, bacteria and fungi, anti-ageing, and preventing and reducing the occurrence of neurodegenerative diseases such as Alzheimer's disease; by reducing the non-specific phagocytosis of the nano-drug by cells, the effect of the nano-drug on target lesions is improved.

The present invention preliminarily demonstrates through experiments that the control of the concentration range of the water-soluble fatty acid complex as a water-soluble carbon chain substance can be carried out by referring to the following concentration and mechanism explanation.
(1) Concentration range of water-soluble fatty acid complex to cells: 0.1 nm-5 mM, preferably 1 nM-0.3 mM, more preferably in the range of 5 nM-0.1 mM; one skilled in the art can refer to this range of experimental concentrations to understand the range of plasma concentrations at which an animal or human would function.
(2) Concentration range for inhibition of cell membrane fluidity: the range of action of each water-soluble fatty acid complex is different from that of another water-soluble fatty acid complex. For most water-soluble fatty acid complexes, cell membrane fluidity can be inhibited at a concentration of 0.1 nm-30 mmolar. Decreased cell membrane fluidity can be associated with: 1). Hypertension: the reduction in cell membrane fluidity in hypertensive patients can be associated with vascular endothelial dysfunction. 2). Diabetes: the reduction in cell membrane fluidity in diabetic patients can be associated with oxidative stress caused by hyperglycemia and the production of glycosylation endproducts. 3). Heart disease: the reduction in cell membrane fluidity in patients with heart disease can be associated with the structural and functional dysfunctions of myocardial cell membranes. 4). Autoimmune diseases: the reduction in cell membrane fluidity in patients with autoimmune diseases can be associated with aberrant activation of the immune system and inflammatory responses. The above diseases can be treated by moderately increasing the fluidity of cell membranes.
(3) When cell membrane fluidity decreases: transmembrane transport of substances is limited and inhibited, including transmembrane transport of large and small molecules and particulate-type substances.
(4) Concentration range to increase cell membrane fluidity: the range of action of each water-soluble fatty acid complex is different from that of another water-soluble fatty acid complex. Generally, at a concentration of 50 micromolar to 500 micromolar, cell membrane fluidity is increased.
(5) Physiological and pathological processes corresponding to enhanced cell membrane fluidity include: when the cell membrane fluidity is increased, the cells are easier to divide and proliferate. One of the reasons is that the cell membrane fluidity is higher than that of normal cells, and the cell deformation and migration are also closely related to the cell membrane fluidity, such as the chemotaxis of leukocytes is also the reason for the high fluidity.
   The ability of leukocytes to chemotaxis to the inflamed area is also reduced.
(6) When the fluidity of the cell membrane increases to a certain extent and the cell membrane changes from liquid crystal to liquid state, the cell membrane ruptures, and the concentration of water-soluble fatty acids at this time is generally above 500 micromoles.
(7) With regard to the concentration range in which the water-soluble fatty acid complex affects the transmembrane transport of a substance: all water-soluble fatty acid complexes affect the transmembrane transport of membrane substances, either increasing transmembrane transport or inhibiting transmembrane transport.
   For example: when a virus enters a cell, it belongs to transmembrane transport; Insulin secreted into the extracellular space through exosomes also belongs to transmembrane transport.
(8) One of the important reasons that currently block the widespread use of nano-drugs is that when injected into the body, these nano-drugs are phagocytosed by the body's innate immune-related phagocytic and reticuloendothelial systems, which is a protective mechanism. This results in insufficient nano-drug entering the target lesion area. Water-soluble fatty acid complexes inhibit non-specific endocytosis of nano-drug by the reticuloendothelial system, allowing more nano-drug to enter the target lesion.
(9) The concentration range for inhibiting aging is between 5 nm and 100 uM, while the concentration range for killing microorganisms is between 0.2 mM and10 mM.

Compared with the prior art, the present invention has the beneficial effects as follows:
1) The group of water-soluble carbon chain substances involved in the present invention affects the transmembrane transport of substances, cell division and proliferation, cell movement and migration, and cell senescence by affecting the fluidity of the cell membrane;
2) A group of water-soluble carbon-chain substances according to the present invention can increase the fluidity of the cell membrane. When the fluidity of the cell membrane increases to a certain extent and it transitions from a liquid crystal state to a liquid state, the cell membrane ruptures. Then, lipid envelopes of the pathogenic microorganisms including enveloped viruses, bacteria, and fungi can be disrupted. It can be used for the treatment of infections by pathogenic microorganisms including enveloped viruses, bacteria and fungi.
3) A group of water-soluble carbon-chain substances according to the present invention affect the fluidity of cell membranes by altering their composition, function, and structure.
4) A group of water-soluble carbon-chain substances according to the present invention can bind to proteins in the phospholipid bilayer of cell membrane at a certain concentration range, change their conformation, thereby affecting the protein deformability and the movement thereof in the phospholipid bilayer, resulting in a reduction in the fluidity of cell membrane.
5) A group of water-soluble carbon chain substances involved in the present invention can bind to endocytosis-related proteins, receptor proteins and scaffold proteins of cell membrane phospholipid bilayer in a certain concentration range, change their conformation, and inhibit the protein deformation ability, thereby inhibiting the transmembrane transport properties of the substances. For example, endocytosis of viruses and nano-drugs into cells can be prevented. This can prevent viral infection, inhibit non-specific phagocytosis of nano-drugs, and enhance their ability to act on target lesions.
6) A group of water-soluble carbon chain substances involved in the present invention can bind to endocytosis-related proteins, receptor proteins and scaffold proteins of cell membrane phospholipid bilayer in a certain concentration range, change their conformation, and inhibit the protein deformation ability, thereby inhibiting the transmembrane transport properties of the substances. For example, endocytosis of bacterial and fungal into cells can be prevented. This prevents bacterial and fungal infections.
7) A group of water-soluble carbon chain substances according to the present invention can prevent viral infection by acting on host cells; viral infection can be prevented without destroying the virus in the host.
8) A group of water-soluble carbon chain substances involved in the present invention can inhibit the protein deformation ability by acting on the skeleton protein in a certain concentration range to change its conformation; further inhibit cell movement and deformation ability. It can be used for inflammatory response syndrome caused by leukocyte migration in severe inflammation.
9) A group of water-soluble carbon chain substances according to the present invention inhibit the cell movement and deformation ability by reducing the fluidity of cell membrane in a certain concentration range; it can be used for inflammatory response syndrome caused by leukocyte migration in severe inflammation.
10) A group of water-soluble carbon chain substances according to the present invention inhibit cell senescence by improving the fluidity of cell membrane in a certain concentration range; it can be used against body aging and against skin aging.
11) A group of water-soluble carbon chain substances according to the present invention have anti-inflammatory and anti-aging effects by changing the conformation of inflammatory factors (proteins) in combination with oxygen free radicals, thereby inhibiting the inflammatory reaction. On the other hand, a group of water-soluble carbon-chain substances can stabilize the cell membrane by reducing the fluidity of the cell membrane in a certain range of concentration, and inhibit the cell membrane rupture under the action of inflammatory factors, resulting in more inflammatory factors released in the cell, thereby inhibiting the occurrence of inflammation.

### Brief Description of the Drawings

Fig. 1 is a graph showing the comparison of the infrared spectra of linolenic acid-serum albumin prepared in Example 1;
Fig. 2 shows Coomassie brilliant blue staining results of fumaric acid, linolenic acid, eicosapentaenoic acid, and docosahexaenoic acid-serum albumin prepared in Example 1;
Fig. 3A is a graph showing mass spectrometry analysis of linolenic acid-serum albumin prepared in Example 1;
Fig. 3B is a graph showing the site analysis of the linolenic acid modified albumin prepared in Example 1;
Fig. 4A is a graph showing the mass spectrometry analysis of docosahexaenoic acid-serum albumin prepared in Example 1;
Fig. 4B is a graph showing the site analysis of docosahexaenoic acid modified albumin prepared in Example 1;
Fig. 5 is a graph showing mass spectrometry of oleic acid-serum albumin prepared in Example 2;
Fig. 6 is a graph showing the site analysis of oleic acid modified serum albumin in the compound prepared in Example 2;
Fig. 7 is a graph showing the comparison of infrared spectra of eicosapentaenoic acid-serum albumin prepared in Example 3;
Fig. 8 is a graph showing the mass spectrometry of eicosapentaenoic acid-serum albumin prepared in Example 4;
Fig. 9 is a graph showing the site analysis of eicosapentaenoic acid modified serum albumin in the compound prepared in Example 4;
Fig. 10 is a graph showing the mass spectrometry of linoleic acid-serum albumin prepared in Example 5;
Fig. 11 is a graph showing the site analysis of linoleic acid modified serum albumin in the compound prepared in Example 5;
Fig. 12 is a graph showing the comparison of the infrared spectra of docosahexaenoic acid-serum albumin prepared in Example 6;
Fig. 13 is a graph showing the mass spectrum of docosahexaenoic acid-serum albumin prepared in Example 6;
Fig. 14 is a graph showing the site analysis of docosahexaenoic acid modified serum albumin in the compound prepared in Example 6;
Fig. 15 is a graph showing the comparison of the infrared spectra of linoleic acid-hyaluronic acid prepared in Example 7;
Fig. 16 is a graph showing the comparison of the infrared spectrum of docosahexaenoic acid-hyaluronic acid prepared in Example 8;
Fig. 17 is a graph showing the comparison of infrared spectra of fatty acid-SBP1 prepared in Example 9;
Fig. 18 is a graph showing the comparison of infrared spectra of 9-tetradecenoic acid-SBP1 prepared in Example 10;
Fig. 19 is a graph showing the infrared spectrum of a saturated carbon chain with 8 carbon atoms-glucose complex prepared in Example 14;
Fig. 20 is a graph showing the inhibition results for the saturated carbon chain with 8 carbon atoms-glucose complex prepared in Example 14;
Fig. 21 is a graph showing the infrared spectrum of the saturated carbon chain with 8 carbon atoms-sucrose complex prepared in Example 15;
Fig. 22 is a graph showing the inhibition results for the saturated carbon chain with 8 carbon atoms-sucrose complex prepared in Example 15;
Fig. 23 is a graph showing the infrared spectrum of the fatty acid-adenosine monophosphate complex prepared in Example 16;
Fig. 24 is a graph showing the inhibition results for the saturated carbon chain with 8 carbon atoms-adenosine monophosphate complex prepared in Example 16;
Fig. 25 is a graph showing the infrared spectrum of the saturated carbon chain with 8 carbon atoms-ascorbic acid complex prepared in Example 17;
Fig. 26 is a graph showing the inhibition results of the saturated carbon chain with 8 carbon atoms-ascorbic acid complex prepared in Example 17;
Fig. 27 a graph showing the infrared spectrum of the saturated carbon chain with 8 carbon atoms - polyethylene glycol 400-COOH complex prepared in Example 18;
Fig. 28 is a graph showing the inhibition results for the saturated carbon chain with 8 carbon atoms-polyethylene glycol 400-COOH complex prepared in Example 18;
Fig. 29 is an image under a transmission electron microscope of the ethyl oleate liposome prepared in (1) of Example 19;
Fig. 30 is an image under a transmission electron microscope of the linoleic acid liposome obtained in (2) of Example 19;
Fig. 31 shows an injection of linolenic acid-serum albumin prepared in Example 20;
Fig. 32 shows the particle size distribution measured by a Malvern Mastersizer of linolenic acid-serum albumin prepared in Example 20;
Fig. 33 is an image under a transmission electron microscope of the linolenic acid-serum albumin prepared in Example 20;
Fig. 34 shows a lyophilized powder injection of linoleic acid-hyaluronic acid prepared in Example 21;
Fig. 35 shows a lyophilized powder injection of docosahexaenoic acid-hyaluronic acid prepared in Example 21;
Fig. 36 is an image under a transmission electron microscope of linoleic acid-hyaluronic acid lyophilized powder prepared in Example 21 after reconstitution in water;
Fig. 37 shows the particle size distribution measured by a Malvern Mastersizer of the linoleic acid-hyaluronic acid lyophilized powder prepared in Example 21 after reconstitution in water;
Fig. 38 shows a dodecanoic acid aspartic acid complex liposome lyophilized powder preparation prepared in Example 22;
Fig. 39 is a scanning electron micrograph of the dodecanoic acid aspartic acid complex liposome lyophilized powder preparation prepared in Example 22;
Fig. 40 is a graph showing the particle size distribution of the dodecanoic acid aspartic acid complex liposome lyophilized powder preparation prepared in Example 22 after reconstitution in water;
Fig. 41 is a graph showing the potential distribution of the dodecanoic acid aspartic acid complex liposome lyophilized powder preparation prepared in Example 22;
Fig. 42 is a transmission electron micrograph of ethyl eicosapentaenoate injection prepared in Example 23;
Fig. 43 is a graph showing the particle size distribution of ethyl eicosapentaenoate injection prepared in Example 23;
Fig. 44 is a graph showing the potential distribution of ethyl eicosapentaenoate injection prepared in Example 23;
Fig. 45 is an image under a transmission electron micrograph of docosahexaenoic acid-SBP1 prepared in Example 24;
Fig. 46 is graph showing the particle size distribution measured by a Malvern Mastersizer of the docosahexaenoic acid-SBP1 prepared in Example 24;
Fig. 47 shows the product image of the peptide SBP1 grafted with different ω-3 fatty acids (ALA: linolenic acid, EPA: eicosapentaenoic acid, and DHA: docosahexaenoic acid) prepared in Example 24;
Fig. 48 is a transmission electron micrograph of a reconstituted CD14 protein-grafted dodecenoic acid lyophilized powder injection prepared in Example 25;
Fig. 49 is a transmission electron micrograph of the reconstituted CD14 protein-grafted tetradecenoic acid lyophilized powder injection prepared in Example 25;
Fig. 50 is a transmission electron micrograph of the reconstituted CD14 protein-grafted eicosapentaenoic acid lyophilized powder injection prepared in Example 25;
Fig. 51 is a graph showing the result of VERO E6 cell safety test for the saturated carbon chain with 8 carbon atoms-threonine prepared in Example 29;
Fig. 52 is a graph showing the result of VERO E6 cell safety test for the saturated carbon chain with 8 carbon atoms-serine prepared in Example 29;
Fig. 53 is a graph showing the result of VERO E6 cell safety test for the monounsaturated carbon chain with 18 carbon atoms-serine prepared in Example 29;
Fig. 54 is a graph showing the result of VERO E6 cell safety test for the monounsaturated carbon chain with 18 carbon atoms-threonine prepared in Example 29;
Fig. 55 is a graph showing the result of VERO E6 cell safety test for the polyunsaturated carbon chain with 22 carbon atoms-threonine prepared in Example 29;
Fig. 56 is a graph showing the result of VERO E6 cell safety test for the polyunsaturated carbon chain with 22 carbon atoms-serine prepared in Example 29;
Fig. 57 is a graph showing the result of VERO E6 cell safety test for the monosaturated carbon chain with 18 carbon atoms-lysine prepared in Example 29;
Fig. 58 is a graph showing the result of VERO E6 cell safety test for the polysaturated carbon chain with 22 carbon atoms-lysine prepared in Example 29;
Fig. 59 is a graph showing the result of VERO E6 cell safety test for the polyunsaturated carbon chain with 18 carbon atoms-threonine prepared in Example 29;
Fig. 60 is a graph showing the result of VERO E6 cell safety test for the saturated carbon chain with 8 carbon atoms-adenosine 5'-monophosphate-unsaturated carbon chain with 4 carbon atoms-carboxyl group prepared in Example 29;
Fig. 61 is a graph showing the result of VERO E6 cell safety test for N-octyl-N-methylglucamine prepared in Example 29;
Fig. 62 is a graph showing the result of VERO E6 cell safety test for N-nonyl-N-methylglucamine prepared in Example 29;
Fig. 63 is a graph showing the inhibition results of the saturated carbon chain with 8 carbon atoms-threonine prepared in Example 30 against *Staphylococcus aureus;*
Fig. 64 is a graph showing the inhibition results of the saturated carbon chain with 8 carbon atoms-serine prepared in Example 30 against *Staphylococcus aureus;*
Fig. 65 is a graph showing the inhibition results of the monounsaturated carbon chain with 18 carbon atoms-serine prepared in Example 30 against *Staphylococcus aureus;*
Fig. 66 is a graph showing the inhibition results of the monounsaturated carbon chain with 18 carbon atoms-threonine prepared in Example 30 against *Staphylococcus aureus;*
Fig. 67 is a graph showing the inhibition results of the polyunsaturated carbon chain with 22 carbon atoms-threonine prepared in Example 30 against *Staphylococcus aureus;*
Fig. 68 is a graph showing the inhibition results of the polyunsaturated carbon chain with 22 carbon atoms-serine prepared in Example 30 against *Staphylococcus aureus;*
Fig. 69 is a graph showing the inhibition results of the monounsaturated carbon chain with 18 carbon atoms-lysine prepared in Example 30 against *Staphylococcus aureus;*
Fig. 70 is a graph showing the inhibition results of the polyunsaturated carbon chain with 22 carbon atoms-lysine prepared in Example 30 against *Staphylococcus aureus;*
Fig. 71 is a graph showing the inhibition results of the polyunsaturated carbon chain with 18 carbon atoms-threonine prepared in Example 30 against *Staphylococcus aureus;*
Fig. 72 is a graph showing the inhibition results of the saturated carbon chain with 8 carbon atoms-adenosine 5'-monophosphate-unsaturated carbon chain with 4 carbon atoms-carboxyl prepared in Example 30 against *Staphylococcus aureus;*
Fig. 73 is a graph showing the inhibition results of N-octyl-N-methylglucamine in Example 30 against *Staphylococcus aureus;*
Fig. 74 is a graph showing the inhibition results of N-nonyl-N-methylglucamine in Example 30 against *Staphylococcus aureus;*
Fig. 75 shows the results of the VERO E6 cytotoxicity assay of the ω-3 fatty acid-serum albumin complex prepared in Example 32;
Fig. 76 shows the results of the cytotoxicity of the fatty acid-serum albumin complex prepared in Example 33 against hepatocytes;
Fig. 77 shows the cytotoxicity assay of carboxy-unsaturated carbon chain with 8 carbon atoms-taurocholic acid prepared in Example 34 against VERO E6 cells;
Fig. 78 is a graph showing the results of liver and kidney function in the animal safety test in (1) of Example 35;
Fig. 79 is a graph showing the results of liver and kidney function in the animal safety test in (2) of Example 35;
Fig. 80 is a graph showing the results of liver and kidney function in the animal safety test in (3) of Example 35;
Fig. 81 is a graph showing the results of liver and kidney function in the animal safety test in (4) of Example 35;
Fig. 82 is a graph showing the results of liver and kidney function in the animal safety test in (5) of Example 35;
Fig. 83 is a graph showing the results of hemolysis test in the animal safety test in (6) of Example 35;
Fig. 84 shows the neutralization inhibition rate of the fatty acid (ω-3 fatty acid)-serum albumin complex of Example 36 against SARS-CoV-2 pseudovirus;
Fig. 85 shows the neutralization inhibition of hexacosenoic acid-cyclodextrin inclusion of Example 37 against rabies pseudovirus;
Fig. 86 shows the neutralization inhibition rate of dotriacontahexaenoic acid-SBP1 complex of Example 38 against SARS-CoV-2 pseudovirus;
Fig. 87 shows the neutralization inhibition rate of the hexanoic acid-hyaluronic acid complex of Example 39 against the HIV pseudovirus HIV 18A-41;
Fig. 88 shows the neutralization inhibition rate of n-nonanoic acid-hyaluronic acid complex of Example 40 against influenza pseudovirus H7N9-Fluc;
Fig. 89 shows the neutralization inhibition rate of octadecanoic acid-serum albumin complex of Example 41 aginst HIV pseudovirus;
Fig. 90 shows the neutralization inhibition rate of the eicosanoid-hyaluronic acid complex of Example 42 against H7N9-Fluc pseudovirus;
Fig. 91 shows the neutralization inhibition rate of octacosanoic acid-serum albumin complex of Example 43 against HSN1-Fluc pseudovirus;
Fig. 92 shows the results of transfection of HIV -derived lentiviruses with hepatocytes from Example 44 pretreated with fatty acid-serum albumin complex;
Fig. 93 is a transmission electron micrograph of the SARS-CoV-2 pseudovirus of Example 45 after treatment;
Fig. 94 is a graphical representation of the hydrophobic sequestration process of the "carbon chain acting moiety + small molecule water-soluble moiety/binding moiety" complex of Example 46 on human papilloma virus;
Fig. 95 is an illustration of the in vitro simulation of N-octyl-N-methylglucamine encapsulation of protein particles loaded with L1 protein in Example 46;
Fig. 96 shows the neutralization inhibition of docosahexaenoic acid-coupled serine in Example 47 against HPV pseudovirus;
Fig. 97 is a graph of showing the inhibition (methicillin-resistant *Staphylococcus aureus*) results for various concentrations of the docosahexaenoic acid-serum albumin complex prepared in Example 48;
Fig. 98 is a graph of showing the inhibition ( *Escherichia coli)* results for various concentrations of the docosahexaenoic acid-serum albumin complex prepared in Example 48;
Fig. 99 shows the structural changes of *Escherichia coli* observed under scanning electron microscopy after treatment with the docosahexaenoic acid-serum albumin complex prepared in Example 48;
Fig. 100 shows the structural changes of *Staphylococcus aureus* observed under scanning electron microscopy after treatment with the docosahexaenoic acid-serum albumin complex prepared in Example 48;
Fig. 101 shows the phenomenon of membrane detachment of *Staphylococcus aureus* observed under transmission electron microscopy after the treatment with the docosahexaenoic acid serum albumin complex prepared in Example 48;
Fig. 102 is a graph showing the comparison of the binding rates of docosahexaenoic acid-serum albumin complex by the SARS-CoV-2 pseudovirus, *Staphylococcus aureus, Escherichia coli,* and hepatic stellate cells of Example 49;
Fig. 103 is a graph showing the residence time in the lung of the docosahexaenoic acid-serum albumin complex of Example 50;
Fig. 104 is a graph showing the retention time in the lung of the eicosapentaenoic acid-hyaluronic acid complex of Example 50;
Fig. 105 is a graph showing the results of an animal experiment of docosahexaenoic acid-serum albumin complex of Example 51;
Fig. 106 is a graph showing the results of an animal pulmonary administration experiment of the eicosapentaenoic acid-hyaluronic acid complex of Example 51;
Fig. 107 is a graph showing the results of lung fluorescence in the animal experiment of oral administration of small molecule complex in Example 52;
Fig. 108 a graph showing the analysis results of the average lung fluorescence value ImageJ in the animal experiment of oral administration of small molecule complex in Example 52;
Fig. 109, panel A, shows the results of binding sites of interaction between Ser-EPA and Caveolin-1 in Example II-1.

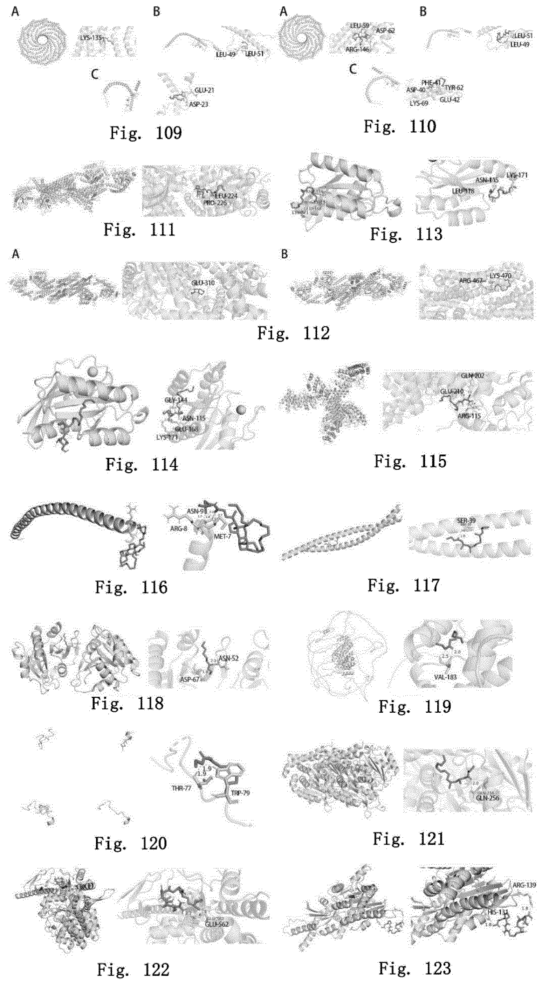

Fig. 109, panel B, shows the results of binding sites of interaction between Ser-EPA and Caveolin-2 in Example II-1.
Fig. 109, panel C, shows the results of binding sites of interaction between Ser-EPA and Caveolin-3 in Example II-1.
Fig. 110, panel A, shows the results of binding sites of interaction GA-DHA and Caveolin-1 in Example II-2.
Fig. 110, panel B, shows the results of binding sites of interaction GA-DHA and Caveolin-2 in Example II-2.
Fig. 110, panel C, shows the results of binding sites of interaction between GA-DHA and Caveolin-3 in Example II-2.
Fig. 111 shows the results of binding sites of interaction between GA-DHA and Dynamin in Example II-3.
Fig. 112, panel A, shows the results of binding sites of interaction between Ser-EPA and Dynamin in Example II-4.
Fig. 112, panel B, shows the results of binding sites of interaction between Ser-EPA and Dynamin in Example II-4.
Fig. 113 shows the results of binding sites of interaction between Ser-EPA and Rab protein in Example II-5.
Fig. 114 shows the results of binding sites of interaction between GA-DHA and Rab protein in Example II-6.
Fig. 115 shows the results of binding sites of interaction between Ser-EPA and Trpv1 (transient receptor cation channel) in Example II-7.
Fig. 116 shows the results of binding sites of interaction between cholyltaurine-C14 and SNAP-25 (synaptosome-associated protein-25) in Example II-8.
Fig. 117 shows the results of binding sites of interaction between PEG-C18-2 and SNAP-25 (synaptosome-associated protein-25) in Example II-9.
Fig. 118 shows the results of binding sites of interaction between PEG-C7 and Arf (ADP ribosylation factor) in Example II-10.
Fig. 119 shows the results of binding sites of interaction between PEG-C9 and AP180 (bridging protein) in Examples II-11.
Fig. 120 shows the results of binding sites of interaction between PEG-C11 and α-actin in Examples II-12.
Fig. 121 shows the results of binding sites of interaction between PEG-C18 and α-tubulin in Examples II-13.
Fig. 122 shows the results of binding sites of interaction between RGD-2-OH-PA and Muscle Myosin in Example II-14.
Fig. 123 shows the results of binding sites of interaction between RGD-DA and Septin protein in Example II-15.

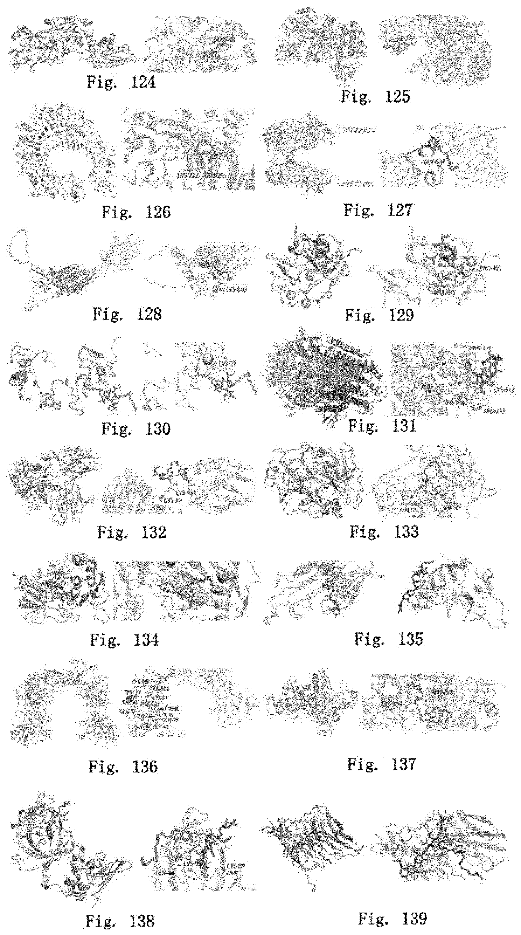

Fig. 124 shows the results of binding sites of interaction between RGD-FA and CD36 protein in Example II-16.
Fig. 125 shows the results of binding sites of interaction between RGD-C8 and TfR1 (transferrin receptor 1) in Example II-17.
Fig. 126 shows the results of binding sites of interaction between Thr-BA and a TLR1 receptor in Example II-18.
Fig. 127 shows the results of binding sites of interaction between Vitamin-C-OH and a TLR7 receptor in Example II-19.
Fig. 128 shows the results of binding sites of interaction between THr-C24 and a G protein-coupled receptor in Example II-20.
Fig. 129 shows the results of binding sites of interaction between Chondroitin sulfate-2-C8 and a Scavenger receptor in Example II-21.
Fig. 130 shows the results of binding sites of interaction between Heparin-SA and a VLDL receptor in Example II-22.
Fig. 131 shows the results of binding sites of interaction between UDPGA-BA and a chloride channel receptor in Example II-23.
Fig. 132 shows the results of binding sites of interaction between UDPGA-C12 and an Endoglin receptor in Example II-24.
Fig. 133 shows the results of binding sites of interaction between Thr-n-CA and a CD44 receptor in Example II-25.
Fig. 134 shows the results of binding sites of interaction between UDPGA-C25 and cathepsin in Example II-26.
Fig. 135 shows the results of binding sites of interaction between UDPGA-PA and TNF (tumor necrosis factor) in Example II-27.
Fig. 136 shows the results of binding sites of interaction between UDPGA-OA and VEGF (angiogenic factor) in Example II-28.
Fig. 137 shows the results of binding sites of interaction between Thr-C36 and Glutathione S-transferases (GSTs) in Example II-29.
Fig. 138 shows the results of binding sites of interaction between HA-OH-1 and a protease inhibitor (TIMPs) in Example II-30.
Fig. 139 shows the results of binding sites of interaction between HA-SA and a protease inhibitor (α2-macroglobulin) in Example II-31.

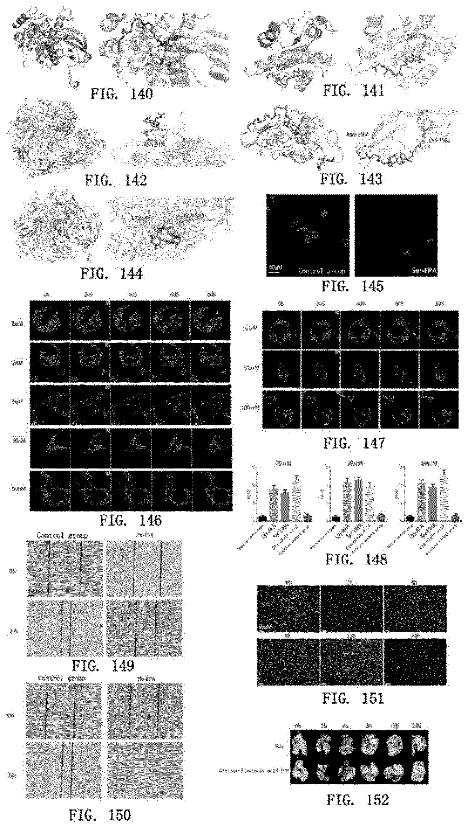

Fig. 140 shows the results of binding sites of interaction between Vitamin-C-C30 and tyrosine kinase receptors (TKRs) in Example II-32.
Fig. 141 shows the results of binding sites of interaction between fat-soluble vitamins and Toll-like receptors (TLRs) in Example II-33.
Fig. 142 shows the results of binding sites of interaction between Adenosine-C22-1 and Integrins in Example II-34.
Fig. 143 shows the results of binding sites of interaction between cholylglycine-C18-1-OH and Fibrillin in Example II-35.
Fig. 144 shows the results of binding sites of interaction between UDPGA-C12 and Collagen in Example II-36.
Fig. 145 shows the results of inhibiting the phagocytosis of nano-drug by Ser-EPA in Example II-37.
Fig. 146 is a graph showing the results of inhibiting cell membrane fluidity by the water-soluble fatty acid complex Thr-DHA in Example II-40.
Fig. 147 is a graph shows the results of promoting cell membrane fluidity by water-soluble fatty acid complex Thr-DHA in Example II-41.
Fig. 148 shows the results of promoting human hair follicle stem cell proliferation by Lys-ALA, Ser-DHA, and Glu-oleic acid in Example II-42.
Fig. 149 shows the results of inhibiting cell migration by Thr-EPA in Example II-43.
Fig. 150 shows the results of promoting cell migration by Thr-EPA in Example II-44.
Fig. 151 is a graph showing the result of the action of the water-soluble fatty acid complex with the cell membrane in Example II-52.
Fig. 152 is a graph showing the results of the interaction between the water-soluble fatty acid complex and the alveolar epithelial cell membrane in Examples II-53.

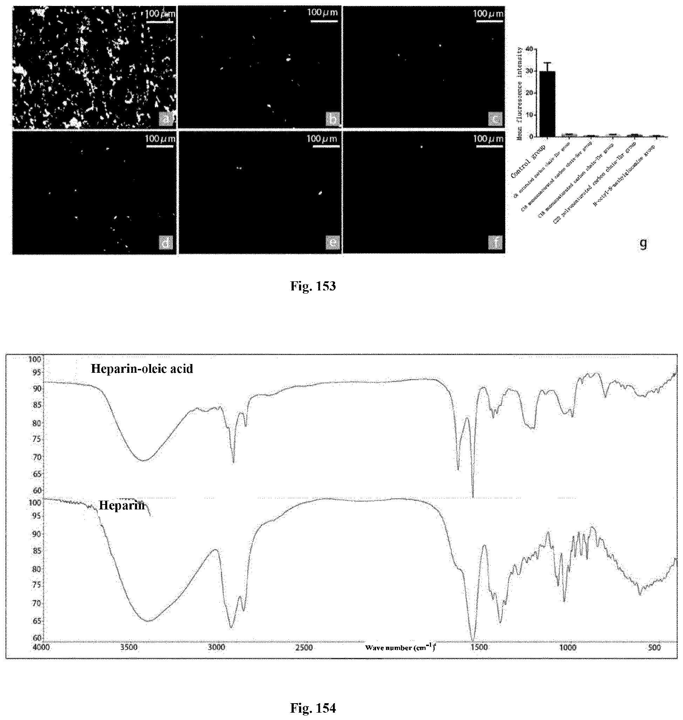

Fig. 153 is a graph showing the results of lung fluorescence in the animal experiment administred by gavage with small molecule complex in Example 52;
Fig. 154 a graph showing the infrared spectrum of the heparin-oleic acid complex prepared in Example 53;

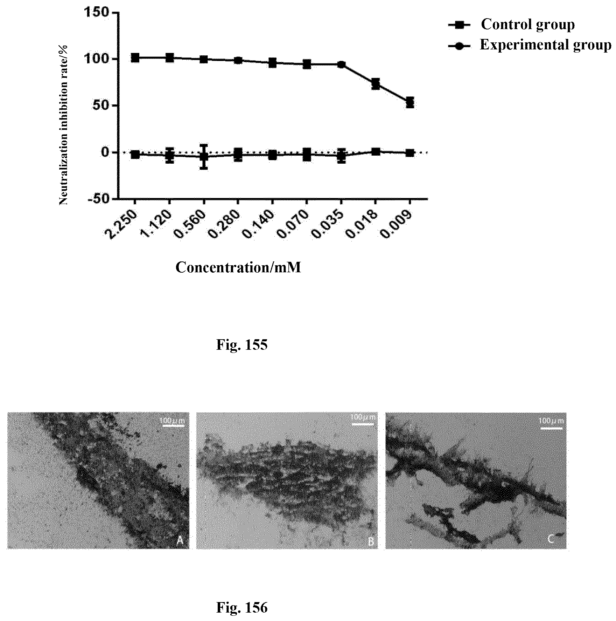

Fig. 155 is a graph showing the neutralization inhibition rate of the heparin-oleic acid complex against hepatitis B pseudovirus in Example 53;
Fig. 156 is a graph showing the HE staining results of a section of the mouse sinus mucosa in Example 54;

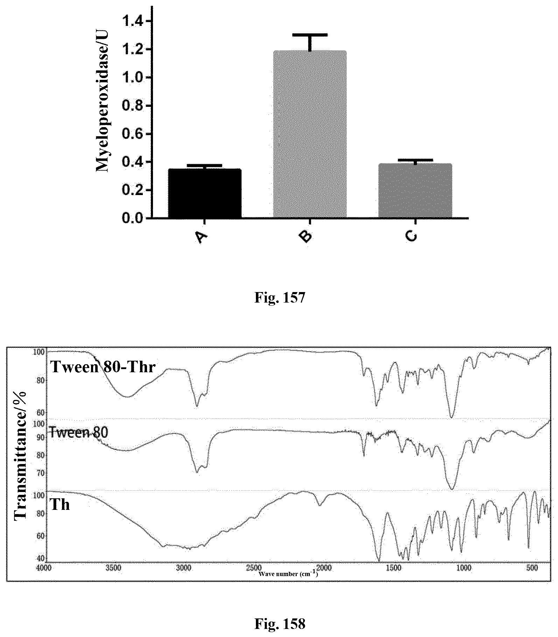

Fig. 157 is a graph showing the detection results of myeloperoxidase activity in nasal mucosal tissue of mice in Example 54;
Fig. 158 is a graph showing the infrared spectrum of the Tween 80-threonine complex prepared in Example 55;

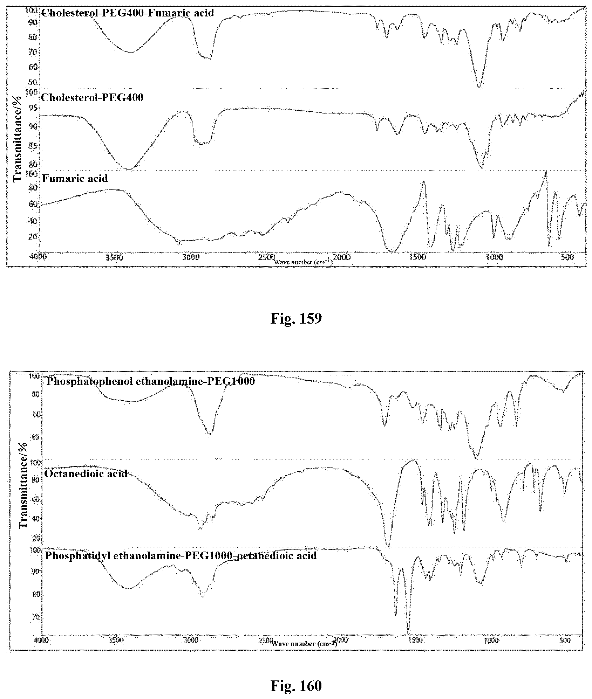

Fig. 159 is a graph showing the infrared spectrum of the cholesterol-PEG400-fumaric acid complex prepared in Example 56;
Fig. 160 is a graph showing the infrared spectrum of the phosphatidyl ethanolamine-PEG1000-octanedioic acid complex prepared in Example 57;

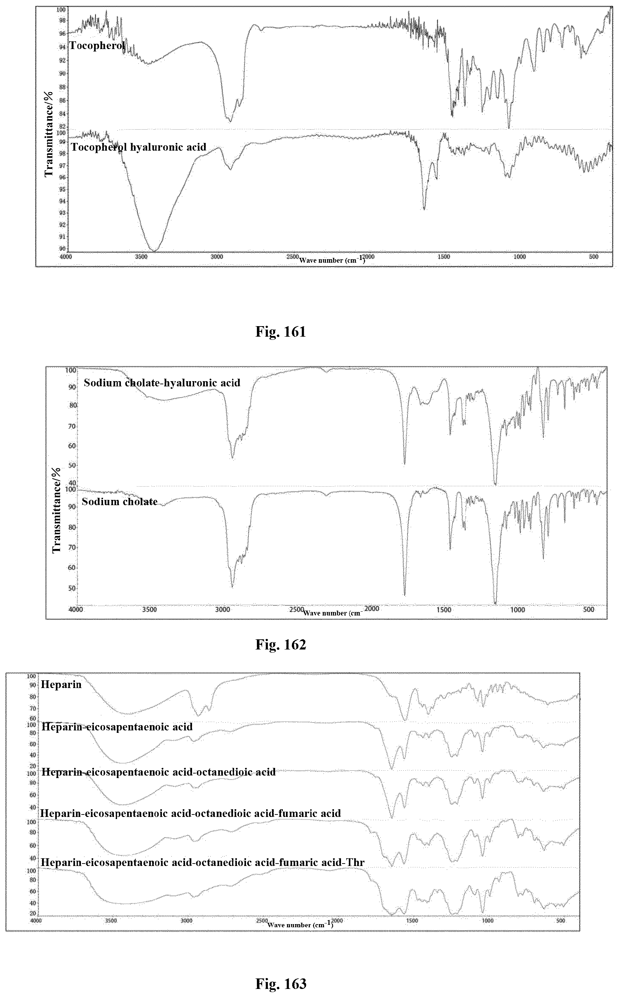

Fig. 161 is a graph showing the infrared spectrum of α-tocopherol-hyaluronic acid complex prepared in Example 58;
Fig. 162 is a graph showing the infrared spectrum of the sodium cholate-hyaluronic acid complex prepared in Example 59; and
Fig. 163 is a graph showing the infrared spectrum of the complex prepared in Example 60.

### Detailed Description of the Invention

The present invention is directed to a group of water-soluble carbon chain substances that affect the transmembrane transport of the substances, membrane fluidity, membrane structure and function, the carbon chain substances can be compounds, complexes, or mixtures;
1.1. The water-soluble carbon chain substance comprises: (a), a carbon chain originally having some water solubility, the structure of which includes at least one or more hydrophobic carbon chain moieties, and one or more hydrophilic groups or residues; (b), a highly hydrophilic complex also comprising hydrophobic carbon chain moieties formed by covalent coupling of water-soluble molecules;
1.2. the water-soluble carbon chain substances affect the structure and function of cell membranes, affect the transmembrane transport of the substances, affect the division and proliferation of cells, affect the movement and migration of cells, affect the fluidity of cell membranes, and affect the senescence of cells;
1.3. the effects include increase and promotion, or inhibition and reduction;
1.4. the water-soluble carbon chain substances and the same water-soluble carbon chain substances show increasing and promoting effects in a certain concentration range; shows inhibition and reduction in another concentration range;
1.5. the cell membrane comprises a cytoplasmic membrane and an organelle membrane; including cell membranes of human, animal, plant, bacterial and fungal, and viral envelopes;
1.6. the cell membrane structure comprises a phospholipid bilayer, proteins and polysaccharides adsorbed on the surface of the phospholipid bilayer and embedded throughout the phospholipid bilayer; including receptor proteins, transmembrane proteins, cytoskeletal proteins, and enzymes, etc.;
1.7. the affecting the function of the cell membrane comprises affecting the transmembrane transport function of the cell membrane, i.e. increasing and promoting or inhibiting or reducing the transmembrane transport function of the cell membrane; the transmembrane transport includes the functions of active transport, passive transport, endocytosis, and exocytosis;
1.7.1. the active transport comprises: ATP-driven pump transport including Na⁺-K⁺ pump and Ca²⁺ pump, class P, class V, class F and ABC superfamily, and coordinated transport;
   the passive transportation comprises simple diffusion and facilitated diffusion;
   the endocytosis comprises: phagocytosis, pinocytosis, and receptor-mediated endocytosis;
1.8. In the function of inhibiting or reducing transmembrane transport, substances transported across cell membranes include: small molecule compounds, medium molecule compounds, macromolecular compounds, pathogenic microorganisms such as viruses, bacteria, mycoplasma, chlamydia, and fungi, and nanoparticles and nanomedcines;
1.9. the inhibiting or reducing the transmembrane transport of the virus, i.e. inhibiting or reducing the entry of the virus into the cell by endocytosis, or inhibiting or reducing viral entry into the cell through fusion of the viral envelope with the cell membrane; it can be used for preventing all viral infections;
   the inhibition or reduction of transmembrane transport of mycoplasma, chlamydia, bacteria, and fungi may used for the prevention of mycoplasma and chlamydia infections and the prevention of bacterial and fungal infections;
1.10. the inhibiting or reducing transmembrane transport of the nanoparticle or nano-drug, i.e. inhibiting or reducing entry of the nanoparticle or nano-drug into the cell by endocytosis, can prevent, inhibit or reduce the non-specific phagocytosis of nanoparticles or nanomedcines by cells, thereby enabling more nano-drugs to enter the target site and improving the effective utilization of nano-drugs;
1.11. the affecting transmembrane transport of cell membranes includes increasing and promoting the release of intracellular exocrine out of the cells, and includes inhibiting or reducing the release of intracellular exocrine out of the cells;
1.12. the affecting the structure and function of the cell membrane comprises affecting the fluidity of the cell membrane, and promoting cell proliferation and division by increasing cell membrane fluidity;
1.13. the increasing the fluidity of the cell membrane can also cause the phospholipid bilayer in the cell membrane to change from a liquid crystal state to a liquid state by increasing the fluidity of the cell membrane, resulting in cell membrane rupture;
1.14. the increasing cell membrane fluidity, causing the phospholipid bilayer in the cell membrane to change from a liquid crystal state to a liquid state by increasing the fluidity of the cell membrane, resulting in cell membrane rupture, can be used for disrupting lipid envelopes of viruses, mycoplasma, chlamydia, bacteria, and fungi having lipid envelopes, and used in the treatment of infections of viruses, mycoplasma, chlamydia, bacteria, and fungi;
1.15. the reducing cell membrane fluidity, can maintain membrane stability, reducing cell division and proliferation; reduce and inhibit the release of intracellular inflammatory factors caused by cell membrane rupture, so as to reduce the inflammatory response and extent, and thus have anti-aging, life-prolonging, and anti-skin aging effects on humans and other animals;
1.16. the inhibition or reduction of endocytosis or membrane fusion of viruses, bacteria and mycoplasma into cells can be used for the prevention of viral diseases and infection by other pathogenic microorganisms; to prevent chronic diseases caused by viral, bacterial and mycoplasma infections, including neurodegenerative diseases including Alzheimer's disease (AD), Parkinson's disease (PD), Huntington's disease (HD), senile dementia, amyotrophic lateral sclerosis (ALS), different types of spinocerebellar ataxia (SCA), Pick's disease, and the like; degenerative neuropathy caused by intracranial nerve infection caused by herpes virus;
1.17. the affecting the function of the cell membrane, further comprises affecting the movement and migration of the cell, including promoting or inhibiting the migration of macrophages and immune cells; promoting or inhibiting migration and movement of melanocytes, promoting or inhibiting migration and movement of hair follicle cells.
1.18. the ability to reduce the fluidity of cell membranes, inhibit cell movement and deformation, can be used for inflammatory response syndrome caused by leukocyte migration in severe inflammation, reducing the fluidity of the cell membrane to stabilize the cell membrane, inhibiting the cell membrane from rupturing under the action of inflammatory factors, and inhibiting the occurrence of inflammation.
1.19. the increasing the fluidity of the cell membrane can inhibit cell senescence; it can be used against body aging and against skin aging.
1.20. The group of water-soluble carbon chain substances have anti-inflammatory and anti-aging effects by changing the conformation of inflammatory factors (proteins) in combination with oxygen free radicals, thereby inhibiting the inflammatory reaction.
2. Structural composition of water-soluble carbon chain substance
   In the present invention, the "water-soluble carbon chain" or "carbon chain" includes both two cases, i.e. a substance and residue having a carbon chain. The "water-soluble carbon chain substance" or "carbon chain substance" includes a substance (which can be a molecule) containing a carbon chain or a carbon chain residue or carbon chain moiety or a complex or physical mixture of the substance (which can be a molecule) and another molecule or residue containing a hydrophilic group. That is, in some cases, the water-soluble carbon chain substance of the present invention is itself a compound itself having both a hydrophilic group and a hydrophobic carbon chain moiety or residue of the carbon chain. In some cases, the water-soluble carbon chain substance is a carbon chain residue of an aliphatic hydrocarbon, a fatty acid, a fatty alcohol or an ether or an ester having a carbon chain (preferably a carbon chain with 3-100 carbon atoms) or a surfactant or various derivatives thereof, or a compound or complex obtained by the reaction of the carbon chain substance itself with other materials such as at least one selected from the group consisting of a protein, polypeptide, oligopeptide, oligosaccharide, monosaccharide, disaccharide, nucleotide, vitamin, amino acid, water-soluble polymer, water-soluble polyamino acid and polysaccharide molecules, or a mixture of unreacted carbon chain residues or carbon chain substance and/or unreacted protein, polypeptide, oligopeptide, oligosaccharide, monosaccharide, disaccharide, nucleotide, vitamin, amino acid, water-soluble polymer, water-soluble polyamino acid and/or polysaccharide molecule.
2.1. The carbon chain moiety having a certain water solubility is a polar group or residue having water solubility in the molecule, such as a hydroxyl group, a carboxyl group, an amino group, an amine group, a quaternary ammonium group, a guanidine group, a sulfhydryl group, a sulfonyl group, a sulfonyloxy group, a phosphate group, and the number of carbon atoms constituting the carbon chain is 3-100;
2.1.1. The carbon chain substances with some water solubility include mid-short chain fatty acids that are inherently less water-soluble, including acetic acid, oxalic acid, propionic acid, lactic acid, pyruvic acid, citric acid, butyric acid, succinic acid, malic acid, tartaric acid, fumaric acid, pentanoic acid, glutaric acid, hexanoic acid, hexane diacid, heptanoic acid, heptanedioic acid, octoic acid, octenoic acid, octanedioic acid, decanoic acid, and decanedioic acid; and fatty acid derivatives, including surfactants: fatty acid salts, alkyl sulfonates, alkyl benzene sulfonates, alkyl sulfates, alkyl phosphates, alkyl amine salts, alkyl quaternary ammonium salts, fatty acyl amino acids, betaines, fatty alcohol polyoxyethylene ethers, alkylphenol polyoxyethylene ethers, fatty amine polyoxyethylene ethers, polyol fatty acid esters, polyol polyoxyethylene ether fatty acid esters, fatty acid polyoxyethylene esters, alkyl glycosides, and alcohol ether glycosides;
   The water-soluble carbon chain substances of the present invention include some small molecule compounds containing hydroxyl groups and benzene rings:
   flavone, isoflavone, anthocyanin, *Glycine max* (soybean) isoflavone, *Aloe vera* (aloe) emodin, *Vitis vinifera* (grape) seed extract, green tea flavone, naringenin, *Citrus limon* (lemon) flavone, baicalein, riboflavin, quercetin, graphite flavone, *Cinnamomum cassia* (cinnamon) flavone, *Buxus sinica* (buxolin) flavone, *Crataegus pinnatifida* (hawthorn) flavone, morin, luteolin, *Melilotus officinalis*(sweet clover) flavone, gypsum flavone, *Lonicera japonica* (honeysuckle) flavone, *Gentiana scabra Bunge* (gentian) flavone, *Platycodon grandiflorus* (platycodon) flavone, *Viola phillipina* (Chinese violet) flavone, *Perilla frutescens* (perilla) flavone, *Dendranthema morifolium* (chrysanthemum) flavone, artemisinin, *Cynanchum officinale* (red peony) flavone, *Salvia miltiorrhiza Bunge* (salvia) flavone, *Saposhnikovia divaricata* flavone, *Chelonopsis pseudobracteata* (safflower) flavone, *Rhodiola rosea* L.(rhodiola) flavone, *Ziziphus jujuba var. inermis* (jujube) flavone, *Lycium chinense Mill.* (wolfberry) flavone, prepared *Rehmannia glutinosa* (rehmannia) flavone, *Ganoderma lucidum* flavone, *Schisandra chinensis* (schizandrin) flavone, *Glycyrrhiza uralensis* (licorice) flavone, *Panax pseudoginseng* (notoginseng) flavone, curcumin, apigenin, carotene, anthocyanin, lutein, zeaxanthin, Sinapis alba L. flavone, Ruta graveolens L. flavone, genkwanin, pollen flavone, *Hippophae rhamnoides L.* (sea-buckthorn) flavone, *Calendula officinalis L.* (marigold) flavone, cinnamon flavone, isoflavonoids and anthocyanins; rutin, emodin, fucoxanthin, gallic acid, persimmon peel extract, morin, echinacoside, grapeseed procyanidin, phenolic acid, tea polyphenol, naringin, citric acid, flavonol, glycyrrhizic acid, cinnamic acid, flavone glycoside, silymarin, matrine, tanshinone, Mangrove bark extract, hippophaetic acid, perillyl alcohol, anisic acid, amurensin, hesperidin, punicalin, morchellin, naringin, onionoside(Cleistocalyx operculatus extract), gentiopicrin, jasmine, naringol, carotene, apigenin, *Vatica mangachapoi Blanco* (green plum) extract, *Folium mori* (mulberry leaf) extract, loniceroside, *Dendranthema morifolium* (chrysanthemumin) extract, *Canarium album* (olive) extract, Oolong tea extract, theanine, vin rouge essence, platycodin, perillyl alcohol glycoside, mulberry bark extract, carthaminol, matsuba enzyme, pachymic acid, caffeic acid, chlorogenic acid, resveratrol, white tea polyphenol, resveratrol disaccharide, *Musa basjoo* (banana) lutein, anthocyanidin, arachidonic acid, *Arachis hypogaea* (peanut) flavone, xanthotol, anethol, flavonoid, baicalein, flavonoid glycoside, flavanol, vin rouge polyphenol, rhodiol, carthaminol, black tea flavone, black sesamin, *Secale cereale* (rye) phenol, trehalose alcohol, seaweed polysaccharide, alginic acid, *Albizzia julibrissin* extract (albizarin), cannabidiol, polydatin, cucurbitacin, *Trigonella foenum-graecum* (Huluba) extract, cucurbic acid, pollen phenol, pollen flavone, pollen glycoside, pollen ester, arachidic acid, peanut flavone glycoside, peanut isoflavone, peanut isoflavone glycoside, peanut isoflavone disaccharide, peanut isoflavone trisaccharide, peanut resveratrol, peanut resveratrol disaccharide, peanut resveratrol trisaccharide, peanut resveratrol tetrasaccharide, peanut resveratrol pentasaccharide, peanut resveratrol hexasaccharide, peanut resveratrol heptasaccharide, peanut resveratrol octasaccharide, peanut resveratrol nonasaccharide, peanut resveratrol decasaccharide, peanut resveratrol undecasaccharide, catechin, epicatechin, tea polyphenol, catechol, chlorophyll, protocatechin, hesperidin, anthocyanin, cyanine glucoside, cyanine aglycone, cyanine alcohol, glucoside, glucose aglycone, alfalfa extract, soybean isoflavone, flavanol, quercitannin, *Fagopyrum tataricum* (buckwheat) extract, persimmon peel extract, persimmon tannin, punicic acid, punicalin, blueberry extract, resveratrol, lycopene, naringenin, morin, chlorogenic acid, chlorogenic acid triglycoside, chlorogenic acid diglucoside, methyl chlorogenate, ethyl chlorogenate, propyl chlorogenate, butyl chlorogenate, isoamyl chlorogenate, hexyl chlorogenate, octyl chlorogenate, benzyl chlorogenate, phenethyl chlorogenate, phenylpropyl chlorogenate, phenylbutyl chlorogenate, phenyl isoamyl chlorogenate, phenylhexyl chlorogenate, phenyloctyl chlorogenate, styrene chlorogenate, chlorogenic acid benzyl alcohol ester, chlorogenic acid phenethyl alcohol ester, anthocyanin, proanthocyanidin glycoside, and catechin.
2.2. The complex of a hydrophobic carbon chain substance or carbon chain moiety with a water-soluble molecule refers to a complex of a hydrophobic carbon chain directly bonded to a water-soluble molecule through a chemical bond or bonded to a water-soluble molecule through a linker.
2.2.1. The hydrophobic carbon chain substance or carbon chain moiety is a molecule or a residue of the molecule with 3-100 carbon atoms.
2.2.2. The carbon chain is saturated or unsaturated. The unsaturated bond is a double bond or a triple bond, and the number of unsaturated bonds is 1 or 2 or more.
2.2.3. The carbon chain substance or carbon chain moiety is a straight chain, a cyclic carbon chain, or a carbon chain with a branched chain or a cyclic structure on the straight chain, or a straight chain or cyclic carbon chain containing oxygen, sulfur, and nitrogen hybridization.
2.2.4. The cyclic carbon chain-water-soluble carbon chain substance include monocyclic and polycyclic rings.
2.3. The hydrophobic carbon chain substance or carbon chain moiety is a residue of a carbon chain substance or carbon chain with 3-100 carbon atoms selected from the group consisting of saturated and/or unsaturated aliphatic hydrocarbons, saturated and/or unsaturated fatty alcohols or oxo-fatty alcohols, saturated and/or unsaturated fatty acids, saturated and/or unsaturated hydroxy fatty acids, saturated and/or unsaturated oxo fatty acids, hydrophobic amino acids, fat-soluble vitamins, carotenoids, sterols and steroid lipidoids, fatty acid glycerides, phospholipids, sphingomyelin, glycolipids, and/or surfactants.
2.3.1. The number of carbon atoms of the saturated and/or unsaturated fatty acid is 3-50.
2.3.2. The number of carbon atoms of the saturated and/or unsaturated fatty acid is 3-26.
2.3.3. The saturated and/or unsaturated fatty acid is one or two or more fatty acids selected from the group consisting of acetic acid, oxalic acid, propionic acid, lactic acid, pyruvic acid, citric acid, butyric acid, succinic acid, malic acid, tartaric acid, crotonic acid, fumaric acid, pentanoic acid, glutaric acid, hexanoic acid, hexenoic acid, hexane diacid, heptanoic acid, heptenoic acid, heptanedioic acid, octanoic acid, octenoic acid, octanedioic acid, nonanoic acid, decanoic acid, decanedioic acid, undecanoic acid, undecenoic acid, dodecanoic acid, dodecenoic acid, dodecanedioic acid, tridecanoic acid, tridecanedioic acid, tetradecanoic acid, pentadecanoic acid, pentadecanedioic acid, hexadecanoic acid, hexadecenoic acid, hexadecanedioic acid, octadecanoic acid, octadecamonoenoic acid, octadecadienoic acid, octadecatrienoic acid, octadecanedioic acid, eicosanoic acid, eicosapentaenoic acid, docosanoic acid, docosomonoenoic acid, docosahexaenoic acid, docosadioic acid, tetracosanoic acid, triacontanoic acid, and hexatriacontanoic acid.
2.3.4. The hydrophobic carbon chain substance comprises fatty acid ozonation products: fatty acid peroxides, hydroxy fatty acids, oxo fatty acids, fatty acid dicarboxylic acids, fatty aldehydes, fatty alcohols, and fatty acid esters.
2.4. The water-soluble moiety is a molecule or a residue of the molecule that is soluble in water.
2.4.1. water-soluble portion of the molecule contains one or more functional groups selected from the group consisting of amide group, phosphooxy groups, carboxylic acid grouamide group, phosphoryloxy group, carboxyl group, phosphate group, sulfonyl group, sulfonyloxy group, hydroxyl group, quaternary ammonium group, thioether group, disulfide bond, ether group, thiol group, aldehyde group, ester group, amine group, amino group, urea group, and guanidine group.
2.4.2. One or more water-soluble macromolecules or their residues selected from the group consisting of proteins, polysaccharides, nucleic acids, and artificially synthesized water-soluble polymers;
   and/or, one or two or more medium molecules selected from the group consisting of polypeptides, oligopeptides, oligosaccharides, oligonucleotides, and synthetic water-soluble medium molecular weight polymers, or residues thereof;
   and/or, one or two or more water-soluble small molecules selected from the group consisting of amino acids, monosaccharides, disaccharides, nucleotides, deoxynucleotides, and water-soluble vitamins, or residues thereof.
2.4.3. The protein as the water-soluble macromolecule is one or two or more water-soluble macromolecules selected from the group consisting of serum albumin, immunoglobulin, water-soluble collagen, chaperone protein, and water-soluble glycoprotein.

The polysaccharide as the water-soluble macromolecule is one or more water-soluble macromolecules selected from the group consisting of dextran, hyaluronic acid, sialic acid, heparin sulfate, heparan sulfate, chondroitin sulfate, dermatan sulfate, keratan sulfate, acetyl water-soluble cellulose derivatives, β-cyclodextrin and derivative thereof, and water-soluble chitosan derivatives.

The water-soluble polymer as the water-soluble macromolecule is one or two or more water-soluble macromolecules selected from the group consisting of polyethylene glycol and carboxylated or aminated polyethylene glycol, polyvinyl alcohol and carboxylated or aminated polyvinyl alcohol, polyacrylic acid, and ammonium polyacrylate;
the water-soluble medium molecular weight polymer is one or two or more substances selected from the group consisting of polypeptides, oligopeptides, oligosaccharides, oligonucleotides and/or water-soluble polyamino acids;
The water-soluble small molecule monosaccharide and/or disaccharide is one or two or more selected from the group consisting of glucose, fructose, rhamnose, sorbose, sucrose, maltose, lactose, and trehalose;
the nucleotide and/or deoxynucleotide as the water-soluble small molecule is one or two or more selected from the group consisting of adenosine, guanylate, uranylate, cytidylate, thymidylate, inosine, deoxyadenosine, deoxyguanosine, deoxycytidylate, and deoxythymidylate; the amine acid as the water-soluble small molecule is one or two or more of amino acids such as serine, threonine, cysteine, asparagine, glutamine, tyrosine, lysine, arginine, histidine, aspartate, glutamate, citrulline, ornithine, taurine, and aminobutyric acid;
the vitamin as the water-soluble small molecule is one or two or more selected from the group consisting of vitamin B1, pantothenic acid, vitamin B6, and vitamin C;
the water-soluble proteins and peptides include any one of proteins that specifically target microbial lipid membranes, bacterial and fungal cell walls, virus surface protein domains, or neutralizing antibody fragment; and
the water-soluble polyamino acid is any one selected from the group consisting of polyglutamic acid, polylysine, and/or polyaspartic acid, oligopeptides, oligosaccharides, and oligonucleotides.

3. The water-soluble carbon chain affects the structure and function of the cell membrane.

3.1. Structure and composition of the cell membrane phospholipid bilayer: a phospholipid bilayer comprising lecithin, sphingomyelin, and cholesterol; a lipid raft structure; an inner leaflet and an outer leaflet of the phospholipid bilayer.

3.2. The structure of the cell membrane includes proteins involved in cell endocytosis and exocytosis functions: membrane proteins of cell membranes, transmembrane proteins of cell membranes, receptors of cell membranes, cytoskeletal proteins.

3.3. The structure of the cell membrane includes glycoproteins and lipoproteins on the surface of the cell membrane, including polysaccharide on the cell membrane surface.

3.4. The proteins involved in cell endocytosis and exocytosis functions:
3.4.1. Endocytosis proteins in cells:
   3.4.1.1. Clathrin proteins: Clathrin Heavy Chain 1 (CHC1), Clathrin Heavy Chain 2 (CHC2), Clathrin Light Chain A (CLTA), Clathrin Light Chain B (CLTB);
   3.4.1.2. Caveolin proteins: Caveolin-1 (CAV1), Caveolin-2 (CAV2), and Caveolin-3 (CAV3);
   3.4.1.3. Dynamin proteins: Dynamin1, Dynamin2, Dynamin3, Dynamin-like protein (DNM1L, also known as Drp1), and Dynamin-like protein 2 (DNM2);
   3.4.1.4. Rab proteins: Rab1, Rab2, Rab5, Rab7, Rab11, Rab27, and Rab35;
   3.4.1.5. SNARE proteins: VAMP2, VAMP3, VAMP4, Syntaxin, SNAP-25, Munc18, Munc13, and SNAP23;
   3.4.1.6. Arf (ADP ribosylation factor): Arf1, Arf2, Arf3, and Arf6; and
   3.4.1.7. Bridging integrators: AP180/CALM, Epsin, EHD (Eps15 Homology Domain), and Dab2 (Disabled-2);
3.4.2. Endocytosis-related skeleton proteins:
   3.4.2.1. Actins: alpha-actin, beta-actin, and gamma-actin;
   3.4.2.2. Tubulins: alpha-tubulin, beta-tubulin, and gamma-tubulin;
   3.4.2.3. Intermediate filament: Keratin, Myosin, Neurofilament, Glial fibrillary acidic protein (GFAP), nuclear lamins;
   3.4.2.4. Myosins: muscle myosin, non-muscle myosin, adhesion myosin, neural myosin, and cardiac myosin;
   3.4.2.5. Septin protein: SEPT2 subtype, SEPT3 subtype, SEPT4 subtype, SEPT5 subtype, SEPT6 subtype, SEPT7 subtype, SEPT9 subtype, and SEPT11 subtype;
3.4.3. Endocytosis-related cell membrane receptor proteins:
   3.4.3.1. High density lipoprotein receptor (HDL receptor): SR-BI, CD36, ABCA1, SR-BII, CLA-1, and GPIHBP1;
   3.4.3.2. LDL receptors (LDLRs): LDLR-A, LDLR-B, LDLR-related protein (LRP), LDLR-related protein 1B (LRP1B), and sortilin;
   3.4.3.3. Transferrin receptors: transferrin receptor 1 (transferrin receptor 1, TfR1), and transferrin receptor 2 (transferrin receptor 2, TfR2);
   3.4.3.4. EGF receptors (EGFRs): EGFR(ErbB1), ErbB2(HER2), ErbB3(HER3), and ErbB4(HER4);
   3.4.3.5. TLR receptors (Toll-like receptors): TLR1, TLR2, TLR3, TLR4, TLR5, TLR6, TLR7, TLR8, TLR9, and TLR10;
   3.4.3.6. G protein-coupled receptors (GPCR): Rhodopsin-like GPCR family, Secretin-like GPCR family, Metabotropic glutamate GPCR family, Adhesion GPCR family, Frizzled/Smoothened GPCR family;
   3.4.3.7. Scavenger receptors: Class A Scavenger Receptor, Class B Scavenger Receptor, Class C Scavenger Receptor, and Class D Scavenger Receptor;
   3.4.3.8. VLDL Receptors: VLDLR, and LRP8;
   3.4.3.9. LRP (Low-Density Lipoprotein Receptor-Related Protein) receptor: LRP1, LRP2, LRP4, and LRP5;
   3.4.3.10. Chloride channel receptors: GABA-A receptor, GlyR receptor, CFTR (Cystic Fibrosis Transmembrane Conductance Regulator), CLC family, Bestrophin, and Anoctamin;
   3.4.11. Endoglin Receptors: CD105, and CD105a;
   3.4.12. CD44 protein: CD44s, and CD44v;
   3.5. Exocytosis-related proteins: cathepsin, tumor necrosis factors (TNF), angiogenesis factors (VEGF), glutathione S-transferases (GGSTs), cathepsin inhibitor (TIMPs), ovalbumin (α2-macroglobulin), tyrosine kinase receptor (TKRs), G protein-coupled receptor (GPCRs), Toll-like receptors (TLRs), integrins, Fibrillin, collagen, Heparan Sulfate Proteoglycans, and Syndecan;
   4. The application of water-soluble carbon chains affecting the transmembrane transport function of cell membranes
   4.1. In the function of inhibiting or reducing transmembrane transport, substances transported across cell membranes include: small molecule compounds, medium molecule compounds, macromolecular compounds, pathogenic microorganisms such as viruses, bacteria, mycoplasma, and chlamydia, and nanoparticles and nanomedcines.
   4.1.1. The small molecule compounds include: water, glucose, amino acids, ions, O₂, CO₂, and N₂;
   4.1.2. The medium molecule compounds include: cholesterol, iron, vitamin B12, polypeptides, etc.;
   4.1.3. The macromolecular compounds include: proteins and growth factors;
   4.1.4. The viruses include:
      Influenza virus, coronavirus, hepatitis virus, hepatitis B virus, hepatitis C virus, HIV virus (Human immunodeficiency virus), varicella-zoster virus, measles virus, rubella virus, adenovirus, enterovirus, yellow fever virus, human papilloma virus, human respiratory syncytial virus, rotavirus, adenovirus type 36, adenovirus type 6, adenovirus type 11, adenovirus type 7, adenovirus type 5, adenovirus type 4, adenovirus type 3, adenovirus type 2, adenovirus type 1, adenovirus type 40, adenovirus type 41, parainfluenza virus, encephalitis A B virus, HIV-2, human herpes simplex virus, respiratory syncytial virus-type 1, respiratory syncytial virus-type 2, respiratory syncytial virus-type 3, herpes zoster virus, Lyme disease virus, HIV-1, porcine parvovirus, picornaviridae, Norovirus, parainfluenza virus, influenza virus, Norovirus, hepatitis A virus, and Calicivirus;
      Susceptible viruses of animals
      Feline infectious peritonitis virus (FIPV), feline panleukopenia virus (FPV), feline coronavirus (FCoV), feline immunodeficiency virus (FIV), feline leukemia virus (FeLV), feline calicivirus, feline viral rhinotracheitis virus (FVRV), Haemobartonella felis, feline Borna disease virus, Borrelia burgdorferi, rabies virus, canine distemper virus, canine coronavirus, canine infectious hepatitis virus, canine parvovirus, canine influenza virus, canine viral diarrhea virus, canine oral papillomavirus, canine herpesvirus, canine immunodeficiency virus, foot-and-mouth disease virus, bovine respiratory syncytial virus, rinderpest virus, bovine reproductive and respiratory syndrome virus (bovine viral diarrhea virus), ovine viral diarrhea virus, bluetongue virus, infectious bronchitis virus, Marek's disease virus, Newcastle disease virus, duck plague virus, duck hepatitis virus, goose parvovirus, infectious laryngotracheitis virus, goose hepatitis B virus, goose astrovirus, equine foot-and-mouth disease virus, porcine parvovirus, swine influenza virus, Bovine virus of viral diarrheabovine and respiratory disease complex, porcine transmissible gastroenteritis virus (Porcine epidemic diarrhea virus), porcine viral diarrhea virus, porcine parainfluenza virus, mycoplasma hyopneumoniae, porcine brain virus, porcine vesicular virus, herpes virus, bovine viral diarrhea virus (BVDV), bovine papilloma virus, bovine virus of contagious bovine pleuropneumonia, bovine enterovirus, bovine coronavirus, goose plague virus, duck infectious hepatitis virus, duck infectious laryngotracheitis virus, duck infectious gastroenteritis virus, goose infectious gastroenteritis virus, avian influenza virus, newcastle disease virus, infectious bronchitis virus, infectious bursal disease virus, infectious chicken anemia virus, infectious rhinitis virus, infectious laryngotracheitis virus, infectious chicken hepatitis virus, and chicken infectious diarrhea virus;
   4.1.5. The pathogenic microorganisms includes:
      Mycoplasma, chlamydia, bordetella pertussis, candida, actinomyces, treponema pallidum; *Escherichia coli, Staphylococcus aureus, Mycobacterium tuberculosis, Pseudomonas aeruginosa, Klebsiella pneumoniae, Salmonella, Proteus mirabilis, Bacillus, Streptococcus pneumoniae,* Methicillin-resistant *Staphylococcus aureus* (MRSA), *Vibrio cholerae, Clostridium difficile, Neisseria gonorrhoeae, Clostridium tetani, Listeria monocytogenes, Clostridium perfringens, Mycobacterium tuberculosis, Candida albicans, Aspergillus, Histoplasma, Trichophyton, Cryptococcus, Blastomyces, Pneumocystis, Clostridium, Phytophthora, Saccharomyces, Fusarium, Sporothrix, Malassezia, Coccidioides, Actinomyces, Bacillus anthracis, Rhizopus, Pneumocystis carinii,* and*Zygomycetes*;
   4.1.6. The nano-drug includes polymer, metal, semiconductor or ceramic nano-drug, liposome drug, nanotube drug, and nucleic acid nano-drug.

4.1.7. The nanoparticles include metal nanoparticles, quantum dots, magnetic nanoparticles, and high molecular polymer nanoparticles.

4.2. The prevention, inhibition or reduction of virus, bacteria, chlamydia, mycoplasma and fungi entering cells by endocytosis or by transmembrane transport such as membrane fusion is applicable to the prevention of virus, bacteria, chlamydia, mycoplasma, and fungi infection.

4.3. The prevention, inhibition or redution of the non-specific phagocytosis of nanoparticles or nanomedcines by cells enable more nano-drugs to enter the target site and improve the effective utilization of nano-drugs.

### 4.5. The effect of the water-soluble carbon chain on exocrine

4.5.1. The water-soluble carbon chain can increase and promote the release of insulin exosomes from pancreatic islet cells into the extracellular space within a certain concentration range, and inhibit or reduce the release of insulin exosomes from pancreatic islet cells into the extracellular space within another concentration range.

4.5.2 The water-soluble carbon chain can increase and promote the release of leukocytes, hepatocytes, breast cells and nerve cells exosomes to the outside of cells within a certain concentration range, and inhibit or reduce the release of leukocytes, hepatocytes, breast cells and nerve cells exosomes to the outside of cells within another concentration range.

### 5. Use of water-soluble carbon chain in increasing cell membrane fluidity

5.1. The water-soluble carbon chain can promote cell proliferation and division by increasing membrane fluidity within a certain concentration.

5.2. It can cause the phospholipid bilayer in the cell membrane to change from a liquid crystal state to a liquid state by increasing the fluidity of the cell membrane, resulting in cell membrane rupture, can be used for disrupting cell membranes of viruses, mycoplasma, chlamydia, bacteria, and fungi having lipid envelopes, and can be used in the treatment of infections of viruses, mycoplasma, chlamydia, bacteria, and fungi.

5.2.1. Enveloped viruses that can be killed include enveloped viruses such as one or more of coronavirus, influenza virus, AIDS virus, hepatitis B virus, hepatitis C virus, herpes virus, Zika virus, Dengue virus, Japanese encephalitis virus, Ebola virus, monkeypox virus, respiratory syncytial virus and/or Hantavirus.

5.2.2. Bacteria, mycoplasma, chlamydia, fungi that can be killed. The bacteria are Gram-positive and/or Gram-negative bacteria and the fungi are pathogenic fungi and/or conditionally pathogenic fungi; the Chlamydia is *Chlamydia trachomatis, Chlamydia pneumoniae,* and/or *Chlamydia psittaci;* the mycoplasma include *Mycoplasma pneumoniae, Ureaplasma urealyticum, Mycoplasma hominis, and*/*or Mycoplasma genitalium.*

5.2.3. The bacteria are one or more selected from the group consisting of *Escherichia coli, Staphylococcus aureus,* Methicillin-resistant *Staphylococcus aureus, Streptococcus pneumoniae, Klebsiella pneumoniae* and *Pseudomonas aeruginosa;* the fungus is one or more selected from the group consisting of *Candida albicans, Aspergillus niger, Actinomyces viscosus, Chaetomium globosum, Aspergillus protuberus* and *Microsporum canis.*

6. The use of the water-soluble carbon chain in reducing cell membrane fluidity at a range of concentrations.

6.1. The water-soluble carbon chains maintain membrane stability by reducing cell membrane fluidity, reducing cell division and proliferation.

6.2. The water-soluble carbon chain can reduce and inhibit the release of intracellular inflammatory factors caused by cell membrane rupture, so as to reduce the inflammatory response and extent.

6.3. The water-soluble carbon chain has anti-aging and skin anti-aging effects by affecting the fluidity of cell membranes.

6.4. The water-soluble carbon chain has an anti-aging and anti-skin aging effect by neutralizing inflammatory mediators and in turn inhibiting inflammation.

7. The water-soluble carbon chain can prevent, inhibit or reduce the virus entering the host cell via endocytosis or membrane fusion within a certain concentration range, and can be used for preventing viral infection in humans or animals.

### 7.1. The viruses include

7.1.1. Enveloped virus and non-enveloped virus

7.1.2. Dna virus and RNA virus: double-stranded DNA virus, single-stranded DNA virus, double-stranded RNA virus and positive-sense single-stranded RNA virus: antisense single-stranded RNA virus, retrovirus, RNA retrovirus, DNA retrovirus.

### 7.2. Prevention of infection including human, animal and plant viruses

7.3. The viruses include: coronavirus, influenza virus, AIDS virus, hepatitis B virus, hepatitis C virus, herpes virus, Zika virus, Dengue virus, Japanese encephalitis virus, Ebola virus, Hantavirus, polio virus, adenovirus, herpesvirus: HSV, VZV, human papilloma virus (HPV), orthopoxvirus including one or more of variola virus and monkeypox virus, Asfarviridae, rotavirus, measles virus, Bunya virus, flavivirus, reovirus, arenaviridae, filoviridae, rhabdoviridae, rabies virus, paramyxoviridae, Nipah virus, and prions.

8. The water-soluble carbon chain can prevent a chronic disease caused by a viral infection within a certain concentration range.

8.1. Neurodegenerative diseases that can be prevented by bacterial infections include Alzheimer's disease (AD), Parkinson's disease (PD), Huntington's disease (HD), senile dementia, amyotrophic lateral sclerosis (ALS), different types of spinocerebellar ataxia (SCA), and Pick's disease.

8.2. It can prevent acquired immunodeficiency syndrome (AIDS) caused by human immunodeficiency virus (HIV).

It can prevent oral herpes, genital herpes, and other skin lesions caused by herpes simplex virus (HSV) infection.

It can prevent herpes zoster and postherpetic neuralgia caused by herpes zoster virus (VZV) infection.

It can prevent infections and diseases caused by human cytomegalovirus (HCMV) after organ transplantation, congenital infections in newborns, and lesions in immunocompromised individuals.

It can prevent adult T-cell leukemia/lymphoma (ATL) and HTLV-1-related myeloid disease caused by human T-cell lymphovirus (HTLV-1) infection.

It can prevent mumps, rosacea, and post infection fatigue syndrome caused by human cytomegalovirus type 6 (HHV-6) infection.

9. The use of the water-soluble carbon chain in influencing the movement and migration of cells

9.1. It can promote the deformation and movement of macrophages, T lymphocytes, B lymphocytes, dendritic cells, and NK cells within a certain concentration range. It can be used for adjuvant therapy in immunotherapy. It inhibits the deformation and movement of macrophages, immune cell T lymphocytes, B lymphocytes, dendritic cells, NK cells within another concentration range.

9.2. It can promote the deformation and movement of melanocytes within a certain concentration range, and inhibit melanocyte deformation and movement within another concentration range.

9.3. It can promote the deformation and movement of hair follicle cells within a certain concentration range, and inhibit deformation and movement of the hair follicle cells within another concentration range.

9.4. It can promote the deformation and movement of endothelial cells within a certain concentration range, and inhibit the deformation and movement of endothelial cells within another concentration range.

9.5. It can promote the deformation and movement of epithelial cells within a certain concentration range, and inhibit deformation and migration of epithelial cells within another concentration range.

9.6. It can promote the deformation and movement of leukocytes within a certain concentration range, and inhibit deformation and movement of the leukocytes within another concentration range.

### 10. The water-soluble carbon chain substance can neutralize inflammatory mediators

10.1. The water-soluble carbon chain substances can neutralize small molecule inflammatory mediators: oxygen free radicals, nitrogen free radicals, prostaglandins, histamine, leukotrienes, 5-hydroxytryptamine, and other neurotransmitters.

10.2. Water-soluble carbon chains can neutralize macromolecular inflammatory mediators such as tumor necrosis factor (TNF), interleukins (IL-1, IL-6, IL-8, etc.), thromboxanes, proteases, angiotensin, interact with these macromolecular inflammatory molecules to inhibit the inflammatory response.

11. The water-soluble carbon chain substance can be used as a pharmaceutical preparation, a cosmetic, a skin care product, a health care product and an environmental disinfection and sterilization preparation.

11.1. The drug preparation is one selected from the group consisting of an inhalation, a nasal spray, an injection, an oral preparation, a liposome lotion dosage form, an ointment, an oil, and a transdermal topical dosage form.

11.2. Use of the water-soluble carbon chain substance in the preparation of a pharmaceutical preparation or environmental microbial disinfection and sterilization agent for preventing, blocking and/or treating microbial infections.

11.3. A preparation method of the water-soluble carbon chain substance, the water-soluble carbon chain substance being obtained by the reaction of a compound having a fat-soluble saturated and/or unsaturated carbon chain with a branched, cyclic, and/or linear structure with a water-soluble molecule, and optionally added protein, polypeptide, amino acid, oligopeptide, oligosaccharide, monosaccharide, and/or polysaccharide molecule as needed capable of binding to a microbial lipid membrane, or cell wall, and optionally added a linker molecule as needed, in the presence of a catalyst.

11.3.1. The preparation method of the water-soluble carbon chain substance, wherein the water-soluble carbon chain substance is a product obtained by purifying the compound obtained from the reaction.

11.4. The preparation method of the water-soluble carbon chain substance, wherein the complex is obtained by physically mixing a compound with fat-soluble saturated and/or unsaturated carbon chains with branched, cyclic, and/or linear structures with a water-soluble molecule, and optionally added protein, peptide, amino acid, oligopeptide, oligosaccharide, monosaccharide, and/or polysaccharide molecules that can bind to the surface or cell wall of microbial lipid membranes as needed.

11.5. The preparation method of the water-soluble carbon chain substance, wherein the complex is obtained by the reaction of a saturated and/or unsaturated fatty acid with 3-100 carbon atoms with any one of a protein, peptide, oligopeptide, oligosaccharide, monosaccharide, disaccharide, oligonucleotide, vitamin, water-soluble polymer, water-soluble polyamino acid, and/or polysaccharide in the presence of a catalyst.

11.5.1. The preparation method of the water-soluble carbon chain substance, wherein the complex is a product obtained by purifying the compound obtained from the reaction.

11.6. The preparation method of the water-soluble carbon chain substance, wherein the complex is obtained by directly physical compounding a saturated and/or unsaturated fatty acid with 3-100 carbon atoms and at least one selected from the group consisting of protein, polypeptide, oligopeptide, oligosaccharide, monosaccharide, disaccharide, oligonucleotide, vitamin, water-soluble polymer, water-soluble polyamino acid, and/or polysaccharide molecule, in a physicochemical action, or by directly physical mixing the same.

Specifically, in an embodiment, the present invention provides a complex for preventing, blocking or treating a microbial infection. The complex of the present invention includes an acting moiety, a binding moiety, and a water-soluble moiety.

The acting moiety is a fat-soluble hydrophobic carbon chain. The carbon chain may exist in the form of a molecule or in the form of a residue of a molecule. The carbon chain is a saturated and/or unsaturated carbon chain with a branched and/or linear structure, and can be inserted/incorporated into a lipid membrane of a microorganism so as to destroy the lipid membrane structure, or encapsulate a non-enveloped virus so that the virus is hydrophobically isolated.
the binding moiety is a molecule or a residue of the molecule capable of binding to a microbial lipid membrane, a microorganism surface protein, a microorganism surface polysaccharide, or a cell wall component (including a polysaccharide or protein) or capable of binding to a polysaccharide or a protein or polypeptide in the microorganism, so that the complex is linked to the microbial lipid membrane or virus surface.

The water-soluble moiety is a water-soluble molecule or residue of the molecule, containing the water-soluble group. It can render the complex water-soluble so that the complex can be uniformly dispersed in an aqueous solution and avoid the aggregation of lipophilic hydrophobic groups, thus preventing the lipophilic hydrophobic groups of the complex from aggregating and forming lipid droplets in aqueous solution or blood.

In further detail, the acting moiety is a fat-soluble saturated and/or unsaturated carbon chain with a branched, cyclic and/or linear structure; the carbon chain is a molecule or a residue of the molecule, and the carbon chain is a carbon chain with 3-100 carbon atoms.
the water-soluble moiety is a water-soluble molecule or a residue of the molecule; the molecule contains one or two or more groups selected from the group consisting of amide group, phosphoryloxy group, carboxyl group, phosphate group, sulfonyl group, sulfonyloxy group, hydroxyl group, quaternary ammonium group, thioether group, disulfide bond, ether group, thiol group, amine group, amino group, urea group, and guanidine group; and the water-soluble moiety can be a group linked to the carbon chain as the acting moiety.

The binding moiety is a molecule or a residue of the molecule capable of binding to a microbial lipid membrane, a microorganism surface protein, a microorganism surface polysaccharide, or a cell wall component or capable of binding to a polysaccharide or a protein or polypeptide in the microorganism. The binding moiety can be the same as the water-soluble moiety, i.e. a protein, polypeptide, amino acid, oligopeptide, oligosaccharide, monosaccharide and/or polysaccharide molecule or a residue thereof capable of binding to a microbial lipid membrane and a surface domain. The binding moiety may also be the same as the water-soluble moiety, namely, one or two or more groups of free carboxyl groups, phosphate groups, sulfonyl groups, hydroxyl groups, sulfhydryl groups, amine groups, amino groups, urea groups and guanidine groups remain after forming the complex. In some cases, the binding moiety can be a dibasic or polybasic fatty acid, a fat-soluble amino acid molecule or residue thereof that binds to a microbial lipid membrane or a surface domain, in which case the carboxyl group and the amino acid group in the dibasic or polybasic fatty acid, or the fat-soluble amino acid molecule or residue substantially plays a binding role.

Specifically, the acting moiety is fat-soluble carbon chain, including saturated or unsaturated carbon chains with branched and cyclic structures. Preferably the acting moiety is a carbon chain or carbon chain residue with 3-48, more preferably 3-26 carbon atoms, selected from the group consisting of saturated and/or unsaturated aliphatic hydrocarbons, saturated and/or unsaturated aliphatic or oxo-fatty alcohols, saturated and/or unsaturated fatty acids, hydrophobic amino acids, fat-soluble vitamins, steroid lipids, phospholipids, sphingomyelin, glycolipids, surfactants; wherein the number of carbon atoms is preferably 3-26.

The water-soluble moiety is a water-soluble molecule or a residue of the molecule containing one or more functional groups selected from the group consisting of a thiol group, amino group, phosphate group, carboxyl group, sulfonyl group, hydroxyl group, amine group, urea group, guanidine group, and disulfide group. The functional groups that play a binding role in the binding moiety (which can bind to microbial lipid membranes, microorganism surface proteins, microorganism surface polysaccharides, or cell wall components, or can bind to polysaccharides, proteins, or peptides in microorganisms) are one or two or more groups selected from the group consisting of a thiol group, amino group, phosphate group, carboxyl group, sulfonyl group, hydroxyl group, amine group, urea group, guanidine group, and disulfide group from the water-soluble moiety, or one or two or more groups selected from the group consisting of a thiol group, amino group, phosphate group, carboxyl group, sulfonyl group, hydroxyl group, amine group, urea group, guanidine group, and disulfide group that provides carbon chain to carbon chain connection.

In particular, for the complex of the invention, the water-soluble moiety is a water-soluble molecule or residue of the molecule, including macromolecular proteins, polysaccharides, nucleic acids, artificially synthesized water-soluble polymers, medium-molecular polypeptides, oligopeptides, oligosaccharides, oligonucleotides, artificially synthesized water-soluble medium-degree polymers, and small molecules including amino acids, mono-or disaccharides, nucleotides, and water-soluble vitamins. It can also be a functional group such as an amide group, phosphoryloxy group, carboxyl group, phosphate group, sulfonyl group, sulfonyloxy group, hydroxyl group, quaternary ammonium group, thioether group, disulfide bond, ether group, sulfhydryl group, aldehyde group, ester group, amine group, amino group, urea group, guanidine group, and the like directly bonded to a carbon chain to increase the water solubility. The water-soluble moiety can be a group linked to a carbon chain as the acting moiety and/or binding moiety.

The binding moiety is a molecule or a residue of the molecule (including a functional group on the molecule) that binds to a microbial lipid membrane, a microorganism surface protein, a microorganism surface polysaccharide, or a cell wall component, and can be a third moiety that forms a complex, can be the same as a water-soluble moiety, or can be linked to the acting moiety. When the binding moiety and the water-soluble moiety are the same moiety such as a protein, polypeptide, polysaccharide, etc. that can bind to a microbial lipid membrane, microorganism surface protein, microorganism surface polysaccharide, the water-soluble moiety contains one selected from the group consisting of a thiol group, amino group, urea group, guanidine group, carboxyl group, hydroxyl group and disulfide group. When the binding moiety is, in some cases, a dibasic or polybasic fatty acid, a fat-soluble amino acid, or the like, that can bind to a microbial lipid membrane, microorganism surface protein, or microorganism surface polysaccharide.

Without being limited by the mechanism of reaction, the acting moiety in the binding moiety that serves the binding function can be a carboxyl group, sulfonyl group, phosphate group, hydroxyl group, hydroxyl group of an aldehyde group or hemiacetal (saccharide), amino group, urea group, guanidine group, thiol group, and the like.
1. The following is a further illustration of fatty acids as carbon chain donors (i.e. acting moiety donors):
   (1) The fatty acids are insoluble in water or have extreme low water solubility and cannot be directly injected into the blood circulation. Direct injection into the vein can lead to pulmonary embolism, while injection into the artery can cause tissue necrosis due to arterial embolism.
   (2) Fatty acids are re-esterified in the intestinal cells and mixed with bile salts and monoglycerides to form 4-6 nm fat particles which are directly absorbed by the intestinal epithelial cells by pinocytosis and coat the outside with a lecithin and protein membrane to become chylomicrons which enter the lymphatic system, pass through the lymphatic vessels and the thoracic duct and return to the blood circulation in the form of an oil-in-water emulsion. Most medium chain fatty acids, except for a small amount that exists in the surrounding blood for a short period of time, non covalently bind to serum proteins and quickly reach the liver through the portal vein system. In the liver, medium-chain fatty acids can rapidly pass through the mitochondrial bilayer membrane and are rapidly acylated under the action of capryloyl CoA, while hardly being synthesized into fat. The excess acetyl CoA produced by acylation undergoes various metabolic actions in the mitochondrial cytoplasm, most of which tend to synthesize ketone bodies.
   (3) Fatty acids covalently bound to large, medium and small molecules change fat-soluble fatty acids to water-soluble fatty acids and are not readily cleared and metabolised by the liver.
   (4) The highly water-soluble and high-affinity complex of the present invention has an anti-microbial effect, and can be used in nasal spray and dry powder inhalant, and further intravenous injection and oral dosage forms in addition to transdermal topical dosage form.
2. Complex of fatty acids with water-soluble amino acids, mono-or disaccharides, nucleotides, water-soluble vitamins

In this case, the carbon chain of the fatty acid is an acting moiety, a water-soluble amino acid, a mono-or disaccharide, a nucleotide, or a water-soluble vitamin is the water-soluble moiety, and then a binding moiety is linked to form a complex having an antimicrobial infection effect according to the present invention. The binding moiety can be selected from dibasic or polybasic fatty acid, amino acid, targeting protein, targeting peptide, and targeting polysaccharide. Wherein the binary fatty acid or poly-fatty acid and the fat-soluble amino acid are both a binding part and an action part; water-soluble amino acids, targeting proteins, targeting polypeptides and targeting polysaccharides are both the binding moiety and the water-soluble moiety.

In a specific embodiment, the complex of the present invention is selected from a complex formed by linking a fatty trienoic acids with 3-50 carbon atoms and a water-soluble amino acid, such as a complex formed by linking octadecatrienoic acids and aspartyl lysine as the compound shown below:

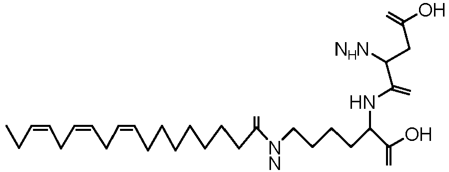

Where the carbon chain of octadecatrienoic acid is the acting moiety, and aspartyl lysine is both the water-soluble moiety and the binding moiety.

### 3. Complex of fatty acid with protein, polypeptide, and polysaccharide

In this case, the carbon chain of the fatty acid is the acting moiety that targets the protein or polypeptide of the virus surface domain, lipid membrane, or cell wall, and the polysaccharide is the binding moiety as well as the water-soluble moiety.

In a specific embodiment, the complex of the invention is selected from a complex formed by linking a fatty acid with 3-50 carbon atoms to a targeting polypeptide. For example, the formula shown below is a schematic representation of a complex formed by linking an octadecenoic acid to a targeting polypeptide, wherein the octadecenoic acid is linked to a lysine residue in the polypeptide via an amide bond.

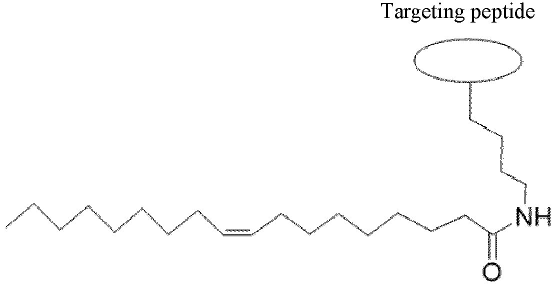

Where the carbon chain of octadecenoic acid is the acting moiety, the targeting polypeptide is both the water-soluble moiety and the binding moiety.

4. In the above three cases, if the water solubility of the complex is poor, or it is necessary to enlarge the molecule of the complex, a water-soluble high molecular polymer, such as fatty acid + targeting polypeptide + PEG, can be added, namely, the complex is formed by the reaction of a fatty acid with a carbon number of 3-50, a targeting polypeptide, and PEG.

In this case, the carbon chain of the fatty acid is the acting moiety, the targeting polypeptide is the binding moiety, and the PEG is the water-soluble moiety.

5. Fatty alcohol polyoxyethylene ethers, fatty acid polyoxyethylene esters, alkyl glycosides, fatty acid sucrose esters, sorbitan fatty acid esters, sorbitan polyoxyethylene fatty acid esters, mannose erythritol esters, N-fatty acyl-N-methylglucamine and other compounds have fatty alcohols or fatty acids as carbon chain donors, and have good water solubility. But the binding effect with virus surface domains, lipid membranes or cell wall components is weak, so it needs higher concentration to kill microorganisms. At this concentration, these compounds will also have destructive effect on human cells, and are not suitable for internal use in human body. When the above-mentioned compound is linked to the binding moiety to form a new complex, the compound has the effect of killing microorganisms at a lower concentration in the human body to play an anti-microbial infection effect. Also, at this therapeutic concentration, the new complex with the acting moiety + water-soluble moiety + binding moiety has no effect on human tissue cells and organs. The binding moiety can be selected from dibasic or polybasic fatty acid, amino acid, targeting protein, targeting peptide, and targeting polysaccharide. That is, in this case, the complex is formed by the reaction of a surfactant with one or two or more selected from the group consisting of dibasic or polybasic fatty acid, amino acids, targeting proteins, targeting peptides, and targeting polysaccharides.

In this case, the carbon chain of the fatty alcohol or fatty acid is the acting moiety, PEG, dextran, sucrose, sorbitan, mannose erythritol, or glucosamine is the water-soluble moiety, the linked dibasic or polybasic fatty acid and fat-soluble amino acid are both the binding moiety and acting moiety, and the water-soluble amino acid, targeting protein, targeting polypeptide or targeting polysaccharide is both the binding moiety and the water-soluble moiety.

Specifically, in a specific embodiment of the present invention, the present invention provides a group of complex for preventing and treating viral, bacterial and fungal infections, whose main structure is formed by an acting moiety, binding moiety, water-soluble moiety through coupling of a covalent bond, hydrogen bond, or Van Der Waals force;
The acting moiety imparts to the complex an action of disrupting a microbial lipid membrane or hydrophobically isolating a non-enveloped virus; the binding moiety imparts to the complex an action of binding to a lipid membrane or virus surface domain of the microorganism, and the specific binding moiety may also imparts to the complex an action of specifically targeting the microorganism; the water-soluble moiety imparts to the complex water solubility so that the complex can be uniformly dispersed in an aqueous solution and prevents the hydrophobic groups from agglomerating to form lipid droplets.

The lipid membrane refers to an envelope formed by a phospholipid bilayer of a microorganism.

The acting moiety, the binding moiety, and the water-soluble moiety in the complex can be a natural compound, an artificially synthesized compound, or a natural compound coupled to an artificially synthesized compound;
The number of groups of like moieties in the complex can be one or more. The arrangement and order of the groups are not fixed. Groups of the same or different type can be coupled linearly or in side chain mode.

The complex can be used to prevent and treat infectious diseases caused by viruses, bacteria, fungi, chlamydia, and mycoplasma.

Further, the acting moiety is a natural or synthetic hydrophobic group, including a straight carbon chain, a carbon chain with a branched chain, a carbon chain with a cyclic structure. The carbon chain can be a saturated/unsaturated carbon chain. The unsaturated carbon chain may carry one or more unsaturated bonds, wherein the unsaturated bonds can be double or triple bonds.

For microorganisms with lipid membrane lipid membrane structures, such as enveloped viruses, bacteria, fungi, chlamydia and mycoplasma, the acting moiety can penetrate, insert and blend into lipid membrane, destroy the structural stability of lipid membrane, and then destroy the integrity of lipid membrane and cell wall, so as to achieve the function of killing microorganisms.

For non-enveloped viruses, the binding moiety binds to a virus surface protein domain, and the acting moiety encapsulates on the surface of the non-enveloped virus, resulting in hydrophobic sequestration of the non-enveloped virus, which is then cleared by immune cells to prevent and treat non-enveloped virus infection.

Further, the binding moiety has one or more functional groups, such as carboxyl group, hydroxyl group, amino group, thiol group, urea group, guanidine group, that can bind to protein, polysaccharide or bindable domain of lipid membrane or virus surface, so that the complex is linked to lipid membrane or virus surface.

The binding moiety may also be designed to have a molecular structure that specifically targets lipid membrane, bacterial and fungal cell wall components, virus surface protein domains, thereby imparts to the complex viral, bacterial and fungal targeting functions.

The binding moiety can be the same group as the hydrophilic group, either binding to the lipid membrane, cell wall or virus surface protein domain, or imparts to the complex water solubility.

Still further, the binding moiety that can specifically target microbial lipid membranes, bacterial and fungal cell wall components, virus surface protein domains can be a protein, polypeptide, or polysaccharide, including:
(1) proteins that target coronavirus envelope including: neutralizing antibodies to Spike glycoprotein (S), small envelope glycoprotein (E), membrane glycoprotein (M), and hemagglutinin glycoprotein (HE), and amino acid sequences and small molecular polypeptides that specifically bind to the above protein domains;
(2) proteins that target the human immunodeficiency virus envelope including: neutralizing antibodies to gp120 and gp41 proteins, and amino acid sequences and small molecular polypeptides that specifically bind to the above protein domains;
(3) proteins that target hepatitis B virus envelope including: neutralizing antibodies to SHBs proteins, MHBs proteins and LHBs proteins, and amino acid sequences and small molecular polypeptides that specifically bind to the above protein domains;
(4) proteins that target hepatitis C virus envelope including: neutralizing antibodies to E1 and E2 proteins, and amino acid sequences and small molecular polypeptides that specifically bind to the above protein domains;
(5) proteins that target rabies virus envelope including: neutralizing antibodies to envelope glycoproteins, and amino acid sequences and small molecular polypeptides that can specifically bind to the above protein domains;
(6) proteins that target herpesvirus envelope including: neutralizing antibodies to gB, gC, gD, gE, gG, and gH glycoproteins, and amino acid sequences and small molecular polypeptides that specifically bind to the above protein domains;
(7) proteins that target Ebola virus envelope including neutralizing antibodies to envelope glycoproteins on the viral outer membrane, as well as amino acid sequences and small molecular polypeptides that specifically bind to the above protein domains;
(8) proteins that target Hantavirus envelope including: neutralizing antibodies to G1 and G2 glycoproteins, and amino acid sequences and small molecular polypeptides that specifically bind to the above protein domains;
(9) proteins that target Dengue virus envelope including: neutralizing antibodies to protein E and protein M, and amino acid sequences and small molecular polypeptides that specifically bind to the above protein domains;
(10) proteins that target Japanese encephalitis virus envelope including: neutralizing antibodies to glycoprotein E (i.e. viral hemagglutinin) and protein M, as well as amino acid sequences and small molecular polypeptide that specifically bind to the above protein domains;
(11) proteins that target influenza virus envelope including: neutralizing antibodies to hemagglutinin and neuraminidase, and amino acid sequences and small molecular polypeptides that specifically bind to the above protein domains;
(12) proteins that target Hepatitis A Virus capsid including: neutralizing antibodies to VP1, VP2, VP3, and VP4 proteins, and amino acid sequences and small molecule polypeptides that specifically bind to the above protein domains;
(13) proteins that target human papilloma virus capsid includes: neutralizing antibodies to L1 and L2 proteins, and amino acid sequences and small molecular polypeptides that specifically bind to the above protein domains;
(14) proteins that target adenoviral capsid including: neutralizing antibodies to P II, P III, P IIIa, P IV, P VI, P VIII, P IX proteins, and amino acid sequences and small molecular polypeptides specifically binding to the above protein domains;
(15) proteins that target poliovirus capsid including: neutralizing antibodies to VP1, VP2, VP3, and VP4 proteins, and amino acid sequences and small molecule polypeptides specifically binding to the above protein domains;
(16) proteins that target Coxsackie virus capsid including: neutralizing antibodies to VP1, VP2, VP3, and VP4 proteins, and amino acid sequences and small molecule polypeptides specifically binding to the above protein domains;
(17) proteins that target bacterial or fungal cell walls include: CD14 and amino acid sequences and small molecular polypeptides that specifically bind to its domain; and
(18) ligands targeting the cell walls of enveloped viruses, bacteria, or fungi designed for low affinity receptors such as heparan sulfate, proteoglycans, etc.

That is, for the purpose of the present invention, the basic structure of the complex of the present invention includes any one of the following compositions for the preventing, blocking or treating microbial infectious diseases:
A binding moiety + a water-soluble moiety + an acting moiety;
A binding moiety + an acting moiety + a water-soluble moiety;
A water-soluble moiety + an acting moiety + a binding moiety;
A water-soluble moiety + a binding moiety + an acting moiety;
A binding moiety + a water-soluble moiety + a binding moiety + an acting moiety +... + an XX moiety;
A binding moiety + a water-soluble moiety + an acting moiety + a water-soluble moiety +... + an XX moiety;
A binding moiety + a water-soluble moiety + an acting moiety + a binding moiety +... + an XX moiety;
A water-soluble moiety + binding moiety + acting moiety + binding moiety +... + an XX moiety; and
A water-soluble moiety + acting moiety + binding moiety + acting moiety +... + an XX moiety.

Wherein the term "XX moiety" refers to any one or more of a "water-soluble moiety", a "binding moiety", and an "acting moiety".

The number of like moieties in the complex can be one or more, and the arrangement and order of the moieties is not fixed.

Still further, in a more specific embodiment, the structure of the composite of the present invention includes the structure described below.

The small molecule water-soluble moiety/binding moiety is covalently bound to the acting moiety of the medium-short chain carbon chain, wherein the medium-short chain carbon chain includes a saturated or unsaturated straight chain carbon chain, a branched chain carbon chain, and a carbon chain with a cyclic structure, and can be a carbon chain with 3-10 carbon atoms. The small molecule water-soluble moiety/binding moiety can be a small molecule, a medium molecule and a large molecule with a carboxyl group/hydroxyl group, e.g.:
a complex formed by an amino acid or monosaccharide with carboxyl/hydroxyl groups, a nucleotide, and a carbon chain with 3-10 carbon atoms, which has a significantly improved water solubility, is well dispersed and does't agglomerate.
a complex formed by a macromolecular water-soluble moiety + a binding moiety + a long-chain carbon chain (more than 10 carbon atoms) acting moiety, the long-chain carbon chain comprising a saturated or unsaturated straight-chain carbon chain, a branched-chain carbon chain, and a carbon chain with a cyclic structure, such as a complex formed by the following structure:
   protein/targeting protein + binding moiety + saturated/unsaturated carbon chain with linear, branched or cyclic structure,
   targeting small molecules + proteins + binding moiety + saturated/unsaturated carbon chains with linear, branched, or cyclic structures,
   polysaccharide + binding moiety + saturated/unsaturated carbon chain with linear, branched, or cyclic structure,
   targeting small molecule + polysaccharide + binding moiety + saturated/unsaturated carbon chain with linear, branched or cyclic structure,
   water-soluble high molecular polymer + binding moiety + saturated/unsaturated carbon chain with linear, branched or cyclic structure, and
   targeting small molecule + water-soluble high molecular polymer + binding moiety + saturated/unsaturated carbon chain with linear, branched or cyclic structure.

Further, the complex is a derivative of the water-soluble moiety, the binding moiety, and the lipid covalently coupled, including the following construction modes:
macromolecular water-soluble moiety/binding moiety/targeting binding moiety + lipid,
macromolecular water-soluble moiety + binding moiety/targeting binding moiety + lipid,
Two or more small molecule water-soluble moieties/binding moieties/targeting binding moieties + lipid, and
Two or more small molecule water-soluble moieties + binding moiety/targeting binding moiety + lipids.

Still further, the lipids include fatty alcohols, fatty acids, phospholipids, fat-soluble vitamins and steroid lipids.

The fatty alcohols include one or more of saturated fatty alcohols, monounsaturated fatty alcohols, polyunsaturated fatty alcohols, and the derivatives of the fatty acids described above. The unsaturated bond in the carbon chain of the unsaturated fatty alcohol is a double bond or a triple bond. The carbon chain of the fatty alcohol can be a linear, branched, cyclic carbon chain.

The fatty acids include one or more of saturated fatty acids, monounsaturated fatty acids, di-or polyunsaturated fatty acids, and the derivatives of the fatty acids described above. The unsaturated bond in the carbon chain of the unsaturated fatty acid is a double bond or a triple bond. The carbon chain of the fatty acid can be a linear, branched, cyclic, hydroxyl-bearing carbon chain. The carboxyl group of the fatty acid can be one or more.

The phospholipids include one or more of glycerophospholipids and sphingomyelin, wherein glycerophospholipids include phosphatidylglycerol, phosphatidyl ethanolamine, phosphatidyl choline, phosphatidyl serine, phosphatidyl inositol, and the derivatives of the phospholipids described above.

The fat-soluble vitamins include one or more of vitamins A, D, E, and D, and derivatives of the fat-soluble vitamins described above.

The steroids include one or more of cholesterol, lanosterol, sitosterol, stigmasterol, ergosterol, bile acids, bile alcohols, and derivatives of the steroid lipidoids described above.

Further, the complex should ensure that the ratio of the molecular weight of the hydrophobic group to the molecular weight of the hydrophilic group is suitable. If the molecular weight of the hydrophilic group is much larger than that of the hydrophobic group, insertion of the hydrophobic group into the biological membrane will be hindered, so as to weaken the ability of the hydrophobic group to damage the biological membrane. If the molecular weight of the hydrophilic group is significantly smaller than that of the hydrophobic group, the hydrophobic group will be aggregated to form an oil-in-water structure, and the hydrophobic group will not contact the biological membrane and cannot play a damaging role.

Further, as the acting moiety of the present invention, it can be a carbon chain or carbon chain residue formed from a saturated and/or unsaturated aliphatic hydrocarbon, a saturated and/or unsaturated fatty alcohol, and a saturated and/or unsaturated fatty acid, wherein the number of carbon atoms is 3-48, more preferably 3-26; wherein the number of carbon atoms is preferably 3-26.

Among these, saturated and/or unsaturated fatty acids used to provide the above carbon chain or carbon chain residue specifically include those shown below.

The saturated fatty acids having 3-46 carbon atoms, including:
propionic acid, butyric acid, pentanoic acid, hexanoic acid, heptanoic acid, octanoic acid, nonanoic acid, decanoic acid, undecanoic acid, dodecanoic acid, tridecanoic acid, tetradecanoic acid, pentadecanoic acid, hexadecanoic acid, heptadecanoic acid, octadecanoic acid, nonadecanoic acid, eicosanoic acid, heneicosanoic acid, docosanoic acid, tricosanoic acid, tetracosanoic acid, pentacosanoic acid, hexacosanoic acid, heptacosanoic acid, octacosanoic acid, nonacosanoic acid, triacontanoic acid, hentriacontanoic acid, dotriacontanoic acid, tritriacontanoic acid, tetratriacontanoic acid, pentatriacontanoic acid, hexatriacontanoic acid, heptatriacontanoic acid, octatriacontanoic acid and hexatetracontanoic acid.

Monoenoic acids having 3-34 carbon atoms, including:
2-acrylic acid, 2-butenoic acid, 3-butenoic acid, 2-pentenoic acid, 3-pentenoic acid, 4-pentenoic acid, 2-hexenoic acid, 3-hexenoic acid, 4-hexenoic acid, 5-hexenoic acid, 2-heptenoic acid, 3-heptenoic acid, 4-heptenoic acid, 5-heptenoic acid, 6-heptenoic acid, 2-octenoic acid, 3-octenoic acid, 4-octenoic acid, 5-octenoic acid, 6-octenoic acid, 7-octenoic acid, 2-nonenoic acid, 3-nonenoic acid, 4-nonenoic acid, 6-nonenoic acid, 8-nonenoic acid, 2-decenoic acid, 3-decenoic acid, 4-decenoic acid, 5-decenoic acid, 6-decenoic acid, 8-decenoic acid, 9-decenoic acid, 2-undecenoic acid, 3-undecenoic acid, 4-undecenoic acid, 5-undecenoic acid, 6-undecenoic acid, 7-undecenoic acid, 8-undecenoic acid, 9-undecenoic acid, 10-undecenoic acid, 2-dodecenoic acid, 3-dodecenoic acid, 4-dodecenoic acid, 5-dodecenoic acid, 6-dodecenoic acid, 7-dodecenoic acid, 9-dodecenoic acid, 10-dodecenoic acid, 11-dodecenoic acid, 2-tridecenoic acid, 7-tridecenoic acid, 8-tridecenoic acid, 11-tridecenoic acid, 12-tridecenoic acid, 2-tetradecenoic acid, 3-tetradecenoic acid, 4-tetradecenoic acid, 5-tetradecenoic acid, 7-tetradecenoic acid, 8-tetradecenoic acid, 9-tetradecenoic acid, 11-tetradecenoic acid, 2-pentadecenoic acid, 6-pentadecenoic acid, 7-pentadecenoic acid, 9-pentadecenoic acid, 10-pentadecenoic acid, 14-pentadecenoic acid, 2-hexadecenoic acid, 3-hexadecenoic acid, 4-hexadecenoic acid, 5-hexadecenoic acid, 6-hexadecenoic acid, 7-hexadecenoic acid, 9-hexadecenoic acid, 10-hexadecenoic acid, 11-hexadecenoic acid, 13-hexadecenoic acid, 2-heptadecenoic acid, 3-heptadecenoic acid, 7-heptadecenoic acid, 8-heptadecenoic acid, 9-heptadecenoic acid, 10-heptadecenoic acid, 11-heptadecenoic acid, 12-heptadecenoic acid, 16-heptadecenoic acid, 2-octadecenoic acid, 3-octadecenoic acid, 4-octadecenoic acid, 5-octadecenoic acid, 6-octadecenoic acid, 7-octadecenoic acid, 8-octadecenoic acid, 9-octadecenoic acid, 10-octadecenoic acid, 11-octadecenoic acid, 12-octadecenoic acid, 13-octadecenoic acid, 14-octadecenoic acid, 15-octadecenoic acid, 16-octadecenoic acid, 17-octadecenoic acid, 2-nonadecenoic acid, 5-nonadecenoic acid, 6-nonadecenoic acid, 7-nonadecenoic acid, 9-nonadecenoic acid, 10-nonadecenoic acid, 11-nonadecenoic acid, 12-nonadecenoic acid, 13-nonadecenoic acid, 16-nonadecenoic acid, 3-eicosenoic acid, 5-eicosenoic acid, 6-eicosenoic acid, 7-eicosenoic acid, 8-eicosenoic acid, 9-eicosenoic acid, 10-eicosenoic acid, 11-eicosenoic acid, 13-eicosenoic acid, 14-eicosenoic acid, 15-eicosenoic acid, 16-eicosenoic acid, 7-uneicosenoic acid, 12-uneicosenoic acid, 5-docosenoic acid, 7-docosenoic acid, 9-docosenoic acid, 11-docosenoic acid, 13-docosenoic acid, 15-docosenoic acid, 19-docosenoic acid, 9-tricosenoic acid, 14-tricosenoic acid, 16-tricosenoic acid, 17-tricosenoic acid, 18-tricosenoic acid, 22-tricosenoic acid, 11-tetracosenoic acid, 15-tetracosenoic acid, 17-tetracosenoic acid, 5-pentacosenoic acid, 16-pentacosenoic acid, 17-pentacosenoic acid, 18-pentacosenoic acid, 19-pentacosenoic acid, 5-hexacosenoic acid, 9-hexacosenoic acid, 11-hexacosenoic acid, 14-hexacosenoic acid, 17-hexacosenoic acid, 19-hexacosenoic acid, 21-hexacosenoic acid, 18-heptacosenoic acid, 20-heptacosenoic acid, 9-octacosenoic acid, 11-octacosenoic acid, 19-octacosenoic acid, 21-octacosenoic acid, 23-octacosenoic acid, 20-nonacosenoic acid, 21-triacontenoic acid, 22-hentriacontenoic acid, 23-dotriacontenoic acid, and 25-tetratriacontenoic acid.

Dienoic acids having 5-30 carbon atoms, including:
2, 4-pentadienoic acid, 2, 4-hexadienoic acid, 3, 5-hexadienoic acid, 2, 7-octadienoic acid, 4, 7-octadienoic acid, 5, 7-octadienoic acid, 2, 4-nonadienoic acid, 2, 6-nonadienoic acid, 5, 7-nonadienoic acid, 2, 4-decadienoic acid, 2, 5-decadienoic acid, 2, 6-decadienoic acid, 2, 7-decadienoic acid, 3, 5-decadienoic acid, 4, 6-decadienoic acid, 4, 8-decadienoic acid, 4, 9-decadienoic acid, 5, 8-decadienoic acid, 5, 9-decadienoic acid, 6, 8-decadienoic acid, 7, 9-decadienoic acid, 2, 4-undecadienoic acid, 2, 4-dodecadienoic acid, 2, 6-dodecadienoic acid, 2, 8-dodecadienoic acid, 3, 6-dodecadienoic acid, 5, 7-dodecadienoic acid, 7, 9-dodecadienoic acid, 8, 10-dodecadienoic acid, 3, 5-tridecadienoic acid, 2, 4-tetradecadienoic acid, 3, 5-tetradecadienoic acid, 5, 8-tetradecadienoic acid, 6, 9-tetradecadienoic acid, 10, 12-tetradecadienoic acid, 2, 4-hexadecadienoic acid, 3, 9-hexadecadienoic acid, 4, 7-hexadecadienoic acid, 5, 9-hexadecadienoic acid, 6, 9-hexadecadienoic acid, 7, 10-hexadecadienoic acid, 8, 10-hexadecadienoic acid, 9, 12-hexadecadienoic acid, 10, 12-hexadecadienoic acid, 8, 11-heptadecadienoic acid, 9, 12-heptadecadienoic acid, 2, 4-octadecadienoic acid, 2, 5-octadecadienoic acid, 2, 6-octadecadienoic acid, 3, 6-octadecadienoic acid, 3, 7-octadecadienoic acid, 3, 12-octadecadienoic acid, 4, 7-octadecadienoic acid, 4, 8-octadecadienoic acid, 4, 9-octadecadienoic acid, 5, 8-octadecadienoic acid, 5, 9-octadecadienoic acid, 5, 10-octadecadienoic acid, 5, 11-octadecadienoic acid, 5, 12-octadecadienoic acid, 6, 8-octadecadienoic acid, 6, 9-octadecadienoic acid, 6, 10-octadecadienoic acid, 6, 11-octadecadienoic acid, 6, 12-octadecadienoic acid, 7, 9-octadecadienoic acid, 7, 10-octadecadienoic acid, 7, 11-octadecadienoic acid, 7, 12-octadecadienoic acid, 8, 10-octadecadienoic acid, 8, 11-octadecadienoic acid, 8, 12-octadecadienoic acid, 9, 11-octadecadienoic acid, 9, 12-octadecadienoic acid, 9, 13-octadecadienoic acid, 10, 12-octadecadienoic acid, 10, 14-octadecadienoic acid, 5, 9-nonadecadienoic acid, 10, 13-nonadecadienoic acid, 5, 9-eicosadienoic acid, 5, 11-eicosadienoic acid, 5, 13-eicosadienoic acid, 5, 15-eicosadienoic acid, 6, 9-eicosadienoic acid, 6, 11-eicosadienoic acid, 7, 11-eicosadienoic acid, 7, 13-eicosadienoic acid, 7, 14-eicosadienoic acid, 8, 11-eicosadienoic acid, 11, 13-eicosadienoic acid, 11, 14-eicosadienoic acid, 11, 15-eicosadienoic acid, 5, 14-heneicosadienoic acid, 5, 16-heneicosadienoic acid, 12, 15-heneicosadienoic acid, 5, 13-docosadienoic acid, 7, 15-docosadienoic acid, 13, 16-docosadienoic acid, 5, 9-tetracosadienoic acid, 15, 18-tetracosadienoic acid, 5, 9-hexacosadienoic acid, 17, 20-hexacosadienoic acid, 17, 21-hexacosadienoic acid, 9, 21-octacosadienoic acid, 19, 23-octacosadienoic acid, 5, 9-nonacosadienoic acid, 5, 9-triacontadienoic acid, and 9, 23-triacontadienoic acid.

Trienoic acids having 7-30 carbon atoms, including:
2, 4, 6-heptatriene, 2, 6, 8-decatriene, 4, 7, 10-hexadecatrienoic acid, 5, 8, 11-hexadecatrienoic acid, 6, 9, 12-hexadecatrienoic acid, 6, 10, 14-hexadecatrienoic acid, 7, 10, 13-hexadecatrienoic acid, 7, 11, 14-hexadecatrienoic acid, 9, 12, 15-hexadecatrienoic acid, 5, 9, 12-heptadecatrienoic acid, 2, 9, 12-octadecatrienoic acid, 3, 9, 12-octadecatrienoic acid, 5, 8, 11-octadecatrienoic acid, 5, 9, 12-octadecatrienoic acid, 6, 9, 12-octadecatrienoic acid, 6, 10, 14-octadecatrienoic acid, 7, 9, 12-octadecatrienoic acid, 8, 10, 12-octadecatrienoic acid, 9, 11, 13-octadecatrienoic acid, 9, 11, 14-octadecatrienoic acid, 9, 12, 14-octadecatrienoic acid, 9, 12, 15-octadecatrienoic acid, 10, 12, 14-octadecatrienoic acid, 10, 12, 15-octadecatrienoic acid, 11, 13, 15-octadecatrienoic acid, 2, 4, 8-eicosatrienoic acid, 3, 6, 9-eicosatrienoic acid, 5, 8, 11-eicosatrienoic acid, 5, 8, 14-eicosatrienoic acid, 5, 9, 14-eicosatrienoic acid, 5, 9, 12-eicosatrienoic acid, 5, 11, 14-eicosatrienoic acid, 5, 13, 16-eicosatrienoic acid, 7, 10, 13-eicosatrienoic acid, 7, 11, 14-eicosatrienoic acid, 8, 11, 14-eicosatrienoic acid, 8, 12, 14-eicosatrienoic acid, 9, 11, 14-eicosatrienoic acid, 11, 14, 17-eicosatrienoic acid, 5, 14, 17-heneicosatrienoic acid, 3, 9, 15-docosatrienoic acid, 5, 11, 17-docosatrienoic acid, 7, 10, 13-docosatrienoic acid, 8, 11, 14-docosatrienoic acid, 13, 16, 19-docosatrienoic acid, 15, 18, 21-tetracosatrienoic acid, 5, 9, 17-hexacosatrienoic acid, 5, 9, 19-hexacosatrienoic acid, 5, 9, 21-hexacosatrienoic acid, 5, 9, 20-heptacosatrienoic acid, 5, 9, 21-octacosatrienoic acid, 5, 9, 23-nonacosatrienoic acid, 5, 9, 23-triacontatrienoic acid, and 5, 9, 25-triacontatrienoic acid.

Tetraenoic acids having 12-38 carbon atoms, including:
2, 4, 8, 10-dodecatetraenoic acid, 2, 6, 8, 10-dodecatetraenoic acid, 2, 6, 8, 12-hexadecatetraenoic acid, 4, 7, 10, 13-hexadecatetraenoic acid, 4, 7, 11, 14-hexadecatetraenoic acid, 4, 8, 12, 16-hexadecatetraenoic acid, 6, 9, 12, 15-hexadecatetraenoic acid, 2, 4, 6, 11-octadecatetraenoic acid, 3, 9, 12, 15-octadecatetraenoic acid, 5, 8, 11, 14-octadecatetraenoic acid, 5, 9, 12, 15-octadecatetraenoic acid, 6, 9, 12, 15-octadecatetraenoic acid, 9, 11, 13, 15-octadecatetraenoic acid, 9, 12, 15, 17-octadecatetraenoic acid, 2, 8, 11, 14-eicosatetraenoic acid, 4, 7, 10, 13-eicosatetraenoic acid, 4, 8, 11, 14-eicosatetraenoic acid, 4, 8, 12, 16-eicosatetraenoic acid, 5, 8, 11, 14-eicosatetraenoic acid, 5, 11, 14, 17-eicosatetraenoic acid, 5, 13, 16, 19-eicosatetraenoic acid, 6, 10, 14, 18-eicosatetraenoic acid, 7, 11, 14, 17-eicosatetraenoic acid, 8, 11, 14, 17-eicosatetraenoic acid, 8, 11, 14, 18-eicosatetraenoic acid, 4, 7, 10, 13-docosatetraenoic acid, 7, 10, 13, 16-docosatetraenoic acid, 8, 12, 16, 19-docosatetraenoic acid, 2, 4, 6, 8-tetracosatetraenoic acid, 9, 12, 15, 18-tetracosatetraenoic acid, 11, 14, 17, 20-hexacosatetraenoic acid, 13, 16, 19, 22-octacosatetraenoic acid, 15, 18, 21, 24-triacontatetraenoic acid, 17, 20, 23, 26-dotriacontatetraenoic acid, 19, 22, 25, 28-tetratriacontatetraenoic acid, 21, 24, 27, 30-hexatriacontatetraenoic acid, and 23, 26, 29, and 32-octatriacontatetraenoic acid.

Pentenoic acids having 12-38 carbon atoms, including:
3, 5, 7, 9, 11-dodecapentaenoic acid, 5, 7, 9, 11, 13-tetradecapentaenoic acid, 3, 6, 9, 12, 15-octadecapentaenoic acid, 2, 5, 8, 11, 14-eicosapentaenoic acid, 4, 8, 12, 15, 18-eicosapentaenoic acid, 5, 7, 9, 14, 17-eicosapentaenoic acid, 5, 8, 11, 14, 16-eicosapentaenoic acid, 5, 8, 11, 14, 17-eicosapentaenoic acid (EPA), , 4, 7, 10, 13, 16-docosapentaenoic acid, 4, 8, 12, 15, 19-docosapentaenoic acid, 7, 10, 13, 16, 19-docosapentaenoic acid, 6, 9, 12, 15, 18-tetracosapentaenoic acid, 9, 12, 15, 18, 21-tetracosapentaenoic acid, 8, 11, 14, 17, 20-hexacosapentaenoic acid, 11, 14, 17, 20, 23-hexacosapentaenoic acid, 10, 13, 16, 19, 22-octacosapentaenoic acid, 13, 16, 19, 22, 25-octacosapentaenoic acid, 12, 15, 18, 21, 24-triacontapentaenoic acid, 15, 18, 21, 24, 27-triacontapentaenoic acid, 14, 17, 20, 23, 26-dotriacontapentaenoic acid, 17, 20, 23, 26, 29-dotriacontapentaenoic acid, 16, 19, 22, 25, 28-tetratriacontapentaenoic acid, 19, 22, 25, 28, 31-tetratriacontapentaenoic acid, 18, 21, 24, 27, 30-hexatriacontapentaenoic acid, 21, 24, 27, 30, 32-hexatriacontapentaenoic acid, 20, 23, 26, 29, 32-octatriacontapentaenoic acid, and 23, 26, 29, 32, 35-octatriacontapentaenoic acid.

Hexenoic acids having 22-38 carbon atoms, including:
4, 7, 10, 13, 16, 19-docosahexaenoic acid, 2, 4, 6, 8, 10, 12-tetracosahexaenoic acid, 4, 8, 12, 15, 19, 22-tetracosahexaenoic acid, 6, 9, 12, 15, 18, 21-tetracosahexaenoic acid (THA), 8, 11, 14, 17, 20, 23-hexacosahexaenoic acid, 10, 13, 16, 19, 22, 25-octacosahexaenoic acid, 12, 15, 18, 21, 24, 27-triacontahexaenoic acid, 14, 17, 20, 23, 26, 29-dotriacontahexaenoic acid, 16, 19, 22, 25, 28, 31-tetratriacontahexaenoic acid, 18, 21, 24, 27, 30, 32-hexatriacontahexaenoic acid, and 20, 23, 26, 29, 32, 35-octatriacontahexaenoic acid.

Alkynic acids having 6-22 carbon atoms, including:
3-hexynoic acid, 4-hexynoic acid, 5-hexynoic acid, 3-heptynoic acid, 4-heptynoic acid, 6-heptynoic acid, 2-octynoic acid, 7-octynoic acid, 2-nonynoic acid, 4-nonynoic acid, 5-nonynoic acid, 6-nonynoic acid, 7-nonynoic acid, 8-nonynoic acid, 3-decynoic acid, 4-decynoic acid, 5-decynoic acid, 6-decynoic acid, 7-decynoic acid, 8-decynoic acid, 9-decynoic acid, 2-undecynoic acid, 3-undecynoic acid, 4-undecynoic acid, 5-undecynoic acid, 6-undecynoic acid, 7-undecynoic acid, 8-undecynoic acid, 9-undecynoic acid, 10-undecynoic acid, 3-dodecynoic acid, 4-dodecynoic acid, 5-dodecynoic acid, 6-dodecynoic acid, 7-dodecynoic acid, 8-dodecynoic acid, 9-dodecynoic acid, 10-dodecynoic acid, 11-dodecynoic acid, 3-tridecynoic acid, 4-tridecynoic acid, 5-tridecynoic acid, 6-tridecynoic acid, 7-tridecynoic acid, 8-tridecynoic acid, 9-tridecynoic acid, 10-tridecynoic acid, 11-tridecynoic acid, 12-tridecynoic acid, 13-tridecynoic acid, 3-tetradecynoic acid, 4-tetradecynoic acid, 5-tetradecynoic acid, 6-tetradecynoic acid, 7-tetradecynic acid, 8-tetradecynic acid, 9-tetradecynic acid, 10-tetradecynic acid, 11-tetradecynic acid, 12-tetradecynic acid, 13-tetradecynic acid, 3-pentadecynic acid, 14-pentadecynic acid, 2-hexadecynic acid, 4-hexadecynic acid, 7-hexadecynic acid, 10-hexadecynic acid, 7-heptadecynic acid, 8-heptadecynic acid, 9-heptadecynic acid, 12-heptadecynic acid, 16-heptadecynic acid, 2-octadecynic acid, 3-octadecynic acid, 4-octadecynic acid, 5-octadecynic acid, 6-octadecynic acid, 7-octadecynic acid, 8-octadecynic acid, 9-octadecynoic acid, 10-octadecynoic acid, 11-octadecynic acid, 12-octadecynic acid, 13-octadecynic acid, 14-octadecynic acid, 15-octadecynic acid, 16-octadecynic acid, 17-octadecynic acid, 18-nonadecynic acid, and 13-docosynic acid.

Dialkynoic acids having 10-22 carbon atoms include:
2, 4-decadiynoic acid, 5, 11-dodecadiynoic acid, 3, 9-hexadecadiynoic acid, 7, 10-hexadecadiynoic acid, 8, 10-hexadecadiynoic acid, 5, 8-heptadecadiynoic acid, 6, 9-heptadecadiynoic acid, 7, 10-heptadecadiynoic acid, 10, 16-heptadecadiynoic acid, 2, 5-octadecadiynoic acid, 2, 6-octadecadiynoic acid, 2, 7-octadecadiynoic acid, 3, 6-octadecadiynoic acid, 3, 7-octadecadiynoic acid, 3, 8-octadecadiynoic acid, 4, 6-octadecadiynoic acid, 4, 7-octadecadiynoic acid, 4, 8-octadecadiynoic acid, 4, 9-octadecadiynoic acid, 5, 7-octadecadiynoic acid, 5, 8-octadecadiynoic acid, 5, 9-octadecadiynoic acid, 5, 10-octadecadiynoic acid, 5, 12-octadecadiynoic acid, 6, 8-octadecadiynoic acid, 6, 9-octadecadiynoic acid, 6, 10-octadecadiynoic acid, 6, 11-octadecadiynoic acid, 6, 12-octadecadiynoic acid, 7, 9-octadecadiynoic acid, 7, 10-octadecadiynoic acid, 7, 11-octadecadiynoic acid, 7, 12-octadecadiynoic acid, 8, 10-octadecadiynoic acid, 8, 11-octadecadiynoic acid, 8, 12-octadecadiynoic acid, 9, 11-octadecadiynoic acid, 9, 12-octadecadiynoic acid, 9, 13-octadecadiynoic acid, 10, 12-octadecadiynoic acid, 10, 13-octadecadiynoic acid, 10, 14-octadecadiynoic acid, 11, 14-octadecadiynoic acid, 11, 15-octadecadiynoic acid, 12, 14-octadecadiynoic acid, 12, 15-octadecadiynoic acid, 12, 16-octadecadiynoic acid, 13, 16-octadecadiynoic acid, 13, 17-octadecadiynoic acid, 14, 17-octadecadiynoic acid, 10, 13-nonadecadiynoic acid, 7, 3-eicosadiynoic acid, 8, 11-eicosadiynoic acid, 10, 13-eicosadiynoic acid, 12, 14-pentacosadiynoic acid, and 12, 14-heptacosadiynoic acid.

Trialkynoic acids having 12-22 carbon atoms include:
5, 8, 11-dodecatriynoic acid, 9, 11, 13-pentadecatriynoic acid, 5, 8, 11-heptadecatriynoic acid, 5, 8, 11-octadecatriynoic acid, 6, 9, 12-octadecatriynoic acid, 8, 11, 14-octadecatriynoic acid, 8, 11, 14-nonadecatriynoic acid, 5, 8, 11-eicosatriynoic acid, 6, 9, 12-eicosatriynoic acid, 7, 10, 13-eicosatriynoic acid, 8, 11, 14-eicosatriynoic acid, 9, 12, 15-eicosatriynoic acid, 3, 9, 15-docosatriynoic acid, 8, 11, 14-docosatriynoic acid, and 10, 13, 16-docosatriynoic acid.

Alkynoic acids having 8-20 carbon atoms, preferably acids containing one or two C = C double bonds and one or two or three triple bonds, including:
10-en-8-heptadecynoic acid, 9-en-12-octadecynoic acid, 11-en-9-octadecynoic acid, 17-en-9-octadecynoic acid, 9, 12-dien-6-octadecynoic acid, 9, 14-dien-12-octadecynoic acid, 11, 13-dien-9-octadecynoic acid, 5, 8, 14-trien-11-eicosynoic acid, 5, 11, 14-trien-8-eicosynoic acid, 8, 11, 14-trien-5-eicosynoic acid, 6-en-2, 4-octadiynoic acid, 8-en-4, 6-decadiynoic acid, 2, 8-dien-4, 6-decadiynoic acid, 8-dien-4, 6-undecadiynoic acid, 10, 12-dien-4, 6-tetradecadiynoic acid, 5-en-7, 9-octadecadiynoic acid, 9-en-12, 14-octadecadiynoic acid, 13-en-9, 11-octadecadiynoic acid, 17-en-9, 11-octadecadiynoic acid, 13, 17-dien-9, 11-octadecadiynoic acid, 3-en-5, 7, 10-undecatriynoic acid, and 4-en-6, 8, 10-undecatriynoic acid.

Saturated fatty acids or fatty acids with C = C double bonds with 3-30 carbon atoms on the main chain, 1-10 alkyl and/or 1-3 hydroxyl groups on the branches, preferably 1-3 methyl groups, including:
2-methylpropanoic acid, 2-methylbutanoic acid, 3-methylbutanoic acid, 2, 2-dimethylpropanoic acid, 2-methyl-2-butenoic acid, 3-methyl-2-butenoic acid, 3-methyl-3-butenoic acid, 2-ethyl-2-propenoic acid, 2-methylpentanoic acid, 3-methylpentanoic acid, 3, 5-dihydroxy-3-methylpentanoic acid, 2-hydroxy-3-methylpentanoic acid, 2-ethylbutanoic acid, 2, 2-dimethylbutanoic acid, 3, 3-dimethylbutanoic acid, 3-methyl-2-pentenoic acid, 3-methyl-3-pentenoic acid, 3-methyl-4-pentenoic acid, 4-methyl-2-pentenoic acid, 4-methyl-3-pentenoic acid, 4-methyl-4-pentenoic acid, 2, 2-dimethyl-3-butenoic acid, 2, 3-dimethyl-2-butenoic acid, 2-methylhexanoic acid, 3-methylhexanoic acid, 4-methylhexanoic acid, 5-methylhexanoic acid, 2-ethylpentanoic acid, 2, 2-dimethylpentanoic acid, 3, 3-dimethylpentanoic acid, 3, 4-dimethylpentanoic acid, 4, 4-dimethylpentanoic acid, 2-ethyl-methylbutanoic acid, 2-methyl-2-hexenoic acid, 5-methyl-5-hexenoic acid, 2-butyl-2-propenoic acid, 2-isopropyl-2-butenoic acid, 3-isopropyl-3-butenoic acid, 2, 2-dimethyl-4-pentenoic acid, 4, 4-dimethyl-2-pentenoic acid, 2-methylheptanoic acid, 3-methylheptanoic acid, 5-methylheptanoic acid, 6-methylheptanoic acid, 2-ethylhexanoic acid, 2, 2-dimethyl-hexanoic acid, 2-ethyl-2-methyl-pentanoic acid, 2-methyl-2-heptenoic acid, 5-hydroxy-4-methyl-2-heptenoic acid, 6-methyl-5-heptenoic acid, 2-ethyl-3-hexenoic acid, 3-tert-butyl-3-butenoic acid, 2-methyloctanoic acid, 3-methyloctanoic acid, 4-methyloctanoic acid, 5-methyloctanoic acid, 6-methyloctanoic acid, 7-methyloctanoic acid, 2-isopropylhexanoic acid, 6, 6-dimethyl-heptanoic acid, 3, 5, 5-trimethylhexanoic acid, 6-methyl-5-octenoic acid, 2-pentyl-3-butenoic acid, 6-methyl-2, 4-octadienoic acid, 8-hydroxy-6-methyl-2, 4-octadienoic acid, 2-methylnonanoic acid, 3-methylnonanoic acid, 4-methylnonanoic acid, 7-methylnonanoic acid, 8-methylnonanoic acid, 3-methyl-2-nonenoic acid, 2, 7-dimethyl-6-octenoic acid, 3, 7-dimethyl-2-octenoic acid, 3, 7-dimethyl-6-octenoic acid, 3, 7-dimethyl-2, 6-octadienoic acid, 2-methyldecanoic acid, 3-methyldecanoic acid, 4-methyldecanoic acid, 5-methyldecanoic acid, 6-methyldecanoic acid, 7-methyldecanoic acid, 8-methyldecanoic acid, 9-methyldecanoic acid, 3, 3-dimethylnonanoic acid, 4, 8-dimethylnonanoic acid, 8, 8-dimethylnonanoic acid, 2, 7-dimethyl-6-nonenoic acid, 4-ethyl-2-methyl-2-octenoic acid, 2-methylundecanoic acid, 3-methylundecanoic acid, 4-methylundecanoic acid, 5-methylundecanoic acid, 6-methylundecanoic acid, 8-methylundecanoic acid, 9-methylundecanoic acid, 10-methylundecanoic acid, 4, 9-dimethyldecanoic acid, 5-methyl-2-undecenoic acid, 2-methyldodecanoic acid, 3-methyldodecanoic acid, 4-methyldodecanoic acid, 5-methyldodecanoic acid, 6-methyldodecanoic acid, 7-methyldodecanoic acid, 8-methyldodecanoic acid, 9-methyldodecanoic acid, 10-methyldodecanoic acid, 11-methyldodecanoic acid, 2, 6-dimethylundecanoic acid, 10, 10-dimethylundecanoic acid, 2-methyl-2-dodecenoic acid, 11-methyl-2-dodecenoic acid, 2-decyl-2-acrylic acid, 2-methyldodecanedioic acid, 3-methyldodecanedioic acid, 4-methyldodecanedioic acid, 6-methyldodecanedioic acid, 11-methyl-2, 5-dodecadienoic acid, 11-hydroxy-4-methyl-2, 4, 6-dodecatrienoic acid, 2-methyltridecanoic acid, 3-methyltridecanoic acid, 4-methyltridecanoic acid, 6-methyltridecanoic acid, 9-methyltridecanoic acid, 12-methyltridecanoic acid, 2, 4-dimethyldodecanoic acid, 2, 5-dimethyldodecanoic acid, 2, 6-dimethyldodecanoic acid, 4, 8-dimethyldodecanoic acid, 4, 10-dimethyldodecanoic acid, 4, 11-dimethyldodecanoic acid, 2, 6, 10-trimethylundecanoic acid, 5-methyl-2-tridecenoic acid, 2, 4-dimethyl-2-dodecenoic acid, 2-methyl-tridecanedioic acid, 3-methyl-tridecanedioic acid, 4-methyl-tridecanedioic acid, 2-methyltetradecanoic acid, 3-methyltetradecanoic acid, 11-methyltetradecanoic acid, 12-methyltetradecanoic acid, 13-methyltetradecanoic acid, 4, 12-dimethyltridecanoic acid, 12, 12-dimethyltridecanoic acid, 3, 7, 11-trimethyldodecanoic acid, 2, 5-dimethyl-2-tridecenoic acid, 3-methyltetradecanedioic acid, 5-methyltetradecanedioic acid, 3-methylpentadecanoic acid, 13-methylpentadecanoic acid, 14-methylpentadecanoic acid, 2-propyltridecanoic acid, 2-heptylnonanoic acid, 4-hexyldodecanoic acid, 6-ethyltetradecanoic acid, 2, 4-dimethyltetradecanoic acid, 2, 6-dimethyltetradecanoic acid, 2, 8-dimethyltetradecanoic acid, 2, 12-dimethyltetradecanoic acid, 2, 13-dimethyltetradecanoic acid, 3, 5-dimethyltetradecanoic acid, 4, 12-dimethyltetradecanoic acid, 4, 13-dimethyltetradecanoic acid, 10, 13-dimethyltetradecanoic acid, 13, 13-dimethyltetradecanoic acid, 2-ethyl-2-butyldecanoic acid, 3-ethyl-3-methyltridecanoic acid, 4, 8, 12-trimethyltridecanoic acid, 13-methyl-4-pentadecenoic acid, 14-methyl-4-pentadecenoic acid, 2-hexyl-2-decenoic acid, 2, 4-dimethyl-2-tetradecenoic acid, 6-isopentyl-9-methyl-5-decenoic acid, 2-methylhexadecanoic acid, 3-methylhexadecanoic acid, 4-methylhexadecanoic acid, 5-methylhexadecanoic acid, 6-methylhexadecanoic acid, 7-methylhexadecanoic acid, 8-methylhexadecanoic acid, 9-methylhexadecanoic acid, 10-methylhexadecanoic acid, 11-methylhexadecanoic acid, 12-methylhexadecanoic acid, 13-methylhexadecanoic acid, 14-methylhexadecanoic acid, 15-methylhexadecanoic acid, 2, 6-dimethyl-pentadecanoic acid, 4, 8-dimethyl-pentadecanoic acid, 8, 14-dimethyl-pentadecanoic acid, 9, 14-dimethyl-pentadecanoic acid, 7-methyl-6-hexadecenoic acid, 9-methyl-10-hexadecenoic acid, 10-methyl-9-hexadecenoic acid, 14-methyl-8-hexadecenoic acid, 15-methyl-6-hexadecenoic acid, 15-methyl-8-hexadecenoic acid, 15-methyl-9-hexadecenoic acid, 15-methyl-10-hexadecenoic acid, 15-methyl-11-hexadecenoic acid, 2-methylhexadecanedioic acid, 3-methylhexadecanedioic acid, 4-methylhexadecanedioic acid, 5-methylhexadecanedioic acid, 8-methylhexadecanedioic acid, 2-methylheptadecanoic acid, 10-methylheptadecanoic acid, 14-methylheptadecanoic acid, 15-methylheptadecanoic acid, 16-methylheptadecanoic acid, 3-hydroxy-16-methylheptadecanoic acid, 2, 6-dimethyl-hexadecanoic acid, 2, 14-dimethyl-hexadecanoic acid, 4, 8-dimethyl-hexadecanoic acid, 4, 14-dimethyl-hexadecanoic acid, 6, 14-dimethyl-hexadecanoic acid, 10, 15-dimethyl-hexadecanoic acid, 11, 15-dimethyl-hexadecanoic acid, 12, 15-dimethyl-hexadecanoic acid, 15, 15-dimethyl-hexadecanoic acid, 2-methyl-16-heptadecenoic acid, 7-methyl-12-heptadecenoic acid, 9-methyl-6-heptadecenoic acid, 15-methyl-4-heptadecenoic acid, 16-methyl-4-heptadecenoic acid, 16-methyl-6-heptadecenoic acid, 16-methyl-8-heptadecenoic acid, 8, 9-methylene-8-heptadecenoic acid, 16-methyl-6, 9-heptadecadienoic acid, 16-methyl-9, 12-heptadecadienoic acid, 4, 6-dimethyl-2, 4-hexadecadienoic acid, 5, 7-dimethyl-2, 4-hexadecadienoic acid, 16-methyl-6, 9, 12-heptadecatrienoic acid, 2-methyloctadecanoic acid, 3-methyloctadecanoic acid, 4-methyloctadecanoic acid, 5-methyloctadecanoic acid, 6-methyloctadecanoic acid, 7-methyloctadecanoic acid, 8-methyloctadecanoic acid, 9-methyloctadecanoic acid, 10-methyloctadecanoic acid, 11-methyloctadecanoic acid, 3-hydroxy-11-methyloctadecanoic acid, 11, 12-methylene-octadecanoic acid, 12-methyloctadecanoic acid, 13-methyloctadecanoic acid, 14-methyloctadecanoic acid, 15-methyloctadecanoic acid, 16-methyloctadecanoic acid, 17-methyloctadecanoic acid, 4, 14-dimethyl-heptadecanoic acid, 10, 16-dimethyl-heptadecanoic acid, 12, 16-dimethyl-heptadecanoic acid, 10-methyl-9-octadecenoic acid, 11-methyl-12-octadecenoic acid, 17-methyl-6-octadecenoic acid, 17-methyl-7-octadecenoic acid, 17-methyl-13-octadecenoic acid, 2, 5-dimethyl-2-heptadecenoic acid, 4-heptyl-2-methyl-2-undecenoic acid, 9, 10-methylene-9-octadecenoic acid, 16-methyl-5, 9-octadecadienoic acid, 17-methyl-5, 9-octadecadienoic acid, 16-methyl-5, 9, 12-octadecatrienoic acid, 11-methylnonadecanoic acid, 17-methylnonadecanoic acid, 18-methylnonadecanoic acid, 2, 11-dimethyl-octadecanoic acid, 2, 14-dimethyl-octadecanoic acid, 4, 14-dimethyl-octadecanoic acid, 6, 14-dimethyl-octadecanoic acid, 4, 16-dimethyl-octadecanoic acid, 6, 16-dimethyl-octadecanoic acid, 12, 17-dimethyl-octadecanoic acid, 6-methyl-9-nonadecenoic acid, 18-methyl-8, 11, 14-nonadecatrienoic acid, 18-methyl-5, 8, 11, 14-nonadecatetraenoic acid, 18-methyl eicosanoic acid, 19-methyl eicosanoic acid, 2, 6-dimethyl nonadecanoic acid, 12, 18-dimethyl nonadecanoic acid, 2-methyl-2-eicosenoic acid, 2-propyl-9-octadecenoic acid, 18-methyl-5, 9-eicosadienoic acid, 19-methyl-5, 9-eicosadienoic acid, 3-methyl heneicosanoic acid, 19-methyl heneicosanoic acid, 20-methyl heneicosanoic acid, 14, 19-dimethyl eicosanoic acid, 2, 4-dimethyl-2-eicosenoic acid, 7, 7-dimethyl-5, 8-eicosadienoic acid, 7, 7-dimethyl-5, 8, 11-eicosatrienoic acid, 10, 10-dimethyl-5, 8, 11-eicosatrienoic acid, 20-methyl docosanoic acid, 21-methyl docosanoic acid, 22-methyl tricosanoic acid, 21-methyl tricosanoic acid, 2, 4-dimethyl docosanoic acid, 3, 15-dimethyl docosanoic acid, 23-methyl tetracosanoic acid, 2, 4-dimethyl tricosanoic acid, 23-methyl-5, 9-tetracosadienoic acid, 3, 7, 11-trimethyl-2, 6-docosadienoic acid, 23-methyl pentacosanoic acid, 24-methyl pentacosanoic acid, 2, 4-dimethyltetracosanoic acid, 3, 13, 19-trimethyltricosanoic acid, 24-methylhexacosanoic acid, 2-methyl-2-hexacosenoic acid, 2, 4-dimethyl-2-pentacosenoic acid, 2, 4, 6-trimethyl-2-tetracosenoic acid, 9, 10-dimethyloctacosanoic acid, 28-methyltriacontanoic acid, 2, 4, 6-trimethyloctacosanoic acid, and 15, 16-dimethyltriacontanedioic acid.

Saturated linear and branched dicarboxylic and tricarboxylic acids having 3-38 carbon atoms, including:
malonic acid, succinic acid, 2-methyl malonic acid, glutaric acid, 2, 2-dimethyl malonic acid, 2-methyl succinic acid, 2-ethyl malonic acid, hexane diacid, 2, 2-dimethyl succinic acid, 2-methyl-glutaric acid, 3-methyl-glutaric acid, 2-hydroxyhexane diacid, 2, 3, 4, 5-tetrahydroxyhexane diacid, 3-hydroxymethyl-glutaric acid, heptanedioic acid, 3, 3-dimethyl glutaric acid, 3-methyl-hexane diacid, octanedioic acid, ninandioic acid, decanedioic acid, undecanedioic acid, dodecanedioic acid, tridecanedioic acid, tetradecanedioic acid, pentadecanedioic acid, hexadecanedioic acid, heptadecanedioic acid, octadecanedioic acid, 9, 10-dihydroxy-octadecanedioic acid, nonadecanedioic acid, eicosanedioic acid, heneicosanedioic acid, docosanedioic acid, tricosanedioic acid, tetracosanedioic acid, hexacosanedioic acid, heptacosanedioic acid, nonacosanedioic acid, triacontanedioic acid, and 13, 14-dimethyl-octacosanedioic acid.

Unsaturated linear or branched dicarboxylic and tricarboxylic acids having 4-18 carbon atoms (which may also be dicarboxylic or tricarboxylic acids containing hydroxyl or amino groups), including:
butenedioic acid, 2-hydroxy-2-butenedioic acid, 2-methyl-2-butenedioic acid, 2-methyl-2-pentendioic acid,
3-hexenedioic acid, 3-hydroxy-2, 4-hexadienedioic acid, 2-pentyl-4-tridecenedioic acid, 2-(2-octenyl)-1, 10-decanedioic acid, 2-(2, 5-octadienyl)-1, 10-decanedioic acid, 2-(2-pentenyl)-4-tridecene-1, 13-dioic acid, and 2-en-4-octadecyne-dioic acid.
Tricarboxylic acids having 4 carbon atoms and substituted with a hydroxyl group, including:
3-hydroxypropane-1, 2, 3-tricarboxylic acid, 2-hydroxybutane-1, 2, 3-tricarboxylic acid, 3-hydroxybutane-1, 2, 3-tricarboxylic acid, 1-butene-1, 2, 4-tricarboxylic acid, 1, 3-butadiene-1, 2, 4-tricarboxylic acid.

Saturated and/or unsaturated fatty acids useful in the present invention also include amino-, hydroxyl-, oxo-, and/or alkyl-substituted fatty acids as described below. In particular, it can be:
1) Amino fatty acids and fatty amido fatty acids: an amino-, hydroxyl-, oxo-and/or methyl-substituted carboxylic acid having 3-18 carbon atoms, comprising one or two or more amino fatty acids selected from the group consisting of:
   2-amino-3-hydroxy-propionic acid, 2, 3-diamino-propionic acid, 2-amino-butyric acid, 4-amino-butyric acid, 2-amino-3, 4-dihydroxybutyric acid, 2-methyl-2-amino-propionic acid, 2-methyl-3-amino-propionic acid, 3-methyl-3-amino-propionic acid, 2, 4-diamino-butyric acid, 2-amino-3-oxo-butyric acid, 2-amino-pentanoic acid, 4-amino-pentanoic acid, 5-amino-pentanoic acid, 2-amino-3-methyl-butyric acid, 2, 5-diamino-pentanoic acid, 2-amino-4-hydroxy-3-methylpentanoic acid, 2-amino-4-oxo-pentanoic acid, 2-oxo-5-amino-pentanoic acid, 4-oxo-5-amino-pentanoic acid, 2-amino-hexanoic acid, 6-amino-hexanoic acid, 2-amino-3-methyl-pentanoic acid, 2-amino-4-methyl-pentanoic acid, 3-oxo-5-amino-hexanoic acid, 2-amino-hexane diacid, 2-amino-3-oxo-hexane diacid, 2-amino-6-oxo-2, 4-hexadienoic acid, 2-amino-2, 4-hexadienedioic acid, 2-amino-heptanoic acid, 2, 6-diamino-heptanedioic acid, 2-amino-4, 5-dihydroxy-6-oxo-heptanoic acid, 2-amino-octanoic acid, 3-amino-octanoic acid, 8-amino-octanoic acid, 3-amino-nonanoic acid, 9-amino-nonanoic acid, 7, 8-diamino-nonanoic acid, 7-oxo-8-amino-nonanoic acid, 2-amino-decanoic acid, 3-amino-decanoic acid, 9-amino-decanoic acid, 10-amino-decanoic acid, 11-amino-undecanoic acid, 12-amino-dodecanoic acid, 2-amino-tridecanoic acid, 13-amino-tridecanoic acid, 2-amino-tetradecanoic acid, 2-amino-hexadecanoic acid, 2-amino-octadecanoic acid, and 12-amino-octadecanoic acid.
2) N-fatty acyl amino acids including N-fatty acyl amino acids having 6-30 carbon atoms as described below: N-hexadecanoyl-γ-aminobutyric acid, N-octadecanoyl-γ-aminobutyric acid, N-(9-octadecenoyl)-γ-aminobutyric acid, N-(5, 8, 11, 14-eicosatetraenoyl)-γ-aminobutyric acid, N-(12-hydroxy-5, 8, 10, 14-eicosatetraenoyl)-γ-aminobutyric acid, N-(15-hydroxy-5, 8, 11, 13-eicosatetraenoyl)-γ-aminobutyric acid, N-(4, 7, 10, 12, 16, 19-docosahexaenoyl)-γ-aminobutyric acid;
   N-(6-aminocaproyl)-6-aminohexanoic acid; N-hexadecanoylalanine, N-octadecanoylalanine, N-(9-octadecenoyl) alanine, N-(5, 8, 10, 14-eicosatetraenoyl) alanine, N-(12-hydroxy-5, 8, 10, 14-eicosatetraenoyl) alanine, N-(15-hydroxy-5, 8, 11, 13-eicosatetraenoyl) alanine; N-octadecanoylarginine; N-octadecanoyl asparagine, N-(9-octadecenoyl) asparagine, N-tetradecanoyl glutamine, N-hexadecanoyl glutamine, N-(9-hexadecenoyl) glutamine, N-octadecanoyl glutamine, N-(9-octadecenoyl) glutamine, N-(9, 12-octadecadienoyl) glutamine, N-(17-hydroxy-9, 12-octadecadienoyl) glutamine, N-(9, 12, 15-octadecatrienoyl) glutamine, N-(17-hydroxy-9, 12, 15-octadecatrienoyl) glutamine, N-(5, 8, 11, 14-eicosatetraenoyl) glutamine, N-(4, 7, 10, 12, 16, 19-docosahexaenoyl) glutamine; N-hexadecanoyl glutamic acid, N-octadecanoyl glutamic acid, N-(9-octadecenoyl) glutamic acid, N-(9, 12-octadecadienoyl) glutamic acid, N-(9, 12, 15-octadecatrienoyl) glutamic acid, N-(5, 8, 11, 14-eicosatetraenoyl) glutamic acid, and N-(4, 7, 10, 12, 16, 19-docosahexaenoyl) glutamic acid;
   N-dodecanoyl glycine, N-(3-hydroxy-tetradecanoyl) glycine, N-(14-methyl-3-(13-methyl-4-tetradecenoyloxy)-pentadecanoyl)-glycine, N-hexadecanoyl glycine, N-(3-hydroxy-hexadecanoyl) glycine, N-(3-hydroxy-9-hexadecenoyl) glycine, N-(15-methylhexadecanoyl) glycine, N-(3-hydroxy-15-methylhexadecanoyl) glycine, N-(2, 3, 4-trihydroxy-15-methylhexadecanoyl) glycine, N-octadecanoyl glycine, N-(9-octadecenoyl) glycine, N-(3-hydroxy-9-octadecenoyl) glycine, N-(5, 8, 11, 14-eicosatetraenoyl) glycine, N-(12-hydroxy-5, 8, 10, 14-eicosatetraenoyl) glycine, and N-(15-hydroxy-5, 8, 11, 13-eicosatetraenoyl) glycine;
   N-hexanoyl histidine, N-octanoyl histidine, N-decanoyl histidine, N-(3, 4-methylene-decanoyl) histidine, N-hexadecanoyl histidine, N-octadecanoyl histidine, N-(9-octadecenoyl) histidine, and N-(4, 7, 10, 12, 16, 19-docosahexaenoyl) histidine; N-hexadecanoyl isoleucine, N-(9-octadecenoyl) isoleucine, and N-(5, 8, 11, 14-eicosatetraenoyl) isoleucine;
   N-hexadecanoyl leucine, N-(9-octadecenoyl) leucine, and N-(5, 8, 11, 14-eicosatetraenoyl) leucine; α-N-dodecanoyl lysine, 6-N-dodecanoyl lysine, α-N-tetradecanoyl lysine, 6-N-tetradecanoyl lysine, α-N-(5-tetradecenoyl) lysine, 6-N-(5-tetradecenoyl) lysine, α-N-(5, 8-tetradecadienoyl) lysine, and 6-N-(5, 8-tetradecadienoyl) lysine; N-hexadecanoyl methionine, and N-(9-octadecenoyl) methionine; α-N-(3-hydroxy-13-methyl-tetradecanoyl) ornithine, α-N-(3-hydroxy-hexadecanoyl) ornithine, α-N-(3-hydroxy-14-methyl-pentadecanoyl) ornithine, and α-N-(3-hydroxyoctadecanoyl) ornithine; N-hexadecanoylphenylalanine, N-octadecanoylphenylalanine, N-(9-octadecenoyl) phenylalanine, and N-(4, 7, 10, 12, 16, 19-docosahexaenoyl) phenylalanine; N-hexadecanoyl proline, N-octadecanoyl proline, and N-(9-octadecenoyl) proline;
   N-hexadecanoylserine, N-octadecanoylserine, N-(9-octadecenoyl) serine, and N-(5, 8, 11, 14-eicosatetraenoyl) serine; N-hexadecanoyl taurine, N-heptadecanoyl taurine, N-octadecanoyl taurine, N-(9-octadecenoyl) taurine, N-(9, 12-octadecadienoyl) taurine, N-nonadecanoyl taurine, N-(9-nonadecenoyl) taurine, N-eicosanoyl taurine, N-(11-eicosenoyl) taurine, N-(5, 8, 11, 14-eicosatetraenoyl) taurine, N-(12-hydroxy-5, 8, 10, 14-eicosatetraenoyl) taurine, N-(15-hydroxy-5, 8, 11, 13-eicosatetraenoyl) taurine, N-heneicosanoyl taurine, N-docosanoyl taurine, N-(13-docosenoyl) taurine, N-tricosanoyl taurine, N-(14-tricosenoyl) taurine, N-tetracosanoyl taurine, N-(15-tetracosenoyl) taurine, N-pentacosanoyl taurine, and N-hexacosanoyl taurine; N-hexadecanoylthreonine, and N-(9-octadecenoyl) threonine; N-hexadecanoyl tryptophan, N-octadecanoyl tryptophan, and N-(9-octadecenoyl) tryptophan; N-dodecanoyl-6-methyl-tyrosine, N-hexadecanoyl tyrosine, N-hexadecanoyl-a, O-dimethyl tyrosine, N-octadecanoyl tyrosine, N-(9-octadecenoyl) tyrosine, N-(5, 8, 11, 14-eicosatetraenoyl) tyrosine, N-nonanoyl-2, 3-dehydro-tyrosine, N-decanoyl-2, 3-dehydro-tyrosine, N-(8-methylnonanoyl)-2, 3-dehydro-tyrosine, N-dodecanoyl-6-methyl-2, 3-dehydro-tyrosine, N-(2-dodecenoyl)-6-methyl-2, 3-dehydro-tyrosine, N-hexadecanoyl-α, O-dimethyltyrosine, N-octadecanoyl-O-methyl-2, 3-dehydro-tyrosine, and N-(9-octadecenoyl)-O-methyl-2, 3-dehydro-tyrosine; N-hexadecanoyl valine, N-octadecanoyl valine, and N-(9, 12-octadecadienoyl) valine.
3) Amino acids having two or more fatty acyl groups, including the following:
   N-(15-methyl-3-(12-methyltridecanoyloxy)-hexadecanoyl)-glycine, N-(15-methyl-3-(13-methyltetradecanoyloxy)-hexadecanoyl)-glycine, N-(15-methyl-3-(13-methyl-4-tetradecenoyloxy)-hexadecanoyl)-glycine, and N-(15-methyl-3-(13-methyl-tetradecanoyloxy)-hexadecanoyl) glycine;
   α-N-(3-hexadecanoyloxy-hexadecanoyl) ornithine, α-N-(3-octadecanoyloxy-octadecanoyl) ornithine, α-N-(3-(3-hydroxyoctadecanoyloxy)-octadecanoyl) ornithine, α-N-(3-(11, 12-methylene) octadecanoyloxy-hexadecanoyl) ornithine, α-N-(3-(11, 12-methylene) octadecanoyloxy-octadecanoyl) ornithine, and α-N-(3-(3-hydroxy-(11, 12-methylene) octadecanoyloxy)-octadecanoyl) ornithine;
   N-(3-oxo-decanoyl)-leucyl) alanine, N-((3-(13-methyl-tetradecanoyloxy)-13-methyl-hexadecanoyl) glycyl) serine, N-((15-methyl-3-(13-methyltetradecyloxy)-hexadecanoyl)-glycyl)-serine, N-(((3-hydroxy-15-methyl-hexadecanoyl)-glycyl)-seryl)-omithine, N-((3-hydroxy-13-methyl-hexadecanoyl)-glycyl)-serine, and N-((9-octadecenoyl)-β-alanyl)-L-histidine.
4) A plurality of acids linked by thioether and amide bonds, including the following:
   11, 15-dihydroxy-14-(S-cysteinyl-glycyl)-5, 8, 12-eicosatrienoic acid, and 11, 15-dihydroxy-14-(S-glutathione)-5, 8, 12-eicosatrienoic acid.

Among these, the saturated and/or unsaturated fatty alcohols used to provide the above-mentioned carbon chain or carbon chain residue specifically include the fatty alcohols shown below.

Saturated astraight or branched chain fatty alcohols having 3-33 carbon atoms and 1-3 hydroxyl groups, including:
propan-1-ol, butan-1-ol, 2-methylpropan-1-ol, pentan-1-ol, 2-methylbutan-1-ol, 3-methylbutan-1-ol, hexan-1-ol, hexan-2-ol, hexan-3-ol, hexan-1, 5-diol, 3-methylpentan-1-ol, 3-methylpentan-3-ol, 4-methylpentan-1-ol, 1-methyl-cyclopentan-1-ol, heptan-1-ol, heptan-2-ol, heptan-3-ol, heptan-4-ol, 3-methylhexan-2-ol, 4-methylhexan-3-ol, 5-methylhexan-3-ol, heptan-1, 2, 3-triol, octan-1-ol, octan-2-ol, octan-3-ol, octan-1, 2-diol, octan-1, 3-diol, octan-1, 8-diol, 2-methylheptan-4-ol, 3-methylheptan-2-ol, 4-methylheptan-2-ol, 4-methylheptan-3-ol, nonan-1-ol, nonan-2-ol, nonan-3-ol, nonan-5-ol, 2-methyloctan-4-ol, 3-methyloctan-4-ol, 4-methyloctan-1-ol, 5-methyloctan-4-ol, 6-methyloctan-3-ol, 3, 5, 5-trimethylhexan-1-ol, decan-1-ol, decan-2-ol, decan-3-ol, 4-methylnonan-1-ol, 4-methylnonan-5-ol, 6-methylnonan-3-ol, 3, 7-dimethyloctan-1-ol, 3, 7-dimethyloctan-1, 7-diol, undecan-1-ol, undecan-2-ol, undecan-3-ol, dodecan-1-ol, tridecan-1-ol, tridecan-2-ol, 4-methyl-dodecan-7-ol, 10-methyl-dodecan-1-ol, tetradecan-1-ol, 4-methyl-tridecan-7-ol, 3, 9-dimethyl-dodecan-6-ol, 2, 2, 10-trimethyl-undecane-1, 10-diol, pentadecan-1-ol, pentadecan-2-ol, 4-methyl-tetradecan-7-ol, 3, 7-dimethyltridecan-2-ol, 4, 10-dimethyltridecan-7-ol, hexadecan-1-ol, 14-methyl-pentadecan-1-ol, 3, 7-dimethyltetradecan-2-ol, heptadecan-2-ol, 4-methyl-hexadecan-7-ol, 3, 7-dimethylpentadecan-2-ol, 6, 10, 13-trimethyl-tetradecan-1-ol, 2-methyl-hexadecan-1, 2-diol, heptadecan-1, 17-diol, octadecan-1-ol, 3, 7-dimethylhexadecan-2-ol, 2-methyl-heptadecan-1, 2-diol, 3-methyl-heptadecan-1, 2-diol, 11-methyl-heptadecan-1, 2-diol, nonadecan-1, 2-diol, nonadecan-1, 2, 4-triol, 2-methyl-octadecan-1, 2-diol, eicosan-1-ol, eicosan-1, 2-diol, eicosan-1, 3-diol, eicosan-1, 20-diol, 13-methyl-eicosan-1, 2-diol, heneicosan-1, 2-diol, heneicosan-1, 21-diol, 15-methyl-heneicosan-1, 2-diol, docosan-1-ol, docosan-1, 2-diol, docosan-1, 3-diol, 15-methyl-docosan-1, 2-diol, tricosan-12-ol, tricosan-1, 2-diol, tetracosan-1-ol, tetracosane-1, 2-diol, tetracosane-1, 3-diol, tetracosane-1, 24-diol, hexacosane-1-ol, hexacosane-1, 26-diol, 23-hexacosen-1-ol, heptacosane-1-ol, heptacosane-14-ol, heptacosane-6, 8-diol, octacosane-1-ol, octacosane-1, 28-diol, nonacosane-1-ol, nonacosane-10-ol, nonacosane-15-ol, nonacosane-6, 8-diol, triacontan-1-ol, triacontan-1, 11-diol, triacontane-1, 14-diol, dotriacontane-1-ol, tritriacontane-1-ol, and tetratriacontane-1-ol.

Unsaturated linear or branched fatty alcohols having 3-33 carbon atoms, 1-5 double bonds, 1-5 triple bonds, and 1-3 hydroxyl groups, including:
2-penten-1-ol, 2-methylbutan-1-ol, 2-methyl-2-buten-1-ol, 2-methyl-3-buten-1-ol, 2-hexen-1-ol, 3-hexen-1-ol, 3-hexen-3-ol, 4-hexen-1-ol, 4-hepten-1-ol, 4-hepten-2-ol, 2, 4-heptadien-1-ol, 6-methylheptan-3-ol, 2, 4-dimethyl-hexan-1-ol, 2-ethylhexan-1-ol, 1-octen-3-ol, 2-octen-1-ol, 3-octen-1-ol, 3-octen-2-ol, 5-octen-1-ol, 7-octen-2-ol, 4-methyl-4-hepten-3-ol, 6-methyl-2-hepten-4-ol, 6-methyl-5-hepten-2-ol, 5-octen-1, 3-diol, 1, 5-octadien-3-ol, 9, 12-octadien-1-ol, 2, 4-dimethyl-2, 4-hexadien-1-ol, 2, 4, 6-octatriyn-1-ol, 1-nonen-3-ol, 2-nonen-1-ol, 3-nonen-1-ol, 6-nonen-1-ol, 6-nonen-2-ol, 2, 4-dimethyl-5-hepten-1-ol, 2, 6-dimethyl-5-hepten-1-ol, 2, 6-dimethyl-6-hepten-1-ol, 2, 4-nonadien-1-ol, 3, 6-nonadien-1-ol, 6.8-nonadien-2-ol, 2, 4-dimethyl-2, 4-heptadien-1-ol, 1-decen-3-ol, 2-decen-1-ol, 3-decen-1-ol, 4-decen-1-ol, 5-decen-1-ol, 7-decen-1-ol, 7-methyl-6-nonen-3-ol, 2, 6-dimethyl-6-octen-2-ol, 2, 6-dimethyl-7-octen-2, 3, 6-triol, 7-methyl-3-methylene-octan-1, 6, 7-triol, 2, 4-decadien-1-ol, 7, 9-decadien-1-ol, 3, 7-dimethyl-3, 6-octadien-1-ol, 4, 6-decadiyn-1-ol, 2, 6-dimethyl-2, 7-octadien-1, 6-diol, 2-methyl-6-methylene-2, 7-octadien-1-ol, 2-en-4, 6, 8-decatriyn-1-ol, 1-undecen-3-ol, 2-undecen-1-ol, 6-undecen-2-ol, 10-undecen-1-ol, 3-methyl-4-decen-1-ol, 3, 4, 7-trimethyl-2, 6-octadien-1-ol, dodecan-2-ol, 2-butyloctan-1-ol, 3-dodecen-1-ol, 5-dodecen-1-ol, 6-dodecen-1-ol, 7-dodecen-1-ol, 8-dodecen-1-ol, 9-dodecen-1-ol, 10-dodecen-1-ol, 11-dodecen-1-ol, 3, 5-dodecadien-1-ol, 3, 6-dodecadien-1-ol, 5, 7-dodecadien-1-ol, 7, 9-dodecadien-1-ol, 8, 10-dodecadien-1-ol, 9, 11-dodecadien-1-ol, 3, 4, 7-trimethyl-2, 6-nonadien-1-ol, 3, 6, 8-dodecatrien-1-ol, 3, 6, 9-dodecatrien-1-ol, 6-tridecen-2-ol, 10-tridecen-2-ol, 11-ene-3, 5, 7, 9-tridecatetrayn-1-ol, 3-tetradecen-1-ol, 5-tetradecen-1-ol, 7-tetradecen-1-ol, 8-tetradecen-1-ol, 9-tetradecen-1-ol, 11-tetradecen-1-ol, 9-(2-cyclopentenyl)-1-ol, 8, 10-tetradecadien-1-ol, 9, 11-tetradecadien-1-ol, 9, 12-tetradecadien-1-ol, 10, 12-tetradecadien-1-ol, 11, 13-tetradecadien-1-ol, 3-methyl-6-(1-methyl-ethyl)-3, 9-decadien-1-ol, 13-en-2, 4-tetradecadien-1-ol, 13-en-1, 3-tetradecadiyn-6, 7-diol, 9-pentadecen-1-ol, 5, 10-pentadecadien-1-ol, 8, 10-pentadecadien-1-ol, 3, 7, 11-trimethyl-6, 10-dodecadien-1-ol, 7-hexadecen-1-ol, 9-hexadecen-1-ol, 11-hexadecen-1-ol, 4, 6-hexadecadien-1-ol, 6, 11-hexadecadien-1-ol, 7, 11-hexadecadien-1-ol, 10, 12-hexadecadien-1-ol, 11, 13-hexadecadien-1-ol, 10-propyl-5, 9-tridecadien-1-ol, 13-en-11-hexadecyn-1-ol, 4, 6, 10-hexadecatrien-1-ol, 8-heptadecen-2-ol, 11-heptadecen-1-ol, 14-methyl-8-hexadecen-1-ol, 16-heptadecen-1, 2, 4-triol, 16-heptadecyn-1, 2, 4-triol, 2, 6, 8, 12-tetramethyl-2, 4-tridecadien-1-ol, 4, 6-heptadecadiyn-3, 9, 10-triol, 1-en-4, 6-heptadecadiyn-3, 9-diol, 1-en-4, 6-heptadecadiyn-3, 9, 10-triol, 1, 9-diene-4, 6-heptadecadiyn-3-ol, 1, 8-diene-4, 6-heptadecadiyn-3, 10-diol, 1, 9-diene-4, 6-heptadecadiyn-3, 8-diol, 2, 9-diene-4, 6-heptadecadiyn-1, 8-diol, 1, 16-diene-4, 6-heptadecadiyn-3, 9, 10-triol, 1, 9, 16-triene-4, 6-heptadecadiyne-3, 8-diol, 9-octadecen-1-ol, 11-octadecen-1-ol, 13-octadecen-1-ol, 2, 13-octadecadien-1-ol, 3, 13-octadecadien-1-ol, 9, 12-octadecadien-1-ol, 9, 12, 15-octadecatrien-1-ol, 11-eicosen-1-ol, 15-eicosen-1-ol, 6, 9-eicosadien-11-ol, 3, 7, 11, 15-tetramethyl-6, 10, 14-eicosatrien-1-ol, 6-heneicosen-11-ol, 6, 9-heneicosadien-11-ol, and 3, 7, 11, 15, 19-pentamethyl-2, 6, 10, 14, 18-eicosapentaen-1-ol.

Oxo fatty alcohols (alcohol ketones having 8-31 carbon atoms, 1-3 double or triple bonds, and 1-3 hydroxyl groups, the ketone being a mono-or di-ketone), including:
1-hydroxyoctan-3-one, 3-hydroxymethylheptan-2-one, 6-methyl-7-hydroxy-3, 5-heptadien-1-one, 6-methyl-7-hydroxy-3, 5-heptadien-2-one, 1-hydroxy-nonan-3-one, 1-hydroxy-nonan-6-one, 3-hydroxymethyloctan-2-one, 1, 3-dihydroxy-8-decen-5-one, 1-hydroxy-5-phenyl-pentan-3-one, 3-hydroxypentadecan-4-one, 1-(furan-3-yl)-6-hydroxy-4, 8-dimethyl-1-one, 1-hydroxy-2, 12, 15-heneicosatrien-4-one, 2-(12-hydroxy-5, 10-dodecadiyn-1-yl)-3, 5, 6-trimethyl-2, 5-cyclohexadiene-1, 4-dione, and 25-hydroxy-hentriacontane-14, 16-dione.

Further, the water-soluble moiety is a natural or artificial compound having a carboxyl group, a sulfonyl group, a sulfonyloxy group, a phosphate group, a hydroxyl group, an amino group, a urea group, a guanidine group, a quaternary ammonium group, a sulfhydryl group structure, including proteins, polypeptides, nucleic acids, polysaccharides, and high molecular compounds having the above structures, such as:
Water-soluble macromolecules: water-soluble proteins such as serum albumins, immunoglobulins, water-soluble collagens, chaperones, water-soluble glycoproteinss, dextran (D-glucoside), hyaluronic acid, sialic acid, heparin sulfate, heparan sulfate, chondroitin sulfate, dermatan sulfate, keratin sulfate, water-soluble cellulose derivatives, β-cyclodextrin and its derivatives, water-soluble chitosan derivatives, polyethylene glycol and carboxylated or aminated polyethylene glycol, polyvinyl alcohol and carboxylated or quaternized polyvinyl alcohol, polyacrylic acid, and ammonium polyacrylate;
water-soluble medium molecules: one or more medium molecules of peptides, oligopeptides, water-soluble polyamines (polymers formed by polymerization of the same amino acid), oligosaccharides, oligonucleotides, and artificially synthesized polymers with moderate water solubility; and
Water-soluble small molecules: including mono-or disaccharides, amino acids, nucleotides and vitamins;
The above-mentioned water-soluble molecules can impart hydrophobic properties to partially dissolve and uniformly disperse in aqueous solutions, while avoiding the aggregation of hydrophobic structures into clusters.

Further in a preferred embodiment, the water-soluble macromolecule can be a water-soluble protein such as serum albumins, immunoglobulins, water-soluble collagens, chaperones, water-soluble glycoproteins, and CD14.

Further in a preferred embodiment, the water-soluble macromolecule can be a water-soluble polysaccharide such as dextran (D-glucoside), hyaluronic acid, sialic acid, heparin sulfate, heparan sulfate, chondroitin sulfate, dermatan sulfate, keratin sulfate, acetyl water-soluble cellulose derivatives, β-cyclodextrin and its derivatives, and water-soluble chitosan derivatives;
In addition, the water-soluble macromolecule may also be a water-soluble high molecular polymer such as polyethylene glycol and carboxylated or aminated polyethylene glycol, polyvinyl alcohol and carboxylated or quaternized polyvinyl alcohol, polyacrylic acid, and ammonium polyacrylate.

Further in a preferred embodiment, the medium-sized water-soluble molecule (abbreviated as "water-soluble medium molecule") can be a targeting polypeptide including proteins or neutralizing antibody fragments specifically targeting microbial lipid membranes, bacterial and fungal cell walls, virus surface protein domains, such as the taurine transporter peptide and SBP1; water-soluble polyamino acids such as polyglutamic acid, polylysine, polyaspartic acid; and oligopeptides, oligosaccharides, and oligonucleotides.

Further in a preferred embodiment, as water-soluble small molecules, they can be mono-and disaccharides such as glucose, fructose, rhamnose, sorbose, sucrose, maltose, lactose, and trehalose;
nucleotides and deoxynucleotides such as adenosine monophosphate, guanosine monophosphate, uridine monophosphate, cytidine monophosphate, thymidine monophosphate, inosine monophosphate, deoxyadenosine monophosphate, deoxyguanosine monophosphate, deoxycytidine monophosphate, deoxythymidine monophosphate;
amino acids such as serine, threonine, cysteine, asparagine, glutamine, tyrosine, lysine, arginine, histidine, aspartic acid, glutamic acid, citrulline, ornithine, taurine, and aminobutyric acid; and
vitamins such as vitamin B1, pantothenic acid, vitamin B6, and vitamin C.

Further, for the complexes of the invention, the coupling between the acting moiety, the binding moiety, and the water-soluble moiety includes the following manners.
(1) coupling by hydrogen bond and intermolecular force;
(2) coupling with amide bond, ester bond, hydrazone bond, and thioether bond;

Further specifically, the complex for preventing, blocking, or treating microbial infections of the present invention is a compound obtained by a reacting saturated and/or unsaturated fatty acid with 3-100 carbon atoms with a protein, peptide, oligopeptide, oligosaccharide, monosaccharide, and/or polysaccharide molecule; or it is a mixture of the compound obtained by the reaction of a saturated and/or unsaturated fatty acid with 3-50 carbon atoms with a protein, peptide, oligopeptide, oligosaccharide, monosaccharide, disaccharide, oligonucleotide, vitamin, water-soluble polymer, water-soluble polyamino acid, and/or polysaccharide molecule, as well as unreacted fatty acid, and/or unreacted protein, peptide, oligopeptide, oligosaccharide, monosaccharide, disaccharide, oligonucleotide, vitamin, water-soluble polymer, water-soluble polyamino acid, and/or polysaccharide molecule.

Further specifically, the complex for preventing, blocking, or treating microbial infections of the present invention is a complex obtained by directly physical compounding a saturated and/or unsaturated fatty acid with 3-100 carbon atoms and a protein, polypeptide, oligopeptide, oligosaccharide, monosaccharide, disaccharide, nucleotide, vitamin, water-soluble polymer, water-soluble polyamino acid, and/or polysaccharide molecule, in a physicochemical action including hydrogen bond or van der Waals force or a combination thereof, or a mixture obtained by directly physical mixing the same.

For example, a carboxyl group in the acting group reacts with an amino group in a lysine residue in a water-soluble protein or polypeptide to form an amide, and the reaction is represented by the following equation; 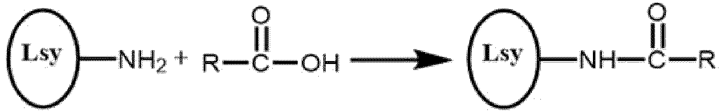 where R is a straight or branched, saturated or unsaturated fatty acid with 3-100 carbon atoms.

Terminal amino groups in water-soluble proteins and polypeptides react with carboxyl groups of fatty acids to form amide bonds: 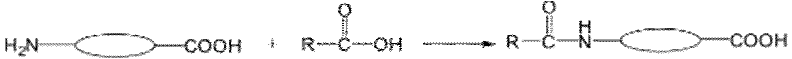 where R is a straight or branched, saturated or unsaturated fatty acid with 3-100 carbon atoms.

In particular, in a preferred embodiment, the protein is exemplified by human serum albumin (HSA), the polypeptide is exemplified by SBP1, and the carbon chain donor is exemplified by docosahexaenoic acid (DHA), eicosapentaenoic acid (EPA), octadecatrienoic acid (linolenic acid), octadecadienoic acid (linoleic acid), octadecenoic acid (oleic acid), octanoic acid, and butenedioic acid (fumaric acid) in order to form the acting moiety. Preferred complexes of the invention are exemplified below:
(1) amidated complexes of reaction products of lysine side chains in albumin (HSA) or SBP1 molecules with fatty acids:

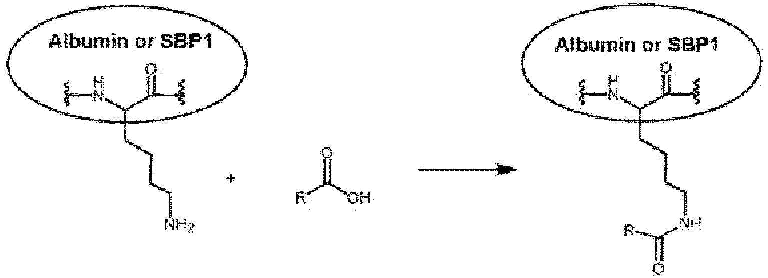

| | |
|---|---|
| 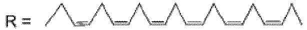 | Carbon chain of docosahexaenoic acid |
| 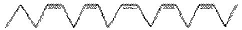 | Carbon chain of eicosapentaenoic acid |
| 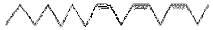 | Octadecatrienoic acid |
| 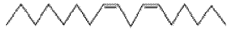 | Carbon chain of octadecadienoic acid |
| 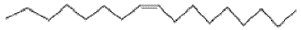 | Octadecenoic acid |
| 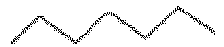 | Carbon chain of octanoic acid |
| 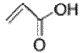 | Carbon chain of fumaric acid |

the reaction products are illustrated below: 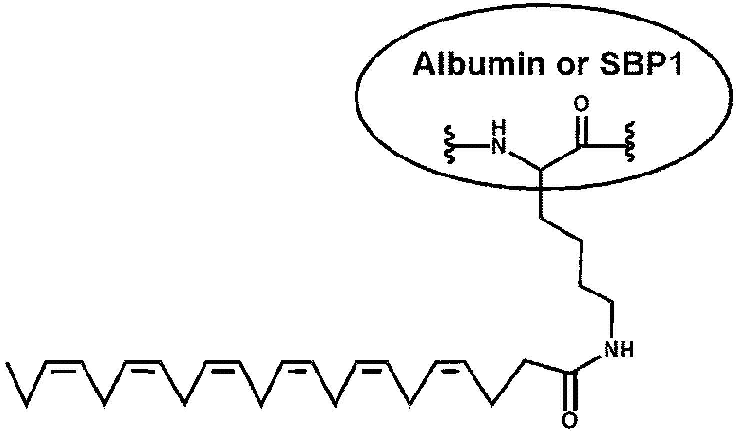 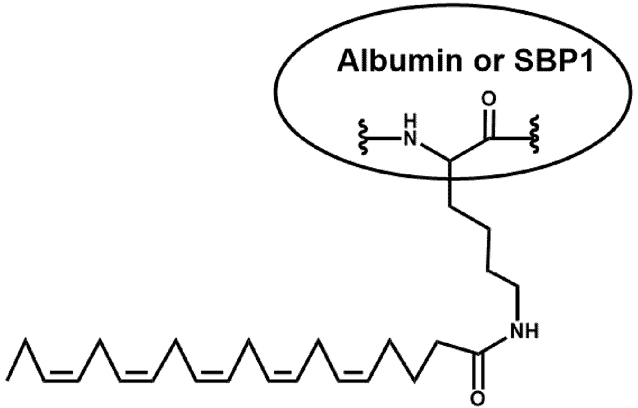 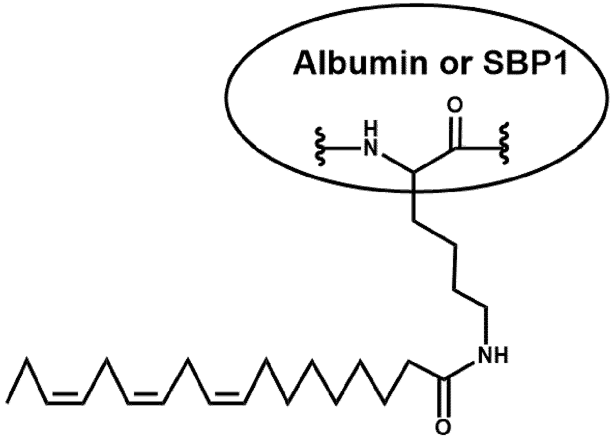 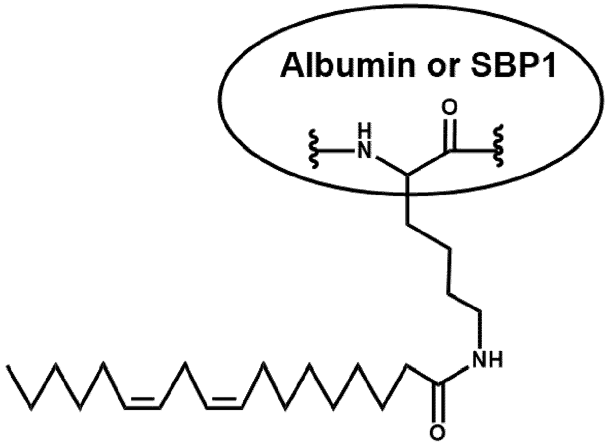 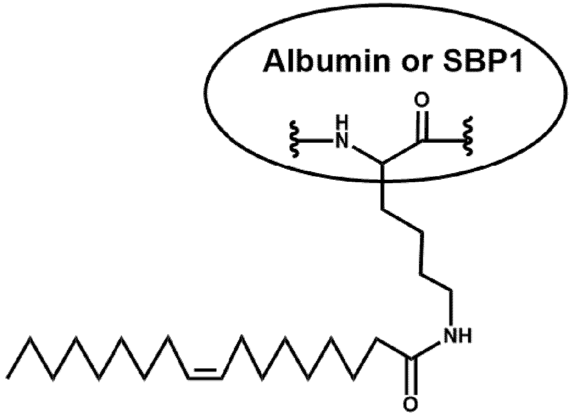 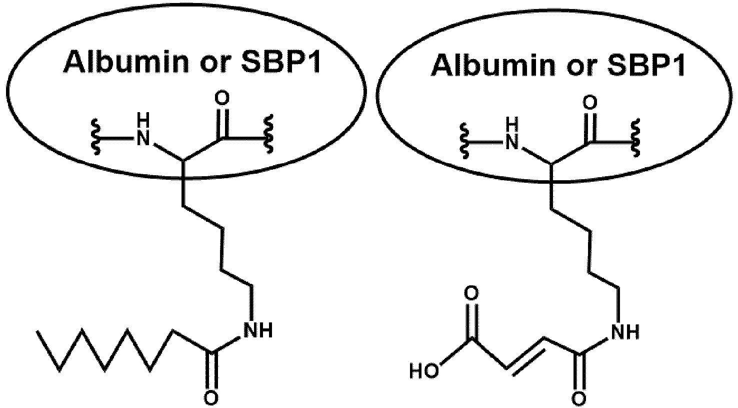 In the above products, the carbon chain of fatty acid is the acting moiety, and the albumin and the polypeptide are both the binding moiety and water-soluble moiety.
(2) The fatty acids and proteins are linked by a linker comprising one or more of amino acids, succinic acid, butadienoic acid, glutaconic acid, hexaminodioic acid, carbamates, short peptides, polyethylene glycol, and derivatives of the compounds described above.

For example, a thioetherification product of a free sulfhydryl group of 34-Cys in HSA with a fatty acid and N-hydroxymaleimide:

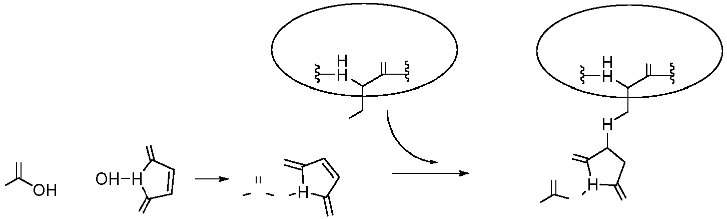

| | |
|---|---|
| 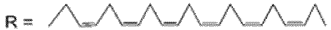 | Carbon chain of docosahexaenoic acid |
| 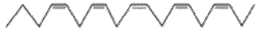 | Carbon chain of eicosapentaenoic acid |
| 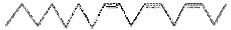 | Carbon chain of octadecatrienoic acid |
| 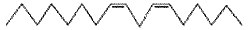 | Carbon chain of octadecadienoic acid |
| 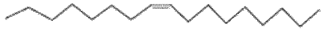 | Carbon chain of octadecenoic acid |
| 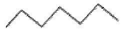 | Carbon chain of octanoic acid |
| 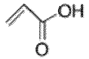 | Carbon chain of fumaric acid |

The schematic diagram of the product structure is as follows:

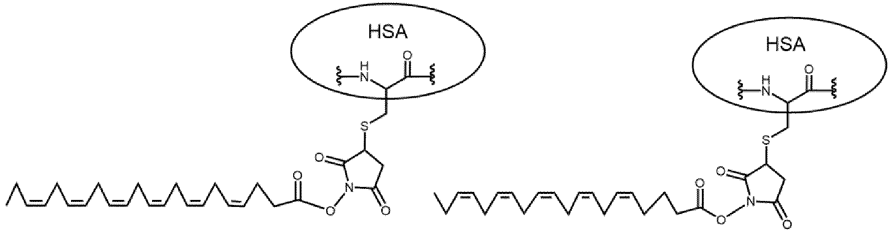

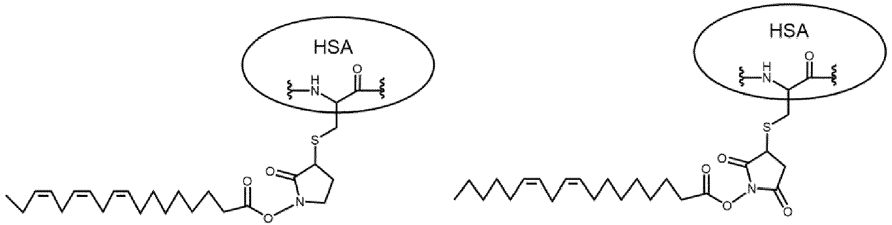

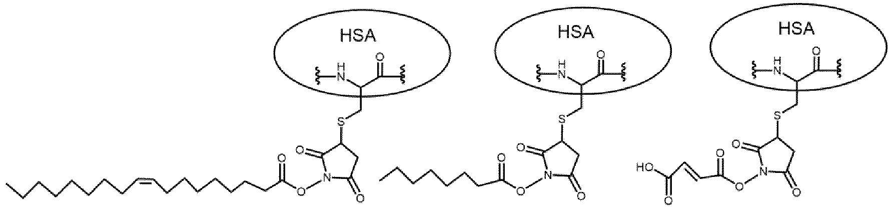

In the above products, the fatty acid together with the linker molecule is the acting moiety, and HSA is both the binding moiety and the water-soluble moiety.

In another embodiment of the present invention, the complex for preventing, blocking or treating microbial infection of the present invention can be an ester of a hydroxyl group of a water-soluble polysaccharide (dextran, hyaluronic acid, water-soluble cellulose derivative, cyclodextrin, etc.) with a carboxyl group of a fatty acid.

In particular, the polysaccharide is exemplified by dextran and hyaluronic acid, and the carbon chain donor is exemplified docosahexaenoic acid (DHA), eicosapentaenoic acid (EPA), octadecatrienoic acid (linolenic acid), octadecadienoic acid (linoleic acid), octadecenoic acid (oleic acid), octanoic acid, and butenedioic acid (fumaric acid). In a more specific embodiment, the complex of the present invention includes complexes of the following structures.
(1) A complex resulting from reaction of dextran with a fatty acid:

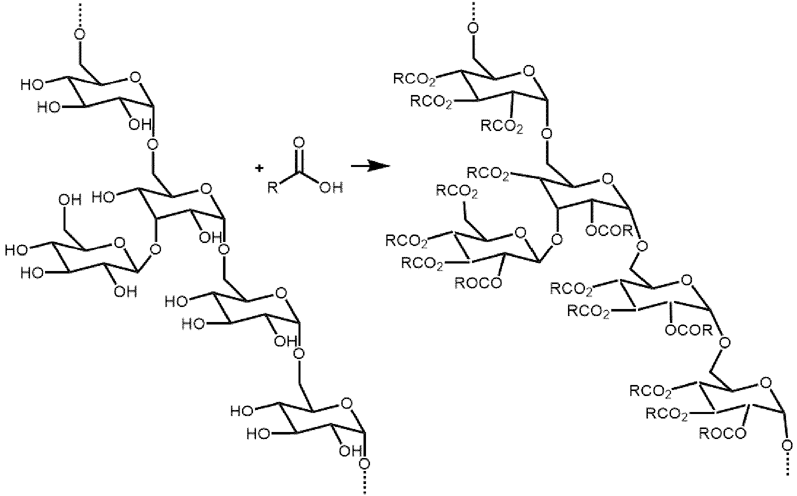

| | |
|---|---|
| 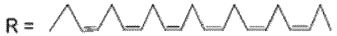 | Carbon chain of docosahexaenoic acid |
| 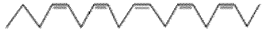 | Carbon chain of eicosapentaenoic acid |
| 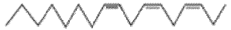 | Octadecatrienoic acid |
| 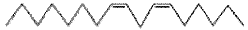 | Carbon chain of octadecadienoic acid |
| 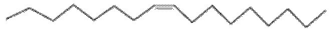 | Octadecenoic acid |
| 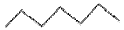 | Carbon chain of octanoic acid |
| 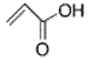 | Carbon chain of fumaric acid |

The resulting complex has one or two of the following structural formulas: 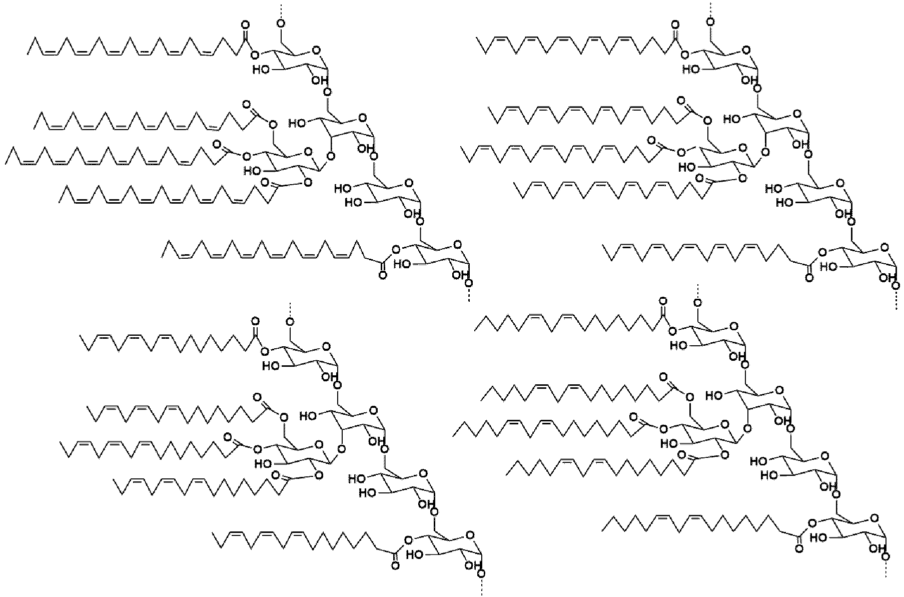 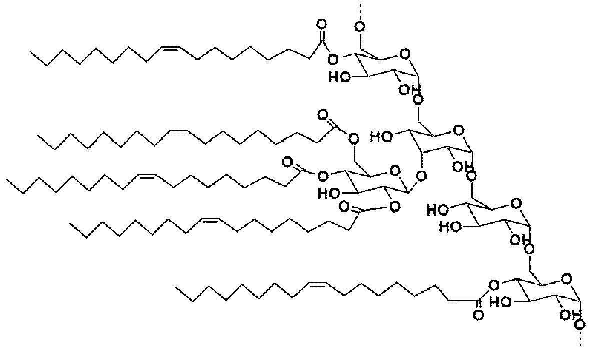 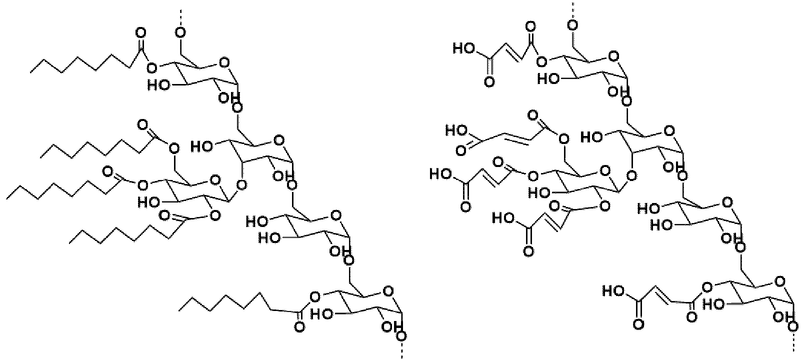 The carbon chain of the fatty acid in the above product is the acting moiety, and the dextran is both the binding moiety and the water-soluble moiety;
(2) A complex resulting from the reaction of hyaluronic acid with a fatty acid: 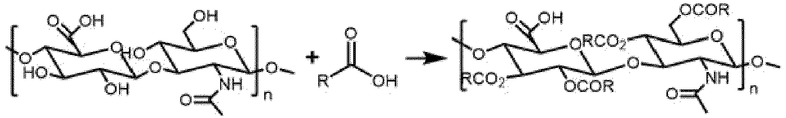 where n is an integer of 1-200;

| | |
|---|---|
| 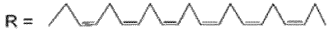 | Carbon chain of docosahexaenoic acid |
| 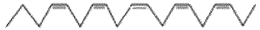 | Carbon chain of eicosapentaenoic acid |
| 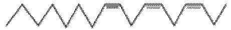 | Octadecatrienoic acid |
| 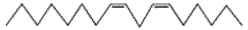 | Carbon chain of octadecadienoic acid |
| 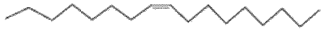 | Octadecenoic acid |
| 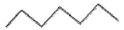 | Carbon chain of octanoic acid |
| 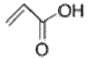 | Carbon chain of fumaric acid |

The resulting hyaluronic acid esterification product has one or two of the following structural formulas: 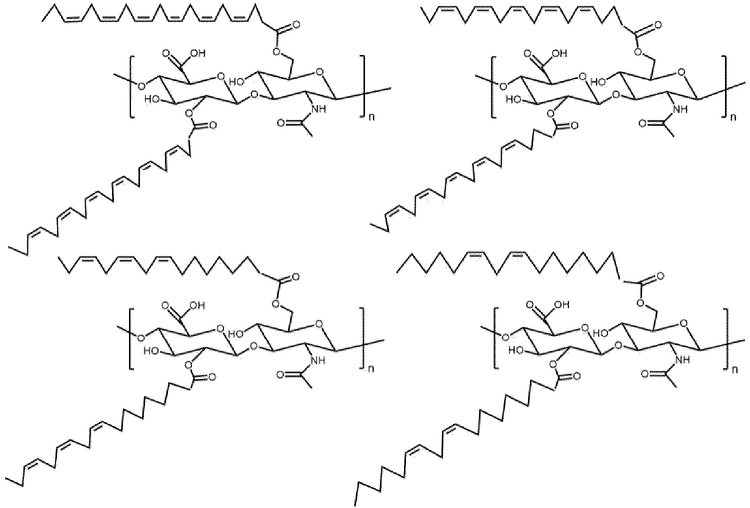 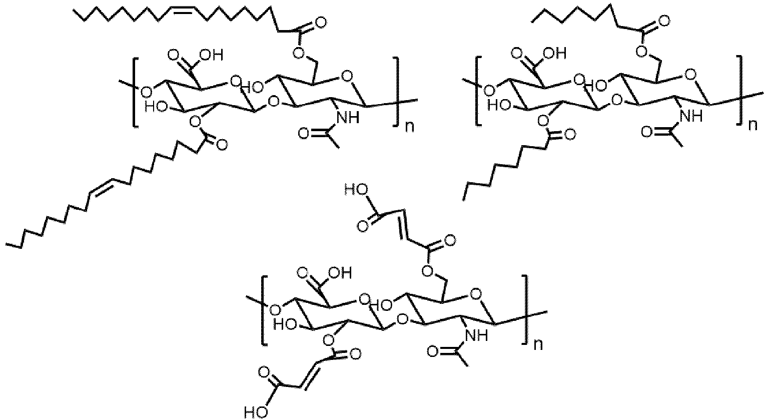 where n is an integer of 1-200.
In the above products, the carbon chain of fatty acid is the acting moiety, and hyaluronic acid is both the binding moiety and the water-soluble moiety.
(3) As the complex for preventing, blocking or treating microbial infection according to the present invention, a compound may obtained by reductive amination of cystamine with a terminal hemiacetal structure of polysaccharide and amidation of another amino group of the cystamine with a fatty acid, for example, a compound obtained by the reaction of dextran with cystamine and fatty acid and a compound obtained by the reaction of hyaluronic acid with cystamine and fatty acid.

The reaction process of dextran (DEX) with cystamine and a fatty acid is simplified as follows:

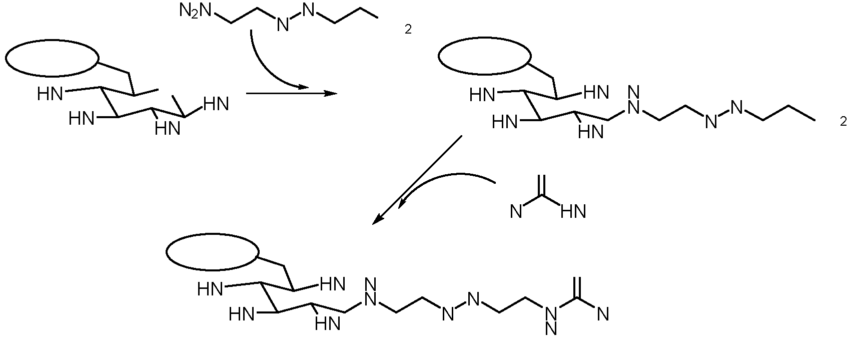

| | |
|---|---|
| 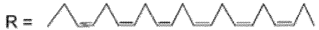 | Carbon chain of docosahexaenoic acid |
| 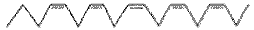 | Carbon chain of eicosapentaenoic acid |
| 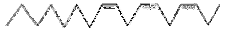 | Octadecatrienoic acid |
| 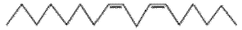 | Carbon chain of octadecadienoic acid |
| 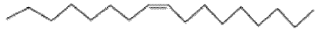 | Octadecenoic acid |
| 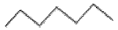 | Carbon chain of octanoic acid |
| 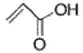 | Carbon chain of fumaric acid |

The product of DEX and cystamine with a fatty acid as the complex of the present invention has the following formulas:

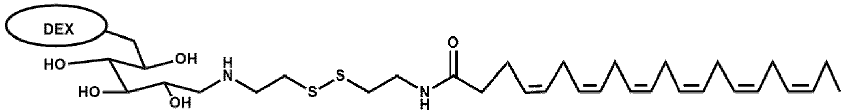

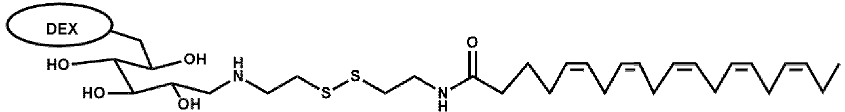

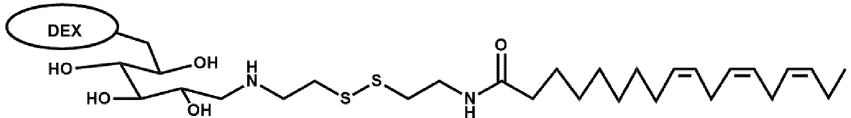

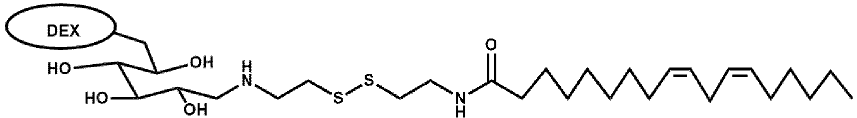

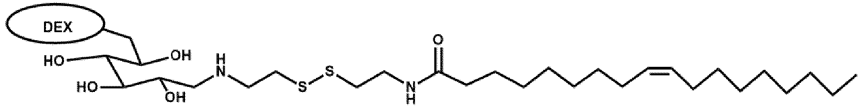

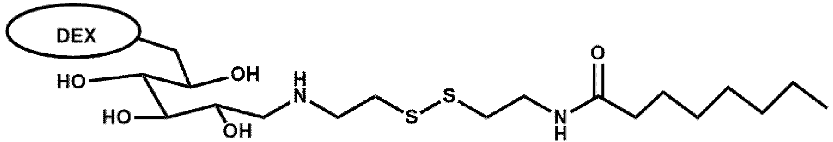

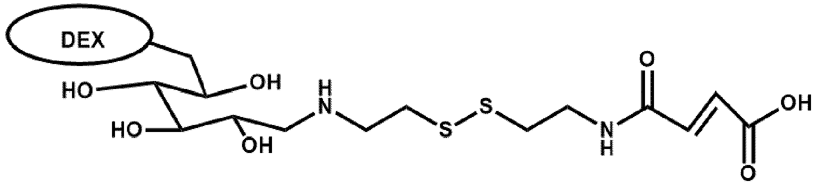

In the above products, the fatty acid and cystamine are the acting moieties, and dextran is both the binding moiety and the water-soluble moiety.

The reaction of hyaluronic acid (HA) with cystamine and a fatty acid is simplified as follows:

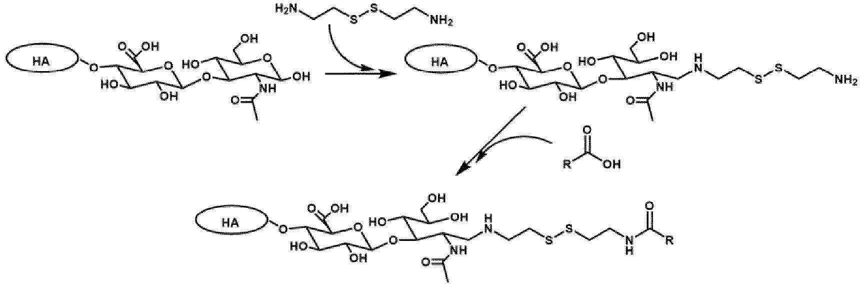

| | |
|---|---|
| 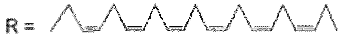 | Carbon chain of docosahexaenoic acid |
| 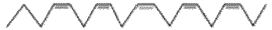 | Carbon chain of eicosapentaenoic acid |
| 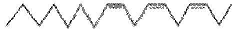 | Octadecatrienoic acid |
| 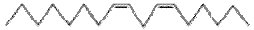 | Carbon chain of octadecadienoic acid |
| 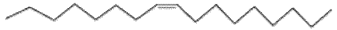 | Octadecenoic acid |
| 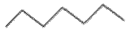 | Carbon chain of octanoic acid |
| 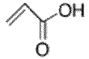 | Carbon chain of fumaric acid |

(too many attached fatty acid may affect the water solubility of the product, so one molecule of hyaluronic acid can be linked to one molecule of fatty acid by the reaction of with the hemiacetal hydroxyl group present at the terminal of the hyaluronic acid chain).

Wherein the product can be obtained by the reaction of hyaluronic acid (HA) with cystamine and a fatty acid has the following structural formula as a complex of the present invention:

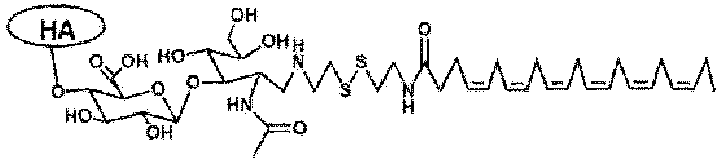

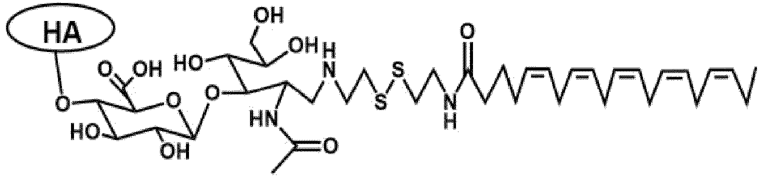

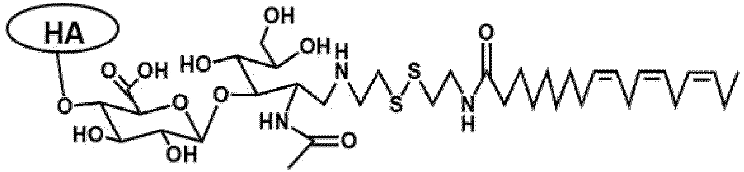

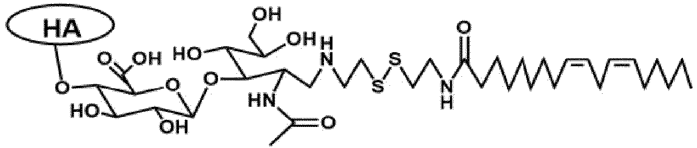

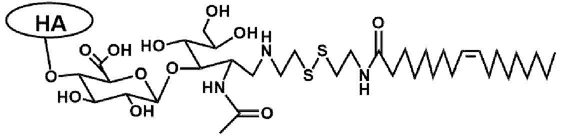

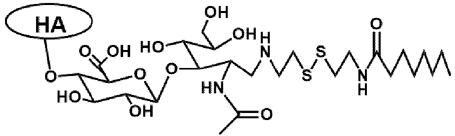

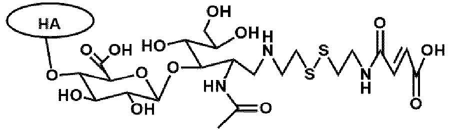

In the above products, the fatty acid and cystamine are the acting moieties, and hyaluronic acid is both the binding moiety and the water-soluble moiety.

In another embodiment of the invention, a complex of the invention for preventing, blocking or treating a microbial infection can be a compound formed by the reaction of a fatty acid with an oligosaccharide, such as a compound formed by the reaction of a fatty acid with sodium fondaparinux.

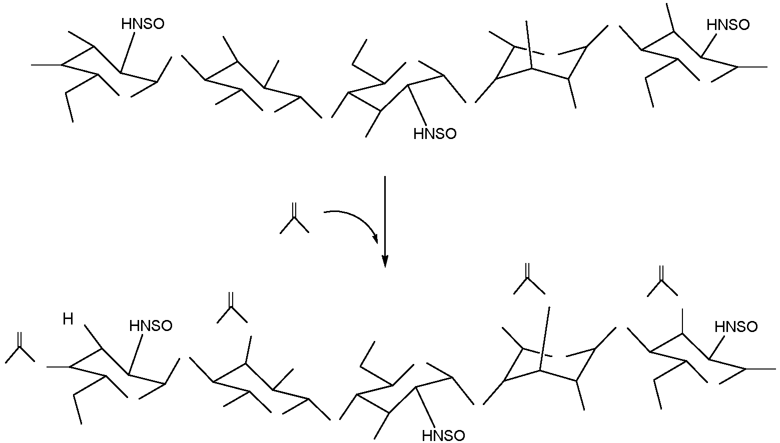

| | |
|---|---|
| 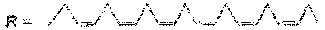 | Carbon chain of docosahexaenoic acid |
| 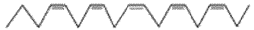 | Carbon chain of eicosapentaenoic acid |
| 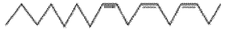 | Octadecatrienoic acid |
| 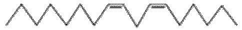 | Carbon chain of octadecadienoic acid |
| 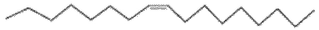 | Octadecenoic acid |
| 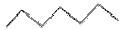 | Carbon chain of octanoic acid |
| 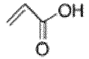 | Carbon chain of fumaric acid |

In the above products, the carbon chain of the fatty acid is the acting moiety, and fondaparinux is both the binding moiety and the water-soluble moiety.

In another embodiment of the present invention, the complex for preventing, blocking or treating microbial infections of the present invention can be a compound formed by the reaction of a fatty acid with a water-soluble small molecule including a monosaccharide or polysaccharide, amino acid, nucleotide or deoxynucleotide, and vitamin; for example, it can be a compound having a structure shown below, wherein the following R means a carbon chain having an integer number of carbon atoms of 1-99:
(1) A compound formed by a fatty acid and glucose 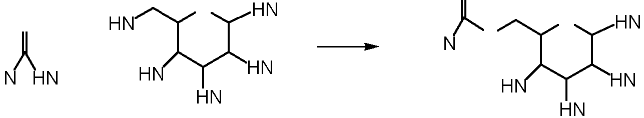
   The carbon chain of the fatty acid in the above product is the acting moiety and glucose is both the binding moiety and the water-soluble moiety.
   It is also possible to further attach a binding moiety, and the attached binding moiety can be selected from the group consisting of a dibasic or polybasic fatty acid, amino acid, targeting protein, targeting polypeptide and targeting polysaccharide. Here, as an example of attaching glutamic acid, the reaction is as follows: 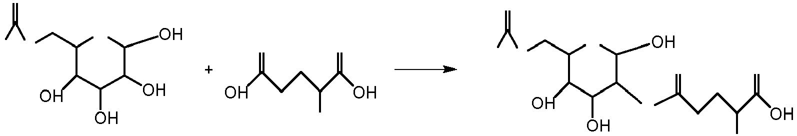
   At this time, the carbon chain of fatty acid in the product is the acting moiety, glucose is the water-soluble moiety (the binding action of glucose is weakened, and the action of increasing water-solubility is retained), and the glutamic acid is both the binding moiety and the water-soluble moiety.
(2) A compound formed by a fatty acid and sucrose 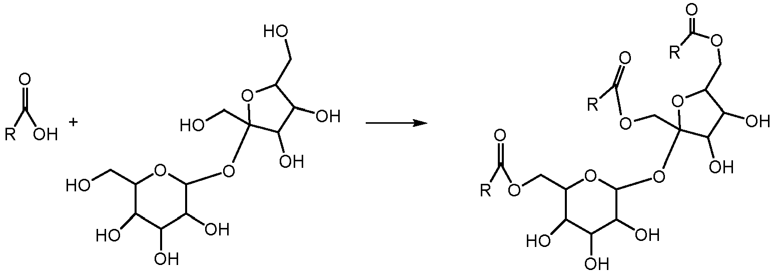
   The carbon chain of the fatty acid in the above product is the acting moiety and sucrose is both the binding moiety and the water-soluble moiety.
   It is also possible to further attach a binding moiety, and the attached binding moiety can be selected from the group consisting of a dibasic or polybasic fatty acid, amino acid, targeting protein, targeting polypeptide and targeting polysaccharide. Here, as an example of attaching butenedioic acid, the reaction is as follows: 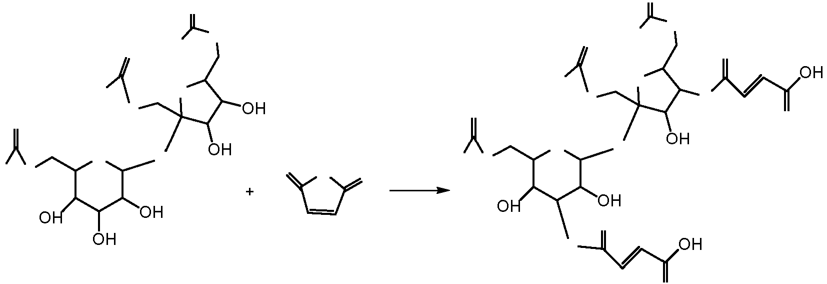
   At this time, the carbon chain of the fatty acid in the product is the acting moiety, sucrose is the water-soluble moiety (the binding effect of sucrose is weakened, and the effect of increasing water solubility is retained), and butenedioic acid is both the binding moiety and the acting moiety.
(3) A compound formed by a fatty acid and taurine 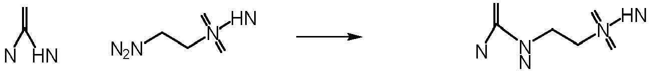 The carbon chain of the fatty acid in the above product is the acting moiety and taurine is both the binding moiety and the water-soluble moiety.
(4) A compound formed by a fatty acid and lysine 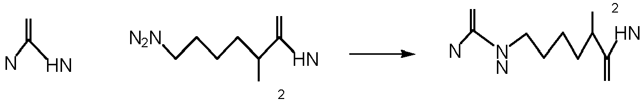 The carbon chain of the fatty acid in the above product is the acting moiety and lysine is both the binding moiety and the water-soluble moiety.
(5) A compound formed by a fatty acid and serine 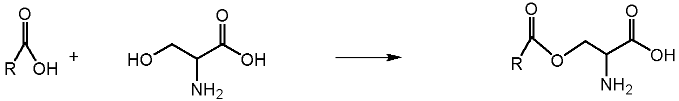 The carbon chain of the fatty acid in the above product is the acting moiety and serine is both the binding moiety and the water-soluble moiety.
(6) A compound formed by a fatty acid and threonine 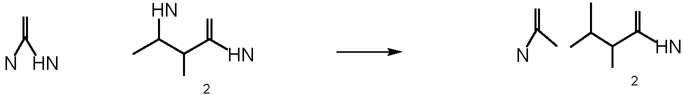 The carbon chain of the fatty acid in the above product is the acting moiety and threonine is both the binding moiety and the water-soluble moiety.
(7) A compound formed by a fatty acid and adenosine monophosphate and aspartic acid 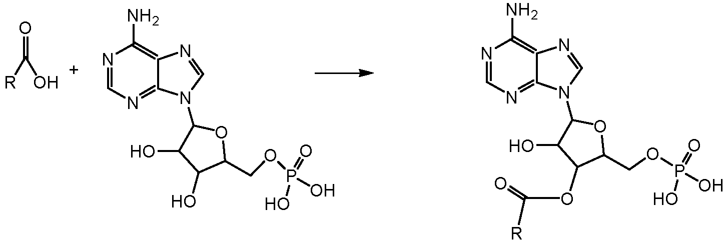 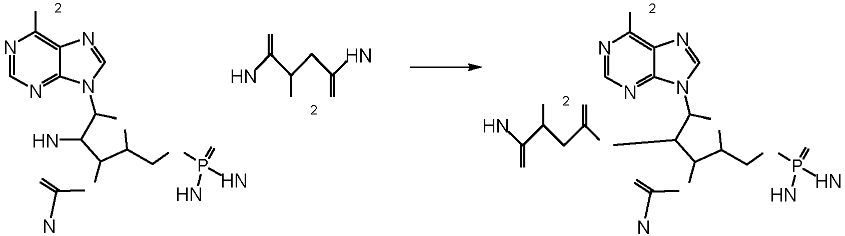 Wherein the carbon chain of the fatty acid is the acting moiety, the AMP is the water-soluble moiety, and the aspartic acid is both the binding moiety and the water-soluble moiety.
(8) Compounds formed by a fatty acid with ascorbic acid, glutaric acid or glutaric anhydride

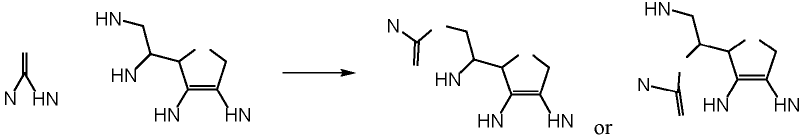

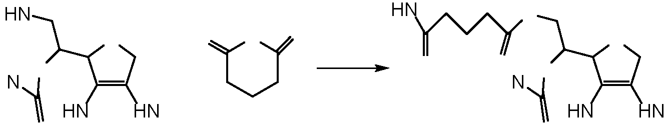

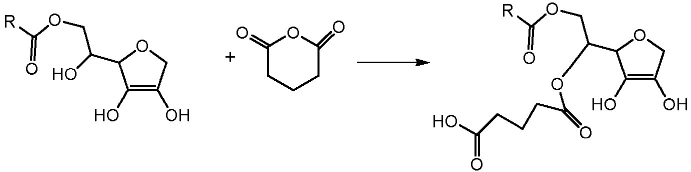

Wherein the carbon chain of the fatty acid is the acting moiety, ascorbic acid is the water-soluble moiety, and glutaric acid is both the binding moiety and the acting moiety.

In the above examples, R is an integer of 1-100 carbon atoms, and preferably R is as shown below.

| | |
|---|---|
| 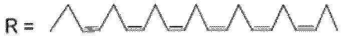 | Carbon chain of docosahexaenoic acid |
| 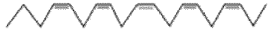 | Carbon chain of eicosapentaenoic acid |
| 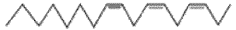 | Octadecatrienoic acid |
| 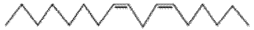 | Carbon chain of octadecadienoic acid |
| 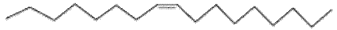 | Octadecenoic acid |
| 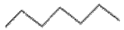 | Carbon chain of octanoic acid |
| 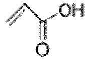 | Carbon chain of fumaric acid |

In another embodiment of the present invention, the complex for preventing, blocking or treating microbial infection of the present invention can be a compound obtained by linking a water-soluble moiety and a binding moiety using a fat-soluble vitamin as an acting moiety. The fat-soluble vitamins include vitamin A, vitamin E, vitamin K, and vitamin D. Retinoic acid in vitamin A group and a-tocopherol in vitamin E group are exemplified below.
(1) The composition of the complex is retinoic acid + PEG + succinic acid or succinic anhydride + alanine, wherein retinoic acid, succinic acid or succinic anhydride, alanine are the acting moieties, PEG is the water-soluble moiety, and alanine is the binding moiety; 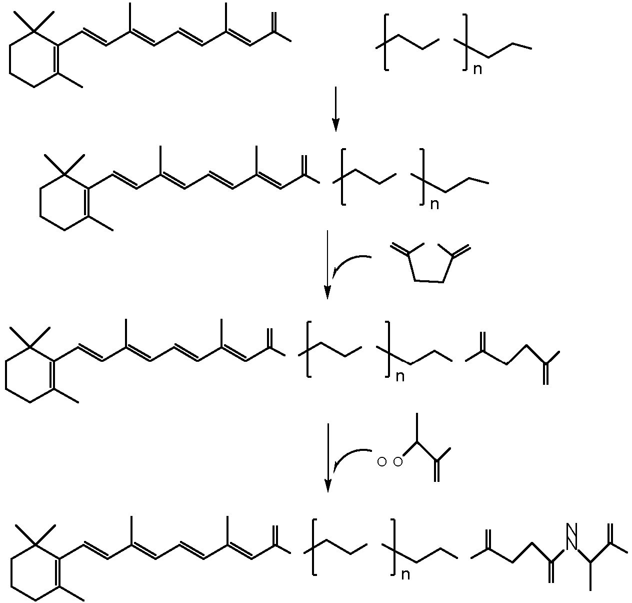 where n is an integer of 1-200.
(2) The composition of the complex is α-tocopherol succinate + PEG + butenedioic acid or butenedioic anhydride, wherein α-tocopherol succinate and butenedioic acid or butenedioic anhydride are the acting moieties, PEG is a water-soluble moiety, and butenedioic acid or butenedioic anhydride is the binding moiety; 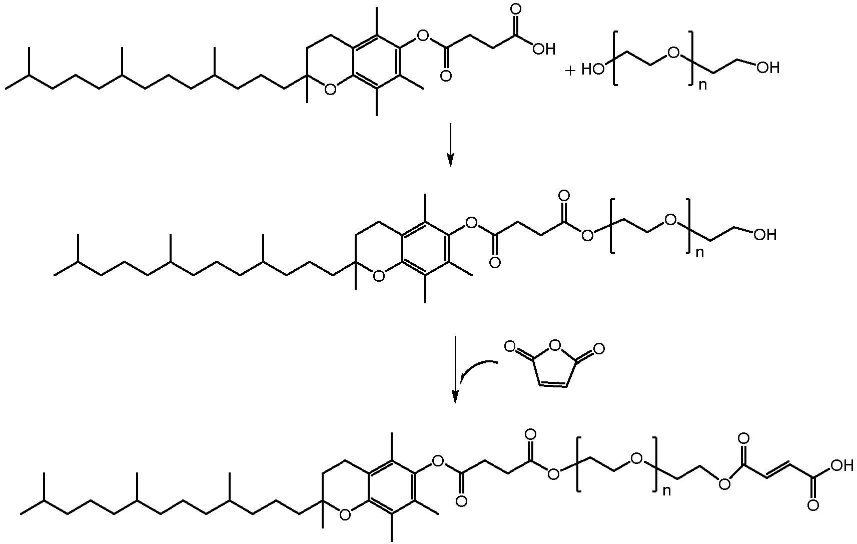 where n is an integer of 1-200.

In another embodiment of the present invention, the complex for preventing, blocking or treating microbial infection of the present invention can be a compound obtained by linking a water-soluble moiety and a binding moiety using a steroid lipidoid as an acting moiety. The steroid lipidoids include one or more of cholesterol, lanosterol, sitosterol, stigmasterol, ergosterol, bile acids, bile alcohols, and derivatives of the steroid lipidoids described above. Cholesterol and glycine cholic acid among bile acids are exemplified below.
(1) The composition of the complex is cholesterol succinate (carbon chain with a cyclic structure) + PEG + glutamic acid, wherein cholesterol succinate is the acting moiety, PEG and glutamic acid are the water-soluble moieties, and glutamic acid is the binding moiety. 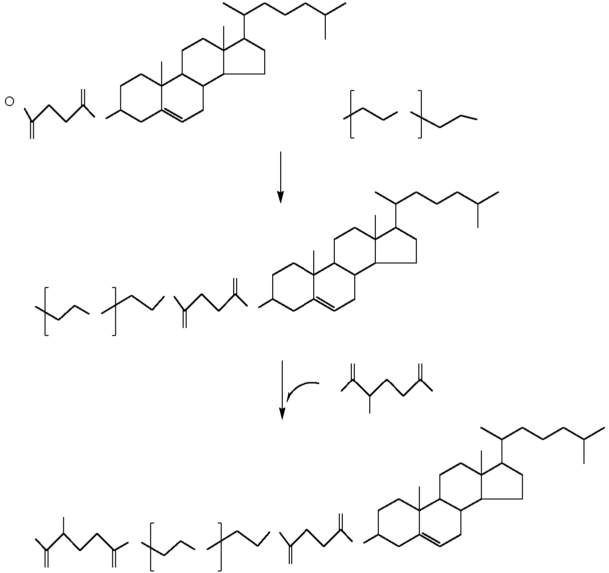 where n is an integer of 1-200.
(2) The composition of the complex is glycocholic acid + succinic acid + PEG + octadecatrienoic acid, wherein the cholestane skeleton in the glycine cholic acid, succinic acid and octadecatrienoic acid are the acting moieties, the acylglycine moiety in the glycine cholic acid and PEG are the water-soluble moieties, and glycine is the binding moiety. 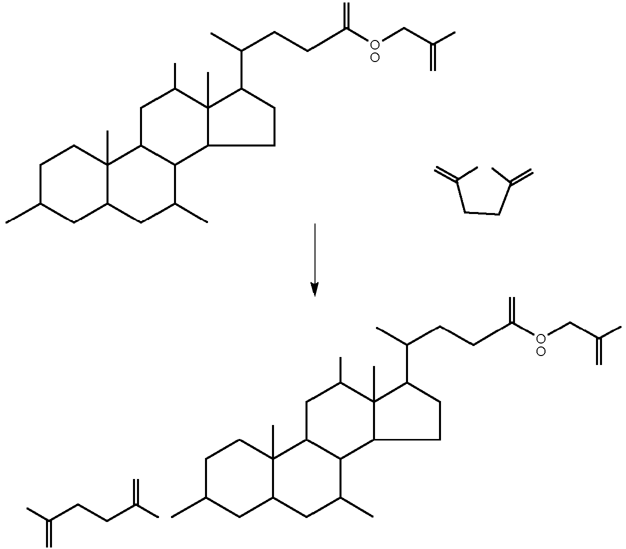 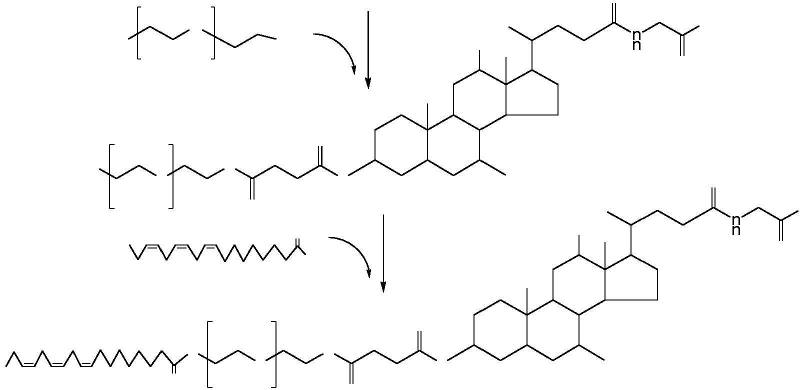 where n is an integer of 1-200.

In the complex of the present invention, PEG units are contained in various degrees or PEG (polyethylene glycol) is added when forming the complex. Specifically, in order to form the complex of the present invention, the complex should contain PEG units, i.e. the number of repetitions of -CH₂-CH₂-O-(ethoxy) or the degree of polymerization n in the complex is an integer of 1 to 200, as necessary. Further preferably, first, when the PEG unit is used as the backbone of the complex to link other acting moiety, binding moiety, and the water-soluble moiety, while also imparting the complex water solubility, then n = an integer of 4-200; second, where the PEG unit acts as the water-soluble moiety, n = an integer of 4-20; third, when the PEG unit acts as a linker arm, extending the distance of the macromolecule from the carbon chain to expand the interaction space, then n = an integer of 1-10.

In another embodiment of the present invention, the complex for preventing, blocking or treating microbial infections of the present invention can be a fatty alcohol polyoxyethylene ether, fatty acid polyoxyethylene ester, alkyl glycoside, fatty acid sucrose ester, sorbitan fatty acid ester, sorbitan polyoxyethylene fatty acid ester, mannose erythritol ester, or N-fatty acyl-N-methylglucamine. For the above-mentioned compounds having fatty alcohols or fatty acids as carbon chain donors, they have a good water solubility, but a weak binding effect with virus surface domains, lipid membrane or cell wall components. A relatively high concentration is required to kill microorganisms, but at this concentration, they also have a damaging effect on human body cells, not suitable for use in the human body. When the above-mentioned compound is linked to the binding moiety to form a new complex, it has the effect of killing microorganisms at a lower concentration in the human body an anti-microbial infection, and at this therapeutic concentration, the new complex having an acting moiety + a water-soluble moiety + a binding moiety has no effect on human tissue cells and organs. The binding moiety can be selected from the group consisting of dibasic or polybasic fatty acid, amino acid, targeting protein, targeting peptide, and targeting polysaccharide. For example, it can be a compound of the structure shown below.
(1) A compound obtained by linking a fatty alcohol polyoxyethylene ether with butenedioic acid, wherein the carbon chain moiety of the fatty alcohol and the linked carbon chain moiety of the butenedioic acid in the fatty alcohol polyoxyethylene ether are the acting moieties, the PEG unit is the water-soluble moiety, and the butenedioic acid is the binding moiety; 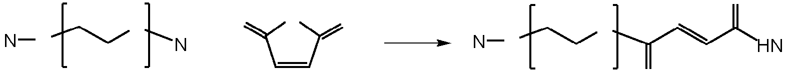 where n is an integer of 1-200.
(2) A compound obtained by linking a fatty acid polyoxyethylene ester with aspartic acid, wherein the carbon chain moiety of the fatty acid in the fatty acid polyoxyethylene ester is an acting group, the PEG unit and aspartic acid are water-soluble groups, and the aspartic acid is a binding group; 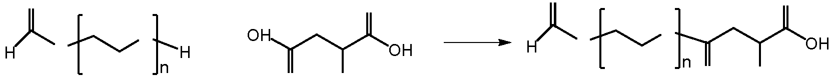 where n is an integer of 1-200.
(3) A compound obtained by linking a polyoxyethylene sorbitan fatty acid ester with a glutamic acid, wherein the carbon chain moiety of the fatty acid of the polyoxyethylene sorbitan fatty acid ester is the acting moiety, the sorbitan and PEG unit and the linked glutamic acid are the water-soluble moieties, and glutamic acid is the binding moiety; 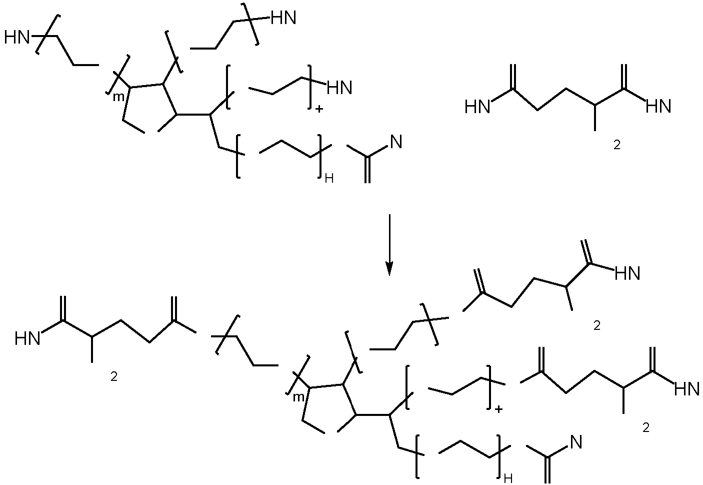 where n is an integer of 1-200.

In yet another embodiment, the present invention also provides an embodiment of a method of preparing a complex of the present invention for preventing, blocking or treating a microbial infection.

The complex of the present invention is prepared by the reaction of a compound providing a carbon chain, such as a fatty acid, with a protein, polypeptide, oligopeptide, oligosaccharide, monosaccharide, disaccharide, oligonucleotide, vitamin, water-soluble polymer, water-soluble polyamino acid and/or polysaccharide molecule, or adding a linker such as any one or two or more of PEG, N-hydroxy butenimide, amino acid, succinic acid, butadienoic acid, glutaconic acid, hexaminodioic acid, carbamate, short peptide and the like and derivatives thereof according to needs to obtain a reaction mixture. In a preferred embodiment, the reaction mixture is further purified to isolate a purified reaction product (the "purified reaction product" is also referred to herein as "reaction-derived compound" which refers to a substance remaining after the reaction mixture is isolated by purification means to remove unreacted substances, and such remaining substance is referred to as the "reaction-derived compound ").

For specific purification methods, the classification and description are as follows.

### Case of a fatty acid coupling a protein or polysaccharide

For example, a fatty acid coupling a proteins, polypeptide, and/or polysaccharide can be purified by either dialysis or ultrafiltration to obtain a reaction mixture. Where both the unreacted fatty acid and the catalyst added to the process are small molecules, they can be removed by dialysis (laboratory miniprep) or ultrafiltration (post-conversion large scale production). The specific purification process is to perform dialysis after the reaction is complete, including using dialysis bags (molecular weight cut-off can be 500-1000, 1000-1500, 1500-3000) for dialysis, changing water every 4 hours, and dialysing for 24 hours. The small molecular compounds all have a molecular weight of less than 500, and the catalyst and unreacted fatty acid in the reaction solution can be removed.

### 2. Case of fatty acid coupling small molecules

Molecular sieve chromatography can be used for purifying the fatty acid coupled with a small molecule. The molecular weight of the coupling product is different from that of the reaction substrate, and the molecular weight of the product is also greater than that of the catalyst molecule, so the molecular exclusion method can be used for separation. The filler can be selected from fillers made of dextran, agarose, polypropylene, etc. such as commercialized Shephadex, Sephacryl, Shepharose, Fractogel^{®} EMD SEC, Bio-Gel P, Bio-Gel A, etc. The eluent collected step by step is obtained after chromatography purification. The eluent is detected by any one of phenol sulfuric acid method, ninhydrin method, ultraviolet spectrophotometry, barium chloride-iodine solution method, and sulfuric acid formaldehyde chromogenic method, and the first eluting peaks are combined to give the product obtained by the reaction. Taking Shephadex G10 as an example, the column size and elution flow rate can be optimally adjusted according to the production scale. The specific process comprises the following steps.

### 2.1. (Fatty acid coupling mono-or disaccharides)

Performing chromatographic purification after the completion of the reaction; adding the reaction solution to a Shephadex G10 chromatographic column and eluting with normal saline to give the reaction product firstly; collecting the eluents step by step, and detecting with the phenol sulfuric acid method; and combining the first eluting peaks to give the reaction product.

### Phenol sulfuric acid method detection

Taking 100 ul of the sample in the collection tube and placing it a test tube with a stopper; taking deionized water as a blank; adding 100 ul of 5% phenol solution and shaking to mix well; quickly adding 500 ul of concentrated sulfuric acid, shaking, and quickly transferring to a 80°C water bath and holding 10 min; cooling for 3 min in an ice bath; and determining the absorbance at 487 nm.

### 2.2. (fatty acid coupling amino acid)

Performing chromatographic purification after the completion of the reaction; adding the reaction solution to a Shephadex G10 chromatographic column and eluting with normal saline to give the reaction product firstly; collecting the eluents step by step, and detecting with the ninhydrin method; and combining the first eluting peaks to give the reaction product.

### Ninhydrin method detection:

Taking 200 ul of the sample in the collection tube and placing it in a test tube with a stopper; taking deionized water as a blank; adding 300 ul of 2% ninhydrin solution and 200 ul of sodium acetate buffer solution (pH6); shaking and mixing well, and placing it in a water bath at 90°C; heating for 15 min, and cooling for 3 min in an ice bath; adding 300 ul of deionized water, mixing well, and determining the absorbance at 568 nm.

### 2.3. (fatty acid coupling ascorbic acid)

Performing chromatographic purification after the completion of the reaction; adding the reaction solution to a Shephadex G10 chromatographic column and eluting with normal saline to give the reaction product firstly; collecting the eluents step by step, and detecting the absorbance of the eluent by the ultraviolet spectrophotometry (243 nm); and combining the first eluting peaks to give the reaction product.

### 2.4. (fatty acid coupling nucleotides)

Performing chromatographic purification after the completion of the reaction; adding the reaction solution to a Shephadex G10 chromatographic column and eluting with normal saline to give the reaction product firstly; collecting the eluents step by step, and detecting the absorbance of the eluent by the ultraviolet spectrophotometry (260 nm); and combining the first eluting peaks to give the reaction product.

### 2.5. (fatty acid-PEG coupling)

Performing chromatographic purification after the completion of the reaction; adding the reaction solution to a Shephadex G10 chromatographic column and eluting with normal saline to give the reaction product firstly; collecting the eluents step by step, and detecting with the barium chloride-iodine solution method; and combining the first eluting peaks to give the reaction product.

### Barium chloride-iodine solution detection method (referring to the Method Improvement of General Rule 3202 in Part 4 of the Chinese Pharmacopoeia (2020 Edition)

Taking 100 ul of the sample in the collection tube, and placing it in a test tube with a stopper; taking deionized water as a blank; adding 300 ul of deionized water, 100 ul of 5% barium chloride solution, and 50 ul of 0.1 mol/L iodine solution; shaking well; incubating at room temperature for 15min; and determining the absorbance at 535 nm.

### 2.6. (steroid complexes)

Performing chromatographic purification after the completion of the reaction; adding the reaction solution to a Shephadex G10 chromatographic column (Φ26mm x 50 cm); eluting with normal saline at a flow rate of 50 ml/h; collecting the eluates step by step and detecting with the sulfuric acid formaldehyde chromogenic method; combining the first eluting peaks to give the reaction product.

### Formaldehyde sulfate chromogenic method

Taking 100 ul of the sample in the collection tube, and placing it in a test tube with a stopper; taking deionized water as a blank; adding 500 ul of concentrated sulfuric acid and 20 ul of formaldehyde; shaking and mixing well; incubating at room temperature for 5 min, then adding 500 ul of deionized water; shaking well; and determining the absorbance at 365 nm.

In particular, the present invention provides a method for preparing a complex including (an acting moiety + a macromolecular water-soluble moiety/a binding moiety), wherein the complex is formed by grafting a fatty acid onto serum albumin using the fatty acid as a carbon chain donor under the action of a catalyst. The molar ratio of the fatty acid to the serum albumin (585 amino acids in total for human serum albumin and 607 amino acids in total for bovine serum albumin, both calculated based on 66 kDa) is 20: 1 to 1: 1. The molar ratio of the catalyst to the fatty acid is 0.5: 1 to 10: 1. The catalyst can be one or more of EDC, DCC, NHS, DMAP, HoBt and derivatives and analogues thereof, etc. Wherein the carbon chain of the fatty acid is the acting moiety, and the serum albumin is the water-soluble moiety and the binding moiety.
the reaction equation is as follows:

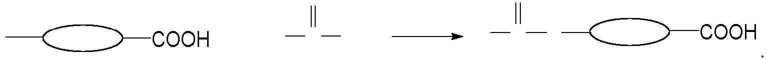

R represents a carbon chain of a fatty acid selected from the group consisting of fumaric acid, octanoic acid, undecanoic acid, hexadecenoic acid, oleic acid, linoleic acid, linolenic acid, eicosapentaenoic acid (EPA), docosahexaenoic acid (DHA) and triacontenoic acid, respectively, which reacts with serum albumin.

Preferably, the molar ratio of fatty acid to albumin (585 amino acids in total for human serum albumin and 607 amino acids in total for bovine serum albumin, both calculated based on 66 kDa) is from 20: 1 to 1: 1. The molar ratio of catalyst to fatty acid is from 0.5:1 to 10:1, preferably from 1: 1 to 10: 1. More preferably, the molar ratio of fatty acid to albumin is 10: 1 and the molar ratio of catalyst to fatty acid is 1: 1. The catalyst can be one or more of EDC, DCC, NHS, DMAP, HoBt, derivatives and analogues thereof, and the catalyst is preferably selected from the group consisting of 1-ethyl-(3-dimethylaminopropyl) carbodiimide (EDC) and N-hydroxysulfosuccinimide (sulfo-NHS), wherein the molar ratio of 1-ethyl-(3-dimethylaminopropyl) carbodiimide (EDC) to N-hydroxysulfosuccinimide (sulfo-NHS) is from 0.1: 1 to 10: 1, preferably 1: 1.

Further, in the compound obtained by the reaction of the fatty acid described above with serum albumin, it was found that the total fatty acid binding efficiency to amino acid is 0.10-15%. Wherein one molecule of protein binds to 7-24 molecules of linolenic acid, preferably 8 molecules of linolenic acid. Among the compounds obtained by the reaction of docosenoic acid with serum albumin, one molecule of serum protein binds 6-24 molecules of docosahexaenoic acid, preferably 10 molecules of docosahexaenoic acid. Wherein one molecule of serum protein binds 1-24 molecules of oleic acid, preferably 1 molecule of oleic acid. Wherein one molecule of protein binds about 6-24 eicosapentaenoic acid. Preferably, 17 molecules of EPA (eicosapentaenoic acid) are bonded to the amino group of the amino acid of one molecule of protein in the form of removing one molecule of water. Wherein one molecule of serum albumin binds about 11-16 molecules of linoleic acid. Preferably, 13 molecules of linoleic acid are bound to the amino acid of one protein molecule in the form of removing one molecule of water, wherein all are bound to a free amino group of lysine in the form of an amide bond, and the total degree of substitution of amino acids is more than 1.9%. Wherein one molecule of serum albumin binds about 6-15 DHA molecules. Preferably 9 DHA molecules are bound to the amino groups of amino acids of one protein molecule in the form of removing one molecule of water.

In addition, the present invention also provides a method for preparing a complex composed of (an acting moiety + a macromolecular water-soluble moiety/a binding moiety), wherein using an unsaturated fatty acid as a carbon chain donor, the complex is formed by the reaction of the fatty acid and hyaluronic acid under the action of a catalyst (wherein the preferred preparation process includes firstly adding the catalyst, reacting the fatty acid with hyaluronic acid to obtain an intermediate product, and then adding sodium hydroxide to adjust the pH value to be neutral to continue the reaction to obtain the complex). Wherein the molar ratio of the carboxyl group of the fatty acid to the hydroxyl group of the hyaluronic acid is from 4n: 1 to 1: 1 (n refers to the number of repetitions of a single molecule of hyaluronic acid). The molar ratio of the catalyst to the fatty acid is from 0.5: 1 to 10: 1, preferably from 1: 1 to 10: 1. The catalyst can be one or more of EDC, DCC, NHS, DMAP, HoBt and derivatives and analogues thereof. The catalyst is preferably carbodiimide and succinimide in a molar ratio of from 0.1: 1 to 10: 1. Wherein the carbon chain of the fatty acid is the acting moiety, and hyaluronic acid is the water-soluble moiety and the binding moiety.

In addition, the present invention provides a method for preparing a complex composed of (an acting moiety + a targeting binding moiety/a water-soluble moiety) wherein, using an unsaturated fatty acid as a carbon chain donor, the complex is formed by the reaction of the fatty acid with a polypeptide such as SBP1 (ACE2 derived peptide; binds SARS-CoV-2 spike protein receptor binding domain having the sequence of IEEQAKTFLDKFNHEAEDLFYQS (modifications: Ser-23 = C-terminal amide)) under the action of a catalyst. Wherein the fatty acid is one or two or more of oleic acid (OA), linoleic acid (LA), linolenic acid (ALA), eicosapentaenoic acid (EPA) and docosahexaenoic acid (DHA). The molar ratio of the carboxyl group of the fatty acid to the amino group of the polypeptide is from 8: 1 to 1: 4, preferably 2: 1. The molar ratio of the catalyst to the fatty acid is from 0*.*5*:* 1 to 10: 1. The catalyst can be one or more of EDC, DCC, NHS, DMAP, HoBt, derivatives and analogues thereof, etc. The catalyst is preferably a carbodiimide and a succinimide in a molar ratio of from 0.1: 1 to 10: 1, preferably from 1: 1 to 1: 10.

In addition, the present invention also provides a method for preparing a complex composed of (an acting moiety + a targeting binding moiety/a water-soluble moiety) wherein using an unsaturated fatty acid as a carbon chain donor, the complex is formed by the reaction of the fatty acid with CD14 under the action of a catalyst. Wherein the fatty acid is one or two or more of oleic acid, linoleic acid, linolenic acid, eicosapentaenoic acid (EPA), and docosahexaenoic acid. The molar ratio of the carboxyl group of the fatty acid to the CD14 is from 17: 1 to 1: 1, preferably 17: 1, and the molar ratio of the catalyst to the fatty acid is from 0.5: 1 to 10: 1. The catalyst can be one or more of EDC, DCC, NHS, DMAP, HoBt and derivatives and analogues thereof. The catalyst is preferably a carbodiimide and a succinimide in a molar ratio of 0.1: 1 to 10: 1, preferably 1: 1 to 1: 10.

In addition, the present invention also provides a method for preparing a complex composed of (a medium and long chain saturated carbon chain acting moiety + a macromolecular water-soluble moiety/binding moiety) wherein using a medium and long chain saturated fatty acid as a carbon chain donor, the complex is formed by the reaction of the fatty acid with hyaluronic acid under the action of a catalyst. Wherein the fatty acid is any one or two or more saturated fatty acids with 5-20 carbon atoms, preferably one or two or more saturated fatty acids of pentanoic acid, hexanoic acid, heptanoic acid, octanoic acid, nonanoic acid, decanoic acid, undecanoic acid, dodecanoic acid, tridecanoic acid, tetradecanoic acid, pentadecanoic acid, hexadecanoic acid, heptadecanoic acid, octadecanoic acid, nonadecanoic acid, and eicosanoic acid. The molar ratio of the carboxyl group of the fatty acid to the hyaluronic acid is from 4n: 1 to 1: 1 (n is the number of repetitions of a single molecular unit of hyaluronic acid, and n is an integer from 1 to 2000), and the molar ratio of the catalyst to the fatty acid is from 0.5: 1 to 10: 1, preferably from 0.5: 1 to 2: 1. The catalyst is preferably a carbodiimide and a succinimide in a molar ratio of from 0.1: 1 to 10: 1.

Also, through the experiment, the fatty acid-serum protein complex, the fatty acid-hyaluronic acid complex, the fatty acid-SBP1 complex, the fatty acid-CD14 complex, the fatty acid-dextran complex, etc. of the present invention show bactericidal and antibacterial effects on any bacteria selected from the group consisting of: *Escherichia coli,* Staphylococcus *aureus,* methicillin-resistant *Staphylococcus aureus, Streptococcus pneumoniae, Klebsiella pneumoniae* and *Pseudomonas aeruginosa,* with a sterilization rate of more than 99%; show a fungicide and antifungal effects on any fungus selected from the group consisting of: *Candida albicans, Aspergillus niger, Actinomyces viscosus, Chaetomium globosum, Aspergillus protuberus* and *Microsporum canis,* with the sterilization rate of more than 99%; and show virucidal effect on any virus selected from the group consisting of H7N9 influenza virus, H5N1 influenza virus, HIV virus, novel coronavirus virus, HPV virus, and rabies virus, with the sterilization rate of more than 99%.

In addition, the present invention provides a method for preparing a complex composed of (an acting moiety + a small molecule water-soluble moiety/binding moiety) wherein using a fatty acid as a carbon chain donor, the complex is formed by the reaction of the fatty acid and a monosaccharide such as glucose or sucrose in the presence of a catalyst; or the fatty acid and a nucleotide such as adenosine monophosphate, amino acid, water-soluble vitamin, PEG400-COOH with a low degree of polymerization, a substance having a carbon chain of a cyclic structure such as taurocholic acid (sodium salt) (for example, a complex of 4-octendioic acid and taurocholic acid), etc. (wherein the preferred preparation process includes adding a catalyst, and reacting the fatty acid with a monosaccharide such as glucose, etc. to obtain a complex, or adding sodium hydroxide to adjust the pH value to be neutral according to needs to continue the reaction to obtain a final complex; it is also preferable to further purify the resulting reaction product mixture solution). Wherein the fatty acid is preferably octanoic acid. The molar ratio of the carboxyl group of the fatty acid to the water-soluble small molecule such as glucose, sucrose, nucleotide (such as adenosine monophosphate), amino acid, water-soluble complex vitamin, PEG400-COOH with a low degree of polymerization, etc. is

from 1: 1 to 1: 4. The molar ratio of the catalyst to the fatty acid is from 0.5*:* 1 to 10: 1, preferably from 1: 1 to 10: 1. The catalyst is carbodiimide and succinimide, or carbodiimide and dimethylamino pyridine, the molar ratio of the two is from 0.1: 1 to 10: 1, preferably 1: 1. Wherein the inhibition rate of the fatty acid-small molecule water-soluble molecule complex obtained in the present invention is more than 99%. The complex of the present invention show bactericidal and antibacterial effects on any bacteria selected from the group consisting of: *Escherichia coli, Staphylococcus aureus,* methicillin-resistant *Staphylococcus aureus, Streptococcus pneumoniae, Klebsiella pneumoniae* and *Pseudomonas aeruginosa,* with a sterilization rate of more than 99%; show a fungicide and antifungal effects on any fungus selected from the group consisting of: *Candida albicans, Aspergillus niger, Actinomyces viscosus, Chaetomium globosum, Aspergillus protuberus* and *Microsporum canis,* with the sterilization rate of more than 99%; and show virucidal effect on any virus selected from the group consisting of H7N9 influenza virus, H5N1 influenza virus, HIV virus, novel coronavirus virus, HPV virus, and rabies virus, with the sterilization rate of more than 99%.

Wherein in a further preferred embodiment, the invention provides a complex obtained by the reaction of a fatty acid with an amino acid. Wherein the fatty acid is one or two or more selected from the group consisting of n-octanoic acid, linoleic acid, linolenic acid, eicosapentaenoic acid, and docosahexaenoic acid; and the amino acid is one or two or more selected from the group consisting of serine, threonine, and lysine.

In addition, the present invention also provides a method for preparing a complex having (noncovalently coupled water-soluble moiety + acting moiety), including mixng an unsaturated fatty acid, or fatty acid ester, or lecithin as a carbon chain donor with a protein or polysaccharide, or amino acid to give a liposome (for example, octadecanoic acid-glutamic acid liposome, dodecanoic acid-aspartic acid liposome, pentacosanoic acid liposome (liposome obtained by complexation of carboxylated lecithin, β-sitosterol, glycocholate sulfuric acid, pentacosanoic acid, and ethanol), fatty acid ethyl ester liposome ( liposomes obtained by complexation of a surfactant, aminated lecithin, cholesterol, and fatty acid ethyl ester, wherein the fatty acid can be medium-chain hexanoic acid (ethyl hexanoate), heptanoic acid, octanoic acid, nonanoic acid, decanoic acid, stearic acid, oleic acid, linoleic acid, linolenic acid, eicosapentaenoic acid, docosahexaenoic acid, etc.). The experiments of the present invention show that the liposome show bactericidal and antibacterial effects on any bacteria selected from the group consisting of: *Escherichia coli, Staphylococcus aureus,* methicillin-resistant *Staphylococcus aureus, Streptococcus pneumoniae, Klebsiella pneumoniae* and *Pseudomonas aeruginosa,* with a sterilization rate of more than 99%; show a fungicide and antifungal effects on any fungus selected from the group consisting of: *Candida albicans, Aspergillus niger, Actinomyces viscosus, Chaetomium globosum, Aspergillus protuberus* and *Microsporum canis,* with the sterilization rate of more than 99%. The above liposomes can also show virucidal effect on any virus selected from the group consisting of H7N9 influenza virus, H5N1 influenza virus, HIV virus, novel coronavirus virus, HPV virus, and rabies virus. Wherein the virocidal rates of both ethyl oleate liposomes and linoleic acid liposomes can reach more than 99%.

The present invention also provides a method for preparing a complex (mixture) having (amino/carboxyl group + water-soluble moiety + acting moiety non-covalent coupling). Wherein it is preferable that the mixture is a lipid emulsion obtained by mixing a surfactant with a fatty acid ester having a carbon chain, for example, a nano-liposome emulsion (particle size of 500 nm -800 nm) obtained by liposome emulsion of ethyl oleate, aminated lecithin, β-sitosterol and vitamin E palmitate; it can be a preparation (having a particle size of 500 nm-800 nm) obtained by the complexion of an unsaturated fatty acid with a carboxylated lecithin liposome, for example, a nano liposome emulsion (having a particle size of 500nm to 800nm) obtained by mixing a surfactant, linoleic acid, β-sitosterol, and carboxylated lecithin.

The experiments of the present invention show that the nano-liposome emulsions show bactericidal and antibacterial effects on any bacteria selected from the group consisting of: *Escherichia coli, Staphylococcus aureus,* methicillin-resistant *Staphylococcus aureus, Streptococcus pneumoniae, Klebsiella pneumoniae* and *Pseudomonas aeruginosa,* with a sterilization rate of more than 99%; show a fungicide and antifungal effects on any fungus selected from the group consisting of: *Candida albicans, Aspergillus niger*, *Actinomyces viscosus*, *Chaetomium globosum*, *Aspergillus protuberus* and *Microsporum canis,* with the sterilization rate of more than 99%. The above nano-liposome emulsions can also show virucidal effect on any virus selected from the group consisting of H7N9 influenza virus, HSN1 influenza virus, HIV virus, novel coronavirus virus, HPV virus, and rabies virus. Wherein the virocidal rates of both ethyl oleate liposomes and linoleic acid liposomes can reach more than 99%.

Further, the compound can be formulated into an injection, nasal spray, dry powder inhalant, oral dosage form, transdermal topical dosage form, and disinfectant, etc.

Wherein the oral preparation can be liquid (e.g. a syrup, solution, or suspension) or solid (e.g. granules, tablets, or capsules). The oral preparation can be coupled with a targeting ligand to cross the endothelial barrier. Some fatty acid derivative preparations can be formed into a fatty acid derivative powder by, for example, spray drying with a disaccharide. The solid composition can be prepared in conventional manner using pharmaceutically acceptable excipients such as a binding agent (e.g. pregelatinised maize starch, polyvinylpyrrolidone, or hydroxypropyl methylcellulose); a filler (e.g. lactose, mannitol, microcrystalline cellulose, or calcium hydrogen phosphate); a lubricants (e.g. magnesium stearate, talc, or silica); a disintegrant (e.g. potato starch or sodium starch glycolate); or a wetting agent (e.g. sodium lauryl sulfate). The tablets can be coated by methods well known in the art with, for example, a sugar, film, or enteric coating. The method for preparing such dosage forms is known or obvious to those skilled in the art. Emulsions of fatty acids can be administered orally, topically or by injection. In the preparation, the composition of excipients can be appropriately adjusted according to different administration modes, and other suitable preparation methods can be adopted according to different properties of excipients.

Furthermore, in the case of transdermal topical dosage forms or disinfectant dosage forms, the complex can be directly dissolved in a solvent and formulated with excipients to give a preparation for killing and preventing viruses, bacteria, and fungi.

Furthermore, the nasal sprays and dry powder inhalants require the addition of a mucosa-promoting adsorbent.

The mucosa-promoting adsorbent includes one or more of hyaluronic acid (HA), polyvinyl alcohol (PVA), polyvinyl pyrrolidone (PVP), polyethylene glycol (PEG), carbomer (CP), sodium carboxymethyl cellulose (CMC-Na), methyl cellulose (MC), hydroxyethyl cellulose (HEC), hydroxypropyl cellulose (HPC), and hydroxypropylmethyl cellulose (HPMC).

Furthermore, the dry powder inhalant is prepared by adding a fatty acid complex solution into a mucosa-promoting adsorbent and then performing spray drying or freeze drying to obtain a complex micropowder.

A lyoprotectant is need in the lyophilization method to prepare the dry powder inhalant.

Such lyoprotectants include one or more of glycerol, mannitol, sorbitol, inositol, thiols, proline, tryptophan, sodium glutamate, alanine, glycine, lysine hydrochloride, sarcosine, L-tyrosine, phenylalanine, arginine, polyethylene glycol, polyvinylpyrrolidone, gelatin, glucose, α-D-mannopyranose, sucrose, lactose, trehalose, cellobiose, mannose, maltose, inositol, inulin, dextran, maltodextrin, maltopolysaccharide, sucrose octasulfate, heparin, 2-hydroxypropyl-beta cyclodextrin, tween 80, Brij, Pluronic, and sodium dodecylsulfate.

Furthermore, the virus includes enveloped viruses and non enveloped viruses, such as coronavirus, HIV virus (also called AIDS virus), hepatitis B virus, hepatitis C virus, rabies virus, herpes virus, Ebola virus, Hantavirus, Dengue virus, Japanese encephalitis virus, Zika virus, influenza virus, hepatitis A virus, human papilloma virus, adenovirus, polio virus, and Coxsackie virus.

The coronaviruses include HCoV-229E, HCoV-OC43, SARS-CoV, HCoV-NL63, HCoV-HKU1, MERS-CoV, and SARS-CoV-2.

Further, the bacteria include gram-positive and gram-negative bacteria, wherein
Gram-positive bacteria include *Staphylococcus genus, Streptococcus genus, Bacillus genus, Clostridium genus, Listeria genus, Corynebacterium genus,* including *Staphylococcus aureus, Staphylococcus epidermidis, Staphylococcus saprophyticus, Streptococcus pneumoniae, Streptococcus pyogenes, Streptococcus agalactiae, Enterococcus, Bacillus anthracis, Bacillus cereus, Clostridium botulinum, Clostridium perfringens, Clostridium difficile, Clostridium tetani, Listeria monocytogenes, Bacillus diphtheria*, and *Mycobacterium tuberculosis.*
Gram-negative bacteria include *Escherichia coli, Pseudomonas aeruginosa, Proteus mirabilis, Bacillus dysenteriae, Klebsiella pneumoniae, Brucella, Haemophilus influenzae, Haemophilus parainfluenzae, Acinetobacter genus, Yersinia genus, Legionella pneumophila, Bordetella pertussis, Bordetella parapertussis, Neisseria genus, Shigella genus, Salmonella genus, Pasteurella genus, Vibrio cholerae, Bacillus parahaemolyticus,* and *Plesiomonas shigelloides.*

Such bacteria include clinically common multi-drug resistant bacteria (MDRO), such as methicillin-resistant *Staphylococcus aureus* (MRSA), vancomycin and SULPERAZON-resistant (Cefoperazone Sodium and Sulbactam Sodium) enterococcus (VRE), Enterobacteriaceae bacteria (such as *Escherichia coli* and *Klebsiella pneumoniae*) producing extended spectrum β-lactases (ESBLs), carbapenem-resistant Enterobacteriaceae, multi-drug resistant *Pseudomonas aeruginosa* (MDR-PA), and multi-drug resistant *Acinetobacter baumannii* (MDR-AB).

Further, the fungi include
histoplasma, coccidioidomycosis, paracoccidioidomycosis, blastomyces dermatitidis, chromoblastomycosis, mycetoma of foot, and sporotrichosis;
Conditioned pathogenic fungi: *Candida genus, Cryptococcus, Aspergillus, Actinomyces, Fusarium, Nocardia genus, Scedosporium genus, Trichomycetes order,* and *Phaeohyphomycosis order.*

Further the *Chlamydia* include *Chlamydia trachomatis, Chlamydia pneumoniae, Chlamydia psittaci.* The *Mycoplasma* includes *Mycoplasma pneumoniae, Ureaplasma urealyticum, Mycoplasma hominis,* and *Mycoplasma genitalium.*

Further, the mechanism by which the complex acts on microorganisms is as follows:
(1) A binding moiety + a medium-short chain/long chain acting moiety + a macromolecular water-soluble moiety are coupled to form a complex I, wherein the complex I can be retained on the mucosal surface of the respiratory tract or in the blood circulation. At the first time, the complex I inactivates a virus, a bacterium or a fungus, and prevents the virus, the bacterium or the fungus from spreading in the body. The macromolecular complex I cannot enter normal tissues, and can only enter the inflammatory site after the infection of the virus, the bacterium or the fungus to exert its effect.
(2) A binding moiety + a medium-short chain/long chain acting moiety + a water-soluble targeting polypeptide group/2 or more small molecule water-soluble moieties are coupled to form a complex II. The complex II can penetrate the vascular wall into the interstitial space and interstitial fluid, and target microorganisms to exert its effect.
(3) A binding moiety + a medium-short chain/long chain acting moiety + a water-soluble polypeptide targeting group/a small molecule water-soluble moiety + a water-soluble macromolecular polymer are coupled to form a complex III. At the first time, the complex III inactivates a virus, a bacterium or a fungus, and prevents the virus, the bacterium or the fungus from spreading in the body. The macromolecular complex III cannot enter normal tissues, and can only enter the inflammatory site after the infection of the virus, the bacterium or the fungus to exert its effect.

In some preferred embodiments of the invention, the complex may kill microorganisms including viruses, bacteria, fungi, chlamydia and mycoplasma; wherein the viruses include enveloped viruses such as coronavirus, influenza virus, AIDS virus, hepatitis B virus, hepatitis C virus, herpes virus, Zika virus, Dengue virus, Japanese encephalitis virus, Ebola virus, Hantavirus, etc. and the non-enveloped viruses such as hepatitis A virus, human papilloma virus, polio virus, Coxsackie virus, etc.

The coronaviruses preferably include HCoV-229E, HCoV-OC43, SARS-CoV, HCoV-NL63, HCoV-HKU1, MERS-CoV, and SARS-CoV-2.

In some preferred embodiments of the invention, the pharmaceutical dosage form comprises a dry powder inhalant, nasal spray, injection, oral dosage form, and transdermal topical dosage form.

In some preferred embodiments of the invention, the pharmaceutical dosage form is a dry powder inhalant, nasal spray, injection, or a transdermal topical dosage form for coronavirus (preferably SARS-CoV-2 virus), rabies virus, and influenza virus.

In some preferred embodiments of the invention, the pharmaceutical dosage form is an injection or oral dosage form for HIV virus.

In some preferred embodiments of the invention, the pharmaceutical dosage form is an injection or oral dosage form for HPV virus.

In some preferred embodiments of the invention, the pharmaceutical dosage form is a nasal spray for upper respiratory infections.

In some preferred embodiments of the invention, the pharmaceutical dosage form is a nasal spray for sinusitis.

The fatty acid and/or derivatives thereof bind according to the number of binding sites on the surface of a protein, polypeptide, or polysaccharide.

In some preferred embodiments of the present invention, the pharmaceutical excipient includes a pharmaceutically acceptable excipient.

In some preferred embodiments of the invention, the nasal spray excipients include glucose, cyclodextrin, microcrystalline cellulose and sodium hydroxymethylcellulose, sodium bisulfite, deoxycholic acid, thiourea, urea, hydroquinone, phenol, silica gel, graphite, protein, benzyl alcohol, phenylethanol, benzalkonium chloride, an emulsifier such as Tween 80, tocopherol, hydroxypropyl methyl methacrylate, gelatin, chitosan, alginate, arabic gum, polylactic acid, a high polymer such as polyhydroxyacetic acid, dilute hydrochloric acid, alcohol, and pure water.

In some preferred embodiments of the invention, the dry powder inhalant excipients include mucosa-promoting adsorbents such as one or more of hyaluronic acid (HA), polyvinyl alcohol (PVA), polyvinyl pyrrolidone (PVP), polyethylene glycol (PEG), carbomer (CP), sodium carboxymethyl cellulose (CMC-Na), methyl cellulose (MC), hydroxyethyl cellulose (HEC), hydroxypropyl cellulose (HPC), and hydroxypropylmethyl cellulose (HPMC);
lyoprotectants, such as one or more of glycerol, mannitol, sorbitol, inositol, thiols, proline, tryptophan, sodium glutamate, alanine, glycine, lysine hydrochloride, sarcosine, L-tyrosine, phenylalanine, arginine, polyethylene glycol, polyvinylpyrrolidone, gelatin, glucose, α-D-mannopyranose, sucrose, lactose, trehalose, cellobiose, mannose, maltose, inositol, inulin, dextran, maltodextrin, maltopolysaccharide, sucrose octasulfate, heparin, 2-hydroxypropyl-beta cyclodextrin, tween 80, Brij, Pluronic, and sodium dodecylsulfate.

The use of the complex and the preparation thereof according to the present invention for preventing and treating viral, bacterial and fungal infections, specifically including
it can be used before infection to prevent viral, bacterial, and fungal infections;
it can be used after infection to kill viruses, bacteria and fungi *in vivo*;
It can disinfect and and sterilize the environment of the article to prevent the spread of viruses, bacteria and fungi.

In the present invention, it is understood that the use of the complex for the preparation of a medicament for the prevention and/or treatment of viral (enveloped and non-enveloped viruses), bacterial and fungal infectious diseases falls within the intended scope of the invention.

The invention is further illustrated by the following specific examples, however, it is to be understood that these examples are included merely for purposes of illustration and are not to be construed as limiting the invention in any manner. The methods used in the following examples are conventional unless otherwise specified. The consumables and reagents used in the following examples are either commercially available or self-synthesized unless otherwise specified.

### Examples in Part I

The list of equipment and instruments in examples in Part I of the present invention is shown in Table 1-1 below. The sources of various microorganisms are shown in Table 1-2, table 1-3 and Table 1-4 below. In addition, in the present invention, the starting materials for each of the substances used for preparing the complex of the present invention are substances conventionally commercially available to those skilled in the art, except that the preparation method thereof is specifically described. In the following, only the commercial sources of some macromolecules or medium molecules or oligomers in the examples of the present invention are listed, which are specifically shown in Tables 1-5a and 1-5b.

**Table 1-1 Name of each instrument and equipment in the examples**

| Serial No. | Name | Model |
|---|---|---|
| 1 | Agitator | IKA RH basic 1 |
| 2 | Lyophilizer | Telstar LYOQUEST-85 |
| 3 | Fourier infrared spectrometer | Nicolet iS 5 |
| 4 | Transmission electron microscope | Tecnai G2 Spirit BioTWIN |
| 5 | Scanning electron microscope | Hitachi TM3030 |
| 6 | Energy dispersive spectrometer | Oxford AZtecOne |
| 7 | Inverted laboratory microscope | Leica DM IL LED |
| 8 | Inverted fluorescence microscope | Leica DM IL LED |
| 9 | CO₂ incubator | Thermo Heracell VIOS 160i |
| 10 | Microorganism incubator | Thermo Heratherm IMH100 SS |
| 11 | Multifunctional microplate reader | TECAN Spark |
| 12 | Refrigerated centrifuge | Thermo Fresco 17 |
| 13 | Vacuum lyophilizer | Thermo Savant DNA120 |
| 14 | Nanoliter liquid phase system | Thermo Easy-nLC1200 |
| 15 | High resolution mass spectrometer | Thermo Q Exactive |

**Table 1-2 Sources of various virusus in the examples**

| Vendor name | No. | Product name |
|---|---|---|
| Beijing Tiantan Pharmaceutical and Biotechnology Technology Development Company | 80020 | HPV pseudovirus HPV6-GFP |
| | 80062 | SARS-CoV-2 pseudovirus B. 1.526.2 |
| | 80041 | Influenza pseudovirus HSN1-Fluc |
| | 80018 | HIV pseudovirus HIV18A-41 |
| | 80036 | Influenza pseudovirus H7N9-Fluc |
| | 80052 | Rabies pseudovirus CVS-11 |
| | 80012 | HIV pseudovirus HIV 6A-40 |

**Table 1-3 Sources of various bacteria in examples**

| Vendor name | Product No. | Product name |
|---|---|---|
| BNCC | BNCC301345 | *Klebsiella pneumoniae* |
| BNCC | BNCC338118 | *Pseudomonas aeruginosa* |
| BNCC | BNCC269342 | *Escherichia coli* |
| BNCC | BNCC310011 | *Staphylococcus aureus* |
| BNCC | BNCC337371 | Methicillin-resistant *Staphylococcus aureus* |
| BNCC | BNCC338425 | *Streptococcus pneumoniae* |

**Table 1-4 Sources of various fungal in examples**

| Vendor name | No. | Product name |
|---|---|---|
| BNCC | BNCC263676 | *Candida albicans* |
| BNCC | BNCC185689 | *Aspergillus niger* |
| BNCC | BNCC336944 | *Actinomyces viscosus* |
| BNCC | BNCC185704 | *Chaetomium globosum* |
| BNCC | BNCC185686 | *Aspergillus protuberus* |
| BNCC | BNCC259680 | *Microsporum canis* |

**Table 1-5a Sources of various macromolecules, medium molecules, or oligomer molecules in examples**

| Vendor name | Product name | Product No. |
|---|---|---|
| Wuhan ABclonal Biotechnology Co. Ltd. | SBP1: IEEQAKTFLDKFNHEAEDLFYQS-NH2, (C-terminal amide), (Examples 9, 10, 24, 35, 38, and 41) | - |
| Wuhan ABclonal Biotechnology Co. Ltd. | Recombinant Human CD14 Protein | RP00990 |
| | Examples 11 and 25 | |
| Solarbio | Bovine serum albumin (Examples 1, 2, 4, 5, 6, 35, 36, and 48) | A8850 |
| Solarbio | Human serum albumin (Examples 3, 20, 32, 33, 37, 41, 43, 44, 48, 50(1), and 51(1)) | A8230 |
| Shanghai Macklin Biochemical Technology Co. Ltd. | Hyaluronic acid 40-100 KDa (Examples 8 and 40) | H874944 |
| Shanghai Macklin Biochemical Technology Co. Ltd. | Hyaluronic acid 10-200 000 D (Examples 7, 21, and 39) | H909935 |
| Shanghai Macklin Biochemical Technology Co. Ltd. | Hyaluronic acid 200, 000-400, 000 Da (Examples 13 and 35) | H909936 |
| Shanghai Macklin Biochemical Technology Co. Ltd. | Hyaluronic acid 400, 000-800, 000 Da (Examples 12, 42, 50(2), and 51(2)) | H909937 |
| Shanghai Macklin Biochemical Technology Co. Ltd. | PEG-COOH (Example 18) | - |

**Table 1-5b Sources of various macromolecules, medium molecules, or oligomer molecules in examples**

| Vendor name | Product name |
|---|---|
| Shanghai jinpan Biotech Co., Ltd. | DSPE PEG Amine, DSPE-PEG-NH₂, 1000 Da (Example 19 (1)) |
| Shanghai jinpan Biotech Co., Ltd. | DSPE PEG Amine, DSPE-PEG-NH₂, 2000 Da (Example 23 (2)) |
| Shanghai jinpan Biotech Co., Ltd. | DSPE PEG Acid, DSPE-PEG-COOH, 1000Da (Example 19 (2)) |
| Shanghai jinpan Biotech Co., Ltd. | DSPE PEG Acid, DSPE-PEG-COOH, 2000Da (Example 23 (1)) |

### Example 1 Preparation and characterization of human serum albumin/bovine serum albumin grafted fatty acid complex (acting moiety + macromolecular water-soluble moiety/binding moiety)

The reaction equation 1-1 is as follows:

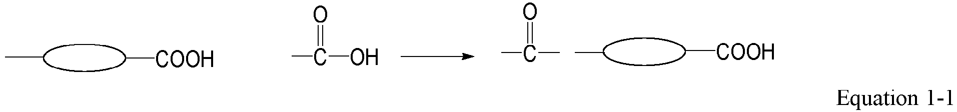

In this example, the molar ratio of fatty acid to albumin was 10: 1. Fumaric acid, octanoic acid, undecanoic acid, hexadecenoic acid, oleic acid, linoleic acid, linolenic acid, eicosapentaenoic acid (EPA), docosahexaenoic acid (DHA), and triacontenoic acid were respectively selected as the fatty acid to react with the serum albumin. The molar ratio of catalyst to fatty acid was 1: 1. 1-Ethyl - (3-dimethylaminopropyl) carbodiimide (EDC) and N-hydroxysulfonylsuccinimide were selected as the catalyst. The reaction process is as follows:
0.36 mmol of fatty acid, 0.36 mmol of EDC and 0.36 mmol of sulfo-NHS were accurately weighed. An acid was added to catalyze and activate the carboxyl groups to form an activated fatty acid.15 min of stirring for activation was performed under an ice bath. 0.0375 mmol of bovine serum albumin (calculated as mass: 2.5 g) was accurately weighed and dissolved in 5 ml of PBS solution (phosphate buffer solution). NaOH solution was added to adjust pH to neutral, evenly stirred to obtain a serum albumin solution. The resulting serum albumin solution was added to the activated fatty acid in stirring. The reaction was continued with stirring in an ice bath overnight to obtain a fatty acid-serum albumin complex solution with a concentration of 72 mM. The the above-mentioned solution was precipitated with ice acetone. Then, the unreacted fatty acid was washed off in the precipitate with ethanol. The precipitate was dialyzed to be complete dissolved and small molecule impurities were removed at the same time (performing the dialysis using a dialysis bag with a cut-off molecular weight of 500-1000, and changing water every 4 hours. All the small molecule compounds with molecular weights less than 500 can be removed). Then gradient low-temperature drying was perfomed in a vacuum lyophilizer (-pre-freezing at 80°C for 24 hours and vacuuming for 12 hours, vacuuming at -20°C for 12 hours, and continuing to vacuum at 4°C for at least 24 hours until the product was completely dry) to obtain a complex of fatty acid-linked serum albumin. The vacuum-dried complex was used for subsequent performance evaluation on microorganisms. Serum albumin compounds of fumaric acid, octanoic acid, undecanoic acid, hexadecenoic acid, oleic acid, linoleic acid, linolenic acid, EPA, DHA, and triacontanoic acid were respectively prepared according to the above method, with the yields of fumaric acid 68.2%, octanoic acid 68.7%, undecanoic acid 68.9%, hexadecenoic acid 69.3%, oleic acid 68.4%, linoleic acid 68.2%, linolenic acid 69.0%, EPA 68.7%, DHA 68.6%, and triacontanoic acid 67.8%, respectively. Here, yields refer to the mass ratio of the final product relative to the total mass of the original reactants.

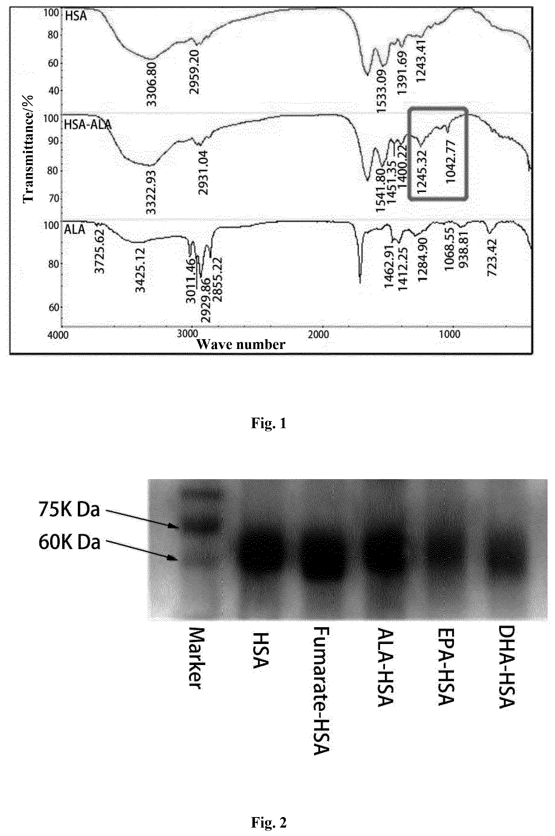

The Fourier transform infrared spectrum of linolenic acid- serum albumin is shown in Fig. 1. Compared with serum albumin, the serum albumin grafted linolenic acid (ALA-HSA) exhibits new absorption peaks at wave numbers of 1245 cm⁻¹ and 1042 cm⁻¹, which are assigned to γ = C-H (bending vibration of unsaturated carbon-hydrogen bond outside the plane) and ν_{C-N} absorption peak of amide, which serves as evidence to identify the amide bond group. After grafting, the absorption peak of the olefin at 1710 cm⁻¹ wavenumber shifts to the lower band due to conjugation, which all come from the grafted small molecule linolenic acid (ALA), so it could be preliminarily determined that ALA was successfully grafted onto the molecular chain of serum albumin.

As shown in Fig. 2, protein electrophoresis of fumaric acid-serum albumin complex, linolenic acid-serum albumin complex, eicosapentaenoic acid-serum albumin complex, and docosahexaenoic acid-serum albumin complex showed that the molecular weight of the product was between 60, 000 and 75, 000 Da, indicating that the serum albumin molecule itself was not polymerized, and the molecular weight did not change significantly due to the small molecules attached.

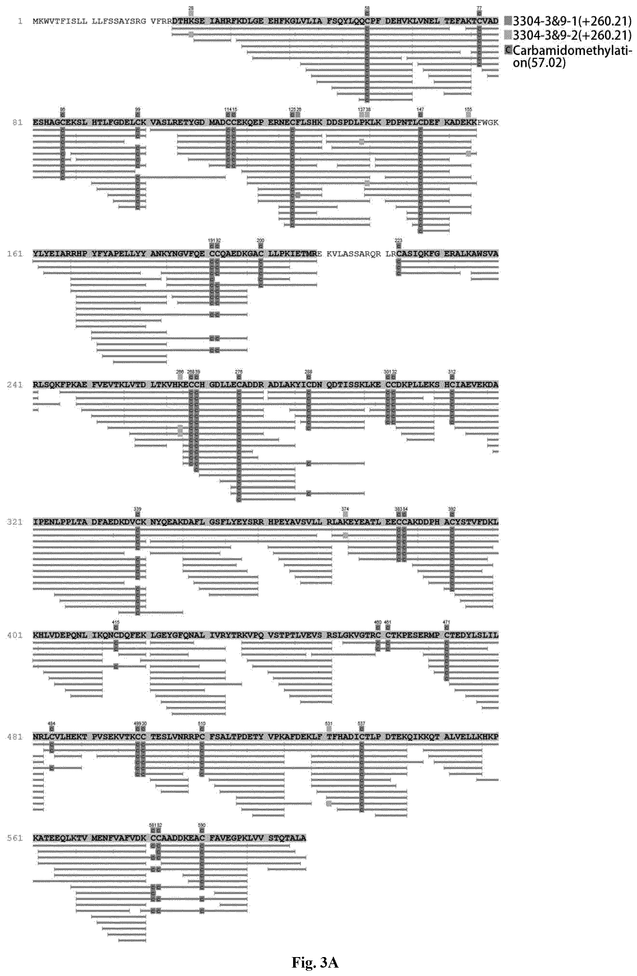

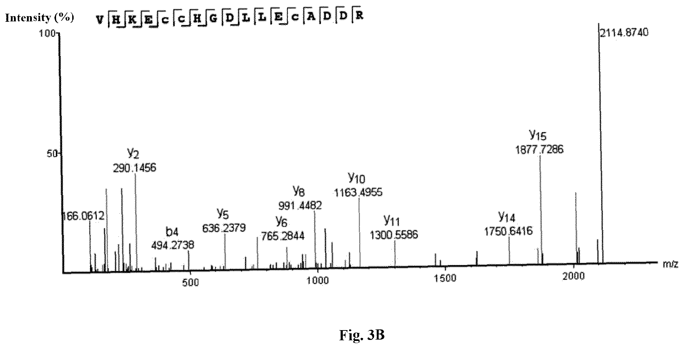

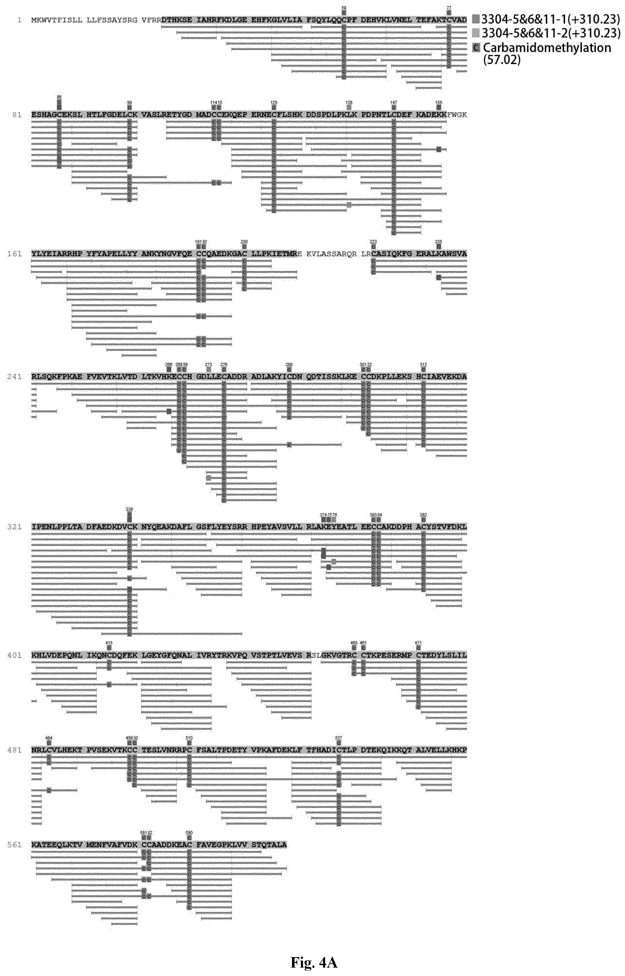

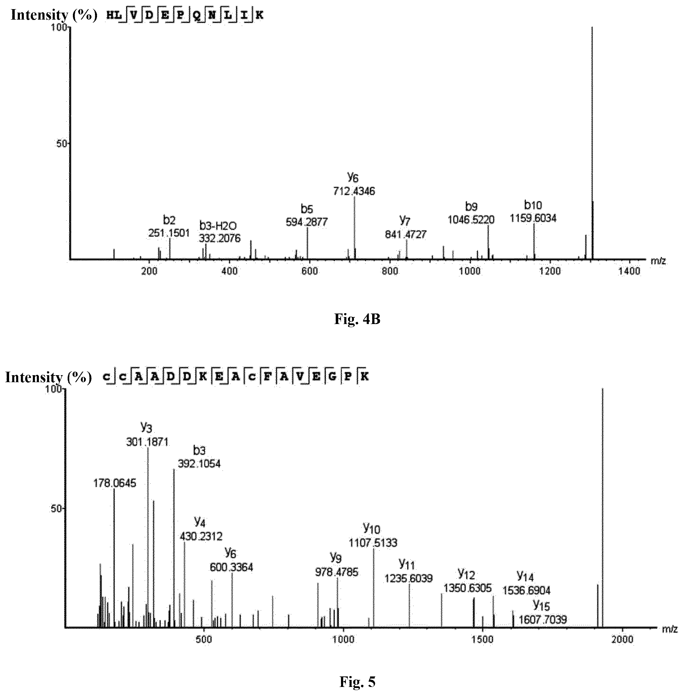

Linolenic acid-serum albumin and DHA-serum albumin were analyzed by LC-MS to determine the modification sites of serum albumin, which are shown in Fig. 3A and Fig. 3B, and Fig. 4A, and Fig. 4B, respectively. From the results in the figure and the sequence alignment of bovine serum albumin (a total of 607 amino acids, including 60 lysine, 17 glycine, 48 alanine, 38 valine, 65 leucine, 15 isoleucine, 30 phenylalanine, 3 tryptophan, 21 tyrosine, 40 aspartic acid, 14 asparagine, 59 glutamic acid, 20 glutamine, 5 methionine, 32 serine, 34 threonine, 35 cysteine, 28 proline, 17 histidine, and 26 arginine), it can be concluded that 1 molecule of serum albumin bound to 8 molecules of linolenic acid, with a total fatty acid binding efficiency of 1.32%. Among them, 1 molecule was a threonine binding, and the substitution degree of threonine was 2.94%; 1 molecule was phenylalanine binding, and the substitution degree of phenylalanine was 3.33%; 1 molecule was proline binding, and the substitution degree of proline was 3.57%; and 5 molecules were lysine bounding, and the substitution degree of the fatty acid in lysine was 8.33%.

1 molecule of serum albumin bound 10 molecules of docosahexaenoic acid, and the total fatty acid binding amino acid efficiency was 1.65%. Among them, 1 molecule was glutamic acid binding, and the substitution degree of glutamic acid was 1.69%; 1 molecule was tyrosine binding, and the substitution degree of tyrosine was 4.76%; 2 molecules were leucine binding, and the substitution degree of leucine was 3.08%; 2 molecules were cysteine binding, and the substitution degree of cysteine was 5.71%; and 4 molecules were lysine binding, and the substitution degree of lysine was 6.67%.

In this example, the serum albumin was both the water-soluble moiety and the binding moiety, and the fatty acid carbon chain is the acting moiety.

### Example 2 Preparation and characterization of bovine serum albumin grafted fatty acid complex (acting moiety + macromolecular water-soluble moiety/binding moiety)

0.36 mmol of oleic acid; 0.36 mmol of EDC, and 0.36 mmol of sulfo-NHS were accurately weighed. An acid was added to catalyze and activate the carboxyl groups to obtain an activated oleic acid.10 min of stirring for activation was performed under an ice bath. 0.018 mmol of bovine serum albumin (calculated as mass: 1.2 g) was accurately weighed and dissolved in 5 ml of PBS solution. NaOH solution was added to adjust pH to neutral, evenly stirred to obtain a serum albumin solution. The serum albumin solution was added to the stirred activated oleic acid in stirring. The reaction was continued with stirring in an ice bath overnight to obtain an oleic acid-albumin initial solution with an oleic acid concentration of 72 mM. The the above-mentioned solution was precipitated with ice acetone. Then, the fatty acids that did not participate in the reaction was washed off in the precipitate with ethanol. The small molecule impurities were removed by dialysis at the same time (performing the dialysis using a dialysis bag with a cut-off molecular weight of 500-1000, and changing water every 4 hours. All the small molecule compounds with molecular weights less than 500 can be removed). Then gradient low-temperature drying was perfomed in a vacuum lyophilizer (-pre-freezing at 80°C for 24 hours and vacuuming for 12 hours, vacuuming at -20°C for 12 hours, and continuing to vacuum at 4°C for at least 24 hours until the product was completely dry), with a yield of 68%. The vacuum-dried complex was used for subsequent performance evaluation on microorganisms.

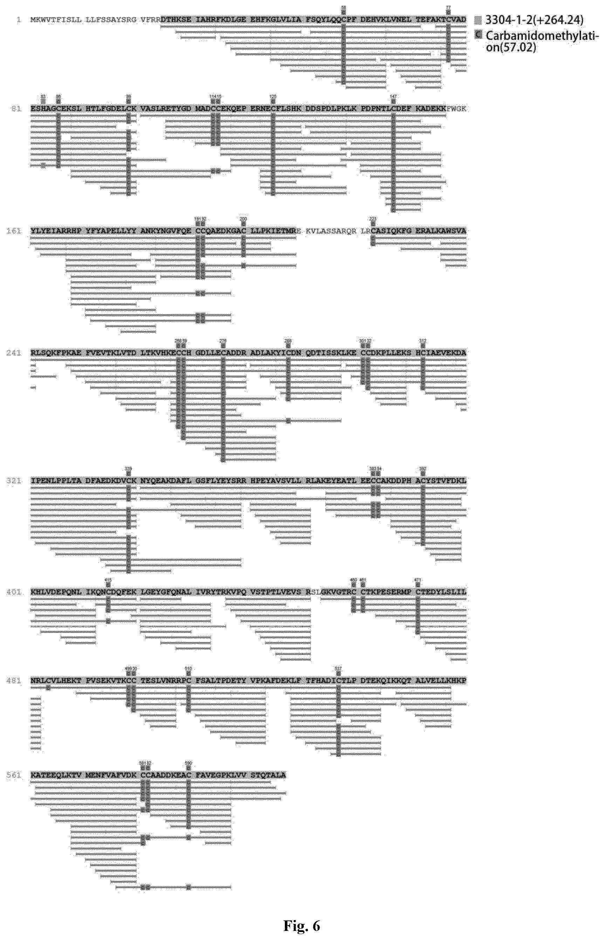

Analysis of the molecular structure of bovine serum albumin (a total of 607 amino acids, including 60 lysine, 17 glycine, 48 alanine, 38 valine, 65 leucine, 15 isoleucine, 30 phenylalanine, 3 tryptophan, 21 tyrosine, 40 aspartic acid, 14 asparagine, 59 glutamic acid, 20 glutamine, 5 methionine, 32 serine, 34 threonine, 35 cysteine, 28 proline, 17 histidine, and 26 arginine) using LC-MS revealed that, an amidation reaction occurred at the position of histidine, resulting in the removal of one molecule of water. The mass spectrum and amino acid sequence alignment are shown in FIGs. 5 and 6, respectively, leading to the conclusion that the serum albumin was coupled to oleic acid in such a manner that one oleic acid molecule coupled to one serum albumin molecule, with a total substitution degree of 0.16% for the fatty acid. Among them, histidine bound to one molecule, with a substitution degree of 5.88% for histidine.

In this example, the serum albumin was both the water-soluble moiety and the binding moiety, and the oleic acid carbon chain was the acting moiety.

### Example 3 Preparation and characterization of human serum albumin grafted fatty acid complex (acting moiety + macromolecular water-soluble moiety/binding moiety)

0.036 mmol of eicosapentaenoic acid (EPA), 0.036 mmol of catalyst (EDC), and 0.036 mmol of sulfo-NHS were accurately weighed. An acid was added to catalyze and activate the carboxyl groups to obtain an activated EPA. 20 min of stirring for activation was performed under an ice bath. 0.036 mmol of bovine serum albumin (calculated as mass: about 2.4 g) was accurately weighed and dissolved in 24 ml of PBS solution. NaOH solution was added to adjust pH to neutral, evenly stirred to obtain a serum albumin solution. The serum albumin solution was added to the activated EPA in stirring. The reaction was continued with stirring in an ice bath overnight to obtain an EPA-serum albumin initial solution with an EPA concentration of 1.5 mM. The the above-mentioned solution was precipitated with ice acetone. Then, the fatty acids that did not participate in the reaction was washed off in the precipitate with ethanol. The small molecule impurities were removed by dialysis at the same time (performing the dialysis using a dialysis bag with a cut-off molecular weight of 500-1000, and changing water every 4 hours. All the small molecule compounds with molecular weights less than 500 can be removed). Then gradient low-temperature drying was perfomed in a vacuum lyophilizer (-pre-freezing at 80°C for 24 hours and vacuuming for 20 hours, vacuuming at -20°C for 12 hours, and continuing to vacuum at 4°C for at least 24 hours until the product was completely dry), with a yield of 79%. The vacuum-dried complex was used for subsequent performance evaluation on microorganisms.

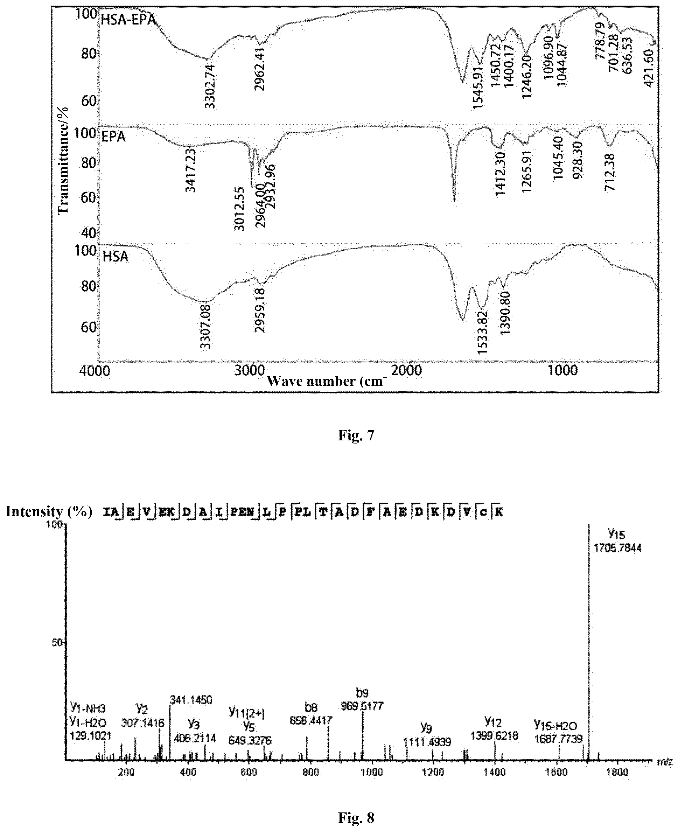

Fourier transform infrared spectroscopy (FTIR) is shown in Fig. 7. Compared with human serum albumin, the serum albumin grafted eicosapentaenoic acid (EPA-HSA) exhibits a strong new absorption peak at 1044 cm⁻¹ wavenumber, which is assigned to y = C-H (bending vibration of unsaturated carbon-hydrogen bond outside the plane) from the grafted small molecular eicosapentaenoic acid. The absorption peak at 1708 cm ⁻¹ wavenumber of the grafted olefin shifts to a lower band due to conjugation and become the main characteristic peak ν_{C=O} of amide. Therefore, it could be preliminarily determined that EPA was successfully grafted onto the molecular chain of serum albumin.

In this example, the serum albumin was both the water-soluble moiety and the binding moiety, and the eicosapentaenoic acid carbon chain was the acting moiety.

### Example 4 Preparation and characterization of bovine serum albumin grafted fatty acid complex (acting moiety + macromolecular water-soluble moiety/binding moiety)

0.036 mmol of eicosapentaenoic acid (EPA), 0.036 mmol of catalyst (EDC), and 0.036 mmol of sulfo-NHS were accurately weighed. An acid was added to catalyze and activate the carboxyl groups to obtain an activated EPA. 15min of stirring for activation was performed under an ice bath. 0.018 mmol of serum albumin (calculated as mass: about 1.2 g) was accurately weighed and dissolved in 10 ml of PBS solution. NaOH solution was added to adjust pH to neutral evenly stirred to obtain a serum albumin solution. The serum albumin solution was added to the activated EPA in stirring. The reaction was continued with stirring in an ice bath overnight to obtain an EPA-serum albumin initial solution with an EPA concentration of 36 mM. The the above-mentioned solution was precipitated with ice acetone. Then, the fatty acids that did not participate in the reaction was washed off in the precipitate with ethanol. The small molecule impurities were removed by dialysis at the same time (performing the dialysis using a dialysis bag with a cut-off molecular weight of 500-1000, and changing water every 4 hours. All the small molecule compounds with molecular weights less than 500 can be removed). Then gradient low-temperature drying was perfomed in a vacuum lyophilizer (-pre-freezing at 80°C for 24 hours and vacuuming for 15 hours, vacuuming at -20°C for 12 hours, and continuing to vacuum at 4°C for at least 24 hours until the product was completely dry), with a yield of 79%. The vacuum-dried complex was used for subsequent performance evaluation on microorganisms.

The setting of each time can be adjusted depending on how much solution is prepared.

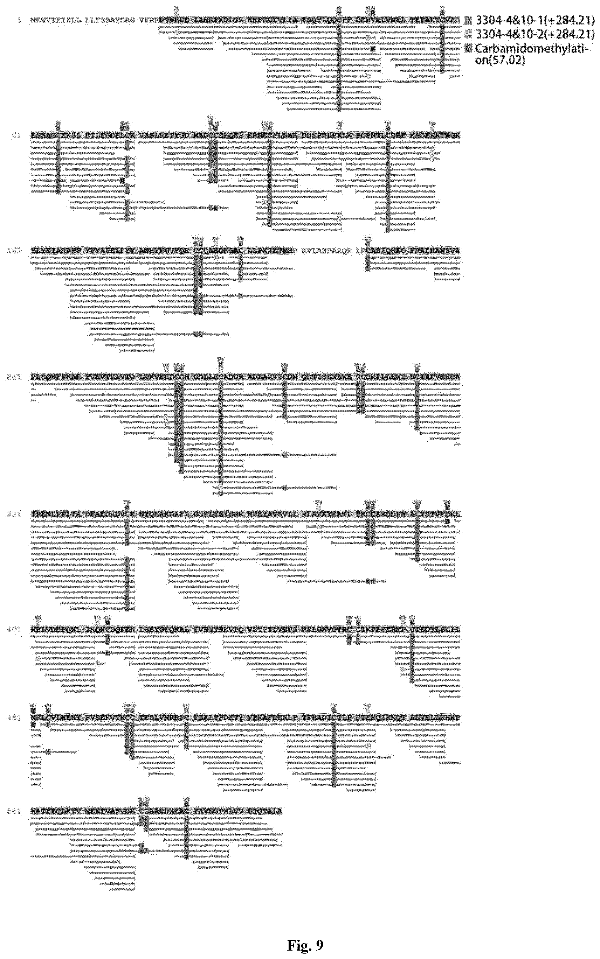

The structural changes of bovine serum albumin (a total of 607 amino acids, including 60 lysine, 17 glycine, 48 alanine, 38 valine, 65 leucine, 15 isoleucine, 30 phenylalanine, 3 tryptophan, 21 tyrosine, 40 aspartic acid, 14 asparagine, 59 glutamic acid, 20 glutamine, 5 methionine, 32 serine, 34 threonine, 35 cysteine, 28 proline, 17 histidine, and 26 arginine) were analyzed by LC-MS. The mass spectrogram and amino acid sequence alignment chart are shown in Fig. 8 and Fig. 9, respectively. It can be concluded that 17 EPA molecules were bonded to the amino group of 1 serum protein molecule by removing 1 molecule of water, and the total binding efficiency of fatty acid to amino acid was 2.8%. Among them, glutamic acid bound to 2 molecules, and the substitution degree of glutamic acid was 3.39%; histidine was bound to 2 molecules, and the substitution degree of histidine was 11.76%; cysteine bound to 2 molecules, and the substitution degree of cysteine was 5.71%; leucine, proline, aspartic acid, asparagine, valine, and glutamine respectively bound 1 molecule, and the substitution degrees were 1.54%, 3.57%, 2.5%, 7.14%, 2.63%, and 5%; and lysine bound to 5 molecules, and the substitution degree was 8.33%.

In this example, the serum albumin was both the water-soluble moiety and the binding moiety, and the eicosapentaenoic acid carbon chain was the acting moiety.

### Example 5 Preparation and characterization of bovine serum albumin grafted fatty acid complex (acting moiety + macromolecular water-soluble moiety/binding moiety)

0.036 mmol of linoleic acid; catalyst (0.036 mmol of EDC and 0.036 mmol of sulfo-NHS) were accurately weighed. An acid was added to catalyze and activate the carboxyl groups.10-30 min of stirring for activation was performed under an ice bath to obtain an activated linoleic acid. 0.036 mmol of serum albumin (calculated as mass: about 2.4 g) was accurately weighed and dissolved in 5 ml of PBS solution. NaOH solution was added to adjust pH to neutral evenly stirred to obtain a serum albumin solution. The serum albumin solution was added to the stirred activated linoleic acid in stirring. The reaction was continued with stirring in an ice bath overnight to obtain a linoleic acid-serum albumin initial solution with a linoleic acid concentration of 72 mM. The above-mentioned solution was precipitated with ice acetone. Then, the fatty acids that did not participate in the reaction was washed off in the precipitate with ethanol. The small molecule impurities were removed by dialysis at the same time (performing the dialysis using a dialysis bag with a cut-off molecular weight of 500-1000, and changing water every 4 hours. All the small molecule compounds with molecular weights less than 500 can be removed). Then gradient low-temperature drying was perfomed in a vacuum lyophilizer (-pre-freezing at 80°C for 24 hours and vacuuming for 12 hours, vacuuming at -20°C for 12 hours, and continuing to vacuum at 4°C for at least 24 hours until the product was completely dry), with a yield of 79%. The vacuum-dried complex was used for subsequent performance evaluation on microorganisms.

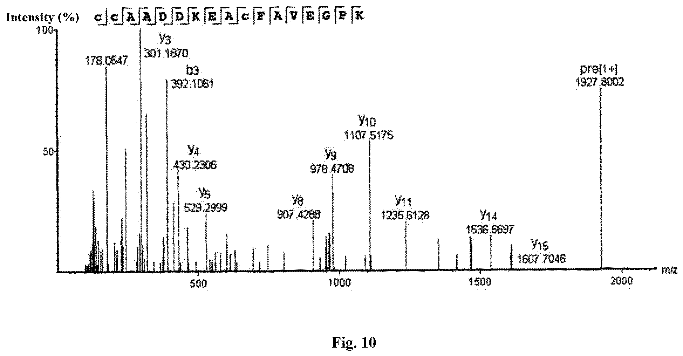

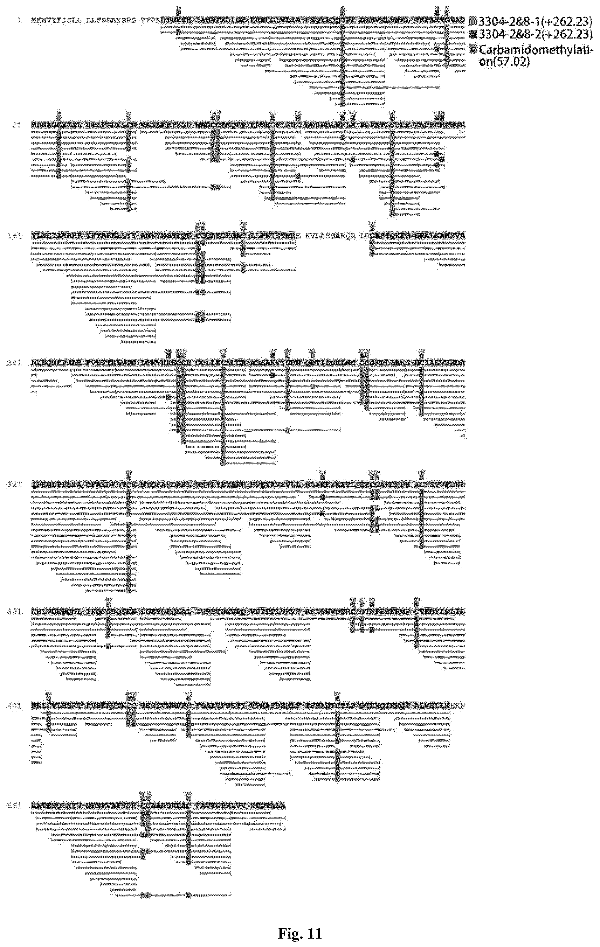

The molecular weight of bovine serum albumin (a total of 607 amino acids, including 60 lysine, 17 glycine, 48 alanine, 38 valine, 65 leucine, 15 isoleucine, 30 phenylalanine, 3 tryptophan, 21 tyrosine, 40 aspartic acid, 14 asparagine, 59 glutamic acid, 20 glutamine, 5 methionine, 32 serine, 34 threonine, 35 cysteine, 28 proline, 17 histidine, and 26 arginine) was analyzed by LC-MS. It can be concluded that 12 linoleic acid molecules were bonded to the amino acid of 1 serum albumin molecule in the form of removing one molecule of water, with a total substitution degree of 1.98%. Among them, lysine bound to 11 molecules, and the substitution degree of lysine was 18.33%; and aspartic acid bound to 1 molecule, and the substitution degree of aspartic acid was 2.5%. Mass spectra and amino acid sequence alignments are shown in FIGs. 10 and 11, respectively.

In this example, the serum albumin was both the water-soluble moiety and the binding moiety, and the linoleic acid carbon chain was the acting moiety.

### Example 6 Preparation and characterization of human serum albumin/bovine serum albumin grafted fatty acid complex (acting moiety + macromolecular water-soluble moiety/binding moiety)

0.36 mmol of docosahexaenoic acid (DHA), and catalyst (0.36 mmol of EDC and 0.36 mmol of sulfo-NHS were accurately weighed. An acid was added to catalyze and activate the carboxyl groups to obtain an activated DHA. 10-30 min of stirring for activation was performed under an ice bath. 0.036 mmol of serum albumin (calculated as mass: about 2.4 g) was accurately weighed and dissolved in 5 ml of normal saline. NaOH solution was added to adjust pH to neutral evenly stirred to obtain a serum albumin solution. The serum albumin solution was added to the activated DHA in stirring. The reaction was continued with stirring in an ice bath overnight to obtain a DHA-albumin solution with a concentration of 72mM (here, the concentration refers to the molar concentration of the complex DHA-albumin in the reaction mixture solution, and the following examples have similar meanings). The the above-mentioned solution was precipitated with ice acetone. Then, the fatty acids that did not participate in the reaction was washed off in the precipitate with ethanol. The small molecule impurities were removed by dialysis at the same time (performing the dialysis using a dialysis bag with a cut-off molecular weight of 500-1000, and changing water every 4 hours. All the small molecule compounds with molecular weights less than 500 can be removed). Then gradient low-temperature drying was perfomed in a vacuum lyophilizer (-pre-freezing at 80°C for 24 hours and vacuuming for 12 hours, vacuuming at -20°C for 12 hours, and continuing to vacuum at 4°C for at least 24 hours until the product was completely dry), with a yield of 79%. The vacuum-dried complex was used for subsequent performance evaluation on microorganisms.

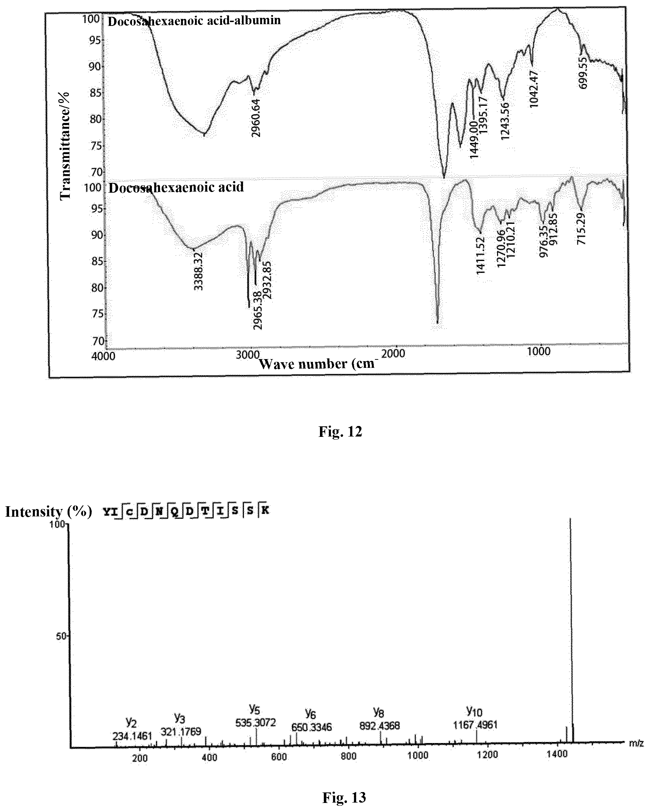

Fourier transform infrared spectroscopy (FTIR) is shown in Fig. 12. Compared with docosahexaenoic acid, the docosahexaenoic acid grafted serum albumin exhibits a stronger new absorption peak at 1044 cm⁻¹ wavenumber, which was assigned to y = C-H (bending vibration of unsaturated carbon-hydrogen bond outside the plane) from the grafted small molecule docosahexaenoic acid (DHA). The absorption peak at 1708 cm⁻¹ wavenumber of the grafted olefin shifted to lower band due to conjugation, which was due to the ν_{C=O} of carboxyl group in docosahexaenoic acid becoming ν_{C=O} of amide bond after binding with serum albumin. Therefore, it can be preliminarily determined that DHA was successfully grafted onto the molecular chain of serum albumin.

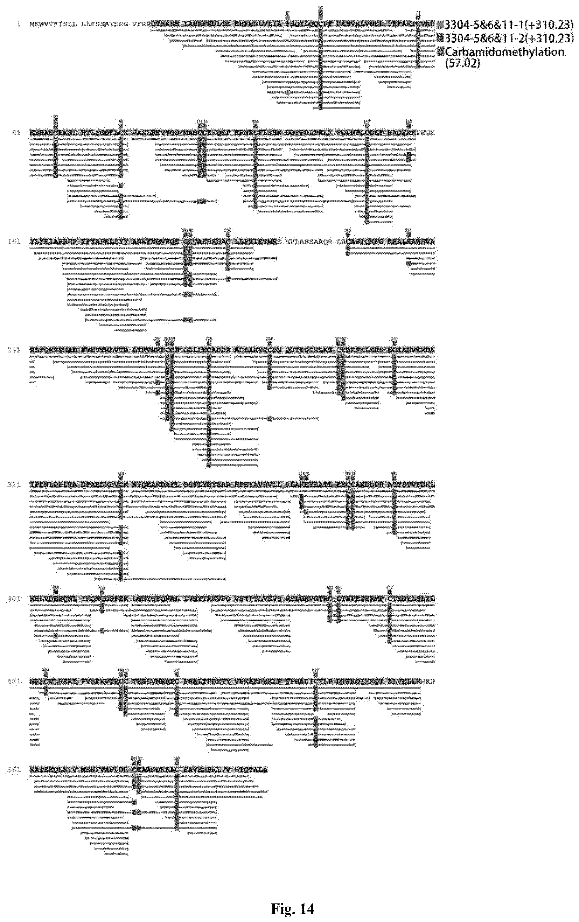

Further, the structural changes of bovine serum albumin (a total of 607 amino acids, including 60 lysine, 17 glycine, 48 alanine, 38 valine, 65 leucine, 15 isoleucine, 30 phenylalanine, 3 tryptophan, 21 tyrosine, 40 aspartic acid, 14 asparagine, 59 glutamic acid, 20 glutamine, 5 methionine, 32 serine, 34 threonine, 35 cysteine, 28 proline, 17 histidine, and 26 arginine) were analyzed by LC-MS. The mass spectrogram and amino acid sequence alignment chart are shown in Fig. 13 and Fig. 14, respectively. It can be concluded that 9 DHA molecules were bonded to the amino acid of 1 serum albumin molecule in the form of removing one molecule of water, with the total fatty acid substitution of 1.48%. Among them, phenylalanine bound 1 molecule, and the substitution degree of phenylalanine was 3.33%; glutamic acid bound to 2 molecules, and the substitution degree of glutamic acid was 3.39%; cysteine bound to 2 molecules, and the substitution degree of cysteine was 5.71%; and lysine bound to 4 molecules, the substitution degree of lysine was 6.67%.

In this example, the serum albumin was both the water-soluble moiety and the binding moiety, and the docosahexaenoic acid carbon chain was the acting moiety.

### Example 7 Preparation of unsaturated fatty acid coupled hyaluronic acid complex (acting moiety + macromolecular water-soluble moiety/binding moiety)

There are 4 alcoholic hydroxyl groups on the single molecular unit of hyaluronic acid which can be esterified with carboxyl groups of fatty acid molecules.

According to the molar ratio of fatty acid carboxyl groups to hyaluronic acid hydroxyl groups of 4n: 1 to 1: 1, and the molar ratio of catalyst to fatty acid carboxyl groups of 10: 1 to 1: 1, the catalyst can be one or more of EDC, DCC, NHS, DMAP, HoBt, derivatives and analogues thereof, etc.

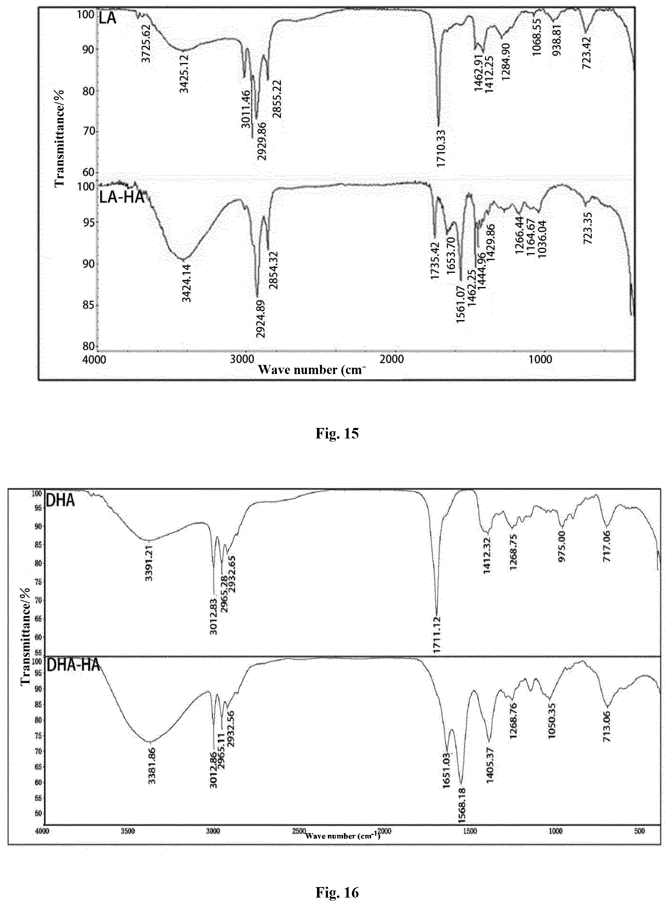

Specifically in this example, 0.001 mmol of linoleic acid was accurately weighed and added with 0.001 mmol of EDC and 0.001 mmol of DMAP as the catalysts. An acid solution was added to stir and activate for 10 min to obtain an activated linoleic acid. 0.0025 mmol of hyaluronic acid (molecular weight: 200 kDa; calculated by mass: 20 mg) was accurately weighed and dissolved in 5 ml of normal saline. NaOH solution was added to adjust pH to neutral and stirred well to obtain hyaluronic acid solution. The hyaluronic acid solution was added to the activated linoleic acid in stirring and continued stirring for reaction at room temperature for 12 h to obtain a linoleic acid-hyaluronic acid solution with a concentration of 72 mM. After the reaction was finished, the linoleic acid-hyaluronic acid reaction solution was dialyzed using a dialysis bag with a molecular weight cut-off of 500-1000 to purify the compound obtained in the reaction. The water was changed every 4 h (the molecular weight of both the catalyst and the unreacted fatty acid was less than 500 and can be removed). Dialysis was performed for 24 h. The obtaining efficiency of the product was calculated in the same manner as in Examples 1 and 2, and the yield was 75%. The Fourier transform infrared spectrum of linoleic acid hyaluronic acid was shown in Fig. 15. The complex exhibits an absorption peak at the wavelength of 1735 cm⁻¹, which was assigned to the absorption peak of v C=O. Therefore, it can be preliminarily determined that linoleic acid has successfully grafted onto the hyaluronic acid molecular chain.

In this example, the hyaluronic acid was both the water-soluble moiety and the binding moiety, and the linoleic acid carbon chain was the acting moiety.

### Example 8 Preparation of polyunsaturated fatty acid coupled hyaluronic acid complex (acting moiety + macromolecular water-soluble moiety/binding moiety)

0.36 mmol of docosahexaenoic acid (DHA) was accurately weighed, and added with 0.36 mmol of EDC and 0.36 mmol of DMAP as the catalysts. An acid solution was added to stir and activate for 10 min to obtain activated DHA. 0.045 mmol of hyaluronic acid (molecular weight: 50 kDa; calculated by mass: 2.5 mg) was accurately weighed and dissolved in 5 ml of normal saline. NaOH solution was added to adjust pH to neutral and stirred well to obtain hyaluronic acid solution. The hyaluronic acid solution was added to the activated DHA in stirring and continued stirring for reaction at room temperature for 8-24 h to obtain a DHA-hyaluronic acid solution with a concentration of 72 mM. The compound obtained in the reaction was purified using the same purification operation procedure as that of example 7 to remove the unreacted fatty acid and the catalysts. The obtaining efficiency of the product was calculated in the same manner as in Examples 1 and 2, and was 70%. The Fourier infrared spectrum of docosahexaenoic acid-hyaluronic acid is shown in Fig. 16. Since the ring tension of the six-membered ring of hyaluronic acid increases the ν _{C=C} frequency at 1412 cm⁻¹, and the absorption peaks appear at the wave numbers of 1649 cm⁻¹ and 1568 cm⁻¹ of the complex, which shifts the v _{C=O} absorption peak (1649 cm⁻¹) to a lower band (the characteristic v _{C=O} absorption peak in general esters is at 1750-1735 cm⁻¹). Therefore, it can be preliminarily determined that docosahexaenoic acid was successfully grafted onto the molecular chain of hyaluronic acid.

Eicosapentaenoic acid (EPA)-hyaluronic acid complex was prepared in the same manner for subsequent experiments.

In this example, the hyaluronic acid was both the water-soluble moiety and the binding moiety, and eicosapentaenoic acid and docosahexaenoic acid carbon chains were the acting moieties.

### Example 9 Preparation of unsaturated fatty acid-polypeptide complex (acting moiety + target binding moiety/water-soluble moiety)

A fatty acid can be bonded to the molecular structure of the polypeptide through amide bonds (free carboxyl groups of the fatty acid and free amino groups of the polypeptide) and ester bonds (free carboxyl groups of the fatty acid and free hydroxyl groups of the polypeptide).

The peptide SBP1 (ACE2 derived peptide; binds SARS-CoV-2 spike protein receptor binding domain) was taken as an example. Its sequence is: IEEQAKTFLDKFNHEAEDLFYQS (modification: Ser-23 = C-terminal amide), containing 6 free amino groups. The carboxyl group of the fatty acid can be reacted with the amino group. Using the polypeptide as a carrier, the molar ratio of the fatty acid to the polypeptide was 8: 1 to 1: 4; the molar ratio of the catalyst to the fatty acid was 0.5: 1 to 10: 1. A carbodiimide and a succinimide can be used as the catalysts, and the ratio of the two catalysts was 1: 1 to 1: 10.

Oleic acid (OA), linoleic acid (LA), linolenic acid (ALA), eicosapentaenoic acid (EPA), and docosahexaenoic acid (DHA) were respectively reacted with SBP1 in this example.

0.36 mmol of fatty acid and 0.36 mmol of catalysts were accurately weighed. An acid was added to catalyze and activate the carboxyl group. 10 min of stirring for activation was performed obtain an activated fatty acid. 0.72 mmol of polypeptide SBP1 was accurately weighed and dissolved in 20 ml of normal saline. NaOH solution was added to adjust the pH and evenly stirred to obtain an SBP1 solution. The polypeptide SBP1 solution was added to the activated fatty acid in stirring and continued stirring for the reaction under an ice bath for 1 h so as to obtain a fatty acid-SBP1 solution with a concentration of 36 mM. The compound obtained in the reaction was purified using the same purification operation procedure as that of example 7 to remove the unreacted fatty acid and the catalysts. The compound was frozen at - 80°C and dried under vacuum. The compound obtained by vacuum-drying was used for subsequent performance evaluation on microorganisms.

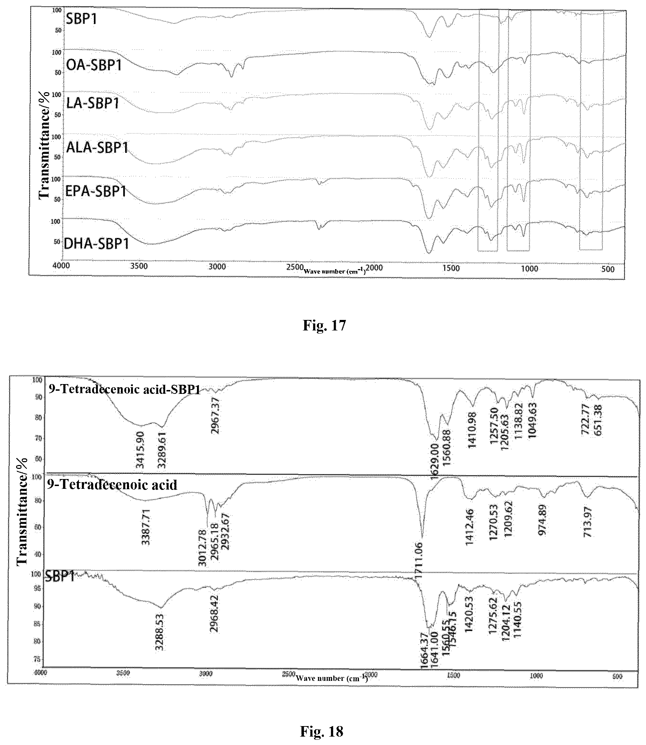

Fourier transform infrared spectrum of the prepared fatty acid-SBP1 complex is shown in Fig. 17. It is determined that fatty acids (oleic acid (OA), linoleic acid (LA), linolenic acid (ALA), eicosapentaenoic acid (EPA), and docosahexaenoic acid (DHA)) were successfully grafted onto the SBP1 molecular chain.

In this example, SBP1 was both the water-soluble moiety and the binding moiety, and the unsaturated carbon chain of the fatty acid was the acting moiety.

### Example 10 Preparation of unsaturated fatty acid-polypeptide complex (acting moiety + target binding moiety/water-soluble moiety)

Using the polypeptide SBP1 as a carrier, the molar ratio of fatty acid to polypeptide SBP1 as 7: 1 to 1: 2; the molar ratio of catalyst to fatty acid was 0.5: 1 to 10: 1. Carbodiimide and succinimide were used as catalysts, and the ratio of the two catalysts was 1: 1 to 1: 10.

This example adopted 9-tetradecenoic acid to react with SBP1.

0.36 mmol of fatty acid and 0.36 mmol of catalysts were accurately weighed. An acid was added to catalyze and activate the carboxyl group. 10 min of stirring for activation was performed obtain an activated fatty acid. 0.36 mmol of SBP1 was accurately weighed and dissolved in 20 ml of normal saline. NaOH solution was added to adjust the pH and evenly stirred to obtain an SBP1 solution. The SBP1 solution was added to the activated fatty acid in stirring and continued stirring for the reaction under an ice bath for 1 h so as to obtain a 9-tetradecenoic acid-SBP1 solution with a concentration of 18 mM. The compound obtained in the reaction was purified using the same purification operation procedure as that of example 7 to remove the unreacted fatty acid and the catalysts. The compound was frozen at -80°C and dried under vacuum.

The Fourier transform infrared spectrum of 9-tetradecenoic acid-SBP1 is shown in Fig. 18. It is preliminarily determined that 9-tetradecenoic acid was successfully grafted onto the molecular chain of SBP1.

In this example, SBP1 was both the water-soluble moiety and the binding moiety, and the unsaturated carbon chain of the fatty acid was the acting moiety.

### Example 11 Preparation of unsaturated fatty acid-cd14 protein complex (acting moiety + target binding moiety/water-soluble moiety)

A fatty acid can be bonded to the molecular structure of the protein through amide bonds (free carboxyl groups of the fatty acid and free amino groups of the protein) and ester bonds (free carboxyl groups of the fatty acid and free hydroxyl groups of the protein).

CD14 (34 kDa) was taken as an example, and its sequence is: TTPEPCELDDEDFRCVCNFSEPQPDWSEAFQCVSAVEVEIHAGGLNLEPFLKRVDADADPRQYAD TVKALRVRRLTVGAAQVPAQLLVGALRVLAYSRLKELTLEDLKITGTMPPLPLEATGLALSSLRL RNVSWATGRSWLAELQQWLKPGLKVLSIAQAHSPAFSYEQVRAFPALTSLDLSDNPGLGERGLM AALCPHKFPAIQNLALRNTGMETPTGVCAALAAAGVQPHSLDLSHNSLRATVNPSAPRCMWSSA LNSLNLSFAGLEQVPKGLPAKLRVLDLSCNRLNRAPQPDELPEVDNLTLDGN PFLVPG, containing 47 free amino groups.

In this example, oleic acid, linoleic acid, linolenic acid, eicosapentaenoic acid (EPA), and docosahexaenoic acid (DHA) were selected to react with CD14, respectively. The molar ratio of carboxyl group of the fatty acid to CD14 was 17: 1 to 1: 1. Carbodiimide and succinimide were used as catalysts, and the ratio of the two catalysts was 1: 1 to 1: 10. The reaction method is as described in Examples 1 and 2.

0.36 mmol of fatty acid and 0.36 mmol of catalysts were accurately weighed. An acid was added to catalyze and activate the carboxyl group. 30min of stirring for activation was performed obtain an activated fatty acid. 0.021 mmol of CD14 was accurately weighed and dissolved in 100 ml of normal saline. NaOH solution was added to adjust the pH and evenly stirred to obtain a CD14 solution. The CD14 solution was added to the activated fatty acid in stirring and continued stirring for the reaction under an ice bath overnight so as to obtain a fatty acid-CD14 solution with a concentration of 3.6 mM. The compound obtained in the reaction was purified using the same purification operation procedure as that of example 7 to remove the unreacted fatty acid and the catalysts. The compound was frozen at -80°C and dried under vacuum. The product yields were 87.3%, 87.6%, 86.8%, 89.0%, and 88.7%, respectively.

In this example, CD14 was both the water-soluble moiety and the binding moiety, and the unsaturated carbon chain of the fatty acid was the acting moiety.

### Example 12 Preparation of saturated fatty acid-hyaluronic acid complex (acting moiety + macromolecular water-soluble part/binding moiety)

In this example, hexanoic acid, octanoic acid, nonanoic acid, dodecanoic acid, tetradecanoic acid, hexadecanoic acid, octadecanoic acid, and eicosanoic acid were respectively selected to be reacted with hyaluronic acid to prepare a saturated fatty acid-hyaluronic acid complex. The molar ratio of the carboxyl group of the fatty acid to the hyaluronic acid was 4n: 1 to 1: 1 (n was the number of repetitions of a single molecular unit of hyaluronic acid, and n was an integer from 1 to 2000). The molar ratio of the catalyst to the fatty acid was 0.5: 1 to 2: 1. Carbodiimide and dimethylamino pyridine were the catalysts, and the molar ratio of the two was 1: 1 to 1: 10.

Specifically, it was performed according to the following Reaction equation 12-1. 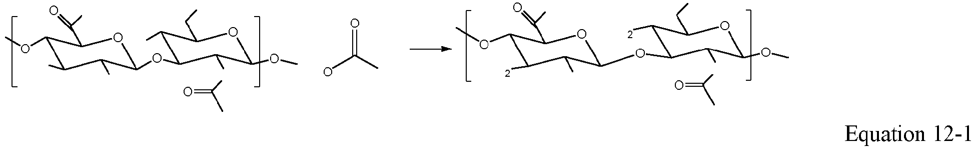 where n is the number of repetitions of the hyaluronic acid monomolecular unit, and n is an integer of 1-2000.

Wherein the R groups are saturated C5, C7, C9, C11, C13, C15, C17, C19 carbon chains, respectively. The reaction process is as follows:
0.36 mmol of fatty acid was accurately weighed and added with 0.4 mmol of EDC and 0.4 mmol of DMAP as the catalysts. An acid solution was added to stir and activate for 10 min to obtain an activated fatty acid. 0.00068 mmol of hyaluronic acid (molecular weight: 500 kDa; calculated by mass: 340 mg) was accurately weighed and dissolved in 5 ml of normal saline. NaOH solution was added to adjust pH to neutral and stirred well to obtain hyaluronic acid solution. The hyaluronic acid solution was added to the activated fatty acid in stirring and continued stirring for reaction at room temperature for 12 h to obtain a fatty acid-hyaluronic acid solution with a concentration of 72 mM. The compound obtained in the reaction was purified using the same purification operation procedure as that of example 7 to remove the unreacted fatty acid and the catalysts.

In this example, the hyaluronic acid was both the water-soluble moiety and the binding moiety, and the saturated carbon chain of the fatty acid was the acting moiety.

### Example 13 Preparation of unaturated fatty acid-hyaluronic acid complex (acting moiety + macromolecular water-soluble part/binding moiety)

Preparation of glutaconic acid-hyaluronic acid. The molar ratio of the carboxyl group of the fatty acid to the hyaluronic acid was 4n: 1 (n was the number of repetitions of a single molecular unit of hyaluronic acid, and n was an integer from 1 to 2000). The molar ratio of the catalyst to the fatty acid was 0.5: 1 to 2: 1. Carbodiimide and dimethylamino pyridine were the catalysts, and the molar ratio of the two was 1: 1 to 1: 10.

It is performed according to the following Reaction equation 13-1.

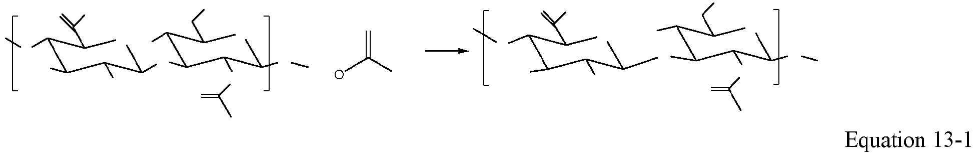

Wherein the R group is a carbon chain of 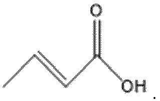 The reaction process is as follows:
0.36 mmol of glutaconic acid was accurately weighed and added with 0.4 mmol of EDC and 0.4 mmol of DMAP as the catalysts. An acid solution was added to stir and activate for 10 min to obtain an activated glutaconic acid. 0.0014 mmol of hyaluronic acid (molecular weight: 300 kDa; calculated by mass: 420 mg) was accurately weighed and dissolved in 5 ml of normal saline. NaOH solution was added to adjust pH to neutral and stirred well to obtain hyaluronic acid solution. The hyaluronic acid solution was added to the activated glutaconic acid in stirring and continued stirring for reaction at room temperature for 12 h to obtain a glutaconic acid-hyaluronic acid solution with a concentration of 72 mM. The compound obtained in the reaction was purified using the same purification operation procedure as that of Example 7 to remove the unreacted fatty acid and the catalysts.

In this example, the hyaluronic acid was both the water-soluble moiety and the binding moiety, and the saturated carbon chain of the glutaconic acid was the acting moiety.

### Example 14 Preparation of saturated carbon chain with 8 carbon atoms-glucose complex (small molecule water-soluble moiety + acting moiety) and performance evaluation

In this example, octanoic acid was used to prepare a saturated fatty acid glucose complex, which was performed according to the following reaction equation 14-1:

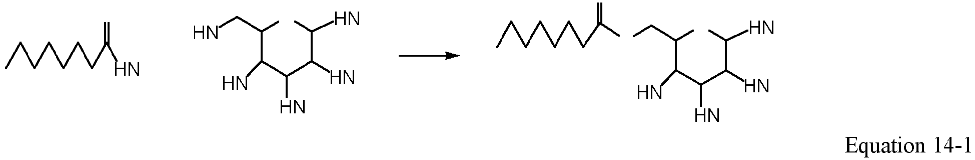

The reaction process is as follows:
0.72 mmol of octanoic acid was weighed and added with 0.72 mmol of EDC and 0.72 mmol of DMAP as the catalysts; and the mixture was stirred and activated for 10 min under an ice bath to obtain an activated octanoic acid. 1.44 mmol of glucose was weighed and dissolved in 10 ml of deionized water; and the mixture was added to the activated octanoic acid solution. The pH value was adjusted to 7.0-7.4 with NaOH, and the reaction was stirred at room temperature for 12 h to obtain a reaction product solution. After the reaction was completed, chromatographic purification was performed. The product solution was fed to a Shephadex G10 chromatographic column (Φ26 mm x 50 cm), eluted with normal saline at a flow rate of 50 ml/h. The eluents were collected step by step, and detected with phenol sulfuric acid method. The first eluting peaks were combined to obtain the reaction product.

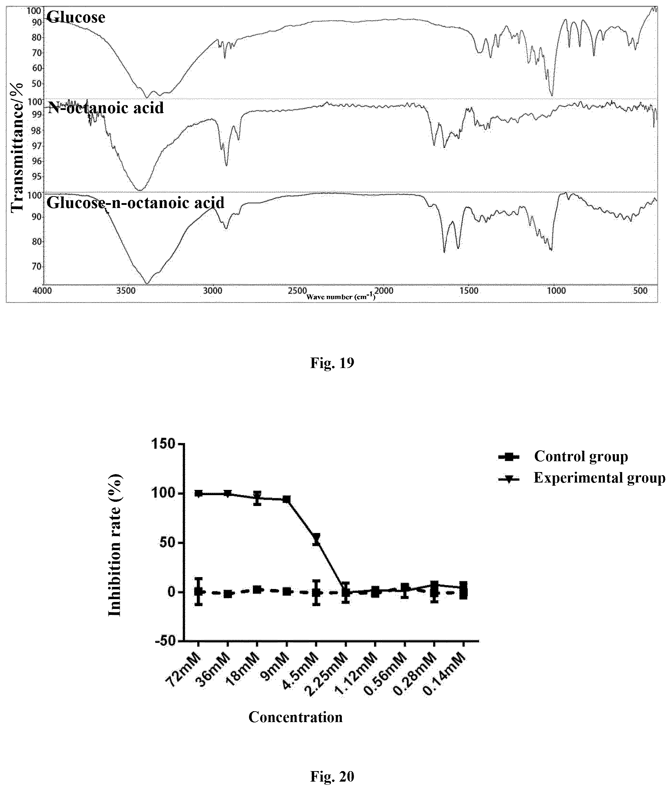

That is, an octanoic acid-glucose complex. The infrared spectrum of the prepared complex is shown in Fig. 19. It can be seen from the figure that due to the fact that only the hydroxyl group of glucose retains the ν_{c-o} peak at 1000-1250 cm⁻¹ when combined with octanoic acid, and the ester bond formed between octanoic acid and glucose shifts its absorption peak from the carboxyl characteristic peak at 1740 cm⁻¹ to the short band at 1660 cm⁻¹ due to conjugation with the hydroxyl group of glucose molecules.

The inhibition rate of bacteria (such as *Staphylococcus aureus*) was tested, including the following test processes: Preparation of the culture medium: the culture medium was prepared using LB agar according to the product manual, pH 7.2-7.4. Preparation and Inoculation of Inoculum: the bacteria were diluted into 10⁵-10⁶ CFU. 100 ul of bacteria were taken, added with 900 ul of liquid medicines that were diluted to different concentrations, and incubated at 37°C for 2 h. Then, the solution was diluted 100 times. 100 ul of the diluted solution was taken, plated, and incubated at 37°C for 16-24 h. The colonies were counted, and the inhibition rate was calculated. The inhibition results are shown in Fig. 20. When the concentration was 72 mM-9 mM, the inhibition rate was greater than 99%, with the antibacterial property. The half maximal inhibition concentration was 4.5 Mm. When the concentration was between 4.5 mM-0.14 mM, the inhibition was less than 50%, without the antibacterial property.

In this example, glucose was both the water-soluble moiety and the binding moiety, and the fatty acid saturated carbon chain was the acting moiety. The inhibition results of this complex are summarized in Table 2-1.

**Table 2-1 Bactericidal antibacterial properties of saturated carbon chain with 8 carbon atoms-glucose (9 mM) (2 h)**

| Tested microorganism | Sterilization rate (%) |
|---|---|
| *Escherichia coli* | >99 |
| Methicillin-resistant *Streptococcus pneumoniae* | >99 |
| *Streptococcus pneumoniae* | >99 |
| *Klebsiella pneumoniae* | >99 |
| *Pseudomonas aeruginosa* | >99 |

### Example 15 Preparation and performance evaluation of saturated carbon chain with 8 carbon atoms-sucrose complex (small molecule water-soluble moiety + acting moiety)

In this example, octanoic acid was used to prepare a saturated fatty acid sucrose complex. The reaction was carried out according to the following reaction equation 15-1:

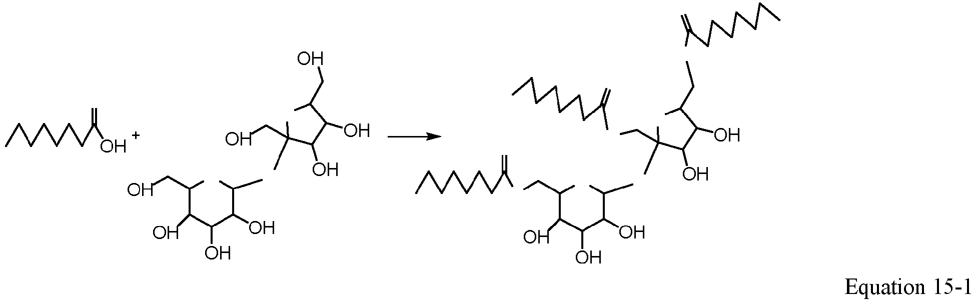

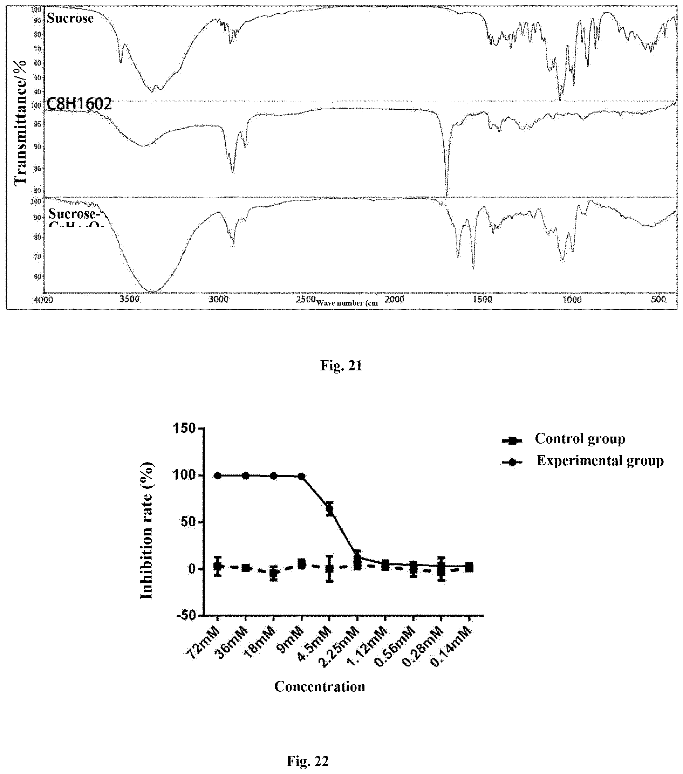

The reaction process is as follows:
0.72 mmol of octanoic acid was taken and added with 0.72mmol of EDC, 0.72 mmol of DMAP as catalysts; and the mixture was stirred and activated for10 min under an ice bath. 0.24 mmol of sucrose was taken, dissolved in 10 ml of deionized water, and added to the activated octanoic acid solution. The pH value was adjusted to 7.0-7.4 with NaOH, and the reaction was stirred at room temperature for 12 h. The reaction product solution was purified according to the same operation as in Example 14 to obtain an octanoic acid-sucrose complex. The infrared spectrum of the complex is shown in Fig. 21. The sucrose molecule has no absorption peak at 1700-1500 cm⁻¹, while n-octanoic acid has a strong absorption peak at 1700 cm⁻¹. In the new compound produced by the reaction of sucrose and n-octanoic acid, there are two strong absorption peaks at 1700-1500 cm⁻¹. It is inferred that the conjugation effect between the formed ester bond and the hydroxyl group of sucrose molecule can shift the peak to the lower band.

The inhibition rate of bacteria (such as *Staphylococcus aureus*) was tested, including the following test processes: Preparation of the culture medium: the culture medium was prepared using LB agar according to the product manual, pH 7.2-7.4. Preparation and Inoculation of Inoculum: the bacteria were diluted into 10⁵-10⁶ CFU. 100 ul of bacteria were taken, added with 900 ul of liquid medicines that were diluted to different concentrations, and incubated at 37°C for 2 h. Then, the solution was diluted 100 times. 100 ul of the diluted solution was taken, plated, and incubated at 37°C for 16-24 h. The colonies were counted, and the inhibition rate was calculated. The inhibition results are shown in Fig. 22. When the concentration was 72 mM-9 mM, the inhibition rate was greater than 99%, with the antibacterial property. The half maximal inhibition concentration was 4.5 Mm. When the concentration was between 4.5 mM-0.14 mM, the inhibition was less than 50%, without the antibacterial property.

In this example, sucrose was both the water-soluble moiety and the binding moiety, and a fatty acid saturated carbon chain was the acting moiety. The specific inhibition results are summarized in Table 2-2.

**Table 2-2 Bactericidal antibacterial properties of saturated carbon chain with 8 carbon atoms-sucrose (9 mM) (2 h)**

| Tested microorganism | Sterilization rate (%) |
|---|---|
| *Escherichia coli* | >99 |
| Methicillin-resistant *Streptococcus pneumoniae* | >99 |
| *Streptococcus pneumoniae* | >99 |
| *Klebsiella pneumoniae* | >99 |
| *Pseudomonas aeruginosa* | >99 |

### Example 16 Preparation and performance evaluation of fatty acid-nucleotide complex (small molecule water-soluble moiety + acting moiety)

In this example, octanoic acid, linolenic acid and docosapentaenoic acid were selected as carbon chain donors to react with adenosine monophosphate respectively, and the reaction equation is as follows, where R is the carbon chain of octanoic acid, linolenic acid, and docosapentaenoic acid respectively.

The specific reaction is carried out according to the following reaction equation 16-1.

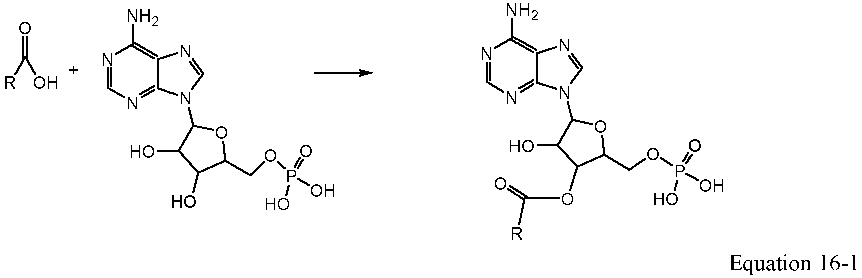

The reaction process is as follows:
0.72 mmol of fatty acid was taken and added with 0.72 mmol of EDC and 0.72 mmol of DMAP as the catalysts; and the mixture was stirred and activated for 10 min under an ice bath. 0.72 mmol of adenosine monophosphate was taken and dissolved in 10 ml of deionized water; and the mixture was added to the activated octanoic acid solution. The pH value was adjusted to 7.0-7.4 with NaOH, and the reaction is stirred at room temperature for 12 h to obtain a fatty acid-adenosine complex solution. After the reaction was completed, the reaction product solution (referred to as "reaction solution") was subjected to chromatographic purification. The reaction solution was fed to a Shephadex G10 chromatographic column (Φ26 mm x 50 cm) and eluted with normal saline at a flow rate of 50 ml/h. The eluents were collected step by step, and the absorbance was detected at a wavelength of 260 nm by ultraviolet spectrophotometry. The first eluting peaks were combined to be the reaction product.

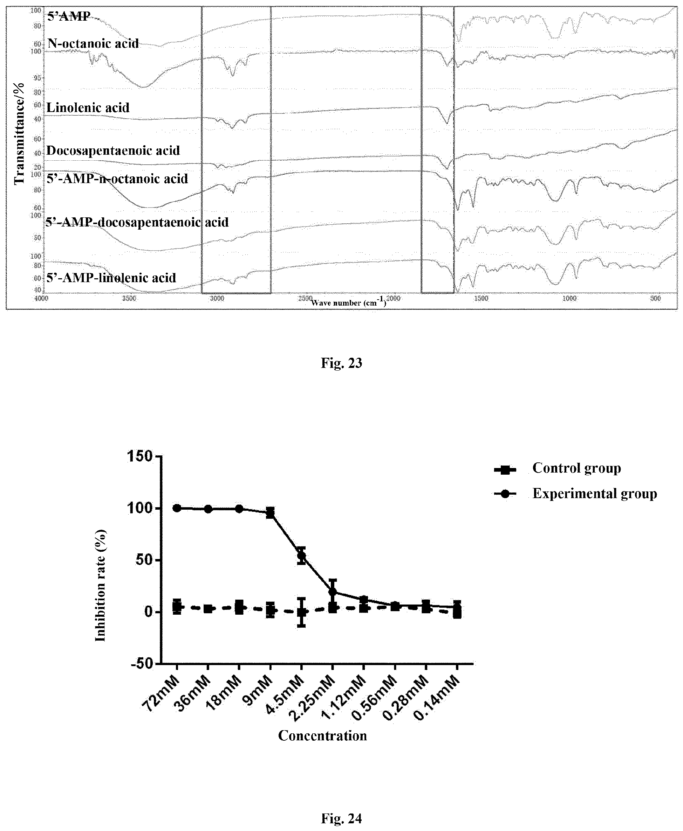

The infrared spectrum of the fatty acid-adenosine monophosphate complex prepared in this example is as shown in Fig. 23. The adenosine monophosphate molecule has no absorption peak at 3000-2700 cm⁻¹, while the n-octanoic acid, docosapentaenoic acid, and linolenic acid have strong absorption peaks at 3000-2700 cm⁻¹. The adenosine monophosphate molecule has no absorption peak at 1700-1500 cm⁻¹, while the n-octanoic acid, docosapentaenoic acid, and linolenic acid have strong absorption peaks at 1700 cm⁻¹. In the new compounds produced by the reaction of the two, the strong absorption peak appears in the waveband of 1700-1500 cm⁻¹. It is inferred that the conjugation effect between the formed ester bond and the hydroxyl group of the adenosine monophosphate molecule can shift the peak to the lower band.

The inhibition rate of the fatty acid-adenosine monophosphate complex against bacteria (such as *Staphylococcus aureus*) was tested, including the following test processes: Preparation of the culture medium: the culture medium was prepared using LB agar according to the product manual, pH 7.2-7.4. Preparation and Inoculation of Inoculum: the bacteria were diluted into 10⁵-10⁶ CFU. 100 ul of bacteria were taken, added with 900 ul of liquid medicines that were diluted to different concentrations, and incubated at 37°C for 2 h. Then, the solution was diluted 100 times. 100 ul of the diluted solution was taken, plated, and incubated at 37°C for 16-24 h. The colonies were counted, and the inhibition rate was calculated. The inhibition results are shown in Fig. 24. When the concentration was 72 mM-9 mM, the inhibition rate was greater than 99%, with the antibacterial property. The half maximal inhibition concentration was 4.5 Mm. When the concentration was between 4.5 mM-0.14 mM, the inhibition was less than 50%, without the antibacterial property.

In this example, adenosine monophosphate was both the water-soluble moiety and the binding moiety, and the fatty acid carbon chain was the acting moiety. The inhibition results are summarized in table 3.

**Table 3 Bactericidal and antibacterial properties of fatty acid carbon chain-adenosine monophosphate complex (9 mM) (2 h)**

| Tested microorganism | Sterilization rate (%) |
|---|---|
| *Escherichia coli* | >99 |
| Methicillin-resistant *Streptococcus pneumoniae* | *>99* |
| *Streptococcus pneumoniae* | >99 |
| *Klebsiella pneumoniae* | >99 |
| *Pseudomonas aeruginosa* | >99 |

### Example 17 Preparation and performance evaluation of saturated carbon chain with 8 carbon atoms-soluble vitamin complex (small molecule water-soluble moiety + acting moiety)

In this example, octanoic acid was used to prepare a saturated fatty acid ascorbic acid complex, which was performed according to the following reaction equation 17-1:

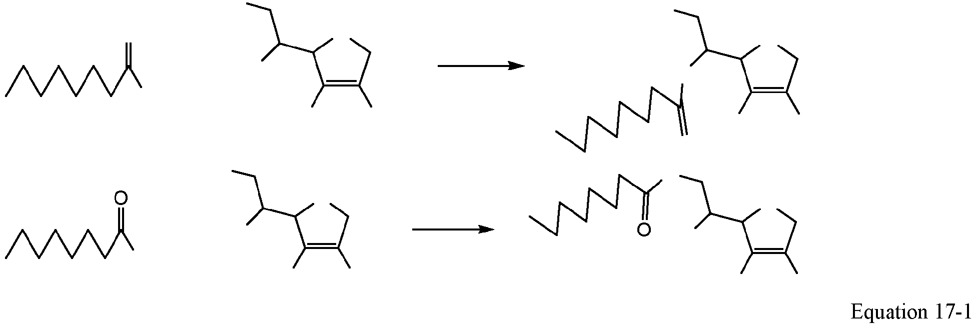

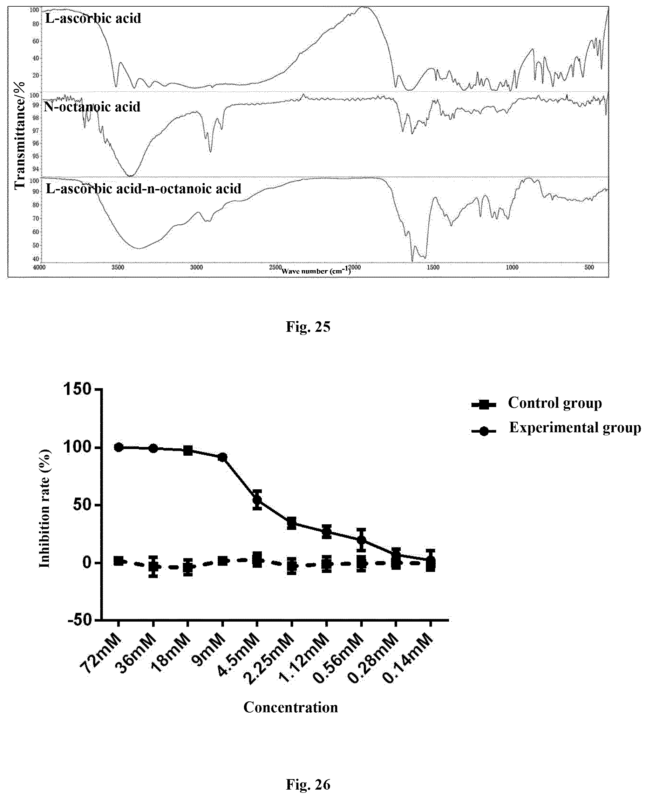

The reaction process is as follows. 0.72 mmol of octanoic acid was weighed and added with 0.72 mmol of EDC and 0.72 mmol of DMAP; and the mixture was stirred and activated for 10 min under an ice bath. 0.72 mmol of ascorbic acid was taken and dissolved in 10 ml of deionized water; and the mixture was added to the activated octanoic acid solution. The pH value was adjusted to 7.0-7.4 with NaOH, and the reaction was stirred at room temperature for 12 h. After the reaction was completed, the reaction solution was subjected to chromatographic purification. The reaction solution was fed to a Shephadex G10 chromatographic column (Φ26 mm x 50cm) and eluted with normal saline at a flow rate of 50 ml/h. The eluates were collected step by step, and the absorbance was detected by UV spectrophotometry at a wavelength of 243 nm. The first eluting peaks were combined to obtain the reaction product, i.e. The octanoic acid-ascorbic acid complex. The infrared spectrum of the prepared octanoic acid-ascorbic acid complex is shown in Fig. 25. It can be seen that no new functional group is generated after the reaction between octanoic acid and ascorbic acid, but the shift of the characteristic peak of ν_{C=O} to the lower band appears, which can be due to the intramolecular conjugation effect, and the carboxyl group of n-octanoic acid is lactonized with the hydroxyl group of ascorbic acid.

The inhibition rate of bacteria (such as *Staphylococcus aureus*) was tested, including the following test processes: Preparation of the culture medium: the culture medium was prepared using LB agar according to the product manual, pH 7.2-7.4. Preparation and Inoculation of Inoculum: the bacteria were diluted into 10⁵-10⁶ CFU. 100 ul of bacteria were taken, added with 900 ul of liquid medicines that were diluted to different concentrations, and incubated at 37°C for 2 h. Then, the solution was diluted 100 times. 100 ul of the diluted solution was taken, plated, and incubated at 37°C for 16-24 h. The colonies were counted, and the inhibition rate was calculated. The inhibition results are shown in Fig. 26. When the concentration was 72 mM-9 mM, the inhibition rate was greater than 99%, with the antibacterial property. The half maximal inhibition concentration was 4.5 Mm. When the concentration was between 4.5 mM-0.14 mM, the inhibition was less than 50%, without the antibacterial property.

In this example, ascorbic acid was both the water-soluble moiety and the binding moiety, and the fatty acid carbon chain was the acting moiety. The specific inhibition results are summarized in table 4.

**Table 4 Bactericidal and antibacterial properties of saturated carbon chain with 8 carbon atoms-water-soluble vitamin complex (9 mM) (2 h)**

| Tested microorganism | Sterilization rate (%) |
|---|---|
| *Escherichia coli* | >99.9 |
| Methicillin-resistant *Streptococcus pneumoniae* | >99.9 |
| *Streptococcus pneumoniae* | >99.9 |
| *Klebsiella pneumoniae* | >99.9 |
| *Pseudomonas aeruginosa* | >99.9 |

### Example 18 Preparation and performance evaluation of saturated carbon chain with 8 carbon atoms- PEG-COOH with low degree of polymerization complex (carboxyl + small molecule water-soluble moiety + acting moiety)

In this example, octanoic acid was used to prepare saturated fatty acid-PEG400-COOH complex, which was performed according to the following reaction equation 18-1.

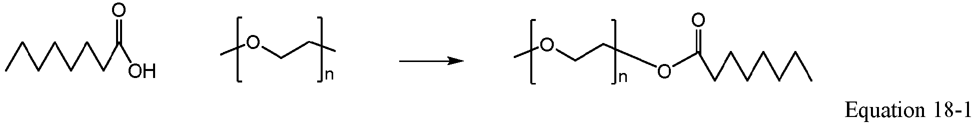

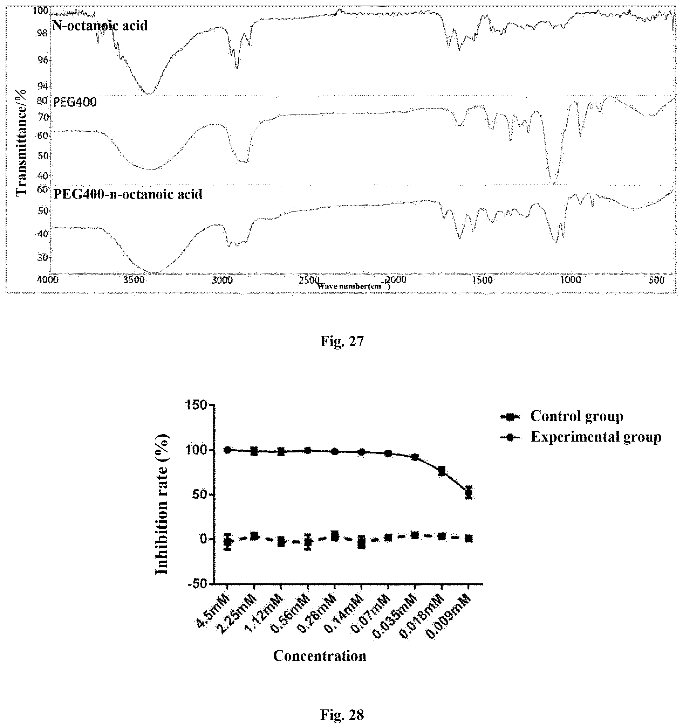

The reaction process is as follows. 0.72 mmol of octanoic acid was weighed and added with 0.72 mmol of EDC and 0.72 mmol of DMAP; and the mixture was stirred and activated for 10 min under an ice bath. 0.36 mol of PEG400-COOH was taken and dissolved in 10 ml of deionized water; and the mixture was added to the activated octanoic acid solution. The pH value was adjusted to 7.0-7.4 with NaOH and the reaction was stirred at room temperature for 12 h. After the reaction was completed, the reaction product solution was subjected to chromatographic purification. The reaction solution was fed to a Shephadex G10 chromatography column (Φ26 mm x 50 cm) and eluted with normal saline at a flow rate of 50 ml/h. The eluents were collected step by step and detected with barium chloride-iodine solution method. The first elution peaks were combined to obtain the reaction product, i.e., the octanoic acid-PEG400-COOH complex. The infrared spectrum of the prepared octanoic acid-PEG400-COOH complex is shown in Fig. 27. It can be seen that no new functional group is generated after the reaction between octanoic acid and PEG400-COOH, but the intensity of absorption peaks at wavelengths of 1550 cm⁻¹ and 1020 cm⁻¹ is enhanced.

The inhibition rate of bacteria (*Staphylococcus aureus*) was tested was tested, including the following test processes: the culture medium was prepared using LB agar medium (Tryptone 10 g/L, Yeast extract 5 g/L, sodium chloride (NaCl) 10 g/L, agar powder/L 15 g-20 g) according to the product manual, pH 7.2~7.4. Preparation and Inoculation of Inoculum: the bacteria were diluted into 10⁵-10⁶ CFU. 100 ul of bacteria were taken, added with 900 ul of liquid medicines that were diluted to different concentrations, and incubated at 37°C for 2 h. Then, the solution was diluted 100 times. 100 ul of the diluted solution was taken, plated, and incubated at 37°C for 16-24 h. The colonies were counted, and the inhibition rate was calculated. The inhibition results are shown in Fig. 28. When the concentration was 4.5 mM-0.035 mM, the inhibition rate was greater than 99%, with the antibacterial property. When the concentration was between 4.5 mM-0.009 mM, the inhibition was less than -90%, without the antibacterial property. The half maximal inhibition concentration was 0.009 Mm.

In this example the low degree of polymerization PEG400-COOH was both the water-soluble moiety and the binding moiety, and the fatty acid carbon chain was the acting moiety. The specific bacteriostasis test results are summarized in table 5.

**Table 5 Bactericidal and antibacterial properties of saturated carbon chain with 8 carbon atoms-low polymerization degree PEG400-COOH (0.035 mM) (2 h)**

| Tested microorganism | Sterilization rate (%) |
|---|---|
| *Escherichia coli* | >99 |
| Methicillin-resistant *Streptococcus pneumoniae* | *>99* |
| *Streptococcus pneumoniae* | >99 |
| *Klebsiella pneumoniae* | >99 |
| *Pseudomonas aeruginosa* | >99 |

### Example 19 Preparation and performance evaluation of liposome emulsion (amino/carboxyl + water-soluble moiety + acting moiety noncovalent coupling)

### (1) Ethyl Oleate and Aminated Lecithin preparation

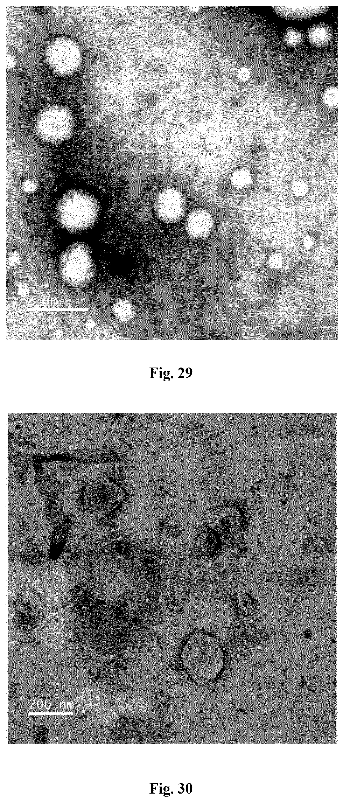

1) 0.7 g of aminated lecithin, 0.3 g of β-sitosterol, 0.2 g of vitamin E palmitate, and 10 g of ethyl oleate were heated and stirred to be dissolved in a water bath at 50°C. Then, a surfactant tween 80 was added and dispersed uniformly.
2) After slowly adding water to 2), the mixture was stirred uniformly and homogenized for 5 min to obtain colostrum. The colostrum was then subjected to ultrasonic for 15 min to obtain a lipid-loaded nano-liposome emulsion. Fig. 29 shows a graph of particle size observed under transmission electron microscopy. The particle size remained approximately between 500 nm and 800 nm, and after standing at room temperature for 6 months, the appearance property remained stable, indicating that the liposome emulsion has good stability.

In this example, water-soluble liposome, an oil-in-water structure, an amino group on the phospholipid was the binding group, ethyl oleate and the carbon chain in the phospholipid molecule were the acting groups. The phosphate group in the phospholipid molecule was the water-soluble group, and a complex (nano liposome encapsulating ethyl oleate) was formed by hydrogen bond and van der Waals force.

### (2) Linoleic acid and carboxylated lecithin liposome preparation

1) 0.7g of carboxylated lecithin (purchased from Aladdin reagent company), 0.3 g of β-sitosterol, and 2 g of linoleic acid were heated and stirred to be dissolved in a water bath at 50°C. Then the surfactant tween 80 was added and dispersed uniformly.
2) After slowly adding the aqueous solution into 1), the mixture was evenly stirred and homogenized for 5 min to obtain colostrum. Then, the colostrum was subjected to ultrasonic for 30 min to obtain a lipid-loaded nano liposome emulsion.

Fig. 30 shows a graph of particle size observed under transmission electron microscopy. The particle size remained approximately between 500 nm and 800 nm, and after standing at room temperature for 6 months, the appearance property remained stable, indicating that the liposome emulsion has good stability.

In this example, water-soluble liposome, an oil-in-water structure, a carboxyl group on the phospholipid is the binding group. Linoleic acid and the carbon chain in the phospholipid molecule were the acting groups. The phosphate group in the phospholipid molecule was the water-soluble group. The complex (a nano liposome encapsulating linoleic acid) was formed by hydrogen bond and van der Waals force.

### Verification of microbial killing effect of liposome

### Virus inhibition assay procedure:

260 µL of autoclaved water was added to 36 wells around a 96-well plate to seal the edge, so as to reduce the error caused by evaporation of medium in edge wells. Column 2 (cell control CC) was added with DMEM complete medium, 150 µL/well. Column 3 (virus control VC) was added with DMEM complete medium, 100 µL/well. The samples liposomes detected with ethyl oleate (linoleic acid) concentrations of 2.25 mM, 1.12 mM and 0.56 mM. SARS-CoV-2 pseudovirus (B.1.526.2, purchased from Beijing Tiantan Pharmaceutical Biotechnology Development Co. No: 80062. The pseudovirus system carries a firefly luciferase reporter gene. After infecting cells with the pseudovirus, the luminescence value can be detected by fluorescent substrates to determine the amount of virus infecting the cells. The pseudovirus uses VSV as a backbone, and the surface of the pseudovirus is embedded with the Spike protein of novel coronavirus, which can simulate the binding process of the true virus to the receptor and then enter the cell. After the sample with neutralizing activity reacts with the pseudovirus, the ability to infect the cell will be lost. By detecting the luminescence value of the control well and the sample well, whether the sample has neutralizing activity or not can be determined, and the effective concentration of the sample, i.e. the EC50 value, can be calculated. The pseudovirus can not replicate autonomously, and has only one round of infection ability, low in biosafety level and easy to operate, which is a common means for vaccine evaluation at present.) was diluted with the DMEM complete medium to 1.3- 2.3 x 10⁴ TCID 50/mL, and added 50 µL per well in column 3-11. The 96-well plate was incubated in a cell culture incubator (37°C, 5% CO₂) for 1 h. After 30 min of incubation, Vero cells was started to be digested. The cell concentration was diluted to 2 x 10⁵ cells/mL. After the incubation, 100 µL cells were added per well to make 2 x 10⁴ cells in each well. The plate was put into a 37°C, 5% CO2 cell incubator for culture for 24h. After the completion of incubation, 250 µL of the supernatant was pipetted away. 100 µL of luciferase detection reagent was added for reaction at room temperature in the dark for 2 min. After repeatedly blowing and beating, 100 µL of liquid was transferred to a white plate. The luminescence values (RLU) were read using a multi-function imaging microplate reader.

The neutralization inhibition rate was calculated according to the following equation E: Inhibition rate=(1-(Average luminous intensity-Average black control CC)/(Average VC-Average CC))*100%

**Table 6 Virucidal properties of liposomes (0.025 mM) (1 h)**

| Tested virus | Sterilization rate (%) | |
|---|---|---|
| | Ethyl oleate liposome | Linoleic acid liposome |
| H7N9 pseudovirus | >99.9 | >99.9 |
| H5N1 pseudovirus | >99.9 | >99.9 |
| HIV pseudovirus | >99.9 | >99.9 |
| SARS-CoV-2 pseudovirus | >99.9 | >99.9 |

### Bacteriostasis test process

Preparation of the culture medium: the culture medium was prepared using LB agar according to the product manual, pH 7.2-7.4. Preparation and Inoculation of Inoculum: the bacteria were diluted into 10⁵⁻10⁶ CFU. 100 ul of bacteria were taken, added with 900 ul of liquid medicines (complex solution prepared according to the present invention), and incubated at 37°C for 1-2 hours. Then, the solution was diluted by 100 times. 100 ul of the diluted solution was taken, plated, and incubated at 37°C for 16-24 h. The colonies were counted, and the inhibition rate was calculated. The results are summarized in Tables 7 and 8 below.

**Table 7 Bactericidal and antibacterial properties of liposomes (0.025 mM) (2 h)**

| Tested microorganism | Sterilization rate (%) | |
|---|---|---|
| | Ethyl oleate liposome | Linoleic acid liposome |
| *Escherichia coli* | >99 | >99 |
| *Staphylococcus aureus* | >99 | >99 |
| Methicillin-resistant *Streptococcus pneumoniae* | >99 | >99 |
| *Streptococcus pneumoniae* | >90 | >99 |
| *Klebsiella pneumoniae* | >99 | >99 |
| *Pseudomonas aeruginosa* | >99 | >99 |

**Table 8 Fungicidal properties of liposomes (0.025 mM) (1 h)**

| Tested microorganism | Sterilization rate (%) | |
|---|---|---|
| | Ethyl oleate liposome | Linoleic acid liposome |
| *Candida albicans* | >99 | >99 |
| *Aspergillus niger* | >99 | >99 |
| *Actinomyces viscosus* | >99 | >99 |
| *Chaetomium globosum* | >99 | >99 |
| *Aspergillus protuberus* | >99 | >99 |
| *Microsporum canis* | >99 | >99 |

### Example 20 Preparation and performance evaluation of serum albumin-grafted fatty acid complex (acting moiety+ macromolecular water-soluble part/binding moiety)

The lyophilized powder of linolenic acid-serum albumin complex was prepared according to the method of Example 1, i.e. lyophilized powder for injection of fatty acid-serum albumin complex for animal experiments, which was stored at 4°C for later use. In use, the lyophilized powder for injection was dissolved with normal saline, and filtered and sterilized using 0.22 um sterile filter membrane.

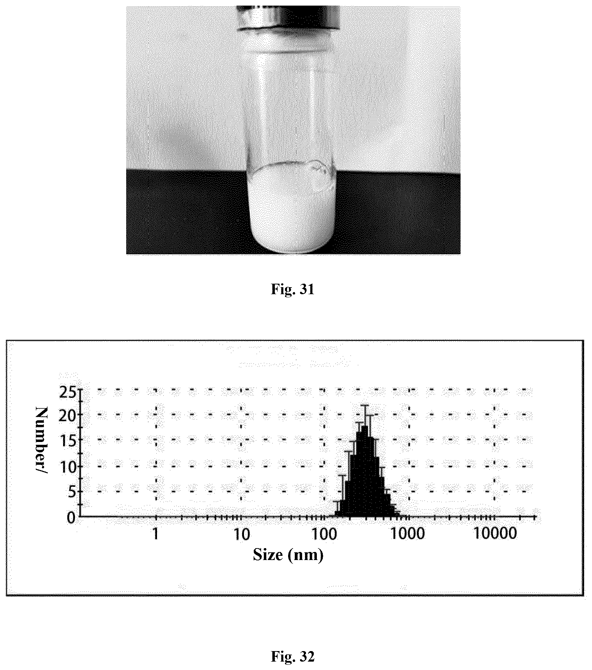

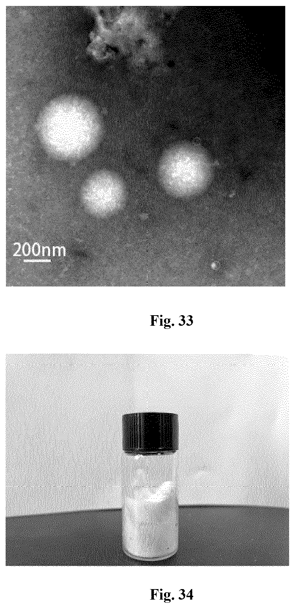

Fig. 31 shows the injection reconstituted after lyophilization of linolenic acid-serum albumin. Fig. 32 shows the particle size distribution measured by Malvern Mastersizer. It can be seen that 90% of the particle size is distributed within the range of 200-300 nm, and the particle size dispersion coefficient is 0.136, indicating good dispersion. Fig. 33 shows the image under transmission electron microscopy.

In this example, serum albumin was both the water-soluble moiety and a binding moiety, and the linolenic acid carbon chain was the acting moiety.

Referring to the method of the microbial experiments in Example 19, the results in Tables 9, 10, and 11 show that the virus killing and bacterial killing abilities are greater than 99% when the concentration of linolenic acid is 0.035 mM.

**Table 9 Virucidal properties of linolenic acid-serum albumin complex (0.035 mM) (1 h)**

| Tested virus | Sterilization rate (%) |
|---|---|
| H7N9 pseudovirus | >99.9 |
| H5N1 pseudovirus | >99.9 |
| HIV pseudovirus | >99.9 |
| SARS-CoV-2 pseudovirus | >99.9 |

**Table 10 Bactericidal antibacterial properties of linolenic acid-serum albumin complex (0.035 mM) (2 h)**

| Tested microorganism | Sterilization rate (%) |
|---|---|
| *Escherichia coli* | >99 |
| *Staphylococcus aureus* | >99 |
| Methicillin-resistant *Streptococcus pneumoniae* | *>99* |
| *Streptococcus pneumoniae* | >99 |
| *Klebsiella pneumoniae* | >99 |
| *Pseudomonas aeruginosa* | >99 |

**Table 11 Fungicidal properties of linolenic acid-serum albumin complex (0.035 mM) (2 h)**

| Tested microorganism | Sterilization rate (%) |
|---|---|
| *Candida albicans* | >99 |
| *Aspergillus niger* | >99 |
| *Actinomyces viscosus* | >99 |
| *Chaetomium globosum* | >99 |
| *Aspergillus protuberus* | >99 |
| *Microsporum canis* | >99 |

### Example 21 Performance evaluation of lyophilized powder for injection of fatty acid-hyaluronic acid complex (acting moiety + macromolecular water-soluble part/binding moiety)

For the preparation of linoleic acid-hyaluronic acid complex, see Example 7; for the preparation of docosahexaenoic acid-hyaluronic acid complex, see Example 8. The lyophilized powder after the reaction was prepared as follows.

The reaction product was dialyzed in deionized water for 24 h to remove small molecule impurities, followed by gradient low-temperature drying in a vacuum lyophilizer (pre-freezing at -80°C for 24 h and vacuuming for 12 h, evacuating at -20°C for 12 h, and continuing to evacuate at 4°C for more than 24 h until the product was completely dried), thus obtaining a lyophilized powder for injection of fatty acid-hyaluronic acid complex for animal experiments, which was stored at 4°C for later use. In use, the lyophilized powder for injection was dissolved with normal saline, and filtered and sterilized using 0.22 um sterile filter membrane.

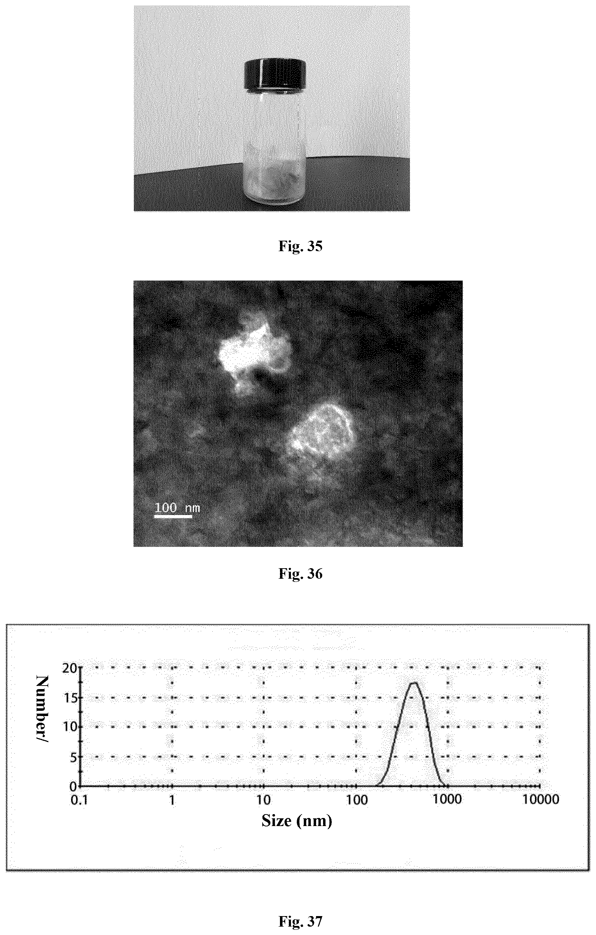

Fig. 34 shows a lyophilized powder injection of linoleic acid-hyaluronic acid, and Fig. 35 shows a lyophilized powder injection of docosahexaenoic acid-hyaluronic acid.

Taking linoleic acid-hyaluronic acid as an example, the lyophilized powder thereof was reconstituted in water. Fig. 36 shows an image under transmission electron microscope. Fig. 37 shows the particle size distribution measured by a Malvern particle size analyzer. It can be seen that the particle size is distributed within 100-1000 nm, 80% of which is within 200-400nm; the average particle size is 360 nm; and the particle size distribution coefficient is 0.231, indicating that the dispersion property is good.

In this example, the hyaluronic acid was both the water-soluble moiety and the binding moiety, and the linoleic acid carbon chain was the acting moiety.

Referring to the method of the microbial experiments in Example 19, the results in Tables 12, 13, and 14 below show that the virus killing and bacterial killing abilities are greater than 99% when the concentration of linolenic acid-hyaluronic acid complex is 0.035%.

**Table 12 Virucidal properties of linoleic acid-hyaluronic acid complex (0.035 mM) (1 h)**

| Tested virus | Sterilization rate (%) |
|---|---|
| H7N9 pseudovirus | >99.9 |
| H5N1 pseudovirus | >99.9 |
| HIV pseudovirus | >99.9 |
| SARS-CoV-2 pseudovirus | >99.9 |

**Table 13 Bactericidal and antibacterial properties of linoleic acid-hyaluronic acid complex (0.035 mM) (2 h)**

| Tested microorganism | Sterilization rate (%) |
|---|---|
| *Escherichia coli* | >99 |
| *Staphylococcus aureus* | >99 |
| Methicillin-resistant *Streptococcus pneumoniae* | *>99* |
| *Streptococcus pneumoniae* | >99 |
| *Klebsiella pneumoniae* | >99 |
| *Pseudomonas aeruginosa* | >99 |

**Table 14 Fungicidal properties of linoleic acid-hyaluronic acid complex (0.035 mM) (2 h)**

| Tested microorganism | Sterilization rate (%) |
|---|---|
| *Candida albicans* | >99 |
| *Aspergillus niger* | >99 |
| *Actinomyces viscosus* | >99 |
| *Chaetomium globosum* | >99 |
| *Aspergillus protuberus* | >99 |
| *Microsporum canis* | >99 |

### Example 22 Performance evaluation of oral, lyophilized powder preparation (acting moiety + water-soluble moiety)

### Method 1: taking octadecanoic acid-glutamic acid complex (reaction product of octadecanoic acid and glutamic acid) as an example

Gum arabic and cod liver oil were ground well, added with purified water, and ground to obtain a colostrum. Sodium saccharin aqueous solution and octadecanoic acid glutamic acid complex solution were added. The mass ratio of oil, water and gum in the colostrum was 4: 2: 1. Then, tragacanth gum mucilage was slowly added. Water was added appropriately. The mixture was stirred well to obtain an emulsion containing 6 mg/ml octadecanoic acid-glutamic acid complex.

Among them, octadecanoic acid was the acting moiety; glutamic acid was the binding moiety; glutamic acid, gum arabic and tragacanth gum were the water-soluble moiety. The complex (emulsion) was formed by hydrogen bond or van der Waals force.

### Method 2: taking dodecanoic acid-aspartic acid complex (being a reaction product of dodecanoic acid and aspartic acid) as an example

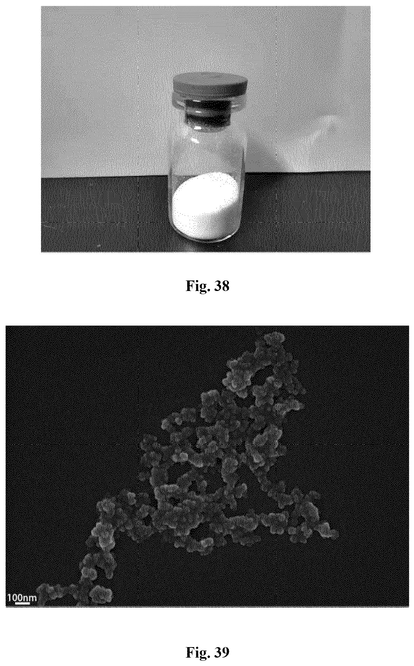

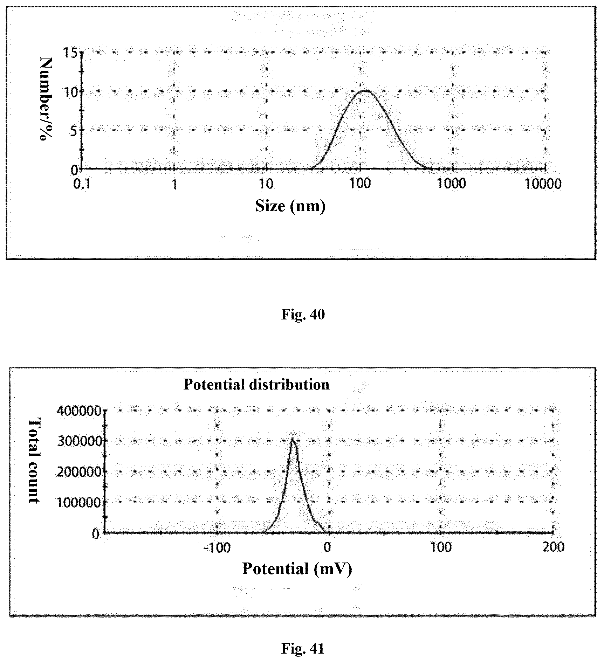

450 mg of Poloxamer 188, 300 mg of soy lecithin, 10 ml of purified water were used as an aqueous phase; 200 mg of dodecanoic acid-aspartic acid complex, 100 mg of glyceryl stearate, and 100 mg of glyceryl tricaprylate were used as an oil phase and uniformly stirred. The aqueous phase was added to the oil phase, continued stirring for 20-30 min, and ultrasonicated for 30 min to obtain an emulsion containing 2% (mass fraction) of dodecanoic acid-aspartic acid complex. The emulsion was frozen at -80°C, and then dried under vacuum at room temperature to obtain a liposome lyophilized powder preparation of dodecanoic acid-aspartic acid complex, see Fig. 38. Fig. 39 shows an image under a scanning electron microscope. The particle size and potential after re-dissolution in water are shown in Figs. 40 and 41. The figure shows that the average particle size was at 110 nm; the particle size distribution coefficient was 0.263, with good distribution property. The potential shows a mean potential absolute value of 26.4 mV in a more stable potential distribution (a potential absolute value above 20 indicates good stability).

Among them, the carbon chain in dodecanoic acid, glyceryl stearate, glyceryl tricaprylate, or soybean lecithin was the acting moiety; aspartic acid was the binding moiety; aspartic acid, soybean lecithin phosphorylcholine part, gum arabic, and tragacanth gum were the water-soluble moiety. The complex (liposome nanoemulsion) was formed by hydrogen bond or van der Waals force.

Referring to the method of the microbial experiments in Example 19, the results in the table below show that the virus killing and bacterial killing abilities are greater than 99% when the concentration of the complex in the emulsion is 0.05 mM.

**Table 15 Virucidal properties of liposomes (0.05 mM) (1 h)**

| Tested virus | Sterilization rate (%) | |
|---|---|---|
| | Octadecanoic acid-glutamic acid complex | Dodecanoic acid-aspartic acid complex |
| H7N9 pseudovirus | >99.9 | >99.9 |
| H5N1 pseudovirus | >99.9 | >99.9 |
| SARS-CoV-2 pseudovirus | >99.9 | >99.9 |
| HIV pseudovirus | >99.9 | >99.9 |

**Table 16 Bactericidal and antibacterial properties of liposomes (0.05 mM) (2 h)**

| Tested microorganism | Sterilization rate (%) | |
|---|---|---|
| | Octadecanoic acid-glutamic acid complex | Dodecanoic acid-aspartic acid complex |
| *Escherichia coli* | >99 | >99 |
| *Staphylococcus aureus* | >99 | >99 |
| Methicillin-resistant *Streptococcus pneumoniae* | >99 | >99 |
| *Streptococcus pneumoniae* | >99 | >99 |
| *Klebsiella pneumoniae* | >99 | >99 |
| *Pseudomonas aeruginosa* | >99 | >99 |

**Table 17 Fungicidal properties of liposomes (0.05 mM) (1 h)**

| Tested microorganism | Sterilization rate (%) | |
|---|---|---|
| | Octadecanoic acid-glutamic acid complex | Dodecanoic acid-aspartic acid complex |
| *Candida albicans* | >99 | >99 |
| *Aspergillus niger* | >99 | >99 |
| *Actinomyces viscosus* | >99 | >99 |
| *Chaetomium globosum* | >99 | >99 |
| *Aspergillus protuberus* | >99 | >99 |
| *Microsporum canis* | >99 | >99 |

### Example 23 Lecithin Conjugate Injection of Fatty Acids and Derivatives (acting moiety + water-soluble moiety + binding moiety, non-covalent coupling)

### (1) Pentacosanoic acid liposome

0.7g of carboxylated lecithin (purchased from Aladdin reagent company), 0.3 g of β-sitosterol, 0.05 g of glycocholic acid sulfate, and 2 g of pentacosanoic acid were taken, added with 2 ml of ethanol, heated and stirred in a water bath at 50°C to be dissolved. Then, ethanol was removed by rotary evaporation. Normal saline and surfactant tween 80 were added, stirred and dispersed evenly. The mixture was homogenized for 5 min to obtain a colostrum. Then, the colostrum was ultrasonicated for 30 min to obtain the nano-liposome emulsion loaded with pentacosanoic acid.

The pentacosanoic acid liposome prepared was a water-in-oil-in-water structure of a water-soluble liposome. Glycocholic acid sulfate was both the acting group and the binding group, wherein the carboxyl group and the sulfonic group on the glycocholic acid sulfonate molecule were the binding groups; and the cholestane skeleton was the acting group. The carbon chain in the molecules of pentacosanoic acid and phospholipid was the acting group, and the phosphate group in the molecule of phospholipid was the water-soluble group. The complex was formed through hydrogen bond and van der Waals force (a nano liposome encapsulating pentacosanoic acid).

### (2) Fatty acid ethyl ester liposome

Water phase: Tween-80 for injection 20 mg, and water 10ml;
Oil phase: aminated lecithin 0.7 g, cholesterol 0.3 g, and fatty acid ethyl ester 0.72 mmol.

The aqueous phase was added to the oil phase, stirred for 10-20 min and ultrasonicated (320 W).

Here, the fatty acid can be medium-chain hexanoic acid (ethyl hexanoate), heptanoic acid, octanoic acid, nonanoic acid, decanoic acid, stearic acid, oleic acid, linoleic acid, linolenic acid, eicosapentaenoic acid, docosahexaenoic acid, etc.

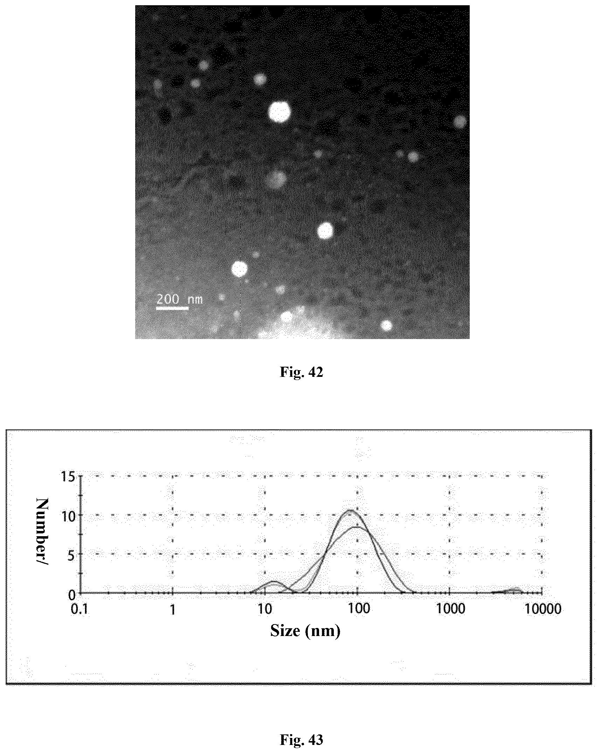

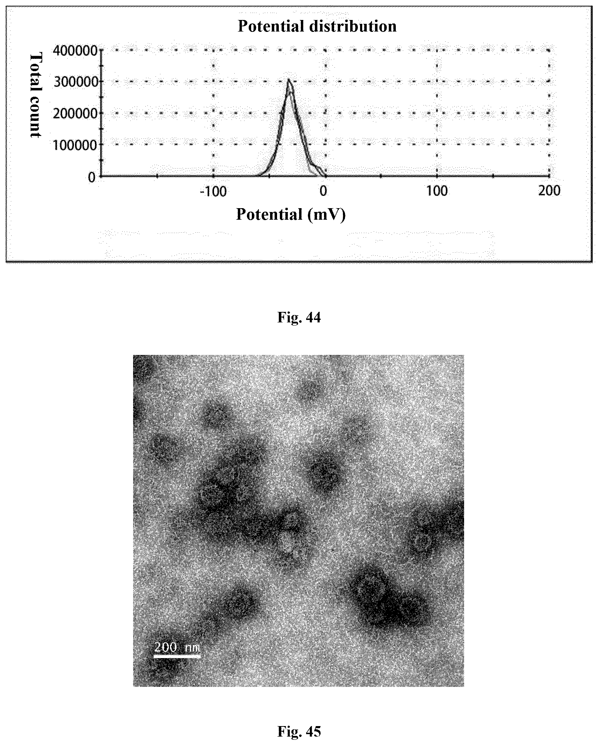

Fig. 42 is a transmission electron microscope image of eicosapentaenoic acid ethyl ester liposome. Fig. 43 shows the particle size distribution of eicosapentaenoic acid ethyl ester liposome measured by Malvern Mastersizer. It can be seen that more than 80% of particle size is distributed around 100 nm, a small part is 20-60 nm and 140-200 nm, and the dispersion coefficient is 0.162, indicating good dispersibility in water. Fig. 44 shows the potential distribution of eicosapentaenoic acid ethyl ester liposomes measured by Malvern Mastersizer, with an average potential of -25 mV and good product stability. After 6 months at room temperature, the appearance remained stable, indicating good stability of the liposome emulsion.

In this example, the fatty acid ethyl ester liposome was the water-soluble liposome having a water-in-oil-in-water structure, wherein the fatty acid ethyl ester, cholesterol, and the fatty acyl carbon chain in the molecule of phospholipid were the acting moiety; the aminated phospholipid was the binding moiety and the water-soluble moiety; the amino group in the molecule thereof was the binding group; the phosphate group in the molecule thereof was the water-soluble group. The complex (a nano liposome encapsulating the fatty acid ethyl ester) was formed by hydrogen bond and van der Waals force.

Referring to the method of the microbial experiments in Example 19, the results in Tables 18 and 19 below show that the virus killing and bacterial killing abilities are greater than 99% when the concentration of pentacosanoic acid is 0.05% in the liposomes.

**Table 18 Virucidal properties of pentacosanoic acid liposomes (0.05%) (1 h)**

| Tested virus | Sterilization rate (%) |
|---|---|
| H7N9 pseudovirus | >99.9 |
| H5N1 pseudovirus | >99.9 |
| HIV pseudovirus | >99.9 |
| SARS-CoV-2 pseudovirus | >99.9 |

**Table 19 Bactericidal and antibacterial properties of pentacosanoic acid liposome (0.05%) (2 h)**

| Tested microorganism | Sterilization rate (%) |
|---|---|
| *Escherichia coli* | >99 |
| *Staphylococcus aureus* | >99 |
| Methicillin-resistant *Streptococcus pneumoniae* | *>99* |
| *Streptococcus pneumoniae* | >99 |
| *Klebsiella pneumoniae* | >99 |
| *Pseudomonas aeruginosa* | >99 |

### Example 24 Performance evaluation of unsaturated fatty acid-polypeptide dry powder inhalant (acting moiety + polypeptide target binding moiety/water-soluble moiety)

The preparation of unsaturated fatty acid-SBP1 complex was carried out according to Example 10.

The prepared composite lyophilized powder was pulverized and used as a dry powder inhalant for animal experiments.

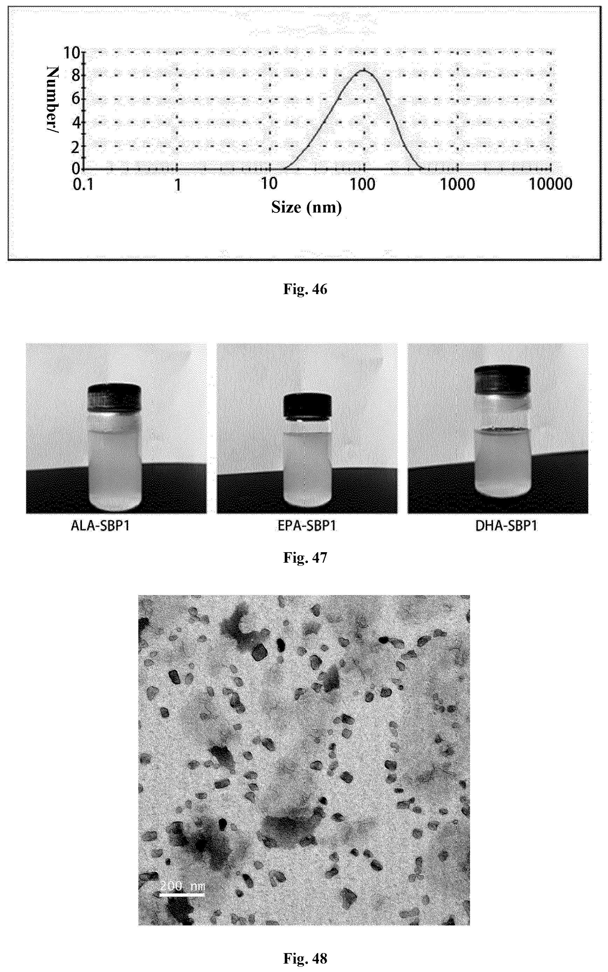

Taking docosahexaenoic acid-SBP1 as an example, Fig. 45 is an image under transmission electron microscopy and Fig. 46 shows the particle size distribution as measured by a Malvern Mastersizer. Fig. 47 shows the product images of peptide SBP1 grafted with different ω-3 fatty acids (ALA: linolenic acid, EPA: eicosapentaenoic acid, DHA: docosahexaenoic acid).

In this example, SBP1 was both the water-soluble moiety and the targeted binding moiety, and the fatty acid carbon chain was the targeting moiety.

Referring to the method of the microbial experiments in Example 19, the results in Table 20 below show that the virus killing and bacterial killing abilities are greater than 99% when the concentration of the ω-3 fatty acid-SBP1 is 0.035 mM.

**Table 20 Virucidal properties of ω-3 fatty acids-SBP1 (0.035 mM) (1 h)**

| Tested virus | Sterilization rate (%) | | |
|---|---|---|---|
| | SBP1-ALA | SBP1-EPA | SBP1-DHA |
| H7N9 pseudovirus | >99.99 | >99.99 | >99.99 |
| H5N1 pseudovirus | >99.99 | >99.99 | >99.99 |
| SARS-CoV-2 pseudovirus | >99.99 | >99.99 | >99.99 |
| HIV pseudovirus | >99.99 | >99.99 | >99.99 |

**Table 21 Bactericidal and antibacterial properties of ω-3 fatty acid-SBP1 (0.035 mM) (2 h)**

| Tested microorganism | Sterilization rate (%) | | |
|---|---|---|---|
| | SBP1-ALA | SBP1-EPA | SBP1-DHA |
| *Escherichia coli* | >99 | >99 | >99 |
| *Staphylococcus aureus* | >99 | >99 | >99 |
| *Staphylococcus aureus* | >99 | >99 | >99 |
| *Streptococcus pneumoniae* | >99 | >99 | >99 |
| *Klebsiella pneumoniae* | >99 | >99 | >99 |
| *Pseudomonas aeruginosa* | >99 | >99 | >99 |

### Example 25 Performance evaluation of fatty acid-CD14 lyophilized powder for injection (acting moiety + target binding moiety/water-soluble moiety)

Preparation of fatty acid-CD14 complex was carried out referring to Example 11. The lyophilized powder prepared was ready for injection of fatty acid-CD14 complex for animal experiments and stored at 4°C for later use. In use, the lyophilized powder for injection was dissolved with normal saline, and filtered and sterilized using 0.22 um sterile filter membrane.

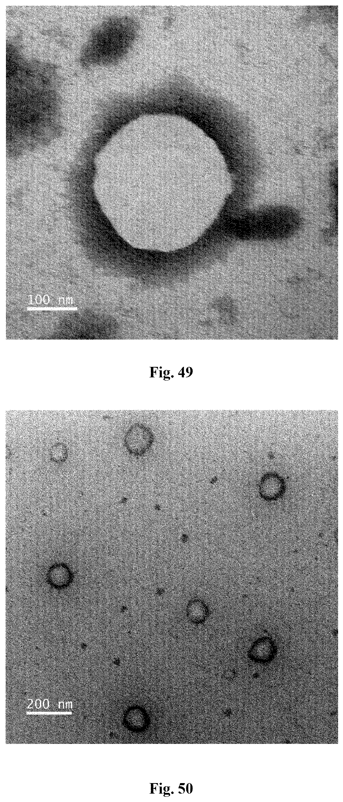

FIGs. 48, 49, and 50 are TEM images of CD14 protein grafted with dodecenoic acid, tetradecenoic acid, and eicosapentaenoic acid, respectively. It can be seen in the figure that the particle size of the lyophilized powder preparation after reconstitution was below 100 nm, with good dispersibility.

In this example, CD14 was both the water-soluble moiety and the targeting binding moiety, and the fatty acid carbon chain was the targeting moiety.

Referring to the method of the microbial experiments in Example 19, the results in Tables 22, 23, and 24 show that the virus killing and bacterial killing abilities are greater than 99% when the concentration of fatty acid-CD14 is 035 mM.

**Table 22 Virucidal properties of fatty acid-CD14 (0.035 mM) (1 h)**

| Tested virus | Sterilization rate (%) | |
|---|---|---|
| | CD14-dodecanoic acid | CD14-EPA |
| H7N9 pseudovirus | >99.99 | >99.99 |
| H5N1 pseudovirus | >99.99 | >99.99 |
| SARS-CoV-2 pseudovirus | >99.99 | >99.99 |
| HIV pseudovirus | >99.99 | >99.99 |

**Table 23 Bactericidal and antibacterial properties of fatty acid-CD14 (0.035 mM) (2 h)**

| Tested microorganism | Sterilization rate (%) | |
|---|---|---|
| | CD14-dodecanoic acid | CD14-EPA |
| *Escherichia coli* | >99 | >99 |
| *Staphylococcus aureus* | >99 | >99 |
| Methicillin-resistant *Streptococcus pneumoniae* | >99 | >99 |
| *Streptococcus pneumoniae* | >99 | >99 |
| *Klebsiella pneumoniae* | >99 | >99 |
| *Pseudomonas aeruginosa* | >99 | >99 |

**Table 24 Fungicidal properties of fatty acid-CD14 (0.035 mM) (2 h)**

| Tested microorganism | Sterilization rate (%) | |
|---|---|---|
| | CD14-dodecanoic acid | CD14-EPA |
| *Candida albicans* | >99 | >99 |
| *Aspergillus niger* | >99 | >99 |
| *Actinomyces viscosus* | >99 | >99 |
| *Chaetomium globosum* | >99 | >99 |

### Example 26 Performance Evaluation of (unsaturated carbon chain with 8 carbon atoms-threonine) complex oral preparation (acting moiety + small molecule water-soluble moiety/binding moiety)

### Capsule

The starch and cyclodextrin were firstly dried, crushed, sieved to 120 meshes, and mixed uniformly according to a mass ratio of 1: 1 to 1: 5. The crushed and sieved unsaturated carbon chain with 8 carbon atoms-threonine powder was added and ground uniformly. The drug and the excipient were mixed in a mass ratio of 0.1: 2 to 1: 2 and divided into capsules.

In this example, the unsaturated carbon chain with 8 carbon atoms can be considered as the acting moiety; threonine was both the water-soluble moiety and the binding moiety, wherein the carboxyl group can be considered as the binding moiety.

### Example 27 (Saturated carbon chain with 18 carbons-serine) complex oral preparation (acting moiety + small molecule water-soluble moiety/binding moiety)

### Capsule

Starch, sucrose, and cyclodextrin were taken, dried crushed, sieved to 120 meshes, and mixed evenly according to the mass ratio of 1: 0.1: 1 to 1: 10: 10. The saturated carbon chain with 18 carbon atoms-threonine was added and ground evenly. The drug was completely encapsulated with excipients with the mass ratio of drug to excipients of 0.1: 2 to 1: 2, and dispensed into capsules.

In this example, the saturated carbon chain with 18 carbon atoms can be considered as the acting moiety; serine was both the water-soluble moiety and the binding moiety, with the carboxyl group being considered as the binding moiety.

### Example 28 Preparation of carboxy-saturated carbon chain with 8 carbon atoms -adenosine 5'-monophosphate-unsaturated carbon chain with 4 carbon atoms-carboxyl complex (carboxyl + unsaturated carbon chain with 4 carbon atoms + small molecule water-soluble moiety + saturated carbon chain with 8 carbon atoms + carboxy) complex oral preparation

### Tablet

Preparation of carboxy-saturated carbon chain with 8 carbon atoms -adenosine 5'-monophosphate-unsaturated carbon chain with 4 carbon atoms-carboxyl complex: 1 mmol of octanedioic acid and 1 mmol of EDC were weighed. Carboxyl was activated for 10 min under magnetic stirring. 1 mmol of DMAP was added. 1 Mmol of adenosine 5'-monophosphate monosodium was weighed, and dissolved in 0.5 ml of water. The adenosine 5'-monophosphate monosodium solution was slowly added to octanedioic acid dropwise, continuing the stirring for reaction for 12 h. Then, 1 mmol of activated butenedioic acid (with reference to the activation method of octanedioic acid) was added, continued the stirring for reaction for 12 h. The reaction product was precipitated with acetone. The solvent was removed in vacuo. The catalyst was removed by column chromatography gradient elution to obtain a carboxyl-saturated carbon chain with 8 carbon atoms-adenosine 5'-monophosphate- unsaturated carbon chain with 4 carbon atoms-carboxyl complex. Wherein the purification process was carried out in the following manner. After the completion of the reaction, the reaction product solution was subjected to chromatographic purification. The reaction solution was fed to a Shephadex G10 chromatographic column (Φ26 mm × 50 cm), and eluted with normal saline at a flow rate of 50 ml/h. The eluates were collected step by step, and the absorbance was detected at a wavelength of 260 nm by ultraviolet spectrophotometry. The first elution peaks were combined to obtain the reaction product, i.e. carboxy-saturated carbon chain with 8 carbon atoms -adenosine 5'-monophosphate-unsaturated carbon chain with 4 carbon atoms-carboxyl complex.

The starch and cyclodextrin were firstly dried, crushed, sieved to 120 meshes, and mixed uniformly according to a mass ratio of 1: 1 to 1: 5. Water was added to prepare a 10% starch paste for future. 1-5 g of the above-mentioned complex, 2.5 g of hypromellose, and 8 g of starch were weighed. The materials were mixed using an equal amount of progressive addition and pass through an 80-mesh sieve to obtain uniform granules. The above-mentioned 10% starch paste was then added to the granular material, and was continuously squeezed and kneaded by hand so that the material and starch slurry were thoroughly mixed to prepare a soft material. The obtained soft material was extruded and kneaded in a 16-mesh nylon sieve to obtain suitable granules. The prepared granules were dried in an oven. The dried granules were sieved again. 1-3 g of magnesium stearate was weighed and added to the dried granules and mixed well to obtain

suitable granules. The granules were fed to a tabletting machine for tabletting, i.e. tablets of carboxy-saturated carbon chain with 8 carbon atoms -adenosine 5'-monophosphate-unsaturated carbon chain with 4 carbon atoms-carboxyl complex.

Among them, the saturated carbon chain with 8 carbon atoms and an unsaturated carbon chain with 4 carbon atoms were both the acting moiety and the binding moiety wherein the carboxyl group was the binding group and adenosine 5'-monophosphate was used as the water-soluble moiety.

Each ratio in the above method can be further optimized in the process.

### Example 29 Cytotoxicity validation (carboxyl + saturated/unsaturated carbon chain + small molecule water-soluble moiety)

### Preparation of amino acid complex

1 mmol of each of fatty acid (including n-octanoic acid, linoleic acid, linolenic acid, eicosapentaenoic acid, and docosahexaenoic acid) and amino acid (including serine, threonine and lysine) were weighed, respectively. 1 mmol of EDC was added to the fatty acid. The carboxyl was activated for 5 min under magnetic stirring. 1 mmol of NHS was added and stirred for 5 min. The amino acid was dissolved in 0.5 ml of water. The amino acid solution was slowly added dropwise to the activated fatty acid, continued stirring for reaction for 12 h. After the completion of the reaction, the reaction product solution was subjected to chromatographic purification. The reaction solution was fed to a Shephadex G10 chromatography column (Φ26 mm × 50 cm) and eluted with normal saline at a flow rate of 50 ml/h. The eluates were collected stepwise and detected by ninhydrin method. The first elution peaks were combined to obtain the reaction product, i.e. the amino acid complex, for subsequent experiments.

### Preparation of adenylate complex

1 mmol of each of n-octanoic acid, adenosine 5'-monophosphate monosodium, butenedioic acid, EDC and DMAP (4-dimethylamino pyridine) was weighed, and the reaction was carried out according to the synthesis method in Example 28. After the reaction, the reaction product solution was subjected to chromatographic purification. The reaction solution was added to a Shephadex G10 chromatographic column (Φ26 mm × 50 cm) and eluted with normal saline at a flow rate of 50 ml/h. The eluents were collected step by step, and the absorbance was detected at a wavelength of 260 nm with ultraviolet spectrophotometry. The first eluting peaks were combined to obtain the reaction product, i.e., an octanoic acid-adenosine monophosphate-butenedioic acid complex (namely, a saturated carbon chain with 8 carbon atoms -adenosine 5'-monophosphate-unsaturated carbon chain with 4 carbon atoms-carboxyl group), for subsequent experiments.

### Preparation of N-methylglucamine complex

1 mmol of each of n-octanoic acid, n-nonanoic acid, N-methylglucamine, EDC, and NHS was weighed, and the reaction was carried out according to the preparation method of amino acid complex. After the reaction, the reaction product solution was subjected to chromatographic purification. The reaction solution was added to a Shephadex G10 chromatographic column (Φ26 mm × 50 cm) and eluted with normal saline at a flow rate of 50 ml/h. The eluents were collected step by step and detected with phenol sulfuric acid method. The first elution peaks were combined to obtain the reaction product, i.e., N-methylglucamine complex, for subsequent experiments.

### Cytotoxicity validation was performed on the prepared complex.

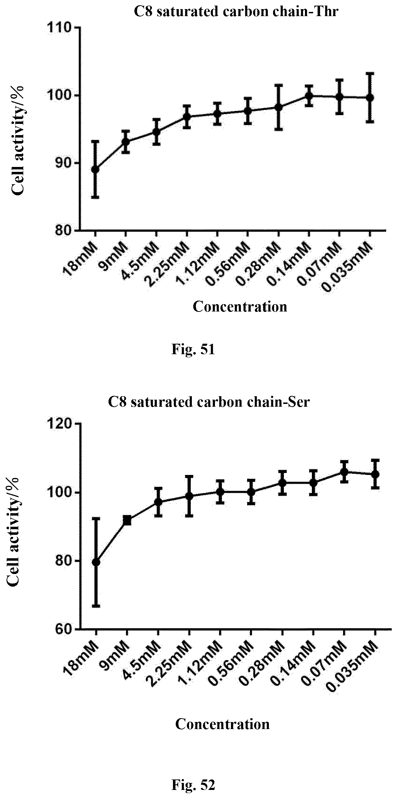

For the saturated carbon chain with 8 carbon atoms-threonine, the saturated carbon chain with 8 carbon atoms can be regarded as the acting moiety; threonine as the water-soluble moiety; and the carboxyl group and amino group in the threonine molecule can be regarded as the binding moiety. The cytotoxicity results are shown in Fig. 51, indicating that the effect of the complex on cells from 9 mM to 0.035 mM is less than 10%, with good safety.

For the saturated carbon chain with 8 carbon atoms-serine, the saturated carbon chain with 8 carbon atoms (octanoic carbon chain) can be regarded as the acting moiety; serine as the water-soluble moiety; and the carboxyl group and amino group in the serine molecule can be regarded as the binding moiety. The cytotoxicity results are shown in Fig. 52, indicating that the effect of the complex on cells from 9 mM to 0.035 mM is less than 10%, with good safety.

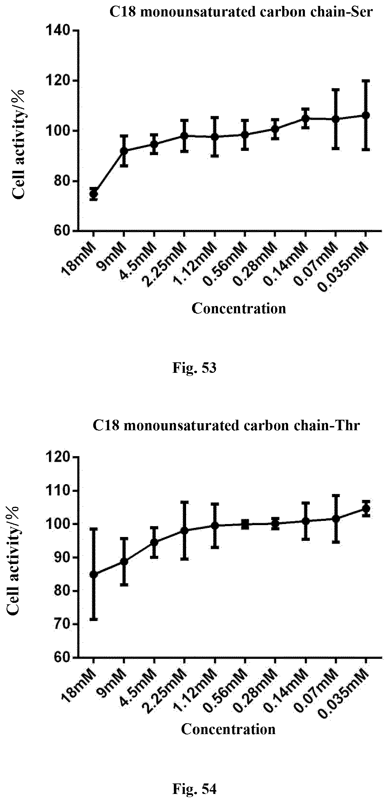

For the monounsaturated carbon chain with 18 carbon atoms-serine, the monounsaturated carbon chain with 18 carbon atoms (oleic acid carbon chain) can be regarded as the acting moiety; serine as the water-soluble moiety; and the carboxyl group and amino group in the serine molecule can be regarded as the binding moiety. The cytotoxicity results are shown in Fig. 53, indicating that the effect of the complex on cells at concentrations of 9 mM-0.035 mM is less than 10%, with good safety.

For the monounsaturated carbon chain with 18 carbon atoms-threonine, the monounsaturated carbon chain with 18 carbon atoms (oleic acid carbon chain) can be regarded as the acting moiety; threonine as the water-soluble moiety; and carboxyl group and amino group in the threonine molecule can be regarded as the binding moiety. The cytotoxicity results are shown in Fig. 54, indicating that the effect of the complex on cells at concentrations of 4.5 mM-0.035 mM is less than 10%, with good safety.

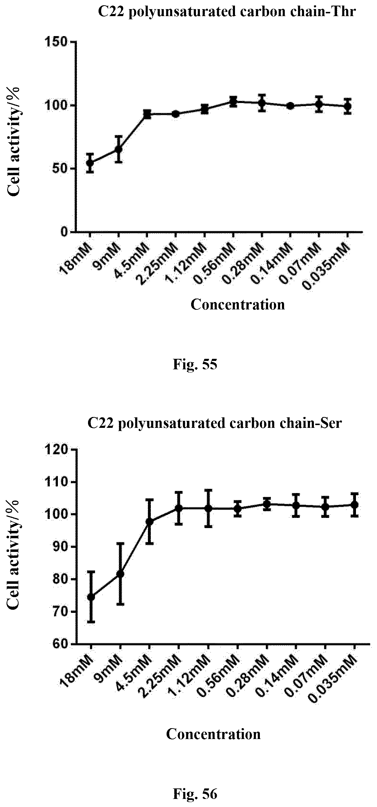

For the polyunsaturated carbon chain with 22 carbon atoms-threonine, the polyunsaturated carbon chain with 22 carbon atoms (the carbon chain of docosahexaenoic acid) can be regarded as the acting moiety; threonine as the water-soluble moiety; and the carboxyl group and amino group in the threonine molecule can be regarded as the binding moiety. The cytotoxicity results are shown in Fig. 55, indicating that the effect of the complex on cells at concentrations of 4.5 mM-0.035 mM is less than 10%, with good safety.

For the polyunsaturated carbon chain with 22 carbon atoms-serine, the polyunsaturated carbon chain with 22 carbon atoms (the carbon chain of docosahexaenoic acid) can be regarded as the acting moiety; serine as the water-soluble moiety; and the carboxyl group and the amino group in the serine molecule can be regarded as the binding moiety. The cytotoxicity results are shown in Fig. 56, indicating that the effect of the complex on cells at concentrations of 4.5 mM-0.035 mM is less than 10%, with good safety.

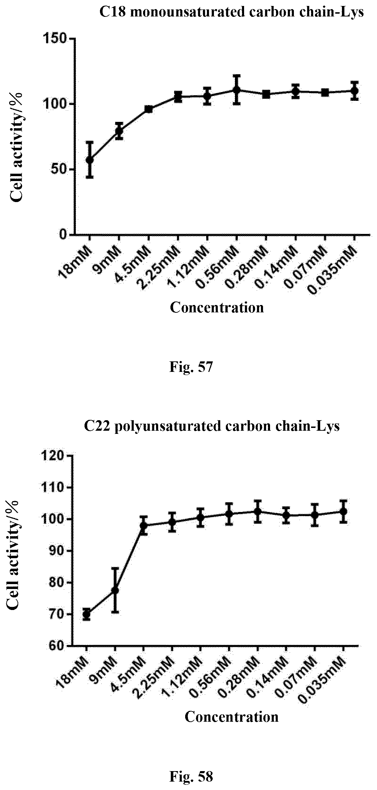

For the monounsaturated carbon chain with 18 carbon atoms-lysine, the monounsaturated carbon chain with 18 carbon atoms (oleic acid carbon chain) can be regarded as the acting moiety; lysine as the water-soluble moiety; and the carboxyl group and amino group in the lysine molecule can be regarded as the binding moieties. The cytotoxicity results are shown in Fig. 57, indicating that the effect of the complex on cells at concentrations of 4.5 mM-0.035 mM is less than 1%, with good safety.

For the polyunsaturated carbon chain with 22 carbon atoms-lysine, the polyunsaturated carbon chain with 22 carbon atoms (the carbon chain of docosahexaenoic acid) can be regarded as the acting moiety; lysine as the water-soluble moiety; and the carboxyl group and amino group in the lysine molecule can be regarded as the binding moieties. The cytotoxicity results are shown in Fig. 58, indicating that the effect of the complex on cells from 4.5 mM to 0.035 mM is less than 10%, with good safety.

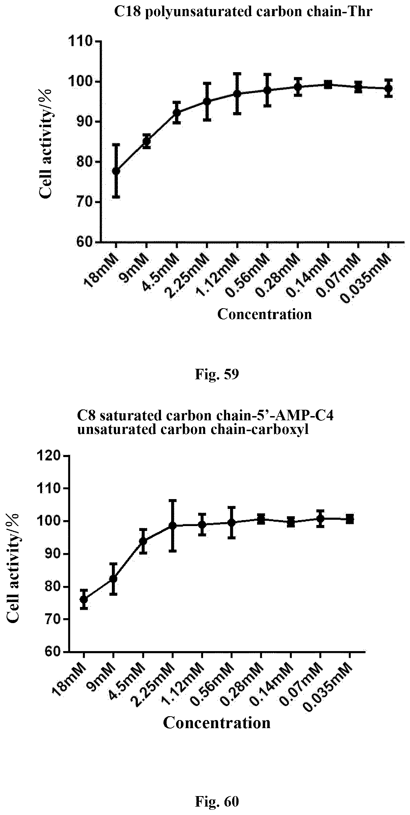

For the polyunsaturated carbon chain with 18 carbon atoms-threonine, the 18-carbon polyunsaturated carbon chain (oleic acid carbon chain) can be regarded as the acting moiety; threonine as the water-soluble moiety; and carboxyl group and amino group in the threonine molecule can be regarded as the binding moiety. The cytotoxicity results are shown in Fig. 59, indicating that the effect of the complex on cells from 4.5 mM to 0.035 mM is less than 10%, with good safety.

For the saturated carbon chain with 8 carbon atoms-adenosine 5'-monophosphate-unsaturated carbon chain with 4 carbon atoms-carboxyl group, the saturated carbon chain with 8 carbon atoms and the unsaturated carbon chain with 4 carbon atoms can be regarded as the action moieties; adenosine 5'-monophosphate as the water-soluble moiety; and the carboxyl group can be regarded as the binding moiety.

The cytotoxicity results are shown in Fig. 60, indicating that the effect of the complex on cells at concentrations of 0.035 mM-4.5 mM is less than 10%, with good safety.

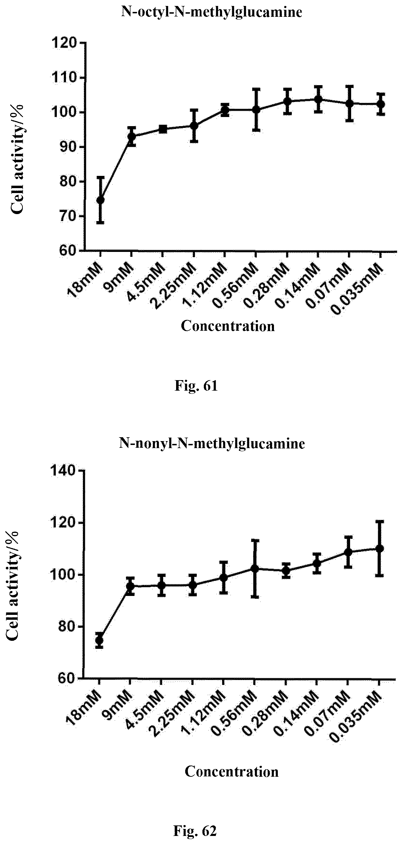

For N-octyl-N-methylglucamine, the saturated carbon chain with 8 carbon atoms can be regarded as the acting moiety; methylglucamine as the water-soluble moiety; and the hydroxyl group in the methylglucamine molecule can be regarded as the binding moiety. The cytotoxicity results are shown in Fig. 61, indicating that the effect of the complex on cells from 9 mM to 0.035 mM is less than 10%, with good safety.

For N-nonyl-N-methylglucamine, the saturated carbon chain with 9 carbon atoms can be regarded as the acting moiety; methylglucamine as the water-soluble moiety; and the amino group in the molecule of methylglucamine can be regarded as the binding moiety. The cytotoxicity results are shown in Fig. 62, indicating that the effect of the complex on cells (VERO E6 cells) at concentrations of 9 mM -0.035 mM is less than 10%, with good safety.

### Example 30 Bacteriostasis performance validation (carboxyl + saturated/unsaturated carbon chain + small molecule water-soluble moiety)

The compound in Example 29 was subjected to bacteriostasis test. A control solution was prepared according to the method steps of Example 1 (no fatty acid was added in the solution reaction, and the other steps were the same) to exclude the influence of other components (it can be seen from the inhibition result that the inhibition effect of the control group was almost 0, and the influence of other components in the composite product can be excluded).

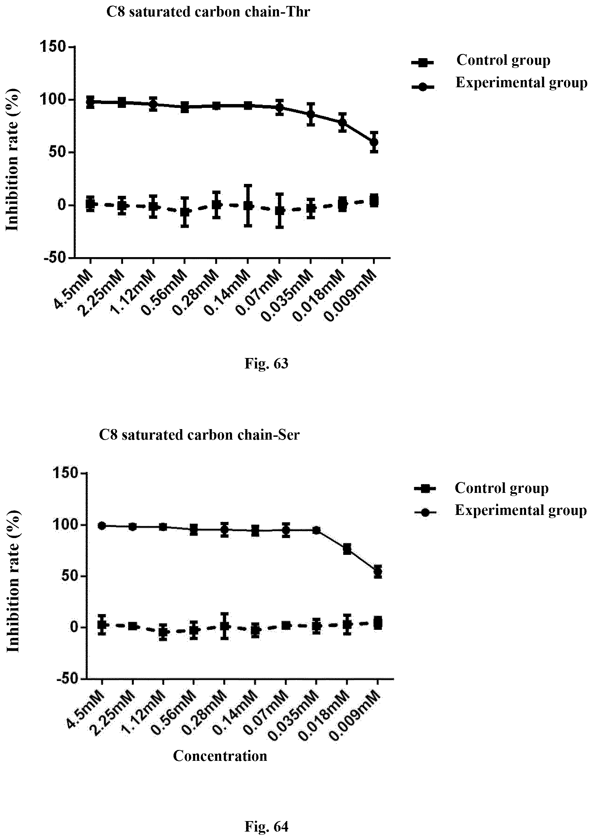

For the saturated carbon chain with 8 carbon atoms-threonine, the saturated carbon chain with 8 carbon atoms can be regarded as the acting moiety; threonine as the water-soluble moiety; and the carboxyl group and amino group in the threonine molecule can be regarded as the binding moiety. The inhibition results are shown in Fig. 63. It can be seen that the inhibition rate of the control group was 0; when the concentration of the experimental group was 0.035 mM, the inhibition rate was greater than 99%, with the bactericidal performance; when the concentration was between 0.035 mM and 0.009 mM, the inhibition rate was between 50% and 99%, with the half inhibition concentration being 0.009 mM.

For the saturated carbon chain with 8 carbon atoms-serine, the saturated carbon chain with 8 carbon atoms can be regarded as the acting moiety; serine as the water-soluble moiety; and the carboxyl group and amino group in the serine molecule can be regarded as the binding moiety. The inhibition results are shown in Fig. 64. It can be seen that the inhibition rate of the control group was 0; when the concentration of the experimental group was 0.035 mM, the inhibition rate was greater than 99%, with the bactericidal performance; when the concentration was between 0.035 mM and 0.009 mM, the inhibition rate was between 50% and 99%, with the half inhibition concentration being 0.009 mM.

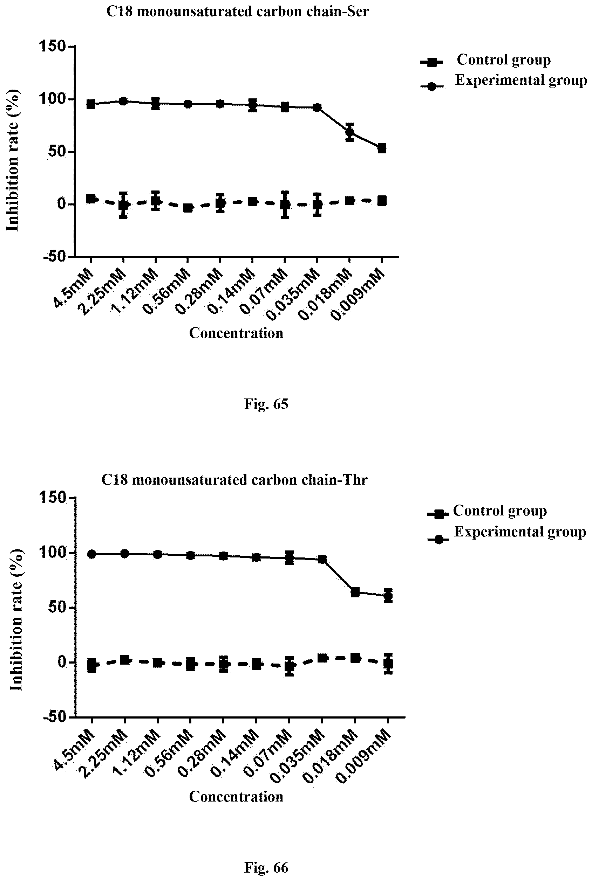

For the monounsaturated carbon chain with 18 carbon atoms-serine, the unsaturated carbon chain with 18 carbon atoms can be regarded as the acting moiety; serine as the water-soluble moiety; and the carboxyl group and amino group in the serine molecule can be regarded as the binding moieties. The inhibition results are shown in Fig. 65. It can be seen that the inhibition rate of the control group was 0; when the concentration of the experimental group was 0.035 mM, the inhibition rate was greater than 99%, with the bactericidal performance; when the concentration was between 0.035 mM and 0.009 mM, the inhibition rate was between 50% and 99%, with the half inhibition concentration being 0.009 mM.

For the monounsaturated carbon chain with 18 carbon atoms-threonine, the octadecane unsaturated carbon chain can be regarded as the acting moiety; threonine as the water-soluble moiety; and the carboxyl group and amino group in the threonine molecule can be regarded as the binding moiety. The inhibition results are shown in Fig. 66. It can be seen that the inhibition rate of the control group was 0; when the concentration of the experimental group was 0.035 mM, the inhibition rate was greater than 99%, with the bactericidal performance; when the concentration was between 0.035 mM and 0.009 mM, the inhibition rate was between 50% and 99%, with the half inhibition concentration being 0.009 mM.

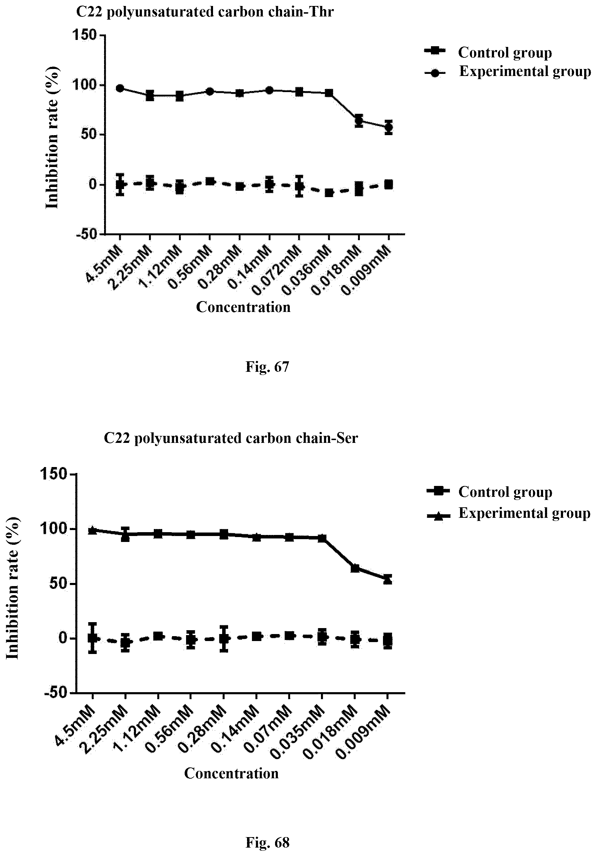

For the polyunsaturated carbon chain with 22 carbon atoms-threonine, the polyunsaturated carbon chain with 22 carbon atoms can be regarded as the acting moiety; threonine as the water-soluble moiety; and the carboxyl group and the amino group in the threonine molecule can be regarded as the binding moiety. The inhibition results are shown in Fig. 67. It can be seen that the inhibition rate of the control group was 0; when the concentration of the experimental group was 0.035 mM, the inhibition rate was greater than 99%, with the bactericidal performance; when the concentration was between 0.035 mM and 0.009 mM, the inhibition rate was between 50% and 99%, with the half inhibition concentration being 0.009 mM.

For the polyunsaturated carbon chain with 22 carbon atoms-serine, the polyunsaturated carbon chain with 22 carbon atoms can be regarded as the acting moiety; serine as the water-soluble moiety; and the carboxyl group and amino group in the serine molecule can be regarded as the binding moiety. The inhibition results are shown in Fig. 68. It can be seen that the inhibition rate of the control group was 0; when the concentration of the experimental group was 0.035 mM, the inhibition rate was greater than 99%, with the bactericidal performance; when the concentration was between 0.035 mM and 0.009 mM, the inhibition rate was between 50% and 99%, with the half inhibition concentration being 0.009 mM.

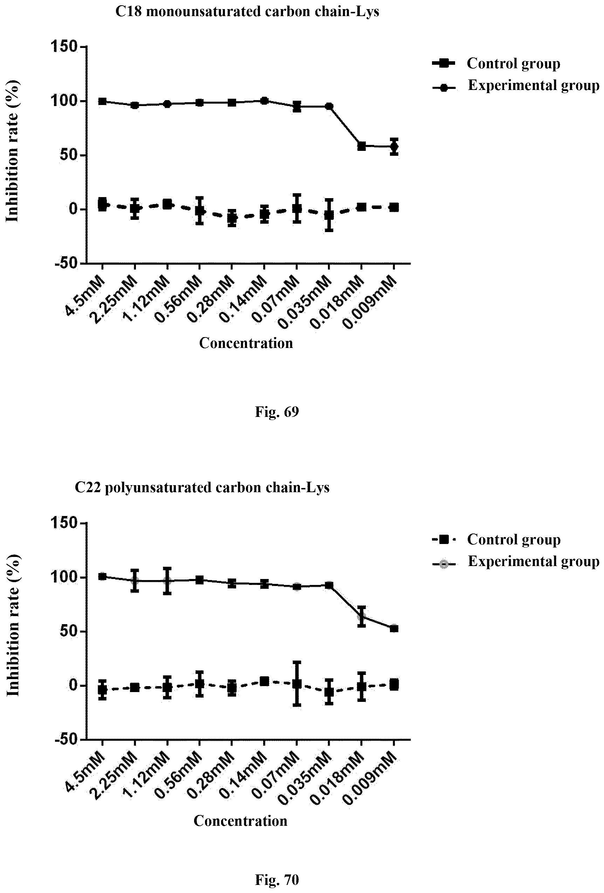

For monounsaturated carbon chain with 18 carbon atoms-lysine, the monounsaturated carbon chain with 18 carbon atoms can be regarded as the acting moiety; lysine as the water-soluble moiety; and the carboxyl group and amino group in the lysine molecule can be regarded as the binding moiety. The inhibition results are shown in Fig. 69. It can be seen that the inhibition rate of the control group was 0; when the concentration of the experimental group was 0.035 mM, the inhibition rate was greater than 99%, with the bactericidal performance; when the concentration was between 0.035 mM and 0.009 mM, the inhibition rate was between 50% and 99%, with the half inhibition concentration being 0.009 mM.

For the polyunsaturated carbon chain with 22 carbon atoms-lysine, the polyunsaturated carbon chain with 22 carbon atoms can be regarded as the acting moiety; lysine as the water-soluble moiety; and the carboxyl group and the amino group in the lysine molecule can be regarded as the binding moiety. The inhibition results are shown in Fig. 70. It can be seen that the inhibition rate of the control group was 0; when the concentration of the experimental group was 0.035 mM, the inhibition rate was greater than 99%, with the bactericidal performance; when the concentration was between 0.035 mM and 0.009 mM, the inhibition rate was between 50% and 99%, with the half inhibition concentration being 0.009 mM.

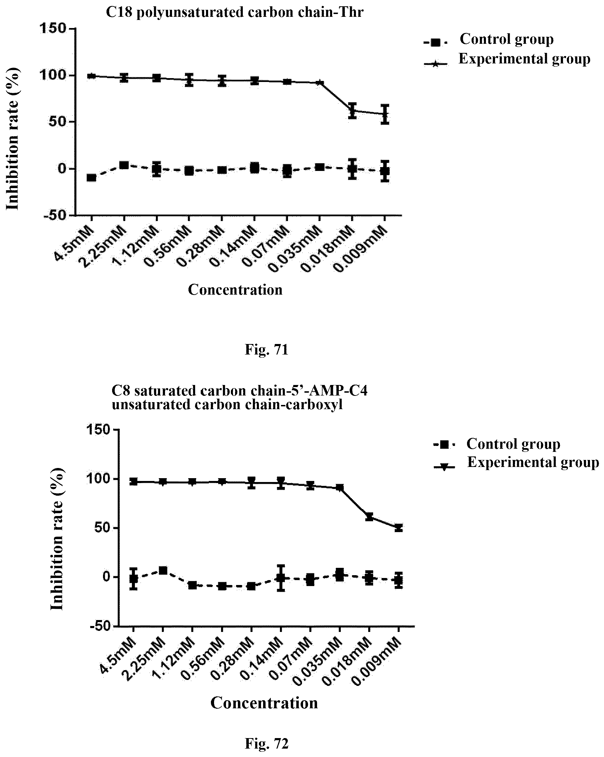

For the polyunsaturated carbon chain with 18 carbon atoms-threonine, the polyunsaturated carbon chain with 18 carbon atoms can be regarded as the acting moiety; threonine as the water-soluble moiety; and the carboxyl group and amino group in the threonine molecule can be regarded as the binding moiety. The inhibition results are shown in Fig. 71. It can be seen that the inhibition rate of the control group was 0; when the concentration of the experimental group was 0.035 mM, the inhibition rate was greater than 99%, with the bactericidal performance; when the concentration was between 0.035 mM and 0.009 mM, the inhibition rate was between 50% and 99%, with the half inhibition concentration being 0.009 mM.

For the saturated carbon chain with 8 carbon atoms-adenosine 5'-monophosphate-unsaturated carbon chain with 4 carbon atoms-carboxyl group, the saturated carbon chain with 8 carbon atoms and the unsaturated carbon chain with 4 carbon atoms can be regarded as the action moieties; adenosine 5'-monophosphate as the water-soluble moiety; and the carboxyl group can be regarded as the binding moiety. The inhibition results are shown in Fig. 72. It can be seen that the inhibition rate of the control group was 0; when the concentration of the experimental group was 0.035 mM, the inhibition rate was greater than 99%, with the bactericidal performance; when the concentration was between 0.035 mM and 0.009 mM, the inhibition rate was between 50% and 99%, with the half inhibition concentration being 0.009 mM.

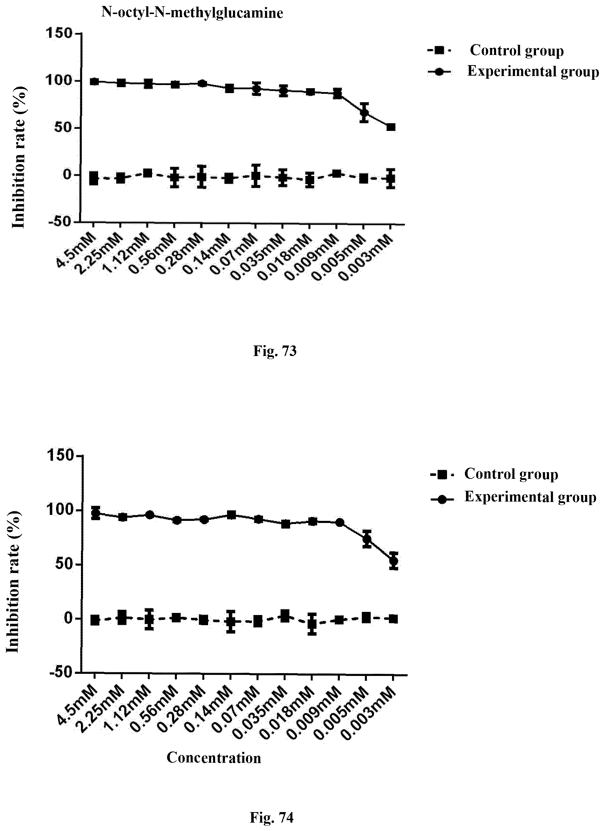

For N-octyl-N-methylglucamine, the saturated carbon chain with 8 carbon atoms can be regarded as the acting moiety; methylglucamine as the water-soluble moiety; and the hydroxyl group in the methylglucamine molecule can be regarded as the binding moiety. The inhibition results are shown in Fig. 73. It can be seen that the inhibition rate of the control group was 0; when the concentration of the experimental group was 0.009 mM, the inhibition rate was greater than 99%, with the bactericidal performance; when the concentration was between 0.035 mM and 0.009 mM, the inhibition rate was between 50% and 99%, with the half inhibition concentration being 0.003 mM.

For N-nonyl-N-methylglucamine, the saturated carbon chain with 9 carbon atoms can be regarded as the acting moiety; methylglucamine as the water-soluble moiety; and the hydroxyl group in the methylglucamine molecule can be regarded as the binding moiety. The inhibition results are shown in Fig. 74. It can be seen that the inhibition rate of the control group was 0; when the concentration of the experimental group was 0.009 mM, the inhibition rate was greater than 99%, with the bactericidal performance; when the concentration was between 0.035 mM and 0.009 mM, the inhibition rate was between 50% and 99%, with the half inhibition concentration being 0.003 mM.

Further bacteriostasis tests of the above compounds (at a concentration of 0.035mM) were carried out with reference to Example 19, and the results are shown in Table 25. The bacteriostasis efficiency of each compound was> 99%.

**Table 25**

| Bacteri a | Inhibition rate (%) | | | | | | | | | | | |
|---|---|---|---|---|---|---|---|---|---|---|---|---|
| | Satur ated carbo n chain with 8 carbo n atoms - threo nine | Sat ura ted car bo n cha in wit h 8 car bo n ato ms - ser ine | Mon ouns atura ted carb on chain with 18 carb on atom s-threo nine | Polyun saturat ed carbon chain with 22 carbon atoms-threoni ne | Polyu nsatur ated carbon chain with 22 carbon atoms-serine | Mono unsatu rated carbon chain with 18 carbon atoms-serine | Mono unsat urated carbo n chain with 18 carbo n atoms - lysine | Polyu nsatur ated carbo n chain with 22 carbo n atoms - lysine | Polyu nsatu rated carbo n chain with 18 carbo n atom s-threo nine | Satura ted carbon chain with 8 carbon atoms-adenos ine 5'-monop hosph ateunsatu rated carbon chain with 4 carbon atoms-carbox yl | N-octy l-N-met hylg luca min e | N-nony l-N-meth ylglu cami ne |
| *Escheri chia coli* | 99 | 99 | 99 | 99 | 99 | 99 | 99 | 99 | 99 | 99 | 99 | 99 |
| Methic illin-resistan t *Staphyl ococcu* s *aureus* | 99 | 99 | 99 | 99 | 99 | 99 | 99 | 99 | 99 | 99 | 99 | 99 |
| *Staphyl ococcu* s *aureus* | 99 | 99 | 99 | 99 | 99 | 99 | 99 | 99 | 99 | 99 | 99 | 99 |
| *Strepto coccus pneum oniae* | 99 | 99 | 99 | 99 | 99 | 99 | 99 | 99 | 99 | 99 | 99 | 99 |
| *Klebsie lla pneum oniae* | 99 | 99 | 99 | 99 | 99 | 99 | 99 | 99 | 99 | 99 | 99 | 99 |
| *Pseudo monas aerugi nosa* | 99 | 99 | 99 | 99 | 99 | 99 | 99 | 99 | 99 | 99 | 99 | 99 |

### Example 31 Virucidal property validation of (carboxyl + saturated/unsaturated carbon chain + small molecule water-soluble moiety)

The compound of Example 29 (used at a concentration of 0.009 mM) was subjected to virus neutralization according to the experimental method of Example 19, and the results are shown in Table 26. The virus neutralization efficiency of each compound was> 99%.

**Table 26**

| Test ed virus | Sterilization rate (%) | | | | | | | | | | | |
|---|---|---|---|---|---|---|---|---|---|---|---|---|
| | Sat urat ed car bon cha in wit h 8 car bon ato ms-thre oni ne | Sat urat ed car bon cha in wit h 8 car bon ato ms-seri ne | Monou nsatura ted carbon chain with 18 carbon atoms-threoni ne | Polyu nsatur ated carbon chain with 22 carbon atoms-threon ine | Polyu nsatur ated carbon chain with 22 carbon atoms-serine | Monou nsatura ted carbon chain with 18 carbon atoms-serine | Monou nsatura ted carbon chain with 18 carbon atoms-lysine | Polyu nsatur ated carbon chain with 22 carbon atoms-lysine | Polyu nsatur ated carbon chain with 18 carbon atoms-threon ine | Saturat ed carbon chain with 8 carbon atoms-adenos ine 5'-monop hospha te-unsatu rated carbon chain with 4 carbon atoms-carbox yl | N-octyl-N-methyl glucam ine | N-nonyl-N-methyl glucam ine |
| H7N 9 pseu dovi rus | 99 | 99 | 99 | 99 | 99 | 99 | 99 | 99 | 99 | 99 | 99 | 99 |
| H5N 1 pseu dovi rus | 99 | 99 | 99 | 99 | 99 | 99 | 99 | 99 | 99 | 99 | 99 | 99 |
| SAR S-CoV -2 pseu dovi rus | 99 | 99 | 99 | 99 | 99 | 99 | 99 | 99 | 99 | 99 | 99 | 99 |
| HIV pseu dovi rus | 99 | 99 | 99 | 99 | 99 | 99 | 99 | 99 | 99 | 99 | 99 | 99 |

### Example 32 Cytotoxicity of fatty acid-serum albumin complex (acting moiety + macromolecular water-soluble moiety/binding moiety)

### Cytotoxicity of fatty acid-serum albumin complex on VERO E6 cells

In this case, toxicity tests were conducted on Vero cells using linolenic acid serum albumin (ALA-HSA), eicosapentaenoic acid serum albumin (EPA-HSA), and docosahexaenoic acid serum albumin (DHA-HSA) prepared in Example 1.

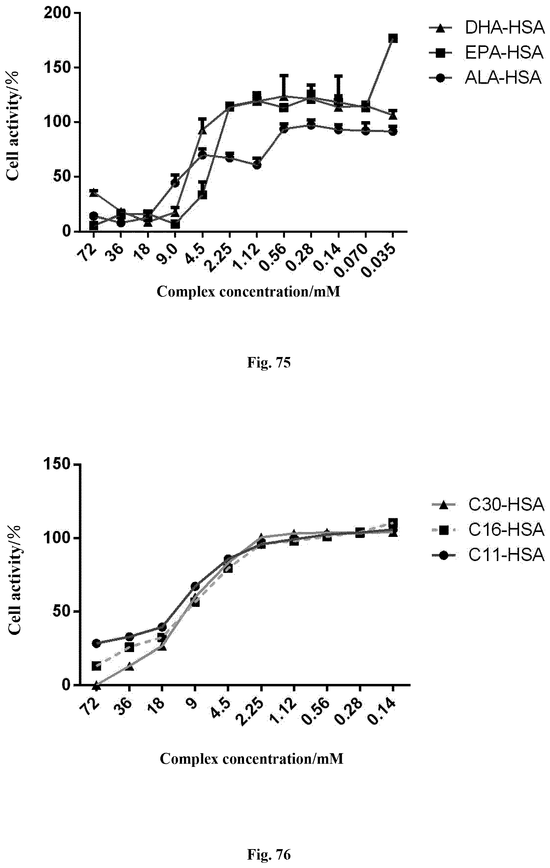

The cytotoxicity of the fatty acid or its derivative complexes was examined in Vero cells. VERO E6 cells (VERO E6:VERO African Green Monkey Kidney Cells, Art.No.: iCell-c014, manufacturer: iCell) were inoculated into a 96-well plate cell culture plate at a density of 5 × 10³ cells/well, 100ul per well. The 96-well plate was edge-sealed with sterile water or buffer and incubated at 37°C in a 5% CO₂ incubator for 24 h. The cells were treated with 72 mM, 36 mM, 18 mM, 9 mM, 4.5 mM, 2.25 mM, 1.12 mM, 0.56 mM, 0.28 mM, 0.14 mM, 0.07 mM, 0.035 mM fatty acid-serum albumin complex, respectively. There were three duplicate wells for each group, 10 ul per well. The blank control group was the culture medium with the same volume and drug-free group was a simple cell control. After drug action for 24 h, 10 ul of CCK-8 dye solution was added per well. The incubation was continued in the incubator for 1-4 h. OD value at the wavelength of 450 nm was detected, and the cell survival rate was analyzed. As shown in Fig. 75, when the concentration of fatty acids is below 4.5 mM, it is considered safe and non-toxic

In this example, serum albumin was both the water-soluble moiety and the binding moiety; and linolenic acid, eicosapentaenoic acid, and docosahexaenoic acid carbon chains were the acting moieties.

### Example 33 (Action moiety + macromolecular water-soluble moiety/binding moiety) Cytotoxicity of complex-fatty acid-serum albumin complex

### Detection of endothelial cell toxicity of fatty acid or its derivative complexes

In this case, toxicity of undecanoic acid-serum albumin (C11-HSA), hexadecenoic acid-serum albumin (C16-HSA), and triacontenoic acid-serum albumin (C30-HSA) prepared in Example 1 to hepatocytes was tested.

In hepatocytes (LX-2 (human hepatic stellate cells), art. No.: CL-0560, manufacturer: Procell), the cytotoxicity of the complex of fatty acids or derivatives thereof was tested. The cells were inoculated into a 96-well plate cell culture plate at a density of 5 × 10³ cells/well, 100 µl per well. The 96-well plate was edge-sealed with sterile water or buffer, incubated at 37°C in a 5% CO₂ incubator for 24 h. The cells were treated with 72 mM, 7.2 mM, and 0.72 mM fatty acid serum albumin complex, respectively. There were three duplicate wells for each group, 10 ul per well. The blank control group was the culture medium with the same volume and drug-free group was a simple cell control. After drug action for 24 h, 10 ul of CCK-8 dye solution was added per well. The incubation was continued in the incubator for 1-4 h. OD value at the wavelength of 450 nm was detected, and the cell survival rate was analyzed. As shown in Fig. 76, when the concentration of fatty acids is below 4.5 mM, it is considered safe and non-toxic. At a high concentration, the longer the carbon chain, the higher the cytotoxicity.

In this example, serum albumin was both the water-soluble moiety and the binding moiety, and the carbon chains of undecanoic acid, hexadecenoic acid and triacontanoic acid were the acting moieties.

### Example 34 Cytotoxicity assay (binding moiety + acting moiety + water-soluble moiety + binding moiety)

### Synthesis of Carboxy-Unsaturated Carbon Chain with 8 carbon atoms-Taurocholic Acid

1 mmol of 4-octenedioic acid and 1 mmol of EDC were weighed. The carboxyl was activated for 10 min under magnetic stirring. 1 mmol of DMAP (4-dimethylaminopyridine) was added. 1 mmol of sodium taurocholate was weighed and dissolved in 0.5 ml of water. The sodium taurocholate solution was slowly added to the activated 4-octenedioic acid dropwise and continued the stirring for reaction for 12 h. After the completion of the reaction, the reaction mixture solution was subjected to chromatographic purification. The reaction solution was fed to a Shephadex G10 chromatographic column (Φ26 mm × 50 cm) and elutes with normal saline at a flow rate of 50 ml/h. The eluents were collected step by step and detected using sulfuric acid formaldehyde chromogenic method. The first eluting peaks were combined to obtain the reaction product, i.e., a solution of 4-octenedioic acid-taurocholic acid complex (carboxy-unsaturated carbon chain with 8 carbon atoms-taurocholic acid), for subsequent experiments.

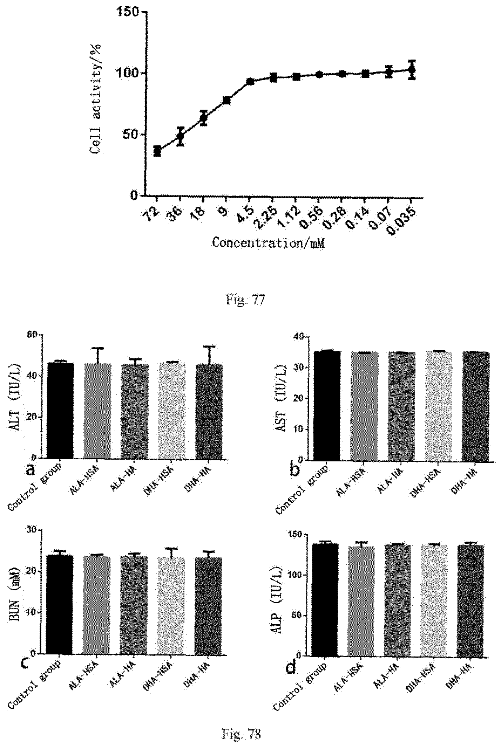

The toxicity test was performed on VERO E6 cells (VERO African green monkey kidney cells, Art. No.: iCell-c014, manufacturer: iCell):
Preparation of carboxy-unsaturated carbon chain with 8 carbon atoms-taurocholic acid solution. Cytotoxicity was tested in VERO E6 cells. The cells were inoculated into a 96-well plate cell culture plate at a density of 5 × 10³ cells/well, 100 µl per well. The 96-well plate was edge-sealed with sterile water or buffer, incubated at 37°C in a 5% CO2 incubator for 24 h. There were three duplicate wells for each group, 10 ul per well. The cells were treated with 72 mM, 36 mM, 18 mM, 9 mM, 4.5mM, 2.25 mM, 1.12 mM, 0.56 mM, 0.28 mM, 0.14 mM, 0.07 mM, and 0.035 mM carboxy-unsaturated carbon chain with 8 carbon atoms-taurocholic acid, respectively. There were three duplicate wells for each group, 10 ul per well. The blank control group was the culture medium with the same volume and drug-free group was a simple cell control. After drug action for 24 h, 10 ul of CCK-8 dye solution was added per well. The incubation was continued in the incubator for 1-4 h. OD value at the wavelength of 450 nm was detected, and the cell survival rate was analyzed. As shown in Fig. 77, when the concentration of fatty acids is ≤4.5mM, it is considered safe and non-toxic, and has no significant toxicity to VERO E6 cells.

In this example, 4-octenedioic acid was the binding moiety, wherein the free carboxyl group was the binding group of the binding moiety; the unsaturated carbon chain with 8 carbon atoms and the cholestane ring structure in the taurocholic acid structure were the acting moieties; and taurocholic acid was the water-soluble moiety.

### Example 35 Animal safety test (acting moiety + macromolecular water-soluble part/binding moiety)

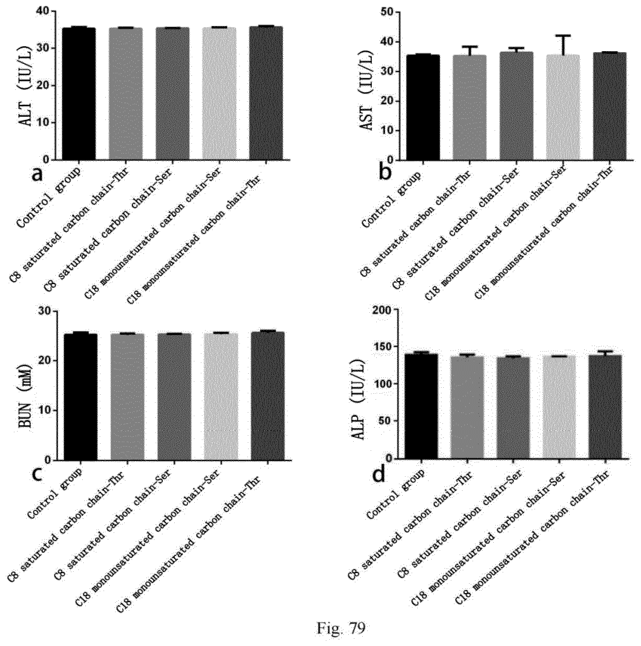

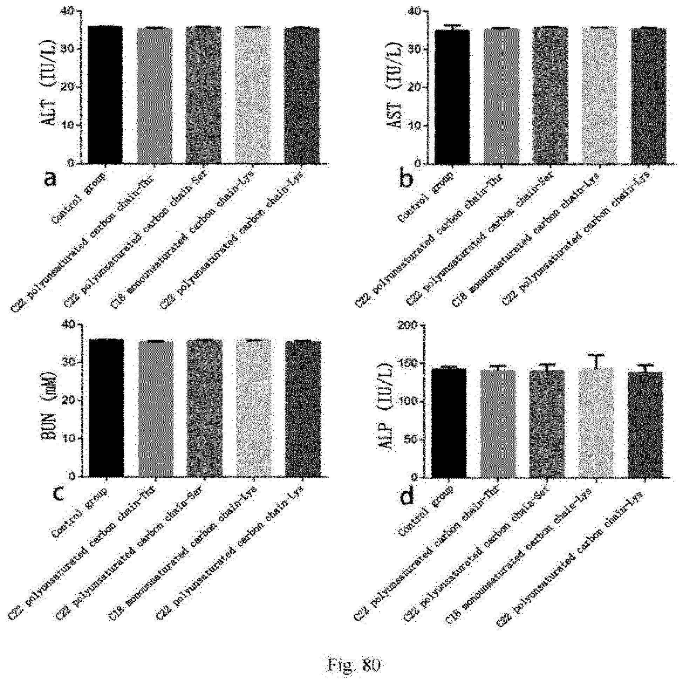

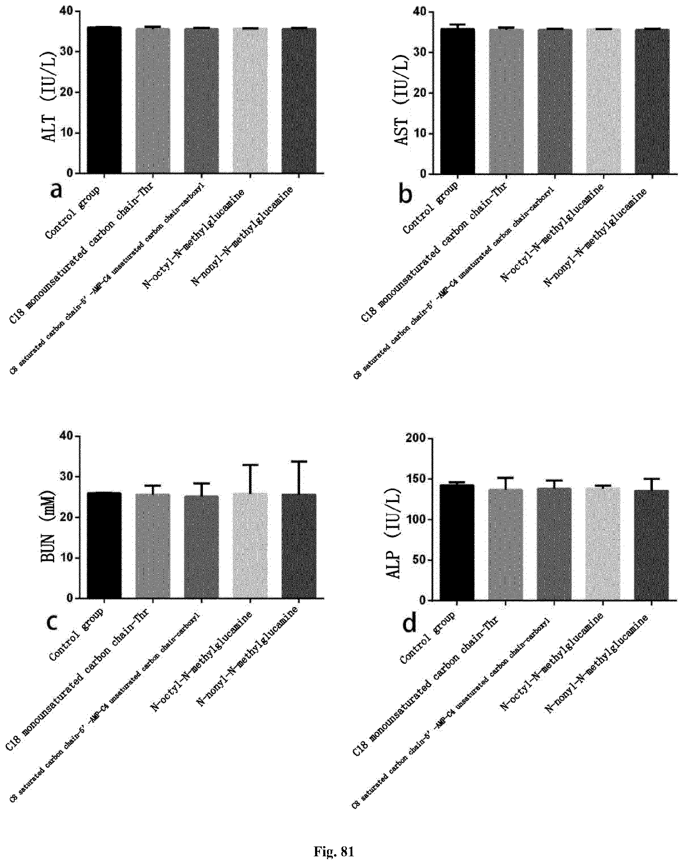

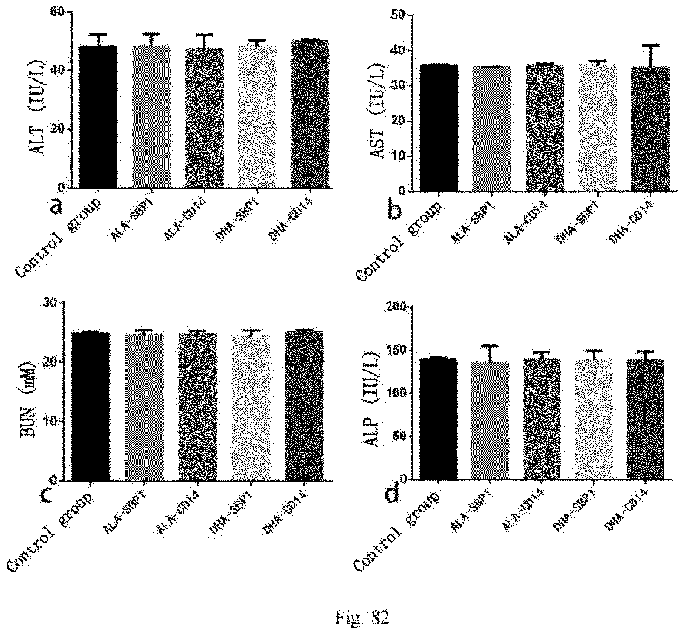

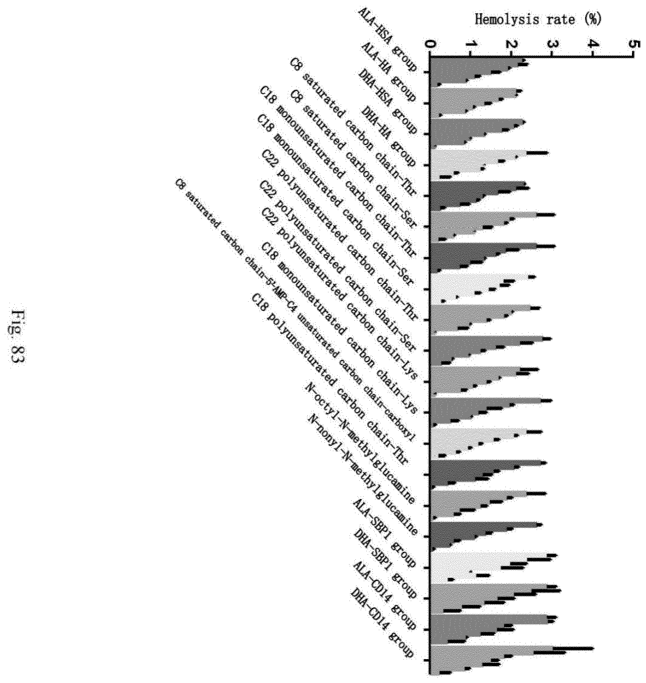

(1) Animal safety tests were performed with the fatty acid-macromolecular water-soluble moiety complexes synthesized in Examples 1 and 8 above.
   For pulmonary administration, the experimental groups (synthetic ALA-HSA group, ALA-HA group, DHA-HSA group, and DHA-HA group of Examples 1 and 8, administered with 562.5 uM calculated as fatty acid) via tail vein injection, 1 ml/mouse/time, three times. The blood was taken for detection. As shown in Fig. 78, a, b, c and d, they respectively represent liver function, liver/heart function, kidney/liver function and bone/hepatobiliary function. The results showed that there was no difference between the experimental groups (ALA-HSA group, ALA-HA group, DHA-HSA group, and DHA-HA group) and the control group, indicating that the compound had good safety.
   Mouse sources used in (2)-(5) below: C57BL/6 normal grade, 4-6 weeks, female.
(2) Animal safety tests were performed with the complex synthesized in Example 29.
   The mice were orally administered with 4.5 mM of saturated carbon chain with 8 carbon atoms-threonine, saturated carbon chain with 8 carbon atoms-serine, monounsaturated carbon chain with 18 carbon atoms-serine, and monounsaturated carbon chain with 18 carbon atoms-threonine (drinking water, mixed with food, intaking 5 mg of drug per mouse per day, a total of 5 days). The physiological state, body weight, fur color, food consumption, and mental state of the mice were not significantly different from those of the control group, indicating good safety. The blood was taken for detection. As shown in Fig. 79, a, b, c and d, they respectively represent liver function, liver/heart function, kidney/liver function and bone/hepatobiliary function. The results showed that there was no difference between the experimental groups and the control group, indicating that the compound had good safety.
(3) Animal safety tests were performed with the complex synthesized in Example 29.
   The mice were orally administered with 4.5 mM of polyunsaturated carbon chain with 22 carbon atoms-threonine, polyunsaturated carbon chain with 22 carbon atoms-serine, monounsaturated carbon chain with 18 carbon atoms-lysine, and polyunsaturated carbon chain with 22 carbon atoms-lysine (drinking water, mixed with food, intaking 5 mg of drug per mouse per day, a total of 5 days). The physiological state, body weight, fur color, food consumption, and mental state of the mice were not significantly different from those of the control group, indicating good safety. The blood was taken for detection. As shown in Fig. 80, a, b, c and d, they respectively represent liver function, liver/heart function, kidney/liver function and bone/hepatobiliary function. The results showed that there was no difference between the experimental groups and the control group, indicating that the compound had good safety.
(4) Animal safety tests were performed with the complex synthesized in Example 29.
   The mice were orally administered with polyunsaturated carbon chain with 18 carbon atoms-threonine, saturated carbon chainn with 8 carbon atoms -adenosine 5'-monophosphate-unsaturated carbon chain with 4 carbon atoms-carboxyl, N-octyl-N-methylglucamine, N-nonyl-N-methylglucamine (drinking water, mixed with food, intaking 5 mg of drug per mouse per day, a total of 5 days). The physiological state, body weight, fur color, food consumption, and mental state of the mice were not significantly different from those of the control group, indicating good safety. The blood was taken for detection. As shown in Fig. 81, a, b, c and d, they respectively represent liver function, liver/heart function, kidney/liver function and bone/hepatobiliary function. The results showed that there was no difference between the experimental groups and the control group, indicating that the compound had good safety.
(5) Caudal vein safety validation for ala-sbp1, dha-sbp1, ala-cd14, and dha-cd14 complexes
   The mice were administered with ALA-SBP1, DHA-SBP1, ALA-CD14, and DHA-CD14 complexes at a concentration of 4.5 mM via tail vein (100 ul, once a day, a total of 5 days). The physiological state, body weight, fur color, food consumption, and mental state of the mice were not significantly different from those of the control group, indicating good safety. The blood was taken for detection. As shown in Fig. 82, a, b, c and d, they respectively represent liver function, liver/heart function, kidney/liver function and bone/hepatobiliary function. The results showed that there was no difference between the experimental groups and the control group, indicating that the compound had good safety.
(6) Fresh blood (2.0 mL) from healthy rats was collected into a centrifuge tube (previously coated with heparin on the tube wall). After centrifugation at 4°C (4000 rpm, 10 min), the resulting pellet was red blood cells. The red blood cell pellet was then diluted 10-fold with sterile PBS. 0.6 mL of red blood cell suspension was taken and added with 0.4 mL of complexes with different concentrations (dispersed with PBS), so that the final concentrations were 0.56 mM, 1.12 mM, 2.25 mM, 4.5 mM, 9 mM and 18 mM. The mixture was gently blown and homogenized using a pipette, and incubated at 37°C for 6 h. The same volume of distilled water was used as positive control, and normal saline as negative control group. After the incubation, each sample was centrifuged at 4000 rpm for 10 min and photographed. 200 µL of the supernatant was transferred to a 96-well plate, and its absorbance at 560 nm was measured with a microplate reader to calculate the hemolysis rate. As shown in Fig. 83, the hemolysis rate increased slightly with increasing concentration, but all within the normal range (5%). Wherein, the hemolysis test rats in the hemolysis test described above: SD rats, normal grade, 200-250 g, male.

### Example 36 Antiviral activity test of fatty acid serum albumin complex against SARS-CoV-2 pseudovirus (acting moiety + macromolecular water-soluble part/binding moiety)

Antiviral activity assays were conducted using linolenic acid serum albumin (ALA-HSA), eicosapentaenoic acid serum albumin (EPA-HSA), and docosahexaenoic acid serum albumin (DHA-HSA) prepared in Example 1.

In this example, serum albumin was both the macromolecular water-soluble moiety and the binding moiety, and fatty acid was the acting moiety.

260 µL of autoclaved water was added to 36 wells around a 96-well plate to seal the edge, so as to reduce the error caused by evaporation of medium in edge wells. Column 2 (cell control CC) was added with DMEM complete medium, 150 µL/well. Column 3 (virus control VC) was added with DMEM complete medium, 100 µL/well. Fatty acid serum albumin samples with concentrations of 2.25 mM, 1.12 mM, 0.56 mM, 0.28 mM, 0.14 mM, and 0.07 mM were tested. SARS-CoV-2 pseudovirus (B.1.526.2, purchased from Beijing Tiantan Pharmaceutical Biotechnology Development Co. No: 80062. The pseudovirus system carries a firefly luciferase reporter gene. After infecting cells with the pseudovirus, the luminescence value can be detected by fluorescent substrates to determine the amount of virus infecting the cells. The pseudovirus uses VSV as a backbone, and the surface of the pseudovirus is embedded with the Spike protein of novel coronavirus, which can simulate the binding process of the true virus to the receptor and then enter the cell. After the sample with neutralizing activity reacts with the pseudovirus, the ability to infect the cell will be lost. By detecting the luminescence value of the control well and the sample well, whether the sample has neutralizing activity or not can be determined, and the effective concentration of the sample, i.e. the EC50 value, can be calculated. The pseudovirus can not replicate autonomously, and has only one round of infection ability, low in biosafety level and easy to operate, which is a common means for vaccine evaluation at present.) was diluted with the DMEM complete medium to 1.3- 2.3 × 10⁴ TCID 50/mL, and added 50 µL per well in column 3-11. The 96-well plate was incubated in a cell culture incubator (37°C, 5% CO2) for 1 h. After 30 min of incubation, Vero cells was started to be digested. The cell concentration was diluted to 2 × 10⁵ cells/mL. After the incubation, 100 µL cells were added per well to make 2 × 10⁴ cells in each well. The plate was put into a 37°C, 5% CO2 cell incubator for culture for 24h. After the completion of incubation, 250 µL of the supernatant was pipetted away. 100 µL of luciferase detection reagent was added for reaction at room temperature in the dark for 2 min. After repeatedly blowing and beating, 100 µL of liquid was transferred to a white plate. The luminescence values (RLU) were read using a multi-function imaging microplate reader.

The neutralization inhibition rate can be calculated by the following inhibition rate equation E1: Inhibition rate=(1-(Average luminous intensity-Average black control CC)/(Average VC-Average CC))*100%

Meanwhile, a control solution was prepared according to the method steps of Example 1 (no fatty acid was added in the solution reaction, and the other steps were the same) to exclude the influence of other components (it can be seen from the virus inhibition result that the virus inhibition effect of the control group was almost 0, and the influence of other components in the composite product can be excluded).

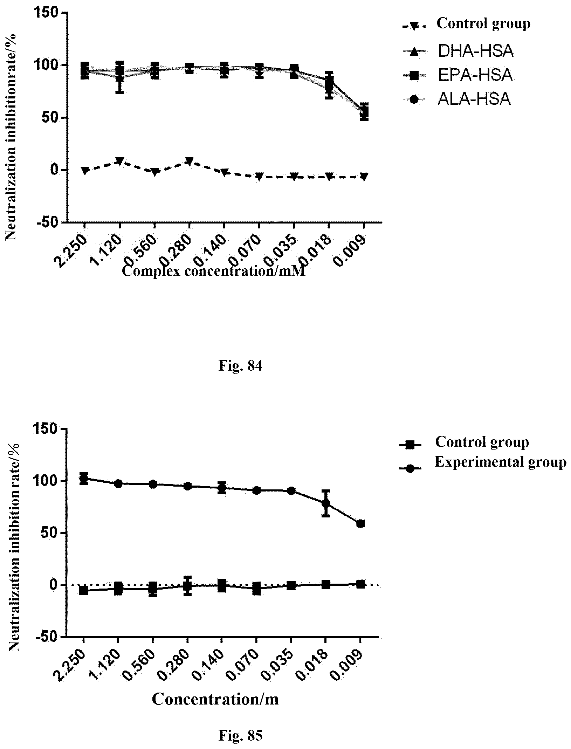

The results of neutralization inhibition rate are shown in Fig. 84. It can be seen that the neutralization inhibition rate of the control group was 0; the neutralization inhibition rate of the experimental groups of ALA-HSA, EPA-HSA and DHA-HSA at the concentration of 0.009 mM was 50%-60%. The specific bacteriostasis and sterilization results are summarized in Table 27.

**Table 27 Bactericidal and antibacterial properties of fatty acid-serum albumin (2 h)**

| Tested microorganism | Sterilization rate (%) | | |
|---|---|---|---|
| | ALA-HSA | EPA-HSA | DHA-HSA |
| *Escherichia coli* | >99 | >99 | >99 |
| *Staphylococcus aureus* | >99 | >99 | >99 |
| Methicillin-resistant *Streptococcus pneumoniae* | >99 | >99 | >99 |
| *Streptococcus pneumoniae* | >99 | >99 | >99 |
| *Klebsiella pneumoniae* | >99 | >99 | >99 |
| *Pseudomonas aeruginosa* | >99 | >99 | >99 |

### Example 37 Effect of eicosahexaenoic acid-serum albumin cyclodextrin encapsulation on rabies pseudovirus (acting moiety + water-soluble macromolecular group/binding Moiety)

A hexacosahexaenoic acid-serum albumin complex was prepared with reference to the method of example 1. The prepared complex and cyclodextrin were uniformly ground, wherein the mass ratio of the hexacosahexaenoic acid-serum albumin complex to cyclodextrin was 1: 1 to 1: 10. A method of low-temperature drying or spray drying was used to obtain particles, i.e., an oral preparation of a cyclodextrin clathrate, which may also be filled into a hard capsule. Other conventional oral preparation excipients may also be used herein.

In this example, serum albumin and cyclodextrin were macromolecular water-soluble moieties, while serum albumin was also the binding moiety, and eicosahexaenoic acid was the acting moiety.

The antiviral activity of rabies pseudovirus CVS-11 (purchased from Beijing Tiantan Pharmaceutical Biotechnology Development Co. No. 80052) was tested with the hexacosahexaenoic acid-serum albumin complex.

At the same time, a control solution was prepared according to the same method steps (no hexacosenoic acid was added in the solution reaction, and the other steps are the same) to exclude the influence of other components (it can be seen from the virus inhibition results that the virus inhibition effect of the control group was almost 0, and the influence of other components in the composite product can be excluded).

The method was the same as that of Example 36. The luminescence value (RLU) was read using a multi-functional imaging microplate reader. The results of neutralization inhibition rate are shown in Fig. 85. The neutralization inhibition rate of rabies pseudovirus CVS-11 in the control group was 0. The median inhibition concentration of rabies pseudovirus CVS-11 in the experimental group was 0.009 mM.

### Example 38 Antiviral activity assay of dotriacontahexaenoic acid-SBP1 complex against SARS-CoV-2 pseudovirus (acting moiety + polypeptide targeting binding moiety/water-soluble moiety)

In this example, SBP1 was both the polypeptide targeting binding moiety and the water-soluble moiety, and dotriacontahexaenoic acid was the acting moiety.

A dotriacontahexaenoic acid-SBP1 complex was prepared according to the method of Example 10. Dotriacontahexaenoic acid-SBP1 complex was tested for antiviral activity against SARS-CoV-2 pseudovirus (B.1.526.2, purchased from Beijing Tiantan Pharmaceutical Biotechnology Development Co. No: 80062. The pseudovirus system carries a firefly luciferase reporter gene. After infecting cells with the pseudovirus, the luminescence value can be detected by fluorescent substrates to determine the amount of virus infecting the cells. The pseudovirus uses VSV as a backbone, and the surface of the pseudovirus is embedded with the Spike protein of novel coronavirus, which can simulate the binding process of the true virus to the receptor and then enter the cell. After the sample with neutralizing activity reacts with the pseudovirus, the ability to infect the cell will be lost. By detecting the luminescence value of the control well and the sample well, whether the sample has neutralizing activity or not can be determined, and the effective concentration of the sample, i.e. the EC50 value, can be calculated. The pseudovirus can not replicate autonomously, and has only one round of infection ability, low in biosafety level and easy to operate, which is a common means for vaccine evaluation at present.).

Meanwhile, a control solution (no dotriacontahexaenoic acid was added in the solution reaction, and the other steps are the same) was prepared to exclude the influence of other components (it can be seen from the virus inhibition results that the inhibition effect of the control group was almost 0, and the influence of other components in the composite product can be excluded).

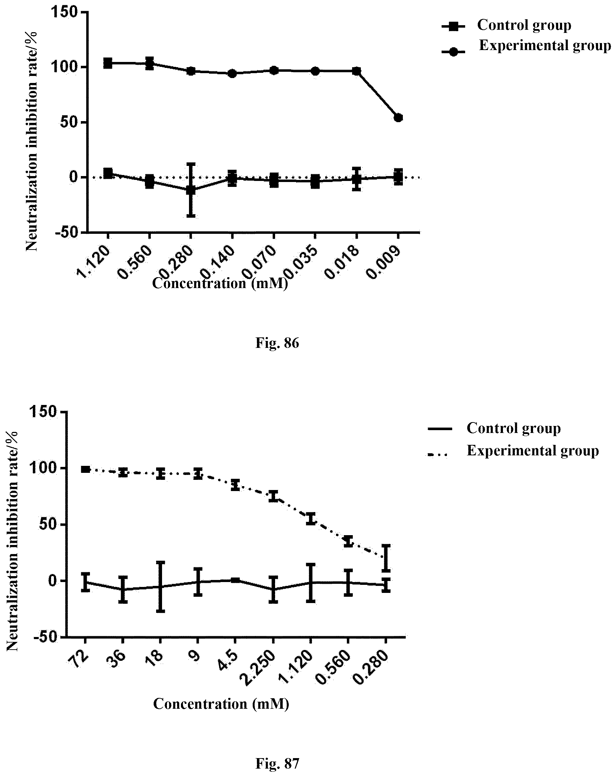

The method was the same as that of Example 36. The luminescence value (RLU) was read by the multi-functional imaging microplate reader. The results of neutralization inhibition rate are shown in Fig. 86. The neutralization inhibition rate of control group was 0, and the concentration of median inhibition (neutralization inhibition rate was 50%) of experimental group was 0.009 mM. The specific bacteriostasis and sterilization results are summarized in Table 28.

**Table 28 Bactericidal and antibacterial properties of dotriacontahexaenoic acid-SBP1 complex (2 h)**

| Tested microorganism | Sterilization rate (%) |
|---|---|
| *Escherichia coli* | >99 |
| *Staphylococcus aureus* | >99 |
| *Staphylococcus aureus* | >99 |
| *Streptococcus pneumoniae* | >99 |
| *Klebsiella pneumoniae* | >99 |
| *Pseudomonas aeruginosa* | >99 |

### Example 39 Effect of hexanoic acid-hyaluronic acid complex on HIV pseudovirus HIV18A-41 (short chain carbon chain acting moiety + macromolecular water-soluble moiety/binding moiety)

In this example, hyaluronic acid was both the macromolecular water-soluble moiety and the binding moiety, and the short carbon chain hexanoic acid moiety was the acting moiety.

The hexanoic acid-hyaluronic acid complex prepared in Example 12 was tested for antiviral activity against HIV pseudovirus HIV18A-41.

Meanwhile, a control solution (no hexanoic acid was added in the solution reaction, and the other steps are the same) was prepared to exclude the influence of other components (it can be seen from the virus inhibition results that the inhibition effect of the control group was almost 0, and the influence of other components in the composite product can be excluded).

The method was the same as that of Example 36. The luminescence value (RLU) was read using a multi-functional imaging microplate reader. The results of neutralization inhibition rate are shown in Fig. 87. It can be seen that the neutralization inhibition rate in the control group was 0, and the neutralization inhibition rate of the HIV pseudovirus HIV 18A-41 in the experimental group at a concentration of 1.12 mM was 50%, so the concentration of median inhibition is 1.12 mM.

### Example 40 Effect of n-nonanoic acid-hyaluronic acid complex on influenza pseudovirus H7N9-Fluc (short chain carbon chain acting moiety + macromolecular water-soluble moiety/binding moiety)

In this example, hyaluronic acid was both the macromolecular water-soluble moiety and the binding moiety, and the nonanoic acid short carbon chain moiety was the acting moiety.

The n-nonanoic acid-hyaluronic acid complex prepared in Example 12 was tested for antiviral activity against the influenza pseudovirus H7N9-Fluc.

Meanwhile, a control solution (no n-nonanoic acid was added in the solution reaction, other steps are the same) was prepared to exclude the influence of other components.

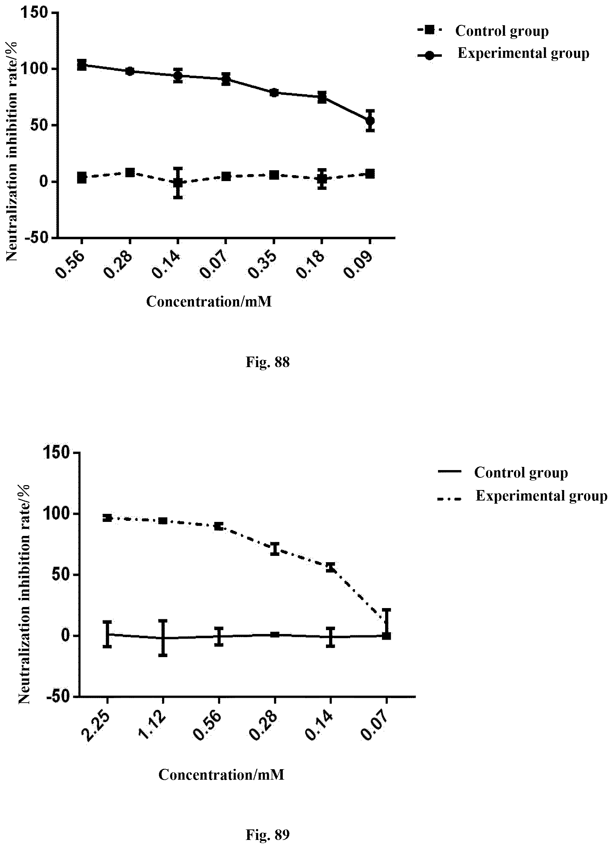

The method was the same as that of Example 36. The luminescence value (RLU) was read using a multi-functional imaging microplate reader. The results of neutralization inhibition rate are shown in Fig. 88. It can be seen that the neutralization inhibition rate in the control group was 0, and the neutralization inhibition rate of the H7N9-Fluc in the experimental group at a concentration of 0.009 mM was 54%, so the concentration of median inhibition is 0.009 mM.

### Example 41 Antiviral activity assay of octadecanoic acid-serum albumin complexes against HIV pseudovirus (long chain carbon chain acting moiety + macromolecular water-soluble moiety/binding moiety)

In this example, serum albumin was both the macromolecular water-soluble moiety and the binding moiety, and the octadecanoic acid long chain carbon chain moiety was the acting moiety.

Octadecanoic acid-serum albumin complex prepared referring to Example 1 was tested for antiviral activity against HIV pseudovirus.

Meanwhile, a control solution (no octadecanoic acid was added in the solution reaction, other steps are the same) was prepared to exclude the influence of other components.

The method was the same as that of Example 36. The luminescence value (RLU) was read using a multi-functional imaging microplate reader. The results of neutralization inhibition rate are shown in Fig. 89. The neutralization inhibition rate in the control group was 0, and the neutralization inhibition rate of the HIV pseudovirus in the experimental group at a concentration of 0.009 mM was 52%, so the concentration of median inhibition is 0.009 mM.

### Example 42 Effect of hyaluronic acid-coupled eicosanoid complex against H7N9-Fluc pseudovirus (long chain carbon chain acting moiety + macromolecular water-soluble moiety/binding moiety)

In this example, the hyaluronic acid macromolecule was both the macromolecular water-soluble moiety and the binding moiety, and the long carbon chain moiety of eicosanic acid was the acting moiety.

The eicosanoid-hyaluronic acid complex prepared in Example 12 was tested for antiviral activity against H7N9-Fluc pseudovirus. Meanwhile, a control solution (no eicosanoic acid was added in the solution reaction, other steps are the same) was prepared to exclude the influence of other components.

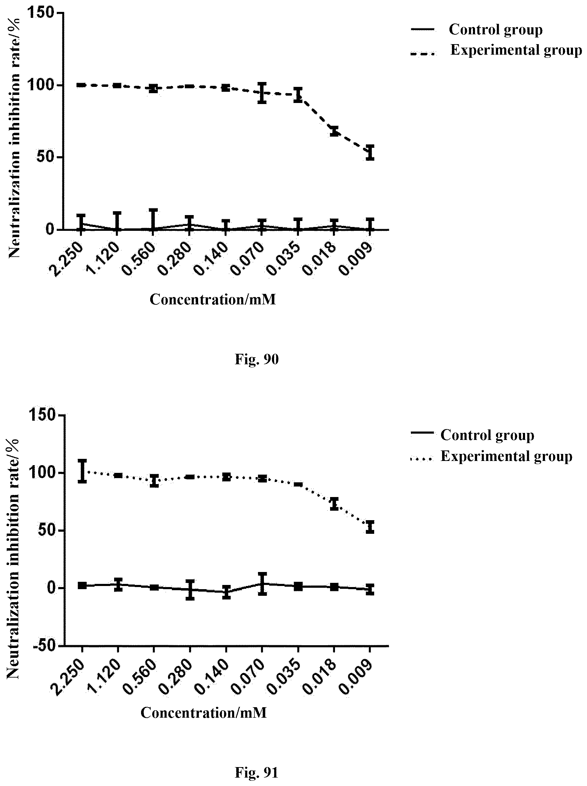

The method was the same as that of Example 36. The luminescence value (RLU) was read using a multi-functional imaging microplate reader. The results of neutralization inhibition rate are shown in Fig. 90. The neutralization inhibition rate in the control group was 0, and the neutralization inhibition rate of the H7N9-Fluc pseudovirus in the experimental group at a concentration of 0.009 mM was 53%, so the concentration of median inhibition is 0.009 mM.

### Example 43 Effect of octacosanoic acid-serum albumin complex on Influenza pseudovirus H5N1-Fluc (long chain carbon chain acting moiety + macromolecular water-soluble moiety/binding moiety)

In this example, serum albumin was both the macromolecular water-soluble moiety and the binding moiety, and the octacosanoic acid long carbon chain moiety was the acting moiety.

Octacosanoic acid-serum albumin complex prepared referring to Example 1 was tested for antiviral activity against influenza pseudovirus H5N1-Fluc.

Meanwhile, a control solution (no octacosanoic acid was added in the solution reaction, other steps are the same) was prepared to exclude the influence of other components.

The method was the same as that of Example 36. The luminescence value (RLU) was read using a multi-functional imaging microplate reader. The results of neutralization inhibition rate are shown in Fig. 91. The neutralization inhibition rate of control group was 0. The neutralization inhibition rate of experimental group against influenza pseudovirus H5N1-Fluc at the concentration of 0.009 mM was 53%, and the median inhibition concentration was presumed to be about 0.009 mM. Bacteriostasis tests were performed with the complexes and the specific results are summarized in Table 29.

**Table 29 Bactericidal and antibacterial properties of octacosanoic acid-serum albumin complex (0.018 mM) (2 h)**

| Tested microorganism | Sterilization rate (%) |
|---|---|
| *Escherichia coli* | >99 |
| *Staphylococcus aureus* | >99 |
| Methicillin-resistant *Streptococcus pneumoniae* | >99 |
| *Streptococcus pneumoniae* | >99 |
| *Klebsiella pneumoniae* | >99 |
| *Pseudomonas aeruginosa* | >99 |

### Example 44 Effect of serum albumin-coupled ω-3 fatty acid complex on HIV-derived lentivirus (long chain carbon chain acting moiety + macromolecular water-soluble moiety/binding moiety)

Linolenic acid serum albumin (ALA-HSA), eicosapentaenoic acid serum albumin (EPA-HSA), and docosahexaenoic acid serum albumin (DHA-HSA) prepared in Example 1 were tested for the effect on HIV-derived lentiviruses.

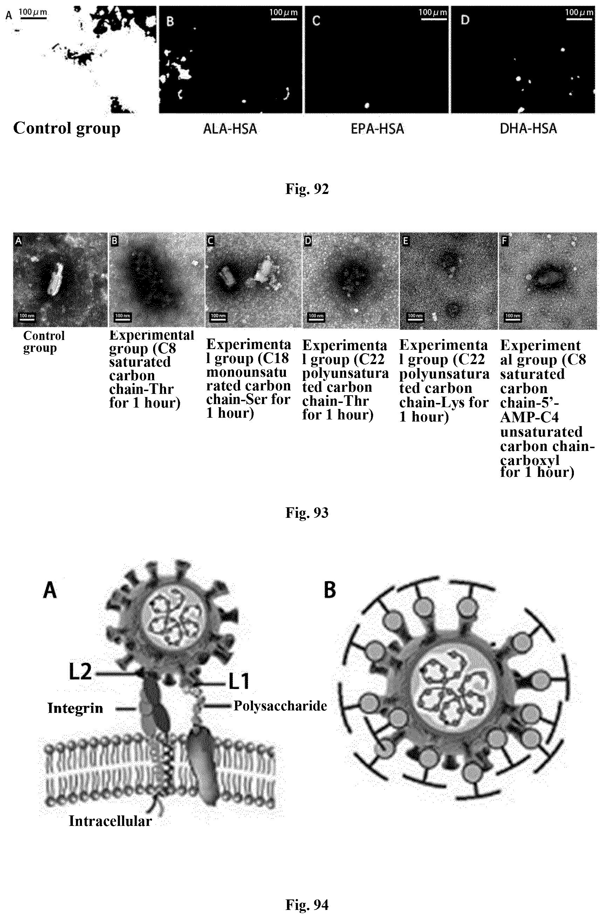

Hepatocytes (LX-2 (human hepatic stellate cells), code: CL-0560, manufacturer: Procell) were seeded at 2 × 10⁴ cells/well in a 96-well plate cell culture plate and incubated for 24 h at 37°C in a 5% CO₂ incubator. The virus was pretreated with fatty acid serum albumin complex at the concentration of 2.25 mM. After incubation for 1h, the virus was added into the 96-well plate. After 12-16 h, the medium was replaced with a complete medium. After continuous culture for 48 h, the fluorescence of cells was observed as shown in Fig. 92 A, B, C and D, which are the virus-infected cells in control group A (untreated group), group B after 1 h of treatment with ALA-HSA, group C after 1 h of treatment with EPA-HSA, and group D after 1 h of treatment with DHA-HSA, respectively. As can be seen from the fluorescence of the treated cells, the fluorescence intensity of the experimental group was significantly weaker than that of the control group, and the mean fluorescence intensity was analyzed using the software of ImageJ. The calculated P value was <0.01 (B: ALA-HSA group <0.001, C: EPA-HSA group <0.001, D: DHA-HSA group <0.001), indicating that the fatty acid serum albumin complex had significant inhibition effect on virus transfection.

### Example 45 Antiviral effect of the complex, disruption of the viral envelope structure (carboxyl + short chain unsaturation moiety)

The compounds of Example 29 (saturated carbon chain with 8 carbon atoms-threonine, eight-carbon monounsaturated carbon chain with 8 carbon atoms-serine, polyunsaturated carbon chain with 22 carbon atoms-threonine, polyunsaturated carbon chain with 22 carbon atoms-lysine, and saturated carbon chain with 8 carbon atoms-adenosine 5'-monophosphate-unsaturated carbon chain with 4 carbon atoms-carboxyl) were tested for the structure disruption on SARS-CoV-2 pseudovirus (B.1.526.2, purchased from Beijing Tiantan Pharmaceutical Biotechnology Development Co. No: 80062. The pseudovirus system carries a firefly luciferase reporter gene. After infecting cells with the pseudovirus, the luminescence value can be detected by fluorescent substrates to determine the amount of virus infecting the cells. The pseudovirus uses VSV as a backbone, and the surface of the pseudovirus is embedded with the Spike protein of novel coronavirus, which can simulate the binding process of the true virus to the receptor and then enter the cell). The morphology of the control group of SARS-CoV-2 pseudovirus is shown as form a in Fig. 93, and the morphology of the virus treated with each compound for 1 hour is shown as forms b-f in Fig. 93, respectively. It can be seen that the membrane structure of the virus treated with each compound for 1 hour was broken and the integrity of the structure was lost.

This example demonstrated the disruption of the envelope structure of enveloped viruses by fatty acid complexes from an apparent structural perspective using the compounds of Example 29, saturated carbon chain with 8 carbon atoms-threonine, monounsaturated carbon chain with 18 carbon atoms-serine, polyunsaturated carbon chain with 22 carbon atoms-threonine, polyunsaturated carbon chain with 22 carbon atoms-lysine, saturated carbon chain with 8 carbon atoms-adenosine 5'-monophosphate-unsaturated carbon chain with 4 carbon atoms-carboxyl.

### Example 46 Hydrophobic sequestering effect of complex on human papilloma virus (acting moiety + small molecule water-soluble moiety/binding moiety)

### (1) Process of action of compounds on non-enveloped viruses (Fig. 94 shows an action process profile)

The water-soluble compound increases the water solubility of the fatty acid, wherein the water-soluble compound can be a general water-soluble compound, such as polyethylene glycol, an amino acid, a polysaccharide, a monosaccharide, or an antigen/polypeptide which can recognize a regional site of a non-enveloped virus; thus, fatty acids loaded on molecules of water-soluble compounds can easily find non-enveloped viruses and tightly surround them to form fat globules, which are then excreted through the lymphatic system to play a role in preventing and treating of non-enveloped virus infection.

### (2) HPV non-enveloped virus was simulated with HPV L1 protein-loaded microparticles, and the binding process of N-octanoyl-N-methylglucamine to HPV non-enveloped virus was simulated in vitro

Preparation of HPV L1 protein-loaded microparticle: 200 mg of BSA and 10 ug of HPV L1 recombinant protein were weighed, dissolved in 20 ml of deionized water, and stirred with a magnetic stirrer at 500 rpm. The pH was ajusted to 9 with 10% NaOH solution. 0.1% sodium citrate (based on the mass fraction of total protein) was added and stirred overnight at 500 rpm to complete hydration. After overnight, desolventized acetone was added, and an equal volume of acetone was added at a rate of 1 ml/min. The solution was then turbid. Crosslinking was performed by adding 200 ul of 0.5 mg/ml pyridinium iodide (red fluorescence) followed by 0.01 ml of 4% paraformaldehyde in ethanol at 500 rpm for 3 h at room temperature. After completion of cross-linking, the proteins that were not formed into particles were removed by centrifugation, at 20000 g, 30 min/time, twice, to obtain HPV L1 protein-loaded microparticles, and then were dispersed evenly by using deionized water (200 µl).

32.2 mg of N-octanoyl-N-methylglucamine, 38.8 mg of indocyanine green dye, 20 mg of EDC were weighed, added with 100 ul of deionized water, and incubated overnight at 4 degrees with shaking to obtain N-octanoyl-N-methylglucamine carrying a green luminescent group.

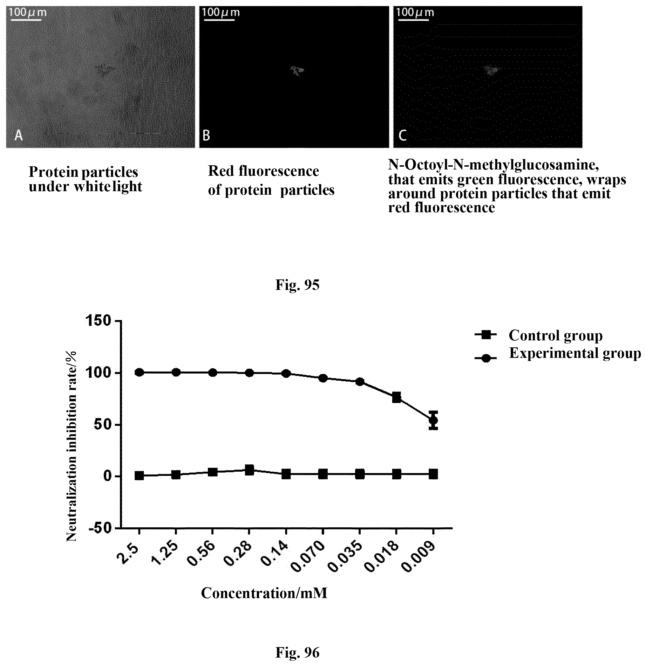

20 ul of HPV L1 protein-loaded microparticles and 20 ul of N-octanoyl-N-methylglucamine carrying a green luminescent group were pipetted, blown and shaken to be mixed evenly. The protein particles were observed under a fluorescence microscope, as shown in Fig. 95. A shows the protein particles seen under white light; B shows the red fluorescent protein particles carrying L1 protein prepared by simulation; and C shows the homogeneous mixture after adding the green fluorescent N-octanoyl-N-methylglucamine into the protein particle solution. It can be seen that after mixing with N-octanoyl-N-methylglucamine carrying the green luminescent group, the protein microparticles were surrounded by a layer of green fluorescence, indicating that N-octanoyl-N-methylglucamine could be targeted to attach around the surface of microHPV L1 protein-loaded microparticles, validating the theoretical scenario in Fig. 94. As specifically shown in Fig. 94, A refers to a non-enveloped virus such as HPV, the surface of which contains L1 and L2 proteins that can recognize cell surface integrins and polysaccharides, and is connected thereto, and then enters into the cell through endocytosis; B shows the L1 protein of the HPV recognized and linked by the heparan fragment contained in the drug of the present invention, and the other end is a hydrophobic structure; a large number of drug molecules can form a hydrophobic coating on the surface of the HPV, preventing the virus from connecting with the polysaccharide on the cell surface and invading the cell.

### Example 47 Inhibition effect of complex on HPV pseudovirus (long chain acting moiety + binding group/water-soluble moiety)

Docosahexaenoic acid coupled serine was taken as an example. Sample dilution: the multichannel electric pipette was adjusted to a 40 µl pipetting and 100µl mixing procedure to gently and repeatedly blow and pipette the liquid in B4-B11 well for 8 times and fully mix well. Then, 40 µl of liquid was transferred to the corresponding C4-C11 well, gently and repeatedly blown and pipetted for 8 times and then transferred to D4-D11 well, et cetera... Finally, 40 µl of liquid pipetted off from the G4-G11 wells.

HPV pseudovirus (HPV6-GFP) was diluted to 200TCID50 in DMEM complete medium and added to wells B3-G11, 120 ul per well. The dilution plate was brought to 4°C for 1 hour. 100 µl of pseudo virus serum mixture (or culture medium) was pipetted from each well of the dilution plate, and slowly added to the corresponding well of the pre laid cell culture plate by adhering to the wall. The edges of the culture plate were gently patted and mixed well. The cell culture plates were incubated for 60-96 hours at 37°C in a 5% CO₂ incubator. The detection was performed by an ELISPOT plate reader, and the inhibition rate of infection (%) = 1-(sample detection value-cell control value)/(virus control value-cell control value) × 100% was calculated.

The neutralization inhibition results are shown in Fig. 96. It can be seen that the median neutralization concentration is 0.009 mM.

### Example 48 Antibacterial effect of the complex, film detachment of Staphylococcus aureus was observed under transmission electron microscope (long-chain carbon chain acting moiety + macromolecular water-soluble part/binding moiety)

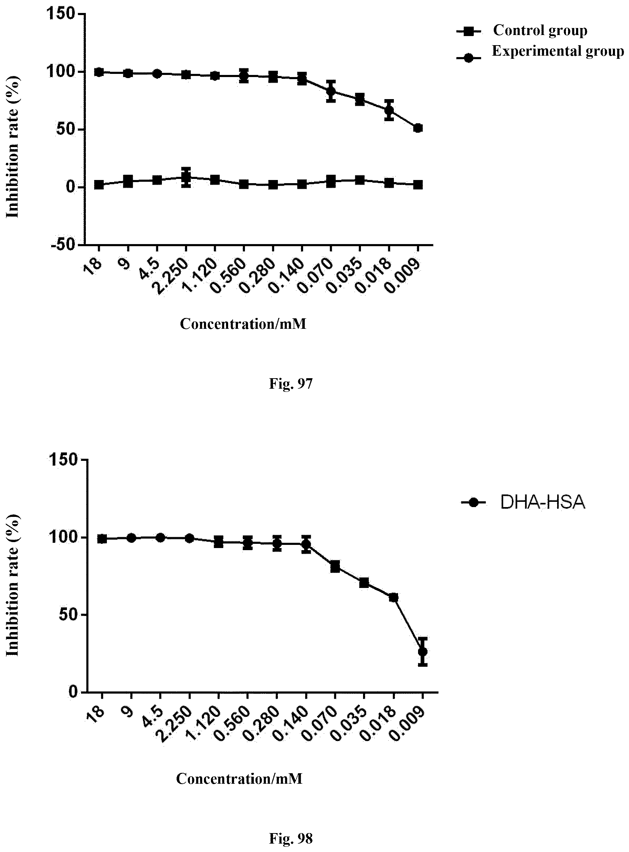

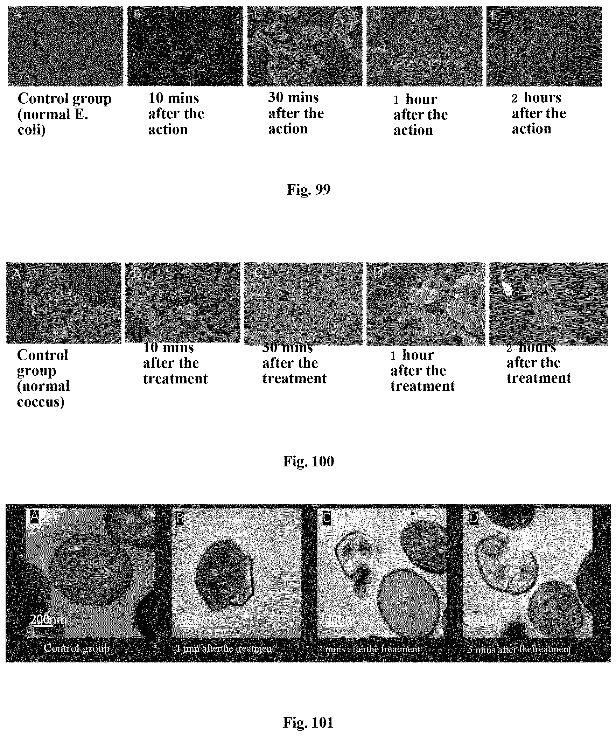

The method for grafting docosahexaenoic acid onto serum albumin was carried out as described in Example 1 to obtain a stock solution with a concentration of 20 mg/ml, which was diluted in a gradient of 18 mM, 9 mM, 4.5 mM, 2.25 mM, 1.12 mM, 0.56 mM, 0.28 mM, 0.14 mM, 0.07 mM, 0.035 mM, 0.018 mM, and 0.09 mM, respectively. Taking methicillin sodium (concentrations of 18 mM, 9 mM, 4.5 mM, 2.25 mM, 1.12 mM, 0.56 mM, 0.28 mM, 0.14 mM, 0.07 mM, 0.035 mM, 0.018 mM and 0.09 mM, respectively), the methicillin-resistant *Staphylococcus aureus* was diluted to 2 × 10⁶ cells/ml. 500 ul of the bacterial solution and 4.5 ml of the serum albumin fatty acid complex solution at different dilution concentrations were taken and mixed well by vortex, and continued to be shaken and mixed well at 37°C for 2 h. The inhibition rate was calculated using the plate count method. The inhibition results are as shown in Fig. 97. It can be seen that when the concentration of DHA-HSA was 18 mM-0.14 mM, the inhibition rate remained at 100%. The median inhibition concentration (MIC) was 0.009 mM. The bacteriostasis test against *Escherichia coli* was performed at the same concentration gradient. The bacteriostasis results are shown in Fig. 98. It can be seen from the graph that when the concentration of DHA-HSA was 18 mM-0.14 mM, the bacteriostasis rate remained at 100%, and the median inhibition concentration was 0.009 mM. The changes of bacterial structure were observed under scanning electron microscope (SEM), as shown in Fig. 99, A, B, C, D and E, which are the changes of appearance structure of *Escherichia coli* exposed to the drug for 10 min, 30 min, 1 h and 2 h, respectively, and as shown in Fig. 100, A, B, C, D and E, which are the changes of appearance structure of *Staphylococcus aureus* exposed to the drug for 10 min, 30 min, 1 h and 2 h, respectively. It can be seen that with the increase of exposure time, the external membrane structure of the bacteria first changed, becoming uneven, wrinkled, and gradually losing its normal shape. After 2 hours of action, the normal shape of the bacteria can no longer be seen and had been completely enveloped and destroyed. The membrane detachment of *Staphylococcus aureus* was observed under transmission electron microscope, as shown in Fig. 101, wherein A shows *Staphylococcus aureus* control group, B, C, and D show bacterial changes after treatment for 1 min, 2 min, and 5 min, respectively. The degree of membrane rupture increases with the increase of action time, wherein bacterial membrane detachment can be seen at 1 min, and partial bacterial membrane rupture can be seen after 2 min, resulting in bacterial cell rupture.

This example takes the long-chain unsaturated fatty acid complex docosahexaenoic acid serum albumin complex as an example, and demonstrates the structural damage process of fatty acid complexes on bacteria (taking *Staphylococcus aureus* as an example) from the perspective of epigenetics.

### Example 49

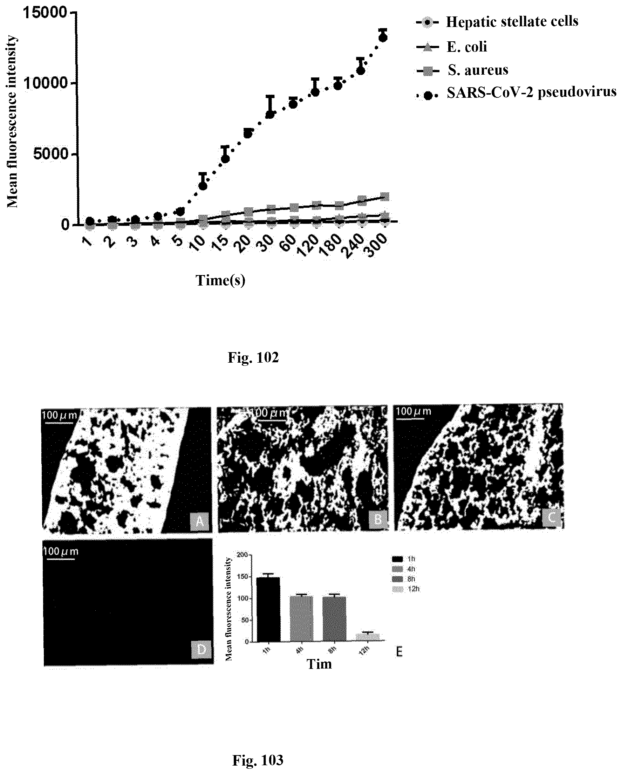

Taking docosahexaenoic acid-serum albumin labeled with DiI dye (red fluorescence) as an example, taking novel crownvirus pseudo virus (≤ 100 nm), *Staphylococcus aureus* (600-800 nm), *Escherichia coli* (2-3 um) and hepatic stellate cell (10-20 um) as receptors, and taking fluorescence intensity as examination index, as shown in Fig. 102, it can be seen that smaller individuals can more rapidly absorb docosahexaenoic acid-serum albumin labeled with red fluorescence, and absorb more fluorescein, indicating such compounds in this example, namely: the fatty acid carbon chain was the acting moiety, serum albumin/hyaluronic acid/polypeptide/small molecule water-soluble molecule was both the water-soluble moiety and the composition of the acting moiety, the speeds of which entering virus, bacteria and other microorganisms were higher than that of larger cells.

### Example 50 Animal experiment-retention time of macromolecular drugs in the lung

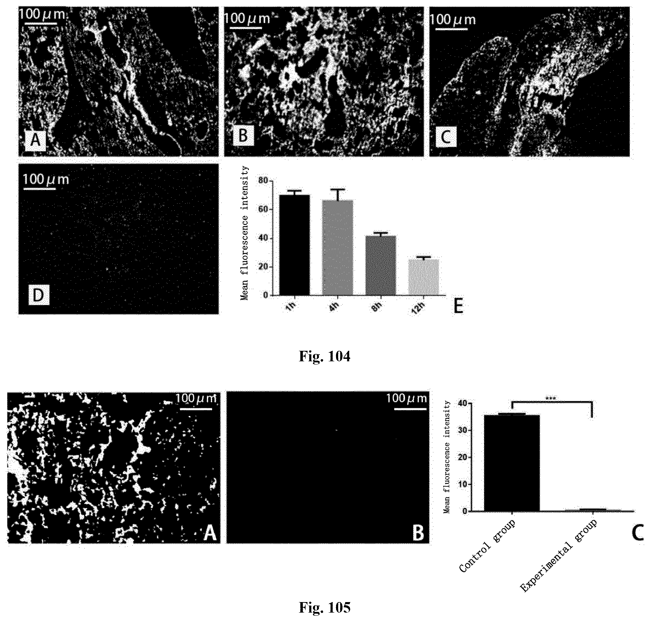

The following mice are referred to as transgenic mice: KI-hACE2 genotype C57BL/6mice, female, 4-6 weeks, Beijing Vital River Laboratory Animal Technology Co., Ltd.
(1) Docosahexaenoic acid-albumin labeled with DiI dye (DHA-HSA prepared in Example 1) was administered into mice in the form of lung lavage. The lung tissues of mice were removed after 1 h, 4 h, 8 h, and 12 h. As shown in Fig. 103, A (after 1 h), B (after 4 h), C (after 8 h), D (after 12 h) and E (pooled comparison), the complex showed a large retention capacity (with obvious fluorescence after 8 h). Thus, the albumin complex can be an ideal unknown factor for viral mutation to protect host cells after inhalation from infection.
(2) Eicosapentaenoic acid-hyaluronic acid complex labeled with DiI dye (DHA-HSA prepared in Example 1) was administered into mice in the form of lung lavage. The lung tissues of mice were removed after 1 h, 4 h, 8 h, and 12 h. As shown in Fig. 104, A (after 1 h), B (after 4 h), C (after 8 h), D (after 12 h) and E (pooled comparison), the complex showed a large retention capacity (with obvious fluorescence after 8 h). Thus, the hyaluronic acid complex can be an ideal unknown factor for viral mutation to protect host cells after inhalation from infection.

### Example 51 Animal experiment - pulmonary administration

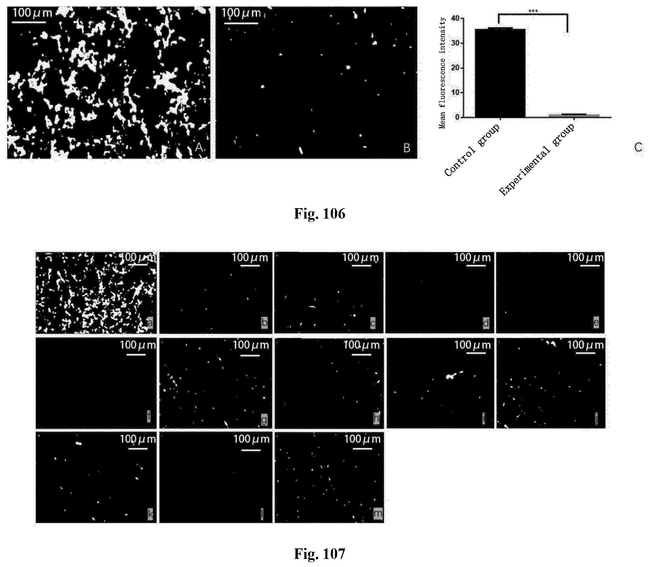

SARS-CoV-2 pseudovirus B.1.526.2, purchased from Beijing Tiantan Pharmaceutical Biotechnology Development Co. No: 80062. The pseudovirus system carries a firefly luciferase reporter gene. After infecting cells with the pseudovirus, the luminescence value can be detected by fluorescent substrates to determine the amount of virus infecting the cells. The pseudovirus has VSV as a backbone with the Spike protein embedded on the surface. The following mice are referred to as transgenic mice: KI-hACE2 genotype C57BL/6mice, female, 4-6 weeks, Beijing Vital River Laboratory Animal Technology Co., Ltd.
(1) The effectiveness of the nasal spray of docosahexaenoic acid-serum albumin (prepared according to the method of Example 1) was verified in animal experiments, in which the novel coronavirus carrying LUCI gene was used instead of the true virus, and C57 mice overexpressing hACE2 gene were used as experimental animals. The administration was performed by lung lavage. In the control group, 50 ul of SARS-CoV-2 pseudovirus (7 × 10⁵ TCID₅₀/ml) was perfused in the control group, and a temporary mixture of Novel crownvirus Pseudovirus (25 ul, 1.4 × 10⁶ TCID₅₀/ml) and docosahexaenoic acid-serum albumin solution (25 ul, 9 M) was infused into the lung in the experimental group (perfusion immediately after mixing). The administration was repeated 4 hours later. Three days later, the lungs of mice were taken for immunofluorescence of lung tissue. See Fig. 105, where A shows the positive control group without drug treatment, B shows the experimental group, and C shows the results of fluorescence analysis of A and B by ImageJ software. Compared with the control group, the fluorescence intensity of the experimental group was significantly weaker, and P value = 0.0164 <0.05 calculated by ImageJ software analysis, indicating significant difference.
(2) Taking eicosapentaenoic acid-hyaluronic acid complex (prepared according to the method of Example 8) as an example, the effectiveness of pulmonary administration experiment in animals was verified, in which the novel coronavirus carrying LUCI gene was used instead of the true virus, and C57 mice overexpressing hACE2 gene were used as experimental animals. The same as (1), the control group was given 50 ul of physiological saline solution by lung lavage, and the experimental group was given 50 ul of eicosapentaenoic acid-hyaluronic acid complex (based on eicosapentaenoic acid, concentration was 9 M). 4 hours later, the experimental group was given with 50 ul of novelcrown virus pseudovirus (with the concentration of 1.4 × 10⁶ TCID₅₀/ml). Three days later, the lungs of mice were taken for immunofluorescence of lung, see Fig. 106, where A shows the positive control group without drug treatment; B shows the experimental group; and C shows the results of fluorescence analysis of A and B by ImageJ software. Compared with the control group, the fluorescence intensity of the experimental group was significantly weaker, and P value = 0.0017 <0.01 calculated by ImageJ software analysis, indicating significant difference.

### Example 52 Animal experiment - oral administration

The following mice are referred to as transgenic mice: KI-hACE2 genotype C57BL/6mice, female, 4-6 weeks, Beijing Vital River Laboratory Animal Technology Co., Ltd.

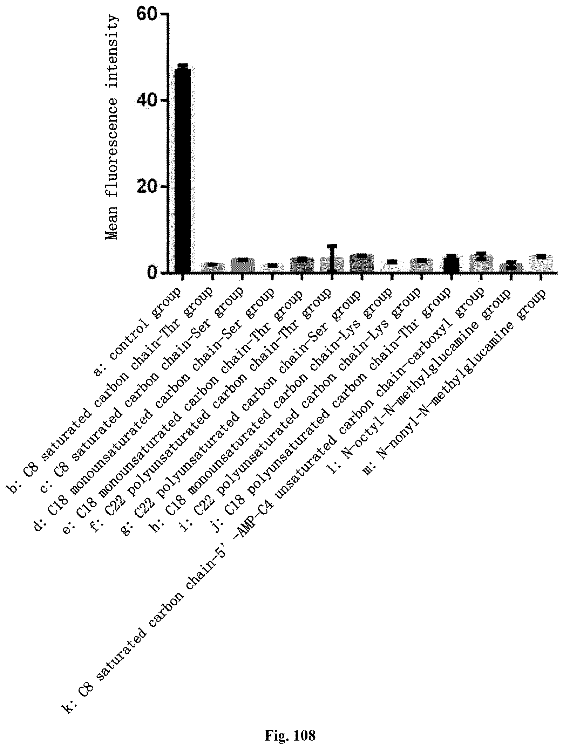

The compounds in Example 29, saturated carbon chainn with 8 carbon atoms-threonine, saturated carbon chain with 8 carbon atoms-serine, monounsaturated carbon chain with 18 carbon atoms-serine, monounsaturated carbon chain with 18 carbon atoms-threonine, polyunsaturated carbon chain with 22 carbon atoms-threonine, polyunsaturated carbon chain with 22 carbon atoms-serine, monounsaturated carbon chain with 18 carbon atoms-lysine, polyunsaturated carbon chain with 22 carbon atoms-lysine, polyunsaturated carbon chain with 22 carbon atoms-threonine, saturated carbon chainn with 8 carbon atoms-adenosine 5'-monophosphate-unsaturated carbon chain with 4 carbon atoms-carboxyl, N-octyl-N-methylglucamine, N-nonyl-N-methylglucamine, were validated for oral administration in animals. The drug solution was diluted to a concentration of 4.5 mM. Mice were fed an average of 5-10 mg of drug (based on the mass of the carbon chain of the acting moiety) per day with water and food for 48 h followed by pulmonary infusion of pseudovirus (same as the virus of Example 51 above) (at a concentration of 1.4 × 10⁶ TCID50/ml). Immunofluorescence of lung tissue was performed on the lungs of mice 2 days later, as shown in Fig. 107, in which a, b, c, d, e, f, g, h, i, j, k, l and m are respectively a control group, a saturated carbon chain with 8 carbon atoms-threonine group, a saturated carbon chain with 8 carbon atoms-serine group, an monounsaturated carbon chain with 18 carbon atoms-serine group, an monounsaturated carbon chain with 18 carbon atoms-threonine group, a polyunsaturated carbon chain with 22 carbon atoms-threonine group, a polyunsaturated carbon chain with 22 carbon atoms-serine group, a monounsaturated carbon chain with 18 carbon atoms-lysine group, a polyunsaturated carbon chain with 22 carbon atoms-lysine group, polyunsaturated carbon chain with 18 carbon atoms-threonine group, saturated carbon chain with 8 carbon atoms-adenosine 5' monophosphate-unsaturated carbon chain with 4 carbon atoms-carboxyl group, N-octyl-N-methylglucamine group, and N-nonyl-N-methylglucamine group. Compared with the control group, the experimental groups had significantly weaker fluorescence intensity. The mean fluorescence intensity was analyzed by the ImageJ software. See Fig. 108, P value was calculated <0.01 (P values in Fig. 108 are the follows: b: <0.001, c: <0.001, d: <0.001, e: <0.001, f=0.001, g: <0.001, h: <0.001, i: <0.001, j: <0.001, k: <0.001, l: <0.001, and m: <0.001), indicating a significant difference; where a of Fig. 108 is the control group, so there is no P value.

Further, saturated carbon chain with 8 carbon atoms-threonine, monounsaturated carbon chain with 18 carbon atoms-serine, monounsaturated carbon chain with 18 carbon atoms-threonine, polyunsaturated carbon chain with 22 carbon atoms-threonine, and N-octyl-N-methylglucamine were selected for validation of animal experiments. Mice were given 0 mg/kg, 8.6 mg/kg, 13 mg/kg, 13.4 mg/kg, 15 mg/kg and 11.2 mg/kg of drug by gavage administration once a day. Two days after administration, pseudovirus (the same as the virus in Example 51 above) was infused via the lung (concentration: 1.4 × 10⁶ TCID50/ml). Immunofluorescence of lung tissue was performed on the lungs of mice 2 days late. The results are shown in Fig. 153, in which, a, b, c, d, e, f, and g are respectively a control group, a saturated carbon chain with 8 carbon atoms-threonine group, monounsaturated carbon chain with 18 carbon atoms-serine group, monounsaturated carbon chain with 18 carbon atoms-threonine group, polyunsaturated carbon chain with 22 carbon atoms-threonine group, and N-octyl-N-methylglucamine group. Compared with the control group, the fluorescence intensity of the experimental group was significantly weaker. The mean fluorescence intensity was analyzed by ImageJ software. P value was calculated <0.01, indicating significant difference.

### Example 53 Effect of heparin-oleic acid complex on hepatitis B pseudovirus (long chain carbon chain acting moiety + macromolecular water-soluble moiety/binding moiety)

In this example, heparin was both the macromolecular water-soluble moiety and the binding moiety, and the oleic acid long carbon chain moiety was the acting moiety.

The heparin-oleic acid complex was prepared referring the method in Example 7, except replacing linoleic acid with oleic acid and hyaluronic acid with heparin (weight average molecular weight 16200 Da). Meanwhile, a control solution (no oleic acid was added in the solution reaction, other steps are the same) was prepared to exclude the influence of other components. The infrared spectrum of the prepared heparin-oleic acid complex is shown in Fig. 154. The absorption peak at 1561.50 cm⁻¹ of the product is from the ν_{C=C} of oleic acid, and the absorption peak at 1645.71 cm⁻¹ is from the ester bond formed after the esterification reaction between oleic acid and heparin. Due to the conjugation effect of the side group (-OH/-CN), the characteristic absorption peak of the ester bond shifts to the lower band.

The heparin-oleic acid complex was tested for the antiviral activity against HBVpp. Hepatitis B pseudovirus was self-made using the method described in the reference literature of our laboratory (Wang Junxue et al. Establishment of a primary liver cell model of tree shrews infected with hepatitis B virus pseudovirus in vitro, Biotechnology Letters. 2009, 20 (06): 753-759 ( 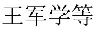 , 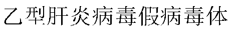 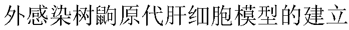 , 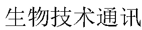 . 2009,20(06): 753-759)):
Package of Hepatitis B pseudovirus (HBVpp): 293T cells were co-transfected with HBsAgs plasmid, lentiviral packaging, and shuttle plasmid, and added to 200 µL of CaCl₂ solution to be mixed well, to obtain DNA-CaCl₂ solution. The DNA-CaCl₂ solution was added into 200 µL of BBS solution to be mixed well and inoculated for 10-20 min at room temperature to obtain a DNA-CaCl₂-BBS mixture. The DNA-CaCl₂-BBS mixture was dropped evenly into the whole 6 cm culture dish and cultured in a 37°C cell culture incubator containing 3% CO₂. After 8 h, the medium was replaced with the complete culture medium containing 10 mmol/L sodium butyrate to induce. After 8 h of induction, the solution was replaced. The residual sodium butyrate was removed by washing with PBS and 4 mL of fresh culture medium was added. The culture was continued for 24-48 h. The cell supernatant was collected, centrifuged at 3-5 min (4°C) to remove cell debris. The supernatant was filtered with a 0.45 µm filter. The cell supernatant obtained by filtration was directly used for cell infection or cryopreserved at -80°C for later use.

HBVpp infection of HepG2 cells: prior to infection, 800 µL of cell supernatant containing pseudovirus (after adding 200 µL of 200 g/L PEG8000 solution and mixing well) was taken and added to the wells of HepG2 cells to be infected, with 3 replicate wells set for each sample, and incubated in a cell culture incubator at 37°C and 5% CO₂ for 15 h. The cell supernatant containing HBVpp was pipetted, washed with PBS for 2-3 times, and added with DMEM medium to continue culture. The solution was changed every 2d. After 3-4 days of pseudovirus infection, the positive cells were observed and counted under fluorescent microscope. The infection rate of target cells was more than 80%.

HBVpp Infection Inhibition Assay: per 800µL of cell supernatant containing pseudovirus was added with heparin-glycerol complex and incubated at 4°C for 1 h. 200 µL of 200 g/L PEG8000 solution was added and mixed well. 800 µl of pseudo virus serum mixture (or culture medium) was pipetted from each well of the dilution plate, and slowly added to the corresponding well of the pre laid cell culture plate by adhering to the wall. The edges of the culture plate were gently patted and mixed well. The cell culture plates were incubated for 60-96 hours at 37°C in a 5% CO₂ incubator. The detection was performed by an ELISPOT plate reader. The neutralization inhibition rate was calculated according to the following formula: Neutralization inhibition rate (%) = 1-(sample detection value-cell control value)/(virus control value-cell control value) × 100%

The results of neutralization inhibition rate are shown in Fig. 155. The neutralization inhibition rate of control group was 0. The neutralization inhibition rate of experimental group against HBVpp at the concentration of 0.009 mM was 53%, and it is presumed that the concentration of median inhibition was about 0.009 mM.

Bacteriostasis tests were performed with the complexes and the specific results are summarized in Table 30.

**Table 30 Bactericidal and antibacterial properties of oleic acid-hyaluronic acid complex (2 h)**

| Tested microorganism | Sterilization rate (%) |
|---|---|
| *Escherichia coli* | >99 |
| *Staphylococcus aureus* | >99 |
| Methicillin-resistant *Streptococcus pneumoniae* | >99 |
| *Streptococcus pneumoniae* | >99 |
| *Klebsiella pneumoniae* | >99 |
| *Pseudomonas aeruginosa* | >99 |

### Example 54 Effect of hyaluronic acid-DHA complex on mouse model of sinusitis

BALB/C mice were weighed. The distilled water was mixed with chloral hydrate in a ratio of 10: 1. After chloral hydrate was fully dissolved, the mice are anesthetized with intraperitoneal anesthesia at a rate of 0.004 mL/g. The best anesthesia effect was that the whole body of the mouse was paralyze; the eyes were focusing and not closed; and there was no or little response to pain stimuli. The mouse head was disinfected with alcohol and the experiment was started after the alcohol was completely evaporated.

Microsurgical forceps were taken to hold a cotton strip in axial position and insert the cotton strip into the right nostril of group A, group B, and group C (5 mice in each group). The insertion depth of forceps shall not exceed 1.5 mm. The cotton strip was slowly inserted to the nasal cavity by 4 mm. In group A, the cotton strip was dripped with 10 µL of sterile normal saline. Groups B and C, the cotton strips were dripped with 10 µL of bacterial suspension containing 1.2 × 10⁹ CFU *Staphylococcus aureus,* and the remaining exposed portion of the strips was inserted into the nostrils.

Mice of groups A and B were sacrificed at 14 days by intraperitoneal injection of a respiratory failure dose of sodium pentobarbital (120 mg/kg). The skin of the head was removed. The ramus was cut and the mandible was removed. A coronal incision was made 1 mm behind the orbit and the nose was excised. The sinus mucosa was removed on an ice plate of a super clean bench, fixed with 4% paraformaldehyde solution, and made to HE sections by HE staining. The changes of sinus mucosa were observed under a light microscope. Nasal mucosa was taken for determination of myeloperoxidase (MPO) activity.

In group C, the cotton strips were removed and 2 drops of hyaluronic acid-DHA drug (concentration 4.5 mM) (after the hyaluronic acid-DHA complex prepared according to Example 8 was adjusted to the target concentration) were dripped in the right nasal cavity, three times a day, for a total treatment period of 7 days. Seven days after treatment the mice were sacrificed by intraperitoneal injection of a respiratory failure dose of sodium pentobarbital (120 mg/kg). The skin of the head was removed. The ramus was cut and the mandible was removed. A coronal incision was made 1 mm behind the orbit and the nose was excised. The sinus mucosa was removed on an ice plate of a super clean bench, fixed with 4% paraformaldehyde solution, and made to HE sections by HE staining. The changes of sinus mucosa were observed under a light microscope. Nasal mucosa was taken for determination of myeloperoxidase (MPO) activity.

Results: HE staining results of histological changes are shown in groups A, B, and C of Fig. 156. In group A, the mucosal epithelial tissue structure of nasal sinus and nasal cavity is regular and orderly, no obvious inflammatory cell infiltration in submucosa is found, and a small number of goblet cells are occasionally found; in group B, the nasal sinus and nasal mucosa shows inflammatory manifestations, uneven distribution of mucosal epithelium and a large number of inflammatory cell infiltration. The nasal sinus and nasal mucosa epithelial tissue in group C showed the same as that in group A, without obvious inflammation and a small amount of inflammatory cell infiltration.

The results of myeloperoxidase (MPO) activity are shown in Fig. 157, groups A, B and C, wherein the activity of MPO in nasal mucosa of mice in group B was significantly increased compared with group A in this figure, and the MPO in nasal mucosa of mice in treated group C was significantly decreased compared with group B.

### Example 55 Preparation and performance evaluation of Tween-80-threonine complex (surfactant as acting moiety providing carbon chain+ binding moiety)

In this example, tween-80 and threonine were used to prepare a surfactant amino acid complex, wherein Tween-80 was both the acting moiety providing the carbon chain, and was also the water-soluble moiety; and threonine was the binding moiety.

The specific process is as follows:
0.045 mmol of Tween-80 (Cas: 9005-65-6, molecular weight: 604.8Da) was weighed and dissolved in 5 ml of normal saline. 0.36 mmol of catalyst EDC and 0.36 mmol of DMAP were added and stirred for 10 min. 0.36 mmol of threonine was accurately weighed and add into the Tween-80 solution in 4 portions, with an interval of 30 min between each addition. After the fourth addition, the reaction was continued with stirring at room temperature for 8-24 h. After completion of the reaction, the reaction solution was dialyzed using a dialysis bag with a molecular weight cut-off of 500-1000 to purify the compound obtained in the reaction. The water was exchanged every 4 h (the molecular weight of both the catalyst and the unreacted threonine was less than 500 and can be removed). Dialysis was performed for 24 h. The infrared spectrum of the product Tween-80 threonine complex is shown in Fig. 158. The product contains the characteristic absorption peak of ester bond at 1736.83 cm⁻¹ (Tween 80 itself contains ester bond, and the reaction product of Tween 80 and threonine also contains ester bond). The absorption peak at 1644.93 cm⁻¹ can be ν_{C=C} contained in Tween 80, which is slightly shifted to lower band by 10 cm⁻¹ compared with Tween 80.

Referring to the method of the microbial experiments in Example 19, the results in Tables 31 and 32 show that the virus killing and bacterial killing abilities are greater than 99% when the concentration of the complex is 0.035 mM.

**Table 31 Virucidal properties of Tween-80-threonine complex (1 h)**

| Tested virus | Sterilization rate (%) |
|---|---|
| H7N9 pseudovirus | >99.9 |
| H5N1 pseudovirus | >99.9 |
| HIV pseudovirus | >99.9 |
| SARS-CoV-2 pseudovirus | >99.9 |

**Table 32 Bactericidal and antibacterial properties of Tween-80-threonine complex (2 h)**

| Tested microorganism | Sterilization rate (%) |
|---|---|
| *Escherichia coli* | >99 |
| *Staphylococcus aureus* | >99 |
| Methicillin-resistant *Streptococcus pneumoniae* | >99 |
| *Streptococcus pneumoniae* | >99 |
| *Klebsiella pneumoniae* | >99 |
| *Pseudomonas aeruginosa* | >99 |

### Example 56 Preparation and performance evaluation of cholesterol-PEG400-fumaric acid complex (steroid as acting moiety providing carbon chain + water-soluble moiety + binding moiety)

In this example, cholesterol was selected as an acting moiety to provide a carbon chain; PEG400 was the water-soluble moiety; and fumaric acid was the binding moiety. The specific process is as follows:
1.5 Mmol of PEG400, 1 mmol of cholesterol chloroformate, 1 mmol of triethylamine were added and the reaction was stirred at room temperature for 12 hours. 1 mmol of fumaric acid, 1.2 mmol of EDC and 1.2 mmol DMAP were further added to the reaction solution, and the reaction was continued with stirring at room temperature for 12 h. After completion of the reaction, the reaction solution was dialyzed using a dialysis bag with a molecular weight cut-off of 500-1000 to purify the compound obtained in the reaction. The water was exchanged every 4 h (the molecular weight of both the catalyst and the unreacted fatty acid was less than 500 and can be removed). Dialysis was performed for 24 h. The infrared spectrum of the product cholesterol-PEG400-fumaric acid complex is shown in Fig. 159. Compared with the peaks of fumaric acid and cholesterol, the peak at 1715.11 cm⁻¹ of the product is mainly increased, and the peak at 1642.52 cm⁻¹ (the original peak of fumaric acid is 1646.62 cm⁻¹ and is slightly shifted to the lower band after the esterification reaction occurs) shifted to the lower band due to conjugation effect is retained. The peaks at 1776.24 cm⁻¹, 1715.11 cm⁻¹, and 1642 cm⁻¹ are all from vC = O.

Referring to the method of the microbial experiments in Example 19, the results in Tables 33 and 34 show that the virus killing and bacterial killing abilities are greater than 99% when the concentration of the complex is 0.035 mM.

**Table 33 Virucidal properties of cholesterol-PEG400-fumaric acid complex (1 h)**

| Tested virus | Sterilization rate (%) |
|---|---|
| H7N9 pseudovirus | >99.9 |
| H5N1 pseudovirus | >99.9 |
| HIV pseudovirus | >99.9 |
| SARS-CoV-2 pseudovirus | >99.9 |

**Table 34 Bactericidal and antibacterial properties of cholesterol-PEG400-fumaric acid complex (2 h)**

| Tested microorganism | Sterilization rate (%) |
|---|---|
| *Escherichia coli* | >99 |
| *Staphylococcus aureus* | >99 |
| Methicillin-resistant *Streptococcus pneumoniae* | >99 |
| *Streptococcus pneumoniae* | >99 |
| *Klebsiella pneumoniae* | >99 |
| *Pseudomonas aeruginosa* | >99 |

### Example 57 Preparation and performance evaluation of phosphatidyl ethanolamine-PEG1000-octanedioic acid complex (phospholipids as acting moiety providing carbon chain + water-soluble moiety + binding moiety)

In this example, phosphatidyl ethanolamine-PEG1000 was selected as the acting moiety to provide a carbon chain, wherein PEG1000 was the water-soluble moiety and octanedioic acid was the binding moiety.

0.36 mmol of phosphatidylethanolamine and PEG1000 in a 1:1 molar ratio was dissolved in 10 ml of deionized water. 0.4 mmol of octanedioic acid, EDC 0.4 mmol, 0.4 mmol of DMAP were weighed and added with an acid solution and stirred in an ice bath to activate for 10 minutes. Activated octanedioic acid was added to a phosphatidylethanolamine-PEG1000 solution. The pH was adjusted to 7.0-7.4 with NaOH. The reaction was stirred at room temperature for 12 hours. After completion of the reaction, the reaction solution was dialyzed using a dialysis bag with a molecular weight cut-off of 500-1000 to purify the compound obtained in the reaction. The water was exchanged every 4 h (the molecular weight of both the catalyst and the unreacted fatty acid was less than 500 and can be removed). Dialysis was performed for 24 h. The infrared spectrum of the phosphatidylethanolamine-PEG1000-octanedioic acid complex is shown in Fig. 160. The product retains the ν_{C=O} of octanedioic acid at 1645.41 cm⁻¹, slightly shifting towards the lower band. The absorption peak of phospholipid ν_{C=O} at 1718.58 cm⁻¹ also shifts towards the lower band.

Referring to the method of the microbial experiments in Example 19, the results in Tables 35 and 36 show that the virus killing and bacterial killing abilities are greater than 99% when the concentration of the complex is 0.035 mM.

**Table 35 Virucidal properties of phosphatidyl ethanolamine-PEG1000-octanedioic acid complex (1 h)**

| Tested virus | Sterilization rate (%) |
|---|---|
| H7N9 pseudovirus | >99.9 |
| H5N1 pseudovirus | >99.9 |
| HIV pseudovirus | >99.9 |
| SARS-CoV-2 pseudovirus | >99.9 |

**Table 36 Bactericidal and antibacterial properties of phosphatidyl ethanolamine-PEG1000-octanedioic acid complex (2 h)**

| Tested microorganism | Sterilization rate (%) |
|---|---|
| *Escherichia coli* | >99 |
| *Staphylococcus aureus* | >99 |
| Methicillin-resistant *Streptococcus pneumoniae* | >99 |
| *Streptococcus pneumoniae* | >99 |
| *Klebsiella pneumoniae* | >99 |
| *Pseudomonas aeruginosa* | >99 |

### Example 58 Preparation and performance evaluation of α-tocopherol-hyaluronic acid complex (liposoluble vitamins carbon as an acting moiety chain providing carbon chain + water-soluble moiety/binding moiety)

In this example, α-tocopherol (vitamin E) was selected as the acting moiety providing carbon chain; and hyaluronic acid was selected as the water-soluble moiety/binding moiety.

The specific process is as follows:
0.045 mmol of hyaluronic acid (taking the molecular weight of 50 kDa as an example, 2.5 mg calculated by mass) was accurately weighed and dissolved in 5 ml of normal saline. 0.045 mmol of catalysts EDC and 0.045 mmol of DMAP were added. An acid solution was added and stirred to activate for 10 min. 0.36 mmol of α-Tocopherol was added and stirred to be dispersed uniformly. The pH was adjusted to neutral by adding NaOH solution. The reaction was continued with stirring at room temperature for 8-24 h. After completion of the reaction, the reaction solution was dialyzed using a dialysis bag with a molecular weight cut-off of 500-1000 to purify the compound obtained in the reaction. The water was exchanged every 4 h (the molecular weight of both the catalyst and the unreacted fatty acid was less than 500 and can be removed). Dialysis was performed for 24 h. The infrared spectrum of the product α-tocopherol-hyaluronic acid complex is shown in Fig. 161. The characteristic peaks of the product are 1647.20 cm⁻¹ (ester bond generated between tocopherol and hyaluronic acid, which shifts to the lower band due to the action of benzene ring) and 1561.08 cm⁻¹ (δ_{N-N} of hyaluronic acid).

Referring to the method of the microbial experiments in Example 19, the results in Tables 37 and 38 show that the virus killing and bacterial killing abilities are greater than 99% when the concentration of the complex is 0.035 mM.

**Table 37 Virucidal properties of alpha-tocopherol-hyaluronic acid complex (1 h)**

| Tested virus | Sterilization rate (%) |
|---|---|
| H7N9 pseudovirus | >99.9 |
| H5N1 pseudovirus | >99.9 |
| HIV pseudovirus | >99.9 |
| SARS-CoV-2 pseudovirus | >99.9 |

**Table 38 Bactericidal and antibacterial properties of alpha-tocopherol-hyaluronic acid complex (2 h)**

| Tested microorganism | Sterilization rate (%) |
|---|---|
| *Escherichia coli* | >99 |
| *Staphylococcus aureus* | >99 |
| Methicillin-resistant *Streptococcus pneumoniae* | >99 |
| *Streptococcus pneumoniae* | >99 |
| *Klebsiella pneumoniae* | >99 |
| *Pseudomonas aeruginosa* | >99 |

### Example 59 Preparation and performance evaluation of Sodium cholate-hyaluronic acid complex (steroid as an acting moiety providing carbon chain +water-soluble moiety/binding moiety)

In this example, sodium cholate was selected as the acting moiety providing a carbon chain, and hyaluronic acid was selected as the water-soluble moiety/binding moiety.

The specific process is as follows:
0.045 mmol of hyaluronic acid (taking the molecular weight of 50 kDa as an example, 2.5 mg calculated by mass) was accurately weighed and dissolved in 5 ml of normal saline. 0.045 mmol of catalysts EDC and 0.045 mmol of DMAP were added. An acid solution was added and stirred to activate for 10 min. 0.36 mmol of sodium cholate Tocopherol was added and stirred to be dispersed uniformly. The pH was adjusted to neutral by adding NaOH solution. The reaction was continued with stirring at room temperature for 8-24 h. After completion of the reaction, the reaction solution was dialyzed using a dialysis bag with a molecular weight cut-off of 500-1000 to purify the compound obtained in the reaction. The water was exchanged every 4 h (the molecular weight of both the catalyst and the unreacted fatty acid was less than 500 and can be removed). Dialysis was performed for 24 h. The infrared spectrum of the product sodium cholate-hyaluronic acid complex is shown in Fig. 162. The product contains 1776.40 cm⁻¹ (characteristic peak of ν_{c=o} for the esterification reaction product during the reaction process, hyaluronic acid, and sodium cholate), and 1669.15 cm⁻¹ is the absorption peak of ν_{N-H}.

Referring to the method of the microbial experiments in Example 19, the results in Tables 39 and 40 show that the virus killing and bacterial killing abilities are greater than 99% when the concentration of the complex is 0.035 mM.

**Table 39 Virucidal properties of sodium cholate-hyaluronic acid complex (1 h)**

| Tested virus | Sterilization rate (%) |
|---|---|
| H7N9 pseudovirus | >99.9 |
| H5N1 pseudovirus | >99.9 |
| HIV pseudovirus | >99.9 |
| SARS-CoV-2 pseudovirus | >99.9 |

**Table 40 Bactericidal and antibacterial properties of sodium cholate-hyaluronic acid complex (2 h)**

| Tested microorganism | Sterilization rate (%) |
|---|---|
| *Escherichia coli* | >99 |
| *Staphylococcus aureus* | >99 |
| Methicillin-resistant *Streptococcus pneumoniae* | >99 |
| *Streptococcus pneumoniae* | >99 |
| *Klebsiella pneumoniae* | >99 |
| *Pseudomonas aeruginosa* | >99 |

### Example 60 Preparation and performance evaluation of Heparin-EPA-octanedioic acid-fumaric acid-threonine complex

In this example, heparin and threonine were the water-soluble moieties and binding moieties; carbon chain of EPA, octanedioic acid, and fumaric acid were acting moieties, and free carboxyl groups of octanedioic acid and fumaric acid were also the binding moieties.

The specific process is as follows:
(1) Heparin-EPA complex (complex A) was prepared according to the method of Example 7
   Complex A was synthesized by replacing hyaluronic acid with heparin (weight average molecular weight 16200 Da) and linoleic acid with EPA. After 12 hours of the reaction, the next reaction was performed directly without dialysis.
(2) Complex A was reacted with octanedioic acid to obtain heparin-EPA-octanedioic acid complex (complex B)
   0.001mmol of octanedioic acid was accurately weighed and added with 0.001 mmol of EDC and 0.001mmol of DMAP as the catalysts. An acid solution was added to stir and activate for 10 min to obtain an activated octanedioic acid. The activated octanedioic acid was added to the reaction solution of (1). The pH was adjusted to neutral with NaOH solution, and the reaction was continued with stirring at room temperature for 12 h.
(3) Complex B was reacted with fumaric acid to obtain heparin-EPA-octanedioic acid-fumaric acid complex (complex C)
   0.001mmol of fumaric acid was accurately weighed and added with 0.001 mmol of EDC and 0.001mmol of DMAP as the catalysts. An acid solution was added to stir and activate for 10 min to obtain an activated fumaric acid. The activated fumaric acid was added to the reaction solution of (2). The pH was adjusted to neutral with NaOH solution, and the reaction was continued with stirring at room temperature for 12 h.
(4) Complex C was reacted with threonine to obtain the final product heparin-EPA-octanedioic acid-fumaric acid-threonine complex (complex D)

0.001mmol of threonine was accurately weighed and added with 0.001 mmol of EDC and 0.001mmol of DMAP as the catalysts. An acid solution was added to stir and activate for 10 min to obtain an activated threonine. The activated threonine was added to the reaction solution of (3). The pH was adjusted to neutral with NaOH solution, and the reaction was continued with stirring at room temperature for 12 h. After completion of the reaction, the reaction solution was dialyzed using a dialysis bag with a molecular weight cut-off of 500-1000 to purify the compound obtained in the reaction. The water was exchanged every 4 h (the molecular weight of both the catalyst and the unreacted fatty acid was less than 500 and can be removed). Dialysis was performed for 24 h obtain a complex D solution.

Infrared spectra of the above complexes A, B, C, and D prepared are shown in Fig. 163. After heparin is bound to the compound with carboxyl group, there is a strong absorption peak near the wavelength of 1650 cm⁻¹, which is the ester bond generated after binding. Due to more unsaturated bond in the molecule, the position of ester bond is slightly affected, and the position of ester bond is lower than that of normal ester bond (generally, the ν_{C=O} absorption peak of ester is 1740 cm⁻¹). In addition, a new stronger absorption peak appears at 1247-1250 cm⁻¹, which is ν_{as (C-O-C)} of the ester, i.e. the second characteristic peak of the ester.

Virus inhibition and bacteriostasis tests were performed with complex D according to the method of the microbial experiments in Example 19, and the results in Tables 41 and 42 below show that the virus killing and bacterial killing abilities are greater than 99% when the concentration of the complex is 0.035 mM.

**Table 41 Virucidal properties of complex D (1 h)**

| Tested virus | Sterilization rate (%) |
|---|---|
| H7N9 pseudovirus | >99.9 |
| H5N1 pseudovirus | >99.9 |
| HIV pseudovirus | >99.9 |
| SARS-CoV-2 pseudovirus | >99.9 |

**Table 42 Bactericidal and antibacterial properties of compound D (2 h)**

| Tested microorganism | Sterilization rate (%) |
|---|---|
| *Escherichia coli* | >99 |
| *Staphylococcus aureus* | >99 |
| Methicillin-resistant *Streptococcus pneumoniae* | >99 |
| *Streptococcus pneumoniae* | >99 |
| *Klebsiella pneumoniae* | >99 |
| *Pseudomonas aeruginosa* | >99 |

### Examples in Part II

**Table II-1A Comparison table of abbreviations for fatty acid complexes present in examples**

| Abbreviations | Fatty acid complex name |
|---|---|
| Ser-EPA | Serine-EPA |
| GA-DHA | Glucosamine-DHA |
| cholyltaurine-C14 | cholyltaurine-tetradecanoic acid |
| PEG-C18-2 | PEG-octadecadienoic acid |
| PEG-C7 | PEG-heptanoic acid |
| PEG-C9 | PEG-nonanoic acid |
| PEG-C11 | PEG-undecanoic acid |
| PEG-C18 | PEG-octadecanoic acid |
| RGD-2-OH-PA | RGD peptide-2-hydroxypalmitic acid |
| RGD-DA | RGD peptide-decanedioic acid |
| RGD-FA | RGD peptide-fumaric acid |
| RGD-C8 | RGD peptide-octanoic acid |
| Thr-BA | Threonine-butyric acid |
| Vitamin-C-OH | Vitamin C-ricinoleic acid |
| THr-C24 | Threonine-tetracosanoic acid |
| Chondroitin sulfate-2-C8 | Chondroitin sulfate -2-octenoic acid |
| Heparin-SA | Heparin-stearic acid |
| UDPGA-BA | Uridine diphosphate glucose-butyric acid |
| UDPGA-C12 | Uridine diphosphate glucose-dodecanoic acid |
| Thr-n-CA | Threonic acid-n-octanoic acid |
| UDPGA-C25 | Uridine diphosphate glucose-pentacosanoic acid |
| UDPGA-PA | Uridine diphosphate glucose-palmitic acid |
| UDPGA-OA | Uridine diphosphate glucose-oleic acid |
| Thr-C36 | Threonic acid-hexatriacontanoic acid |
| HA-OH-1 | Hyaluronic acid-ricinoleic acid |
| HA-SA | Hyaluronic acid-stearic acid |
| Vitamin-C-C30 | Vitamin C-triacontanoic acid |
| Vitamin-E | Vitamin E |
| Adenosine-C22-1 | Adenosine-erucic acid |
| cholylglycine-C18-1-OH | cholylglycine-Ricinoleic acid |

### ●Simulation of drug-membrane protein interaction

### Binding of drugs to endocytic proteins in cell membranes

### Example II-1 Binding site of fatty acid-amino acid complex to caveolin

The Ser-EPA in this example was synthesized according to the method of Example 29 in part I. The 3D structure of the active ingredient was obtained through the Pubmed database and Chemdraw software. The caveolin subtypes (Caveolin-1, Caveolin-2, and Caveolin-3) were downloaded using the PDB database. Related conventional treatment and molecular docking were performed on the target and the active ingredient in combination with the PyMOL 2.4.0 software and Auto Dock Tools 1.5.7 software. It was found that sites of amino acid residues LYS-135 of Caveolin-1 protein, LEU-49 and LEU-51 of Caveolin-2 protein, ASP-23 and GLU-21 of Caveolin-3 protein were involved in the interaction between Ser-EPA and Caveolin protein. The results are shown in Fig. 109, A, B and C.

### Example II-2 Binding site of fatty acid-glycoconjugate to caveolin

The GA-DHA in this example was synthesized according to the method of example 14 in part I. The 3D structure of the active ingredient was obtained through the Pubmed database and Chemdraw software. The caveolin subtypes (Caveolin-1, Caveolin-2, and Caveolin-3) were downloaded using the PDB database. Related conventional treatment and molecular docking were performed on the target and the active ingredient in combination with the PyMOL 2.4.0 software and Auto Dock Tools 1.5.7 software. It was found that sites of amino acid residues LEU-59, ASP-62, and ARG-146 of Caveolin-1 protein, LEU-49 and LEU-51 of Caveolin-2 protein, and ASP-40, GLU-42, LYS-69, PHE-41, and TYR-62 of Caveolin-3 protein were involved in the interaction between Ser-EPA and Caveolin protein. The results are shown in Fig. 110, A, B and C.

### Example II-3 Binding site of fatty acid-glycoconjugate to Dynamin

The GA-DHA in this example was synthesized according to the method of example 14 in part I. The 3D structure of the active ingredient was obtained through Pubmed database and Chemdraw software. The Dynamin protein was downloaded through PDB database. Relevant conventional treatment and molecular docking were performed on the target and the active ingredient in combination with PyMOL 2.4.0 software and Auto Dock Tools 1.5.7 software. It is found that sites of amino acid residues LEU-224 and PRO-226 of the Dynamin protein were involved in the interaction between GA-DHA and Dynamin protein. The results are shown in Fig. 111.

### Example II-4 Binding site of fatty acid-amino acid complex to Dynamin

The Ser-EPA in this example was synthesized according to the method of Example 29 in part I. The 3D structure of the active ingredient was obtained through Pubmed database and Chemdraw software. The Dynamin protein was downloaded through PDB database. Relevant conventional treatment and molecular docking were performed on the target and the active ingredient in combination with PyMOL 2.4.0 software and Auto Dock Tools 1.5.7 software. It was found that sites of amino acid residues GLU-310, ARG-467 and LYS-470 of the Dynamin protein were involved in the interaction between Ser-EPA and Dynamin protein. The results are shown in Fig. 112, A and B.

### Example II-5 Binding site of fatty acid-amino acid complex to Rab protein

The Ser-EPA in this example was synthesized according to the method of Example 29 in part I. The 3D structure of the active ingredient was obtained through Pubmed database and Chemdraw software. The Rab protein was downloaded through PDB database. Relevant conventional treatment and molecular docking were performed on the target and the active ingredient in combination with PyMOL 2.4.0 software and Auto Dock Tools 1.5.7 software. It was found that sites of amino acid residues LEU-118, ASN-115 and LYS-171 of the Rab protein were involved in the interaction between Ser-EPA and Rab protein. The results are shown in Fig. 113.

### Example II-6 Binding site of fatty acid-glycoconjugate to Rab protein

The GA-DHA in this example is synthesized according to the method of example 14 in part I. The 3D structure of the active ingredient was obtained through Pubmed database and Chemdraw software. The Rab protein was downloaded through PDB database. In combination with PyMOL 2.4.0 software and Auto Dock Tools 1.5.7 software, relevant conventional treatment and molecular docking are performed on the target and active ingredient. It was found that sites of amino acid residues GLY-144, ASN-115, GLU-168, and LYS-171 of Rab protein were involved in the interaction between GA-DHA and Rab protein. The results are shown in Fig. 114.

### Example II-7 Binding site of fatty acid-amino acid complex to Trpv1 (transient receptor cation channel)

The Ser-EPA in this example was synthesized according to the method of Example 29 in part I. The 3D structure of the active ingredient was obtained through Pubmed database and Chemdraw software. The Trpv1 protein was downloaded through PDB database. Relevant conventional treatment and molecular docking are performed on the target and active ingredientIn combination with PyMOL 2.4.0 software and Auto Dock Tools 1.5.7 software. It was found that sites of amino acid residues GLN-202, GLU-210, and ARG-115 of Rab protein were involved in the interaction between Ser-EPA and Trpv1 protein. The results are shown in Fig. 115.

### Example II-8: binding site of fatty acid-taurocholic acid complex to SNAP-25 (synaptosome-associated protein -25)

In this example, cholyltaurine-C14 was synthesized by the method in part I of Example 34. The 3D structure of the active ingredient was obtained through Pubmed database and Chemdraw software. The SNAP-25 protein was downloaded through PDB database. Relevant conventional treatment and molecular docking were performed on the target and the active ingredient in combination with PyMOL 2.4.0 software and Auto Dock Tools 1.5.7 software It was found that the sites of amino acid residues ASN-9, ARG-8 and MET-7 of SNAP-25 protein were involved in the interaction between cholyltaurine-C14 and SNAP-25 protein. The results are shown in Fig. 116.

### Example II-9 Binding site of fatty acid-PEG complex to SNAP-25 (synaptosome associated protein-25)

In this example, PEG-C18-2 was synthesized according to the method of Example 18 in part I. The 3D structure of the active ingredient was obtained through Pubmed database and Chemdraw software. The SNAP-25 protein was downloaded through PDB database. Relevant conventional treatment and molecular docking were performed on the target and the active ingredient in combination with PyMOL 2.4.0 software and Auto Dock Tools 1.5.7 software It was found that the site of amino acid residue SER-39 of SNAP-25 protein was involved in the interaction between PEG-C18-2 and SNAP-25 protein. The results are shown in Fig. 117.

### Example II-10 Binding sites of fatty acid-PEG complex to Arf (ADP ribosylation factor)

In this example, PEG-C7 was synthesized according to the method of example 18 in part I. The 3D structure of active ingredient was obtained through Pubmed database and Chemdraw software. The Arf protein was downloaded through PDB database. Relevant conventional treatment and molecular docking were performed on the target and the active ingredient in combination with PyMOL 2.4.0 software and Auto Dock Tools 1.5.7 software It was found that the sites of amino acid residues ASP-67 and ASN-52 of Arf protein were involved in the interaction between PEG-C7 and Arf protein. The results are shown in Fig. 118.

### Example II-11 Binding site of fatty acid-PEG complex to AP180 (bridging protein)

In this example, PEG-C9 was synthesized according to the method of Example 18 in part I. The 3D structure of the active ingredient was obtained through Pubmed database and Chemdraw software. The AP180 protein was downloaded through PDB database. Relevant conventional treatment and molecular docking were performed on the target and the active ingredient in combination with PyMOL 2.4.0 software and Auto Dock Tools 1.5.7 software It was found that the site of amino acid residue VAL-183 of AP180 protein was involved in the interaction between PEG-C9 and AP180 protein. The results are shown in Fig. 119.

### Binding of drugs to cell membrane endocytosis-related skeleton proteins

### Example 11-12 Binding site of fatty acid-nucleotide complex to alpha-actin

In this example, PEG-C11 was synthesized according to the method of example 18 in part I. The 3D structure of active ingredient was obtained through Pubmed database and Chemdraw software. The α-actin protein was downloaded through PDB database. Relevant conventional treatment and molecular docking are performed on target and active ingredientIn combination with PyMOL 2.4.0 software and Auto Dock Tools 1.5.7 software. It was found that sites of amino acid residues THR-77 and TRP-79 of α-actin protein were involved in the interaction between PEG-C11 and α-actin protein. The results are shown in Fig. 120.

### Example 11-13 Binding site of fatty acid-nucleotide complex to α-tubulin

In this example, PEG-C18 was synthesized according to the method of Example 18 in part I. The 3D structure of the active ingredient was obtained through Pubmed database and Chemdraw software. The α-tubulin protein was downloaded through PDB database. Relevant conventional treatment and molecular docking were performed on the target and the active ingredient in combination with PyMOL 2.4.0 software and Auto Dock Tools 1.5.7 software It was found that the site of amino acid residue GLN-256 of α-tubulin protein was involved in the interaction between PEG-C18 and α-tubulin protein. The results are shown in Fig. 121.

### Example 11-14 Binding site of fatty acid-polypeptide complex to Muscle Myosin

The RGD-2-OH-PA in this example was synthesized according to the method of Example 9 in part I. The 3D structure of the active ingredient was obtained Pubmed database and Chemdraw software. The Muscle Myosin protein was downloaded through PDB database. Relevant conventional treatment and molecular docking were performed on the target and the active ingredient in combination with PyMOL 2.4.0 software and Auto Dock Tools 1.5.7 software It was found that the site of amino acid residue GLU-562 of Muscle Myosin protein was involved in the interaction between RGD-2-OH-PA and Muscle Myosin protein. The results are shown in Fig. 122.

### Example 11-15 Binding site of fatty acid peptide complex to Septin protein

The RGD-DA in this example was synthesized according to the method of Example 9 in part I. The 3D structure of the active ingredient was obtained through the Pubmed database and Chemdraw software. The Septin protein was downloaded through the PDB database. Relevant conventional treatment and molecular docking were performed on the target and the active ingredient in combination with PyMOL 2.4.0 software and Auto Dock Tools 1.5.7 software. It was found that the sites of amino acid residues HIS-131 and ARG-139 of the Septin protein were involved in the interaction between the RGD-DA and the Septin protein. The results are shown in Fig. 123.

### Example 11-16 Binding site of fatty acid peptide complex to CD36 protein

In this example, RGD-FA was synthesized according to the method of example 9 in part I. The 3D structure of the active ingredient was obtained through Pubmed database and Chemdraw software. CD36 protein was downloaded through PDB database. Relevant conventional treatment and molecular docking were performed on the target and the active ingredient in combination with PyMOL 2.4.0 software and Auto Dock Tools 1.5.7 software It was found that the sites of amino acid residues LYS-218 and LYS-39 of CD36 protein were involved in the interaction between RGD-FA and CD36 protein. The results are shown in Fig. 124.

### Example 11-17 Binding site of fatty acid peptide complex to TfR1(transferrin receptor 1)

In this example, RGD-C8 was synthesized according to the method of example 9 in part I. The 3D structure of active ingredient was obtained through Pubmed database and Chemdraw software. The TfR1 protein was downloaded through PDB database. Relevant conventional treatment and molecular docking were performed on target and active ingredient in combination with PyMOL 2.4.0 software and Auto Dock Tools 1.5.7 software. It was found that amino acid residues LYS-358, LYS-261, ASP-356 and LYS-240 of TfR1 protein were involved in the interaction between RGD-C8 and TfR1 protein. The results are shown in Fig. 125.

### Example 11-18 Binding site of fatty acid-amino acid complex to Rab protein

Thr-BA in this example was synthesized according to the method of Example 29 in part I. The 3D structure of the active ingredient was obtained through Pubmed database and Chemdraw software. The TLR1 protein was downloaded through PDB database. Relevant conventional treatment and molecular docking were performed on the target and the active ingredient in combination with PyMOL 2.4.0 software and Auto Dock Tools 1.5.7 software. It was found that sites of amino acid residues ASN-253, LYS-222 and GLU-255 of TLR1 protein were involved in the interaction between Thr-BA and TLR1 protein. The results are shown in Fig. 126.

### Example 11-19 Binding site of fatty acid-vitamin complex to TLR receptor

The Vitamin-C-OH in this example was synthesized according to the method of example 17 in part I. The 3D structure of the active ingredient was obtained through Pubmed database and Chemdraw software. The TLR7 protein was downloaded through PDB database. Relevant conventional treatment and molecular docking were performed on the target and the active ingredient in combination with PyMOL 2.4.0 software and Auto Dock Tools 1.5.7 software. It was found that the site of amino acid residue GLY-584 of TLR7 protein was involved in the interaction between Vitamin-C-OH and TLR7 protein. The results are shown in Fig. 127.

### Example II-20 Binding site of fatty acid amino acid complex to G protein coupled receptor

In this example, THr-C24 was synthesized according to the method of Example 29 in part I. The 3D structure of active ingredient was obtained through Pubmed database and Chemdraw software. The Rhodopsin-like GPCR protein was downloaded through PDB database. In combination with PyMOL 2.4.0 software and Auto Dock Tools 1.5.7 software, relevant conventional treatment and molecular docking were performed on target and active ingredient. It was found that the sites of amino acid residues ASN-779 and LYS-840 of Rhodopsin-like GPCR protein were involved in the interaction between THr-C24 and Rhodopsin-like GPCR protein. The results are shown in Fig. 128.

### Example 11-21 Binding site of fatty acid polysaccharide complex to Scavenger receptor

In this example, chondroitin sulfate-2-C8 was synthesized according to the method of Examples 7 and 8 in part I. The 3D structure of the active ingredient was obtained through Pubmed database and Chemdraw software. The Scavenger protein was downloaded through PDB database. Relevant conventional treatment and molecular docking were performed on the target and the active ingredient in combination with PyMOL 2.4.0 software and Auto Dock Tools 1.5.7 software It was found that sites of amino acid residues LEU-395 and PRO-401 of Scavenger protein were involved in the interaction between Chondroitin sulfate -2-C8 and Scavenger protein. The results are shown in Fig. 129.

### Example II-22 Binding site of fatty acid-polysaccharide complex to Scavenger receptor

The Heparin-SA in this example was synthesized according to the method of Examples 7 and 8 in part I. The 3D structure of the active ingredient was obtained through the Pubmed database and Chemdraw software. The VLDLR protein was downloaded through the PDB database. Relevant conventional treatment and molecular docking are performed on the target and the active ingredient in combination with the PyMOL 2.4.0 software and Auto Dock Tools 1.5.7 software. It is found that the site of amino acid residue LYS-21 of the VLDLR protein was involved in the interaction between the Heparin-SA and the VLDLR protein. The results are shown in Fig. 130.

### Example II-23 Binding site of fatty acid-nucleotide complex to chloride channel receptor

The UDPGA-BA in this example was synthesized according to the method of Example 16 in part I. The 3D structure of the active ingredient was obtained through Pubmed database and Chemdraw software. The GABA-A protein was downloaded through PDB database. Relevant conventional treatment and molecular docking are performed on the target and active ingredient in combination with PyMOL 2.4.0 software and Auto Dock Tools 1.5.7 software. It was found that sites of amino acid residues PHE-310, ARG-249, SER-388, ARG-313 and LYS-312 of GABA-A protein were involved in the interaction between UDPGA-BA and GABA-A protein. The results are shown in Fig. 131.

### Example II-24 Binding site of fatty acid-nucleotide complex to Endoglin receptor

UDPGA-C12 in this example was synthesized according to the method of Example 16 in part I. The 3D structure of the active ingredient was obtained through Pubmed database and Chemdraw software. CD105 protein was downloaded through PDB database. Relevant conventional treatment and molecular docking were performed on the target and the active ingredient in combination with PyMOL 2.4.0 software and Auto Dock Tools 1.5.7 software. It was found that sites of amino acid residues LYS-89 and LYS-451 of CD105 protein were involved in the interaction between UDPGA-C12 and CD105 protein. The results are shown in Fig. 132.

### Example II-25 Binding site of fatty acid-amino acid complex to CD44 receptor

The Thr-n-CA in this example was synthesized synthesized according to the method of Example 29 in the first par. The 3D structure of the active ingredient was obtained the Pubmed database and Chemdraw software. The CD44 protein was downloaded through the PDB database. Relevant conventional treatment and molecular docking were performed on the target and the active ingredient in combination with PyMOL 2.4.0 software and Auto Dock Tools 1.5.7 software. It was found that the sites of amino acid residues ASN-120 and PHE-56 of the CD44 protein were involved in the interaction between the Thr-n-CA and the CD44 protein. The results are shown in Fig. 133.

Binding of drugs to extracellular emetic proteins

### Example II-26 Binding sits of fatty acid-nucleotide complex to histone protease

In this example, UDPGA-C25 was synthesized according to the method of Example 16 in part I. The 3D structure of active ingredient was obtained through Pubmed database and Chemdraw software. The Membrane-type MMPs protein was downloaded through PDB database. Relevant conventional treatment and molecular docking were performed on target and active ingredient in combination with PyMOL 2.4.0 software and Auto Dock Tools 1.5.7 software. It was found that the sites of the amino acid residues PRO-1067, SER-1075, GLY-116 and GLN-115 of Membrane-type MMPs protein were involved in the interaction between UDPGA-C25 and Membrane-type MMPs protein. The results are shown in Fig. 134.

### Example II-27 Binding Site of Fatty Acid-Nucleotide Complex to TNF (Tumor Necrosis Factor)

The UDPGA-PA in this example was synthesized according to the method of Example 16 in part I. The 3D structure of the active ingredient was obtained through Pubmed database and Chemdraw software. The TNF protein was downloaded through PDB database. Relevant conventional treatment and molecular docking were performed on the target and the active ingredient in combination with PyMOL 2.4.0 software and Auto Dock Tools 1.5.7 software. It was found that the sites of amino acid residues TYR-98, LYS-61, GLN-70 and SER-67 of the TNF protein were involved in the interaction between UDPGA-PA and TNF protein. The results are shown in Fig. 135.

### Example II-28 Binding site of fatty acid-nucleotide complex to VEGF (angiogenic factor)

The UDPGA-OA in this example was synthesized according to the method of Example 16 in part I. The 3D structure of the active ingredient was obtained through Pubmed database and Chemdraw software. The VEGF protein was downloaded through PDB database. Relevant conventional treatment and molecular docking were performed on the target and the active ingredient in combination with PyMOL 2.4.0 software and Auto Dock Tools 1.5.7 software. It was found that the sites of the amino acid residues CYS-103, GLU-102, THR-30, LYS-73, THR-93, GLY-91, GLN-27, MET-100C, TYR-36, TYR-91, GLN-38, GLN-39, and GLY-42 of the VEGF protein were involved in the interaction between UDPGA-OA and VEGF protein. The results are shown in Fig. 136.

### Example II-29 Binding site of fatty acid-amino acid complex to glutathione S-transferase (GST)

The Thr-C36 in this example was synthesized according to the method of Example 29 in part I. The 3D structure of the active ingredient was obtained through Pubmed database and Chemdraw software. The GST protein was downloaded through PDB database. Relevant conventional treatment and molecular docking were performed on the target and the active ingredient in combination with PyMOL 2.4.0 software and Auto Dock Tools 1.5.7 software. It is found that the sites of amino acid residues LYS-354 and ASN-258 of the GSTs protein were involved in the interaction between the Thr-C36 and the GSTs protein. The results are shown in Fig. 137.

### Example II-30 Binding site of fatty acid-polysaccharide complex to protease Inhibitor

HA-OH-1 in this example was synthesized according to the method of examples 7-8 in part I. The 3D structure of the active ingredient was obtained through the Pubmed database and Chemdraw software. The TIMPs protein was downloaded through the PDB database. Relevant conventional treatment and molecular docking were performed on the target and the active ingredient in combination with PyMOL 2.4.0 software and Auto Dock Tools 1.5.7 software. It was found that sites of amino acid residues ARG-42, LYS-95, GLN-44 and LYS-89 of the TIMPs protein were involved in the interaction between HA-OH-1 and the TIMPs protein. The results are shown in Fig. 138.

### Example 11-31 Binding site of fatty acid-polysaccharide complex to protease inhibitor

The HA-SA in this example was synthesized according to the method of example 7-8 in part I. The 3D structure of the active ingredient was obtained through Pubmed database and Chemdraw software. The α2-macroglobulin protein was downloaded through PDB database. Relevant conventional treatment and molecular docking are performed on the target and active ingredient in combination with PyMOL 2.4.0 software and Auto Dock Tools 1.5.7 software. It was found that sites of amino acid residues ARG-151, GLN-154, GLN-156, ARG-151, LYS-181 and ARG-191 of α2-macroglobulin protein were involved in the interaction between HA-SA and α2-macroglobulin protein. The results are shown in Fig. 139.

### Example II-32 Binding site of fatty acid-vitamin complex to tyrosine kinase receptor (TKR)

In this example, vitamin-C-C30 was synthesized according to the method of Example 17 in part I. The 3D structure of active ingredients was obtained through Pubmed database and Chemdraw software. insulin receptor and IR proteins were downloaded through PDB database. Relevant conventional processing and molecular docking of target and active ingredients were performed in combination with PyMOL 2.4.0 software and Auto Dock Tools 1.5.7 software. It was found that the sites of the amino acid residues GLU-1040 and ARG-1039 of insulin receptor and IR proteins were involved in the interaction between Vitamin-C-C30 and insulin receptor and IR protein. The results are shown in Fig. 140.

### Example II-33 Binding sites of fat-soluble vitamin to Toll-like receptor (TLR)

In this example, vitamin E was selected as the small molecule ligand. The 3D structure of the active ingredient was obtained through Pubmed database and Chemdraw software. The TLR protein was downloaded through PDB database. Relevant conventional treatment and molecular docking of the target and active ingredient were performed in combination with PyMOL 2.4.0 software and Auto Dock Tools 1.5.7 software. It was found that the site of amino acid residue LEU-726 of TLRs protein was involved in the interaction between Vitamin-E and TLR protein. The results are shown in Fig. 141.

### Example II-34 Binding site of fatty acid-nucleotide complex to Integrin

In this example, Adenosine-C22-1 was synthesized according to the method of Example 16 in part I. The 3D structure of active ingredient was obtained through Pubmed database and Chemdraw software. The Integrins protein was downloaded through PDB database. Relevant conventional treatment and molecular docking were performed on the target and the active ingredient in combination with PyMOL 2.4.0 software and Auto Dock Tools 1.5.7 software It was found that the site of the amino acid residue ASN-915 of Integrins protein was involved in the interaction between Adenosine-C22-1 and Integrins protein. The results are shown in Fig. 142.

### Example II-35 Binding site of fatty acid-bile acid complex to Fibrillin

In this example, cholylglycine-C18-1-OH was synthesized according to the method of example 34 in part I. The 3D structure of active ingredient is obtained through Pubmed database and Chemdraw software. The Fibrillin protein was downloaded through PDB database. Relevant conventional treatment and molecular docking are performed on target and active ingredient in combination with PyMOL 2.4.0 software and Auto Dock Tools 1.5.7 software. It was found that sites of amino acid residues ASN-1504 and LYS-1586 of Fibrillin protein were involved in the interaction between cholylglycine-C18-1-OH and Fibrillin protein. The results are shown in Fig. 143.

### Example II-36 Binding site of fatty acid-nucleotide complex to Collagen

The UDPGA-C12 in this example was synthesized according to the method of Example 16 in part I. The 3D structure of the active ingredient was obtained through Pubmed database and Chemdraw software. The Collagen protein was downloaded through PDB database. Relevant conventional treatment and molecular docking were performed on the target and the active ingredient in combination with PyMOL 2.4.0 software and Auto Dock Tools 1.5.7 software. It was found that the sites of amino acid residues LYS-546 and GLN-543 of the Collagen protein were involved in the interaction between the UDPGA-C12 and the Collagen protein. The results are shown in Fig. 144.

### Example II-37: Inhibition of nano-drug phagocytosis by Ser-EPA

Hela cells (cell bank, Chinese Academy of Sciences) were seeded in a 6-well plate at a density of 1.0 * 10⁵ and cultured for 24 h. The cells were treated with 5 nM Ser-EPA for 1 h and washed to remove Ser-EPA. Adriamycin-loaded ferroferric oxide nanoparticles were added followed by incubation for 4 h. Then, the cells were washed with PBS, stained with DAPI, and observed with confocal microscope. The results showed that Ser-EPA-treated cells significantly inhibited the phagocytosis of nanoparticles of iron tetraoxide. The experimental results are shown in Fig. 145.

### Example II-38: Inhibition of insulin release by water-soluble fatty acid complex

Human islet β cells (Wuhan Procell Life Technology Co. Ltd.) were seeded in a 10 cm dish. When the cells reached 80% density, the experimental group was incubated with medium containing 2, 5, 10 and 50 nM Ser-n-butyric acid, Thr-n-hexanoic acid, Lys-n-decanoic acid, adenosine monophosphate-lauroic acid, glucose-pentadecanedioic acid, Ser-stearic acid, Thr-arachidic acid, Lys-eicosanedioic acid, and glucose-docosanedioic acid for 6 h. The supernatant samples of the cells were centrifuged and the supernatant was taken (500 g, 5 min). Insulin levels in cell supernatants were detected using a mouse insulin enzyme-linked immunosorbent assay kit. The results showed that under the conditions of 2, 5, 10, and 50 nM Ser-n-butyric acid, Thr-n-hexanoic acid, Lys-n-decanoic acid, adenosine monophosphate-lauroic acid, glucose-pentadecanedioic acid, Ser-stearic acid, Thr-arachidic acid, Lys-eicosanedioic acid, and glucose-docosanedioic acid, the level of insulin secreted by human pancreatic β-cells was significantly suppressed. The experimental results are shown in Table II-1.

**Table II-1B Water-soluble fatty acid complex inhibit insulin secretion from human pancreatic β-cells**

| Compound | Insulin (ng/ml) | | | | |
|---|---|---|---|---|---|
| | 0 nM | 2 nM | 5 nM | 10 nM | 50 nM |
| Ser-n-butyric acid | 0.83 | 0.41 | 0.39 | 0.49 | 0.53 |
| Thr-n-hexanoic acid | 0.84 | 0.38 | 0.37 | 0.47 | 0.54 |
| Lys-n-decanoic acid | 0.90 | 0.45 | 0.47 | 0.56 | 0.60 |
| Adenosine monophosphate-lauroic acid | 0.84 | 0.37 | 0.38 | 0.43 | 0.49 |
| Glucose-pentadecanedioic acid | 0.85 | 0.22 | 0.23 | 0.36 | 0.43 |
| Ser-stearic acid | 0.84 | 0.28 | 0.30 | 0.41 | 0.48 |
| Thr-arachidic acid | 0.83 | 0.32 | 0.31 | 0.43 | 0.51 |
| Lys-eicosanedioic acid | 0.84 | 0.34 | 0.35 | 0.42 | 0.48 |
| Glucose-docosanedioic acid | 0.86 | 0.25 | 0.27 | 0.41 | 0.50 |

### Example II-39: Promotion of Insulin Release by Water-Soluble Fatty Acid Complex

Human islet β cells (Wuhan Procell Life Technology Co. Ltd.) were seeded in a 10 cm dish. When the cells reached 80% density, the experimental group was incubated with medium containing 50 µM and 100 µM Ser-n-butyric acid, Thr-n-hexanoic acid, Lys-n-decanoic acid, adenosine monophosphate-lauroic acid, glucose-pentadecanedioic acid, Ser-stearic acid, Thr-arachidic acid, Lys-eicosanedioic acid, and glucose-docosanedioic acid for 6 h. The supernatant samples of the cells were centrifuged and the supernatant was taken (500 g, 5 min). Insulin levels in cell supernatants were detected using a mouse insulin enzyme-linked immunosorbent assay kit. The results showed that under the conditions of 50 µM and 100 µM Ser-n-butyric acid, Thr-n-hexanoic acid, Lys-n-decanoic acid, adenosine monophosphate-lauroic acid, glucose-pentadecanedioic acid, Ser-stearic acid, Thr-arachidic acid, Lys-eicosanedioic acid, and glucose-docosanedioic acid, the level of insulin secreted by human pancreatic β-cells was significantly promoted. The experimental results are shown in Table II-2.

**Table II-2 Water-soluble fatty acid complexes promote insulin secretion from human islet beta cells**

| Cpd. | Insulin (ng/ml) | | |
|---|---|---|---|
| | 0µM | 50µM | 100µM |
| Ser-n-butyric acid | 0.83 | 1.42 | 1.51 |
| Thr-n-hexanoic acid | 0.84 | 1.41 | 1.46 |
| Lys-n-decanoic acid | 0.90 | 1.53 | 1.63 |
| Adenosine monophosphate-lauroic acid | 0.84 | 1.49 | 1.53 |
| Glucose-pentadecanedioic acid | 0.85 | 1.48 | 1.52 |
| Ser-stearic acid | 0.84 | 1.38 | 1.41 |
| Thr-arachidic acid | 0.83 | 1.46 | 1.42 |
| Lys-eicosanedioic acid | 0.84 | 1.44 | 1.48 |
| Glucose-docosanedioic acid | 0.86 | 1.41 | 1.46 |

### Example II-40: inhibition of cell membrane fluidity by water-soluble fatty acid complex

Hela cells (cell bank, Chinese Academy of Sciences) were seeded in a quadripartite confocal dish at a lower density. After the cells grew to 50% full, 3 µl of Laurdan fluorochrome solution (10 µM) was added to each well and incubated for 5 h. The old medium was removed. The cells were washed twice with PBS and incubated for 1 h with 2, 5, 10, 50 nM n-octanoic acid, octanedioic acid, Lys-stearic acid, Vc-oleic acid, adenosine monophosphate-linoleic acid, glucose-linolenic acid, Ser-EPA, Thr-DHA, Lys-docosanoic acid, Vc-erucic acid, respectively. Prior to detection of the fluorescent signal, the old medium was removed. The cells were washed twice with PBS and new medium was added. Cell membrane fluorescence intensity was recorded every 20 s, and cell membrane fluorescence recovery rate was calculated. The results showed that 2, 5, 10, 50 nM of Ser-n-octanoic acid, Thr-octanedioic acid, Lys-stearic acid, Vc-oleic acid, adenosine monophosphate-linoleic acid, glucose-linolenic acid, Ser-EPA, Thr-DHA, Lys-docosanoic acid, and Vc-erucic acid inhibited cell membrane fluidity. The results are shown in Fig. 146 and Tables II-3 to II-12.

**Table II-3 Inhibition of cell membrane fluidity by n-octanoic acid**

| Compound | Fluorescence recovery rate % | | | |
|---|---|---|---|---|
| | 20 s | 40 s | 60 s | 80 s |
| 0 nM | 65.20 | 67.58 | 69.29 | 72.68 |
| 2 nM | 65.57 | 66.78 | 66.54 | 67.22 |
| 5 nM | 66.75 | 67.05 | 67.59 | 66.98 |
| 10 nM | 64.02 | 65.23 | 65.45 | 66.51 |
| 50 nM | 67.32 | 67.94 | 68.02 | 68.65 |

**Table II-4 Inhibition of cell membrane fluidity by octanedioic acid**

| Compound | Fluorescence recovery rate % | | | |
|---|---|---|---|---|
| | 20 s | 40 s | 60 s | 80 s |
| 0 nM | 64.88 | 66.75 | 68.29 | 71.89 |
| 2 nM | 63.58 | 64.25 | 65.77 | 65.12 |
| 5 nM | 64.56 | 64.88 | 64.78 | 65.09 |
| 10 nM | 63.99 | 64.23 | 64.45 | 64.66 |
| 50 nM | 66.09 | 66.42 | 66.89 | 67.94 |

**Table II-5 Inhibition of cell membrane fluidity by Lys-stearic acid**

| Compound | Fluorescence recovery rate % | | | |
|---|---|---|---|---|
| | 20 s | 40 s | 60 s | 80 s |
| 0 nM | 64.64 | 67.77 | 69.37 | 73.44 |
| 2 nM | 64.38 | 65.33 | 66.78 | 65.32 |
| 5 nM | 63.98 | 64.03 | 64.52 | 64.77 |
| 10 nM | 64.41 | 64.81 | 65.40 | 65.86 |
| 50 nM | 65.08 | 65.47 | 65.55 | 66.26 |

**Table II-6 Inhibition of cell membrane fluidity by Vc-oleic acid**

| Compound | **Fluorescence recovery rate %** | | | |
|---|---|---|---|---|
| | **20 s** | **40 s** | **60 s** | **80 s** |
| **0 nM** | **61.84** | **66.75** | **68.87** | **71.44** |
| **2 nM** | **61.48** | **62.33** | **63.41** | **64.57** |
| **5 nM** | **61.87** | **62.99** | **63.47** | **63.21** |
| **10 nM** | **63.72** | **63.75** | **64.67** | **65.92** |
| **50 nM** | **62.43** | **62.62** | **63.37** | **64.24** |

**Table II-7 Inhibition of cell membrane fluidity by adenosine monophosphate-linoleic acid**

| Compound | **Fluorescence recovery rate %** | | | |
|---|---|---|---|---|
| | **20 s** | **40 s** | **60 s** | **80 s** |
| **0 nM** | **63.45** | **68.84** | **70.75** | **72.88** |
| **2 nM** | **62.11** | **62.98** | **64.26** | **68.17** |
| **5 nM** | **63.44** | **64.22** | **65.78** | **66.39** |
| **10 nM** | **63.22** | **63.48** | **64.42** | **65.93** |
| **50 nM** | **64.25** | **64.66** | **65.33** | **66.23** |

**Table II-8 Inhibition of cell membrane fluidity by Glucose-linolenic acid**

| Compound | **Fluorescence recovery rate %** | | | |
|---|---|---|---|---|
| | **20 s** | **40 s** | **60 s** | **80 s** |
| **0 nM** | **64.88** | **66.97** | **70.26** | **71.84** |
| **2 nM** | **63.78** | **64.18** | **65.22** | **65.16** |
| **5 nM** | **64.35** | **64.83** | **65.17** | **66.59** |
| **10 nM** | **64.76** | **64.87** | **65.11** | **66.83** |
| **50 nM** | **63.24** | **64.10** | **64.54** | **65.23** |

**Table II-9 Inhibition of cell membrane fluidity by Ser-EPA**

| Compound | **Fluorescence recovery rate %** | | | |
|---|---|---|---|---|
| | **20 s** | **40 s** | **60 s** | **80 s** |
| **0 nM** | **64.98** | **68.68** | **69.34** | **71.83** |
| **2 nM** | **63.44** | **65.36** | **65.21** | **66.35** |
| **5 nM** | **64.73** | **64.59** | **65.66** | **67.97** |
| **10 nM** | **64.66** | **65.11** | **65.24** | **66.83** |
| **50 nM** | **62.84** | **62.94** | **63.04** | **64.54** |

**Table II-10 Inhibition of cell membrane fluidity by Thr-DHA**

| Compound | **Fluorescence recovery rate %** | | | |
|---|---|---|---|---|
| | **20 s** | **40 s** | **60 s** | **80 s** |
| **0 nM** | **67.38** | **68.76** | **70.55** | **73.18** |
| **2 nM** | **65.48** | **66.31** | **66.25** | **66.78** |
| **5 nM** | **65.47** | **65.23** | **65.38** | **66.45** |
| **10 nM** | **65.89** | **66.04** | **66.59** | **66.71** |
| **50 nM** | **67.42** | **67.43** | **67.57** | **68.06** |

**Table II-11 Inhibition of cell membrane fluidity by Lys-docosanoic acid**

| Compound | **Fluorescence recovery rate %** | | | |
|---|---|---|---|---|
| | **20 s** | **40 s** | **60 s** | **80 s** |
| **0 nM** | **64.78** | **67.09** | **69.94** | **71.58** |
| **2 nM** | **62.49** | **63.06** | **63.55** | **63.01** |
| **5 nM** | **63.42** | **64.64** | **64.84** | **65.35** |
| **10 nM** | **63.43** | **63.55** | **64.06** | **65.64** |
| **50 nM** | **65.94** | **66.04** | **66.43** | **67.21** |

**Table II-12 Inhibition of cell membrane fluidity by Vc-Erucic Acid**

| Compound | **Fluorescence recovery rate %** | | | |
|---|---|---|---|---|
| | **20 s** | **40 s** | **60 s** | **80 s** |
| **0 nM** | **63.99** | **68.87** | **70.56** | **72.77** |
| **2 nM** | **63.25** | **64.28** | **64.77** | **65.03** |
| **5 nM** | **64.37** | **64.66** | **65.54** | **65.04** |
| **10 nM** | **64.51** | **64.77** | **65.43** | **65.94** |
| **50 nM** | **65.58** | **65.71** | **66.04** | **67.43** |

### Example II-41: promotion of water-soluble fatty acid complex cell membrane fluidity

Hela cells (cell bank, Chinese Academy of Sciences) were seeded in a quadripartite confocal dish at a lower density. After the cells grew to 50% full, 3 µl of Laurdan fluorochrome solution (10 µM) was added to each well and incubated for 5 h. The old medium was removed. The cells were washed twice with PBS and incubated for 1 h with 50 µM and 100 µM n-octanoic acid, octanedioic acid, Lys-stearic acid, Vc-oleic acid, adenosine monophosphate-linoleic acid, glucose-linolenic acid, Ser-EPA, Thr-DHA, Lys-docosanoic acid, Vc-erucic acid, respectively. Prior to detection of the fluorescent signal, the old medium was removed. The cells were washed twice with PBS and new medium was added. Cell membrane fluorescence intensity was recorded every 20 s, and cell membrane fluorescence recovery rate was calculated. The results showed that 50 µM, 100 µM Ser-n-octanoic acid, Thr-octanedioic acid, Lys-stearic acid, Vc-oleic acid, adenosine monophosphate-linoleic acid, glucose-linolenic acid, Ser-EPA, Thr-DHA, Lys-docosanoic acid, and Vc-erucic acid promoted cell membrane fluidity. The results are shown in Fig. 147 and Tables II-13 to Table II-22.

**Table II-13 Promotion of cell membrane fluidity by n-octanoic acid**

| Compound | Fluorescence recovery rate % | | | |
|---|---|---|---|---|
| | 20 s | 40 s | 60 s | 80 s |
| 0 µM | 65.20 | 67.58 | 69.29 | 72.68 |
| 50 µM | 64.31 | 69.09 | 73.41 | 78.32 |
| 100 µM | 66.15 | 71.58 | 75.11 | 79.84 |

**Table II-14 Promotion of cell membrane fluidity by octanedioic acid**

| Compound | Fluorescence recovery rate % | | | |
|---|---|---|---|---|
| | 20 s | 40 s | 60 s | 80 s |
| 0 µM | 64.88 | 66.75 | 68.29 | 71.89 |
| 50 µM | 64.41 | 70.05 | 74.65 | 80.36 |
| 100 µM | 65.77 | 71.36 | 76.81 | 79.79 |

**Table II-15 Promotion of cell membrane fluidity by lys-stearic acid**

| Compound | Fluorescence recovery rate % | | | |
|---|---|---|---|---|
| | 20 s | 40 s | 60 s | 80 s |
| 0 µM | 64.64 | 67.77 | 69.37 | 73.44 |
| 50 µM | 65.84 | 71.51 | 75.97 | 80.33 |
| 100 µM | 64.77 | 70.54 | 73.46 | 78.44 |

**Table II-16 Promotion of cell membrane fluidity by Vc-oleic acid**

| Compound | Fluorescence recovery rate % | | | |
|---|---|---|---|---|
| | 20 s | 40 s | 60 s | 80 s |
| 0 µM | 61.84 | 66.75 | 68.87 | 71.44 |
| 50 µM | 62.54 | 67.11 | 74.55 | 76.57 |
| 100 µM | 61.66 | 73.65 | 77.32 | 78.61 |

**Table II-17 Promotion of cell membrane fluidity by adenosine monophosphate-linoleic acid**

| Compound | Fluorescence recovery rate % | | | |
|---|---|---|---|---|
| | 20 s | 40 s | 60 s | 80 s |
| 0 µM | 63.45 | 68.84 | 70.75 | 72.88 |
| 50 µM | 64.44 | 70.73 | 74.64 | 80.57 |
| 100 µM | 63.22 | 67.48 | 75.42 | 79.93 |

**Table II-18 Promotion of cell membrane fluidity by glucose-linolenic acid**

| Compound | Fluorescence recovery rate % | | | |
|---|---|---|---|---|
| | 20 s | 40 s | 60 s | 80 s |
| 0 µM | 64.88 | 66.97 | 70.26 | 71.84 |
| 50 µM | 63.11 | 70.49 | 74.88 | 79.24 |
| 100 µM | 65.93 | 69.87 | 75.03 | 80.83 |

**Table II-19 Promotion of cell membrane fluidity by Ser-EPA**

| Compound | Fluorescence recovery rate % | | | |
|---|---|---|---|---|
| | 20 s | 40 s | 60 s | 80 s |
| 0 µM | 64.98 | 68.68 | 69.34 | 71.83 |
| 50 µM | 64.33 | 72.49 | 73.77 | 81.40 |
| 100 µM | 65.72 | 72.99 | 74.34 | 80.39 |

**Table II-20 Promotion of cell membrane fluidity by Thr-DHA**

| Compound | Fluorescence recovery rate % | | | |
|---|---|---|---|---|
| | 20 s | 40 s | 60 s | 80 s |
| 0 µM | 67.38 | 68.76 | 70.55 | 73.18 |
| 50 µM | 66.54 | 73.45 | 77.91 | 84.66 |
| 100 µM | 66.60 | 74.67 | 78.31 | 86.44 |

**Table II-21 promotion of cell membrane fluidity by Lys-docosanoic acid**

| Compound | Fluorescence recovery rate % | | | |
|---|---|---|---|---|
| | 20 s | 40 s | 60 s | 80 s |
| 0 µM | 64.78 | 67.09 | 69.94 | 71.58 |
| 50 µM | 64.53 | 67.44 | 71.54 | 76.43 |
| 100 µM | 63.47 | 71.89 | 73.91 | 78.44 |

**Table II-22 Promotion of cell membrane fluidity by Vc-erucic acid**

| Compound | Fluorescence recovery rate % | | | |
|---|---|---|---|---|
| | 20 s | 40 s | 60 s | 80 s |
| 0 µM | 63.99 | 68.87 | 70.56 | 72.77 |
| 50 µM | 64.53 | 70.66 | 75.73 | 79.32 |
| 100 µM | 65.41 | 69.71 | 74.35 | 78.26 |

### Example II-42: Promotion of Human Hair Follicle Stem Cell Proliferation by Lys-ALA, Ser-DHA, and Glu-Oleic Acid

Human hair follicle stem cells (Wuhan Procell Life Technology Co. Ltd.) were cultured in a 96-well plate at the density of 1.0 * 10⁴. After the cells were cultured overnight and returned to normal state, the experimental groups were incubated with 20 µM, 30 µM, 50 µM Lys-ALA, Ser-DHA, and Glu-oleic acid for 6 h. Then the proliferation effect of human hair follicle stem cells was detected by EdU cell proliferation assay kit (TMB method). The results showed that 20 µM, 30 µM, 50 µM Lys-ALA, Ser-DHA, and Glu-oleic acid could effectively promote the proliferation of human hair follicle stem cells. The experimental results are shown in Fig. 148.

### Example II-43: Inhibition of cell migration by Thr-EPA

Hela cells in logarithmic growth phase (cell bank of Chinese Academy of Sciences) were trypsinized into a single cell suspension and inoculated into a 6-well culture plate. The cells were plated at 6 w/well, and the principle of inoculation was that the fusion rate reached 100% after overnight, and the total amount of the final culture medium for each well was 2 mL. The cells were cultured at 37°C in 5% CO₂ incubator for 24 h. The next day 5 nM Thr-EPA was added by scratching with a 200-microliter gun tip against the horizontal line on the vertical back of the lid of the 6-well plate. 24 h later, samples were taken and photographed. The results showed that 5 nM Thr-EPA inhibited cell migration. The experimental results are shown in Fig. 149.

### Example II-44: Promotion of cell migration by Thr-EPA

Hela cells in logarithmic growth phase (cell bank of Chinese Academy of Sciences) were trypsinized into a single cell suspension and inoculated into a 6-well culture plate. The cells were plated at 6 w/well, and the principle of inoculation was that the fusion rate reached 100% after overnight, and the total amount of the final culture medium for each well was 2 mL. The cells were cultured at 37°C in 5% CO₂ incubator for 24 h. The next day 50 **µM** Thr-EPA was added by scratching with a 200-microliter gun tip against the horizontal line on the vertical back of the lid of the 6-well plate. 24 h later, samples were taken and photographed. The results showed that 50 µM Thr-EPA promoted cell migration. The experimental results are shown in Fig. 150.

### Example II-45: Inhibition of water-soluble fatty acid complex on cell infection by SARS-CoV-2 pseudovirus (B.1.526.2)

The antiviral activity was tested with serine-LA, threonine-LA, lysine-LA, serine-n-octanoic acid, adenosine-erucic acid, **UDPGA**-pentacosanoic acid (UDPGA-C25), UDPGA-OA, UDPGA-dodecanoic acid (UDPGA-C12), glycocholic acid-hydroxyoleic acid, and cholyltaurine-tetradecanoic acid at the concentrations of 100 µM, 50 µM, 10 µM, 1 µM, 100 nM, 50 nM, 10 nM, 5 nM, and 2 nM.

260 µL of autoclaved water was added to 36 wells around a 96-well plate to seal the edge, so as to reduce the error caused by evaporation of medium in edge wells. Column 2 (cell control CC) was added with DMEM complete medium **150** µL/well. Column 3 (virus control ascorbic acid) was added with DMEM complete medium 100 µL/well, as well as water-soluble fatty acid complex. Using DMEM complete medium to dilute the novel crown virus pseudovirus [B. 1.526.2, purchased from Beijing Tiantan Water-Soluble Fatty Acid **Complex** Biotechnology Development Co. No: 80062. The pseudovirus system carries a firefly luciferase reporter gene. After infecting cells with the pseudovirus, the luminescence value can be detected by fluorescent substrates to determine the amount of virus infecting the cells. The pseudovirus uses VSV as a backbone, and the surface of the pseudovirus is embedded with the Spike protein of novel coronavirus, which can simulate the binding process of the true virus to the receptor and then enter the cell. After the sample with neutralizing activity reacts with the pseudovirus, the ability to infect the cell will be lost. By detecting the luminescence value of the control well and the sample well, whether the sample has neutralizing activity or not can be determined. The pseudovirus can not replicate autonomously, and has only one round of infection ability, low in biosafety level and easy to operate, which is a common means for vaccine evaluation at present]

The cells were treated with water-soluble fatty acid complex for 4 h. The culture medium was removed, and the cell concentration was diluted to 2 × 10⁵ cells/mL. 100 µL of cells was added per well, making 2 × 10⁴ cells per well. The pseudovirus was diluted to 1.3-2.3 × 10⁴ TCID50/mL. 50 µL of cells was added per well in columns 3-11. The plate was put into a 37°C, 5% CO2 cell incubator for culture for 24h. After the completion of incubation, 250 µL of the supernatant was pipetted away. 100 µL of luciferase detection reagent was added for reaction at room temperature in the dark for 2 min. After repeatedly blowing and beating, 100 µL of liquid was transferred to a white plate. The luminescence values (RLU) were read using a multi-function imaging microplate reader. The following inhibition rate was calculated by the equation E1: Inhibition rate=(1-(Average luminous intensity-Average black control CC)/(Average VC-Average CC))*100% inhibition results are summarized in Table II-23.

**Table II-23 Inhibition of water-soluble fatty acid complex against novel crown virus pseudovirus infected cells (n = 3)**

| Concentra tion | Inhibition rate (%) | | | | | | | | | |
|---|---|---|---|---|---|---|---|---|---|---|
| | Serin e-LA | Threoni ne-LA | Lysin e-LA | Serin e-n-octan oic acid | Adenosi neerucic acid | UDPG A-C25 | UDPG A-OA | UDPG A-C12 | cholylglyc inehydroxyol eic acid | cholyltaur inetetradecan oic acid |
| 100 µM | 93.6 8 ± 0.73 | 87.98± 0.55 | 83.77 ± 0.58 | 73.55 ± 0.44 | 90.08± 1.21 | 92.33± 0.73 | 83.61± 0.88 | 53.98± 0.78 | 73.68± 0.71 | 58.98± 0.44 |
| 50 µM | 51.3 6 ± 0.87 | 71.36± 0.48 | 80.36 ± 0.64 | 71.36 ± 0.28 | 81.44± 0.54 | 71.36± 0.88 | 73.06± 0.26 | 67.37± 0.54 | 71.09± 0.58 | 67.55± 0.25 |
| 10 µM | 54.2 6 ± 0.53 | - 124.16 ± 0.47 | - 144.2 6± 2.36 | - 44.26 ± 0.03 | -44.26± 1.26 | - 64.26± 0.59 | - 54.00± 0.23 | - 73.16± 0.59 | -54.26± 0.88 | 4.26± 0.95 |
| 1 µM | 102. 33 ± 4.36 | -82.33± 0.87 | - 64.93 ± 1.58 | - 97.39 ± 7.23 | -22.83± 0.93 | - 12.03± 0.83 | - 82.33± 0.53 | - 92.93± 0.58 | -32.33± 0.57 | -94.23± 5.55 |
| 100 nM | 73.6 6 ± 1.33 | 75.99± 2.66 | 86.44 ± 1.58 | 63.22 ± 2.55 | 64.66± 8.23 | 88.64± 4.53 | 84.96± 4.55 | 87.47± 8.23 | 88.76± 4.58 | 79.06± 2.22 |
| 50 nM | 77.3 3 ± 4.56 | 79.83± 8.55 | 87.33 ± 5.59 | 56.33 ± 1.56 | 57.33± 1.58 | 59.03± 1.25 | 67.33± 1.00 | 81.33± 1.25 | 89.33± 2.25 | 80.88± 1.22 |
| 10 nM | 78.34 ± 4.87 | 80.84±4.59 | 78.34 ± 5.69 | 53.84 ± 1.57 | 58.34±1.25 | 48.66± 1.59 | 54.94± 1.84 | 91.38± 3.36 | 78.34±2.55 | 81.84±4.57 |
| 5 nM | 79.6 6 ± 5.97 | 71.66± 2.98 | 79.66 ± 0.69 | 52.06 ± 5.23 | 60.66± 1.59 | 49.66± 0.58 | 50.06± 1.05 | 79.66± 3.69 | 74.66± 1.25 | 79.66± 5.66 |
| 2 nM | 60.5 4 ± 5.59 | 55.64± 1.68 | 62.04 ± 1.58 | 30.54 ± 0.54 | 50.54± 1.57 | 46.04± 0.98 | 44.54± 1.23 | 56.54± 3.94 | 72.59± 5.58 | 70.54± 3.20 |

A positive value indicates that the fatty acid complex inhibits viral infection of cells, a value of ≥ 50 indicates that there is a median inhibition rate, and a negative value indicates that the fatty acid complex promotes viral infection of cells.

### Example II-46: Inhibition of water-soluble fatty acid complexes against infection of cells by HIV pseudovirus (18A-41)

The antiviral activity was **tested** with crotonic acid, vitamin E, octanedioic acid, glucosamine-DHA, ascorbic acid-EPA, threonine-tetracosanoic acid, ethylene glycol-OA, lysine-tetradecanoic acid, GA-n-octanoic acid, and glucose-hexadecanoic acid. The sample concentration was 100 µM, 50 µM, 10 µM, 1 µM, 100 nM, 50 nM, 10 nM, 5 nM, and 2 nM.

2 × 10⁴ cells were seeded in a 96-well plate and incubated overnight, till the cell density reaches 70%. Crotonic acid, vitamin E, octanedioic acid, GA-DHA, ascorbic acid-EPA, threonine-tetracosanoic acid, ethylene glycol-OA, lysine-tetradecanoic acid, glucosamine-n-octanoic acid, and glucose-hexadecanoic acid were respectively added to act on 293T cells in advance for 4 h, followed by the lentivirus transfection. The results are summarized in Table II-24

**Table II-24 Inhibition of water-soluble fatty acid complex against HIV pseudovirus infected cells (n = 3)**

| Concentr ation | Inhibition rate (%) | | | | | | | | | |
|---|---|---|---|---|---|---|---|---|---|---|
| | Croto nic acid | Vita min E | Octane dioic acid | Glucosa mine-DHA | Ascor bic Acid-EPA | Threoni ne-tetracosa noic acid | Ethyl ene glyco l-OA | Lysine-dodeca noic acid | Glucosa mine-n-octanoic acid | Glucose-hexadeca noic acid |
| 100 µM | 3.68 0.23 | 58.9 8± 6.37 | 98.77± 9.06 | 83.75± 2.77 | 82.84 ± 4.75 | 83.84± 3.84 | 86.47 ± 5.86 | 74.07± 6.03 | 53.68± 6.07 | 98.87± 6.03 |
| 50 µM | 11.36 1.22 | 61.3 6± 8.64 | 90.36± 8.57 | 78.76± 5.36 | 80.74 ± 4.76 | 81.96± 6.75 | 79.46 ± 8.46 | 69.38± 2.33 | 51.09± 9.38 | 87.59± 5.03 |
| 10 µM | - 44.26 1.36 | 4.16 ± 0.11 | -54.26± 6.06 | 4.26± 5.26 | - 64.54 ± 5.62 | -64.26± 6.54 | - 84.04 ± 6.24 | - 73.16± 3.04 | -84.26± 6.61 | -44.88± 1.03 |
| 1 µM | - 102.3 3 ± 8.06 | - 84.3 3± 6.36 | 4.93± 6.03 | 17.49± 5.03 | - 82.83 ± 7.59 | -42.03± 3.83 | - 92.33 ± 2.66 | - 62.93± 4.36 | -62.33± 3.93 | -94.23± 9.06 |
| 100 nM | 73.66 6.22 | 89.9 9± 8.97 | 80.44± 2.30 | 92.22± 2.64 | 98.77 ± 3.22 | 98.64± 5.77 | 93.46 ± 3.23 | 80.47± 4.46 | 88.76± 4.87 | 89.99± 5.07 |
| 50 nM | 77.33 6.31 | 79.3 3± 5.89 | 77.63± 5.59 | 76.33± 6.63 | 97.33 ± 5.33 | 99.01± 6.33 | 94.33 ± 6.32 | 71.34± 5.26 | 89.33± 3.65 | 85.46± 3.99 |
| 10 nM | 58.34 1.23 | 70.0 4± 4.65 | 69.54± 2.04 | 73.84± 6.54 | 86.46 ± 3.84 | 94.06± 6.46 | 89.89 ± 4.06 | 69.48± 2.36 | 78.34± 3.66 | 86.44± 5.03 |
| 5 nM | 29.66 5.44 | 61.8 7± 5.06 | 69.66± 1.87 | 72.06± 5.94 | 68.76 ± 5.79 | 92.25± 5.03 | 83.09 ± 3.25 | 46.54± 4.23 | 54.47± 2.09 | 57.26± 6.09 |
| 2 nM | 30.54 0.66 | 55.6 6± 6.03 | 52.04± 6.66 | 50.54± 2.04 | 72.04 ± 6.02 | 81.01± 5.26 | 82.74 ± 5.06 | 19.54± 3.65 | 22.59± 6.09 | 50.54± 1.56 |

A positive value indicates that the fatty acid complex inhibits viral infection of cells, a value of ≥ 50 indicates that there is a median inhibition rate, and a negative value indicates that the fatty acid complex promotes viral infection of cells.

### Example II-47: Prevention of cell infection from HPV pseudovirus (HPV6-GFP) by water-soluble fatty acid complex

HPV pseudovirus (HPV6-GFP) **was** diluted to 200TCID50 with DMEM complete medium. 100 µl of pseudovirus was pipetted to the corresponding well of the culture plate in which 293FT cells had been previously plated (prior to adding virus, the cells were incubated with water-soluble fatty acid complex for 4 h, and then the water-soluble fatty acid complex was discarded), the culture plate was gently patted and mixed well. The cell culture plates were incubated for 60-96 hours at 37°C in a 5% CO₂ incubator. The detection was performed by an ELISPOT plate reader, and the inhibition rate of infection (%) = 1-(sample detection value-cell control value)/(virus control value-cell control value) × 100% was calculated. The antiviral activity was tested with n-butyric acid, n-hexanoic acid, n-octanoic acid, Tween-80, adenosine-erucic acid, UDPGA-erucic acid, UDPGA-eicosanedioic acid, UDPGA-hexadecanedioic acid, glycocholic acid-palmitic acid, ascorbic acid-docosanedioic acid at the concentrations of 100 µM, 50 µM, 10 µM, 1 µM, 100 nM, 50 nM, 10 nM, 5 nM, and 2 nM. The results are summarized in Table II-25

**Table II-25 Inhibition properties of water-soluble fatty acid complexes against HPV pseudovirus infected cells (n = 3)**

| Concentr ation | Inhibition rate (%) | | | | | | | | | |
|---|---|---|---|---|---|---|---|---|---|---|
| | N-buty ric acid | Hexan oic acid | N-octan oic acid | Twe en-80 | Adenos ineerucic acid | UDP GA-erucic Acid | UDPGA - eicosane dioic acid | UDPGA-hexadecan edioic acid | Cholylgly cine-palmitic acid | Ascorbic acid-dodecane dioic acid |
| 100 µM | 53.6 8 ± 3.65 | 57.98 ± 4.98 | - 43.77 ± 2.54 | 83.9 5± 2.77 | 94.11± 2.69 | 98.34 ± 4.84 | 93.01± 6.22 | 53.58± 0.21 | 53.68± 4.69 | 59.68± 6.03 |
| 50 µM | 51.3 6 ± 5.36 | 51.08 ± 3.33 | - 27.36 ± 2.54 | 71.3 6± 2.36 | 91.84± 3.95 | 91.06 ± 1.05 | 93.56± 5.01 | 50.97± 3.06 | 51.09± 6.36 | 65.51± 4.65 |
| 10 µM | 24.8 6 ± 3.66 | 34.08 ± 3.08 | 44.76 0.57 | 44.8 7± 1.89 | 54.26± 1.83 | 24.49 ± 3.26 | 54.00± 2.96 | -23.16± 3.01 | -34.25± 5.66 | -44.24± 6.03 |
| 1 µM | 12.3 3 ± 4.56 | 22.33 ± 5.33 | 54.93 0.55 | 37.3 9± 1.55 | 32.83± 2.37 | - 32.03 ± 2.26 | 32.33± 1.96 | -2.93± 3.01 | -32.33± 2.66 | -94.23± 6.33 |
| 100 nM | 23.6 6 ± 3.66 | 35.99 ± 6.31 | 86.44 1.23 | 53.0 4± 3.04 | 94.75± 1.67 | 88.64 ± 3.69 | 84.96± 5.33 | 77.47± 3.47 | 58.76± 4.74 | 79.06± 5.13 |
| 50 nM | 57.3 3 ± 6.35 | 59.83 ± 5.03 | 57.33 4.03 | 66.3 3± 6.33 | 92.37± 3.35 | 89.03 ± 1.75 | 87.01± 6.33 | 71.33± 3.22 | 59.33± 2.33 | 70.88± 6.21 |
| 10 nM | 58.3 4 ± 3.34 | 60.84 ± 6.00 | 58.34 ± 6.36 | 63.0 4± 2.05 | 90.35± 4.69 | 80.86 ± 3.89 | 81.74± 2.74 | 61.78± 2.74 | 48.34± 5.26 | 61.84± 2.33 |
| 5 nM | 49.6 6 ± 6.33 | 51.66 ± 1.23 | 49.66 2.66 | 52.0 6± 1.06 | 85.69± 4.23 | 79.66 ± 2.65 | 80.10± 2.65 | 59.26± 2.25 | 54.66± 2.56 | 59.66± 5.69 |
| 2 nM | 40.5 4 ± 2.54 | 45.64 ± 2.33 | 42.04 5.03 | 30.5 4± 3.45 | 80.11± 3.66 | 56.04 ± 4.66 | 66.88± 2.88 | 56.14± 3.15 | 42.59± 6.33 | 60.54± 5.54 |

A positive value indicates that the fatty acid complex inhibits viral infection of cells, a value of ≥ 50 indicates that there is a median inhibition rate, and a negative value indicates that the fatty acid complex promotes viral infection of cells.

### Example II-48: Prevention of cell infection from influenza pseudovirus H7N9-Fluc by water-soluble fatty acid complex

The antiviral activity of influenza pseudovirus H7N9-Fluc was tested with glucosamine-erucic acid, n-decanoic acid, nonanoic acid, sodium undecylenate, serine-palmitic acid, threonine-c-9-hexadecanoic acid, ascorbic acid-undecanedioic acid, lysine-pentadecanedioic acid, glycocholic acid-arachidic acid, ascorbic acid-dodecanedioic acid. The method was the same as that of Example 55. The RLU was read using a multi-functional imaging microplate reader, and the inhibition rate results are shown in Table II-26.

**Table II-26 Inhibition properties of water-soluble fatty acid complexes against influenza pseudovirus infected cells (n = 3)**

| Concent ration | Inhibition rate (%) | | | | | | | | | |
|---|---|---|---|---|---|---|---|---|---|---|
| | Glucosa mine-erucic acid | N-deca noic acid | Nona noic acid | Sodium undecyl enate | Seri nepalm itic acid | Threoni ne-c-9-hexadec anoic acid | Ascorbi c acid-undecan edioic acid | Lysine-pentadeca nedioic acid | Cholylgl ycine-arachidi c acid | Ascorbi c acid-dodecan edioic acid |
| 100 µM | 99.78 ± 5.64 | 47.9 8± 3.09 | 43.77 ± 4.38 | 53.95± 4.39 | 74.0 8± 3.08 | 78.34± 4.39 | 73.01± 4.05 | 51.23± 3.26 | 83.38± 1.59 | 99.68± 6.03 |
| 50 µM | 91.44 ± 4.48 | 41.0 8± 4.08 | 40.06 ± 1.23 | 71.36± 3.26 | 71.8 4± 2.84 | 71.06± 4.08 | 83.56± 4.03 | 52.77± 2.98 | 71.18± 0.56 | 85.55± 12.23 |
| 10 µM | 54.05 ± 5.05 | - 54.3 8± 4.38 | 34.76 ± 3.06 | 40.23± 3.23 | - 43.6 4± 3.69 | 34.49± 4.49 | 44.08± 1.28 | 3.96± 0.12 | -4.64± 0.23 | 34.88± 2.36 |
| 1 µM | 12.07 ± 2.65 | - 32.3 3± 1.56 | - 4.93± 0.23 | -7.39± 0.22 | - 2.84 ± 5.03 | -12.84± 1.03 | 32.33± 2.37 | 12.03± 0.36 | -52.54± 3.69 | -24.83± 3.26 |
| 100 nM | 53.86 ± 3.98 | 75.5 4± 4.00 | 36.44 ± 2.33 | 53.04± 4.06 | 74.7 5± 5.23 | 68.64± 2.03 | 74.96± 2.33 | 57.47± 3.07 | 68.43± 3.34 | 69.06± 3.06 |
| 50 nM | 47.33 ± 4.56 | 75.3 8± 2.03 | 57.33 ± 1.66 | 56.33± 5.03 | 72.3 7± 3.66 | 59.03± 1.64 | 67.01± 5.59 | 61.33± 1.24 | 69.33± 3.88 | 70.88± 5.06 |
| 10 nM | 48.04 ± 7.56 | 66.1 1±2.33 | 58.34 ±4.02 | 53.04± 5.03 | 60.3 5±2.33 | 60.86± 4.03 | 71.74± 5.03 | 65.22± 5.33 | 58.87± 4.56 | 61.84± 2.84 |
| 5 nM | 39.66 ± 3.66 | 31.8 6± 2.23 | 49.66 ± 1.56 | 22.06± 2.14 | 55.6 9± 3.65 | 79.21± 5.22 | 70.10± 2.01 | 59.26± 6.26 | 54.25± 3.87 | 55.99± 3.36 |
| 2 nM | 50.54 ± 3.54 | 35.0 4± 2.04 | 42.04 ± 3.06 | 30.54± 2.36 | 40.1 1± 2.11 | 56.47± 5.47 | 36.88± 5.99 | 46.56± 4.56 | 52.89± 3.89 | 54.40± 1.40 |

A positive value indicates that the fatty acid complex inhibits viral infection of cells, a value of ≥ 50 indicates that there is a median inhibition rate, and a negative value indicates that the fatty acid complex promotes viral infection of cells.

### Example II-49: Prevention of cell infection from rabies pseudovirus CVS-11 by water-soluble fatty acid complex

Antiviral activity test (293T) was performed against rabies pseudovirus CVS-11 (purchased from Beijing Tiantan Water-Soluble Fatty Acid Complex Biotechnology Development Co. No. 80052) with glucosamine-trans-2-hexenoic acid, UDPGA-10-undecenoic acid, serine-tridecanoic acid, ascorbic acid-10-undecenoic acid, glucose-oleic acid, threonine-linolenic acid, ascorbic acid-octadecanedioic acid, lysine-pentacosanedioic acid, glycocholic acid-EPA, and ascorbic acid-DHA. The results are summarized in Table II-27.

**Table II-27 Inhibition properties of water-soluble fatty acid complex against rabies pseudovirus infected cells (n = 3)**

| Concent ration | Inhibition rate (%) | | | | | | | | | |
|---|---|---|---|---|---|---|---|---|---|---|
| | Glucosa mine-trans-2-hexenoi c acid | UDPG A-10-undece noic acid | Serine-trideca neoic acid | Ascor bic acid-10-undece noic acid | Gluc ose-oleic acid | Threo nine-linole nic acid | Ascorbic acid-octadecan edioic acid | Lysine-pentacosa nedioic acid | Glycoch olate-EPA | Asco rbic Acid - DHA |
| 100 µM | 99.78 ± 2.56 | 87.98± 2.97 | 63.77± 5.00 | 73.15± 2.15 | 80.21 ± 5.30 | 88.34 ± 5.66 | 90.01± 2.01 | 91.23± 1.26 | 98.38± 5.37 | 99.08 ± 6.36 |
| 50 µM | 91.44 ± 3.44 | 71.08± 1.29 | 50.06± 6.69 | 71.16± 4.59 | 70.04 ± 5.07 | 81.06 ± 1.36 | 84.16± 4.21 | 82.77± 5.59 | 91.18± 3.69 | 95.55 ± 6.98 |
| 10 µM | 24.51 ± 3.51 | 24.48± 2.48 | - 30.76± 2.66 | - 30.23± 3.32 | - 33.54 ± 2.54 | 4.49± 1.23 | -24.08± 3.06 | 73.96± 4.99 | 4.64± 2.36 | 34.88 ± 5.99 |
| 1 µM | -22.70 ± 2.86 | 32.33± 2.21 | - 14.53± 5.02 | - 57.39± 4.26 | - 14.84 ± 1.54 | - 92.84 ± 2.89 | 32.74± 2.75 | -12.03± 6.33 | 32.54± 1.55 | 14.83 ± 4.26 |
| 100 nM | 53.86 ± 3.70 | 65.87± 3.65 | 46.44± 5.20 | 43.04± 5.36 | 71.05 ± 2.54 | 68.64 ± 5.66 | 64.96± 6.49 | -7.47± 1.23 | 78.43± 5.06 | 79.06 ± 2.39 |
| 50 nM | 67.33 ± 7.23 | 75.21± 6.21 | 59.32± 6.32 | 66.83± 5.23 | 72.37 ± 3.37 | 69.03 ± 6.06 | 64.01± 1.26 | 67.33± 2.36 | 69.33± 1.25 | 70.88 ± 4.22 |
| 10 nM | 68.04 ± 1.59 | 76.22± 3.82 | 58.80± 2.80 | 63.04± 3.04 | 80.35 ± 2.06 | 70.86 ± 1.86 | 71.74± 2.74 | 69.22± 4.66 | 65.87± 5.55 | 64.84 ± 6.57 |
| 5 nM | 69.66 ± 5.04 | 51.86% 6.04 | 59.64± 3.64 | 42.06± 1.56 | 65.69 ± 6.69 | 59.21 ± 6.31 | 65.10± 3.71 | 59.26± 1.26 | 54.25± 6.45 | 51.99 ± 3.99 |
| 2 nM | 30.54 ± 3.54 | 55.04± 3.86 | 52.04± 5.04 | 40.74± 5.06 | 50.11 ± 3.11 | 54.89 ± 1.89 | 56.88± 5.88 | 56.56± 3.65 | 52.89± 2.87 | 51.40 ± 1.54 |

A positive value indicates that the fatty acid complex inhibits viral infection of cells, a value of ≥ 50 indicates that there is a median inhibition rate, and a negative value indicates that the fatty acid complex promotes viral infection of cells.

### Example II-50 Animal experiments-oral administration of water-soluble fatty acid complex against infection by novel crown virus pseudovirus

The following mice are referred to as transgenic mice: KI-hACE2 genotype C57BL/6mice, female, 4-6 weeks, Beijing Vital River Laboratory Animal Technology Co., Ltd.

Validation of the efficacy of animal oral administration against novel coronavirus infection was tested with n-octanoic acid, octanedioic acid, serine-LA, serine-EPA, ascorbic acid-EPA, ascorbic acid-docosanedioic acid, glucosamine-erucic acid, glucosamine-DHA, threonine-tetracosanoic acid, and lysine-pentacosanedioic acid. The mice were fed with the solution diluted to 1 mM by gavage, 200 uL/time, 3 times/day. After 48 h of continuous feeding, pseudovirus was infused through the lungs (concentration of 1.4 x 10⁶ TCID50/mL). Immunofluorescence of lung tissue was performed on the lungs of mice 2 days late. The mean fluorescence intensity was analyzed using ImageJ software. The results of comparative analysis of fluorescence intensity are shown in Table II-28. Compared with the control group, the fluorescence intensity of experimental group was significantly weaker. The mean fluorescence intensity was analyzed using ImageJ software. The P value was calculated as ≤ 0.001, indicating significant difference.

**Table II-28 Inhibition of water-soluble fatty acid complex against in vivo infection by Neocopyovirus (n = 3)**

| | | | |
|---|---|---|---|
| | | Concentration | |
| | | 1 mM | Control group (undosed) |
| | Serine-LA | 2.8± 0.56 | 25± 0.52 |
| | Octanedioic acid | 2.9± 0.22 | |
| | Glucosamine-docosahexaenoic acid | 3.1± 0.65 | |
| Fluorescence intensity | Ascorbic acid-eicosapentaenoic acid | 2.9± 0.68 | |
| | Threonine-tetracosanoic acid | 2.9± 0.11 | |
| | N-octanoic acid | 5.8± 0.76 | |
| | Ascorbic acid-dodecanedioic acid | 2.8± 0.55 | |
| | Glucosamine-erucic acid | 6.8± 0.49 | |
| | Lysine-pentacosanedioic acid | 2.8± 0.56 | |
| | Serine-EPA | 4.8± 0.96 | |

### Example II-51 Effect of water-soluble fatty acid complexes on phagocytic nano-drugs

293T cells were seeded on a 35 cm cell culture dishes and cultured for 24 hours at 37°C in a 5% CO₂ incubator. After the cells were stably adhered, the water-soluble fatty acid complexes in the following table were added to the cell culture medium. The culture was continued for 4 hours. The medium with the water-soluble fatty acid complex was pipetted and lipid vesicles loaded with DiD fluorescence were added. The phagocytosis of lipid vesicles by cell membrane was observed under fluorescence microscope 2 h later. The fluorescence intensity was analyzed by imageJ. The results are summarized in Table II-29

**Table II-29 Effect of water-soluble fatty acid complex on cellular phagocytosis of nano-drugs (n = 3)**

| | | Concentration | | | | | | | | | |
|---|---|---|---|---|---|---|---|---|---|---|---|
| Flu ore sce nce inte nsit y | | Control group (water-soluble fatty acid complex untreated) | 100uM | 50 uM | 10uM | 1uM | 100 nM | 50 nM | 10 nM | 5 nM | 2nM |
| | Serine-LA | 226.46± 1.26 | 14.31± 0.13 | 110.1 5±0.4 1 | 103.5 8±0.7 1 | 458.2 0±0.5 5 | 59.65 ±0.54 | 51.34 ±0.32 | 49.05 ±0.22 | 46.06 ±0.02 | 89.36 ±0.21 |
| | Threonine -LA | | 27.22± 0.16 | 64.86 ±0.19 | 507.6 3±0.2 3 | 412.9 0±0.4 3 | 54.37 ±0.63 | 45.68 ±0.23 | 43.39 ±0.55 | 64.18 ±0.47 | 100.4 6±4.2 3 |
| | Lysine-LA | | 36.75± 1.45 | 44.48 ±2.66 | 553.1 5±5.6 6 | 373.5 0±6.0 3 | 30.71 ±0.69 | 28.69 ±0.22 | 49.05 ±0.83 | 46.06 ±0.93 | 85.96 ±1.13 |
| | Serine-n-octanoic acid | | 59.90± 4.13 | 64.86 ±5.13 | 326.6 9±4.6 6 | 447.0 1±12. 33 | 83.29 ±5.66 | 98.90 ±0.13 | 104.5 3±0.1 3 | 108.5 6±0.1 3 | 157.3 0±0.1 3 |
| | Adenosin e-erucic acid | | 22.46± 0.63 | 42.03 ±0.53 | 326.6 9±10. 13 | 278.1 6±8.1 5 | 80.03 ±5.15 | 96.63 ±6.16 | 94.34 ±4.14 | 89.09 ±4.19 | 112.0 1±8.2 4 |
| | UDPGA-EPA | | 17.37± 0.11 | 64.86 ±1.10 | 371.9 8±12. 11 | 253.7 0±6.8 3 | 25.73 ±0.03 | 92.78 ±5.53 | 116.2 6±7.1 3 | 114.0 0±4.1 3 | 122.2 0±6.1 3 |
| | UDPGA-oleic acid | | 37.12± 0.43 | 61.01 ±0.73 | 348.7 5±9.3 3 | 412.9 0±15. 12 | 34.06 ±0.01 | 73.98 ±0.83 | 102.0 4±5.1 8 | 113.0 9±8.1 7 | 125.5 9±7.4 5 |
| | UDPGA-dodecanoi c acid | | 104.22 ±7.44 | 73.89 ±2.43 | 392.1 4±10. 48 | 436.9 1±16. 43 | 28.38 ±0.30 | 42.28 ±0.46 | 19.52 ±0.13 | 46.06 ±0.43 | 98.42 ±4.49 |
| | Cholylgly cine-hydroxyol eic acid | | 59.60± 1.43 | 65.47 ±0.77 | 349.3 4±10. 43 | 299.6 7±5.4 6 | 25.45 ±0.13 | 24.16 ±0.23 | 49.05 ±0.73 | 57.38 ±0.73 | 62.07 ±4.45 |
| | Taurochol ate butenoic acid | | 92.89± 4.43 | 73.49 ±1.43 | 216.8 1±4.4 3 | 439.8 5±10. 49 | 47.42 ±0.47 | 43.30 ±0.23 | 41.13 ±0.22 | 46.06 ±0.11 | 66.72 ±0.93 |
| | Crotonic acid | | 218.13 ±8.22 | 200.7 3±10. 31 | 326.6 9±20. 40 | 458.2 0±20. 41 | 59.65 ±1.57 | 51.34 ±0.43 | 94.34 ±0.43 | 159.2 9±0.4 3 | 157.3 0±20. 43 |
| | Vitamin E | | 92.89± 5.33 | 87.50 ±2.33 | 217.0 4±7.4 4 | 417.4 3±15. 23 | 22.67 ±0.03 | 46.81 ±1.48 | 67.85 ±1.43 | 86.35 ±2.47 | 100.4 1±4.4 3 |
| | Octanedio ic acid | | 2.79±0. 22 | 21.83 ±1.85 | 349.3 4±9.4 8 | 215.3 0±5.3 6 | 44.30 ±6.35 | 50.66 ±5.66 | 68.98 ±8.56 | 68.71 ±4.36 | 108.6 1±5.6 8 |
| | Glucosam ine-DHA | | 36.80± 3.21 | 48.10 ±4.81 | 216.8 1±14. 23 | 186.8 5±16. 25 | 17.62 ±0.26 | 53.60 ±1.33 | 59.24 ±1.28 | 63.27 ±5.36 | 44.07 ±4.27 |
| | VC-EPA | | 38.86± 6.23 | 43.62 ±2.63 | 372.6 2±22. 22 | 414.0 4±14. 56 | 2.79± 0.25 | 6.05± 1.00 | 30.66 ±5.23 | 70.75 ±4.56 | 63.32 ±6.32 |
| | Threonine -C24 | | 30.64± 5.55 | 46.51 ±5.21 | 416.7 8±12. 33 | 435.5 5±15. 26 | 14.81 ±1.33 | 12.84 ±2.22 | 22.90 ±1.22 | 38.29 ±5.26 | 39.09 ±7.89 |
| | Ethylene glycol-oleic acid | | 58.72± 8.75 | 69.34 ±5.32 | 392.1 4±22. 36 | 368.9 7±18. 56 | 44.23 ±4.26 | 64.90 ±1.56 | 69.12 ±4.26 | 121.0 7±11. 26 | 182.2 1±18. 23 |
| | Lys-C12 | | 104.90 ±6.23 | 110.7 6±8.5 4 | 417.2 8±17. 23 | 367.6 1±27. 11 | 25.45 ±5.22 | 24.16 ±3.36 | 49.05 ±5.26 | 103.1 1±10. 33 | 175.3 0±15. 23 |
| | GA-n-octanoic acid | | 2.56±0. 02 | 28.10 ±4.21 | 328.1 016.2 5± | 439.8 5±19. 25 | 22.67 ±1.22 | 32.93 ±0.33 | 30.71 ±1.25 | 96.79 ±7.26 | 112.0 1±12. 03 |
| | Glucose-C16 | | 104.90 ±4.91 | 110.1 5±10. 22 | 170.1 6±20. 33 | 198.5 4±8.5 5 | 172.8 8±26. 33 | 96.63 ±6.54 | 94.34 ±4.57 | 114.0 0±11. 02 | 134.6 5±15. 22 |
| | N-butyric acid | | 95.16± 5.61 | 110.7 8±9.8 7 | 149.2 8±9.2 8 | 175.8 9±15. 87 | 144.9 6±14. 98 | 90.97 ±4.58 | 88.68 ±8.26 | 109.4 7±7.8 8 | 123.1 0±13. 21 |
| | Hexanoic acid | | 325.58 ±15.26 | 288.4 2±8.3 2 | 125.1 0±12. 55 | 102.0 7±12. 22 | 30.71 ±1.25 | 96.63 ±6.32 | 94.34 ±4.25 | 114.0 0±14. 23 | 131.2 6±11. 25 |
| | N-octanoic acid | | 36.35± 6.23 | 64.86 ± | 124.8 5± | 141.7 9± | 106.3 5± | 76.25 ± | 83.70 ± | 108.5 6± | 157.3 0± |
| | Tween-80 | | 13.34± 3.24 | 18.48 ± | 103.5 8± | 152.1 1± | 11.89 ± | 17.28 ± | 21.85 ± | 32.41 ± | 45.04 ± |
| | Adenosin e-erucic acid | | 3.76±0. 26 | 20.25 ±4.22 | 171.0 0±14. 23 | 299.0 0±23. 21 | 25.73 ±4.85 | 24.84 ±4.62 | 43.34 ±3.36 | 46.06 ±5.06 | 99.55 ±5.66 |
| | UDPGA-erucic Acid | | 15.83± 1.25 | 14.58 ±0.36 | 104.1 7±5.8 9 | 153.2 5±13. 26 | 34.06 ±6.31 | 29.42 ±4.36 | 41.35 ±1.26 | 45.07 ±4.56 | 75.00 ±4.58 |
| | UDPGA-eicosaned ioic acid | | 105.12 ±9.59 | 111.0 3±11. 28 | 278.9 1±15. 26 | 233.1 0±23. 26 | 51.02 ±1.26 | 64.93 ±3.69 | 86.55 ±5.23 | 92.26 ±6.36 | 99.33 ±8.22 |
| | UDPGA-hexadeca nedioic acid | | 104.90 ±10.23 | 110.7 6±11. 22 | 304.0 2±16. 28 | 299.6 7±28. 26 | 93.39 ±5.69 | 92.10 ±2.36 | 116.9 9±16. 23 | 102.6 8±9.6 8 | 130.0 1±12. 36 |
| | Cholylgly cine-palmitic acid | | 91.31± 9.58 | 78.11 ±7.56 | 326.6 5±18. 69 | 439.8 5±19. 58 | 47.42 ±2.36 | 65.95 ±4.36 | 86.42 ±1.23 | 91.35 ±5.35 | 89.36 ±5.36 |
| | Ascorbic acid-dodecane dioic acid | | 0.50±0. 02 | 19.38 ±1.02 | 104.0 6±8.5 6 | 199.1 3±11. 25 | 104.4 9±14. 232 | 119.2 8±15. 33 | 117.6 7±17. 56 | 136.6 5±13. 66 | 112.0 1±10. 33 |
| | GA-Erucic acid | | 117.80 ±9.54 | 133.4 3±12. 32 | 349.6 1±12. 23 | 299.6 7±14. 21 | 55.39 ±5.23 | 55.75 ±2.33 | 76.75 ±7.25 | 154.3 1±11. 23 | 147.1 1±14. 53 |
| | N-decanoic acid | | 127.34 ±12.22 | 135.7 4±15. 22 | 147.7 4±14. 23 | 237.6 2±17. 56 | 143.9 4±13. 26 | 96.63 ±6.99 | 94.34 ±7.54 | 114.0 0±11. 23 | 131.2 6±8.6 3 |
| | Nonanoic acid | | 104.28 ±10.25 | 64.86 ±5.54 | 135.3 6±11. 03 | 243.2 0±13. 65 | 106.3 5±5.0 6 | 98.90 ±8.26 | 106.3 5±4.6 5 | 176.5 0±7.0 6 | 157.3 0±7.0 6 |
| | Sodium undecylenate | | 58.70± 2.66 | 63.77 ±3.37 | 325.2 9±15.22 | 232.8 9±12.26 | 57.18 ±5.23 | 62.57 ±3.60 | 89.79 ±4.58 | 100.3 4±8.02 | 135.6 3±15.23 |
| | Serine-**palmitic** acid | | 49.05± 4.66 | 65.54 ±5.21 | 148.3 5±18. 23 | 255.5 4±12. 26 | 71.02 ±6.23 | 92.78 ±9.06 | 88.64 ±4.56 | 47.08 ±4.26 | 98.05 ±8.26 |
| | Threonine -cis-9-hexadeca noic acid | | 61.12± 5.21 | 37.23 ±2.35 | 126.6 4±11. 02 | 153.2 5±13. 25 | 56.71 ±6.74 | 74.71 ±5.36 | 64.00 ±4.06 | 67.71 ±5.67 | 142.9 4± |
| | VC-undecane dioic acid | | 110.44 ±9.87 | 106.9 6±5.6 4 | 217.4 9±14. 22 | 199.2 2±19. 26 | 96.31 ±6.21 | 87.57 ±5.57 | 78.76 ±7.89 | 92.26 ± | 121.0 2± |
| | Lysine-pentadeca nedioic acid | | 37.64± 2.65 | 65.27 ±4.57 | 236.9 7±16. 39 | 345.4 4±15. 26 | 71.49 ±1.47 | 69.46 ±8.26 | 93.14 ±3.69 | 103.6 1±8.0 6 | 106.6 9± |
| | Cholylgly cine-arachidic acid | | 0.72±0. 02 | 32.72 ±2.73 | 147.4 7±17. 45 | 282.6 9±12. 36 | 70.07 ±4.55 | 65.95 ±5.23 | 86.42 ±5.26 | 99.67 ±8.23 | 103.2 7± |
| | Ascorbic acid-dodecane dioic acid | | 0.50±0. 03 | 19.38 ±5.32 | 170.9 5±15. 22 | 277.8 7±17. 56 | 104.4 9±5.5 7 | 73.98 ±4.69 | 72.38 ±3.68 | 68.71 ±4.58 | 157.3 0± |
| | Glucosam ine-t-2-hexenoic acid | | 27.22± 1.23 | 65.49 ±4.39 | 171.0 2±13. 23 | 153.2 5±23. 26 | 77.29 ±6.29 | 56.14 ±5.23 | 53.85 ±4.39 | 109.0 2±8.9 9 | 101.8 2± |
| | UDPGA-10-undecenoi c acid | | 82.05± 5.67 | 113.0 9±3.2 1 | 296.1 2±17. 56 | 259.3 6±15. 26 | 121.2 9±5.2 7 | 92.12 ±6.32 | 93.30 ±4.36 | 91.40 ±6.23 | 108.6 1± |
| | Serine-tridecane oic acid | | 60.80± 8.56 | 65.31 ±5.21 | 294.9 2±18. 23 | 356.4 3±26. 23 | 128.9 9±7.4 6 | 75.12 ±4.12 | 83.70 ±4.71 | 131.2 1±5.6 6 | 134.2 0± |
| | VC-10-undecenoi c acid | | 44.82± 4.87 | 67.85 ±4.32 | 302.4 1±2.3 1 | 260.0 7±10. 23 | 65.56 ±4.69 | 62.57 ±2.65 | 44.50 ±4.56 | 77.70 ±6.65 | 112.9 8±5.3 9 |
| | Glucose-oleic acid | | 26.41± 5.32 | 42.89 ±2.32 | 216.2 9±5.2 4 | 436.7 1±15. 66 | 71.02 ±4.02 | 70.13 7.23± | 65.99 ±6.23 | 92.37 ±4.56 | 102.1 6±8.6 5 |
| | Threonine -linolenic acid | | 22.62± 4.33 | 35.87 ±4.22 | 280.9 9±2.3 6 | 152.3 2±22. 36 | 79.35 ±7.22 | 81.50 ±5.36 | 64.00 ±3.98 | 79.03 ±7.26 | 97.65 ±9.99 |
| | VC-octadecan edioic acid | | 19.86± 1.02 | 39.02 ±4.29 | 58.97 ±4.33 | 253.7 0±5.6 7 | 243.3 8±2.3 6 | 73.98 ±3.36 | 69.70 7.56± | 92.26 ±7.26 | 98.37 ±7.87 |
| | Lysine-pentacosa nedioic acid | | 3.67±0. 13 | 19.97 ±0.22 | 215.9 5±16. 23 | 152.7 7±15. 22 | 48.85 ±4.21 | 69.46 ±6.32 | 77.29 ±5.22 | 103.6 1±8.5 4 | 106.6 9±9.8 7 |
| | Glycocho late-EPA | | 2.08±0. 10 | 10.08 ±0.56 | 147.4 7±5.6 6 | 192.8 8±12. 33 | 47.42 ±2.36 | 65.95 ±4.02 | 79.62 ± | 108.7 2±8.3 9 | 110.0 6±8.6 6 |
| | Ascorbic Acid-DHA | | 19.86± 0.23 | 39.02 ±1.03 | 58.97 ±1.55 | 253.7 0±10. 23 | 243.3 8±11. 00 | 73.98 ±5.36 | 69.70 ±4.55 | 92.26 ±8.23 | 98.37 ±7.89 |

### Example II-52: Interaction between water-soluble fatty acid complex and cell membrane

The fatty acids were combined with water-soluble small molecules to form a complex, which were labeled with GFP fluorescence to detect cell membrane fluorescence. Epithelial cells were seeded in 6-well plates and cultured in an incubator at 37°C and 5% CO₂ for 24 hours. After the cells were stably adhered, the water-soluble fatty acid complexes in the following table were added to the cell culture medium. The culture was continued for 4 hours. The medium with the drug was pipetted and replaced with normal DMEM medium. The entry of the fatty acid-water-soluble small molecule complex into the cell membrane was observed under a fluorescence microscope at the following time points (see Fig. 151), respectively. The green fluorescence intensity was analyzed by imageJ (see Table II-30, n = 3).

**Table II-30 Interaction results of water-soluble fatty acid complexes and cell membrane**

| **Serial No.** | **Water-soluble fatty acid complex** | **Mean fluorescence intensity** | | | | | |
|---|---|---|---|---|---|---|---|
| | | **0 Hour s** | **2 Hours** | **4 Hours** | **8 Hours** | **12 Hours** | **24 Hours** |
| 1 | Thr-n-butyric acid | 126.4 ± 4.6 | 121.4± 4.6 | 115.0± 0.4 | 60.41± 3.4 | 34.91± 5.6 | 30.67± 4.5 |
| 2 | Lys-crotonic acid | 119.3 ± 3.4 | 120.0± 6.6 | 98.3± 5.5 | 55.9± 6.7 | 46.9± 3.6 | 40.4± 5.3 |
| 3 | VC-succinic acid | 129.5 ± 8.7 | 123.3± 4.8 | 109.8± 3.7 | 70.3± 8.7 | 54.5± 7.7 | 46.0± 5.7 |
| 4 | Adenosine monophosphate-fumaric acid | 125.1 ± 7.6 | 120.5± 7.4 | 113.3± 4.6 | 50.0± 2.4 | 35.8± 4.6 | 31.3± 4.4 |
| 5 | Glucose-n-hexanoic acid | 133.0 ± 4.4 | 131.4± 6.7 | 120.0± 7.3 | 89.4± 6.2 | 68.3± 4.6 | 29.3± 6.4 |
| 6 | Ser-t-2-hexenoic acid | 104.3 ± 5.2 | 101.3± 8.6 | 95.4± 4.5 | 39.9± 8.4 | 32.5± 3.6 | 29.8± 5.2 |
| 7 | Thr-hexane diacid | 128.4 ± 8.0 | 125.9±4. 5 | 119.3± 2.5 | 56.4± 5.7 | 41.3± 0.5 | 34.5± 7.9 |
| 8 | Lys-n-octanoic acid | 103.4 ± 3.1 | 99.3± 7.9 | 92.4± 7.7 | 45.4± 4.7 | 30.4± 7,5 | 24.2± 8.5 |
| 9 | Glucose-t-2-octenoic acid | 99.4± 3.6 | 92.5± 4.0 | 61.4± 4.2 | 42.3± 3.3 | 36.4± 4.3 | 32.4± 6.6 |
| 10 | Thr-octanedioic acid | 125.3 ± 4.2 | 122.4± 7.5 | 115.4± 6.8 | 55.4± 7.8 | 45.0± 6.3 | 35.3± 5.9 |
| 11 | Vc-nonanoic acid | 129.5 ± 6.7 | 115.6± 6.8 | 93.7± 4.2 | 46.3± 5.7 | 36.4± 5.6 | 33.0± 7.0 |
| 12 | Ser-n-decanoic acid | 115.4 ± 4.4 | 113.3± 6.4 | 108.3± 4.4 | 75.5± 2.7 | 56.4± 2.4 | 39.5± 5.7 |
| 13 | Thr-undecanoic acid | 129.5 ± 7.6 | 123.5± 3.5 | 118.7± 5.7 | 43.6± 9.3 | 35.3± 4.0 | 24.6± 3.8 |
| 14 | Lys-10-undecenoic acid | 135.6 ± 2.4 | 130.8± 6.7 | 118.2± 9.0 | 89.5± 4.4 | 58.6± 5.7 | 49.6± 0.5 |
| 15 | Vc-undecanedioic acid | 124.4 ± 4.5 | 116.4± 0.7 | 112.1± 6.3 | 65.3± 3.6 | 41.3± 9.3 | 35.4± 1.6 |
| 16 | Adenosine monophosphate-lauroic acid | 116.2 ± 6.7 | 113.4± 4.5 | 111.3± 4.4 | 88.2± 6.3 | 74.4± 2.1 | 38.2± 4.7 |
| 17 | Glucose-t-2-dodecenoic acid | 129.3 ± 0.6 | 122.0± 6.6 | 109.6± 5.6 | 63.4± 6.0 | 45.7± 4.6 | 38.5± 8.2 |
| 18 | Ser-dodecanedioic acid | 127.3 ± 3.5 | 126.3± 5.2 | 118.3± 7.3 | 69.3± 8.4 | 59.3± 5.5 | 45.3± 1.6 |
| 19 | Thr-tridecaneoic acid | 106.5 ± 6.4 | 104.3± 7.8 | 96.6± 2.6 | 53.5± 2.6 | 42.5± 4.2 | 39.0± 4.3 |
| 20 | Lys-tridecanedioic acid | 134.4 ± 5.7 | 125.0± 6.9 | 113.9± 4.2 | 64.3± 1.6 | 48.4± 6.8 | 42.0± 2.6 |
| 21 | Vc-tetradecanoic acid | 115.4 ± 4.6 | 100.2± 3.7 | 87.2± 4.6 | 41.2± 9.4 | 33.6± 5.2 | 27.5± 7.3 |
| 22 | Glucose-pentadecanedioic acid | 97.5± 6.3 | 92.3± 6.4 | 58.4± 5.5 | 45.5± 5.1 | 36.4± 4.4 | 32.4± 2.2 |
| 23 | Thr-palmitic acid | 123.3 ± 6.6 | 124.4± 7.5 | 119.5± 4.1 | 69.2± 7.5 | 45.3± 5.8 | 42.5± 5.7 |
| 24 | Glucose-c-9-hexadecenoic acid | 126.3 ± 2.5 | 113.5± 8.2 | 105.5± 6.8 | 87.5± 6.8 | 64.3± 5.1 | 43.5± 5.0 |
| 25 | Ser-hexadecanedioic acid | 119.6 ± 9.4 | 116.2± 5.1 | 93.4± 5.3 | 63.9± 4.1 | 46.0± 3.6 | 37.6± 6.7 |
| 26 | Lys-stearic acid | 105.3 ± 5.2 | 103.4± 2.7 | 98.3± 2.5 | 56.5± 5.6 | 46.7± 8.5 | 39.0± 5.3 |
| 27 | Adenosine monophosphate-oleic acid | 114.3 ± 5.7 | 119.5± 2.2 | 102.3± 5.5 | 75.5± 7.2 | 36.4± 3.1 | 30.6± 2.6 |
| 28 | Thr-linoleic acid | 127.9 ± 6.6 | 122.3± 4.7 | 102.0± 1.5 | 65.2± 5.6 | 45.0± 2.5 | 41.5± 7.2 |
| 29 | Vc-linolenic acid | 98.9± 2.7 | 93.3± 8.5 | 69.4± 7.9 | 46.6± 7.2 | 40.3± 6.8 | 42.2± 5.8 |
| 30 | Ser-octadecanedioic acid | 136.2 ± 8.5 | 127.3± 1.6 | 102.3± 4.4 | 76.2± 4.6 | 53.9± 7.2 | 42.4± 5.5 |
| 31 | Thr-arachidic acid | 109.4 ± 3.7 | 104.4± 5.4 | 91.5± 6.7 | 54.3± 7.2 | 41.4± 3.4 | 34.2± 5.2 |
| 32 | Lys-EPA | 123.3 ± 8.8 | 119.2± 3.7 | 109.4± 1.7 | 66.4± 4.7 | 39.4± 5.8 | 32.5± 4.5 |
| 33 | Adenosine monophosphate-eicosanedioic acid | 104.4 ± 6.2 | 94.4± 6.4 | 85.0± 8.8 | 43.0± 4.6 | 34.4± 7.3 | 26.5± 6.3 |
| 34 | Thr-erucic acid | 137.4 ± 5.8 | 121.3± 5.5 | 105.2± 7.3 | 79.5± 4.8 | 48.3± 1.2 | 42.4± 4.6 |
| 35 | Vc-DHA | 126.3 ± 7.4 | 115.4± 5.2 | 109.8± 4.9 | 52.0± 6.1 | 42.0± 3.4 | 37.9± 4.6 |
| 36 | Ser-docosanedioic acid | 114.5 ± 3.3 | 101.4± 6.9 | 95.4± 5.5 | 74.4± 2.4 | 34.5± 5.5 | 20.4± 4.4 |

### Example II-53 Interaction of water-soluble fatty acid complex with alveolar epithelial cell membrane

The mice were anesthetized with sodium pentobarbital (50 mg/kg) and fixed. A venous indwelling needle was inserted into the trachea. The needle core was withdrawn. The needle was connected with a 1 ml syringe (containing 150 µl of water column). It was observed that whether the water column moves up and down with the respiration, and confirm that the indwelling needle was inserted into the mouse bronchus. 50 µl of 0.1 mM Vc-succinic acid, adenosine monophosphate-fumaric acid, Thr-palmitic acid, Ser-c-9-hexadecenoic acid, glucose-hexadecanedioic acid, glucose-linolenic acid, Thr-hexane diacid, Thr-octanedioic acid, Vc-undecanedioic acid was instilled, and 0.3 ml of air was insufflated into bronchi to ensure complete entry of the drug into the lungs. The interaction of the water-soluble fatty acid complex with the alveolar epithelial cell membrane was observed at 2, 4, 8, 12 and 24 h by an *in vivo* imaging instrument. See Fig. 152, and the results showed that the water-soluble fatty acid complex was also stable in the lung tissue at 24 h. The experimental results are shown in Table II-31.

**Table II-31 Fluorescence intensity of water-soluble fatty acid complexes deposited in the lungs (n = 3)**

| Cpd. | Fluorescence intensity | | | | |
|---|---|---|---|---|---|
| | 2h | 4h | 8h | 12 h | 24h |
| ICG | 1.709 | 1.648 | 1.254 | 1.105 | 1.023 |
| Vc-succinic acid-ICG | 1.821 | 1.718 | 1.725 | 1.698 | 1.642 |
| Adenosine monophosphate-fumaric acid-ICG | 1.578 | 1.552 | 1.521 | 1.436 | 1.403 |
| Thr-palmitate-ICG | 1.702 | 1.721 | 1.698 | 1.523 | 1.511 |
| Ser-c-9-hexadecenoic acid-ICG | 1.785 | 1.763 | 1.745 | 1.632 | 1.615 |
| Glucose-hexadecanedioic acid-ICG | 1.657 | 1.620 | 1.569 | 1.456 | 1.423 |
| Glucose-linolenic acid-ICG | 1.802 | 1.789 | 1.687 | 1.456 | 1.444 |
| Thr- hexane diacid-ICG | 1.811 | 1.768 | 1.643 | 1.638 | 1.623 |
| Thr-octanedioic acid-ICG | 1.735 | 1.725 | 1.715 | 1.695 | 1.599 |
| Vc-undecanedioic acid-ICG | 1.632 | 1.569 | 1.489 | 1.342 | 1.364 |

### Example II-54 Improvement on skin photoaging by water-soluble fatty acid complex

The mice used below refer to BALB/c nude mice, female, 10-12 weeks old, Beijing Vital River Laboratory Animal Technology Co., Ltd. BALB/c nude mice were divided into a control group and a model group ( (n = 3). Lys-ricinoleic acid, Ser-linolenic acid, Ser-linoleic acid, Thr-DHA, and Ser-EPA at 30 µM, 50 µM and 100 µM were subcutaneously smeared in the model group. The back skin of nude mice was irradiated with 311 nm UVB UV lamp (installed in a self-made UV irradiation box, the UV lamp was 30 cm away from the back of mice), 40 min/day, for 40 days, at a dose of 120 mJ/cm². The levels of TNF-α, IL-1β, and IL-6 in serum were detected. The results show that the application of water-soluble fatty acid complex can effectively improve skin photoaging. The results are shown in Tables II-32 to II-34.

**Table II-32 Inhibition of TNF-α release by water-soluble fatty acid complex**

| Complex | TNF-α (pg/ml) | | | |
|---|---|---|---|---|
| | 0µM | 30µM | 50µM | 100µM |
| Lys-ricinoleic acid | 98 | 60 | 55 | 57 |
| Ser-linolenic acid | 104 | 51 | 49 | 47 |
| Ser-linoleic acid | 106 | 48 | 51 | 45 |
| Thr-DHA | 95 | 53 | 47 | 49 |
| Ser-EPA | 97 | 54 | 49 | 44 |

**Table II-33 Inhibition of IL-1β release by water-soluble fatty acid complex**

| Complex | IL-1β (pg/ml) | | | |
|---|---|---|---|---|
| | 0µM | 30µM | 50µM | 100µM |
| Lys-ricinoleic acid | 110 | 70 | 65 | 63 |
| Ser-linolenic acid | 101 | 62 | 53 | 58 |
| Ser-linoleic acid | 104 | 53 | 54 | 55 |
| Thr-DHA | 99 | 54 | 56 | 50 |
| Ser-EPA | 113 | 57 | 61 | 54 |

**Table II-34 Inhibition of IL-6 release by water-soluble fatty acid complex**

| Complex | IL-6 (pg/ml) | | | |
|---|---|---|---|---|
| | 0µM | 30µM | 50µM | 100µM |
| Lys-ricinoleic acid | 79 | 34 | 38 | 33 |
| Ser-linolenic acid | 71 | 29 | 33 | 31 |
| Ser-linoleic acid | 65 | 28 | 27 | 29 |
| Thr-DHA | 83 | 36 | 35 | 34 |
| Ser-EPA | 84 | 41 | 38 | 39 |

### Example II-55 Reduction in inflammatory aging in mice by water-soluble fatty acid complex

The mice used below refer to C57BL/6 male mice (8 weeks old), Beijing Vital River Laboratory Animal Technology Co., Ltd. Lipopolysaccharide (LPS) mice were established by intraperitoneal injection of 0.5 mg/kg LPS once a day for 30 days. LPS mice were divided into a control group and an experimental group (3 mice in each group). Experimental group were given 1 mM Lys-ricinoleic acid, Thr-linolenic acid, Ser-linoleic acid, Thr-DHA, and Ser-EPA, 200 µl/time, once a day, for 30 days. The levels of IL-1β, IL-6, and TNF-α in serum were detected. The results showed that feeding water-soluble fatty acid complex could significantly reduce inflammatory aging in mice. The results are shown in Tables II-35 to II-37.

**Table II-35 Inhibition of IL-1β release by water-soluble fatty acid**

| Complex | IL-1β (pg/ml) | |
|---|---|---|
| | 0 mM | 1 mM |
| Lys-ricinoleic acid | 130 | 51 |
| Thr-linolenic acid | 115 | 41 |
| Ser-linoleic acid | 108 | 43 |
| Thr-DHA | 121 | 55 |
| Ser-EPA | 116 | 48 |

**Table II-36 Inhibition of IL-6 release by water-soluble fatty acid**

| Complex | IL-6 (pg/ml) | |
|---|---|---|
| | 0 mM | 1 mM |
| Lys-ricinoleic acid | 114 | 60 |
| Thr-linolenic acid | 103 | 57 |
| Ser-linoleic acid | 116 | 39 |
| Thr-DHA | 112 | 50 |
| Ser-EPA | 109 | 47 |

**Table II-37 Inhibition of TNF-α release by water-soluble fatty acid**

| Complex | TNF-α (pg/ml) | |
|---|---|---|
| | 0 mM | 1 mM |
| Lys-ricinoleic acid | 89 | 43 |
| Thr-linolenic acid | 87 | 52 |
| Ser-linoleic acid | 91 | 49 |
| Thr-DHA | 103 | 53 |
| Ser-EPA | 98 | 48 |

While the present invention has been described by way of example and not limitation, other variations of the disclosed embodiments will become apparent to those skilled in the art upon those skilled in the art upon reference to the description of the invention, are intended to be within the scope of the claimed invention.

## Claims

1. A complex capable of preventing, blocking, and/or treating a viral or bacterial infection, comprising an acting moiety, a binding moiety and a water-soluble moiety, wherein the virus is one or two or more viruses selected from the group consisting of novel coronavirus, influenza virus, AIDS virus, hepatitis B virus, human herpes virus, Ebola virus, rabies virus, and a human papilloma virus; the bacteria is one or two or more bacteria selected from the group consisting of *Escherichia coli, Staphylococcus aureus,* methicillin-resistant *Staphylococcus aureus, Streptococcus pneumoniae, Klebsiella pneumoniae* and *Pseudomonas aeruginosa;*
the acting moiety is a fat-soluble saturated and/or unsaturated carbon chain with a branched, cyclic and/or linear structure; the carbon chain is a molecule or a residue of the molecule, and the carbon chain is a carbon chain with 3-100 carbon atoms; wherein the acting moiety is a carbon chain or residue of the carbon chain with 3-100 carbon atoms formed by saturated and/or unsaturated fatty acids;
the water-soluble moiety is a water-soluble molecule or a residue of the molecule; the molecule contains one or two or more functional groups selected from the group consisting of amide group, phosphoryloxy group, carboxyl group, phosphate group, sulfonyl group, sulfonyloxy group, hydroxyl group, quaternary ammonium group, thioether group, disulfide bond, ether group, thiol group, aldehyde group, ester group, amine group, amino group, urea group, and guanidine group; and the water-soluble moiety can be one or two or more of the above functional groups linked to the carbon chain as the acting moiety;
the binding moiety is a molecule or a residue of the molecule capable of binding to a microbial lipid membrane, a microorganism surface protein, a microorganism surface polysaccharide, or a cell wall component or capable of binding to a polysaccharide or a protein or polypeptide in the microorganism; the binding moiety can be the same as the water-soluble moiety, which is a protein, polypeptide, amino acid, oligopeptide, oligosaccharide, monosaccharide and/or polysaccharide molecule or a residue thereof capable of binding to a microbial lipid membrane or a surface domain; and
wherein the number of any one of the acting moiety, the water-soluble moiety, and the binding moiety in the complex can be 1 or 2 or more.

2. The complex according to claim 1, wherein the number of carbon atoms is 3-48.

3. The complex according to claim 1, wherein the number of carbon atoms is 3-26.

4. The complex according to claim 1, wherein the water-soluble moiety is a water-soluble molecule or a residue of the molecule which contains one or two or more groups selected from the group consisting of a thiol group, amino group, phosphate group, carboxyl group, sulfonyl group, hydroxyl group, amine group, urea group, guanidine group, and disulfide group;
the binding moiety has a group for binding, which is capable of binding to the microbial lipid membrane, the microorganism surface protein, the microorganism surface polysaccharide or the cell wall component or capable of binding to a polysaccharide, protein, or polypeptide in the microorganism; the group is two or more groups from the water-soluble moiety or from groups independently as the binding moiety selected from the group consisting of a thiol group, amino group, phosphate group, carboxyl group, sulfonyl group, hydroxyl group, amine group, urea group, guanidine group, and disulfide group, or from one or two or more groups providing a carbon chain to carbon chain connection which is selected from the group consisting of a thiol group, amino group, phosphate group, carboxyl group, sulfonyl group, hydroxyl group, amine group, urea group, guanidine group, and disulfide group, such that the complex has one or two or more groups selected from the group consisting of thiol group, amino group, phosphate group, carboxyl group, sulfonyl group, hydroxyl group, amine group, urea group, guanidine group, and disulfide group.

5. The complex according to claim 4, wherein the binding moiety is one or two or more groups selected from the group consisting of dibasic or polybasic fatty acid, amino acid, targeting protein, targeting peptide, and targeting polysaccharide.

6. The complex according to claim 4, wherein the complex is a complex formed by linking a fatty acid with 3-50 carbon atoms and a water-soluble amino acid; or the complex is a complex formed by linking a fatty acid with 3-50 carbon atoms to the targeting polypeptide; or the complex is a complex formed by the reaction of a fatty acid with 3-50 carbon atoms, the targeting polypeptide and PEG; or the complex is a complex formed by the reaction of a surfactant and one or two or more selected from the group consisting of a dibasic or polybasic fatty acid, an amino acid, a targeting protein, a targeting polypeptide and a targeting polysaccharide.

7. The complex according to claim 4, wherein the saturated and/or unsaturated fatty acid is selected from the group consisting of saturated fatty acids or unsaturated fatty acids with 3-50 carbon atoms; the fatty acid is a fatty acid or amino acid containing a double bond, triple bond, hydroxyl group, amino group and/or oxo group, and is a monobasic, dibasic, or polybasic acid.

8. The complex according to claim 4, wherein the saturated and/or unsaturated fatty acid is one or two or more selected from the group consisting of saturated fatty acids with 3-46 carbon atoms, monoenoic acids with 3-34 carbon atoms, dienoic acids with 5-30 carbon atoms, trienoic acids with 7-30 carbon atoms, tetraenoic acids with 12-38 carbon atoms, pentaenoic acids with 12-38 carbon atoms, hexaenoic acids with 22-38 carbon atoms, alkynoic acids with 6-22 carbon atoms, dialkynoic acids with 10-22 carbon atoms, trialkynoic acids with 12-22 carbon atoms, enynic acids with 8- 20 carbon atoms, fatty acids with 3-30 carbon atoms in the main chain and 1-10 alkyl and/or 1-3 hydroxyl groups in the branches, saturated linear and branched dicarboxylic and tricarboxylic acids with 3-38 carbon atoms, and unsaturated linear or branched dicarboxylic and tricarboxylic acids with 4-18 carbon atoms that is substitutable with hydroxyl groups, carboxylic acids with 3-18 carbon atoms substituted with amino, hydroxyl, oxo and/or methyl, N-fatty acyl amino acids with 6-30 carbon atoms, amino acids containing 2 or more fatty acyl groups, and polybasic carboxylic acids linked by thioether and amide bonds.

9. The complex according to claim 4, wherein the saturated or unsaturated fatty acid is one or two or more selected from the group consisting of fumaric acid, octanoic acid, glutaconic acid, hexanoic acid, nonanoic acid, dodecanoic acid, tetradecanoic acid, hexadecanoic acid, octadecanoic acid, eicosanoic acid, oleic acid, linoleic acid, linolenic acid, eicosapentaenoic acid, docosapentaenoic acid, docosahexaenoic acid, pentacosanoic acid, heptanoic acid, decanoic acid, dodecenoic acid, tetradecenoic acid, dotriacontahexaenoic acid, octacosanoic acid, or a carbon chain residue formed thereof.

10. The complex according to claim 4, wherein the water-soluble moiety is a molecule or a residue of the molecule containing one or two or more groups selected from the group consisting of a thiol group, amine group, carboxyl group, hydroxyl group, and disulfide group; the molecule is one or two or more water-soluble macromolecules selected from the group consisting of proteins, polysaccharides, nucleic acids, and artificially synthesized water-soluble polymers, or residues thereof;
and/or, one or two or more medium molecules selected from the group consisting of polypeptides, oligopeptides, oligosaccharides, oligonucleotides, and synthetic water-soluble medium molecular weight polymers, or residues thereof;
and/or, one or two or more water-soluble small molecules selected from the group consisting of amino acids, monosaccharides, disaccharides, nucleotides, water-soluble vitamins, and deoxynucleotides, or residues thereof;
and/or, a molecule or a residue thereof linked to the carbon chain as the acting moiety, and the molecule or residue thereof contains one or two or more groups selected from the group consisting of a thiol group,
amine group, carboxyl group, hydroxyl group, and disulfide group.

11. The complex according to claim 10, wherein the protein as the water-soluble macromolecule is one or two or more water-soluble macromolecules selected from the group consisting of serum albumins, immunoglobulins, water-soluble collagens, chaperones, water-soluble glycoproteins, and CD14; the polysaccharide as the macromolecule is one or two or more water-soluble macromolecules selected from the group consisting of dextran, hyaluronic acid, sialic acid, heparin sulfate, heparan sulfate, chondroitin sulfate, dermatan sulfate, keratan sulfate, acetyl water-soluble cellulose derivatives, β-cyclodextrin and derivatives thereof, and water-soluble chitosan derivatives; the water-soluble polymer as the macromolecule is one or two or more water-soluble macromolecules selected from the group consisting of polyethylene glycol and carboxylated or aminated polyethylene glycol, polyvinyl alcohol and carboxylated or quaternized polyvinyl alcohol, polyacrylic acid and ammonium polyacrylate;
the water-soluble medium molecular weight polymer is one or two or more substances selected from the group consisting of targeting polypeptides, oligopeptides, oligosaccharides, oligonucleotides and/or water-soluble polyamino acids;
monosaccharide and/or disaccharide as the water-soluble small molecular is one or two or more selected from the group consisting of glucose, fructose, rhamnose, sorbose, sucrose, maltose, lactose, and trehalose; the nucleotide and/or deoxynucleotide as the water-soluble small molecule is one or two or more selected from the group consisting of adenosine, guanylate, uranylate, cytidylate, thymidylate, inosine, deoxyadenosine, deoxyguanosine, deoxycytidylate, and deoxythymidylate; the amine acid as the water-soluble small molecule is one or two or more of amino acids such as serine, threonine, cysteine, asparagine, glutamine, tyrosine, lysine, arginine, histidine, aspartate, glutamate, citrulline, ornithine, taurine, and aminobutyric acid; the vitamin as the water-soluble small molecule is one or two or more selected from the group consisting of vitamin B1, pantothenic acid, vitamin B6, and vitamin C.

12. The complex according to claim 11, wherein the targeting polypeptide comprises any one of a protein or neutralizing antibody fragment that specifically targets a microbial lipid membrane, a bacterial and fungal cell wall, a virus surface protein domain.

13. The complex according to claim 11, wherein the water-soluble polyamino acid is selected from the group consisting of polyglutamate, polylysine, and/or polyaspartate.

14. The complex according to claim 1, wherein the binding moiety and the water-soluble moiety are the same, which is a protein, polypeptide, oligopeptide, oligosaccharide, monosaccharide, disaccharide, amino acid, nucleotide, vitamin, water-soluble polymer, water-soluble polyamino acid and/or polysaccharide molecule capable of binding to a lipid membrane, surface domain of a microorganism, or a residue thereof; the molecule or residue thereof comprises one or two or more groups selected from the group consisting of a thiol group, amine group, carboxyl group, hydroxyl group, and disulfide group.

15. The complex according to claim 1, wherein the complex is a compound obtained by the reaction of a saturated and/or unsaturated fatty acid with 3-100 carbon atoms with at least one selected from the group consisting of protein, polypeptide, oligopeptide, oligosaccharide, monosaccharide, disaccharide, nucleotide, vitamin, amino acid, water-soluble polymer, water-soluble polyamino acid, and polysaccharide molecule; or is a mixture of a compound obtained by the reaction of a saturated and/or unsaturated fatty acid with 3-50 carbon atoms with at least one selected from the group consisting of protein, polypeptide, oligopeptide, oligosaccharide, monosaccharide, disaccharide, nucleotide, vitamin, amino acid, water-soluble polymer, water-soluble polyamino acid, and polysaccharide molecule, and unreacted fatty acid and/or unreacted protein, polypeptide, oligopeptide, oligosaccharide, monosaccharide, disaccharide, nucleotide, vitamin, amino acid, water-soluble polymer, water-soluble polyamino acid, and/or polysaccharide molecule.

16. The complex according to claim 1, wherein the complex is a complex obtained by directly physically compounding a saturated and/or unsaturated fatty acid with 3-100 carbon atoms and at least one selected from the group consisting of protein, polypeptide, oligopeptide, oligosaccharide, monosaccharide, disaccharide, nucleotide, vitamin, amino acid, water-soluble polymer, water-soluble polyamino acid, and/or polysaccharide molecule, by a physicochemical action including hydrogen bond or van der Waals force or a combination thereof, or a mixture obtained by directly physical mixing the same.

17. The complex according to claim 15, wherein the complex is a compound obtained by the reaction of a saturated and/or unsaturated fatty acid with 3-100 carbon atoms with at least one selected from the group consisting of protein, polypeptide, oligopeptide and amino acid; or a mixture of the compound obtained by the reaction of the saturated and/or unsaturated fatty acid with 3-100 carbon atoms with at least one selected from the group consisting of protein, polypeptide, oligopeptide and amino acid, unreacted fatty acid, and/or unreacted at least one selected from the group consisting of protein, polypeptide, oligopeptide and amino acid.

18. The complex according to claim 15, wherein the complex is a compound obtained by the reaction of a saturated and/or unsaturated fatty acid with 3-100 carbon atoms, PEG, with at least one selected from the group consisting of protein, polypeptide, oligopeptide, and amino acid; or a mixture of the compound obtained by the reaction of the saturated and/or unsaturated fatty acid with 3-100 carbon atoms, and PEG, with at least one selected from the group consisting of protein, polypeptide, oligopeptide and amino acid, unreacted PEG, and/or an unreacted at least one selected from the group consisting of protein, polypeptide, oligopeptide, and amino acid.

19. The complex according to claim 15, wherein the complex is a compound obtained by the reaction of a saturated and/or unsaturated fatty acid with 3-100 carbon atoms with at least one selected from the group consisting of polysaccharide, monosaccharide, disaccharide, and oligosaccharide; or a mixture of the compound obtained by the reaction of the saturated and/or unsaturated fatty acid with 3-100 carbon atoms with at least one selected from the group consisting of polysaccharide, monosaccharide, disaccharide, and oligosaccharide, unreacted fatty acid and/or unreacted polysaccharide, monosaccharide, disaccharide, and/or oligosaccharide.

20. The complex according to claim 15, wherein the complex is a compound obtained by the reaction of a saturated and/or unsaturated fatty acid with 3-100 carbon atoms, PEG and at least one selected from the group consisting of polysaccharide, monosaccharide, disaccharide, and oligosaccharide; or a mixture of the compound obtained by the reaction of the saturated and/or unsaturated fatty acid with 3-100 carbon atoms, PEG and at least one selected from the group consisting of polysaccharide, monosaccharide, disaccharide, and oligosaccharide, unreacted fatty acids, unreacted PEG and/or unreacted polysaccharide, monosaccharide, disaccharide, and/or oligosaccharide.

21. The complex according to claim 15, wherein the protein is one or two or more selected from the group consisting of serum albumins, immunoglobulins, water-soluble collagens, chaperones, water-soluble glycoproteins, and CD14.

22. The complex according to claim 15, wherein the polysaccharide is one or two or more selected from the group consisting of dextran and/or hyaluronic acid, sialic acid, heparin sulfate, heparan sulfate, chondroitin sulfate, dermatan sulfate, keratan sulfate, acetyl water-soluble cellulose derivatives, β-cyclodextrin and derivative thereof, and water-soluble chitosan derivatives.

23. The complex according to claim 15, wherein the complex is a compound obtained by the reaction of a saturated and/or unsaturated fatty acid with 3-100 carbon atoms, a linker, and a thiol-containing protein; or a mixture of the compound obtained from the above reaction, unreacted fatty acid, unreacted linker, and/or unreacted thiol-containing protein; wherein the linker is one or two or more selected from the group consisting of an amino acid, succinic acid, butadienoic acid, glutaconic acid, hexaminodioic acid, carbamate, short peptide, N-hydroxybutenimide, polyethylene glycol, and derivatives of the above compounds.

24. The complex according to claim 23, wherein the complex is a compound obtained by the reaction of a saturated and/or unsaturated fatty acid with 3-100 carbon atoms, N-hydroxybutenimide and a thiol-containing protein; or a mixture of the compound obtained by the above reaction, unreacted fatty acid, unreacted N-hydroxybutenimide, and/or unreacted thiol-containing protein.

25. The complex according to claim 15, wherein the complex is a compound obtained by the reaction of a saturated and/or unsaturated fatty acid with 3-100 carbon atoms and cystamine with at least one selected from the group consisting of polysaccharide, monosaccharide, disaccharide, and oligosaccharide; or a mixture of a compound obtained by the reaction of a saturated and/or unsaturated fatty acid with 3-50 carbon atoms and cystamine with at least one selected from the group consisting of polysaccharide, monosaccharide, disaccharide, and oligosaccharide, unreacted fatty acid, unreacted at least one selected from the group consisting of polysaccharide, monosaccharide, disaccharide, and oligosaccharide, and/or unreacted cystamine.

26. The complex according to any one of claims 15-25, wherein the compound obtained by the reaction contains one or two or more of an amide group, ester group, thioether group, or ether group as a linking moiety between the water-soluble moiety and the acting moiety.

27. The complex according to any one of claims 4-25, wherein the number of carbon atoms of the saturated and/or unsaturated fatty acid is 3-50.

28. The complex according to claim 27, wherein the number of carbon atoms is 3-48.

29. The complex according to claim 27, wherein the number of carbon atoms is 3-26.

30. The complex according to any one of claims 4-25, wherein the saturated and/or unsaturated fatty acid is a fatty acid containing 3-40 carbon atoms, whtich is a fatty acid with 1-8 C=C double bonds, a fatty acid with 1-7 C=C double bonds, a fatty acid with 1-6 double bonds, a fatty acid with 1-5 double bonds, a fatty acid with 1-4 double bonds, a fatty acid with 1-3 double bonds, or a fatty acid with 1-2 double bonds.

31. The complex according to any one of claims 4-25, wherein the saturated and/or unsaturated fatty acid is fatty acids with 1-6 double bonds and 3-30 carbon atoms.

32. The complex according to any one of claims 4-25, wherein the number of carbon atoms of the saturated and/or unsaturated fatty acid is 3-30.

33. The complex according to any one of claims 4-25, wherein the saturated and/or unsaturated fatty acid is one or two or more fatty acids selected from the group consisting of fumaric acid, octanoic acid, glutaconic acid, hexanoic acid, nonanoic acid, dodecanoic acid, tetradecanoic acid, hexadecanoic acid, octadecanoic acid, eicosanoic acid, oleic acid, linoleic acid, linolenic acid, eicosapentaenoic acid, docosapentaenoic acid, docosahexaenoic acid, pentacosanoic acid, heptanoic acid, decanoic acid, dodecenoic acid, tetradecenoic acid, dotriacontahexaenoic acid, and octacosanoic acid.

34. The complex according to any one of claims 4-25, wherein the protein is human or bovine serum albumin, or CD14; alternatively, the polysaccharide is dextran and/or hyaluronic acid.

35. The complex according to claim 17, wherein the compound obtained by the reaction is a compound obtained by the reaction of a fatty acid and albumin or SBP1, and has any one or two of the following structural formulas:

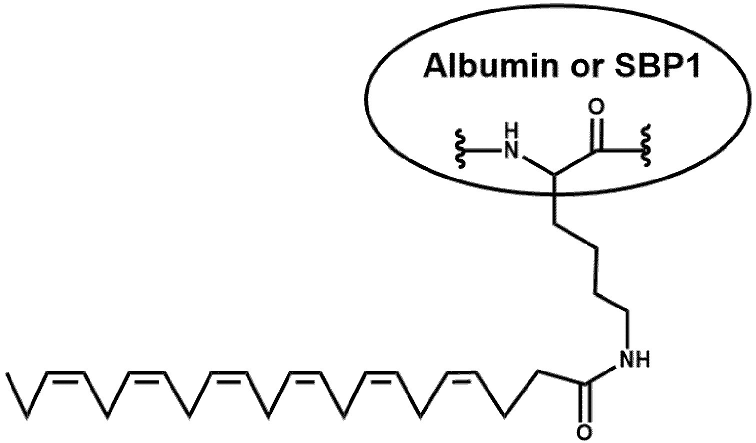

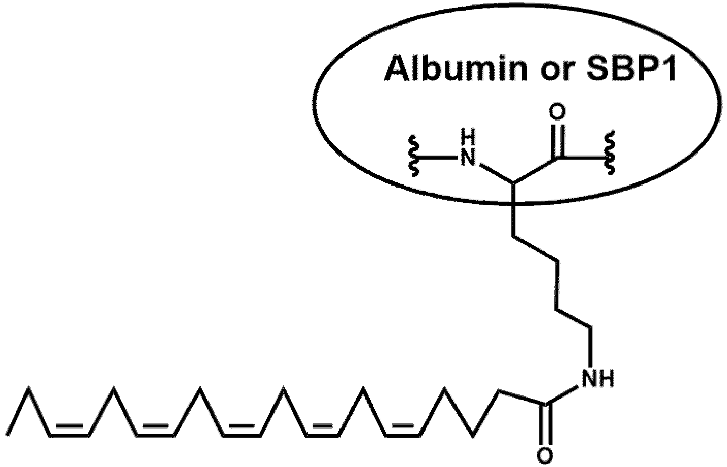

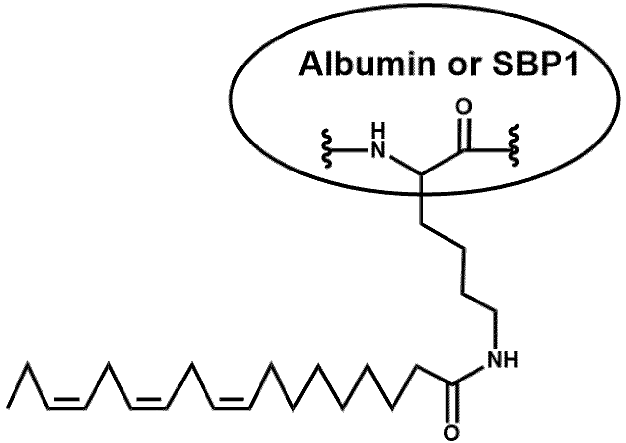

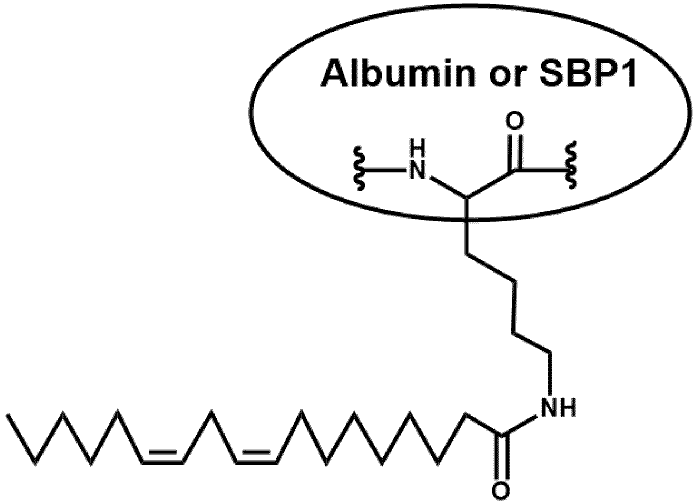

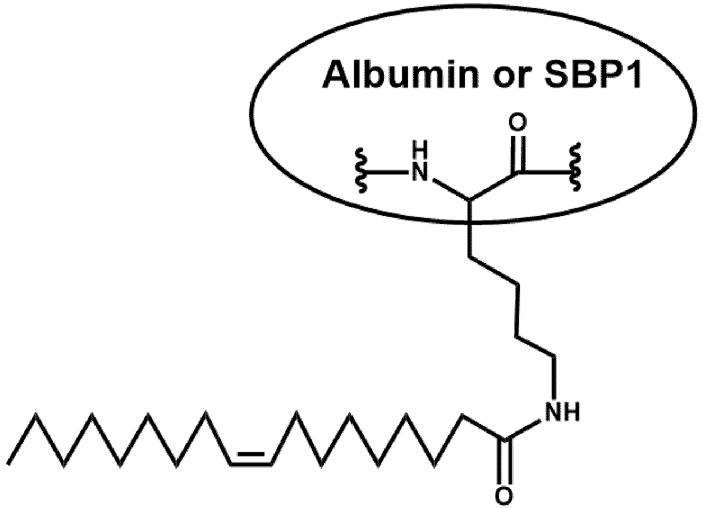

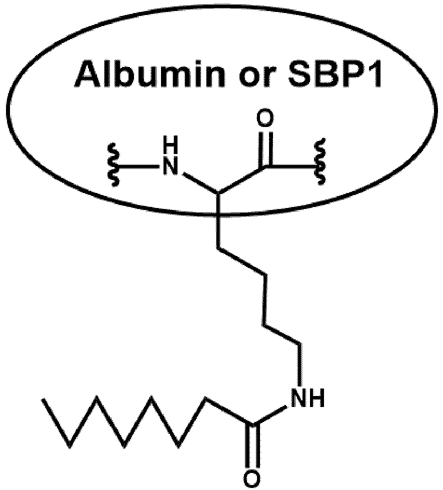

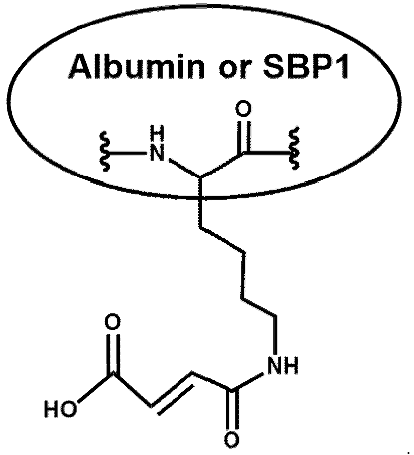

36. The complex according to claim 18, wherein the compound obtained by the reaction is a compound obtained by the reaction of a monobasic fatty acid with 3-10 carbon atoms, PEG, with an amino acid, or a compound obtained by the reaction of a monobasic fatty acid with 3-10 carbon atoms, a saturated dibasic fatty acid with 5-8 carbon atoms, and PEG with taurine.

37. The complex according to claim 36, wherein the compound obtained by the reaction is a compound having at least one of the following structural formulas: 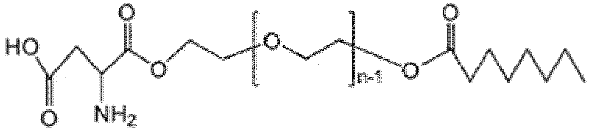 or 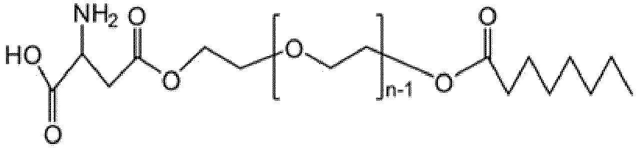 or 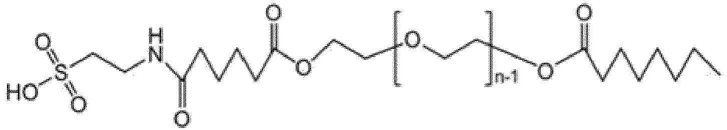 where n is an integer of 1-200.

38. The complex according to claim 19, wherein the compound obtained by the reaction is any one or two or more compounds that are obtained by the reaction of a fatty acid with dextran and have the following structural formulas:

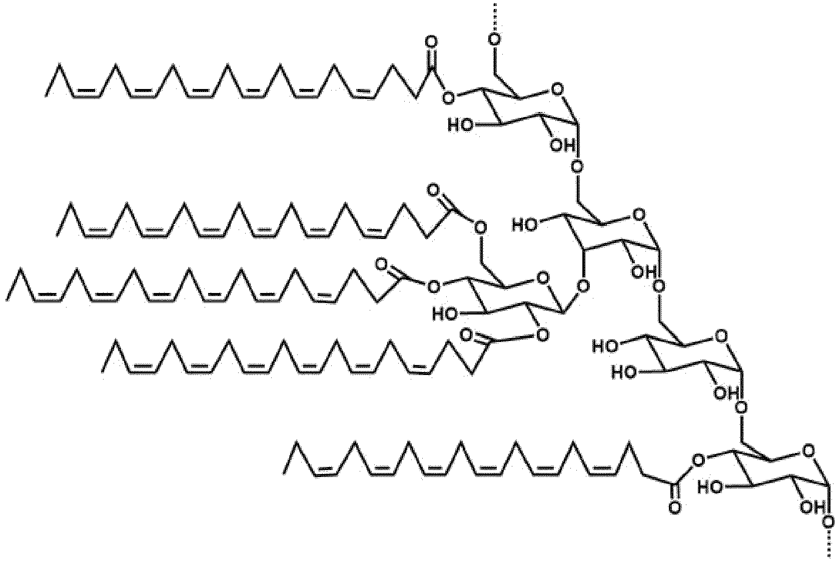

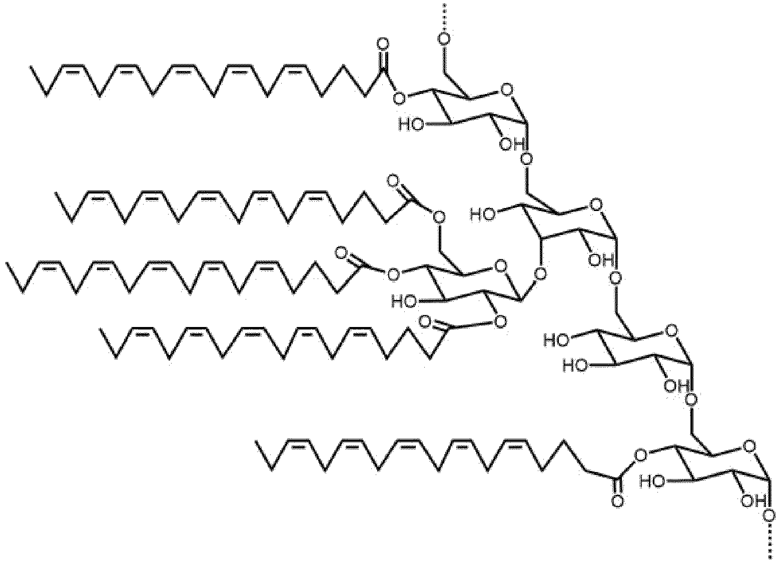

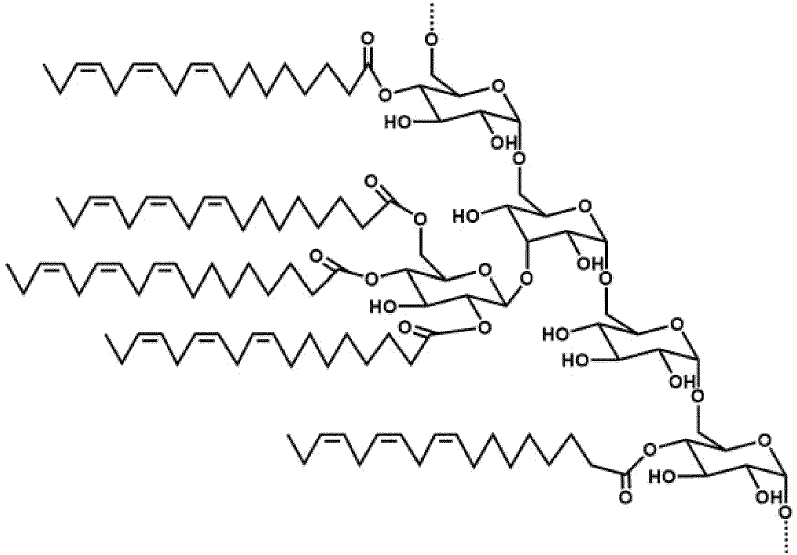

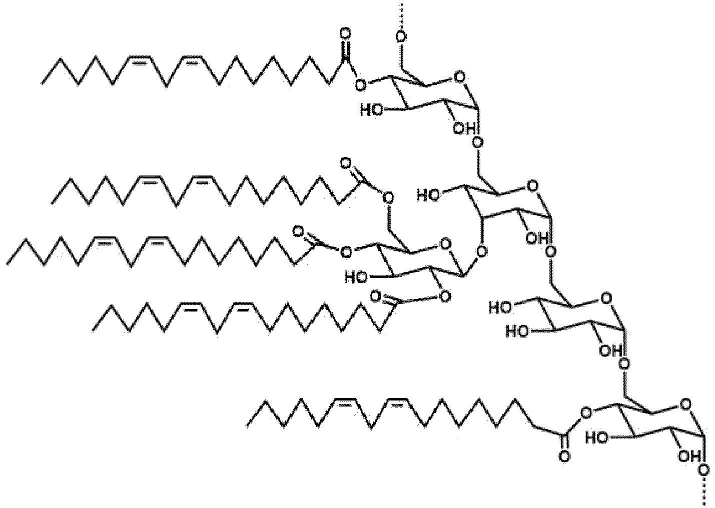

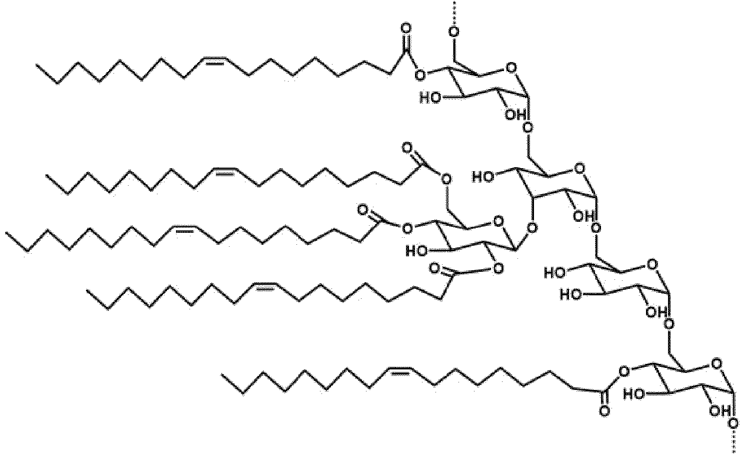

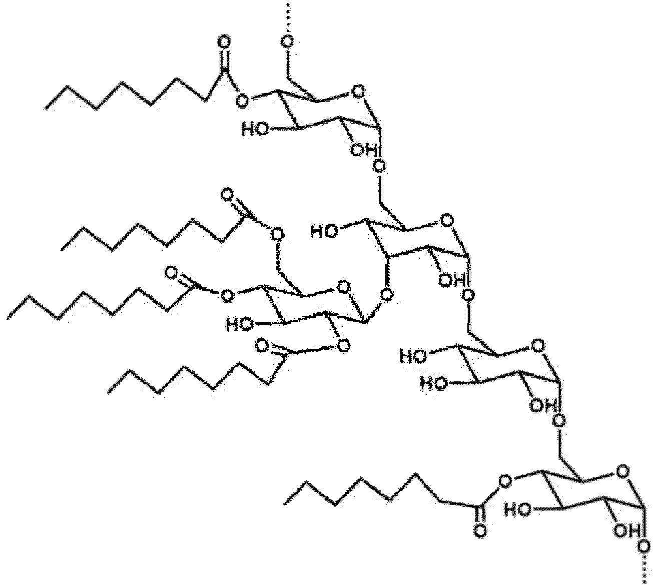

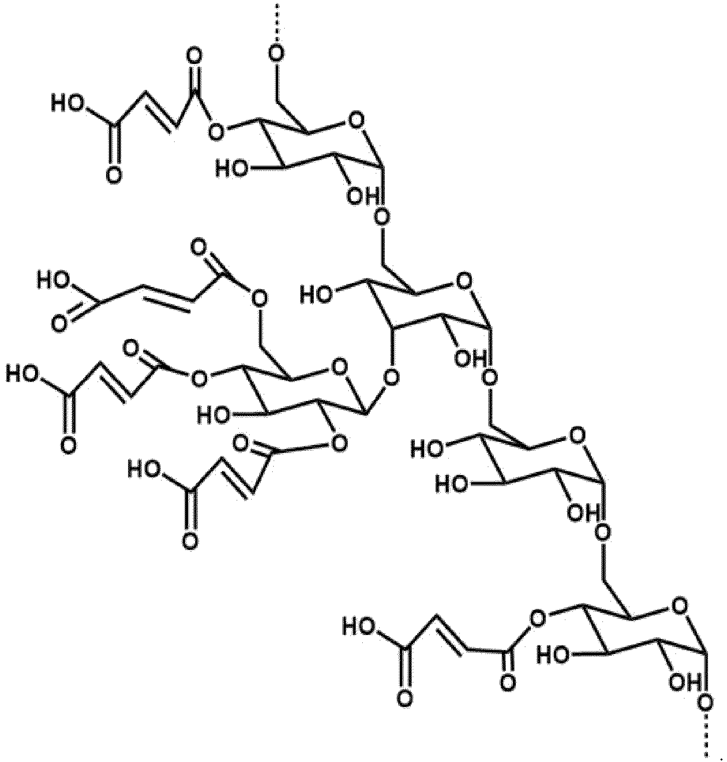

39. The complex according to claim 19, wherein the compound obtained by the reaction is any one or two or more compounds that are obtained by the reaction of a fatty acid with hyaluronic acid and have the following structural formulas: 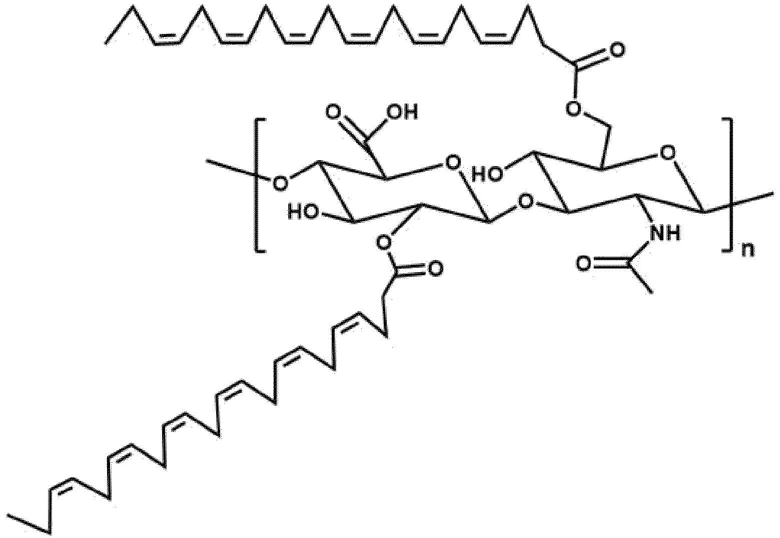 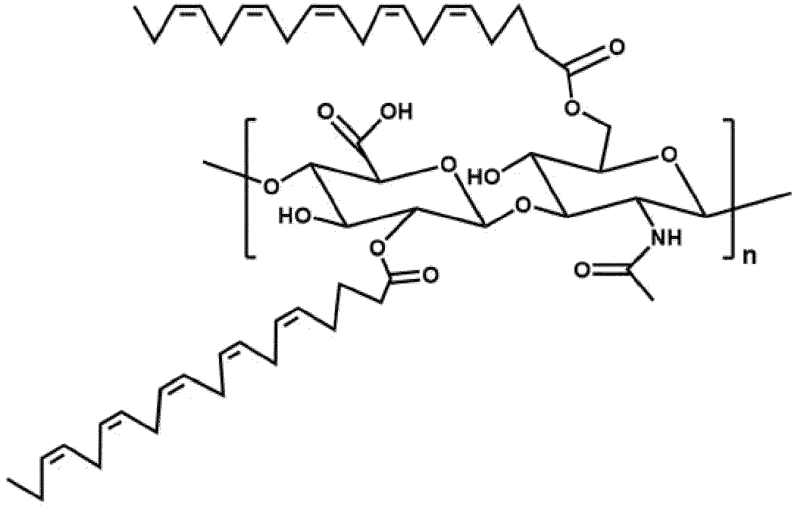 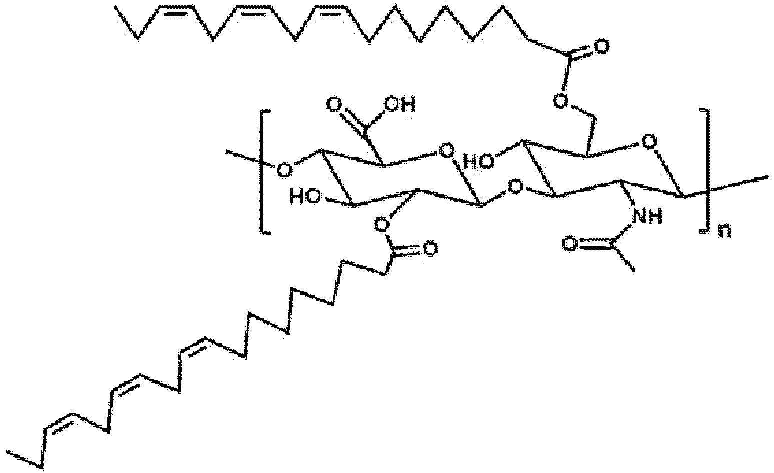 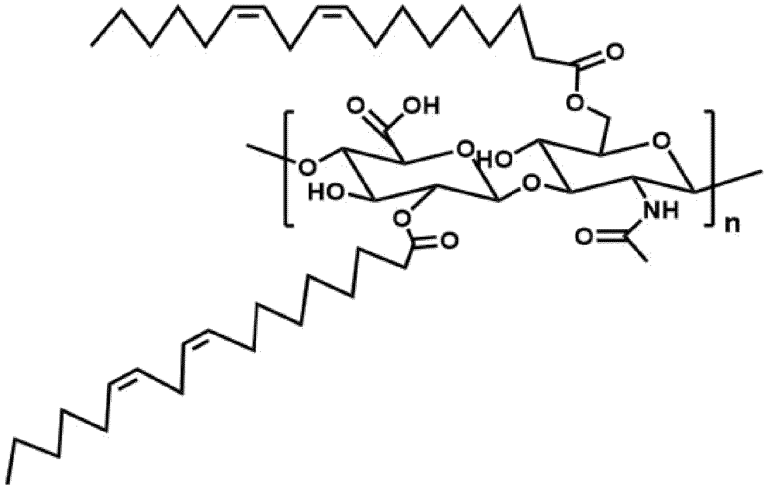 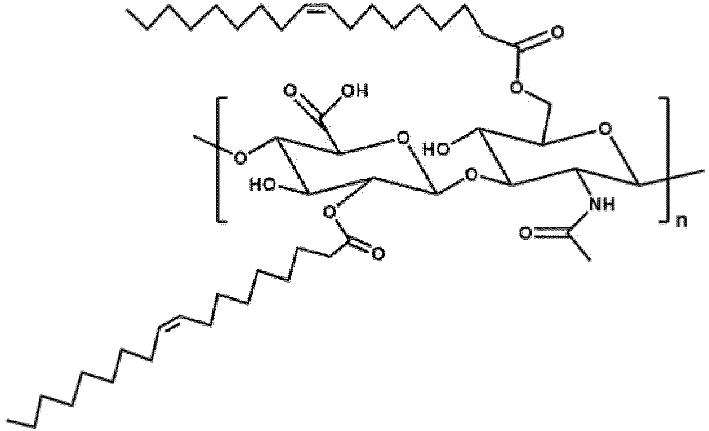 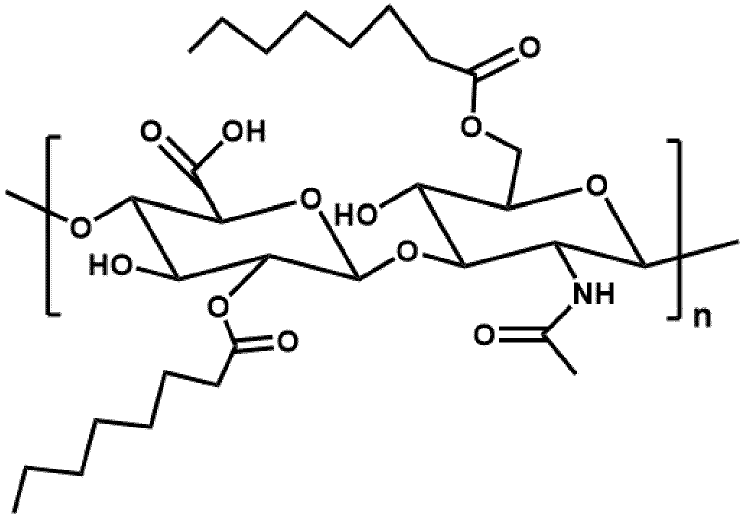 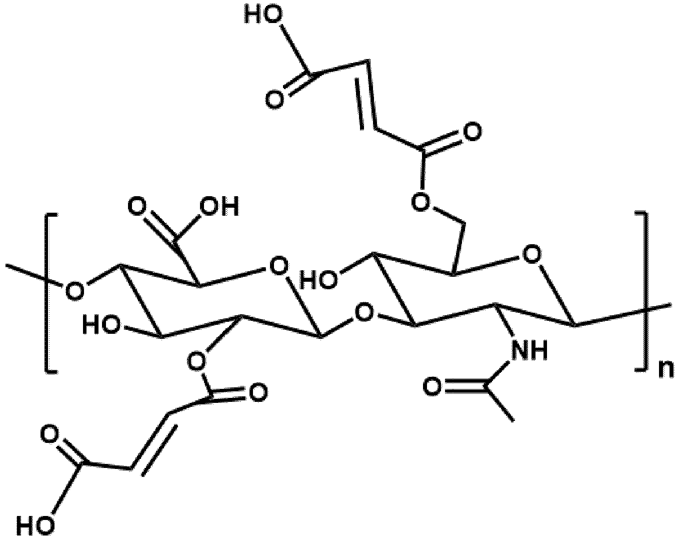 where n is an integer of 1-2000.

40. The complex according to claim 20, wherein the compound obtained by the reaction is a compound obtained by the reaction of a fatty acid with 3-10 carbon atoms with PEG and glucose.

41. The complex according to claim 40, the compound resulting from the reaction is a compound having the following structural formula: 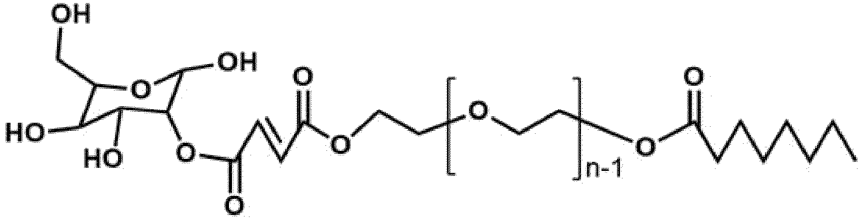 where n is an integer of 1-200.

42. The complex according to claim 23, wherein the compound obtained by the reaction is any one or two or more compounds having a thioether bond, that are obtained by the reaction of a fatty acid, N-hydroxybutenimide, and albumin and have the following structural formulas:

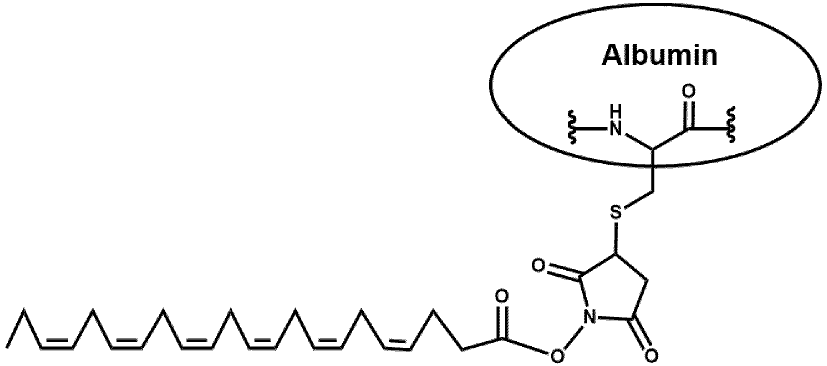

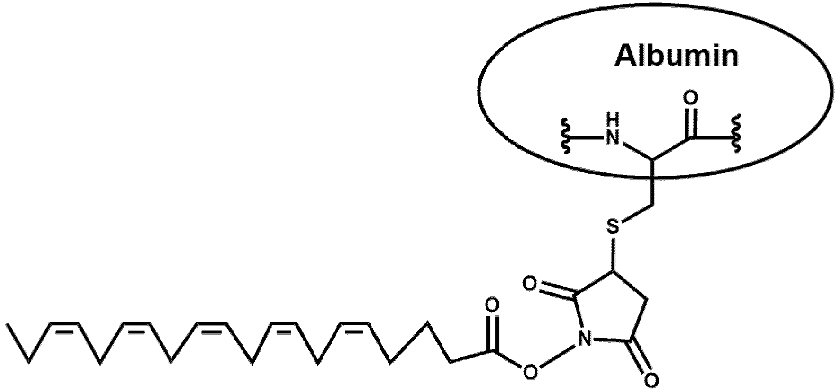

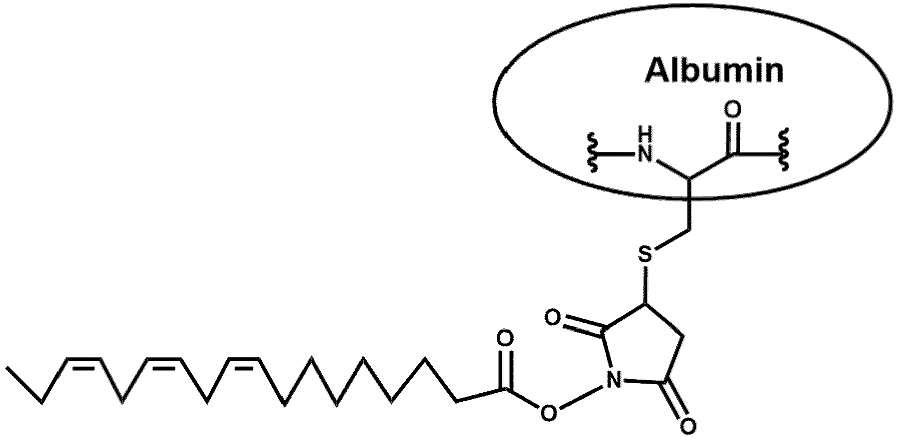

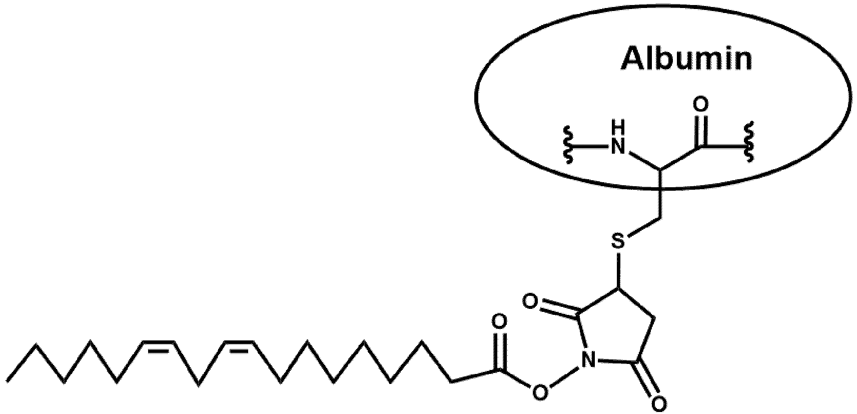

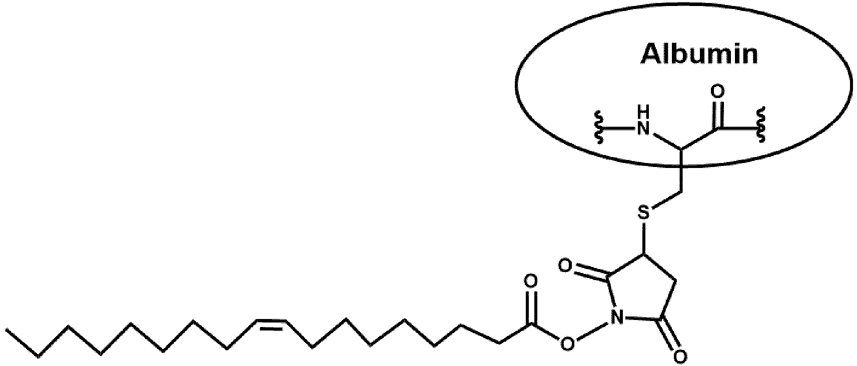

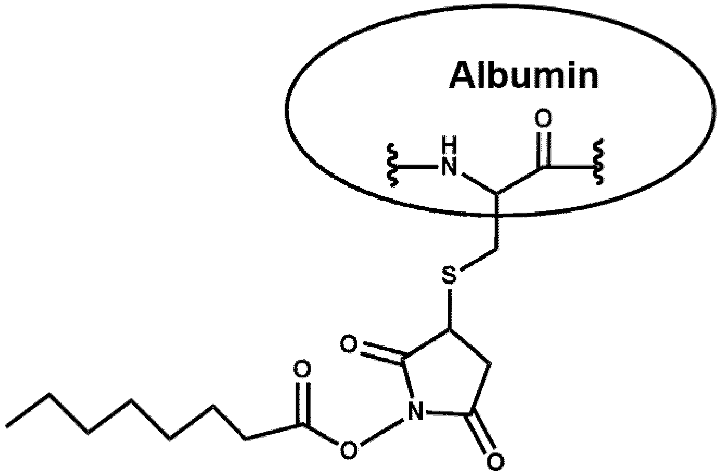

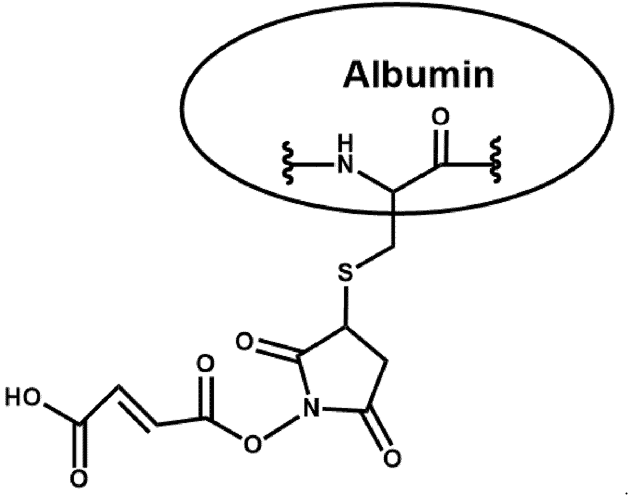

43. The complex according to claim 25, wherein the compound obtained by the reaction is any one or two or more compounds that are obtained by the reaction of a fatty acid, cystamine and dextran and have the following structural formulas:

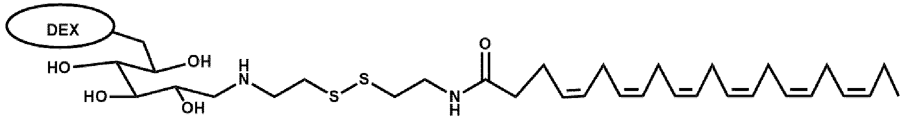

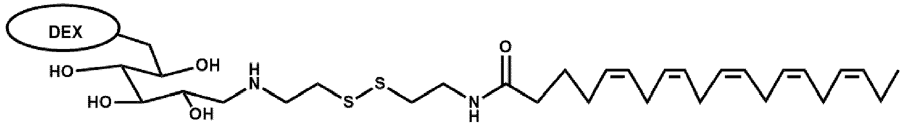

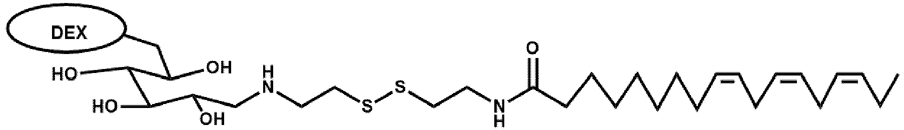

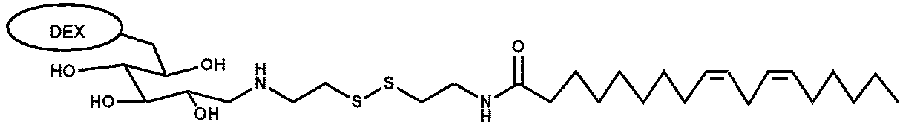

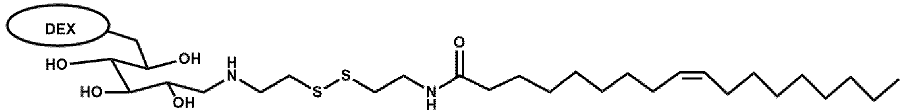

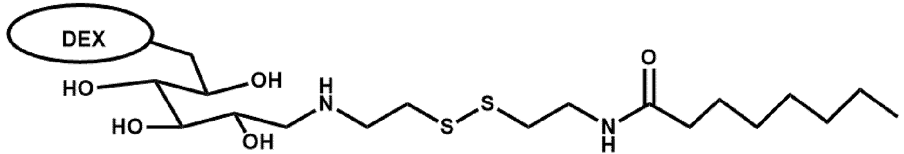

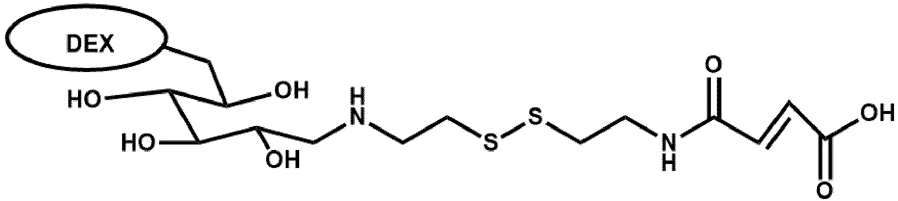

44. The complex according to claim 25, wherein the compound obtained by the reaction is any one or two or more compounds that obtained by the reaction of fatty acid, cystamine, and hyaluronic acid and have the following structural formulas:

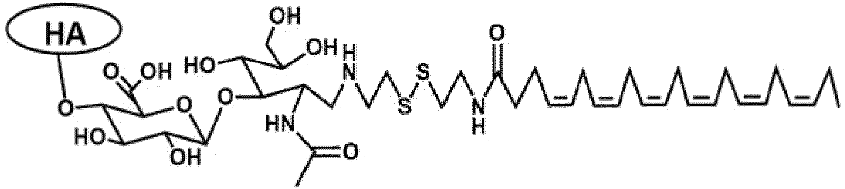

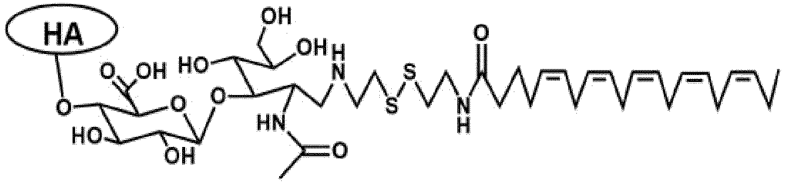

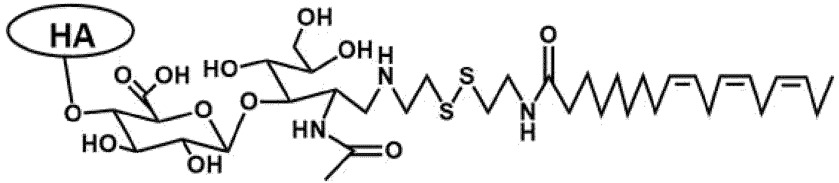

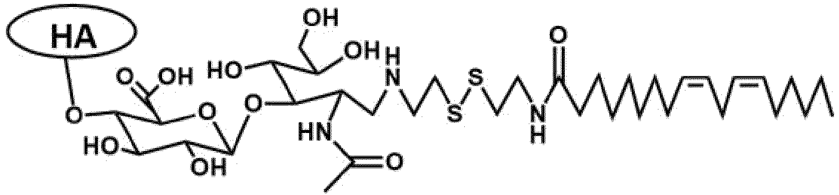

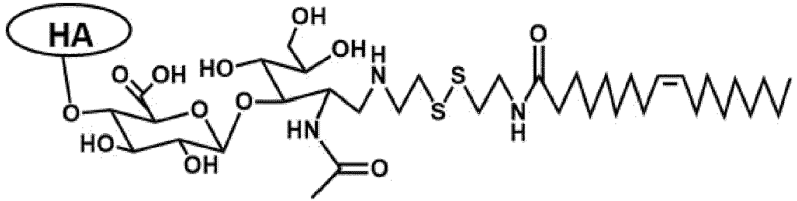

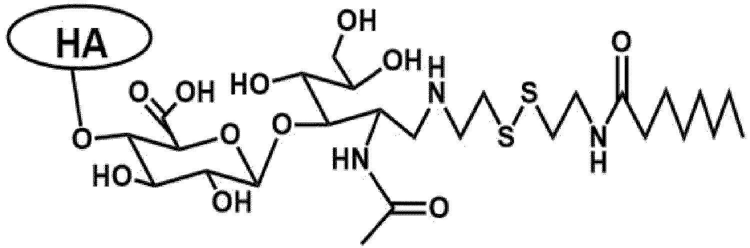

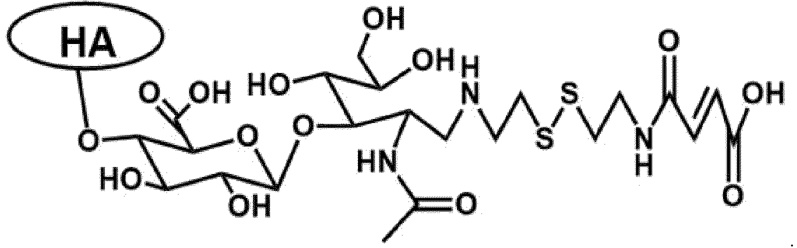

45. A preparation for preventing, blocking or treating microbial infections made using the complex according to any one of claims 1-44.

46. The preparation according to claim 45 being a pharmaceutical preparation or an environmental disinfection and sterilization preparation.

47. The preparation according to claim 46, wherein the pharmaceutical preparation is one selected from the group consisting of an inhalant, a nasal spray, an injection, an oral preparation, and a transdermal topical formulation.

48. Use of the complex according to any one of claims 1-44 in the preparation of a pharmaceutical preparation or environmental microbial disinfection and sterilization reagent for preventing, blocking and/or treating microorganism infections.

49. The use according to claim 48, wherein the microorganism is either or both selected from viruses and bacteria.

50. The use according to claim 49, wherein the virus is an enveloped virus; and/or a non-enveloped virus.

51. The use according to claim 49, wherein the virus is one or two or more viruses selected from the group consisting of novel coronavirus, influenza virus, AIDS virus (HIV), hepatitis B virus, human herpes virus, Ebola virus, rabies virus, and a human papilloma virus (HPV), and the bacteria is one or two or more bacteria selected from the group consisting of *Escherichia coli, Staphylococcus aureus,* methicillin-resistant *Staphylococcus aureus, Streptococcus pneumoniae, Klebsiella pneumoniae* and *Pseudomonas aeruginosa.*

52. The use according to claim 49, wherein the virus is one or more selected from the group consisting of H7N9 influenza virus, H5N1 influenza virus, HIV virus, novel coronavirus, HPV virus, and rabies virus.

53. A preparation method of the complex according to any one of claims 1-44, the complex being obtained by the reaction of a fatty acid having a fat-soluble saturated and/or unsaturated carbon chain with a branched, cyclic, and/or linear structure with a water-soluble molecule, and optionally added protein, polypeptide, amino acid, oligopeptide, oligosaccharide, monosaccharide, and/or polysaccharide molecule when needed capable of binding to a microbial lipid membrane, microorganism surface domain, or cell wall, and optionally added a linker molecule when needed, in the presence of a catalyst.

54. The preparation method of the complex according to claim 53, wherein the complex is a purified product of the compound obtained by the reaction.

55. A preparation method of the complex according to any one of claims 1-44, the complex being obtained by physical mixing of a fatty acid having a fat-soluble saturated and/or unsaturated carbon chain with a branched, cyclic, and/or linear structure with a water-soluble molecule, and optionally added protein, polypeptide, amino acid, oligopeptide, oligosaccharide, monosaccharide, and/or polysaccharide molecule when needed capable of binding to a microbial lipid membrane, virus surface domain, or cell wall.

56. A preparation method of the complex according to any one of claims 1-44, the complex being obtained by the reaction of a saturated and/or unsaturated fatty acid with 3-100 carbon atoms with any one selected from the group consisting of a protein, peptide, oligopeptide, oligosaccharide, monosaccharide, disaccharide, nucleotide, vitamin, amino acid, water-soluble polymer, water-soluble polyamino acid, and/or polysaccharide in the presence of a catalyst.

57. The preparation method of the complex according to claim 56, wherein the complex is a purified product of the compound obtained by the reaction.

58. A preparation method of the complex according to any one of claims 1-44, the complex being obtained by directly physical compounding a saturated and/or unsaturated fatty acid with 3-100 carbon atoms and at least one selected from the group consisting of protein, polypeptide, oligopeptide, oligosaccharide, monosaccharide, disaccharide, nucleotide, vitamin, amino acid, water-soluble polymer, water-soluble polyamino acid, and/or polysaccharide molecule, by a physicochemical action, or by directly physical mixing the same.

59. A water-soluble carbon chain substance for regulating transmembrane transport and/or fluidity of a cell membrane, comprising an acting moiety, a binding moiety, and a water-soluble moiety; the acting moiety is a fat-soluble saturated and/or unsaturated carbon chain with a branched, cyclic and/or linear structure; the carbon chain is a molecule or a residue of the molecule, and the carbon chain is a carbon chain with 3-100 carbon atoms; wherein the acting moiety is a carbon chain or residue of the carbon chain with 3-100 carbon atoms formed by any one or more of unsaturated fatty acids, fatty hydrocarbons, or cyclic hydrocarbons; or aromatic or heterocyclic compounds; or their salts, alcohols, ethers, esters, or other derivatives;
the water-soluble moiety is a water-soluble molecule or a residue of the molecule; the molecule contains one or two or more functional groups selected from the group consisting of amide group, phosphoryloxy group, carboxyl group, phosphate group, sulfonyl group, sulfonyloxy group, hydroxyl group, quaternary ammonium group, thioether group, disulfide bond, ether group, thiol group, aldehyde group, ester group, amine group, amino group, urea group, and guanidine group; and the water-soluble moiety can be one or two or more of the above functional groups linked to the carbon chain as the acting moiety;
the binding moiety is a molecule or a residue of the molecule capable of binding to a microbial lipid membrane, microorganism surface protein, microorganism surface polysaccharide, or a cell wall component, or binding to a polysaccharide, protein, or peptide in a microorganism, plant, animal, or human body, or a molecule or a residue of the molecule capable of binding to a cell membrane or a cell membrane surface polysaccharide or cell wall component in a plant, animal, or human body tissue; the binding moiety can be the same as the water-soluble moiety, which is the protein, peptide, amino acid, oligopeptide, oligosaccharide, monosaccharide, disaccharide, amino acid, nucleotide, vitamin, water-soluble polymer, water-soluble polymeric amino acid and/or polysaccharide molecule or their residues, capable of binding to a microbial lipid membrane, or to the surface domain of a cell membrane in a plant, animal, or human body tissue;
wherein the number of any one moiety of the acting moiety, the water-soluble moiety, and the binding moiety can be 1 or 2 or more.

60. The water-soluble carbon chain substance according to claim 59, wherein the number of carbon atoms is 3-50, preferably 3-48, and more preferably 3-26.

61. The water-soluble carbon chain substance according to claim 59, wherein the water-soluble moiety is a water-soluble molecule or a residue of the molecule of one or two or more groups selected from the group consisting of a thiol group, amino group, phosphate group, carboxyl group, sulfonyl group, hydroxyl group, amine group, urea group, guanidine group, and disulfide group;
the binding moiety has a group for binding, which is capable of binding to a microbial lipid membrane, microorganism surface protein, microorganism surface polysaccharide, or a cell wall component, or binding to a polysaccharide, protein, or peptide in a microorganism, plant, animal, or human body, or a molecule or a residue of the molecule capable of binding to a cell membrane or a cell membrane surface polysaccharide or cell wall component in a plant, animal, or human body tissue; the group is one or two or more groups from the water-soluble moiety or from groups independently as the binding moiety selected from the group consisting of a thiol group, amino group, phosphate group, carboxyl group, sulfonyl group, hydroxyl group, amine group, urea group, guanidine group, and disulfide group, or from one or two or more groups providing a carbon chain to carbon chain connection which is selected from the group consisting of a thiol group, amino group, phosphate group, carboxyl group, sulfonyl group, hydroxyl group, amine group, urea group, guanidine group, and disulfide group, such that the complex has one or two or more groups from thiol group, amino group, phosphate group, carboxyl group, sulfonyl group, hydroxyl group, amine group, urea group, guanidine group, and disulfide group.

62. The water-soluble carbon chain substance according to any one of claims 59-61, wherein the binding moiety is one or two or more selected from the group consisting of dibasic or polybasic fatty acid, amino acid, targeting protein, targeting peptide, and targeting polysaccharide,

63. The water-soluble carbon chain substance according to any one of claims 59-62, wherein the water-soluble carbon chain substance is a compound, complex, or mixture.

64. The water-soluble carbon chain substance according to claim 63, wherein the water-soluble carbon chain substance is a complex formed by linking a fatty acid with 3-50 carbon atoms and a water-soluble amino acid; or a complex formed by linking a fatty acid with 3-50 carbon atoms to the targeting polypeptide; or a complex formed by the reaction of a fatty acid with 3-50 carbon atoms and the targeting polypeptide with PEG; or a complex formed by the reaction of a surfactant and one or two or more selected from the group consisting of a dibasic or polybasic fatty acid, an amino acid, a targeting protein, a targeting polypeptide and a targeting polysaccharide.

65. The water-soluble carbon chain substance according to any one of claims 59-63, wherein the saturated and/or unsaturated fatty acid is selected from the group consisting of saturated fatty acids or unsaturated fatty acids with 3-50 carbon atoms; the fatty acid is a fatty acid or amino acid containing a double bond, triple bond, hydroxyl group, amino group and/or oxo group, and is a monobasic, dibasic, or polybasic acid.

66. The water-soluble carbon chain substance according to claim 65, wherein the saturated and/or unsaturated fatty acid is one or two or more selected from the group consisting of saturated fatty acids with 3-46 carbon atoms, monoenoic acids with 3-34 carbon atoms, dienoic acids with 5-30 carbon atoms, trienoic acids with 7-30 carbon atoms, tetraenoic acids with 12-38 carbon atoms, pentaenoic acids with 12-38 carbon atoms, hexaenoic acids with 22-38 carbon atoms, alkynoic acids with 6-22 carbon atoms, dialkynoic acids with 10-22 carbon atoms, trialkynoic acids with 12-22 carbon atoms, enynic acids with 8-20 carbon atoms, fatty acids with 3-30 carbon atoms in the main chain and 1-10 alkyl and/or 1-3 hydroxyl groups in the branches, saturated linear and branched dicarboxylic and tricarboxylic acids with 3-38 carbon atoms and unsaturated linear or branched dicarboxylic and tricarboxylic acids with 4-18 carbon atoms that is substitutable with hydroxyl groups, carboxylic acids with 3-18 carbon atoms substituted with amino, hydroxyl, oxo and/or methyl, N-fatty acyl amino acids with 6-30 carbon atoms, amino acids containing 2 or more fatty acyl groups, polybasic carboxylic acids linked by thioether and amide bonds.

67. The water-soluble carbon chain substance according to claim 65, wherein the saturated or unsaturated fatty acid is one or two or more selected from the group consisting of fumaric acid, octanoic acid, octenoic acid, glutaconic acid, hexanoic acid, octanedioic acid, nonanoic acid, dodecanoic acid, dodecanedioic acid, tridecaneoic acid, tridecanedioic acid, tetradecanoic acid, hexadecanoic acid, hexadecanedioic acid, octadecanoic acid, eicosanoic acid, eicosanedioic acid, oleic acid, linoleic acid, linolenic acid, eicosapentaenoic acid, docosenoic acid, docosapentaenoic acid, docosahexaenoic acid, pentacosanoic acid, heptanoic acid, decanoic acid, undecenoic acid, dodecenoic acid, tetradecenoic acid, hexadecenoic acid, triacontenoic acid, dotriacontahexaenoic acid, octacosanoic acid, or a carbon chain residue formed thereof.

68. The water-soluble carbon chain substance according to any one of claims 59-63, wherein the water-soluble moiety is a molecule or a residue of the molecule containing one or two or more groups selected from the group consisting of a thiol group, amine group, carboxyl group, hydroxyl group, and disulfide group; the molecule is one or two or more water-soluble macromolecules selected from the group consisting of proteins, polysaccharides, nucleic acids, and artificially synthesized water-soluble polymers, or residues thereof;
and/or, one or two or more medium molecules selected from the group consisting of polypeptides, oligopeptides, oligosaccharides, oligonucleotides, and synthetic water-soluble medium molecular weight polymers, or residues thereof;
and/or, one or two or more water-soluble small molecules selected from the group consisting of amino acids, monosaccharides, disaccharides, nucleotides, water-soluble vitamins, and deoxynucleotides, or residues thereof;
and/or, a molecule linked to the carbon chain as the acting moiety or a residue thereof, and the molecule or residue thereof contains one or two or more groups selected from the group consisting of a thiol group, amine group, carboxyl group, hydroxyl group, and disulfide group.

69. The water-soluble carbon chain substance according to claim 68, wherein the protein as the water-soluble macromolecule is one or two or more water-soluble macromolecules selected from the group consisting of serum albumins, immunoglobulins, water-soluble collagens, chaperones, water-soluble glycoproteins, and CD14; the polysaccharide as the macromolecule is one or two or more water-soluble macromolecules selected from the group consisting of dextran, hyaluronic acid, sialic acid, heparin sulfate, heparan sulfate, chondroitin sulfate, dermatan sulfate, keratan sulfate, acetyl water-soluble cellulose derivatives, β-cyclodextrin and derivatives thereof, and water-soluble chitosan derivatives; the water-soluble polymer as the macromolecule is one or two or more water-soluble macromolecules selected from the group consisting of polyethylene glycol and carboxylated or aminated polyethylene glycol, polyvinyl alcohol and carboxylated or quaternized polyvinyl alcohol, polyacrylic acid and ammonium polyacrylate; the water-soluble medium molecular weight polymer is one or two or more substances selected from the group consisting of targeting polypeptides, oligopeptides, oligosaccharides, oligonucleotides and/or water-soluble polyamino acids;
the water-soluble small molecular weight monosaccharide and/or disaccharide is one or two or more selected from the group consisting of glucose, fructose, rhamnose, sorbose, sucrose, maltose, lactose, and trehalose; the nucleotide and/or deoxynucleotide as the water-soluble small molecule is one or two or more selected from the group consisting of adenosine, guanylate, uranylate, cytidylate, thymidylate, inosine, deoxyadenosine, deoxyguanosine, deoxycytidylate, and deoxythymidylate; the amino acid as the water-soluble small molecule is one or two or more of amino acids such as serine, threonine, cysteine, asparagine, glutamine, tyrosine, lysine, arginine, histidine, aspartate, glutamate, citrulline, ornithine, taurine, and aminobutyric acid; the vitamin as the water-soluble small molecule is one or two or more selected from the group consisting of vitamin B1, pantothenic acid, vitamin B6, and vitamin C.

70. The water-soluble carbon chain substance according to claim 69, wherein the targeting polypeptide comprises any one of a protein or neutralizing antibody fragment that specifically targets a microbial lipid membrane, a bacterial and fungal cell wall, a virus surface protein domain.

71. The water-soluble carbon chain substance according to claim 69, wherein the water-soluble polyamino acid is selected from polyglutamate, polylysine, and/or polyaspartate.

72. The water-soluble carbon chain substance according to claim 59, wherein the binding moiety and the water-soluble moiety are the same, which is a protein, polypeptide, oligopeptide, oligosaccharide, monosaccharide, disaccharide, amino acid, nucleotide, vitamin, water-soluble polymer, water-soluble polyamino acid and/or polysaccharide molecule capable of binding to a microbial lipid membrane, a cell membrane or a cell membrane surface domain of an animal or human body tissue, or a residue thereof; the molecule or residue thereof comprises one or two or more groups selected from the group consisting of a thiol group, amine group, carboxyl group, hydroxyl group, and disulfide group.

73. The water-soluble carbon chain substance according to claim 59, wherein the water-soluble carbon chain substance is a compound obtained by the reaction of a saturated and/or unsaturated fatty acid with 3-100 carbon atoms with at least one selected from a group consisting of a protein, polypeptide, oligopeptide, oligosaccharide, monosaccharide, disaccharide, nucleotide, vitamin, amino acid, water-soluble polymer, water-soluble polyamino acid, and polysaccharide molecule; or is a mixture of the compound obtained by the reaction of the saturated and/or unsaturated fatty acid with 3-100 carbon atoms with at least one selected from the group consisting of protein, polypeptide, oligopeptide, oligosaccharide, monosaccharide, disaccharide, nucleotide, vitamin, amino acid, water-soluble polymer, water-soluble polyamino acid, and polysaccharide molecule, and unreacted fatty acid and/or unreacted protein, polypeptide, oligopeptide, oligosaccharide, monosaccharide, disaccharide, nucleotide, vitamin, amino acid, water-soluble polymer, water-soluble polyamino acid, and/or polysaccharide molecule.

74. The water-soluble carbon chain substance according to claim 59, wherein the water-soluble carbon chain substance is a complex obtained by directly physical compounding a saturated and/or unsaturated fatty acid with 3-100 carbon atoms and at least one selected from the group consisting of protein, polypeptide, oligopeptide, oligosaccharide, monosaccharide, disaccharide, nucleotide, vitamin, amino acid, water-soluble polymer, water-soluble polyamino acid, and/or polysaccharide molecule, by a physicochemical action including hydrogen bond or van der Waals force or a combination thereof, or a mixture obtained by directly physical mixing the same.

75. The water-soluble carbon chain substance according to claim 73, wherein the water-soluble carbon chain substance is a compound obtained by the reaction of a saturated and/or unsaturated fatty acid with 3-100 carbon atoms with at least one selected from the group consisting of protein, polypeptide, oligopeptide and amino acid; or a mixture of the compound obtained by the reaction of the saturated and/or unsaturated fatty acid with 3-100 carbon atoms with at least one selected from the group consisting of protein, polypeptide, oligopeptide and amino acid, unreacted fatty acid, and/or unreacted at least one selected from the group consisting of protein, polypeptide, oligopeptide and amino acid.

76. The water-soluble carbon chain substance according to claim 73, wherein the water-soluble carbon chain substance is a compound obtained by the reaction of a saturated and/or unsaturated fatty acid with 3-100 carbon atoms, and PEG, with at least one selected from the group consisting of protein, polypeptide, oligopeptide, and amino acid; or a mixture of the compound obtained by the reaction of the saturated and/or unsaturated fatty acid with 3-100 carbon atoms, PEG, with at least one selected from the group consisting of protein, polypeptide, oligopeptide and amino acid, unreacted PEG, and/or an unreacted at least one selected from the group consisting of protein, polypeptide, oligopeptide and amino acid.

77. The complex according to claim 73, wherein the complex is a compound obtained by the reaction of a saturated and/or unsaturated fatty acid with 3-100 carbon atoms with at least one selected from the group consisting of polysaccharide, monosaccharide, disaccharide and oligosaccharide; or a mixture of the compound obtained by the reaction of the saturated and/or unsaturated fatty acid with 3-100 carbon atoms with at least one selected from the group consisting of polysaccharide, monosaccharide, disaccharide and oligosaccharide, unreacted fatty acid and/or unreacted polysaccharide, monosaccharide, disaccharide and/or oligosaccharide.

78. The water-soluble carbon chain substance according to claim 73, wherein the water-soluble carbon chain substance is a compound obtained by the reaction of a saturated and/or unsaturated fatty acid with 3-100 carbon atoms, PEG and at least one selected from the group consisting of polysaccharide, monosaccharide, disaccharide, and oligosaccharide; or a mixture of the compound obtained by the reaction of the saturated and/or unsaturated fatty acid with 3-100 carbon atoms, PEG and at least one soelected from polysaccharide, monosaccharide, disaccharide and oligosaccharide, unreacted fatty acids, unreacted PEG and/or unreacted polysaccharide, monosaccharide, disaccharide and/or oligosaccharide.

79. The water-soluble carbon chain substance according to claim 73 or 74, wherein the protein is one or two or more selected from the group consisting of serum albumins, immunoglobulins, water-soluble collagens, chaperones, water-soluble glycoproteins and CD14.

80. The water-soluble carbon chain substance according to claim 73 or 74, wherein the polysaccharide is one or two or more selected from the group consisting of dextran and/or hyaluronic acid, sialic acid, heparin sulfate, heparan sulfate, chondroitin sulfate, dermatan sulfate, keratan sulfate, acetyl water-soluble cellulose derivatives, β-cyclodextrin and derivative thereof, and water-soluble chitosan derivatives.

81. The water-soluble carbon chain substance according to claim 73, wherein the water-soluble carbon chain substance is a compound obtained by the reaction of a saturated and/or unsaturated fatty acid with 3-100 carbon atoms, a linker, and a thiol-containing protein; or a mixture of the compound obtained from the above reaction, unreacted fatty acid, unreacted linker, and/or unreacted thiol-containing protein; wherein the linker is one or two or more of an amino acid, succinic acid, butadienoic acid, glutaconic acid, hexaminodioic acid, carbamate, short peptide, N-hydroxybutenimide, polyethylene glycol, and derivatives of the above compounds.

82. The water-soluble carbon chain substance according to claim 81, wherein the water-soluble carbon chain substance is a compound obtained by the reaction of a saturated and/or unsaturated fatty acid with 3-100 carbon atoms, N-hydroxybutenimide and a thiol-containing protein; or a mixture of the compound obtained by the above reaction, unreacted fatty acid, unreacted N-hydroxybutenimide, and/or unreacted thiol-containing protein.

83. The water-soluble carbon chain substance according to claim 73, wherein the water-soluble carbon chain substance is a compound obtained by the reaction of a saturated and/or unsaturated fatty acid with 3-100 carbon atoms, cystamine and at least one selected from the group consisting of polysaccharide, monosaccharide, disaccharide and oligosaccharide; or a mixture of a compound obtained by the reaction of a saturated and/or unsaturated fatty acid with 3-50 carbon atoms, cystamine and at least one selected from the group consisting of polysaccharide, monosaccharide, disaccharide and oligosaccharide, unreacted fatty acid, at least one unreacted substantce selected from the group consisting of polysaccharide, monosaccharide, disaccharide and oligosaccharide, and/or unreacted cystamine.

84. The water-soluble carbon chain substance according to any one of claims 73-83, wherein the compound obtained by the reaction contains one or two or more of an amide group, ester group, thioether group, or ether group as a linking moiety between the water-soluble moiety and the acting moiety.

85. The water-soluble carbon chain substance according to any one of claims 59-84, wherein the number of carbon atoms in the saturated and/or unsaturated fatty acids is 3-50, preferably 3-48, more preferably 3-30, and even more preferably 3-26.

86. The water-soluble carbon chain substance of any one according to claims 73-84, wherein the saturated and/or unsaturated fatty acid is a fatty acid with 3-40 carbon atoms, which is a fatty acid with 1-8 C=C double bonds, a fatty acid with 1-7 C=C double bonds, a fatty acid with 1-6 double bonds, a fatty acid with 1-5 double bonds, a fatty acid with 1-4 double bonds, a fatty acid with 1-3 double bonds, or a fatty acid with 1-2 double bonds; preferably, the saturated and/or unsaturated fatty acid is a fatty acid with 1-6 double bonds and 3-30 carbon atoms.

87. The water-soluble carbon chain substance according to any one of claims 59-84, wherein the number of carbon atoms of the saturated and/or unsaturated fatty acid is 3-30.

88. The water-soluble carbon chain substance according to any one of claims 59-84, wherein the saturated and/or unsaturated fatty acid is one or two or more fatty acid selected from the group consisting of fumaric acid, octanoic acid, octenoic acid, glutaconic acid, hexanoic acid, octanedioic acid, nonanoic acid, dodecanoic acid, dodecanedioic acid, tridecaneoic acid, tridecanedioic acid, tetradecanoic acid, hexadecanoic acid, hexadecanedioic acid, octadecanoic acid, eicosanoic acid, eicosanedioic acid, oleic acid, linoleic acid, linolenic acid, eicosapentaenoic acid, docosenoic acid, docosapentaenoic acid, docosahexaenoic acid, pentacosanoic acid, heptanoic acid, decanoic acid, undecenoic acid, dodecenoic acid, tetradecenoic acid, hexadecenoic acid, triacontenoic acid, dotriacontahexaenoic acid, and octacosanoic acid.

89. The water-soluble carbon chain substance according to any one of claims 59-84, wherein the protein is human or bovine serum albumin, or CD14; alternatively, the polysaccharide is dextran and/or hyaluronic acid.

90. The water-soluble carbon chain substance according to claim 75, wherein the compound obtained by the reaction is a compound obtained by the reaction of a fatty acid and albumin or SBP1 and has any one or two of the following structural formulas:

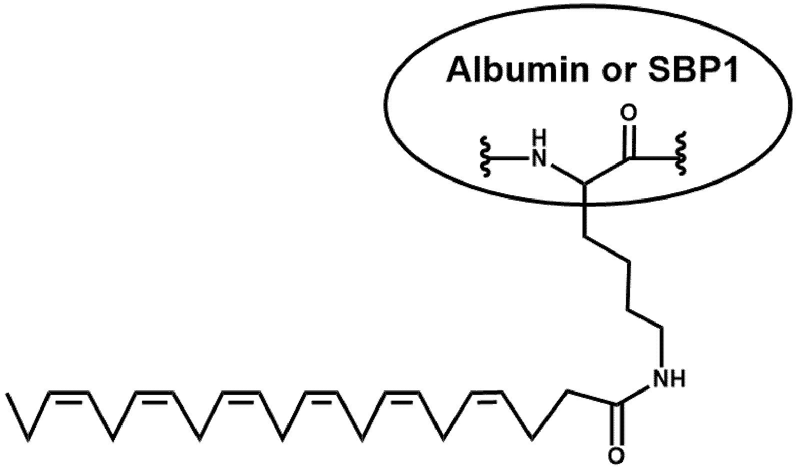

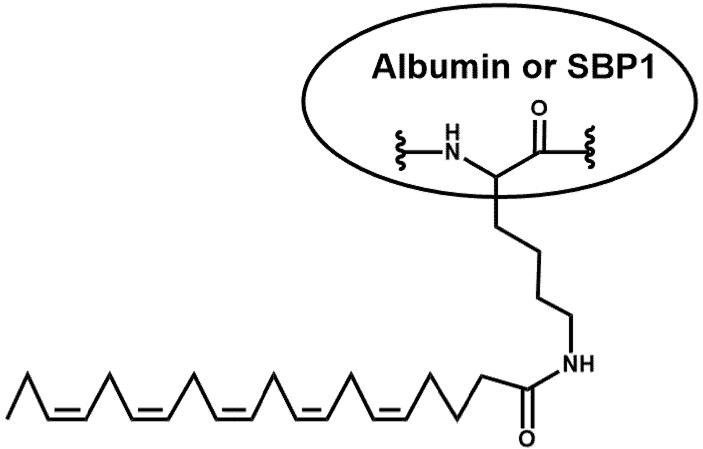

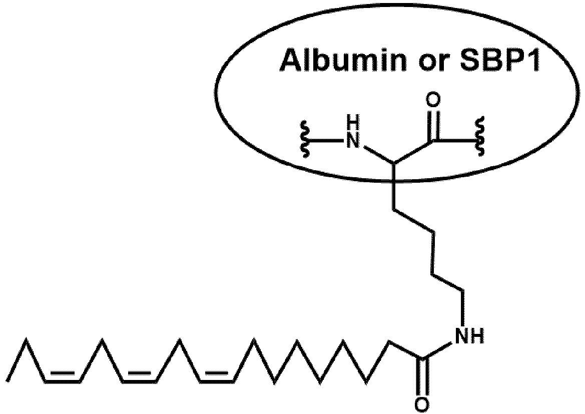

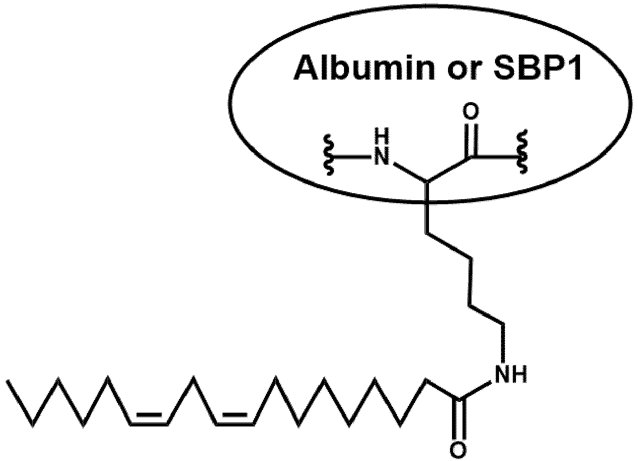

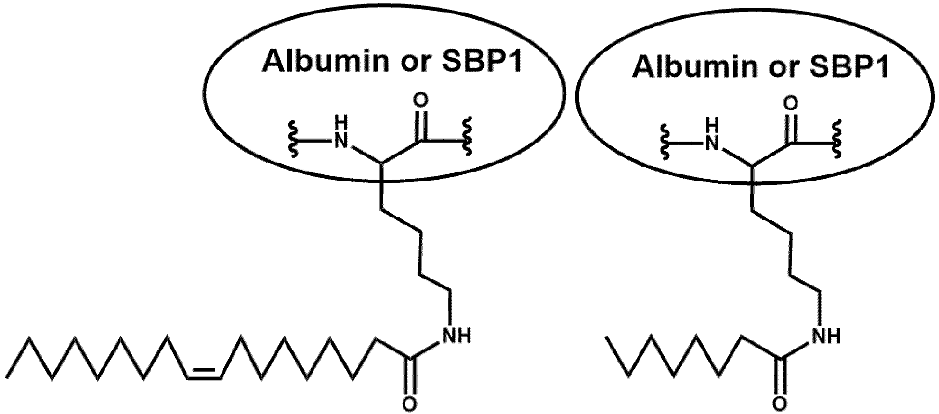

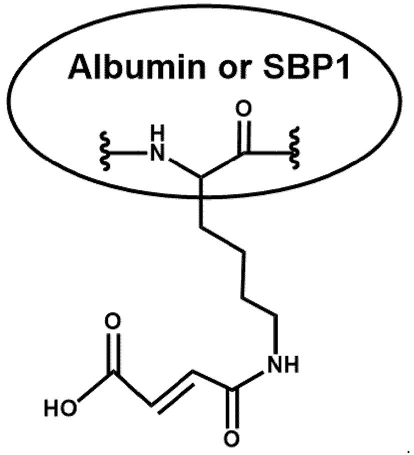

91. The water-soluble carbon chain substance according to claim 76, wherein the compound obtained by the reaction is a compound obtained by the reaction of a monobasic fatty acid with 3-10 carbon atoms , PEG and an amino acid, or a compound obtained by the reaction of a monobasic fatty acid with 3-10 carbon atoms, a saturated dibasic fatty acid with 5-8 carbon atoms and PEG with taurine.

92. The water-soluble carbon chain substance according to claim 91, wherein the compound obtained by the reaction is a compound having at least one of the following structural formulas: 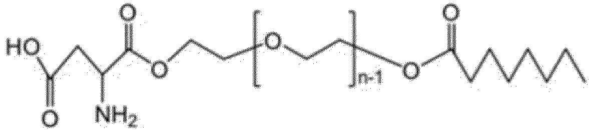 or 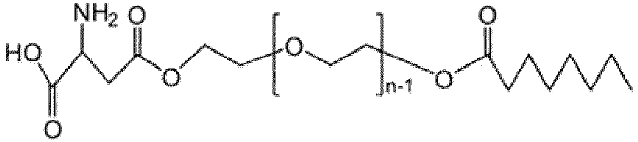 or 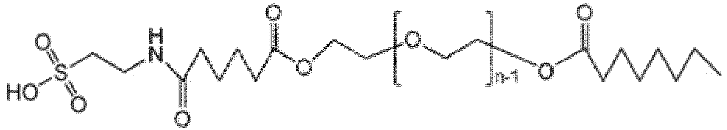 where n is an integer of 1-200.

93. The water-soluble carbon chain substance according to claim 77, wherein the compound obtained by the reaction is any one or two or more compounds that are obtained by the reaction of a fatty acid with dextran and have the following structural formulas:

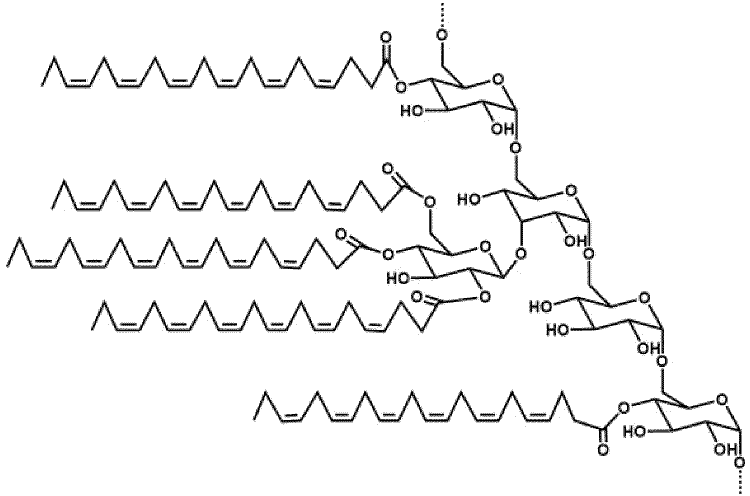

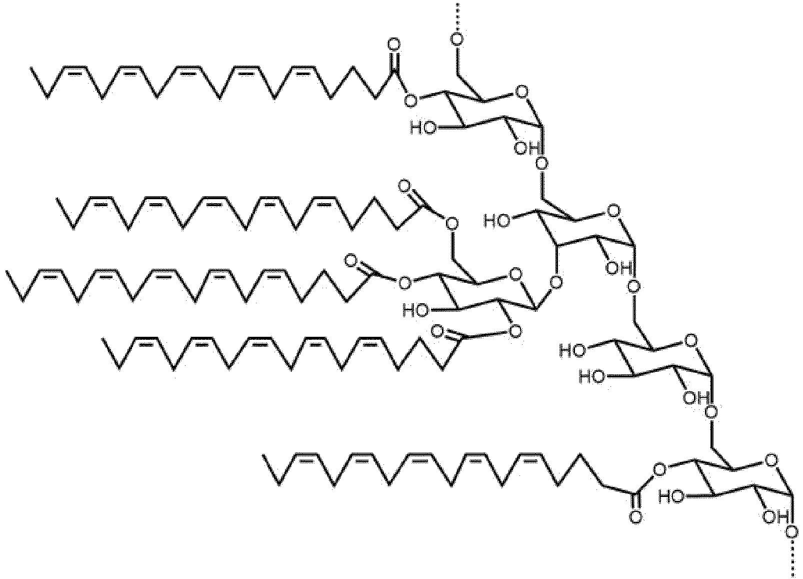

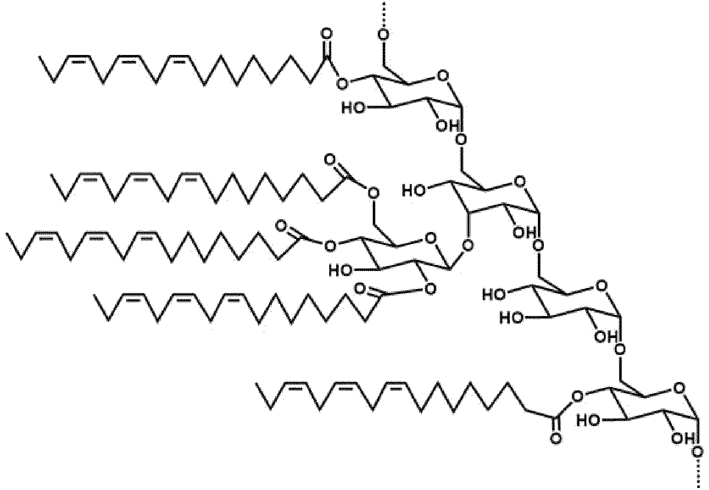

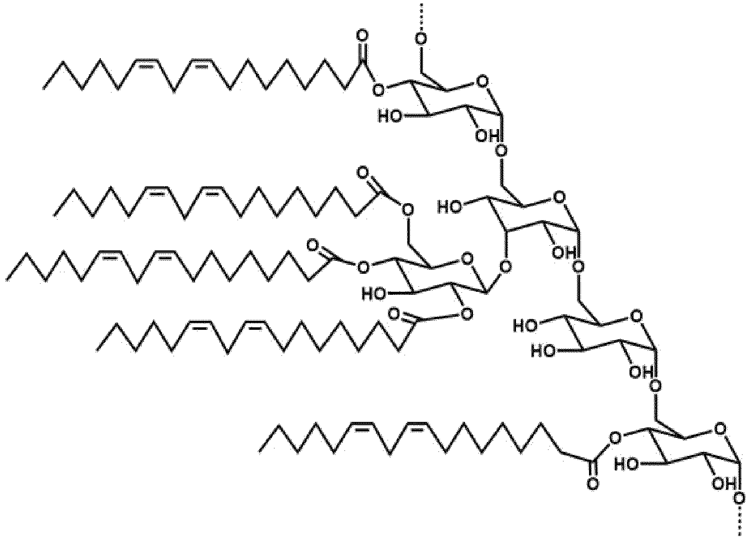

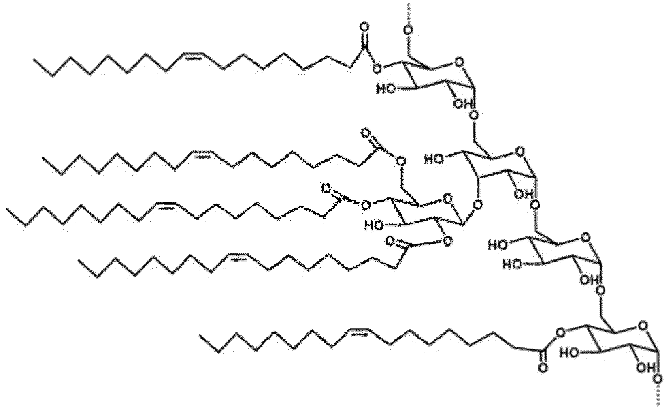

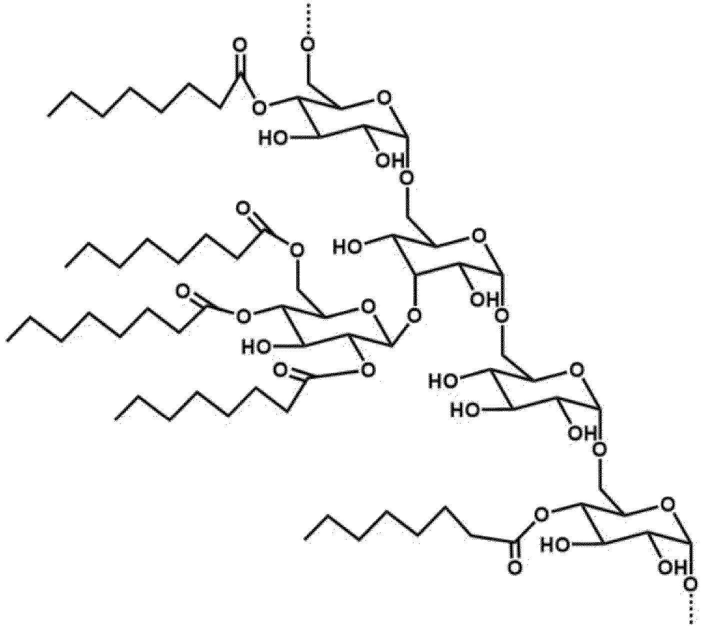

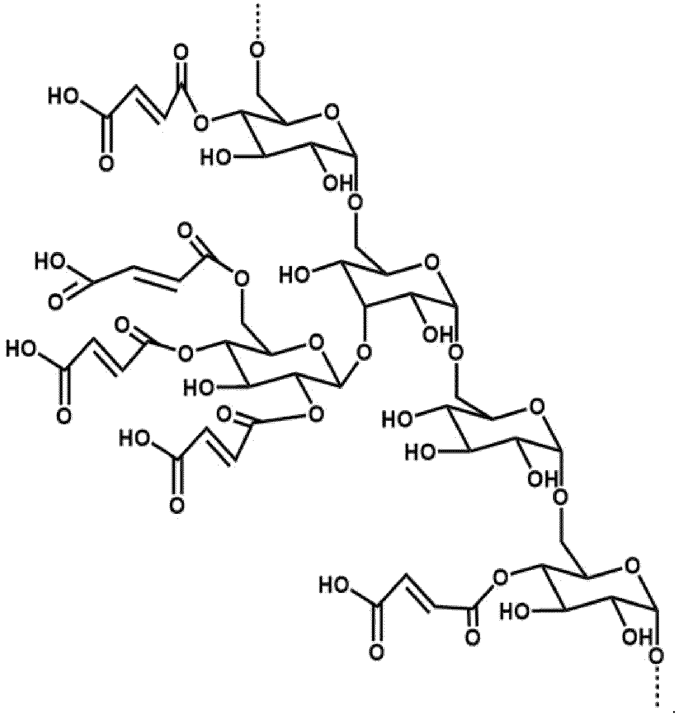

94. The water-soluble carbon chain substance according to claim 77, wherein the compound obtained by the reaction is any one or two or more compounds that are obtained by the reaction of a fatty acid with hyaluronic acid and have the following structural formulas: 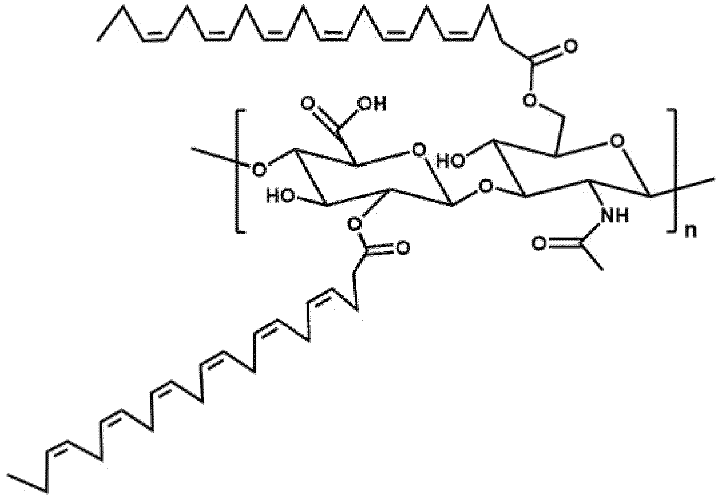 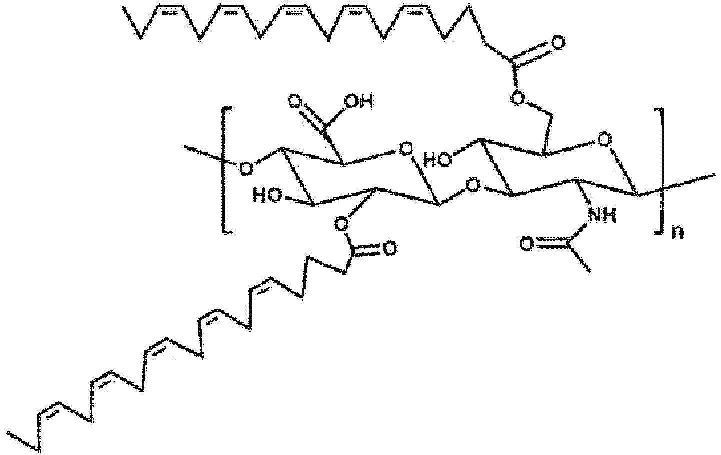 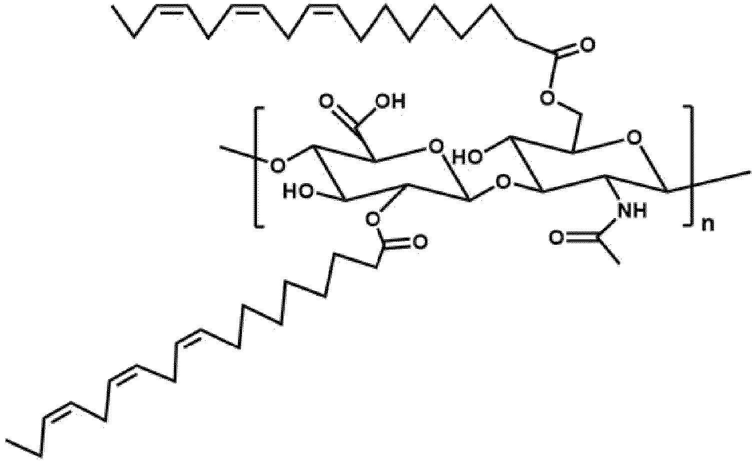 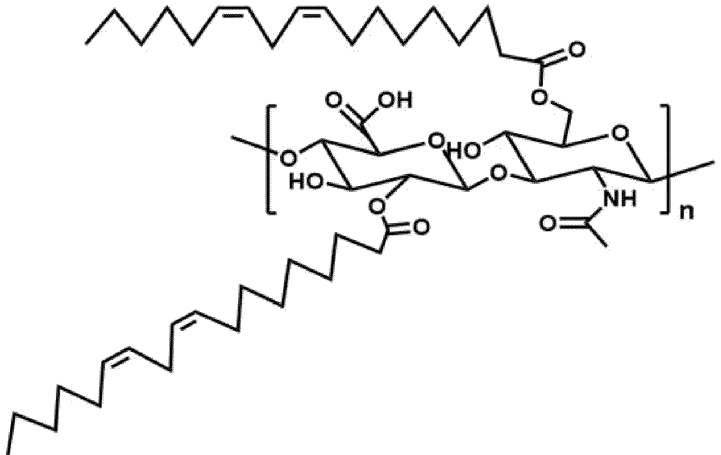 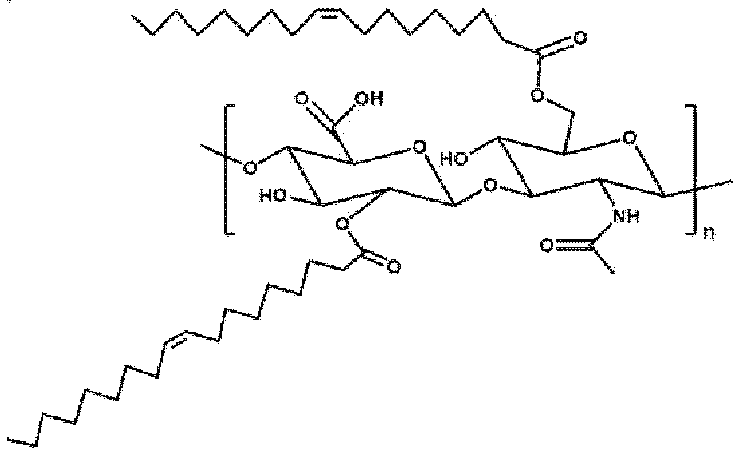 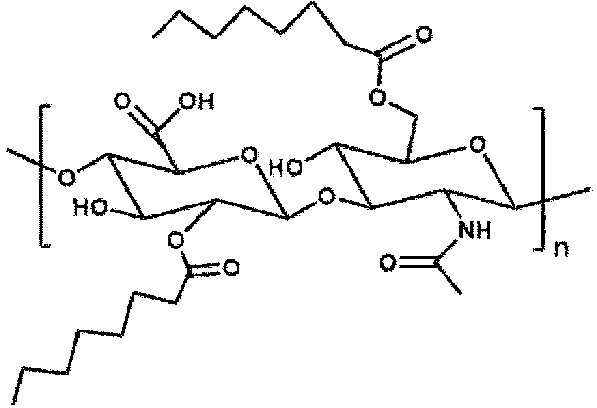 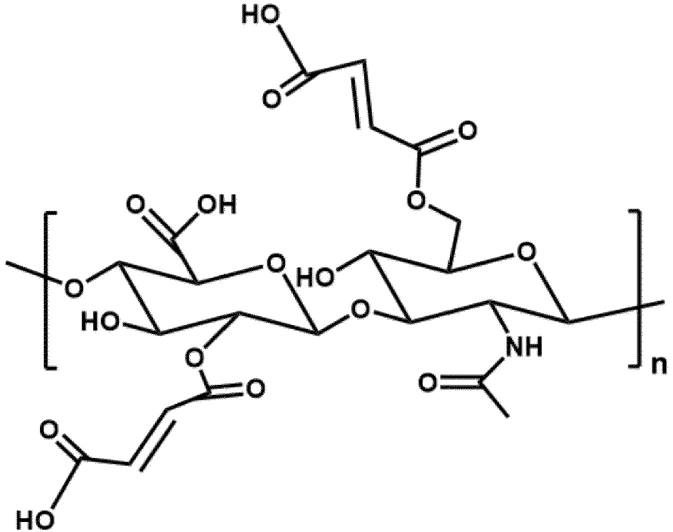 where n is an integer of 1-2000.

95. The water-soluble carbon chain substance according to claim 78, wherein the compound obtained by the reaction is a compound obtained by the reaction of a fatty acid with 3-10 carbon atoms , PEG and glucose.

96. The water-soluble carbon chain substance according to claim 95, wherein the compound obtained by the reaction is a compound having the following structural formula: 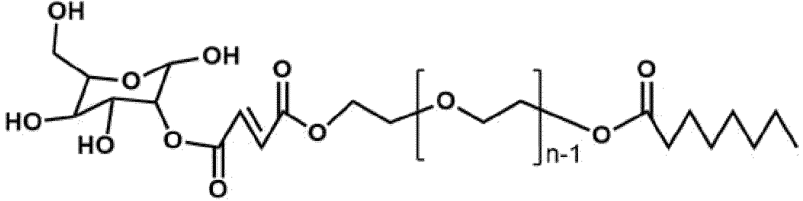 where n is an integer of 1-200.

97. The water-soluble carbon chain substance according to claim 82, wherein the compound obtained by the reaction is any one or two or more compounds having a thioether bond, that are obtained by the reaction of a fatty acid, N-hydroxybutenimide and a protein and have the following structural formulas:

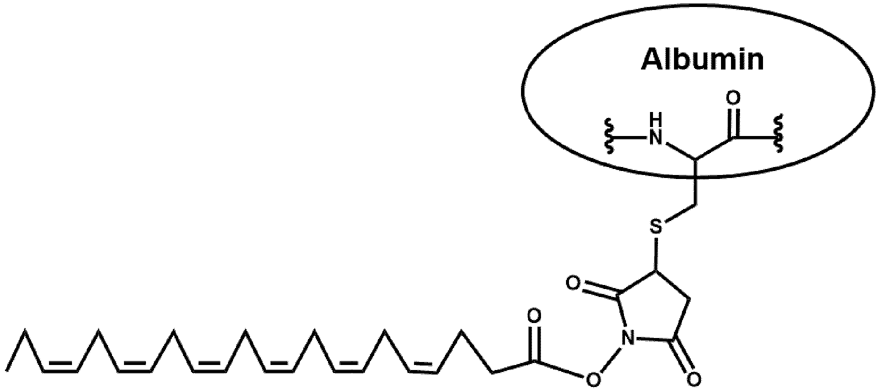

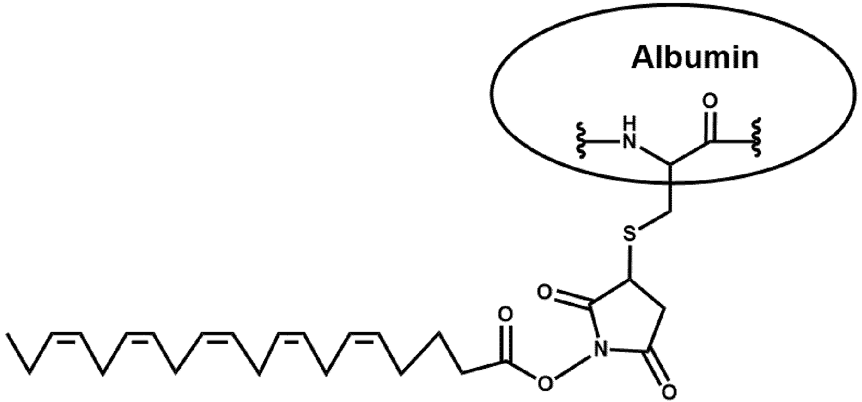

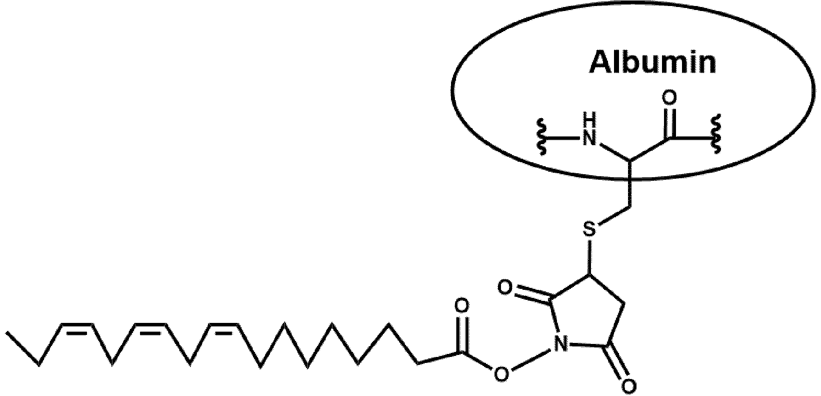

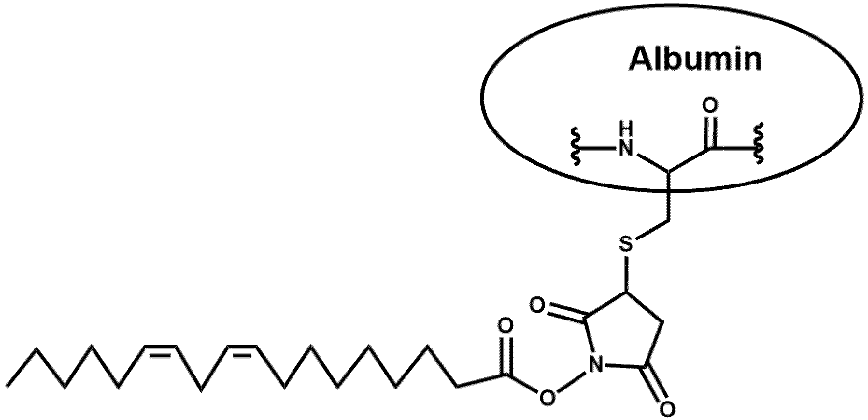

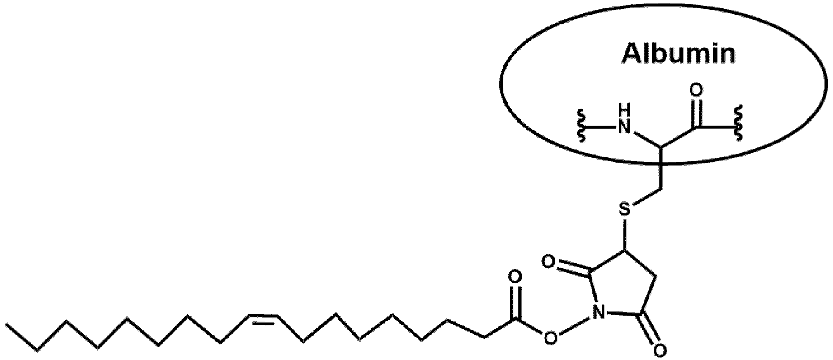

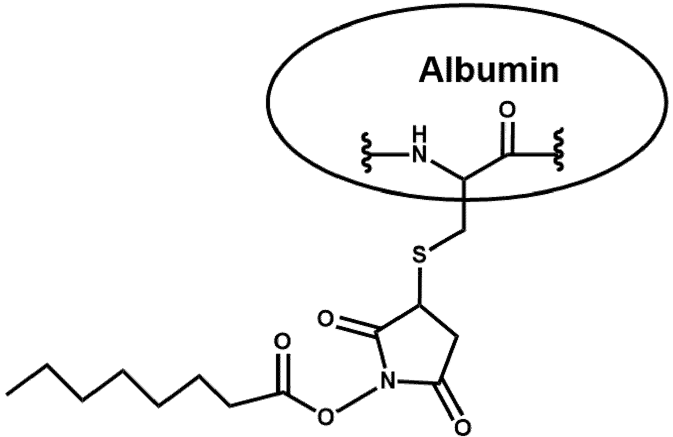

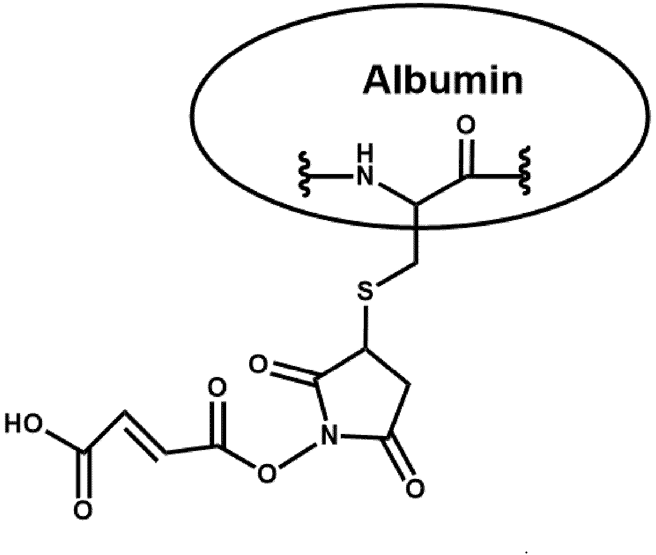

98. The water-soluble carbon chain substance according to claim 83, wherein the compound obtained by the reaction is any one or two or more compounds that are obtained by the reaction of a fatty acid and cystamine with dextran and have the following structural formulas:

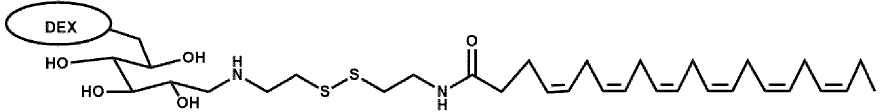

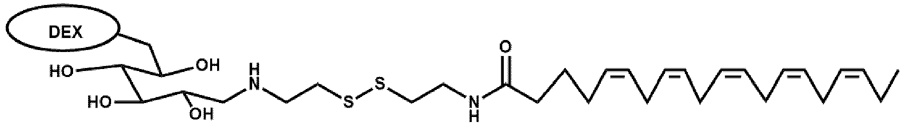

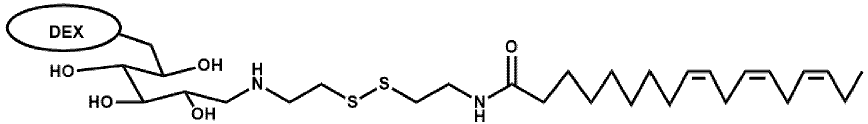

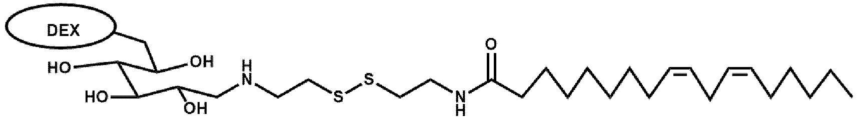

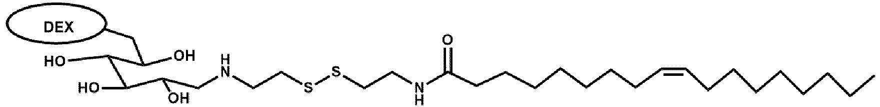

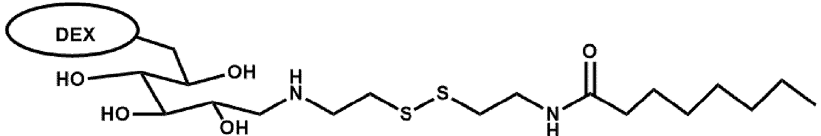

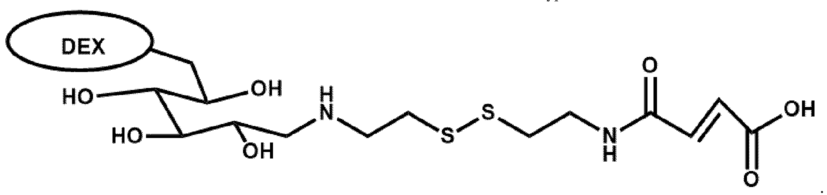

99. The water-soluble carbon chain substance according to claim 83, wherein the compound obtained by the reaction is any one or two or more compounds that are obtained by the reaction of a fatty acid, cystamine and hyaluronic acid and have the following structural formulas:

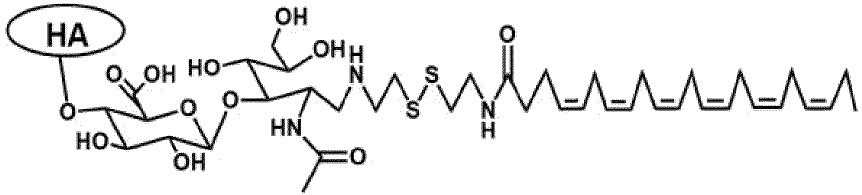

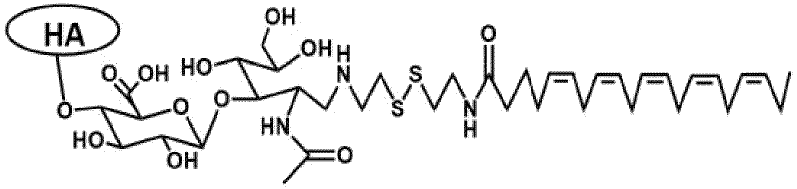

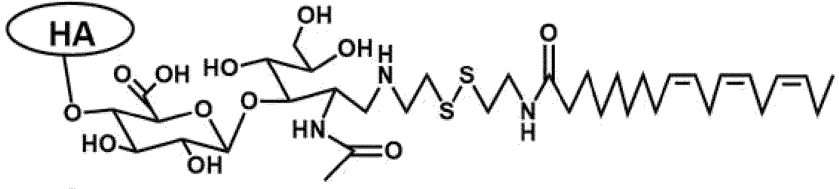

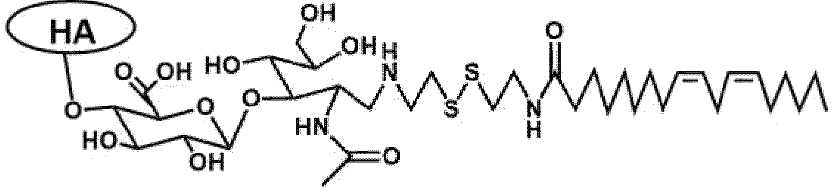

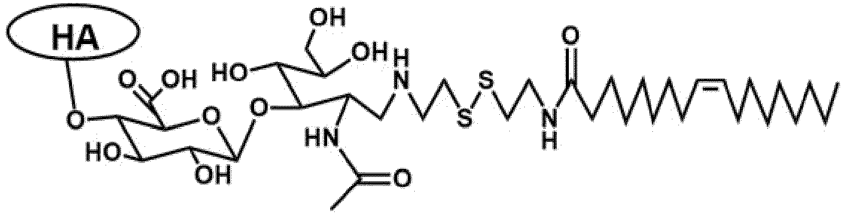

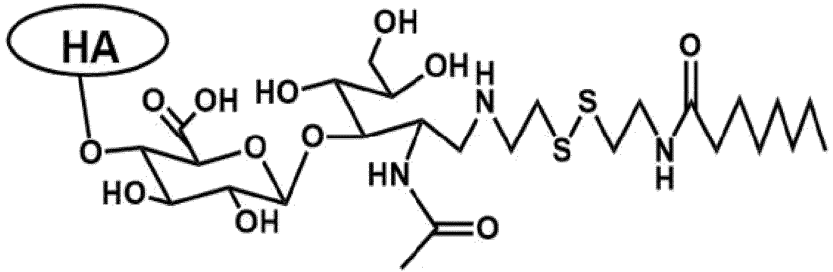

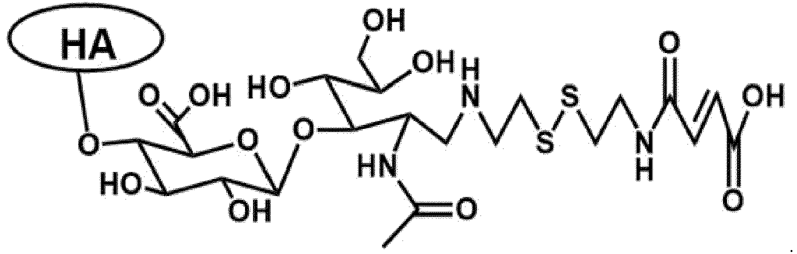

100. The water-soluble carbon chain substance of any one according to claims 59-72, comprising a small molecule compound comprising a hydroxyl group and a benzene ring; preferably, one or two or more substances selected from a group consisting of:
flavone, isoflavone, anthocyanin, *Glycine max* (soybean) isoflavone, *Aloe vera* (aloe) emodin, *Vitis vinifera* (grape) seed extract, green tea flavone, naringenin, *Citrus limon* (lemon) flavone, baicalein, riboflavin, quercetin, graphite flavone, *Cinnamomum cassia* (cinnamon) flavone, *Buxus sinica* (buxolin) flavone, *Crataegus pinnatifida* (hawthorn) flavone, morin, luteolin, *Melilotus officinalis(sweet* clover) flavone, gypsum flavone, *Lonicera japonica* (honeysuckle) flavone, *Gentiana scabra Bunge* (gentian) flavone, *Platycodon grandiflorus* (platycodon) flavone, *Viola phillipina* (Chinese violet) flavone, *Perilla frutescens* (perilla) flavone, *Dendranthema morifolium* (chrysanthemum) flavone, artemisinin, *Cynanchum officinale* (red peony) flavone, *Salvia miltiorrhiza Bunge* (salvia) flavone, *Saposhnikovia divaricata* flavone, *Chelonopsis pseudobracteata* (safflower) flavone, *Rhodiola rosea L.* (rhodiola) flavone, *Ziziphus jujuba var. inermis* (jujube) flavone, *Lycium chinense Mill.* (wolfberry) flavone, prepared *Rehmannia glutinosa* (rehmannia) flavone, *Ganoderma lucidum* flavone, *Schisandra chinensis* (schizandrin) flavone, *Glycyrrhiza uralensis* (licorice) flavone, *Panax pseudoginseng* (notoginseng) flavone, curcumin, apigenin, carotene, anthocyanin, lutein, zeaxanthin, Sinapis alba L. flavone, Ruta graveolens L. flavone, genkwanin, pollen flavone, *Hippophae rhamnoides L.* (sea-buckthorn) flavone, *Calendula officinalis L.* (marigold) flavone, cinnamon flavone, isoflavonoids and anthocyanins; rutin, emodin, fucoxanthin, gallic acid, persimmon peel extract, morin, echinacoside, grapeseed procyanidin, phenolic acid, tea polyphenol, naringin, citric acid, flavonol, glycyrrhizic acid, cinnamic acid, flavone glycoside, silymarin, matrine, tanshinone, Mangrove bark extract, hippophaetic acid, perillyl alcohol, anisic acid, amurensin , hesperidin, punicalin, morchellin, naringin, onionoside (Cleistocalyx operculatus extract), gentiopicrin, jasmine, naringol, carotene, apigenin, *Vatica mangachapoi Blanco* (green plum) extract, *Folium mori* (mulberry leaf) extract, loniceroside, *Dendranthema morifolium* (chrysanthemumin) extract, *Canarium album* (olive) extract, Oolong tea extract, theanine, vin rouge essence, platycodin, perillyl alcohol glycoside, mulberry bark extract, carthaminol, matsuba enzyme, pachymic acid, caffeic acid, chlorogenic acid, resveratrol, white tea polyphenol, resveratrol disaccharide, *Musa basjoo* (banana) lutein, anthocyanidin, arachidonic acid, *Arachis hypogaea* (peanut) flavone, xanthotol, anethol, flavonoid, baicalein, flavonoid glycoside, flavanol, vin rouge polyphenol, rhodiol, carthaminol, black tea flavone, black sesamin, *Secale cereale* (rye) phenol, trehalose alcohol, seaweed polysaccharide, alginic acid, *Albizzia julibrissin* extract (albizarin), cannabidiol, polydatin, cucurbitacin, *Trigonella foenum-graecum* (Huluba) extract, cucurbic acid, pollen phenol, pollen flavone, pollen glycoside, pollen ester, arachidic acid, peanut flavone glycoside, peanut isoflavone, peanut isoflavone glycoside, peanut isoflavone disaccharide, peanut isoflavone trisaccharide, peanut resveratrol, peanut resveratrol disaccharide, peanut resveratrol trisaccharide, peanut resveratrol tetrasaccharide, peanut resveratrol pentasaccharide, peanut resveratrol hexasaccharide, peanut resveratrol heptasaccharide, peanut resveratrol octasaccharide, peanut resveratrol nonasaccharide, peanut resveratrol decasaccharide, peanut resveratrol undecasaccharide, catechin, epicatechin, tea polyphenol, catechol, chlorophyll, protocatechin, hesperidin, anthocyanin, cyanine glucoside, cyanine aglycone, cyanine alcohol, glucoside, glucose aglycone, alfalfa extract, soybean isoflavone, flavanol, quercitannin, *Fagopyrum tataricum* (buckwheat) extract, persimmon peel extract, persimmon tannin, punicic acid, punicalin, blueberry extract, resveratrol, lycopene, naringenin, morin, chlorogenic acid, chlorogenic acid triglycoside, chlorogenic acid diglucoside, methyl chlorogenate, ethyl chlorogenate, propyl chlorogenate, butyl chlorogenate, isoamyl chlorogenate, hexyl chlorogenate, octyl chlorogenate, benzyl chlorogenate, phenethyl chlorogenate, phenylpropyl chlorogenate, phenylbutyl chlorogenate, phenyl isoamyl chlorogenate, phenylhexyl chlorogenate, phenyloctyl chlorogenate, styrene chlorogenate, chlorogenic acid benzyl alcohol ester, chlorogenic acid phenethyl alcohol ester, anthocyanin, proanthocyanidin glycoside, and catechin.

101. The water-soluble carbon chain substance according to any one of claims 59-72, wherein the water-soluble carbon chain substance includes a water-soluble mid-short chain fatty acid or fatty acid salt or fatty acid derivative, is preferably a water-soluble medium-short chain fatty acid or fatty acid salt or fatty acid derivative comprising one or two or more selected from a group consisting of:
water-soluble medium-short chain fatty acids such as butyric acid, succinic acid, fumaric acid, pentanoic acid, glutaric acid, hexanoic acid, hexane diacid, heptanoic acid, heptanedioic acid, octoic acid, octenoic acid, octanedioic acid, decanoic acid, decanedioic acid, and the like; and any one or more of the following fatty acid derivatives including surfactants and the like: fatty acid salts, alkyl sulfonic acid salts, alkyl sulfuric acid ester salts, alkyl phosphoric acid ester salts, alkyl amine salts, alkyl quaternary ammonium salts, fatty acyl amino acids, betaines, fatty alcohol polyoxyethylene ethers, alkylphenol polyoxyethylene ethers, fatty amine polyoxyethylene ethers, polyol fatty acid esters, polyol polyoxyethylene ether fatty acid esters, fatty acid polyoxyethylene esters, alkyl glycosides, and alcohol ether glycosides.

102. A preparation for regulating transmembrane transport of cell membranes, for regulating structure or function of cell membrane, for regulating cell division proliferation or cell migration, for regulating cell senescence, and/or for regulating cell membrane fluidity manufactured by the water-soluble carbon chain substance according to any one of claims 59-101

103. The preparation according to claim 102, wherein the regulating cell membrane fluidity refers to increasing the fluidity of the cell membrane.

104. The preparation according to claim 102, wherein the regulating cell membrane fluidity refers to decreasing the fluidity of the cell membrane.

105. The preparation according to claim 102, wherein the cell membrane comprises a cytoplasmic membrane and an organelle membrane; the cell membrane or cell comprises a cell membrane or cell of a microorganism, or a cell membrane or cell of a plant, animal, or human body tissue; the regulating comprises increasing or promoting, and inhibiting or decreasing, wherein the cell membranes of microorganisms include cell membranes of bacteria and fungi and envelopes of viruses;
wherein the cell membrane structure comprises a phospholipid bilayer, proteins, and polysaccharides adsorbed on the surface of the phospholipid bilayer and embedded throughout the phospholipid bilayer, specifically, including receptor proteins, transmembrane proteins, cytoskeletal proteins, and enzymes; wherein, the transmembrane transport includes active transport, passive transport, and endocytosis.

106. The preparation according to claim 102, wherein the regulating transmembrane transport of cell membranes comprises increasing and promoting the release of extracellular vesicles from inside cells to outside cells; also comprises inhibiting or reducing the release of extracellular vesicles from inside cells to outside cells; and can prevent the virus from entering the cell; the inhibiting or reducing the transmembrane transport of the virus is inhibiting or reducing the entry of the virus into the cell by endocytosis or inhibiting or reducing viral entry into the cell through fusion of the viral envelope with the cell membrane; it is applied to prevention of all viral infections, prevention of mycoplasma and chlamydial infections, prevention of bacterial infections; substances of transmembrane transport include: at least one or two or more of a small molecule compound, a medium molecule compound, a macromolecule compound, a pathogenic microorganism such as a virus, a bacterium, a mycoplasma, a chlamydia and a fungus and the like, and a nanoparticle and a nano-drug.

107. The preparation according to claim 103, wherein the preparation for increasing cell membrane fluidity comprises a preparation for preventing, blocking, ameliorating and/or treating drug delivery, gene transfection, cell therapy, diabetes, amelioration of the nervous system, Alzheimer's disease, enhancement of the immune system, hypercholesterolemia, and/or inflammatory bowel disease.

108. The preparation according to claim 104, wherein the preparation for reducing cell membrane fluidity comprises a preparation for preventing, blocking, ameliorating and/or treating cardiovascular diseases (including coronary heart disease, hypertension, myocardial infarction, heart failure), obesity, autism, osteoporosis, inflammatory diseases, autoimmune diseases, chronic fatigue syndrome, leukemia, cell autolysis, viral infection, and/or neurodegenerative diseases.

109. A preparation for preventing, blocking or treating microbial infections prepared using the water-soluble carbon chain substance according to any one of claims 59-101.

110. The preparation according to claim 102, wherein the preparation is pharmaceutical preparation or an environmental disinfection and sterilization preparation, and the pharmaceutical preparation is preferably one selected from the group consisting of an inhalant, a nasal spray, an injection, an oral preparation, and a transdermal topical formulation.

111. The use of the water-soluble carbon chain substance according to any one of claims 59-101 in the preparation of a pharmaceutical preparation for preventing, blocking and/or treating microbial infections or environmental microbial disinfection and sterilization reagents, wherein preferably, the microorganism is any one or two selected from the group consisting of viruses, bacteria, and fungi; the virus is an enveloped virus and/or non enveloped viruses; more preferably, the virus is one or two or more viruses selected from the group consisting of novel coronavirus, influenza virus, AIDS virus (HIV), hepatitis B virus, human herpes virus, Ebola virus, rabies virus, and a human papilloma virus (HPV), and the bacteria is one or two or more bacteria selected from the group consisting of *Escherichia coli, Staphylococcus aureus,* methicillin-resistant *Staphylococcus aureus, Streptococcus pneumoniae, Klebsiella pneumoniae* and *Pseudomonas aeruginosa;* more further preferably, the virus is one or two of more selected from the group consisting of H7N9 influenza virus, H5N1 influenza virus, HIV virus, novel coronavirus, HPV virus, and rabies virus.

112. The use according to claim 111, wherein the water-soluble carbon chain substance according to any one of claims 59-99, when applied to a microorganism, is formulated to a solution having a concentration of 0.2 mM-10 mM.

113. The use according to claim 111, wherein the water-soluble carbon chain substance is formulated to a solution having a concentration of 0.5 mM-10 mM.

114. A preparation for preventing, blocking, or treating inflammatory response and/or body aging made using the water-soluble carbon chain substance according to any one of claims 59-101.

115. The preparation according to claim 114, wherein the resisting body aging comprises resisting skin aging and preventing, blocking, or treating the inflammatory response comprises altering the conformation of an inflammatory factor (protein).

116. The preparation according to claim 114, wherein the carbon chain substance binds to an oxygen free radical.

117. The preparation according to claim 114, wherein the carbon chain substance binds to or is, or mixed to obtain a preparation with, one or two or more selected from a group consisting of:
flavone, isoflavone, anthocyanin, *Glycine max* (soybean) isoflavone, *Aloe vera* (aloe) emodin, *Vitis vinifera* (grape) seed extract, green tea flavone, naringenin, *Citrus limon* (lemon) flavone, baicalein, riboflavin, quercetin, graphite flavone, *Cinnamomum cassia* (cinnamon) flavone, *Buxus sinica* (buxolin) flavone, *Crataegus pinnatifida* (hawthorn) flavone, morin, luteolin, *Melilotus officinalis(sweet* clover) flavone, gypsum flavone, *Lonicera japonica* (honeysuckle) flavone, *Gentiana scabra Bunge* (gentian) flavone, *Platycodon grandiflorus* (platycodon) flavone, *Viola phillipina* (Chinese violet) flavone, *Perilla frutescens* (perilla) flavone, *Dendranthema morifolium* (chrysanthemum) flavone, artemisinin, *Cynanchum officinale* (red peony) flavone, *Salvia miltiorrhiza Bunge* (salvia) flavone, *Saposhnikovia divaricata* flavone, *Chelonopsis pseudobracteata* (safflower) flavone, *Rhodiola rosea L.* (rhodiola) flavone, *Ziziphus jujuba var. inermis* (jujube) flavone, *Lycium chinense Mill.* (wolfberry) flavone, prepared *Rehmannia glutinosa* (rehmannia) flavone, *Ganoderma lucidum* flavone, *Schisandra chinensis* (schizandrin) flavone, *Glycyrrhiza uralensis* (licorice) flavone, *Panax pseudoginseng* (notoginseng) flavone, curcumin, apigenin, carotene, anthocyanin, lutein, zeaxanthin, Sinapis alba L. flavone, Ruta graveolens L. flavone, genkwanin, pollen flavone, *Hippophae rhamnoides L.* (sea-buckthorn) flavone, *Calendula officinalis L.* (marigold) flavone, cinnamon flavone, isoflavonoids and anthocyanins; rutin, emodin, fucoxanthin, gallic acid, persimmon peel extract, morin, echinacoside, grapeseed procyanidin, phenolic acid, tea polyphenol, naringin, citric acid, flavonol, glycyrrhizic acid, cinnamic acid, flavone glycoside, silymarin, matrine, tanshinone, Mangrove bark extract, hippophaetic acid, perillyl alcohol, anisic acid, amurensin hesperidin, punicalin, morchellin, naringin, onionoside (Cleistocalyx operculatus extract), gentiopicrin, jasmine, naringol, carotene, apigenin, *Vatica mangachapoi Blanco* (green plum) extract, *Folium mori* (mulberry leaf) extract, loniceroside, *Dendranthema morifolium* (chrysanthemumin) extract, *Canarium album* (olive) extract, Oolong tea extract, theanine, vin rouge essence, platycodin, perillyl alcohol glycoside, mulberry bark extract, carthaminol, matsuba enzyme, pachymic acid, caffeic acid, chlorogenic acid, resveratrol, white tea polyphenol, resveratrol disaccharide, *Musa basjoo* (banana) lutein, anthocyanidin, arachidonic acid, *Arachis hypogaea* (peanut) flavone, xanthotol, anethol, flavonoid, baicalein, flavonoid glycoside, flavanol, vin rouge polyphenol, rhodiol, carthaminol, black tea flavone, black sesamin, *Secale cereale* (rye) phenol, trehalose alcohol, seaweed polysaccharide, alginic acid, *Albizzia julibrissin* extract (albizarin), cannabidiol, polydatin, cucurbitacin, *Trigonella foenum-graecum* (Huluba) extract, cucurbic acid, pollen phenol, pollen flavone, pollen glycoside, pollen ester, arachidic acid, peanut flavone glycoside, peanut isoflavone, peanut isoflavone glycoside, peanut isoflavone disaccharide, peanut isoflavone trisaccharide, peanut resveratrol, peanut resveratrol disaccharide, peanut resveratrol trisaccharide, peanut resveratrol tetrasaccharide, peanut resveratrol pentasaccharide, peanut resveratrol hexasaccharide, peanut resveratrol heptasaccharide, peanut resveratrol octasaccharide, peanut resveratrol nonasaccharide, peanut resveratrol decasaccharide, peanut resveratrol undecasaccharide, catechin, epicatechin, tea polyphenol, catechol, chlorophyll, protocatechin, hesperidin, anthocyanin, cyanine glucoside, cyanine aglycone, cyanine alcohol, glucoside, glucose aglycone, alfalfa extract, soybean isoflavone, flavanol, quercitannin, *Fagopyrum tataricum* (buckwheat) extract, persimmon peel extract, persimmon tannin, punicic acid, punicalin, blueberry extract, resveratrol, lycopene, naringenin, morin, chlorogenic acid, chlorogenic acid triglycoside, chlorogenic acid diglucoside, methyl chlorogenate, ethyl chlorogenate, propyl chlorogenate, butyl chlorogenate, isoamyl chlorogenate, hexyl chlorogenate, octyl chlorogenate, benzyl chlorogenate, phenethyl chlorogenate, phenylpropyl chlorogenate, phenylbutyl chlorogenate, phenyl isoamyl chlorogenate, phenylhexyl chlorogenate, phenyloctyl chlorogenate, styrene chlorogenate, chlorogenic acid benzyl alcohol ester, chlorogenic acid phenethyl alcohol ester, anthocyanin, proanthocyanidin glycoside, and catechin.

118. The preparation according to any one of claims 114-117 for use in preventing, blocking or treating an inflammatory response, and/or body ageing, wherein the water-soluble carbon chain substance is formulated as a solution having a concentration of 0.1 nM-5 mM; preferably the carbon chain substance is a complex of a fatty acid and an amino acid, more preferably the concentration of the complex solution is 0.1 nM-300 µ M, more preferably 5 nM-100 µ M.

119. The preparation according to claim 118 for use in preventing, blocking or treating an inflammatory response, and/or body ageing, wherein the water-soluble carbon chain substance is formulated to a solution having a concentration of 0.1 nM-300 µ M, preferably 5 nM-100 µ M.

120. A pharmaceutical preparation for preventing, blocking, slowing down, or treating neurodegenerative diseases made from the water-soluble carbon chain substance according to any one of claims 59-101.

121. The pharmaceutical preparation according to claim 120, wherein the neurodegenerative disease comprises Alzheimer's disease (AD), Parkinson's disease (PD), Huntington's disease (HD), senile dementia, amyotrophic lateral sclerosis (ALS), different types of spinocerebellar ataxia (SCA), or Pick's disease.

122. The pharmaceutical preparation according to claim 120, wherein the water-soluble carbon chain substance is formulated to function at a concentration of 0.1 nM-5 mM.

123. The pharmaceutical preparation according to claim 120 wherein the water-soluble carbon chain substance is formulated to act as a solution having a concentration of 0.1 nM-300 µ M, preferably 5 nM-100 µ M.
